# EUROPEAN PATENT APPLICATION

(11) **EP 2 426 213 A1**
(43) Date of publication of application: **07.03.2012**
(21) Application number: 10075384.7
(22) Date of filing: 03.09.2010
(51) Int. Cl.: C12Q 1/68

(54) **Marker for sunitnib resistance formation**

(71) Applicant: Max-Planck-Gesellschaft zur Förderung der Wissenschaften e.V., 80539 München (DE)
(72) Inventor: Bender, Claus, 82152 Martinsried (DE); Ullrich, Axel, 80331 Munich (DE)
(74) Representative: Arth, Hans-Lothar

(57) **Abstract**

The present invention relates to marker for sunitinib resistance formation and modulators thereof for the prevention and reduction of sunitinib resistance of hyperproliferative cells, in particular cancer cells. Further, the present invention relates to compositions comprising agents suitable for anticancer therapy in combination with the modulators for treating proliferative diseases.

SEQUENCE LISTING

<110>  Max-Planck-Gesellschaft zur Förderung der Wissenschaften
       e.V.

<120>  Marker for sunitinib resistance formation

<130>  ULA-P03214EP

<160>  16

<170>  PatentIn version 3.3

<210>  1
<211>  358
<212>  PRT
<213>  Homo sapiens

<400>  1

Met Glu Ala Pro Gly Leu Ala Gln Ala Ala Ala Ala Glu Ser Asp Ser
1               5                   10                  15

Arg Lys Val Ala Glu Glu Thr Pro Asp Gly Ala Pro Ala Leu Cys Pro
            20                  25                  30

Ser Pro Glu Ala Leu Ser Pro Glu Pro Pro Val Tyr Ser Leu Gln Asp
        35                  40                  45

Phe Asp Thr Leu Ala Thr Val Gly Thr Gly Thr Phe Gly Arg Val His
    50                  55                  60

Leu Val Lys Glu Lys Thr Ala Lys His Phe Phe Ala Leu Lys Val Met
65                  70                  75                  80

Ser Ile Pro Asp Val Ile Arg Leu Lys Gln Glu Gln His Val His Asn
                85                  90                  95

Glu Lys Ser Val Leu Lys Glu Val Ser His Pro Phe Leu Ile Arg Leu
            100                 105                 110

Phe Trp Thr Trp His Asp Glu Arg Phe Leu Tyr Met Leu Met Glu Tyr
        115                 120                 125

Val Pro Gly Gly Glu Leu Phe Ser Tyr Leu Arg Asn Arg Gly Arg Phe
    130                 135                 140

Ser Ser Thr Thr Gly Leu Phe Tyr Ser Ala Glu Ile Ile Cys Ala Ile
145                 150                 155                 160

Glu Tyr Leu His Ser Lys Glu Ile Val Tyr Arg Asp Leu Lys Pro Glu
                165                 170                 175

Asn Ile Leu Leu Asp Arg Asp Gly His Ile Lys Leu Thr Asp Phe Gly
            180                 185                 190

Phe Ala Lys Lys Leu Val Asp Arg Thr Trp Thr Leu Cys Gly Thr Pro
        195                 200                 205

Glu Tyr Leu Ala Pro Glu Val Ile Gln Ser Lys Gly His Gly Arg Ala
    210                 215                 220

Val Asp Trp Trp Ala Leu Gly Ile Leu Ile Phe Glu Met Leu Ser Gly
225                 230                 235                 240

Phe Pro Pro Phe Phe Asp Asp Asn Pro Phe Gly Ile Tyr Gln Lys Ile
                245                 250                 255

Leu Ala Gly Lys Ile Asp Phe Pro Arg His Leu Asp Phe His Val Lys
            260                 265                 270

Asp Leu Ile Lys Lys Leu Leu Val Val Asp Arg Thr Arg Arg Leu Gly
        275                 280                 285

Asn Met Lys Asn Gly Ala Asn Asp Val Lys His His Arg Trp Phe Arg
    290                 295                 300

Ser Val Asp Trp Glu Ala Val Pro Gln Arg Lys Leu Lys Pro Pro Ile
305                 310                 315                 320

Val Pro Lys Ile Ala Gly Asp Gly Asp Thr Ser Asn Phe Glu Thr Tyr
                325                 330                 335

Pro Glu Asn Asp Trp Asp Thr Ala Ala Pro Val Pro Gln Lys Asp Leu
            340                 345                 350

Glu Ile Phe Lys Asn Phe
        355

<210>  2
<211>  1649
<212>  PRT
<213>  Homo sapiens

<400>  2

Met Glu Ser Val Glu Lys Asp Trp Gly Trp Gly Leu Trp Arg Asn Leu
1               5                   10                  15

Arg Cys Leu Gly His Ala His Gln Gly Lys Cys Cys Thr Glu Gly Gly
            20                  25                  30

Ile Ser Ser Thr Thr Lys Thr Ser Ser Glu Asn Gly Ser Cys Cys Phe
        35                  40                  45

Glu Lys Ala Ala Arg Lys Asp His Val Cys Arg Phe Ile Gly Cys Gly
    50                  55                  60

Arg Asn Asp Arg Phe Asn Tyr Val Val Met Gln Leu Gln Gly Arg Asn

65                  70                  75                  80

Leu Ala Asp Leu Arg Arg Ser Gln Ser Arg Gly Thr Phe Thr Ile Ser
                85                  90                  95

Thr Thr Leu Arg Leu Gly Arg Gln Ile Leu Glu Ser Ile Glu Ser Ile
            100                 105                 110

His Ser Val Gly Phe Leu His Arg Asp Ile Lys Pro Ser Asn Phe Ala
        115                 120                 125

Met Gly Arg Phe Pro Ser Thr Cys Arg Lys Cys Tyr Met Leu Asp Phe
    130                 135                 140

Gly Leu Ala Arg Gln Phe Thr Asn Ser Cys Gly Asp Val Arg Pro Pro
145                 150                 155                 160

Arg Ala Val Ala Gly Phe Arg Gly Thr Val Arg Tyr Ala Ser Ile Asn
                165                 170                 175

Ala His Arg Asn Arg Glu Met Gly Arg His Asp Asp Leu Trp Ser Leu
            180                 185                 190

Phe Tyr Met Leu Val Glu Phe Val Val Gly Gln Leu Pro Trp Arg Lys
        195                 200                 205

Ile Lys Asp Lys Glu Gln Val Gly Ser Ile Lys Glu Arg Tyr Asp His
    210                 215                 220

Arg Leu Met Leu Lys His Leu Pro Pro Glu Phe Ser Ile Phe Leu Asp
225                 230                 235                 240

His Ile Ser Ser Leu Asp Tyr Phe Thr Lys Pro Asp Tyr Gln Met Ser
                245                 250                 255

Ile Arg Val Thr Arg Lys Ser Tyr Lys Val Ser Thr Ser Gly Pro Gln
            260                 265                 270

Ala Phe Asn Ser Leu Ser Tyr Thr Ser Gly Pro Gly Ala Cys Ile Ser
        275                 280                 285

Ser Ser Ser Phe Ser Arg Met Gly Ser Ser Ser Phe Arg Gly Gly Leu
    290                 295                 300

Gly Ala Gly Tyr Gly Gly Ala Ser Gly Gly Ile Thr Thr Ile Thr Val
305                 310                 315                 320

Asn Gln Ser Leu Leu Ser Pro Leu Asn Leu Glu Val Asp Pro Asn Ile
                325                 330                 335

Gln Ala Val Arg Thr Gln Glu Glu Lys Gln Ile Lys Thr Leu Asn Asn

            340                 345                 350

Lys Phe Phe Ser Phe Ile Asp Lys Val Arg Phe Leu Glu Gln Gln Asn
        355                 360                 365

Lys Met Leu Glu Thr Lys Trp Ser Leu Val Gln Gln Gln Lys Met Ala
    370                 375                 380

Arg Ser Asn Met Asp Asn Met Phe Glu Ser Tyr Ile Asn Asn Leu Lys
385                 390                 395                 400

Trp Gln Leu Glu Thr Leu Gly Gln Glu Lys Leu Lys Leu Glu Ala Glu
                405                 410                 415

Leu Gly Asn Met His Gly Leu Val Glu Asp Phe Arg Asn Lys Tyr Glu
            420                 425                 430

Val Glu Ile Ser Lys Cys Thr Glu Met Glu Asn Glu Phe Val Leu Ile
        435                 440                 445

Lys Glu Tyr Val Asp Glu Ala Tyr Met Asn Lys Met Glu Leu Glu Ser
    450                 455                 460

Ser Leu Lys Glu Leu Thr Ala Lys Ile Ser Phe Leu Arg Gln Leu Tyr
465                 470                 475                 480

Glu Glu Glu Ile Gly Ser Cys Ser Pro Arg Ser Arg Ile His Leu Trp
                485                 490                 495

Cys Cys Ser Trp Thr Thr Ala Ala Ser Trp Thr Trp Thr Ala Ser Ser
            500                 505                 510

Leu Arg Ser Arg Pro Glu Gly Phe Pro Gly Gly Cys His Ala Asp Thr
        515                 520                 525

Glu Gln Arg Gly Glu Leu Ala Ile Lys Asp Ala Asn Ala Lys Leu Ser
    530                 535                 540

Glu Leu Glu Ala Ala Leu Gln Leu Ala Ser Gln Asp Met Ala Arg Gln
545                 550                 555                 560

Leu Arg Glu Tyr Gln Glu Leu Met Asn Val Lys Leu Ala Leu Asp Ile
                565                 570                 575

Lys Ile Ala Thr Tyr Arg Lys Leu Leu Glu Gly Glu Glu Ser Trp Leu
            580                 585                 590

Glu Ser Gly Met Gln Asn Met Ser Ile His Met Lys Thr Thr Ser Ser
        595                 600                 605

Tyr Ala Gly Gly Gln Ser Leu Ala Tyr Gly Gly Leu Thr Ser Pro Gly

610                 615                 620

Leu Ser Tyr Gly Leu Gly Ser Ser Phe Gly Ser Gly Met Gly Ser Ser
625                 630                 635                 640

Ser Phe Ser His Thr Ser Ser Ser Arg Ala Val Val Met Lys Lys Ile
                645                 650                 655

Glu Thr Arg Asp Gly Lys Leu Val Ser Glu Ser Ser Asn Val Leu Pro
            660                 665                 670

Asn Leu Arg Ile Thr Ala Thr Leu Leu Thr Ser Val Phe Asp Asn Ser
        675                 680                 685

Ile Lys Thr Phe Gly Val Ile Glu Ser Asp Pro Phe Asp Trp Glu Lys
    690                 695                 700

Thr Gly Asn Asp Gly Ser Leu Thr Thr Thr Thr Thr Ser Thr Thr Pro
705                 710                 715                 720

Gln Leu His Thr Arg Leu Thr Pro Ala Ala Ile Gly Ile Ala Asn Ala
                725                 730                 735

Thr Pro Ile Pro Gly Asp Leu Leu Arg Glu Asn Thr Asp Glu Val Phe
            740                 745                 750

Pro Asp Glu Gln Leu Ser Asp Gly Glu Asn Gly Ile Pro Val Gly Val
        755                 760                 765

Ser Pro Asp Lys Leu Pro Gly Ser Leu Gly His Pro Arg Pro Gln Glu
    770                 775                 780

Lys Asp Val Trp Glu Glu Met Asp Ala Asn Lys Asn Lys Ile Lys Leu
785                 790                 795                 800

Gly Ile Cys Lys Ala Ala Thr Glu Glu Glu Asn Ser His Gly Gln Ala
                805                 810                 815

Asn Gly Leu Leu Asn Ala Pro Ser Leu Gly Ser Pro Ile Arg Val Arg
            820                 825                 830

Ser Glu Ile Thr Gln Pro Asp Arg Asp Ile Pro Leu Val Arg Lys Leu
        835                 840                 845

Arg Ser Ile His Ser Phe Glu Leu Glu Lys Arg Leu Thr Leu Glu Pro
    850                 855                 860

Lys Pro Asp Thr Asp Lys Phe Leu Glu Thr Cys Leu Glu Lys Met Gln
865                 870                 875                 880

Lys Asp Thr Ser Ala Gly Lys Glu Ser Ile Leu Pro Ala Leu Leu His

                885                 890                 895

Lys Pro Cys Val Pro Ala Val Ser Arg Thr Asp His Ile Trp His Tyr
            900                 905                 910

Asp Glu Glu Tyr Leu Pro Asp Ala Ser Lys Pro Ala Ser Ala Asn Thr
        915                 920                 925

Pro Glu Gln Ala Asp Gly Gly Gly Ser Asn Gly Phe Ile Ala Val Asn
    930                 935                 940

Leu Ser Ser Cys Lys Gln Glu Ile Asp Ser Lys Glu Trp Val Ile Val
945                 950                 955                 960

Asp Lys Glu Gln Asp Leu Gln Asp Phe Arg Thr Asn Glu Ala Val Gly
                965                 970                 975

His Lys Thr Thr Gly Ser Pro Ser Asp Glu Glu Pro Glu Val Leu Gln
            980                 985                 990

Val Leu Glu Ala Ser Pro Gln Asp Glu Lys Leu Gln Leu Gly Pro Trp
        995                 1000                1005

Ala Glu Asn Asp His Leu Lys Lys Glu Thr Ser Gly Val Val Leu
    1010                1015                1020

Ala Leu Ser Ala Glu Gly Pro Pro Thr Ala Ala Ser Glu Gln Tyr
    1025                1030                1035

Thr Asp Arg Leu Glu Leu Gln Pro Gly Ala Ala Ser Gln Phe Ile
    1040                1045                1050

Ala Ala Thr Pro Thr Ser Leu Met Glu Ala Gln Ala Glu Gly Pro
    1055                1060                1065

Leu Thr Ala Ile Thr Ile Pro Arg Pro Ser Val Ala Ser Thr Gln
    1070                1075                1080

Ser Thr Ser Gly Ser Phe His Cys Gly Gln Gln Pro Glu Lys Lys
    1085                1090                1095

Asp Leu Gln Pro Met Glu Pro Thr Val Glu Leu Tyr Ser Pro Arg
    1100                1105                1110

Glu Asn Phe Ser Gly Leu Val Val Thr Glu Gly Glu Pro Pro Ser
    1115                1120                1125

Gly Gly Ser Arg Thr Asp Leu Gly Leu Gln Ile Asp His Ile Gly
    1130                1135                1140

His Asp Met Leu Pro Asn Ile Arg Glu Ser Asn Lys Ser Gln Asp

1145                1150                1155

Leu Gly  Pro Lys Glu Leu Pro  Asp His Asn Arg Leu  Val Val Arg
    1160                1165                1170

Glu Phe  Glu Asn Leu Pro Gly  Glu Thr Glu Glu Lys  Ser Ile Leu
    1175                1180                1185

Leu Glu  Ser Asp Asn Glu Asp  Glu Lys Leu Ser Arg  Gly Gln His
    1190                1195                1200

Cys Ile  Glu Ile Ser Ser Leu  Pro Gly Asp Leu Val  Ile Val Glu
    1205                1210                1215

Lys Asp  His Ser Ala Thr Thr  Glu Pro Leu Asp Val  Thr Lys Thr
    1220                1225                1230

Gln Thr  Phe Ser Val Val Pro  Asn Gln Asp Lys Asn  Asn Glu Ile
    1235                1240                1245

Met Lys  Leu Leu Thr Val Gly  Thr Ser Glu Ile Ser  Ser Arg Asp
    1250                1255                1260

Ile Asp  Pro His Val Glu Gly  Gln Ile Gly Gln Val  Ala Glu Met
    1265                1270                1275

Gln Lys  Asn Lys Ile Ser Lys  Asp Asp Asp Ile Met  Ser Glu Asp
    1280                1285                1290

Leu Pro  Gly His Gln Gly Asp  Leu Ser Thr Phe Leu  His Gln Glu
    1295                1300                1305

Gly Lys  Arg Glu Lys Ile Thr  Pro Arg Asn Gly Glu  Leu Phe His
    1310                1315                1320

Cys Val  Ser Glu Asn Glu His  Gly Ala Pro Thr Arg  Lys Asp Met
    1325                1330                1335

Val Arg  Ser Ser Phe Val Thr  Arg His Ser Arg Ile  Pro Val Leu
    1340                1345                1350

Ala Gln  Glu Ile Asp Ser Thr  Leu Glu Ser Ser Ser  Pro Val Ser
    1355                1360                1365

Ala Lys  Glu Lys Leu Leu Gln  Lys Lys Ala Tyr Gln  Pro Asp Leu
    1370                1375                1380

Val Lys  Leu Leu Val Glu Lys  Arg Gln Phe Lys Ser  Phe Leu Gly
    1385                1390                1395

Asp Leu  Ser Ser Ala Ser Asp  Lys Leu Leu Glu Glu  Lys Leu Ala

1400                 1405                 1410

Thr Val Pro Ala Pro Phe Cys Glu Glu Glu Val Leu Thr Pro Phe
    1415                 1420                 1425

Ser Arg Leu Thr Val Asp Ser His Leu Ser Arg Ser Ala Glu Asp
    1430                 1435                 1440

Ser Phe Leu Ser Pro Ile Ile Ser Gln Ser Arg Lys Ser Lys Ile
    1445                 1450                 1455

Pro Arg Pro Val Ser Trp Val Asn Thr Asp Gln Val Asn Ser Ser
    1460                 1465                 1470

Thr Ser Ser Gln Phe Phe Pro Arg Pro Pro Pro Gly Lys Pro Pro
    1475                 1480                 1485

Thr Arg Pro Gly Val Glu Ala Arg Leu Arg Arg Tyr Lys Val Leu
    1490                 1495                 1500

Gly Ser Ser Asn Ser Asp Ser Asp Leu Phe Ser Arg Leu Ala Gln
    1505                 1510                 1515

Ile Leu Gln Asn Gly Ser Gln Lys Pro Arg Ser Thr Thr Gln Cys
    1520                 1525                 1530

Lys Ser Pro Gly Ser Pro His Asn Pro Lys Thr Pro Pro Lys Ser
    1535                 1540                 1545

Pro Val Val Pro Arg Arg Ser Pro Ser Ala Ser Pro Arg Ser Ser
    1550                 1555                 1560

Ser Leu Pro Arg Thr Ser Ser Ser Ser Pro Ser Arg Ala Gly Arg
    1565                 1570                 1575

Pro His His Asp Gln Arg Ser Ser Ser Pro His Leu Gly Arg Ser
    1580                 1585                 1590

Lys Ser Pro Pro Ser His Ser Gly Ser Ser Ser Ser Arg Arg Ser
    1595                 1600                 1605

Cys Gln Gln Glu His Cys Lys Pro Ser Lys Asn Gly Leu Lys Gly
    1610                 1615                 1620

Ser Gly Ser Leu His His His Ser Ala Ser Thr Lys Thr Pro Gln
    1625                 1630                 1635

Gly Lys Ser Lys Pro Ala Ser Lys Leu Ser Arg
    1640                 1645

<210>  3

<211>  745
<212>  PRT
<213>  Homo sapiens

<400>  3

Met Leu Pro Phe Ala Pro Gln Asp Glu Pro Trp Asp Arg Glu Met Glu
1               5                   10                  15

Val Phe Ser Gly Gly Gly Ala Ser Ser Gly Glu Val Asn Gly Leu Lys
            20                  25                  30

Met Val Asp Glu Pro Met Glu Glu Gly Glu Ala Asp Ser Cys His Asp
        35                  40                  45

Glu Gly Val Val Lys Glu Ile Pro Ile Thr His His Val Lys Glu Gly
    50                  55                  60

Tyr Glu Lys Ala Asp Pro Ala Gln Phe Glu Leu Leu Lys Val Leu Gly
65                  70                  75                  80

Gln Gly Ser Phe Gly Lys Val Phe Leu Val Arg Lys Lys Thr Gly Pro
                85                  90                  95

Asp Ala Gly Gln Leu Tyr Ala Met Lys Val Leu Lys Lys Ala Ser Leu
            100                 105                 110

Lys Val Arg Asp Arg Val Arg Thr Lys Met Glu Arg Asp Ile Leu Val
        115                 120                 125

Glu Val Asn His Pro Phe Ile Val Lys Leu His Tyr Ala Phe Gln Thr
    130                 135                 140

Glu Gly Lys Leu Tyr Leu Ile Leu Asp Phe Leu Arg Gly Gly Asp Val
145                 150                 155                 160

Phe Thr Arg Leu Ser Lys Glu Val Leu Phe Thr Glu Glu Asp Val Lys
                165                 170                 175

Phe Tyr Leu Ala Glu Leu Ala Leu Ala Leu Asp His Leu His Gln Leu
            180                 185                 190

Gly Ile Val Tyr Arg Asp Leu Lys Pro Glu Asn Ile Leu Leu Asp Glu
        195                 200                 205

Ile Gly His Ile Lys Leu Thr Asp Phe Gly Leu Ser Lys Glu Ser Val
    210                 215                 220

Asp Gln Glu Lys Lys Ala Tyr Ser Phe Cys Gly Thr Val Glu Tyr Met
225                 230                 235                 240

Ala Pro Glu Val Val Asn Arg Arg Gly His Ser Gln Ser Ala Asp Trp
                245                 250                 255

Trp Ser Tyr Gly Val Leu Met Phe Glu Met Leu Thr Gly Thr Leu Pro
            260                 265                 270

Phe Gln Gly Lys Asp Arg Asn Glu Thr Met Asn Met Ile Leu Lys Ala
        275                 280                 285

Lys Leu Gly Met Pro Gln Phe Leu Ser Ala Glu Ala Gln Ser Leu Leu
    290                 295                 300

Arg Met Leu Phe Lys Arg Asn Pro Ala Asn Arg Leu Gly Ser Glu Gly
305                 310                 315                 320

Val Glu Glu Ile Lys Arg His Leu Phe Phe Ala Asn Ile Asp Trp Asp
                325                 330                 335

Lys Leu Tyr Lys Arg Glu Val Gln Pro Pro Phe Lys Pro Ala Ser Gly
            340                 345                 350

Lys Pro Asp Asp Thr Phe Cys Phe Asp Pro Glu Phe Thr Ala Lys Thr
        355                 360                 365

Pro Lys Asp Ser Pro Gly Leu Pro Ala Ser Ala Asn Ala His Gln Leu
    370                 375                 380

Phe Lys Gly Phe Ser Phe Val Ala Thr Ser Ile Ala Glu Glu Tyr Lys
385                 390                 395                 400

Ile Thr Pro Ile Thr Ser Ala Asn Val Leu Pro Ile Val Gln Ile Asn
                405                 410                 415

Gly Asn Ala Ala Gln Phe Gly Glu Val Tyr Glu Leu Lys Glu Asp Ile
            420                 425                 430

Gly Val Gly Ser Tyr Ser Val Cys Lys Arg Cys Ile His Ala Thr Thr
        435                 440                 445

Asn Met Glu Phe Ala Val Lys Ile Ile Asp Lys Ser Lys Arg Asp Pro
    450                 455                 460

Ser Glu Glu Ile Glu Ile Leu Met Arg Tyr Gly Gln His Pro Asn Ile
465                 470                 475                 480

Ile Thr Leu Lys Asp Val Phe Asp Asp Gly Arg Tyr Val Tyr Leu Val
                485                 490                 495

Thr Asp Leu Met Lys Gly Gly Glu Leu Leu Asp Arg Ile Leu Lys Gln
            500                 505                 510

Lys Cys Phe Ser Glu Arg Glu Ala Ser Asp Ile Leu Tyr Val Ile Ser
        515                 520                 525

Lys Thr Val Asp Tyr Leu His Cys Gln Gly Val Val His Arg Asp Leu
    530                 535                 540

Lys Pro Ser Asn Ile Leu Tyr Met Asp Glu Ser Ala Ser Ala Asp Ser
545                 550                 555                 560

Ile Arg Ile Cys Asp Phe Gly Phe Ala Lys Gln Leu Arg Gly Glu Asn
                565                 570                 575

Gly Leu Leu Leu Thr Pro Cys Tyr Thr Ala Asn Phe Val Ala Pro Glu
            580                 585                 590

Val Leu Met Gln Gln Gly Tyr Asp Ala Ala Cys Asp Ile Trp Ser Leu
        595                 600                 605

Gly Val Leu Phe Tyr Thr Met Leu Ala Gly Tyr Thr Pro Phe Ala Asn
    610                 615                 620

Gly Pro Asn Asp Thr Pro Glu Glu Ile Leu Leu Arg Ile Gly Asn Gly
625                 630                 635                 640

Lys Phe Ser Leu Ser Gly Gly Asn Trp Asp Asn Ile Ser Asp Gly Ala
                645                 650                 655

Lys Asp Leu Leu Ser His Met Leu His Met Asp Pro His Gln Arg Tyr
            660                 665                 670

Thr Ala Glu Gln Ile Leu Lys His Ser Trp Ile Thr His Arg Asp Gln
        675                 680                 685

Leu Pro Asn Asp Gln Pro Lys Arg Asn Asp Val Ser His Val Val Lys
    690                 695                 700

Gly Ala Met Val Ala Thr Tyr Ser Ala Leu Thr His Lys Thr Phe Gln
705                 710                 715                 720

Pro Val Leu Glu Pro Val Ala Ala Ser Ser Leu Ala Gln Arg Arg Ser
                725                 730                 735

Met Lys Lys Arg Thr Ser Thr Gly Leu
            740                 745

<210>  4
<211>  109239
<212>  DNA
<213>  Homo sapiens

<400>  4
gagctggagg aggcggcggg cgcgagaccc ggaatgcgca gggcccccgc ctcgcccccc      60

ccagcccggg ccgcggcccc cgccttcccc gcagtcgtcc cgcactcggt gcccgccccc     120

cgaggccggc ggctgctccc actcggggcc gttgctgctt gtgccgtgag cgccgcccag 180
ccattgtccc cgtcgctccg tcagccgcgc cggaccgcgc accaggaggc gagagcgcgc 240
atggggagcc tctgttgatg ccgccgccgc gccgccctcc gaggctgcgt cccgggaagc 300
ccggctcccc gagcgctccg gcctggcccg gtgccccgga cctgagtgcg tccccatgga 360
ggcgcccggg ctggcccagg cggccgcggc ggagagcgac tcccgcaagg tggcggagga 420
gacccccgac ggggcgcccg cgctctgccc cagccctgag gcgctgtcgc cggagccgcc 480
tgtgtacagc ctgcaggact ttgacacgct ggccaccgtg ggtgagtgag tgcgggcggg 540
gactcggccc acaggggcgc gcggcgtggc cgggacgttg tagtaggaca aagggccctg 600
ggtgccgacc tcctggggag ggccctgacc cgctacttcg gctcggagtc cccgtgcggg 660
gctgcacctg cgccccgggt cttcccgggt ggagcgcact ccccagcccc ccagcccagg 720
caagtacccc cgaccggccg ggtgcctaac ctgaaatgcc gacggctcct ctcggagacc 780
accctccacc cccagcacac acagcactct ggggcctggg ccgtccgacg tcacaaaacc 840
tcctgcgggt cacctcgcct gggggacctc gtgctccctc cctggcagcg gccccaggga 900
cactggcgcg gggtgcgaag acccctgcag gcctccccta ggccagcctc cctgtgtgcc 960
cagaggcagg gaatgtacag atttctccag gggctgcagg agcagctggg ctgtggggga 1020
caggtgtccc ggggcgctgt ggggacgagg acggcagcgc tggggacgga tcctaacatg 1080
tcctgacacc gcctgtgctc ttcgtcttgt gcctctgaaa tgggtaattc ttgtatcgga 1140
cgctttatcc gtttcctttg tcctctgtct ttgaacttaa ccccgaatgg gcagcttgac 1200
agagaggttt cgagttctcg gtgctcttgc atccggacac gcgctgcttt atggagcagc 1260
cctgagtggg tcagaatatc ccaactgaac gtgggcgctg gatttaaaca gttgtcatcg 1320
gcccgcctgt gccacttagg gactccgtat ggctaagtgg ggtgttggct gtcaagaaaa 1380
taaatgggag agtagagggg gctgtcctgg gtgtgttggt gaggcgtcgg ctctcaggcc 1440
ctctaactcc tgtttgtcct cattttggaa aggaggaagc tgggctggga agcccaaggg 1500
ctcgaggcca tagcttatga cttaggaaga ccagcgggca tagccagtgg ggcctttaga 1560
accgctgagg aagaggggac ttagcttcct tcagtgacct ctttgccact tagaccttga 1620
ggaagggccc acgaggaaag cctgaatttg gaaggacaga gtgggaggag gccctctctc 1680
tttctcctct ccctccctcc tcctctttat ctcattctct ctctcttttc tctgtttctc 1740
ttctctctcc ctctttctgt ctctgttcaa gtctctctgc atttctcctc ctctgtctct 1800
tggtgtctct ctccctgtcc ccttccggtc ttggtttttc tgtctccctc cctcctctct 1860
ctctgccttc ccccttgtct tggtcccccc ggaaagctgt cctggtctaa tcttagagct 1920
gggtgtgttt tgcgcggaag aagggtgggg caggaaccct gactggcgca gccgccacag 1980
taggtggaaa tacaactcga caatggaaaa ttgatgaggt ccagccgttc cttggcactc 2040
agcaccagcg tttgtcattc tttggggcac ccacatgggt cccttgggaa aggtggagct 2100
ggggctggag gccggggcag agagcaggat gcgggcagga gggcggcaga ggtgaggggt 2160

tctggtgtca cagggggcca ctcagtttga cgtcaagtca gcttagggtg cccagcgggt 2220
aacctcatct ttaaaaatag agtgtcgctg cctctgggga ccaagctggc tgggcgggga 2280
gtcagctttt tttttcagtc cagtcagtgc tctcttaatg aggatgagga tgatgctcct 2340
ttctcaagga taacttcctg tgagctccaa cgatgtgaca ggagggctag gtatcccaca 2400
ctcattggac agcaggggac caggacccag gggtgtgtgt gtgtatgttt gcatgcacgt 2460
ctggaacact ctctcatgac aactgcacag ctttggtgac attatctgca accttttatc 2520
caagcccgtt tctttattgt tggttaaaga gcaatctgag tgtgattcac ctaaaataat 2580
acattttata aaatcctaag ccttttagat ccttcacgat tgtgtctcta agccacaatc 2640
tacagcagct ttggactgtt ttccaaggcg tgatggagaa tagtgagggg tgagcttgag 2700
tctcagtctg gagttgaaac ccagtctggg tgggtgtgac ctctcttcat cctaaactgt 2760
cactacagga acataagttt gcttttaagt gctctttcgc cctcattccg aattctctca 2820
ggccccaaac aagcacccgc aggagtactg aactttttgg ggggtgggca gaggggatct 2880
gtatgcagat tgccagctga gcaatcctat tttctatgac ttaaagccaa tcacaggctg 2940
ggcgtggtgg ctcatacctg tagtcccagc actttgggag gccgaggcag gcggatcact 3000
tgtgctcagg agttccagac cagcctggcc aacatggtga aaccctgtct ctactgagaa 3060
aatacaaaaa ttagccgggc gtggtggcgg gcgcctgtaa tcccagcttc tcaggaggct 3120
gaggcagagg aattgcttgc acccaggagg cagaggttgc agtaagccaa gatcacacca 3180
ctgcgctcca gcctgggcga cagagagaaa aagaaacttg tcagcgttct agattgacca 3240
gttttcctca aggtcaggta gttaggaaga aagagtgcag tttgcagttg tgaaaagtct 3300
gataatggat tctttttttc ttttttatgc gtgaagggat tctggagtac gtctggtcta 3360
aaggccgatt tcgttttagg aactttggat cagaacagtc atactagtcc tcagagaaaa 3420
aatggttttc aatctggttc ttcaaatttc ttgttcatat aaccaagcca tgcttgttcc 3480
tatgatggag aacaattgtg ctttaaaaaa agaaatttca gggccaggta cagtggtgcg 3540
tgcctgtagt cccagctatt tgggaggtcg cggtgggagg atgacttgag gccgggagtt 3600
ccagaccagc ctgggcaata tagtgagacc ctcatctctt aaaaaaaata gtagtaatag 3660
ttagctgggc atggtggcgc atacctgagt tacctgggaa gctgaggcaa gaggatcact 3720
tgagcccagg aggtcaaggc tgcagtcaac cacaatcgcg ccaccgtact ccagcctggg 3780
tgatagagcg agatcctctc tataaaaaat aaaataagaa aatgatatta tggaaatgaa 3840
aaactcaccc tatgtagaga gaagaggatg aatctgtgca gccatctacc agccttattt 3900
taccaatttc ctacttaaaa tgaccacttg agaattcctt tctctatata tcagataaaa 3960
aatacttggg ttttttccca gaagtgtcct tatggaatca tttggcatct acaacccagt 4020
gcttgcttgt catgggtacc ccaagtgtta acctgtcagg aaggaggtaa ttcaacaggt 4080
aaaccagtgg ccaggccttg ggtccacatt tcattttcct tttctcagcc tagttctgca 4140
tttactcatc tacagaggga aataatgacg gaacctgtcc tacacgatga cgatgaggaa 4200

gactccaaag ttcctagacc cctattaaaa atatatattt tttgaaatac agactcacag 4260
gggatttcaa aaacagtacc tggagtctca tggacccttc agccaacttc cccatggtga 4320
cgtcttcata tccgtgggac aatatcagaa ccatgtcatc gatgttggta tatccttatt 4380
aaagagaaca catacctcgt tcagcttttt tttttttttt tttttgagac agggtctcac 4440
tctgttgccc agtctggagt gcagtggtgc gatctcggct cactccagcc ttgaactcct 4500
gggctcaagc aatcctcctg ccttggcctc ccaaagtgct gggactatat acaggcatga 4560
gccatcgtgc ccggcttgtt caggctttat tagttttcaa aaggtgctca tttgtgtttg 4620
cgagtgtgtg tgtagagttc tgtgcagttt tacccagtgt gtggatttgg cagctaccac 4680
cctccaaacc atggtgcagg atgaattccc tcaccacaga acacccctcg gtcttgctcg 4740
acactttttt tttttttttg agacagagtt tcactcttgt tgcccagcct ggagtgtatt 4800
gaccacaacc tctgcttccc gggttcaagc gattctcctg cctcagcctc ccgaggagct 4860
gggattacag gcatgtgcca ccatgcctgg ctaattttat atttttagta gagatggggt 4920
ttctccatgt tgattaggct ggtctcaaac tcctgacctc aggcgatccg cccgccttgg 4980
cctcccaaag aggtgggatt gcaggtgtga gccaccgtgc ctggccagac actttttttt 5040
tttttaaact ttcacacact atacaatctg aaaatatttt attttcttaa gagctaggag 5100
atcttcataa ttaatgatac atggttctca gatctaaagt gcttatactg gtaggttttc 5160
tcttgtcctt ggttctcctg aattggccag aatttccttt cctctccttg ttgctgggtt 5220
cttattaatg cctcaagtta gtcggttcag gcagttgtag caaaacacca ccgactgggt 5280
ggcttataaa ccacagacat ttattcacag ttcagaggct ggaggtccaa gatgaaggca 5340
tggcagattt ggtgtctggt ggggacctgc ttcctggttc atagatggtg ccttctcgct 5400
gtgtcctcac atggtggaag ggtgagggga gctctctggg gcccctttat aagggcagtg 5460
atcccattca tgaggctcca acctcacgac ctcatcacct ccctagggcc tcacctcctg 5520
acaccattac cttgcaggtg aggatttcaa cacaggaatt ttagggggac acagacattg 5580
agtccacggc atccccaccc tgtctgtcac acagcatgct gccgggacga atggcatctg 5640
aactggtgag attctactgt gtgcataaat cagcagcctt gtgggcagtg ttgacatagc 5700
aattaggagt gttgtttgta gcctaacaat acacagaaag tggagctcag ccctttgatc 5760
ttatttacct tatgtgggtg ctccgtgtga gacaggctca tgcatgtgtg aatgacagtt 5820
tcctttaagt gcatcacgca aatgccaaga gatgagatca aatattacac atcagagaac 5880
atttccaagg atagaagtcc tgccggtttc tcctcttcat tgtttatttc attttatttt 5940
tttaatttaa gttaaaatac atgttaaatt aaatttaagt taaattaaga tacatgtgca 6000
ggacttattt taaataaaag agacagggtc tcgctgtgtt gcccaggcta gacttgaacc 6060
cctgggctca agcattctgc ctgcctccgc tgctggcaca taaatgccaa gtgtgcaggg 6120
acctctgtgt gttggagcca ctttcatctc ccacatccaa agaacagacc cggcacttag 6180
tgtgtgccca agaccatcca ccgcatgaat agagaaatca accctcctca tgtgcctggg 6240

gtttcctgag gtgggaaacc ttcagtgcta gctgggagag tcgcaggcag agacagggac 6300
aagctggtca ctctgtgtgt gaataaataa atgaatggat gatggcatta tcggcagttg 6360
ttctttatga cactcactga tgccaagtac tggacgggta caatatgtac attagtttct 6420
ctatgggcag aagctccacc atgacaacat tagtgttatt ttctttttgc ttttttgttt 6480
agagacaggg tcttactctg tcgcccaggc tggggtgccg tggtgcaatc aacagctcac 6540
tgcagcctcg acctcccagg ctcaagcaat cttcccacct cagtctccca agtagctggg 6600
actgcagacg tgcaccacca cactcagcta atttatgtat ttatttttgt agagatgggc 6660
attgctatgt tgcccaggct ggtctcgagc tcctgggctc aagtgatcct cccgcctcag 6720
cctcccacgt agctgggact acaggtgcat ccaccatgct tgtagcctta ttttcttaat 6780
gggaaagagg gtctgagaga agggatgctg tgtgcccaat gggtttcgcc tgcagcacgc 6840
tgcctcctcc ccgggaaagc agggcgtgca cattgggatt ggacgacaaa agcagagtca 6900
tctacagttc atgggcacgc tgcaaagaag ggagacgtta aactctcgaa agcagcagac 6960
accccccacc aggaagagac atacttgtaa aaatcaaagg agggcgaagg catgagaatc 7020
gcttgagccc aggaggttga ggttgcagtg ggctgagatc gtgctactgc attccagcct 7080
gggtgacaga gaccttgtct caaaaaataa aaattcaagg gaggccaggt gtggaggctc 7140
acgcctgtac ccagcacttt gggactctga ggtgggagga tcacttgagc ccaagagctc 7200
aagatctgtc tggacaatat agcactaccc catcgctaca aaaaaaaatt tttttaagta 7260
gctgggtgtg atggaatgca cctgtagtcc cagctactag ggaggctgag gcaggaggat 7320
tgcttgagcc caggaggtgg agactgcact gagccatgat ggtgccactg tgttccagcc 7380
tgggcaacag agcaagaccc tatgtcaaaa ataaagaaag tatcaaggga gatgagtaca 7440
cagtgcctgg cacactgtag ggtctccaaa aagtaaacct tttctatcca tcagtttcct 7500
cttctctcca gcatgaaatc gcatatgtaa agttgaaaaa aagagtgaga gatatatctt 7560
aaaaaggtag taatgttgat gacattgtgg tttttttttt ttttttaaag aaaaaccggc 7620
cgggtgcggt ggctcatgcc tgtaatccca gcactttggg aggctgaggc aggcgggtca 7680
tgaagtgtca ggagatcgag accatcctgg ctaacacggt gaaaccccgt ctctactaaa 7740
aatacaaaaa aaaattagcc gggtgtggtg gcgggcgcct gttagtccca gctgctgggg 7800
aggctgaggc agcggaatgg catgaacccg ggaggtggag cttgcagtga gccgagatca 7860
caccactgca ctccagcctg ggcgacagag caagactctg tctcaaaaaa aaaaaaagaa 7920
aagaaaaacc ttgggggaaa tacagaggaa gccctaaggc atcccttcca aaaagctgaa 7980
agtgctttac ttagaattgt gacctcgttt tccctgttag aaaagtctgt ggttagaagc 8040
ttcctggtaa gcccagtgtg agaaggtgga accgatgttt ctgtgtgacg ggttccctct 8100
gcctgtttct ccaggtggcc tccccgtggt cctctactgt ggtgggtcca gtcccaaaac 8160
caagtctggg gccaccatca tcatatttgt gtcccccacc ccaagattct tatgtcaaaa 8220
ccctaatccc caaggtaatg gtgttaggag gtggggcttt taggaggtga tggggtcact 8280

agggtggggt ctcatgaatg ggatcaatgt ccttataaaa gggaccccag agagctccct 8340
caccccttcc actatatgag gacacagtga gatggcaccg tctgttaatt agaaagccgg 8400
tccccaccaa actctgaatc tcccatacct tcatcttgga cttgtagcct ccaggactga 8460
cagcggtaaa tgtctgttgt ttctaagccc cagtctgccg tgttttgtga tggcagccga 8520
aatggattta gatggggctc tattcacccc acgcggcagg gtccatggaa aggcagctgc 8580
aatgcgctgg tctatcatta cctcttttat gctctttcac actgtcttag tcttgcgtgg 8640
ctgctggaat gaaggaccgc aaatatagta gcttaaaacc acatacagtg aagagatacc 8700
tgcactccca ggttcactgc agcacgagtc acacaacagc caagataggg caacaaccca 8760
cttgcccgtc agcagatgac tgggtgaaga aaatgtggtc tctacacaat ggagtactat 8820
tcagcctgta aaaagaataa agtcctgtca tttgcaacaa tgtggatgca actggaggtc 8880
attatgctct gagaaatgag ccaggcacag agagacaaat acggcattat ctcactcctt 8940
ttttttttgg acagggtctc actcggacac ccaggctgga gtgcagtgac gtggtgtcac 9000
tgcagcattg acgtcccagg ctcaagggat cctctcacct cagcctcctg aggagctggg 9060
actgcaggtg tgcatcacca cgccaggcta ttgttttgat ttttttttgg tagagatagt 9120
ctcgtcatgt tgcccaggct ggtcttgaac tcttggcctc gagtgatcct cccagtttag 9180
cctcccaaag tgctgggatt acaggcgtga gccaccacgc cctgcctgac ctatgtactt 9240
tagaatccca gaagcaaatc ttgtatccta ctggaggagc aagacagaaa cacaggtgct 9300
agagggcaga ttgtttgcac agaaacctgg gcatgtggat gaatttggta atacgtgtgt 9360
gcgggtaaac ttggcaggcc cctttttttc cagggttccc ttgtgcaaga agaatgaaac 9420
taatgcaacc aatactttca agaagctcct cctttctgga gcgttttcac gctgcatttc 9480
ttgggtgggc atgttttccc ggcgtctccg attctgtgct gtgttgggtg agacaggatg 9540
atggacattt tgcttcccca ttaggatctt aagtgaaagt ccagttggca tttgcatttg 9600
atctgagtag gagaaaacat gataaatagc cattagaggc cgggtgtggg ggctcacacc 9660
tgtaattcca gcaatttggg ggccgaggtg ggcagatcac ttgagatcag gagttcgaaa 9720
ccagccttgc caacatgaca aaaccccgtt tgtacccaaa aatacaaaaa ttagctgggc 9780
accgtagtgg gtgcctgtca tcccagctac tcaggaggct gaggcaggag aatcacttga 9840
acccgggaga tagaggttgc agtgagccga gtctgtgcca ctgcactcca acctggggtg 9900
atagagtgag actccatctc aaaaaaaaaa aaaaaaaaac aaccaaaaaa acccaaaaac 9960
acattagatg catttgaagc ctcccagcaa ttctcccatg atggcagaat aggcatccag 10020
gagactcacc ctaccctgtc tgaacacctt tagcgaagat gtaaatgatt tgcagaggag 10080
actcaggtgc gaggatggaa attccttccc cctccagcac tgatggcttc caccttcctg 10140
tggtctaagg agaccacaca gaggttatca gtgtgggagg ctgttgccag tatggtgagt 10200
ttcctatgga ggaatttcct gtgggacacc tctgggcagt tcttccagcc tttcatgtct 10260
tctgtcagat gatacagtgg ccaggtcaag ggattatcat gattttattt gttttgtttt 10320

ttagagacaa ggtgttgctc tgtcgcccag gctggagtgc agtaggggtg caatcaatag 10380
ctcagctgca gcctcaaact acagggctca agtgatcctc ctgcctcagt ctcccaagta 10440
gctgggacta cagggacaca ccaccatgcc cagctcattc tctcataagg tggagagaag 10500
agaagagagg aggacataaa actgtccata tgactagttt gggcctttcc atcctgctta 10560
atccagaact gtcaaggtta gaccctggtc attctttaat atgtttgaaa aatacggctg 10620
ggctttggct cacacgtgta atcccagcac tttggaaggc cagggtgaga ggatcacttg 10680
agtccagacg tttgagacca gcctgggcaa cataagaaga ccccactgct acaaaggggg 10740
aaaacaagtc agccacgcat ggggacgcat gcctgtggtc ccagctattc ggggggctga 10800
gacgggagga ttgcttgagc tcgggaggta gaggcttcag tgagctgcga ttgtgctact 10860
gcactctagc ttgtgtgaca gagtgagacc ctgcctcaat taaaaaaaaa aaaaagaaaa 10920
atagaccttc cttctataca atgtcttccc tgctggcctc catgccctcg tacctgttta 10980
aaaatccact ggaatttttc accaggacat agaaaaagga agttgtctca cgcagttcct 11040
gttaaattgc acaagtccac acagatcctt gcaacccacg ctaccattta cttagcatgc 11100
gttgtgtgtg ggcattacat tattctccga ggtagttatt ttggatgtta tactggggag 11160
gtaccccatc aaagagattt ggagggcagc ctgtggaggg ttcttttaaa atcgttgaca 11220
actaatttgg tggatgtttt caccttggtg aatcattccc ttgggagtca ttttgcagaa 11280
caaaagggtt gcacacaggt gtggccagag aaaccttggc acagaagaaa gaagtgcttt 11340
gtcatgctta gggcacctgg gatctctttg gatgggcagg cagaggggga ccctgtgaag 11400
cctgcctgcg tttatttggg tatgcatgct tatgttggag tcttggcaat gagaggacaa 11460
gagtggaggg aacagagctg ttaacagaaa ggggtccata tccagacacc aagagagggt 11520
tcttggatct tgtgcaagaa aaaattcaag gcaaatccat agagtaaagt gaaagcaagt 11580
ttattgagaa agtagaggaa taaaagaatg gctcctccat agacagagtg gcttcgaggg 11640
ctgctggttg cccattttta tggctatttc ttgattatat ggtaaacgaa aggtgaatta 11700
ttcgtgcctc ccctttttag accaaatagg gtaacttcct gacgttgccg tggcatttgt 11760
aaactgtcat ggcgctgatg ggagtgtagc agtgaggaca accagaggtc actcttgtca 11820
ccatcttggt ttgggtggga tttggccagc ttctttaccg caacctgttt tatcagcaag 11880
ctccttatga ccggcatctt gtgctgacct cctatctcat cccgtgactt agaatgcctt 11940
aaccatctgg gcatgcagcc caacaggctt cagcttcatt ttacccagct cctcttcaag 12000
atggagttgc tctggttcag atgccgctga cagaactagc ttatggttgg agacaatgtc 12060
atagggaatt attcttcaaa gatcgcaaaa tccagcttgg atgagattcc aaaaatcaaa 12120
aggtgctgag aaagcaactt gtctagaatt tcctcttcat gggctggttg attttgttta 12180
caatgacacc ttcagaaaaa gtaggcgaca cacagtggct cacgcctata atcctagtgc 12240
tttgggaggt caaggaggga gaatcatttg aggctagcag ttctagacta gcctggacaa 12300
catagtgaga ccttgtcttt acaaaaaata aaatcagccg tgtgtggtgg cgcataccta 12360

taatcccagc tgttcaggag gctgaggcag gaggatcatt tgagcccagg agttggaggc 12420
tgcagtgagc tatgatggcg ccactgcact ccagccttag tgacagagca acattctgaa 12480
agaaaagaaa gaaaaggaaa aaaagaaaag gaaataaaag atgaagaaag aaaacagaag 12540
aaaaaaataa tttatataga aaaaaagtcc ataatgactc aaaatattcc ctggacactg 12600
gaatagaatg attttgttta ttaaagcttt tctttagttc gtccttaaac agtttagtat 12660
tttcttttga aaacagattg tgtcttgtca acttcattga ttttcgggct gggcttggtg 12720
gctgaacacc tgtaatcccc caacactttg ggaggttgag gtgggtggag cgcttgaggc 12780
caggggttcg agactagcct ctagcctcgg caacatggtg aaaacctgtc tctacaaaca 12840
acaacaacaa caacaacaaa caattagctg ggcgtggtgg cacgtgcctg tagtcccagc 12900
aacttgggag gccgaggtgg gaggatcact tgagcccaag aggtcgaggt cgaagtgagc 12960
tatgattgca ccattgtact ccagcctggg tgacagagtg agaccatgaa agaaaagaaa 13020
gaaaagaaaa aagagaaaga agaaggaaaa aaaaagagaa gaaaaaagta aaagaaaaac 13080
atagaaaaaa aatcagtaac gactcaaaat attccctgga tagttgaata caaatagttg 13140
ttgttggacg tgattttatg tattaaggct tttgtttagt ttatccttaa ataggttagg 13200
attttatttt gagaatagat tctggtttat caatttcatc agtctttgca aagaaacagc 13260
ttagggtttc ctttatgttt ctccgttaac ttcctgtttt cagttacatt gcgttttgct 13320
ctggtttttc tttcgttttt tccactcgtt ttaggtttct ggaatttcct gaaacattgt 13380
attttgatac agattctgag ctggtgcttt ggctgtcaca agccagaggg tcctagaaga 13440
agtagaattg taaccgagga aaactgttag gaaactccat cccttttgac cttcaaaggt 13500
caccgcctaa ttttggtttt ttgtagtctg atgtgttcat agcttgtgac tattaggctc 13560
tgattgagct ctgctttttt ttaagtttct cttactcagc tggtgctgtt ttagctccag 13620
cttcctcctc tgtgtaatca acactcccag tctcctcttc acccaaaaag ccacagaaat 13680
aagtgcagaa aacaaggcag ccgccacctg ctacagaggc atgtccttaa atacgatgca 13740
tttcaggaca gctgtgtgag ttccgggagc ttgtcttcct tcaaagggaa aagtaaaaat 13800
aaacaggaag gttgcactga aagcattctc aattgaaatt gtgccatttg gctgtagtgt 13860
ttctgatgct catttagaac tttggaagtt gtgggatggt gggcaagtgt gtgacctggg 13920
atggagattc tctacctctt taagagtgaa accctggctg ggcgccgtgg ctcactcctg 13980
tcatcccagc actttgggag gccaaggcgg gtggatcacc tgaggtcagg agtttgagac 14040
catcctggcc ataatggtga aaccccgtct ctactaaaaa tacaaaaaaa tagctgggcg 14100
tggtggcagg tgcttgtaat cccagctact tgggaggctg acgtgagaga atcacttgaa 14160
cccggcaggc ggtggttgca gtgagctgag attgtgccac tgcactccag cctgggtgac 14220
agagtgagac tccacctcaa aaaaagaaaa gaaaaagaaa aactttaaaa aatgccgggc 14280
acggtggctc ccgcctgtaa tcccagcact ttgggaggct gaggtgggtg gattgcctga 14340
gcttaggagt ttgagaccag cctgggcaac atggtgaaac cccgtctctc ctaaaatgca 14400

aaaaattagc cgggcgtagt ggtgcacgcc tgtagtccca gctgcttggg aagctgaggc 14460
aggagaattg cttgaacctg ggaggcagag attgcagtga accgagatgg tgccgctgta 14520
ctccagcctg ggcgacagag tgacactctg tctcaaaaaa aaaaataaaa taaaaattag 14580
ctgggcgtgg tggcacacat ctgtgacaga gcgagactcc atctcaaaaa aaaaaaaaaa 14640
agaattttgg aagttgtggg atggtgggcg agtgtgtggt ctgggataga ttctccacct 14700
ctctaaaagt gaaaacctgg ttgggtgggg tggctcacat ctgtaatccc agcactttgg 14760
gaagctgagg tgggcggatc acttgaagtc aggagtttga gaccagcctg gccaacatgg 14820
tgaaactctg tctctaccaa aactacaaaa attagccggg tgtgtggtgg cgggcatctg 14880
taatcccagc tactcgggag gctgagacac gaaaatcgct tcaacccgga aggcggaggt 14940
tgcagtgagc cgagatcaca ccactgtgta tgccactgca ctccagcctg agcggcagtg 15000
agaccctgtc tcaaaaaata cacacacaca caaaaacaaa aaacaaaaca catgtagaaa 15060
aaaccacaaa gacaaaaaca aaacaacaac aacaacaaca aaaagtgaaa acctacttct 15120
ttttctggac acggtggctc atgcctgtaa ttgcaccatt ttgggaggcc gaggtgggag 15180
gatcactctc acctttcaag gcccaggagt tcgagaccag cctgggcaac acagcaaaac 15240
cccatctctc taaaaataaa aaagagaaaa agagaaaaat tgcttctttc tatgtagttg 15300
gtgagttttt tcagtaggat gctttgcatt tcttcctgcg tctgtggctc tgtggtatgc 15360
cagcatatac gatagtcact acaagatccg tagttgtaca cccagccata cgcatcacag 15420
gtttttggct gatttcatga caacgcttaa ggctgctgaa aacactctaa ttctttggat 15480
gttggcaaat gttcagatga aactaagcgg ctggcccacc cacctgctca ggaaagaatg 15540
gccgcattgt gtgggaaagg aatcctctgt tccctagctg gctgcctggt gttcttgttg 15600
agtgagcaga acagagagtg cccctgggta gacctgtgtg tgctccaaaa ggcgattttg 15660
tcaattgttc ccttgggatg cccttgttcc agaaagcatg ttctgtgtga ctctcagctg 15720
gggttcccag actgtgtaaa gaaaacatgg tgattcatgg ctgtgtgcgg ttgcccatgc 15780
ctgtcatccc agcattttgc gcggccgagg cgggaggact gcttgagcct gggaggtcaa 15840
cgccagccta ggcgacacag taagaacctg tctctacaaa aacaaaaaca aaaaagtgta 15900
gccgggcatg gcgacgagca cctgtggtcc cagctactca ggaggctgac gcaggacgat 15960
cgcctgagcc tggaagttgg aactgcagtg agccataatc acaccactct actccagcct 16020
gggtgacaga gtgagaccgc atcttaaaaa aaaaagaaaa aaaaaaggac attgtgatta 16080
atgaaaacaa acattgcctg gtgataagga aagtaagaag taggattgtt ctcagattag 16140
tgacccagga gataaaggag taacagagtg tgcaggaagc aggtctccaa tttaatagca 16200
ggatggtgaa gatagagcaa gtgacttctt tctatttttt ttagaaactg ggtgttgctt 16260
tgtcacccag gctggagtgc agtggcgtaa tcatagctga ctgcagcctt cacctcctgg 16320
gctcaagcag tcctcgtgcc gcgccttccc gagtagctga gactacaggc acataccacc 16380
atacttggtt aattttttgt atttttagta gacatggggg tgtggggatg gtttgccgta 16440

ttatccaagc tggtctcaga cttctggcct caaataatcc tcctgcctcg gcctcccaaa 16500
gtgttgggat tacaggcatg agccactgtg cctggtgagt aggtagcttt ttgtttgttt 16560
gtctgtttgt ttgttctgtt tgtttgtttt tgagacagtc ttgctctgtc gcccaggctg 16620
gagtgcagtg atgcaatctt ggctcacccc aacctccacc tcccgggttc aagtgattct 16680
cgtgcctcag cctctcaagt aggtgggatt acaggcacat gccaccatgc ttggctattt 16740
tttgtttttt tagtagggac agagttttgc cttgttggcc aggctggtct caaactcctg 16800
acctcaagtg atccgcctcc ctcggcctcc cgaagagctg ggattacagt tgtgagccac 16860
cgcgtccagc caaacaaaaa aaagtctgtc ttataaagaa gatccctaat ttatgatgat 16920
tccatttatg atggtttgag ttatggtagt ttgagttagg atagttcgat tgaagatttt 16980
ttgattttac gatggtgaga aagacacagt ccgtggaacc cgcgcttcga gtacccatgc 17040
aactgttctg tttttaactt gcagtgcagt attcagtaaa ttacatgagg cattcaatac 17100
ttgattgtaa actaggcttc acgttaggtg attttgccca gttgtaggct aatgtaaatg 17160
ttgcgagcac gtttagggca ggctgggcta agctgtgatg ttcgtaagtt aggtgtattc 17220
gatgcatttt tgacttacca tattttcaac ttacaaaggg tttatggggt gtaatgtcat 17280
cattgtaagt tgaggagtgt ctgtatcata gactgggggg cttaaacaga catttattgc 17340
tcccagttct ggaggctggg agtcacagat caaggcgtgg cacattcggt gtctggtgag 17400
ggcttcctgg ttcatagacg gcgccttctc actgtgtcct cacatggtgg aaagggtgag 17460
ggagctctct agggtccctt taataaggac attgatccca ttcatgaggc tccaccctca 17520
tgacctcaac acctcccaaa ggccccacct gctaacatca tcaccttggg gatgaggatt 17580
caacacggga attttgggag gatgcaagct ttcaccagta gggttttctt tgtaaagaaa 17640
tagaaggaaa tgaaaaggca ggcactgggt aggagagagc tacagaagac agcggggtgg 17700
tggcactcca ggatagtgct gtcccccagc atcatttaca ccgaagaaac ctgaggcact 17760
ctttctgatg tgtcattctt aggaaaacca ccatgaatct ggcatctagc tatcagtgaa 17820
actgtgggag agcttaatgg aagtaaagac atgtggtccc agaaatgaca gatcattcgg 17880
agaacctaag agaagattaa aagcaaaagg gggtcatgtg cgctgtgact caaactttgg 17940
gaggtcgagt gggaagattg cttgagccca gaagtttgag accagcctgg gcaacatagg 18000
gagaccctat ctctacaaaa aatacaaaaa ttagctgagt gtggtggcac acaccttagt 18060
cccagctact caggaggctg aggtgggagg attgcttgag cctgggagtt caaggctgca 18120
ctgagctaag attgcgccac tgcactccag cctgggcgac agaacaagat cctgtctcaa 18180
aaacaaaaac aaaagcaaaa caaaacaaga aaatccaaaa gggaaatata agagaaaaca 18240
aaaaatctca ctttgagaca tcttttttga agagttggag aggatattgt ctccatccac 18300
aaaaatagga tgttacaata aaagagaaag aggaaggaag agctttatat gggactgctg 18360
aaagttaaaa gaaaaacatt gcattaaata aattagaagg gtgatctcat ctgtgcctgt 18420
gacttcagtt atcatcttaa ggctttgggt ctcaaagcac acatctagtt tagacctttc 18480

ttaggagccc tgaagggtgc attgccaact actgcccgtt ggagggtcca tttttcccaa 18540
gatatccagc aacactctta tatgagtgtc ctgctccatt gtctcaaatt accataaact 18600
ggggaacgca agcaacagaa atttatgctc tcccaggcct ggacaccagg agtctgaaat 18660
caaagtgcag gcaggattgt gctccctctg gaggctctgg gggaggaagc ttcctgcctc 18720
tcccggctcc tgggggctcc aggcatccct gggcttgtgg ccacagcact ccagtctctg 18780
cctctgtctc cacgtggcct tctcatctgt gcctgtgtct cctcttctgt ctcttagaag 18840
tacactggtc attggattta gggcccaccc ttttccagcg cgatctcatc tcaagatccc 18900
tagcttaatc acgtttgcaa agttccttat ttccaaataa gttcccattc caggttctgg 18960
acatgaggat gtgaatatat ctttgtgggg accacagttc agtctactag agttgtatgc 19020
agttccttct ggaggctcta ggggaggatc ctttctgcat ctcccagctc ctgggggctc 19080
caggcatccc tgggcttgtg gctatatcac tccagtctgc ctccgtctcc atgtggcctt 19140
ctcctctgtg tctgtgtctc ctcatctggc tctttatttt tttaaataat ttatttattt 19200
atttattttt tatttttttt ggtgatggag tttcgctctt ttgcccaggc tggagtgcag 19260
tggcatcatc tcggctcact gtaacctctg cctcccgggt tcaaatgatt ctcctgcctc 19320
agcctcctca gtagctggga ttataggcac ccgacaccac gcctggctta ttttttatat 19380
ttttagtgga gacgggattt caccatgttg gccaggctgg tctcgaactc ctgtcctcag 19440
gtgatccacc cgccttggcc tcccaaagtg ctgggattac aggtgtgagc caccatgctc 19500
actggccatt ttttattatt actctttttt cctcttctgt ctcttagaag gacacccgtt 19560
gttggattta ggtcccaccc taaatccagg atggccttat ctggagattg tttacttaat 19620
agaaactaca aagaccctat tttcttttct tttttttttt tttttttttg agacggagtc 19680
tccctctgtt gtccaggctg gagtgcagtg gcgcaatctc ggctcactgc aagctccacc 19740
tcccgggttc acgccattct cctgcctcag cctcccaagt agctgggact acaggcaccc 19800
gccaccacac ccggctaatt tttttttgta tttttagtag agacgcggtt tcacctcgtt 19860
agccaggatg gtctctatct cctgacctcg tgatccgtcc gcctcagcct cccaaagtgc 19920
tgggattaca ggcgtgagcc accgcgcccg gcctcaaaaa ccctattttc aaataagacg 19980
ccattcacag gcaccagggg ttaggatgta gacatatttt tggggggcac cattcaacct 20040
agtctatccc tcaatctcaa tacatcccct gggttaaaca tgaaatgtgt ccccttcttc 20100
ccccaaatca aagcaaccca aaagacccct gtgcacctgc acaccttgcc taagtcggca 20160
gcactcactg cgtagatgcc caaaccctaa gctgtgatca tctccaccct ccctcttgca 20220
gcctaattga gcccattctc tgcccaagca ccttatgaac aggactttaa ccctccatct 20280
ccaccacctt cccattttca ctggaaattt tacttcttaa agaggccttc cctgacccct 20340
tgaccaaggt tagatgcatc ccaatgttct cctatggata tgttgtttga agtcctcaag 20400
tctgaacaaa ctcatgtgag gtcacccaag ttgttagact tactgtcatt aatgcttcct 20460
gccctccttc cagcccgact ggaactaaac aaaactaaat gagggaccaa ccttgtgttg 20520

ttcattgcag tttctcggag gtagacagca cctgagaccc aggaaaatat caatcatgat 20580
ggaagtgatg cattcattta ttatattgac tccttttttt tcccaaaagc tattttgtgt 20640
tcaaaacctg gactgcataa accacttcat tgtggttatg gagtgttaga agccgacacc 20700
taatcaaaga attcagtgtg tctcagtggt aggaactcta aagccatcta acattaaatg 20760
caataaaagt tcagttgctt acgacctggc gccgtggctc acgcctgtaa tcccagcact 20820
ttgggaggct gaggcgggtg gaccacgagg tcaggagatt gagaccatcc tggctaacgt 20880
ggtgaaactc tgtctctact agaaatacaa aaaattagcc aggcgtggtg gtgggtgcct 20940
gtagtcccag ctactcagga ggctgaggca ggagaatggc gtgaacctgg gagacggagc 21000
ttgcagtgag ctgagatcgc gccactgcac tccagcctgg gtgacagagc gagactccgt 21060
ctcaaaaaaa aaaaaaaaaa agttcagttg cttactctcc atagcctcaa ttcaaatcct 21120
cagtagccac ttgtgagctt gtggctacca ttttggacag tgcagataga gaacattcct 21180
atcattgcag gcgatactac gggcagtgct tgctccaaaa cagggggtct caactggggg 21240
ccggccttcc cccacagggc acttgacagt gtctggggac agttgtggtt gtcactactg 21300
ggggtggatg ctgatggcgt gtggtgagtg gagcccaggg acgccactct gcaggtttgc 21360
agtgcacagg atggccctac agagaatcat ccagcctcaa atgtcggcag tgctaggctg 21420
agagaacctg ctttagcgtg agagtcagtc tctctctttc tgtctctctc tctctctccc 21480
cctctctccc ctccccactc tcgctctccc tccctccccc ctccacccat cccatctgtt 21540
atctacctac ctacctacct atcaattatc taaccatcct aattatctat gtatcaccta 21600
tctatcctat ctgttaccta tctacctacc cacctaccca tccatccatc catccaatca 21660
attaatcagt gtatcatctt tctctttcca tgtatgtatc atacccattg tccatccatc 21720
catccatcca tccagtcagt cattcaatca atcaatcatc tttctatcta tccatctatc 21780
tgtctatcat acccatctac ctatctttct atctgtctgt ggcagagatt ctctgccaat 21840
ggcaattcag ctccatcagg gagatacttc ttccctggac aagtctggag acatttttgt 21900
tgtcacagct cagggaggtg ctactgataa caggtgggtg gagtccaggg tcactgcttg 21960
ccaccttaca gtgcacagga tggccccacc acagagaatc atccagctcc aaatgttaat 22020
ggactcagtt tgagaagctg atctcaaggg taattcgggg gagtcctgcg ggattgttgc 22080
atcctattga agggggaatt aatgaatttt ataagtgtaa tggtcgaaaa attgttattt 22140
ttcttgaagg tagaatgtaa tgtagcccac tcccccgggg cggggggact gggttaagag 22200
agaatactaa ctgcttatcc ctcctctatg cccagagagg cttatctgtg ttccatcgtt 22260
ttacattcct tgaggcacgg cgagttcttg cttccctccc tagtgcagct gtaaagtcac 22320
aaggttgaca agcaattgct gcaaaagtat gtattcccaa gaatgtaaga cgtacggtgt 22380
aacaaatgca aaagagtaat taactgcctt tgttctcgct tctgcaagta cgctttctgc 22440
agcacgtaac tcccgccaca aactgcttaa aaggtgattg atccctctgt acggggctca 22500
gactttctag accctagtcc gactgagctg gtgatcacct taataattat aattataatg 22560

gtcatctcct aactgtgctc ggtctctacc gtctctgatt tatcccgcaa cactatcgtg 22620
gaggtaggct aggatgtatc tttaacctgg ctggattgta ttgtagatgt gtgaaaacca 22680
gcccttgttt ttcttggtag agttgcactc aaatgttatt gaaagctgca catactgtgc 22740
ttgtataaat acactgagcc agaaatcacg aacctttccc tggcttcaca tctgttagtg 22800
ttgtgggaga agatgagaat ttcttgtttt tcttttcact tctgtgatta tacattttgt 22860
attttgttgt tgttttttgg gacagggtct cgctctgtca cctaggctgg agtgcagtgg 22920
tgtgatcata gctcactgca acccccgccc ccaggctcaa gtgatcctcc tgcctcagcc 22980
tcccaagtag ctgggactat agtcgcatgc caccatgcct ggctgttttt tgtgttttta 23040
gtagagatgg ggttttgctt tgttgtctag cctggtcttg aactcctgtg ctcaagtgat 23100
ccgcccacct tggcttccca actgtgggga ttacaggtgt gagccactac tcacggccac 23160
attttgtatt tctgatgaga aagtgtaatt taatacactg tacatcggag agtgtagttc 23220
tgaaacatct gcatctttta aattaggaca gtgtaaaaat ggaatgttgt ttgactcagc 23280
aaaattttga gggagtttgt ttccaactgt ctagaccagt gaactcacca aactatctac 23340
cacacactgc ttggctgaat tggctctact gtttttaact gaagtttgaa gtaaatattc 23400
caagcttaaa aatatatctt gttcctcccc cacccactgg ttatagcagc aatataaata 23460
aaaaatacct tcccaagaat atccaaagca tacattcttt tctaaagtaa acattgttta 23520
tagagataca atttctatac cataagattc accctttaaa aacacatagt tctttagttt 23580
tcagtttatt cacagagtaa gcacactgtt ttttgtttgt tttgtttttt tcgagacaga 23640
gtcttgctct gtcacgcagg ctggagtgca gtggtgccat ctcggctcac tgcaagctct 23700
gcctcccggg ttcacgccat cctcctgccc cagcctcctg agtagctggg actacaggct 23760
tccgccacca cactcggcta attttttgta tttttagtgg agacagggtt tcaccgtgtt 23820
agccaggatg gtctcgatct cctgaccttg tgatccgccc gccttggcct cccaaagtgt 23880
tgggattaca ggtgtgagcc accgcgccca gcccattcat tcattttttg agacagagtc 23940
ttgctctctc acccaagtta gagcacaggg gtgcgatcac tactcactgc tgtcttgaac 24000
tcctgggctc aagtgatcct cctgcctcag cttcccaggt aacttggact gcaggtgtgt 24060
aaccaccacg ccctgctaat tttttgatct tttaatagag acggggtctc gttatgttac 24120
ccaggctggt ctaggactcc tgaactgatg tccttccgtg gcctcccaaa gttctgggat 24180
tataggcacg agccaccatg cctggcaaca cacactcttt tttaaaaata ctttttaaca 24240
gctttttttc ctgtctataa aatagaaggt cattatactt gtttgcagca gtaattccaa 24300
aaatcgactt cgtaaagaag aactgcgtgt ttcacacact tatttcctcc atggtagcat 24360
ttaatgttct tttattgatt ttacccaggg gggaaaatgt caaggaaaag aactgagttt 24420
gaggatgata aatgtgtacg ttctgatatc attattttga aacaattatt taatgcaatg 24480
cgtcggtttc acttatcaat ttaacataaa ttgaacctat gttgatgaac tatttctata 24540
gtcataaatt ttaaaaggtg agaaagttat atagtgaaat gcatctgttt ttgtgcctca 24600

cctgtgcaga atttgccctc tccttccacc caggtgccca tattaatgtg tcttgtatat 24660
ttctccaaag tcctttctca atatagaagc aaattatcac ttatggagaa gagcctcact 24720
tttttttttt ttttttaaac agatgggggt cccgctcttt tgcccaggct ggaatgcagt 24780
ggtactatca taattcactg cagcctccaa ctcccgggct caagcaattc tcccatctct 24840
gcctcatgag tagctgggac tactggagca taccaccaca cccggctcat taaaaaaaat 24900
tttttttttt tatagatggt gtctggctat gttgcccagg ctagtctcga tctcctgggc 24960
tcaagtgatc cttctgcttc agccttctaa agtgctggga ttacaggcat gagccactgc 25020
tcctggcctc cacccatcta taggtgtgga acaagagcat gttccctccc aagctaatgt 25080
ggcaggtaac tggcctccca ggctgtagac aagatggatg ggggctgtgc ccacttcttg 25140
agttaacctt ttttttttct ttggagacag agtctcgctc tgtcgcccag gctggaatgc 25200
agtggtgtga tcttggctca ctgcagcctc cgcttcccag gtttaagcaa ttctcctgcc 25260
tcagcctcct gagtagctgg ggttacaggt gcctgccacc atgcccggct aatcgttgta 25320
tttttagtag agatggggtt tcaccatgtt ggctgggctg gtctcgaact cttggcctca 25380
gatcatccac ctgcctcggc ctcccaaagt gctgggatta caggcgtgag ccagcgcgcc 25440
cagctgggtt ttaccttttt tgtttgtttg ttttttgaga cagagtcttg ctctgtcgcc 25500
caggctggag tgcagtggtg tgatcttgcc tcattgtagt ctctgcctcc tgggttcaag 25560
caattctcct gcctcagcct cccaagtagc tgggattaca ggcatgtgct gtcacgccca 25620
gccaattttt tgtattttta gtagagatgg ggtttcccca tgttggccag gatggggttt 25680
tacctttttt gaagtgtatt ttccatgtag ccacctctct tggagtgtcc atgaggaaca 25740
cgatgctgtc cttggtgtct cagcggagcc actgtgacgc tctcctcttg caaagatttc 25800
tggtcatgat gtctcaacat tggcctgttt ggggtttttt ttctcctgca ttttagggag 25860
aattagggct catccaccct caccttctct cccccattga attggtgcat cctgtttttt 25920
tttagcccct gggatgccca tgttcaagat tccttaagca tcacagttta aggaaagaaa 25980
tgcagattat ttaaaatatg tggggtgagt gtgcagggtg gtgatggaca atgcatgtgt 26040
ttaattcagg gactgttgtg ccagctgtgt ttgagcctta ggaattctta tagttgactg 26100
gcatttacag tttattaagg cacttacctc ttaggtgtat aatcctcaaa acatctaaaa 26160
aattagtgat ttttgttatc caagttactt tgacatcagc catttgctgt ctcacccaca 26220
tgatttctca ttatgttacc ttattattgg ctaagttaat ctgcttactg aggacctgca 26280
tgtgactttt cccattaaaa gtaagttaag tctgggcgca gtggctcatg cctgtaattc 26340
tagcactttg ggaggctgag gtgggaggat cccttgactt tgagaccagc ctgggtgaaa 26400
aagtgagatc tcaaaaacaa aattagccag acatggtggc gcatgcctgt agtaccagct 26460
acttgggagg ctgaggtggg ctgatggctt gagcccagga gtttatgctg cagtgagccg 26520
agattgcatc actgcactct agcctgtggc acagagtgag acctgtctct taaaaaaaat 26580
taattaatta attaaaaata aataaaagta agtccaagtg gagatggttg gtggtgttgg 26640

ttggataaca ttgtgaatgt atttaacacc gttaatctgt acacttcaaa atggttaatt 26700
aagatggtaa attttatgtt gtgcgtattt taccataatt aaaaaataga tttggtctgc 26760
gtgatggctc acgcctgtga tcccagcact ttgggaggcg aggtgggcgg atcacctgag 26820
gtcaggagtt ggagaccagc ctggccaaca tggtgaaacc ccgtgtctac taaaactaca 26880
agaattagtc gggcgtagtg gcaggcacct gtaatcccag ctactctgga ggctgaggca 26940
gaaaaatcgc ttgaacccag gaggtggagg ttgcagtgag ctgagatcgc gccactgaac 27000
tccagcctgg gtgacagaat gagactctgt ctcaaaaaaa aaaaaagatt taaaacaaag 27060
taactatgtt caaggccagg tgtagtggct cacgcttgca attctgacac tttggggcgc 27120
tgaggtggaa gattgcttga agccaggagt tcaagaccag cctgggcaac agagtgagac 27180
cccatgtcca aaaaaaaaaa aaaaatcatc agctttcatt ctgggatcgt aagtagagac 27240
attgtttccc agacctggta cagatggaac ctgcctacgt gtcttcaatg ggcatcttaa 27300
gacttatgtt ttggacatat cagacttttg gaataaagga gctgagttgg gagtacaaac 27360
tcctcttctt atccatttcc ctgtggcagg agattttgct ctcagccccc acttactggt 27420
gtgagatcct tgattctgga aggtgagctg tgctgttcag cccacaggtc ctcatgaatg 27480
tctacattca gtgccacgca gaataagaag aaacacacac cagctctgct tctgtgaagc 27540
ttactttttg ttgttgttgt tgagaggggg tcttgctctg tctcccaggc tggagtgcag 27600
tggtgcagtc atggcactca ttgcagcctc tacctcctgg gctcaagaga tcctccctgc 27660
tcagcctcct gcgtaactgg gaccacagat gtgcgccacc atgcccagct aatttttaaa 27720
ttttttgtag agacagggtt tcaccatgtt gcctgggctg gtcttggacg cctgggctcc 27780
agtgatccac ctgccttggc cttccaaagt gctgggatta cagacatgcg ccaccgagcc 27840
tggttttgct tacttttctt tttttttttt aaattcctct tagcctatct tgggggaggc 27900
gggtcagtgt tattccggtg tacatacaac aaaatcaccc atttgaagtg cacactggga 27960
ggagtccttg ccaaatggat agggctgggt aaccactgcc acatgggaca tttgggaagc 28020
cgatgtttga atgttttcac gcttacagat gcattcattt actacgttta ttattctgtg 28080
tgatgtgctt tctgtgtgtt atttcactta aacccagtgg gggtaaagat tatgatccct 28140
atttggtaga tgaattttag agaggttagg gggcttgtca aggtcacaca gcttttaacc 28200
gtgatgggat gacgcctctt gaatgaggct tagtagtgag atggctgagg aaggaaaaaa 28260
gtaggaagga gagaaagaga gaaggaggga aggaagaaag tagggagaaa ggaaggaaag 28320
gagagggaag gaagggagaa aggaggatgg aaagatggaa tgagaggaaa gaaggaagga 28380
aaccgatgaa agaaggaggg aagggctgaa ggaaggaaca ggtgccctgt tcaggtgggt 28440
ttatcctcag gaaggaccca agatggaaag tgtgtatgca agaattattt taggccaggc 28500
gttgtggctc atgcgtgtaa tcccagcact ttgagaggct gaggcaggcg aatcacttga 28560
ggtcaggagt ttgagaccag cctggccaac atggttaaac cccatctcta ctaaaaatac 28620
aaaaattagc tggctgtggt ggcacatgcc tgtaatccca gctacttggg aggctgaggc 28680

cggaggatcg cttgaaccca ggaggtggag gttgcagtga gctgtgatcg tgccactaca 28740
ctccagtctg ggccacagag caggactcta tctcacaaag tagaaataaa taaaaaataa 28800
ataaaaacag agatgaggac acagacgcac atagaggacg accctgtgag gacacaggga 28860
gaagatggca tcgacaagcc taggagagag gcctcaggag gaaccagccc tgcccacacc 28920
tgggtctccg acttccagcc tccaggactg tgagagaaga aagttctctt taagtcctcc 28980
agtctgtgtt aggttattac agtagttcga gcaaagtact ttatttacat agaagtctgc 29040
cacaattatg agccctgaaa ccctttccca ggatgaaccg aggtcagagt gtaattaata 29100
ggtacttttt ttcatttacg tttagccact agattttttt tttcaacaga gagtagcgaa 29160
tactgcagaa gctgaatgag atgaagctta agtcagatct ttatgcacct cagaatattt 29220
gtactgatga aaagaaataa ccaacacttg atagagggaa agattgagac ccagaaagta 29280
atctctccct gtgaccactc ctaccaaatt cttgcttaac taccttattt tgttcgtttg 29340
tttgtttttg tttttttgag acggagtctc gctctgttgc ccaggctgga gtgcagtggc 29400
gcaatcttgg ctcactgtaa cctccacctc ccgggttcaa gcgattctcc tgtctcagcc 29460
tcctgagtag ctgggattac aggagcgcgc cactaccacc cggctaattt ttgtattttt 29520
agtagagacg gggtttcacc atgttggtca ggctgatctt gaactcctga ccttgtgatc 29580
cacctgcctt ggcctcccga agtgctggga ttacaggtgt gagccactgt gcctggccaa 29640
ctaccttgtt ttttttggat gaaaatgtgg ttcttacctc agtaaagatt atgaagggat 29700
ttatgtacag atgactaaaa tatcagccat tagtttccta tttgtttttg ttgttgtcat 29760
tttttgtttt gagacagggt cttgctctgt tgcccaggct ggagtgcagt gatgcgatct 29820
tagctcactg cagcctcgac ctcccaggct caggcaatcc tcccacctca gcctcccaag 29880
tagttgggac gacggctgtg caccaccaca cctgggtaat tttttatttt tatttttgta 29940
gctgggatct cgctatgttg cccaggcagg tcttgaactc ccggcctcaa gtgatcccac 30000
catctaggcc tcccaaagtg ctggacttac aagcgtgagc caccctgccc agcctagttt 30060
acaatttgaa cttggttttt catctcggct cctttgaaga cttctgtctt ctcccatgtt 30120
tggggcggct ctgcgttgtg gacattcctt agtgatcggc ctgggaaggc tcagacatgt 30180
ctaggctgcc tttgtaggaa tagggattag tagcttctta gccccactct ttcctgggat 30240
gttgctgttt gctgaggtct gcacagttca gaccaccctg gaagcctctc tcaggttctc 30300
agagatggtg gagttatacc tttgactgtg agctcgcagc attgctaggg aatgtacttg 30360
gctaaatttg gaactatttg gttgaatttg taccccttgg gcatatttgc tttggtaaca 30420
taccacagac tgggtgcctt aaaataacag aagttaattg tttcacagtc ctggagacca 30480
caactctgaa gtgcagatgc ggcagggctg tgctccctct gcgggctcta ggggaggctc 30540
cttcctgcct ctcccagctc ctgggggctc caggcgtccc tgagcttgtg gccgcatcac 30600
tgcggtctct gcctccgtct ccacatggcc ttctcctctg tgtctgtgtc ttctcttctt 30660
ctcttacagg aacacccgtc attgtgttta gggtccactc caattttgga tgacctcacc 30720

ttgagatctt taacttaatt acatctgcaa agaccctttt cccaaatgag gtctcattta 30780
caggttctgt gggtcaggac atggacatat ctttctggga gaccatagtt cagtccacta 30840
cagttgtatc cagttgtttc tggaggctcc aggggaagct ccttcctgcc tctcccagct 30900
gctgggggct ccaagtgtcc ctgggcttgt ggccgcatca ctgtactctc tgcctctttc 30960
tccacgtggc cttcccctct gcgtctgtgt ctccttatta gttcaccact catattgcac 31020
caccttcagg atgacctcac cttaatatat accttttttt ttatttcatt ttttaaaaaa 31080
gtggagctga ggtcttgctg tgttgcccag gctggtctca aactcctgtg ctgggattac 31140
aggggtgagc cactgtgccc agcctttgtg tatatcttaa tcacatctgc aaagaccctg 31200
tttgcaacta aggtcccatt cacaggtacc aaggattagg atgtcaacgt atcttttcca 31260
gggcacccca ttcaatgcac acaaagtggg gagaggggag attggctgtg tttaggtgac 31320
ctgtgaaagg ccgagctagc tgtgcggcgt tgctcttgca gcacagagga agaactgggc 31380
tcagatggtg tcagctgttg gtgtctgaga tcatccgtga atctactccc gggaatgtgc 31440
tgcagtgaga cacggtcctt ctcccgtcac cttcctgacc ccatcccata tacccacgga 31500
ccccaaggga aagtactcag ggttggttgg tttcttttcc gttttgtgac cgcgtgaaat 31560
tccatgtcac ggtccctgtg tttattccat acagctctcc tgaagtgttt tgagttctag 31620
caaaatttaa aaatataacc aaagaaactt aaatccgtgt ttctcctctt tctggacact 31680
tctttagtat gaatgggcat cgtgatgata aacacggtgg cctgctgtct ttgcttctct 31740
ctctgctggc cgcctccttc cttccctcca aagtcacagg caagagggat ttagggtttg 31800
ctgggcttga ggaagagagg aaaaggccat ctcttttcac agtgagaaat gtgttttctg 31860
ttctttctca tacctcgctt tgtacattta aaaaaattat ttctctttag atttttgaac 31920
caagagagat atatttatta tttaaaaaaa attttttttt gagacaaaat ctccctctgt 31980
tgcccaggct gcagtgcagt ggtgcaatct cagctctctg caacctcctg ctccctggtt 32040
caagacattc tcatgccttt ggagtagctg ggattacagg tacccgccac catgcctggc 32100
taattttttt gtatttttag tagaggctgg ggtctcactg tgttgtccag gctgatctca 32160
aactcctggg ctcaagcgat cctcccatgt tggcctccca aagcactggg attacaggtg 32220
tgagctactg tgcccggcct atttcttttc taaatagagg cagggtctca ctatgttgcc 32280
ctggctggtc tcaacctcct gggctcaagc aatcctcctg cttcggcctc ttgagcagtt 32340
gggactccag gtgcacacaa ccatgcctgg ctaatttttg tattttttcg tagagatagg 32400
gatctcacca tgttgcccag gctggtcggg aattcctggg ctcaagcagt cctctgacct 32460
cagccttcca gtgtgctggg attacaggtg tgcaccactg tgcctggcct tatttatttt 32520
ttatttattt atttttggag acagggtttc attctgttgc ccagactgca gtgcggtggc 32580
aaccatagct cactgcagcc tccacctcct gggctcaagt gatcctccca cttcagcctc 32640
cccagtagct gggactgcag gagtgcatca tgactcctgg ctaatttctt ttaataggga 32700
tgagatctct atgtttttgt agacaagggg ctctcgctgt gttgcccaag ctggtcgaga 32760

actcctggcc tgaagccatc ctcttgcctt ggcctcccaa agtgctggga ttacagttgc 32820
gagctacggt gcctggctgt agattccccc cagcccccaa gccgccagtt gtgagtcttc 32880
actaacaagg gaacctttca gcattttcca tggtcatggt gattcaccta atattttagt 32940
tctgctgagt tgtctaaaca ttcgggataa aggctgtcat tttttggtga ccgtaatgtg 33000
tatgtgtatg tttacagtta cacatgcctg tatttgggtg gagctcagaa gatgcccatg 33060
gcatttttct gggaaagttg aagtttatga tgtttgctct ttattgggag tgtggaggtc 33120
gtctttctcc ttcttcccat ccctccagac agaaagctgg gatgtggcca ggtgcagtgg 33180
ctcatgcctg tactcctagc actttgggag gccgaggtgg gtggatcact tgaggtcagg 33240
agtttgagac cagcctggcc aacatggtga aaccctgtca ctactaaaaa tacaaaaatt 33300
agccaggcgg ggtggcgggc accggtaatc ccagctactt tggaggctga ggcaggagaa 33360
tcgcttgaac ccaggaggca gaggttgcag tgaactgaga ttgcgccatt gcactccagc 33420
ctgggcgaca gagtgagact ctgtaacaac aacaacaaca acaacaacaa caacaacagg 33480
ccaggatgtg tttgtgtgtg agttggaccc aagggcttgg gaaggatgga tagggtaggg 33540
gaggagggat agatggatga gggaatgcag agagagtggc cacactgtca gaggctttca 33600
aaaagcagga ggttctccag tgggagagag gagacctgag tccaccccgc attaatgttt 33660
acagatcaga attgcacaca tagaatggct cataaatctg atccaccttc ctcgtctggc 33720
acttggctgg agaaacaact ctttgaatgt tggaggaaaa gcccactgta ttgcacctgc 33780
tataaaaagg gcgtgcccac cttaaaagta gccacacctt cccgagattg ccatggtcct 33840
tctaagtctc tgaaatgtat agatcaggta tatttgggaa tttggggatt tttctaaatt 33900
tgggatgaga atgggtgcat gtccttttta attatacaac accctcagca gagtctgggc 33960
tggatgttgt tatcaagaca agaatatttc tgtagtgaaa taggtagata ttcaccccaa 34020
ggaggatcaa gcctgaacag tctcacgtta ggcaaggtcc agttttgcgg ccaggtgtgt 34080
tgatgaagac actttgagta ttcagagcct ttagctttag ccactgtggg taagggattg 34140
tagaacccat acttaagctt tccccttaaa gctttgactt taatctccac attgcagccc 34200
tgcaaaagaa cagaatcgtg tcctttgcag caaagtggat gcagctggag gctgctatcc 34260
taaacaaatt catgcaaaaa atcgagagcc agatattgca tgttctcact tataagtggg 34320
atctaaacac tgagtacttc atggacacaa agatgggaac aataaacact ggggattcca 34380
aaaagaggaa gggagggagt ggggaaaaga ctgaaaaact tcctattaga aactgtttac 34440
tacttgggca acgggatcat tagaagccga aacctcagca tcatgcaata tacccatgta 34500
atgaacctgc acatggaccc cctgaatcta aaattaaaag aaaagacata aactccacat 34560
ctgatttccc tcgtgcataa cactactact tagcttggag agagatggag gggatctcta 34620
gggtggggaa tggaccactt accttcaaga tctcccttat caggaaggaa agagctgacc 34680
tgcctagaaa cacttgaaaa cactcttttt ttatgtctta ttgggcaaag ctgggtcaga 34740
tggccaccct tagctgcaga gaaggctaca aatagacatt ttctcctcaa gtgggataat 34800

gataactggg gaatgtgtac tggagagcct tccaacacca agcggggaag atctaccagc 34860
accattaaat gttgcctttg tcttctgtgc acttaagctg ataaacctta aacattacag 34920
catgccactt ctgtctgctt tgagtaaggt tgtttaccga catacttctg cagtctgatt 34980
gtggcgttgg cgggactgac aatatctccc agggtagcgc aaagatccgc tgcagtgcat 35040
aagtaacgtg ttttgagttt tgtatgttta taatgcacac agctcagaga aagctttaaa 35100
gaagtcacag aagtgtaaaa aatctgttga gtcatagctg ttgttcttaa agtaggcatg 35160
gagaattctc tctgttgagg gtcaaataca agtactaaga cagaatgtta tgcagtaaac 35220
acagccaagt aatgaatggg ttccttgggg tgggtgatag agaaagataa ataaggagga 35280
aggaaccaga tagaggaaga aaagtcaagg accatattca ttgcattatt agaattttag 35340
ggtgtggcaa gaatttaatg tttcctaata atatgcaagt tctgttggaa agaagagcaa 35400
caggagacta aaaggttaaa aggttactct gttgcccatt tcataggtcc aatggcagga 35460
taagcttggg ttggatgcca gtgttcctga ttcctaattg tgttttatcc aattgaaaca 35520
tcagtggcca tagtcaaaca cctaaactgt atcagttcac agtcctcaaa gcccttctcc 35580
cacttcctgt ctttgacctg attccaccta attttgtcca caccatgttg tactgtaata 35640
aaacctattt tatttccaaa ttaactaaga gaatatactt taaaaatgtg ttactatttc 35700
attatttgtt tacttttttt aaagaaatat ttaacacctg tcagctattg acattgtttc 35760
cttcttacac tttttcttta tcttgtagtt cactttctat ttctgccata ataaaatatt 35820
cagtatcgga aacttcactt aaaaagcaaa ggttacttct cctcctccct tcccagtctg 35880
ccctataagc tagtaaattc atcattcatt agcttagata ttttacaaca tggggatttt 35940
agtttacagc aaatactcta agtaaaattg gtagcttaag tacaactttt tcttttcacg 36000
ttaccatgtg caatcaatgg cattccagac tcttggaagg aaatccattg tatggttgtg 36060
gtcttttttt ttccttctca aaccagtcaa aaatacccag tcacaattta aaaaattatc 36120
cgcccttggt ggtgcacgcc tgtagtccca actatttggg ctgaggtggg agggtcactt 36180
gagcctggca ggtcaaggct gcagtgagct attgattgtg ccattgcact tcagcctggg 36240
caacagagag agaccctgtc tcaaaaaaaa aaaaaaaaaa agacaaaaca atataatgtg 36300
atctaccctc taagcaaatt ttcatatata caattttact attgtatatg tgagctattt 36360
atatataaaa tttcatttat cagttcagta ttgttaacta tatgaactag gtgtatggta 36420
gatctccatg gtttatcttg ttcaactaaa actttgtatc ctttgaccaa tatatttccc 36480
cttccccctc aagccctacc ctctgataac tacctctcta ctctctgctt ctatgagttt 36540
gactattgta gattctgcat tagtccattc tcatgctgtt aatgaagaca tacctgagac 36600
tggttaattt ataaaggaaa gaggtttaat ggactcacag ttccacatgg ctgcggaggt 36660
tccgacagtc atggcagaag gtaaaaggag gagcaaagtc acgtcttaca tgacagcagg 36720
caagaagaga gcccgagcag ggaaactccc atttattaaa acatctgatc tcgtgagact 36780
tattcactat caggaggaca gcatgggaaa acccaccccc atgattcagt tacctcccac 36840

ctggtctctc ccacaacacg tggggattat gggagccaca cttcaagatt agacttgggt 36900
ggggacacag ccaagccatg tcagattcct tgtataagtg agatcatgca atatttaata 36960
tttgtttttc tgtgcctatc ttatttcact gaacataatg gcctccagtt ccatccatgt 37020
tgctgcaaat gccagagttt ccttcttttt tagggctgaa tggtattcca tcgtgtatat 37080
gtaccatgtt ttctttatcc atttgccctt ggacggacac tgaggttgtt tccacgcctt 37140
ggctgttgtg aatagtgctg tcataaacat gggagtgcag gtgtctggaa gatcctggtt 37200
ctcttgggtt ttgcaatcta gcataaagca ggcacaggag taccagccaa tgaatggata 37260
ggtaaatgga tcaatattga tcggtgagat ttgctcgtca acagatgttg gttcctcaga 37320
aggatacact tccaaaccat cctttctctg aatgtcagtt tctgagaagc ctctctgctg 37380
tttggggtgg gtgtgatcac ataatacggg agggcagttt tgttttagtt gctcatctct 37440
gtctgcagct gtgaatgcgt tgtttaaaaa tattgctgag gaatgacttg catgtcagta 37500
aggggctgct gacgctggga tcaaggtacg tcaaaggcaa tttagggata ctctagaaat 37560
cctgcatcag ttgcagtggc tcatgcctgt agtcccagca ctttgggagg ccgaagcagg 37620
aggatcactt gagcccagga gttcaaggct agcctgagca acgcagtgtg accccctctt 37680
tacaaaaagt acaaaaatta actgggtagg ctgaggcggg aagattgctt gagcccagga 37740
ggtagaggct acagtgagct gtgatggcgc cactgcacta ttcgttccat taacccaaga 37800
acatttatgc tacgtgaaga atatttcaat atgtgcgtga acacgtgcac accttgtagg 37860
atcagtgtgt tacaggaaga ggaggaggat gaagaggaag aagaggagag ggaggaggag 37920
aaaggaggag gaggaacaag caaagcttgt ttagagattt tgattttctt ttatatctca 37980
ttgactatta gatttcttca tttacatgca ccccagtggg aggaagagga gggagaggag 38040
gaggagatgg aggaggagga agaagtggaa aagaaaagga tgtagaggag aaggaagaga 38100
tgaaagagaa ggaggagaag attgaggaag agaaaaaaga gaaggaagag atggaggagg 38160
ggaaggagga gaagagagtg tttggattga aaatgtccca gcagaataca gtagagctat 38220
tggttttctt ttatatttgt agtggttgca gttgttgttg ttgttttgtt ttctgtttgt 38280
gggcacctat atagcacttc atcccaaccc acgccagatt gtagaatacg gactccgtac 38340
tttgagcaag ccttaatgtg cgttaaggaa acagccgtgt tgaggagggc tgtgtgtttt 38400
acaccctgtc tgtatttctg ataaaaccag agagcctgaa gagaaaaagg catgttatat 38460
aaatacattt actattactc taatgcttct ccatgtgggt gtgtggggcg tgtgggtgtg 38520
ctgtgtgcga agactgatct gcagaaatta tggccagttt gtccccaaat tgaggaacca 38580
ttcagaggca gatggctctc tctgtctccc tctctctccc tctttctctc tctgcaattt 38640
ctgtattcag tggaaaattc caggtgagcg cctctcacta aatgccatca gcccacgtgc 38700
accatgacac aaagtctctg aaagtttcac ttggggtctg tgtttgccgt cctggtgcag 38760
ccccccgctg attgcatggc tgtggtggtt tcttcctttg tcggaagact taatgacctc 38820
tgtcgatttc ttctctctcc aggcactggg acgttcgggc gggtgcacct ggtgaaggag 38880

aagacagcca agcatttctt cgccctcaag gtgatgagca ttcccgacgt catccgccta 38940
aagcaggagc aacacgtaca caatgagaag tctgtcctga aggaagtcag ccacccgttc 39000
ctcatcaggc tgtgagtccc ctcctgaagc ctctccccac gcactctccc tagacccctg 39060
tgtgtctctt cttataagaa gctgggtggg ccgagaccat ggcctgcgtg caaagcctta 39120
ggccacagca gtctccacct gtcaggtctt atcagcctct gatcttagag ctggaatacc 39180
tccagggctc ctgacctcca tggctgacca ggtccaaaaa gaaacgctct gtgttcagaa 39240
gtccgtgccc aaggcccctt gctcctctcc tgggtgaacc aagtatctct gcccggaaaa 39300
ggatgcagaa atctttgtct cctcacctcc ttgggttctg catctatagc tctcggcaga 39360
actggttaag ttggttttta agggaccaca gtcacgtgga cggggctggg cttcaggttt 39420
cgttgggttt ctggttgact gtggatgaga gataccccgt tgagatgcag cccacacact 39480
tccagctgat ggtacacaac agtcttatgt cctagggtag gtgtgatagt ccattcaggc 39540
tgctataaca aaggatccta gactcggtaa tttacaaatg acagacattt gcctcatggt 39600
tctggaggct ggaaagtcca agatcaagac acggtggatt cagtgtctgg cgaggacccg 39660
cttcctggtt catagatggc gccttttcac tgtgtcctca catggtggaa ggggtgaggg 39720
atctctctgg ggtccctttt ataagggcac tcgtcccatt catgagactt caccctcatg 39780
acctcccaaa ggacacaaca cctaacacca tcaccttggg ggtgaggatc caacataggg 39840
atttgggagg acacaagata ttcagaccat tccagcaggg taattccatt tccacccaga 39900
tgccttagac aaggatgtcc tgcgtgtctc tcctttccct ccatgggtgc cttccatcca 39960
attcccacgt cttccctccg ccatcttgct accatgcctc tcctgtccac ctatccatct 40020
tctgctttat ctgggtcccg tctctctgcc ctgattttct gtactggctt ccctcctacc 40080
tggtctttct ctcccagcct tgctcccctt gaagacattc ctcactttat agcctcactg 40140
gtcctcccag gtacctctgc aatgacctgc ctctctcctt tcatcctcag ggtactcctg 40200
aagtatccca tataaagccc cagcaatgca gcatcctcct accagtatcc tcgtggactg 40260
gccccctgcc accacctccc tacaggagaa ggtagaaggt gctgttcaca cactcattcc 40320
ggggcttctc ccaggccacc cttctttgac aggcctcctt ctttgctctc atcattgtcc 40380
ttcacttccc ccatctaaga aacacctacc tgttttccct cattcacctg ctgtccccgg 40440
gacacagggt gcctcccatc atgttcttga agcacctcgt gtgtactgga acaagtgcac 40500
ctgccttctc ctctagaccc aatgctgcct aagctccgaa caaaatcata tgcagaggat 40560
gggcgcggtg gctcccttcc tcccttcctt tcttcctttc tgcatttctc tttctttctt 40620
gacagggtct cactccttct tgcccaggct ggaattcagt ggcacaatca tggcccactg 40680
cagcctctgc cgcctcccaa ctagttgaga ctacaggtat atgccaccat gcctgaccaa 40740
cttttaaaag tttttgtagg atgaagtctt gctgtgttgc ccaggctggt ctcaaactcc 40800
tggcctgaag cagtcctcca gcctcagcct cccaaagtgc tgggattgca ggtgtcagcc 40860
cctgcacctg gactgagaat gcatgtctgt tgtctgccgc gtccctccac cccaaccccc 40920

agtttgtggt actttgttaa tgatagccct gaggaactca cacacacccc ctgcgcctcc 40980
tggagcagga gagcagccga gacgatgggc tcctgagtac tctacagttc agaaatctaa 41040
aatcccacag aagaaccgca tggacatggg agtgggtgga gtatacaagt gagagaattc 41100
cccaggatgg aattcttgag ctcccgcaag ttaaaaaata gggaggagct tgtagaacag 41160
agctttagtt aggtagttct cctttcttca aggcaggata gctcaagact gttttccttg 41220
gcagataagg tgcattcctg cagggacgtg gattctgatt ttgatattcc ctgctttcaa 41280
ttaaggaaca aagtcgttta tatttcttaa gtcttatata aagccctaga gactttagaa 41340
attctattca aagttaccta tgttggttta tttttatctt ttatataaaa aagtatatat 41400
atagatttac atttagagac gggttttgct ctgtggccca tcttggagtg caatggtgca 41460
atcacagctc actgcggccc caacctcccg ggcttaagcc atcctccttc ctcagcctcc 41520
agagtagccg ggactacagg cacccatcac tacaccctgc taatgttttt atttattttt 41580
aatttttaaa aaacatatgt atatattttt cacaacctct ttggtgaaga taatgttttt 41640
gttttttctc tatgaacagg gtcttgctat gttgcccagg ctggtcttga actttaggct 41700
gcaagcaatc cacttgcctt ggcctcctga catgctggga ttacaggtgt gcaccaccac 41760
acctacctca ttttaaaatt ttctgtagag atgggatctt actttgctgc ccaggctagt 41820
ctcaaactcc tgggctcaaa tgatcttccc acctcggcct cccaacatgc tgagattata 41880
gatgtgagcc accgtgcctg gcctatttct aacttttaag gagtgaaatt tctctgcacc 41940
gtgacagctt ttgaagagga cattttggat gcttcatggg ttgtcctttg cccgtacagc 42000
tcaccggctc ctccgagtgc tggttacagt cgaatgtgag ctgttatttg tatgtcaggt 42060
ccgcctggtg gtgttgccaa aaagcatgaa tttttcctca tcatttaaag tctacatgga 42120
atttaaggcc actttcgata ttattcttgg gaggatttta ttcagtgttt tgggagaaca 42180
tggcaggggc tcatttcaga attctctgat ttggggccga gctcggtggc tcacacctgt 42240
aatccctgca tttttggagg ccaaggcgga aggatcactt gagaccagga gttccagacc 42300
agcctgggca acatagtgag atctcgtctc ttaagaaaaa aaattaaaaa gaaaattcta 42360
gcctggccaa tatggggaaa ccctgtctct actaaaaata caaaaattag ctgggcgtgg 42420
tggcgcacgt ctataaccca gctacttggg aggctggggc actagaatct cttgagcctg 42480
ggaggcagag cttgcagtga gccaagatcg tgccactgca ctgcagcctg ggtgacagag 42540
caagactttg tctcaaaaaa taataataaa taaaaataaa taaataaata cagttctttg 42600
attttgaaat agtgcagaaa acaaggcagg ccctgaagtc cacagctctc atgaaaagtt 42660
ttggatctag ggaagtgatt gtcaacaatg agcctttaaa gacttaatgg tgatagcaat 42720
ttacaggtga cttttcccaa aaaatgacag aaaatgagtt tgccacttgg gcatgttgac 42780
tcacacttat aatcccagca ttttaggaag ctgaggtggg agtgtccctt gcacccagga 42840
gtttgagacc agcctgggca acatggcaag accctgtctc tacaaaaagt acaaaaatta 42900
gctgggtgtg gtgggggggc ctgtagtccc tgctactcag gaggctgagg tagggggatc 42960

gcttgatcct gggaggcgga gattgcagtg agccgagatt gtgccactgc actccagcct 43020
gggggacaga gcaagaccct gtctcaaaaa agaatccgaa aaacaatgta gacttggagg 43080
gggtgatgta acttagccca tgagaccgcc ttagtgactt gctgataatt gatctttcgg 43140
tgccctgcag tattatgata acctgctata gcattttgct ttttctgtgc attaacactt 43200
tagggaccaa gtggctttaa gaactggatt aaagttgatc cagtgcattc agacacttgg 43260
cattccctgt acataacacg agagagcacg ccgggtctgg ccagccccat ggataagatg 43320
ctggagggtg tccaacgttg catgctgagg agcatgtctt agaggagaat cctagatcct 43380
ctgtgaagaa tgaatttcat cctcccagct gtgcagcttc tcttctaaat gtaagttgcc 43440
ttgctcaaca tctgcttccc atacgggcag ttttaggcct tggacttctt gatgaaggta 43500
taaaacgagg actttcagcg gaggtgcctc ccatttatgg ctggaggagt ctctggggtg 43560
gggccgtcct gcacacttca gggtgttggg gagtgtccct gggctccacc caccagcggc 43620
caggagcagc aacctcagtt atgaaaagca gaaatgtctc catacataac caagtgttcc 43680
ttcagggagg gagggcaaag ttttctccac tagtgaacca ctgtgataga cagacgacgg 43740
acagagagag atgatagata cgataggtag gtatatgttg agatggatag gtgatagata 43800
atagatatta agataggtgt aaatgatggg tgattgatag gtgagtagat agatgatggg 43860
tagctaatag atgatattaa gataggtgat agataataga tactaatata tacagctata 43920
gatgatagat gatattgagg tgggtaggtg attgattgat agattaatga ccgattgcat 43980
gtaggcagag ctctcatgct aaaagagagt ttctcaacct cagcagtgct gacacatggg 44040
gcgggatcat ggaggactct gtggtggggc atcctgtgca ctgtagggtg ttgagcagcg 44100
tccctggact ccacccagca gatgccaaga gcacccatcc catctatgac gagtatctaa 44160
agatgtctct agatactgct aagttccccc tatgggggga caaaaagtta tccccagttg 44220
tgaaccactg tgacagatag ttgatggatg gatggatgga tggatggatg gatggatgga 44280
tggatggatg gagatcaaca ttgagacaga tatttgatag atgacagata ttgaggtaga 44340
tgatagctat agatgataga tggttgatag atattagata agtgattgat agatgatagc 44400
tatagatgat agctgattga tagatagctg gtagctatag atggtagatg gttgatagat 44460
attgagatag ataggtgatt gattgataga ttgatgatgg aacgtattag tagagtttct 44520
caacctcagc actgctgaca tttggggctg gaggattctc tgcggtggaa ccgccctgtg 44580
cactgtaggg tgctgagcaa tgtctctggg ctccacccac cagatgcctc tagcaccccc 44640
actccagcgt gacagccaaa gatgtctcca gacattgtcc agtgtttatt gtggggagca 44700
gaatcagctc cattgacacc cactgtagcg gatgcacaga cagataaata cctgtgcgtg 44760
tggagtccct ttctcccttt tctgccccag cccctgagcc tgcagcccca ctcttctgtc 44820
atgattgatt ggcttatggg ccaaacagtg gttccagtgc catattatct atggcacagc 44880
agccgagcca agccagtggg tttggtctgt ggttgcaggg cttagccagc tttcttctac 44940
agaacttgga aggcaagagg tctctccact ccgggccaca tccttctagt tcctggatga 45000

ccgctcgttt ctccatggag aggagttcat gccagaacct cttcttcggg gagagtggat 45060
gcatctcttg gaaacatctt tttttaaagt ctgcagactt gagtgtggtc cccattgtca 45120
ttggtacttg ttgggggtag gagtaaaatc attcctttgt tttactgaga gttcaggaag 45180
aaagaacacc atgttctctc cttttattta tttatttatt ttgctctcta tttgaatttt 45240
aaaggcaaaa atctagaaat agcgcttctt gctctgtcat cactgcctgc aaagcacaga 45300
gaccagagtg aaggcaggca gctcctgggt cttctcttcc tcttctttgt ccaaaaaatg 45360
atacattgca tgtttagtat ttggggcaaa gcagacgctt caaaaaggga ggcttatttt 45420
attatctata tttttctttt ttattatttt ttaccttgat tccctttttc tatgtatgaa 45480
aagggaggtt taagttcttg caagagaatg caactgtagt gtcttctgaa tggagttttc 45540
catttgatgc caagccttcc agtgccttgg tctaaggaga actgcgtagg gtgagaagag 45600
tcccagcgat acgtggaaaa ggggccttac cagctctgga gggcaagagg tagggggtga 45660
agacgcagtg gacacctaag gagaattttg caggaggagt aggggtttat tggctgaaag 45720
gggtgggtgg tggaaactca ttctagacag ggagtgacac ggaagccatg ggtcactacg 45780
gggcaggagg gacatggggg agaaactcaa atctgagttc agccaggagg tggacacagg 45840
aatgcagtga acccagatgt gtaggagact gccatgggga ctggggtatg aggaaggact 45900
ggctctggct cggctgtgca tataaggaag gactggctct ggctcagctg tgcatttggg 45960
ctgctggaca gtgaggagat ccagggagag agagtgcatt ccaatttggg aaactttgag 46020
ggcaaaacat ctgcagcaac tgtctcagaa gagagcctga ggcagggtcc ttggagggag 46080
gaagccacag gtggatggag ccaccgaggg ctgctaggtc accttggtta agggctagtt 46140
gcagtgcagc caggtcggaa agtagagccg ttctggagcc tcacatggca tagatgttgg 46200
aggagatggg atgaggatgg ctcatttctt tcttctcacc acaaaagccc agctttatgg 46260
cgggctctgg agagaggagg accaacttct acggtaaggc gtctccaaat tagtgaggct 46320
gatttgcctg ttcataaaaa caaaaacaaa agcaaaaaaa tcccttagag gacaagccat 46380
ccccgcagac cctgcaaata aaagactgca gatcagcttc gcggatggaa aagtgctgct 46440
gtcagctaaa gatgaggaca gagctgctca gctgaggtca ctggagagga tggggagggg 46500
acttggaaac ctcccttgtc tcagtcgttg ttgacgacat tgactctttc ttcatgttga 46560
ctttcccccc gttcatagat tcatagattg ctctctatgc tggaaaacat tctttaaaat 46620
ttaatttttt tattttgtta acatatatca cataacagtt tactatttta acttcctttt 46680
tttttttttt tttttttaaa agaaatgagg tcttgctctg ttgcccaggc tgcagtgtac 46740
tggtgcaatc atggcccact gcagcctcaa cctcctgggc tcaggtgatc ctcctgcctc 46800
agcctcccaa gtagctggga ctacaggtgc gcactaccac gcctggctaa ttttttaatt 46860
tttggtagag acagggtctc gctgtgttgc acaggctgat ctccaactac caggctcaag 46920
cagtcctcct acctcaggct ctcaagttga gctgggacta taggcacatg ccaccatgcc 46980
cagttaatat tttaattttt ggcaaacatg tgatcttgct ttgttgccca ggctgttctc 47040

5
aaactcctga tctcactcaa tcctcctgcc ttggcctctc aaagtgctga gattacaggc 47100
atgagcgtcc atgcccggcc tcattttaac catttttaaa atgtacagtt cacggctggg 47160
cgtggtggct cacgcctgta atcccagcac tttgggaggc cgaggtggac ggatcacgag 47220
gtcaggagat caagaccatc ctggctaaca cagtgagacc ccgtctctac taaaaataca 47280
aaaaaattag ctgggcgtgg tggcgggtgc ctgtagtcct cagctacttg ggaggctgag 47340
gcaggagaac ggcatgaacc tgggaggcag agtttgcagt gagccaagat cgcaccactg 47400
cactccagcc tgagtaacag agcgagactc tgtctcaaaa aaaaaaaaaa aaaaaatgta 47460
cagttcacta ctcgggaggc tgaggcagga ggatcacttc aacccaggag gcggagattg 47520
cagtgagctg agattgtgtc gctgcactct atcctgggca acagagtaag actctgtctc 47580
aaaaaaaaaa aaacagaaaa caaaaaaaac aaaacactgt ttagtggcat ggagcacatt 47640
catgtttctg tgcaaccgcc accccatccc catccccatc cccatccatc tccagaactc 47700
cttttcatct tccccaactg aaactctgtc ctcatgaaac acgccttatt gcccctccct 47760
ccagccctgc cctggcaacc tccctcgtac tctctgctct atgaatttgg tgattctagg 47820
agccttatat aggcagaatc atacggcatt tgtccttttg cgactggctt ctttcagtta 47880
ctgtaatgtc cttcagattc ccccgtcctg tagaaaggaa tttccctcct ttctaagcct 47940
gagtactatt ccatcgtata gaggatacag catgttgtgt ttacccattc attcatcctt 48000
ggacactggg gtgccttcca cctcttggct gtagtgaata atgctgctat gaacaaggtt 48060
tggcaaatat ctgtccagtg cctgatttca gctgcttgaa tacattgttt taatttatta 48120
ttattatttt ttagagatgg agtctcgctc tgtcacccca gctggagtgc agtgttgtga 48180
tcttgggtca ctgcaacctg tgcctcctgg gttcaagcga ttcttccaac tcagcctcct 48240
gagtagctgg gattacaggc atgcaccacc acccccagct aattttgtac ttttagtaaa 48300
tatgggtttt caccttgttg actgggctga tctggaattc ctcattgcca ggtaactatt 48360
tcacagcctg gtatggatcc ttctgtactt ttctttgtgt taaaaaagcc ccctcacaca 48420
aacgcacaca cacaagcacg cacacacaca agcacacaca cgtgcacgtg cacacacaca 48480
tgcacgcaca catatatgca tgcacacaca cacacgcgcg cgccccgtgc agttttggcc 48540
tcatgccact agagtatgca tcatttttgt ctcttgtttt cttggagatc cctctggggt 48600
attttgagga ggagaagttg tattattatt gtgttgttgt taatctttag acattcaggg 48660
atacacgtgg acgtttggta catggatgta tcatgttatg gtgagctttg ggcttctagg 48720
gtacccctta cccgaatagt gaacattgta cccaataggt tatttctcag ccctcaaccc 48780
cttcccagcc ttcccctttt gggagtcccc gtatctgtta tttccatttc tgtgtccatg 48840
tggacccact gttgagctcc tacttttgag tgagactatg cggtatttga ctttctgtgt 48900
ctcagttatt ttacttagga tagtggtttc cagttccatc cctgtcgttg caaaagacat 48960
gattttattt tttatggtgg tgtcgatata ctacattttc tgtgactttt atatactaaa 49020
ttttctttat ccagtcatct attggaatgg ctgttacaaa aactcaaaaa aatccaggca 49080

tggtggccca cgcctataat cccagcactt tgagagaccc aggcaggagg atcgcttgag 49140
cccaggagtt ttagaacagc ctgggcaata tagggagacc ctgtctctac aaaaagctcg 49200
aagaaatagc gagggatggt gttgcacgcc tatagtcaca gttcttcagg aggctgaggt 49260
gggagaattg ctcgagctca ggagttgaag gttgcagtga tctatggttg cgccactgca 49320
ctccagccta tgcaacaaca gagcgagacc ctatctagga aaaaaacaaa aaagtaagaa 49380
aacaacagat gttggtgtgg aagtggagga aaggggccat ggatgcgctg ctggtgcgga 49440
ggaggtcttc cagtgcgtgc agcaggggag gtgacctcac ttctcctcga gaggagctca 49500
gctgtgcaag gcagtgttgc agaggatggg agggtgcatg tgaccctagg agcaaggctg 49560
tgtgcctgtg ggcatgtact tcctggtgac gtttccttgg accccacagt gggaaacagg 49620
agcccctctg ccctctcatt cctctttctc tgtatttgca ttcgctgtct tgctggtctc 49680
aagattccca agcgctgctg ccagcccacc ccttcagcct gccaagctac atgtgtgcgc 49740
ccacttgacc actctctgag ccttctgctc agatgcctct caccaaaaat gagttcctgc 49800
tcttcctcct tgcccctcag ccctaatcag ttcctcattt gcacttcccc atttcatagg 49860
tggtccttcc agctcttggg gctcacccag gaaccatttt ttattccttt tctttgactc 49920
tgcattcact gtaatcctga gtcctgccct tttaactttt tttttttttt ttttttttaa 49980
gagatgtggt cttgccgtct catccaggct ggagtgcagt ggtgcaatca tggctcactg 50040
catccttgac ctcctgggct caagagatcc tcccacctca gcctcccaag tagctgggac 50100
cacaagtgca caccactatg tccagctaat tgaatttttt tgtgtgtgtg tgtgtgtgtg 50160
tgtgtgtgtg tgtgtgtgtg tgtagggaca agatctcact gtgttgctca ggctggtctc 50220
aaactcataa tttcaagcat ttcccctgcc tcagcctcct aagtagctag gattataggt 50280
gcacaccgcc atgccttgcc cctttccaat tctcaaacac atctccagag cccatccaca 50340
tcaatgatgt cttcacagaa gctgccatag cttatctaaa ttccctcccc ttctctccac 50400
tgatctctga tctctgctga actcacccct tcctgctgta tcagcccaaa cgtttctgat 50460
gtgaaaatag gcagggcgta gtggctcatg cctataatct cagcactgtc agaggctgag 50520
gtgagaggat tgcttgaggc caggagtttg agaccaacct gggcatctta gtgagacccc 50580
atctctaatt tttttttttt tttttttttt aaagacaggg cctttctctg ttgcccaaga 50640
tggagtgcag tggtgccatc atagttcact gcagcctcat tctcctgggc ccaagttatc 50700
ctcccacctc agcctcccag atagttggga ctacaggcct gcaccaccaa gcctggctga 50760
tttttaaatt ttgggggtct tgctatgttg cccaggctga tcttgaactc ctgggcacaa 50820
gtgattctgc tacctctgtc tctcaagtag ataggagtac aggtgcatat caccatgccc 50880
acctaatttt taattttttt tttttttttt ttttgagatg gagtctcact ctgtccccca 50940
ggctggagtg cagtggtgtg atcttggttc actgcaagct ccgcctccca ggttcacgcc 51000
attctcctgc ctcagcctcc caagtagctg ggactacagg cgcccgccac cacatccagc 51060
taatactttg tatttttagt agagatgggg tttcaccatg ttagccagga tggtctcgat 51120

ctcctgacct cgtgatccac ctaccttggc ctcccaaagt gctgggatta caggcatgag 51180
ccactgcgcc tggccctaat ttttaatttt ttgtagagac gtgatcttgc tttgttgcct 51240
aggctggttt tgagctcctg ggctcaagcg atctgccacc ttggccccgc aaagtgccgg 51300
aattacagac gtgagccacc gcacctggcc tccagctctt ttgtgtctct ccccaatctt 51360
cctttggcct tcagctgtgg ggacagagat gccccctgcc tccctgtccc caccttctac 51420
ctggtcttac aggaccccct ccagccatcc ttccaagttt actatgtaat gtctgttcca 51480
catcaagcct gcaaagctca tgacagtcac aagatctgcc gttcctgata ctatgtaccg 51540
agaacattac ttggcacata aggggccctt gtccaaaaca tttggaaacc aataaagaat 51600
gtggctggtg agttactgat taatgtgttg atgaattcat tgattgctga ggctagtatg 51660
tacagtaagt gttagatctt gaacatactt ctcatatcca ttgtgataaa ccctcttatc 51720
ttctattgct tttgaacttt tatagacttg atgtcagggg cagagtccct caggtaactg 51780
gaatcattga actgatgcat tggtttctaa ttttctaagc tcagatttga aatcacctgt 51840
ggtcactaaa ctcgtcctat cccacagccc ctgcacatga tgacttaagt tttgtgaatg 51900
ttttttggag atagtaaagt cagtatctct ttcaacattt tgttccttat ctttagtgat 51960
acccagctct gtacaaattc tgtccacgta agcaaagtct tttttttttt ttccagatag 52020
ggtctggctc tgtcactgag gctggagtgc agaggttcag tcatagcttc aactcctggg 52080
ctcaggctgt cctgctgcct cagcctctta agtacctggg actacaggcc tgcacaacca 52140
cacctagcta atctttttat tttttgcaga gacagagtct ccctattttg cccaggctag 52200
tctcaaactc ttggtctcaa gtgatcctcc tgcctcagtt tcccaaagtg ctgggattac 52260
aggtgtgagc caccacactc gcctgcaaag tcttactgat aaaagatttg aaggaaggat 52320
gtgtcagaga acttcttttt tcttataata tttacttaat cttattggtt tggggaaggg 52380
caagagaatc ccagttatct agatttttct ttttcttaaa ggagtatgtt tttacttgct 52440
aactctcttt gtaaactgtt acttcttaaa gatgctgaag ggtgatcccg cattagtgta 52500
taggtaaaaa tgatgtctcc atttcatata tactgataag gtgactttat taagccttgt 52560
ggtttgttga tgttgatttt actgagacac ttgtggaaca gatgtatttt catacaacct 52620
tacacatctc agtagttttc cccactcgtg cagtaacttt ttctcccctg ctttctctcc 52680
tcctctcccc acctcttctt ccttcccctc ctctcctcgt cccttccctc ctcttctcat 52740
ttgatacaaa gccctaactc tcttcctcta cagatcatcc tacccatggt tgaaattgca 52800
acttcccctg caagccaagt gaccgaacct gagatgtttg tattgtcagg gcttgcgtat 52860
tctacatgcc tgtgtaatcc tcaaagttct ttcattcctt gcttctaacc tacccaaatc 52920
cttctaatct cccaccatgg ttaattatct ctgatgctcc tgaaaggtga cctgctgcta 52980
cattgtgaca aggtgatgca ttattcttaa gaggatctgt gcagattatt atcccagagt 53040
gaggtagatg ggggaggata caggatatat ccaaaaatgt gttgcaagct aattgataga 53100
gagagagaga gtgtgtttgt atgtgtgtgt gtaagcatgc gtccatcttt ttcttacata 53160

tttgagccat agggaagtag agccatgtat attctcttca accaaattaa cagttgtaga 53220
cagtggtctt gctattgctt tgactattaa tggtattttc aagagctcag tgttaccccc 53280
aaatgttcag tatcacacct tggctggtca gtcgtagcta aattttcctt ccaccttgcg 53340
ccctgaagaa atttagaaag tcaccagctg acacagaagt ttattgtaaa agttcactag 53400
tacgatagta tttggacctt ggaaaatatt tccccaccag aacagtgaaa tcatgaaatc 53460
atggttgtct tctaagctca tccacaaaag tacagtttga ctgtcatgca tttgagatgc 53520
catgtgctta atcacgaaat attactgaga ggaacactct tgcccaacca ataacggtgc 53580
aaagcagaaa aacagggcat ttagctggga gaaaatgagt cgagatgtca gtgataagat 53640
ggaaggaatg tgggtatgtg ctgggtcttt tagaactcca gcagattgct ctggaaaaca 53700
gccactacat accaaaagta gccggtcatg gtggcatgcg cctgtaatcc cagctactcg 53760
ggaggctgag gcgggagaat cacttgaact cgggaggcgg aggttgcagt gagccaagat 53820
cgtgccattg cactccaagc ctgggtgaca gagcaagact gtctcaaaaa aaaaaaaaac 53880
aaaaaaaaaa acaaaaaaaa caccactcca acaaaatact tctccttcct catatagtgt 53940
ccctgcttcc ctttatgcct caagaccaca cattttgaac ttgctgagct ataagaagtg 54000
aatggagagg tgggacacag gtgcacagcc ttcagtaact gaaggaaaaa aaacagaaga 54060
aatgtctaac cctgagcttc ctgagctccc ttcagccacc cagctgctga ggggcaggaa 54120
tccagcagca aggtggagag cagaaggcat tcctgcaacc tgtatgtgtg ggtcattagt 54180
cccccacctc cagcatcatt gcagaatctg caaatttaaa agtgcgttag gtaagagcct 54240
tttttataag aggcccatat ttttgctaat acataaaaac attgtgatta gtcaaataaa 54300
tgttcattta aaattcacag tagcatgtgt ctattttttt ttttttttcc ttttagaaat 54360
gaggtcttgc tctgtcaccc aggttggagt acagtgatgt tgtgatcata gctcactgca 54420
gcctccaatt cctgggctca aatgatcttc ccacctcagc ctcccgaatt gctgggatgc 54480
caccatgcct ggctgattct tttattgtag agacgggatc ttgctgtgct gctcatgctg 54540
ctttccaaca cctggcctca agtgatcctc ctgccttggc ttcccaaagt gctggaatga 54600
caggcattga gccgccgtgc ccagcctatg atttttctta gcattaatct ctgtgtcatt 54660
gcaagctgtc tcccacgtcc ttgccttcat tctacattgt tttggtggaa tggcctgtgc 54720
tagcagacaa gaccatccgg tcgaccttga gcatccttcc ctgtcctcag ggaaggtttc 54780
ctgggcctca ttttgttttc tttgtatacc agcggggtgc agctcatttg aatgagcttg 54840
caggggagca tctgtgatag catcatgatc agatgagaca aaactggcac gggtcctggt 54900
catggttgaa gacctttatg ctcctgattc tattgggtgt cttctactgg tgaaggctca 54960
tgtgctcatg ggaacacggg catgctggtg tcttccgagt cctctccaaa aacaaagccc 55020
aggtcaggta cagtggctca cgtttgtaat cccagcactt cgggaggatg aggcgggagg 55080
attgctggag ccctggagtt caaaaccagc ctgggcaaca cagtgagacc ctgtctctac 55140
aaataacagt aaataaaaat tagccaggca tggtggcatg cacctgtagt cctagctact 55200

tgggaggctg aggtgagagg attgctcgag cccgggaagt tgaggctgca tgagccttac 55260
ctgcaccact gcactccagc ctggcagaca gaataagaac ctgtttcaga aacaaacaga 55320
ccgggcacgg tggctcatgc ctgtaatccc agcatttctg gaggccgagg cgggtggatc 55380
gcctgaggtc aggagttcga gaccggcctg gccaacatgg tgaagcccca tctctactaa 55440
aaatacaaaa attagctggg cgtggtggtg ggcgcctata atcccaacta cttgggaggc 55500
tgaggcagga gaatcgcttg cacccaggag gcggaggttg cagtgagccg agattgcacc 55560
gttgcactcc agcctgggca acaagagcaa aactctctga aaaacaaaca aacaaaaatc 55620
ccaaagccct tagttccaaa tcttgcctca taccccaccc gtcccatctt taggtgaaaa 55680
cgttctttca ttatcagtgc gtatctctcc aaactgtatt tgtaacttgt gctgagatgt 55740
tgaatgcaca gacgcacctc agtatcttct gttccttctt tcatccctac ccccaccttc 55800
ctcaatctct cattgctgcg ttttcttcca ttaatcaaca cctttgtaag gagaagcacc 55860
tcaggcaagc tcccacaagc aagtgcttcg aaatacttca tttggggctt tttaaaaggg 55920
ctaccttttc ctagcaggtt tgggatgcag tctgctagtt tattgttaat tctcaggctt 55980
aatgatagtt ccaggttact gaatcaggtg tgatctctga agatgtgtgt gttgtaggtt 56040
agttagttag tgctgccata acaaagtagc atagacggtg tggattaaac aacactgatt 56100
tattgtttta taatttttga ggctagaatt ttgagaagaa cgtcttaaca gggctggtgc 56160
ctcctgagcc ctctctcctt ggcttgcaga cgctgtcttc tccctgtgtc ctcacgtggt 56220
cgtccctctg tgtgtgtctg tgtcctcatc tcctcttctt aggactccag tcctgttgga 56280
atagggccca ttctagtgac cacattttac cgtaatcacc tgtttacatg acctgtctcc 56340
aaatacagtg gatattggaa ggacacagtt cagcccatag catactgttg gctggtttat 56400
tatgagacca tgatatttac tattcattca tcccaagaca atgagacctt ttacccatgt 56460
cccaggatac acatatctta acatctgctg ttgtctgaat acgtcctttc caaattcata 56520
tattgagaca gtaaccccca aagctatgga attaaaaggt ggagctttgg gggaagtgat 56580
tagatcgtga gggtgaagcc gctgaacagg accagtgccc tcggtgaagg ggcctgaggg 56640
agcccgtttc cccttggtcc atgggaggac agcgagaagg gcccttccct caggaatggg 56700
cccttaccac gcagttcatc agcaggcgcc ccgacctcgg acttccagcc tccagaactg 56760
agggtaatac atttttctta tttgtaagac cccctagtat atggtatttt tgtgatagca 56820
gccagaatgg actaagacaa catctatgta acatctgtgg aacttgtact ttaatgtcca 56880
tctatatgct tatcccagta tctgattata cacctcttaa catctacaca cgtcttagca 56940
tctacctata ctgatgaatt acacctttct atatgcagac ctcagcacct gtctatacct 57000
gtagagtcac atctacacac acgcatatcc taacatctgt ctgtatgcac accttagcat 57060
ctgtctatca catatattag catctatcta cacctacata ttaacatcta cttccaccca 57120
tatcctaata tctgtctgca cccacagagc aacaccggtc tacacgtcta tacccatatc 57180
ctaacatctc tctatacctc tgttaatatc tatctacgtt catattctaa catctgtctt 57240

tacgtgtata ttaacatctc tctacaccca tatcctacta tccatctaca cccatattgt 57300
aacatctgtc tcttcctata tgttaacatc tctctacacc catattctaa catctatgtc 57360
catatattaa cctctgtcta cacccgtatc ctgacatcca tctacaccca tattgtaaca 57420
tctatacgta tagatatcat ctgtcttcac ctatattgta acattgatct ctatccatat 57480
tctaacgtct gcctacatcc atatcctaac atctatacct acatattaac atctgtctac 57540
acctatattc caacatttat ctatctgtat tttatttgtc tatgcccata cattaacacc 57600
tgtctacacc cataatctaa catccatctg cacccatata ctaacacctg tctacatcca 57660
tctacaccca tatattaata cctgtctaca tccatctaca cccatatcct aacatctctc 57720
tatacctaca tattaacatc tgtctacacc tgtattccaa cacttatcca tatccatatt 57780
gtaatatctg tctgcaacca tatcctaaca tctatctata actacatatt aacatctttc 57840
tatacctata tgctaacata tatctatatc catatttaat accatatggt aacactgttt 57900
acacccatgt atcagccccg atgtacaccc atatcctaac atctgtctct aagcacatcg 57960
taacatctgt atgtacgtgc attcgaacac ctatctgtac ccatatatta acatccgtct 58020
ttacccatgt gctgatatct gtgtgctcca tgtcccgaca tctgtcccca gctattactg 58080
tctgtctgta cacacatttt ggtatctagc tgcatctgta ccccaacacc tagctgacgg 58140
cgtaccccct gccctgcctt ggcggggccc tgagcatgtg ctgtctgtcc ccgcaggttc 58200
tggacgtggc atgacgagcg cttcctctac atgctcatgg agtacgtgcc gggcggcgag 58260
ctcttcagct acctgcgcaa ccgggggcgc ttctccagca ccacggggct cttctactct 58320
gcagagatca tctgtgccat cgagtacctg cactccaaag agatcgtcta cagggacttg 58380
aagccagaga acatcctgct ggatagggat ggccacatta agctcacgga ctttgggttc 58440
gccaagaagc tggtagacag gtaagaggaa cgtgaactcc gatggaatct acactgcttg 58500
gcaatgagaa aggtggtggt agttgtggca ggtcaggcag ggaaagattg ggaaccagag 58560
tttaactctc atgtgtagga gacacttgca accagggatg agagagccag gttgacccac 58620
agctcgtttt aggtgggctg cgattgctgg caaagagcca gtgggcagga agtcagtctc 58680
cttcactggc accgtggagg aaatcagaac accacatatc cgctattgat ccgaaatgat 58740
gaggaaatga gtcatatcca agtttgaatt gccctgacta taaaatagat ctgcatcgaa 58800
taggatacaa tatctcttaa aattaaatgc caggtgctgt ggctcacacc tgtaatccca 58860
gcactttggg acgccgaggt gggagaatca tttgagccca ggagtttgag accactgtgg 58920
acaacctagt gagaccccgt ctctacagaa atatatatat ttaaaaaaaa gttgattatg 58980
gtggtggatc cctgtagtcc cagctactta ggaggctgag gttggaggat tatttgagcc 59040
ccggaagttg aggctgcagt gagctgtgat tataccactg cactccagcc tgggtaaaag 59100
agcaagatcc tatctcaaaa aaaaacaaaa caaaaaaaaa aaaaaaaaaa accaaattga 59160
atattccata cacacctgct cacacacacg tgcacttatt tttttctgtt atctgtattt 59220
ctattatgta tatatatatt tccactatat atgtatatta aatagaaata tatataatac 59280

tatattattg tatataacat atagtatatt ttatgtacat atgatatagt attatatatg 59340
tatagtatat attatactat atattatata gtatacaatg actatgatat agtatcatat 59400
aggtatacta tatattataa tatataatat atattatata gcatacctat atcatactat 59460
atcatacata tcactatact ctatatagta tatattatac gtattatata ttacacatac 59520
tacatatata tattatatag tatattatac tatataatat atataatata tatattacat 59580
agtatataat atatatagta tatatcacat agtatataat atatataata tatatatcac 59640
atagtatata atatatatta tatatatcac atagtatata atatatatta tatatatcac 59700
atagtatata atatatataa tatatatatc acatagtata taatatataa tatatatatc 59760
acatagtata taatatataa tatatatatc acatagtata taatatatat tatatatatc 59820
acatagtata taatatatat tatatatatc acatagtata ttatatatat tatatatatc 59880
acatagtata ttatatatat tatatatatc acatagtata ttatatatat tatatatatc 59940
acatagtata ttatatataa tatatatatt acatagtata ttatatataa tatatatatt 60000
acatagtata taatatatat agtataccta tatcatacta tatgacatgt atgatatatg 60060
tgtttctttt ctggaaacac acagacatac cccaaaacag tgtttgaaca aacatctggg 60120
caccccacgg cccatcaaca catgaaacaa accattacat gtcgtctcag ccagtgcggg 60180
ctgcactaac acacactcat agactgggca gcctcaacag cagacattca tggctcacag 60240
ctctggaggc tggaagtccg agatcgaggt gcgacagatt ctgtacctgg agggaaccag 60300
cttcctggtt cgtagacggc gccttctcgc tgtgtcctca catggtggaa ggggcgaggg 60360
ggctctctgg ggcccctttt gtaagggcac tcatcccatt catgagactc caccttcgtg 60420
acctcatcac ctcccaaagg ccccacctcc taatcctatc actttgggga tgatgatttc 60480
aacataggaa ttttggcggg gggcacatac attcagctta taatacatgt atatgtattt 60540
ttttttccat gattggattc atttaaccag tctgtcttga caaaagacca cacactgggc 60600
attttaaaga atagaccttt tcctctcact gtcatggagg ctaaggtctg agatccaggt 60660
gtgggcaggg ctggttcctc ctgaagcctc tctccttggc ttggagacgc tgtctcctcc 60720
ctgtgtcctc acaggattgt cctctgtgca tgtctgtatc ctcatctcct cttcttatga 60780
ggacaccagt cctcttggat caggacccac cctagtgacc tcgttttacc ttaatgacct 60840
ctttaaagat gctctttccc atatagtcac attctgaagt tctgggggtt aggactgcag 60900
catataaggt ttaagggata catttcagcc tggaacaagt atatatagat atgtttccgt 60960
acacacacat actccattga ggagaaaaca gttatccttt tttgtttgtt tttttgtttt 61020
tttttgggac gaagtctcgc tctgtcgtcc aggctggagt gcagtggcac aatctcggct 61080
cactgcaacc tccgcctccc agattcaagt gattctcctg cctcagcctc ccaagtagct 61140
gggattacag gtgcccgcca ccaggcctag ctaacttttt tttttttttt tgagatggag 61200
tctggctctg tcgcccaggc tggagtatag tggcatgatc tcagctcact gcaagctctg 61260
cctcccgagt tcacgccatt ctcctgcctc agccccccaa gtagctggga ctacgggcgc 61320

ccgccaccac gcccggctaa ttttttgtat ttttagtaga gacgggattt cactgtgtta 61380
gccaggatgg tctcaatctc ctgaccttgt gatgcgccca ccttggcatc ccaaagtgct 61440
aggattacag gtgtgagcca ccgcacccgg ccccagtatg tagtctttta tctctcaccc 61500
acctcccacc ctttacccca agtccttgaa gtccgttgta tggttcttat gcctcagcgt 61560
cctcatagct tagctcccaa tggatcttta atttacattc ataaaatcaa cagtcataaa 61620
aagtaaattc acacgcccaa gctgctgaaa aatactcaga aagatttttg gtaactacac 61680
tgagtgtcag tttttcaatt agattaaatt aatgaaagta gaattagata atgacatccg 61740
ttccttggta gacaagctac atttcaaata cccaggattg cacatggcta tcggcttctg 61800
tcttggtcag acaagcctag aaaatacatt ttatatttcc atattctttg atgtggtggg 61860
gttgggtaag gtgataatta cactaattat actcctaaca ttgtaaaacc gtctgcattt 61920
tgagaaactt gatgtggggg cagttttaca ctaaatgact ctcttctctg gcagctttta 61980
gaaaagggct ctcctaggaa aattttatga tgcgtttaca ttcaatctga ttttccccta 62040
tagtatttct tgtactacat ttccaaaaat aaggcacatg gaaattaggt gcattttaca 62100
tggagtagga aagatgcagg attttttaaa atattgattt atagcatctg tgctgccctc 62160
tcttggcagc acacggtata ttagtaaatt cttttaaagt cattttattt gcaagtctaa 62220
ttcaaagaaa acagataaaa tatttcatat attcacttgt ttataagttt gacaaaccat 62280
gttactgtcc agttaattca tcatatctat ttaaatggac gaaaatggtt tcgtatataa 62340
catgcagcaa ttattttact taatgaacat gtgaaaatct tagaaaatgt ctcattacca 62400
tattgataga ttgcttttgc aaagaactgc atcttcctct gtatgggtag tgtagcttct 62460
ggaaactttt tttttgtggg ggcttttttc tccttttttt ttttgaccat cattgtaacg 62520
aagcatctct ctgctggccc ggccccaggg aagttgtgtt catgggggca gatacatggc 62580
cctgcctctg attcaggatt caaacagtac ctgtgccttt tgaataaaac tgaaattcac 62640
cactgtatcg tggaccacta cagaatagat ggaactaaac agggaggcct catcttgaat 62700
aatttttttt tttttttttt ttttttttta aagacagggt cttactctgt cactcaggct 62760
ggagtgcagt ggtgggatct tggctcactg caaccttggc ctcctgggct caagcgatcc 62820
tcccatttca gcctcccaag tagctgggat tacaggcatg taccaccaca cctggctaat 62880
ttttgtattt ttagtagaga cctggtttca ccatgttgcc taggctggtc ttgaacagct 62940
ggcctcaagt gatccacctg ccgcgacctt ccaaagcatt gggattacag gcgtgagcca 63000
ccgtgcccgg ccttgaataa catttttgaa catatcccag aaagaagtct gtttctcatt 63060
tttctctctt tttttttttt ttttttttgg agaggagtct cgctctgtcg cccaggctgg 63120
agtgcagtgg tgcgatctcg gctcactgca agctctgcct cccgggttca cgccattctc 63180
ctgcctcagc ctcccaagta gctgggacta caggcgccca ccaccacgcc cggctaattt 63240
tttgtgtttt tagtagagac ggggtttcac tgtgttagcc aggatggtct cgatctcctg 63300
acctcgtgat ccgcccgcct cggcctccca gagtgctggg attacaggcg tgagccaccg 63360

tgccccgcca tttttctctt atagcatggg gtaaaaacat acttaagcat gttgtaatgt 63420
aatctactca ctacatgaat ttctttcttt gttttttgtt tcttttttga gacagggtct 63480
cactctgtca cccaggctgg agtgcagtag tgcaaccatg gctcaccaca gcctcgaact 63540
tgcaggctca agcaatcctc ctgtctctca gcctcccaag tagctgcaac tacaggcttg 63600
cacaccctgc ctggctaatt tttggatttt ttgtagagac ggggtttcac tatgttgcca 63660
gggctggtct tgaactccta aactgaagca gtcctccaac cttggccccc caaagttttg 63720
ggattacagg cgggagctac ggtactcagc ctctccctcc cttccttcct tccttcctcc 63780
ctccctccct ccctccctcc tttcctctcc ctcccctccc tcccttcctt cctcccctcc 63840
cctcccctcc cattccttcc ccttccctcc cctcccattc tttccccttc cctcccctcc 63900
cattccttcc ccttccctcc cctcccattc cttcgcctcc cctcccctcc tctatcctcc 63960
cctcccctcc cctcgcctcc tctaaccctc ccctcccctc ccctcccctc ccctccccta 64020
gcaaccctgt taactggatg acttgtaccc tacttatttt aaaccactgc tcttggtgac 64080
ttgatttctg actttcctag gttgtcagta tgttcagatg gtgttctttt gtgacttgcg 64140
ttcaccaggc tcttgttttc taacagcaat attacgtttg tattgtaaat attttgcaca 64200
cacactttat gttatctaga tagctcccct aaaagatttg gtggcaaact agaattattt 64260
ctggttttat gaaatggtca aaataaaata ctgatgtgtt taatttgatt ttataattca 64320
aatgcataac acaagcatcc cttacctgaa atacttcaga ccagaattgt tttggatttt 64380
agattttttt cagattgtgg aatatttgaa ttacacttac ctgtgagcat ccctaatttg 64440
aaaatctgaa atccaagatg ctccagtgag gatttccttt gcgctgtgag cctcatgtcg 64500
gtgctggaaa agtattgact tttggagcat ttgggatttc agatttggat taaagatgct 64560
caacctttat acatctgtct ttaaaccata acagatggtt ttctttctct gcaacagctc 64620
ttttttgttt gtttttggtg ttttttattt ctctgcgaca gctttttgtt tgtttgtgtt 64680
tttttgtgtt tcttttgaga cagggtccca ctatgttggc caggatagtc ttgaactcct 64740
ggcctcaagc aatccttcca cctcggcctc ccaaagtgcc aggatggtag ccgtgagcca 64800
ccatgccagg tgcaacagct ctttcctagt tcctctgttg tagcatttta aatccagatg 64860
ttacattgat tactgtcctc tggattttta tttctagcct tatagggata acattgaacc 64920
tagtctttca aaattactac ttaccccttt cacacacaca cacacacaca cacacacaca 64980
cacacacaca cacacacaca caggagtaga gagagtaatt cacattgaaa ccttatggcc 65040
ccatcaccca gcttcgattg ttatcaaagc tttgtcactt tgtttttttt ctctttgccc 65100
atttgtttct tgagttattt taaagcaggt gcactttatc ctatctttaa cagataactc 65160
cctttaaaaa agaacttcag tgccattatc acatctgaca actatttaaa aaaaaaaaac 65220
ccagcctggg taacatagca agacctcatc tctacaaaac atcaaaaaca aaaaaattag 65280
ccttgcatag gttaaatcca gccccctatg tgtctgttaa taaagtttta ttggaacaca 65340
gtcacatctt gggggctttt gagctaccgt agcagagctg gatatttgta acagagacca 65400

tgtgattcac aaggcctaaa atatttgccg tctggctctt tacagaaaat atttgcccac 65460
ccctgagcca ggccatttgc tttgtgaact ggttaaaatt ggccaggagc tctgtctgtg 65520
cagtggaata ttatttggcc ttgaaaagga atataattct gacacattct acgcgttgat 65580
aaaccttgaa aacatgatat tggacgaaat aagccagacc aaaaaggtct catgtcgtgt 65640
gattctactt gtaggagggc cctagattct tccaaatcat aaggacagaa agtagaatgg 65700
tgggtgccag gggccgggga ggtgcaggga aggggaagat gctgtttaat gaggatggag 65760
tttctgtttg gaatgatgaa gaagttccag cagtggacgg tagggagggt tgaacacgaa 65820
ggtcagtgga cttcccacca ccgaactata caattaaaaa tgcttaaaat ggaaaattct 65880
atgctgtgtg tattttacca cattagaaaa tcatacatat aaaatcataa ttatgtatat 65940
aattataaat aactatataa ttgtaatatt tacacataaa taatatatcc gacattaaat 66000
ttatatattt aaatatattt atctctttaa tatattaaat attttagtat attcatatat 66060
aaatttaata cataaagata ttaatgtaat gtgttaatta ctaatataac aatgtatgta 66120
tatattaata tactaatata ttctaattat aattatatat taaatatgta atttttagtt 66180
aaacataatt atatttaaaa aataatttaa tacaattaaa tatataataa atggatacag 66240
ttttatattt aatattacat atttaatttt ataaacatgg ctaccaaata attatatgtg 66300
ctcataaatt atggtataaa tatgtttaga aagttatatg tagctataat tatacattat 66360
tatccatagt tttatatata attttataaa tatatattta tttttaaaat ttaaatttat 66420
atattagtat gtgttacaca tttagtgttt agattataag ttcaaattta aacatataca 66480
tttataaata cataatcgtt ttatatatat ttcataacac cagaaaagtc tttctagtta 66540
ctttagacct tttcccctag cttcttactt tgagtattta acggaagtca aggagggcct 66600
gagatgtcga gtctgtttgc agtcggtgtt gttactttcc cttgagcccc ttttaatttt 66660
gctgtagttc atactaggac gtgggtatta ctgtgccgag gttggtgaca ggcacccttc 66720
tccctgctga tcagtggtgc ctaggagcca ttgtcaccga ggaaacccct cccagggcct 66780
gggctggcca tcggaagctg tcccatgctc gcaaacacac accttctccc ttcacgggcc 66840
tgaaattctc aactgtttgt ccccatcaga cagttctagg aactgtgatt gtgtccccat 66900
aaaaaaaaag atgacaaaat cttccctttt tatgaaaaac cccacaccct gctgctttat 66960
tgcaacccaa gatcctataa gaaaaggcaa tgtagtattt tcaccatgag tactgagcga 67020
acatctattt cctgcattac caaacccaga caaaacactg gctcatactt ttctgataaa 67080
aagggaaaaa atgcatttgt atctgtaatc aagtgtaaaa gctgtaatca gctgtccttc 67140
ctcctgagcc ctaccatgcc cctgttagct gagtgacatg ctcaccatgt cataaagcca 67200
tgaggagaga cagaaagtga ggctggtgac gccacctgct aggacgtaag agggactgga 67260
ctgaaactca gcttggagat gtcatccgtt tctctctgtc tctctctctt tttttttgag 67320
acggagtctc actctgttgc ccaggctgga gtgcagtggt gcgatctcgg ctcactgcaa 67380
cctctgccac ccaggttcaa gtgattctcc tgcctcagcc tccggagtag ctgggattac 67440

aggtgcctgc caccgcgccc agctgatttt tgtagttttt agtagagaca gggtttcacc 67500
atcttggcca ggctggtctt gaactcctgt tcatgatcca cttgcctcgg cctcccaaag 67560
tgttgggatt ataggcatga accaccgtgc ctggcctctt tttctctttt tattgtggta 67620
aaatacactt aaaatgtacc atcgtaacca tctttattaa cattttttat gtttcagaat 67680
agggtcttcc tctgtcagcc aggctggagt gcagtggtac gatcatggct cactgcagct 67740
tccaatgcct gggctgaagc aatcctcctc cctcagcctc ctgagtactt gagactagag 67800
gtgtgagcca ccatgactgg ctaactttga aatatttttg tacagacagg gtcttgctat 67860
gttgccttgg ctggtctgga actctgaggc tcaagtgatc ctcccacctt ggcctcccaa 67920
agcgttgaga ttataggcat gagccactgt acccagcctg tttgaaccat cttgccgtgc 67980
acaattgagt ggcatttatt gcatttacca tgtcgtgcaa ccatcacctc tctctagttc 68040
cagaacattt tcacgatccc aaaagagaac ttgtatctgt taagcaatta ttccccatcc 68100
taagccagga tgatctcatc ttaacttgat tgcagctaca aagatcgtgt ttgcacataa 68160
ggtcccactc acaaatactg gggtttagga ctttgggggg tgcacagttc aaccagtgca 68220
ggtgtggcgg aaggcgtggg cattaagaac acaggagtca ttggatatct atactttata 68280
gataatccta tccttagctc tgtttctcct tcgttaacat ggcctcctcc ctcccctccc 68340
cccggccata gtgaaactaa gaacagtgag aaaactgaga acagcaggtg tcaccttcct 68400
tctacccaaa agccaggtga gcaaaaggag acaggagcag ggaagtggat aattcactgc 68460
ccagaccagt ggtctcgaca taacactcag gctgtctcat ctcatattca ttcacgcagt 68520
cagcatctct ccatagcagc gtatgtctct ctgtacattc agctaggcgt tggggctttt 68580
tgcaggaagg agtgactcta acagggcagg agtcctcctg aggcaagtta gaacctggat 68640
acttatacag cctcatacaa cctcaggtgt gtgtccttac cctaacacgg agccaaaaag 68700
agcctcatct ttcaaaaggt aagtggagaa gttgcagcta atgcctgtag catgacctgt 68760
ggcctctacc tgggcttcat atcagctaag atctaggctt ttagctactt tcaggccact 68820
ttggatgggc tagaaaggtg gaaatacaaa actagcaggc cagtaagtcg ctcgtcttgc 68880
agaatgacag aagtgtcgtg cattgaagca gccagggtgt acttggagtt gattcaactg 68940
ctgggcaggg caggggagag cgtgtttcta aaatagtgcg cattgtactt tccatttgta 69000
gacttaattc taatgagtga gaagcactta acaggaccac aaggtggtcc ctgttccata 69060
cttagctcta acctgcagat agagagtaag tcccttccac atgcttccat gggaggagaa 69120
ctcaatttct ctccttgtct tctttcctgg tttcctgatt tatatctgct acatcctgca 69180
gtaatttttt tttttttttt ttttttttga gacagtgtct tgctctgttg ctcaggctgg 69240
agtacagtga cactatcatg ctccctgcag tgttgacctc ctgggctcaa gtgatccttc 69300
catctcagcc tcccgactag ctgagactac aagtgcactt cagcacacct ggcttttttt 69360
tttttttttt ttttgtaaag gagtggtctt gctgtgttgt ccaagctggt ctgaaactcc 69420
tagcctaaag caatcctcct gcctcaacct cccaaagtac taggattaca ggtgtgaact 69480

accatgcctg accttgtggt acttttattt catggatggc cgacagaatt tgaaatttgg 69540
atggcagcca gtttgttgtt atcccaaggg cttacagatt agtgtggaaa agtctgttgt 69600
ctggaaagag atggggtatg gaggtgtggt ggggaaggga aggaaagata acagtaacaa 69660
cattttgggt gggttcctgc aagcaggaag ctgaggagct gaagccaaga tacgtgccca 69720
caccactgca aacagtgcgg actcaggcaa gatgtgctgg gtataaaata ggctttgcac 69780
atgcttgata cattgtgcaa agaacttgag agtggataaa catgtatatt accagactct 69840
gtggaacttg ttttttgttc tctttacatt caacttacaa aataagggtt tcttctttca 69900
ttgatctgag tggtgggagg gaagggtctt ttgcaaagca tctatatatt ttttccctct 69960
gagggtgttg cttctttctg cctgtctaca gacaggctcc acacaccagg gcagcacttg 70020
gcaggaaggg tggatgtggc ccgcatttgt gctacaggtc tgaattgctg aaagcccgaa 70080
gcgacattct acccagcagt agagcgccaa tggagtacgg cccagacaag gccccctctc 70140
tggggttact gatactcagc tcctttccag cccatgagtc ttccagcttc tgctgcagtc 70200
cttgttatct tgccctgact ttatttagca gattcttctg taaccaaaat gcaggttcag 70260
ccgctcgccg tttgcagagt gcaattagca agagcgaggt ctagtgtaaa gaaagtgatt 70320
ttttattcca gagctttctt aggggaagaa gtacaggctt cctgcttgag gccacccctt 70380
tgcttttgga gcacaaagca ggcaccttta aaggaaaatg gtatgcaggg gaggaagtgt 70440
atttctgtct ttacactggt ttccaggggg caagaaatgt tttttggggg ggtgggggtg 70500
taaagagcca aagagaacat acgtaagctt tgcaaaccat ccagtctcag ttacaagtac 70560
aaacccagct cttgcacctt tttttttttt tttttttttt ttttgagaga gagtctcact 70620
ctgtcgccca ggttggagtg taaagtgcag tggcgcattc ttggctcact gcaacctccg 70680
cctcctgggt tcaaacattt ctcctgcctc agcctccgga gtagcttgga ctataggcat 70740
gcatcaccat gcctggctaa ttttgtattt ttagtagaga tggggtttca ccatgttggc 70800
caggctggtc ttgaactcct ggcctcaagt aatctgccca ccttggcttc tcaaagtgct 70860
gggattatag gtgtgagcca ccatgcccgg cctaagcgag ttcttgaatt attcttttca 70920
ctgccacttt tttttttgga taaaggattt tctttttact taattttatt tatttattta 70980
tttaaagaac ttttaggttc aggggtacac gtgcaggttt gttacatggg taaactgtat 71040
gacacttggt gtactgatta ttttgtcacc tgggtaataa gcatcatacc tgacaggtag 71100
attttccatc ctcatccttc tcctgtgctt ctccttccag cggccaggtg tctgcttttc 71160
cccctctctg tgtctgtgtg tccccagtgt ttagctccca catacaagtg agaacatgtg 71220
agtccactta ggagaatggc ctctccacct ccgtccatgt tgctgcagag gacatgatct 71280
cgttcttttt cctttaatta attaatttta ttctttatga tttatactaa ggtataaata 71340
actcaaaaaa aatgaggttt tttttgcttt ctgagtaaca gtggtaacag taccagcaac 71400
aggagcaagg atgataaaac caattcttag cttccaagcc cgttaagaaa tgtttctatg 71460
gaaaggaatc agcgaaaaca atgttactgt ctttacacat taaaatatgt gcacaggagg 71520

gtaaacggag cgtggtttta ctcaacgaag ttattgtaac aatcagagct tctaaggtga 71580
ctgcattagc caagcaccac tgagtgttgt gggggcctgt ctctgtcgcc catataggac 71640
ttggaccctc tgtggaacac ccgagtacct agcccccgaa gtcattcaga gcaagggcca 71700
cggaagggcc gtggactggt gggccctcgg catcctgata ttcgagatgc tttcggggta 71760
agtagagtct ctgtagagaa tcttcatctt acaggccagc acccccttcc cccacccatt 71820
cgtccactcg gcatttctgt aaccttgaaa acagtacgtg agtgtcgcaa acacacagtg 71880
tggctgcaca cacatctgct gccctgctga ggttggcaat gtgaattagc aattgagctt 71940
gtgtgaataa gaggcaaaaa acccaaactt gtgaaggaac cacccatgca tgcccattga 72000
gatttttata ttgaaatatc catatcttct ttaaatatac aattaacatt ctgggctgct 72060
ctgaaggtag tgagttatct cagttgattt tcacagtcag ctacagattg aactccttgc 72120
tctactcttt tcccccctta tcacttctgc gcttgtttag tcttaaaaag aatttaaaaa 72180
ttaaattaaa aaatgaatct tctgcacaga ccctttctga agtaacctgc agagctcagg 72240
ggggcacagg ttgggtcaag gtaaatcagc caaatacagt tcagtggttt ctggatgaac 72300
agctggcaag gaaggcgaga atatgttctt tcctaaaacc gtatgtctgt ggtacaggaa 72360
aggggtcccg acccacaccc cagcacaggg ttcttggatc tctcgcaata aagaatttgg 72420
ggaaagtcca taaagtgaaa gcaagtttat taagaaagta aaagaataaa agtatggtta 72480
ctccttaggc agagcagccc tgagggctgc tgtttggcta tttcgatggt tatttcttga 72540
ctatatgcta aacaaggggt ggatttttcg tgagttttcc gggaaagtgg tgggcaattc 72600
ctggagctga gggttcctcc cctttttaga gcatacaggg taacatcctg accttgtcat 72660
ggcattcgta aactgtcatg gcccttgtgg gagtgtcttt tagtacctaa tgcattataa 72720
ttaatgtata atgagccatg aagacgatca gaggtcactt gtcgccatct tggttttggt 72780
ggcatttggc cggctttact gcagtctgtt ttatcagcag ggtctttgtg acctgtatct 72840
tgtgctgacc tcctatcttg tcctgtgact tagaatgcct taaccgtctg ggagtgcagc 72900
ccagtaggtc tcagcctcat tttacccggc ccctgttcag gatggagtta ctctcgttcc 72960
aacgcctgtg atacctgtgt tgggtgagat ttggaagtca ggagaggtgc catccaccct 73020
gcaaccacat aacagaggtc ggtcatttgt tgggacgtca gcatgattga gtgttcaagt 73080
ctagcatatg cctgagacag ctccactatg actagaccag aaacgcaaag gaagcacgca 73140
acacttggaa acccaggacg tgggcgtcat cctcgttccg ttgttagtgc taagatgaca 73200
cagtagcgtg gcacttttcc agataggaac ctgtaggaag gagtgagcta cgtgtcagag 73260
gaactgttgc accatgtggc tctgcacgaa aggctcttct ctctgggatg aagacgagag 73320
gaagcccagc cagcgagggc ccagaacacc ggttggggga gcctctgccg cgctttgcca 73380
cagccgacac tctgcctcct aggttctctc ttttcctgtt tccttctttc ttctgcttgc 73440
ccctgagtct caaagacaga caccagcgta tcctctgagt ggcctccatt gtcgcaaaag 73500
ccagatcctc cctcacatct ctctgattcc ctgcaggggg taaagaccag agttttactg 73560

gggaaccttc cctttcagtt ggaaacaaag gagggaagga agagaggcat tctcctgcct 73620
ggaaggcgtg tctaagccag gtcctgctgg tggggagcac cgagcgcttg ttcctggggg 73680
tgtggagcat gtgacattct aacatccacc tcaggaaatc caggcagtcg ctgactttgt 73740
ttggggagca gacaggggtc atcgtcccat gtgcttatct ctgccctctt ttataaagaa 73800
agtgaatccg ttaattccca cactagctta gggatcagaa cgatgttgaa caagcactac 73860
caaagaattc caccacaaaa ccggagcctg agatgccgca ggaggagagt ggaacccgta 73920
aaaacctgta tttcattgtc tagctatcca tgtgtattgt ttatcatcca tccatccatc 73980
tacccacctg tctatttatc tatgtttata tacttttcca tccatccacc cacctacccc 74040
ccaacccctt catccatcca cccacccacc ccgtcatcca tccacccacc cactccttca 74100
tccatccatg tacccactcc cccaccccct accccttcat ccatccaccc ccccacccct 74160
tcatccatcc acccccaccc cttcatccat ccatgcaccc acccccccac ccctctaccc 74220
cttcatccat ctaccacccc accccttcat ccatccaccc ccaccccttc atccatctac 74280
caccccaccc cttcatccat ccaccccccg acccccccac cccttcatcc atccatccat 74340
ccatccatcc acccacctac cccttcatcc atccacccac ccaccccttc atccatccac 74400
ccacctgccc acccaccgct tcatccatcc acccacccct tcatccatcc atccatccac 74460
ccacccttcc atccatccat ccatccaccc acccacccac ccacacacct atccctgtct 74520
atctctatgg tctacctatt ttgtaagtat gtatgtatct gtttatccat ttatccttat 74580
ctatcatcta agttgtcatt tatatattta tatatccatc tctctccatc tctttatctc 74640
tacgatcttt ttatctgcct acctacctgt tatctatgta tttacctatc atctatctgt 74700
cattcatgta ccaatctatc catctacttc ctatttatct atgtgcctat ccatctattt 74760
atcatctgtc tttctatgta actgtttacc tccctaccta tccatctatc aatcatctat 74820
ctacctattc atctatctgc ttttctatat atctatgtat ttatcaatca tctacttacc 74880
tacctatccc tctatctcta cctttctgtc tatctagcta tcaataatct acctacctac 74940
cggcctgttg gtcttttgaa ttttccatta ctctttgctg agacatgcct acaagtcata 75000
tagaaccaaa cttctcaata tgcaccctag cccattatgt cctaggcaaa cccaaaaggt 75060
taaaaataac ttaggcatat tgatcagcgt ccacccaagg aaaagcacca cattttcctt 75120
cggtgataac actgaccaca cgagggcagc attgcctgag gatcgttgta tcctgtagtc 75180
taatggtttt aaatagagtg aggttcgtgt gccccaaatt ttattgattc taggatagag 75240
tagactatta ttagaataca atatactatt aggacataat attgttagaa tacaacatag 75300
aactattaga ctatagttaa aacaatatta gtctcagggt acgctgaagt taaaaataca 75360
taaaaataaa tttaaaaaga atagaatatt acactcttag aatgactgta ttcgaattat 75420
ataaagttac cagaattgtt aataggctat aacattggaa tatttaaata tattagaata 75480
caatagaata gaaaataacc taattgtaat gtaataaatc attaggctaa ttgaaaatga 75540
ataattaggc caggcaccat ggctcacacc tgtaatccca acactttggg aggccgaggt 75600

ggttggatca cctgaggtca ggagttcgag accagcctgg ccaaaatggt aaaaccccgt 75660
ctctactaca catacaaaaa aaagccaggc atggtgcctg taatcctagc tactcgggag 75720
tctgaggcac gagaatcgct tgaacctagg aggcgggtag gttgcggtga gccgagatcg 75780
caccactgca ctccagcctg ggcaacagag gtgagactcc atctcaaaaa gaaaaaaaaa 75840
aaaaagaaag aaaatgaata attaaattaa atgaataacc gtctcccaat caaagcccag 75900
tctgctgacc tgtgcatttc atgaggcagg gagctattga ttcacagtaa gcctattttt 75960
attttccttt tttctaatta gagtctattt ctgggtgaat attgtttttt caatttctca 76020
gcagccctgt gtctcttcac atgggtgggt gttttgagag ttaccagctt cgccgacccc 76080
aactctccca ggcattcctg tgctcttccc cagctccagc cggcctgagc tcgcctggac 76140
caccccaagc cttctcagtc tcctgcatcc cctcctgcct gcagtacatt ttcctggaag 76200
cagctgcagg catcctgtcc acgtacccat cagctgtgtc cctattccct gggagcccgc 76260
ttgttgcttc ccggatgact ggagcagaat cggaggctcg ttatcatatg aggttctgag 76320
acggtctgcc tgtgggttgt ccacccctct tagacaacag cctggcccct ccttgtcttc 76380
ccactgcttt ttaaacgtgc caggaagcct gacctgtccc cctgcccaaa ggctggcttc 76440
cttcctcctt cttttctttt tccctttcct ttccttccct ttcctttcct ccctccctcc 76500
ctctctcctt ccttcctctc tccctccctc tctctctctc tctccttcct cccttccctt 76560
tcccttccct tcttcctttc cttttccttt cccctctccc tcttcctttc ccctttccct 76620
tttccccttt cccctttttc ctttcctttc ctttcatttc ctttccttct tcctttcctt 76680
tctgttccgc ctcgctctgt cacccaggct ggagtgcagt ggcacaatct tggctcactg 76740
taacctctgc tttcttgtct cgctctgtca cccaggctgg actgcagtgg cgcaaatctt 76800
ggttcactgc aacctctgct tcccaggttc aagcaattct ccttcctcag cctcctgagt 76860
agctgggatt acaggtgtgc gccaccacgc ccagctaatt tttgtatttt tagtagagag 76920
ggggtttcac catattggcc aggctggcct caaactcctg acctcaagtg atccacctgc 76980
ttcggcctcc caaagtgctg gggttacagg cgtgagccac cacacccagc cagggctggc 77040
cctttctctt ccactgcaca tgctgcaggc tcacctgtca cctgctccca cagccctctg 77100
gggcctcccc tgttctcctg ggccatgccc tgtccgacca gcccatctgt tttctttata 77160
ctgattagca gctctgaaac catctaaatt ttgtatgtgt tgactgcctc tgagagcaat 77220
gctcccaagc tgctctcctc acctccttga acccagaaac ttctggtggc tggagggagg 77280
gcactgctgc acatacaagt ggagccggcc tgcagtcaga gtcttcctgc tcccttcgca 77340
gcctgtgtct gcacctttcc tatcgcagct ccatttgctg gattcccact cttagcagga 77400
tcaagtgaga ctgtgccttg tctgtgttat tgctttaagt gattatctca atcttccact 77460
aagcacaggg aaaaccatct atttggtaaa atctgccatg tgattaggaa ggagatcata 77520
tcatgatgca gattttattt tcatggaatg agacatgttt aggtcagatg aaaattgtgt 77580
ggcacattct ggaacaaaat cactcatgca ctttccaagg acccaagctc agctccgatt 77640

tcttccatcc tattctcttt cctcatccat ttctgtggtt ttattctccc agagagttta 77700
actcaagagt caggtaaagg ctggacgtgg tggttcatgc ctgtcatccc agcactttgg 77760
gaggctgaga cagaagaact gcttaagccc aagagttcaa gaccagcctg ggcaacatag 77820
tgggacccca tctctacaaa aaataaaaat aaaactagct gggtgtggtt gtatgttcct 77880
gtagtcccag ctactcagga ggctgaggtg ggaggattgc ttcagcccag gaagtcgagg 77940
ctgcagtgag ctgagattgc accagtgcac atcagcttgg gtaacagagc aagacactgt 78000
ctcagaaaaa gaaaaaaaaa aatcagctaa agcatcaaga acaaaacaca gggacacaca 78060
agacatacag cagagtgcag ggtaagagaa agcccctggg ccatcttgtc ttagttgtga 78120
cagcagtgat tctcaaagtg gcatcccaga ggactatggg gttcccgaag tgtgtttcag 78180
cagatgagtg agatcatttt tccaatgaca gatctgaata gtgatgaatt ttcttcatag 78240
gtgtcaacta agacaataca gcatagcaga cagggtgcag aagatcctag agaaggtgga 78300
atttggctgc agtggagcct cttccacggg gagcattagg cattgcgcag tggttctcaa 78360
acatgttggc ctcgggaacc cttcaacaca cagctcaaag ttattaacaa cccacagaac 78420
tttcatttct gtgtgttaca gctgtccgcg ctgactacat ttgaaattaa agtagagact 78480
gaaatataaa ccaacagaat acataaataa tatatatgag aagttaaata atcagaggag 78540
aacataaata gcatttttaa atgaaaaaaa tgtgtaacca cactttctag aattaaaacc 78600
attcaggctg gacatggtgg ctcacgcctg taatcctagc acttcaggag gctgaggtgg 78660
gaggatcact tgagcccagg agttggaggc cagcctgggc aacatagtga gaccccattt 78720
ctacaaaaaa taaaatgaaa taaataaacc aattagctga gcatgatggt acacgcccag 78780
ggtcccagta ctttgggagg ctgaggtggg aggatcactg aggctgggag gtcaacgctg 78840
cggtgagctg tggtctcgtc actacactcc agcctgggtg acagagagac cctgtctcta 78900
aaaagagaga aaacatttgt gaggaggttg gctgtatttt ctgggttttg tgaatctctt 78960
tagtgtccag ctttcacaag ggctgctggc ctctcctccc tctgtctgca gcctgtctgc 79020
tgtgctgcgt ggcacgttgt ttagttgagg cataggagaa aattcatcac tattctgaac 79080
tggagtcaga aaaacgatct cagccatccc taaactgagg tcgggaagcc accctgggac 79140
tccactttga gggccactgc tgtgatgccc aagtcaacgt gcccggggct atattttcac 79200
cctctactct gccccaaccc tcacacaccc cagtgcctgc tccttccgct ttaactggcc 79260
ctcaagttcc cctttctcat ctcccacctc agcctccctg attcatctga ggagcacaga 79320
ttttatattt atttattaat tattattatt ttttttcttt agagggaccc ctgctctgtc 79380
acccaggctg gagtgtagtg gcgcgatctc ggctcactgc aacctctgtc tcctgggttc 79440
aagcaattct cctgcctcag cctgccgagt agctggggtt acaggtgcgt gccaccgtgc 79500
ctggctaatt tttgtatttt tagtagagat ggggtttcgc catgttggcc agcctgctct 79560
caaactcctg acctcgagtg attaacctgc ctcagcctca caaagtgctg agattacagg 79620
tgtgagccac cacgcctggc ctaaatttta gtttttaatc tgaaggtatc gggacaagga 79680

tttttgagga agaagtcaag gtgggatgaa attctagtga aattctagtg acggctttct 79740
tcttttctct aaaaacctct gccttccttt tcttgaatgc atttccaaag cacctgtctt 79800
ccaaggccag ccccggtcct cagaactgga gcaggtagtg tgtcagctga tagtttagga 79860
gggtgttttt ccagctgagt aggagggaga acatcagctc acagcgccca gggtatctct 79920
gcctgcacca tccactttac tggaagatgt aaataggcgg aatggttaca ttcaaatttt 79980
actggcaaat gatagagcta gtggcacaat tggtgtaaat ttttttcatt tttctttttt 80040
tgtttttgca gacagttttg ctctgttgcc cagtcaggag tgcagtggtg cagtcacagc 80100
tcactgcagc ctggacctcc tgggctcaag caaaccccca cctccgcctc ttgagtagct 80160
gggatgacag gtacacgtca ctgtacctgg ctaatttttt aaaatttttt tgtagagata 80220
gggtcttgct atgttaccca ggctgatttc aaattcctag gctcaagtga tccacctgcc 80280
tcggcctccc aaagtgctgg gatgacagcc gtgagccact gcatgagcgt tttttttgtt 80340
tttttttttt ttcttttgag acagggtctt gttctgtgtc cagattggaa tgcagtggtg 80400
cagtcatagc ttactgaagc ctcgacctcc tgggctcaag cgatcctcct gcctcagcct 80460
cctgggacta cagaggctgt agctggggac cgcagaggct gtagctggga acacaggcac 80520
acaccagcac gtccagataa tttttaaatt ttttgtagag acgaggtctc actgtgttgt 80580
ccaggctggt cttgaactcc tgggctcaag cagtcctccc gcgttggcct ccccaaatgc 80640
tgggatgaca ggcatgagcc actgtgccta gcctgggcat gaatttttca ggcgaaaatc 80700
tggagcttgc attcatgctg attagcagat tgtccttgca agtgtagctg tcaccacaga 80760
tcaatttaac ttgcttaata atgccaaatg ccatataatg agaatttatt ctccacttgg 80820
taagaattgg aagcagtctg tatgtttaga gacagaaatt tgagggtttc agagggcact 80880
gcccaggcgt ccgggggagt gctgtttctc agacacacac acacactgtc tctgcttttt 80940
ctttcaggca agatcatcag ttagtgctca cttattattt atctttttcc gcttattaat 81000
acctttccca tgatcttatg taatgtctac tgtccaagag cacttcgttt aatattctcc 81060
ataaatgttt ttaggtatgt atggcacatt tcctcctgga gtttaatgct ccattcctgt 81120
atttgtgcat tattcacaca gctgagtcgc cacccaaacc cagagagaaa caggaataca 81180
actcccccaa ctactgttac agagaacgct gcgggcaggt gagggaggac gggctcggga 81240
ggttgttaac gtctgaggcg gcagggttgg cgcagatcgc atgagtgtct gtactcagag 81300
ctctgcttcc cagcctggcc tgggtggttt ttcagtacgt tgtggtggct cgtaactcca 81360
ggaccagcgg cccaaagaga taccctattg ggaaactcac cttcatgcta aagtgcaaaa 81420
caagcaactg aaatgttatt taagaaggaa gaaatgctgt gttagggcca cgtcgcacgc 81480
acctaatcat ttacctttct ctggtaactt tcatttcctc atgtaaccca cccccacttc 81540
tctgtcacct ctcccattga tttccatttt tgtacttcat atttctcctc tttgtttttc 81600
tttgaatttt tctccactgc agtgtattgg acagttcatg tgtgtagttc tacgtatgaa 81660
tgtcctgacg ctgctgttag aaactaccac agactgaaag gtttaaacaa gagaacttta 81720

tcctctctta gtccgggaga ccagaagttt gagatcaaag tgtctcagga ctgtactcca 81780
gggaaggatc ctttctgcct ctcccagttc ctgggggctc cagcatccct gggcttgtgg 81840
ccacatcact gcagtctctg cctccacctt catgtggcct tctcctctat gtctctgtct 81900
cctcttcagt ctcttagaag gaaggccacc tgtcattgga tttagggcca tcctaatcca 81960
ggacgatctc atctcaagat ccttcaatta atcacatctg caaagaccct attgctgaat 82020
aaggtctcat tccaggtctg ggcattagga cgtggacaga tctttctgag ggccacagtt 82080
caatccacta cacgtgtatc cagttccctc tggaggctct aggggaggat actccctgcc 82140
tctcccagct cctgggggct ccaggcatcc ctgggcttgt ggccgcatca ctgctgtctc 82200
tgcctctgtc cccatgtggc cttctcctct gtgtgtgtgc ctcctcttga gtctcttatg 82260
aggatacctg tcattggatt tgggcctacc ctgttccagg atgatctcat ctgaagctcc 82320
ttaattctat ctgtgaaaac cctgtttccc aataaggtcc cattcacagg ttgttggggt 82380
aagatcatgg atgtatcttt ttggggacct ctcattccgt ccactccact gaattttgtt 82440
aaggtcagga agtgatttgg aatctacagg agaattattt ttgctcatgg acatttctga 82500
taatcaatga gggtgtcatt tgaaagctcc gttttataag gtatttccgt tgctagcata 82560
cctgaattag aatatcttaa gtcaaaagaa gctaaatgga aaacatgact ttgagccaat 82620
atgagtatct gtcctgatca atatgagaaa atctcagaag tacaacttgt gccacggaca 82680
tgtaagtgtc tagtatacac ttactggttg atgaaatggt ttggctttgt gtccccaccc 82740
aaatctcatg tttaaatgta atccccagtg ttggaggtgg gacctggtga gaggtggttg 82800
gatcatggga gtggtttctg acagtttagc aacattgccc tagtacagtc tcatgataga 82860
gttctcagga gatctgatgg ttttaaagtg tagtacttcc ccctttgctc gctttcactc 82920
atctgccacc atgtaaatgt gccttgcttc cccttcgcct tccaccatga ttgtaagttt 82980
cctgaggcct ccccagccat gcggaactgt gagtcaactc aatctctttt cttttctttt 83040
ttgttttttg agacagagtc ttgctctgtc gcccaggctg gagtgcagtg gcgtgatctc 83100
ggctcactgc aagctccgcc tcccgggttc acgccattcc gctgcctcag cctcctgagt 83160
ggctgggatt acaggcgccc gccaccacgc ctggctaatt ttttgtattt ttggtagaga 83220
cagggtttca ctgtgttagc caggatggtc tcgatctcct tgacctcgtg atccgcccac 83280
cttggcctcc caaagtgttg ggattacagg catgagccac cgcgcctggc ctaaatcttt 83340
tttctttata aattacccag tctcaggtag ttctttatag cagactgaaa atggactaat 83400
acagttgact tctagatgtt cttggattta tcttggtctt tgcagtttta agtatatata 83460
tttttttagt ttgtttcaca tattttgaac atttctaggt attagtaaag tctgctggtt 83520
tctgcagcag gactgtaatc tttttactac agtcaacaaa gcatagtatg acaatcttgt 83580
ttttacacat gcaccatttt caaggctgta tttgcatgta tttctgtctt tttcctggtc 83640
tccaggggtc aagaaatggt tttcttgggg caaagagctt cggtcagctt tgcagactac 83700
ccagtctcag ttacaagtac agactctgct cctgcacctt aaaaagtagt cacatagtga 83760

gaccgcatcc ctatagaaag tcaaaaaatt agccagacat ggtggcacac acctgtggtc 83820
cccgctacat gggagcctga ggtgggagga tggcctcctg ggggaggtca aggctacagt 83880
gagctatgat tgcaccactg cactccagcc tgggtgacag agtcaaaccc tgtctcaaaa 83940
caaaagaaaa atatgatatc tcaggctctt tcatttggcc tgtacacatt tattgagcac 84000
ctgttgtgtg ccacatactg ttccatgaat gaggaatata aagaaattca gtaatgggtg 84060
tggtggctca catctgtagt cccattactt tggaaggctg aagtgggagg atcacttgag 84120
cccaagcgtt tgagaccagc ctgggcaaca tagtgagacc ttgtctctac caaaaattaa 84180
aaaaaaaaaa aaaacaaaac cctggcgtgg tggtgcagac ctgtagcccc agctgctcag 84240
gaggctgaag taggagcata gcttgagccc tggaggtgaa ggctgagtgc actgagccag 84300
gatcacgctg cagtactcca gcctgggggc aacagagcaa gaccctgtct caaaaaaaaa 84360
aagtagttca ctgacattca cttgcagttt attgaggatg ccttatagtg ccgtgcaagc 84420
aggctcagta ccgggctgga cataatcagg tattgctcgt gctgtagagt gaaggaagcc 84480
tcgagaaagg tgtcctgttc ttggccatag tggcgtattc accactgtaa actttatttt 84540
aacaattatt ttatatttca tttcatttat gtactccatc tctctctgtc acccaggctg 84600
gagtgcagtg gcacgatctc agctcactgc aacctctgcc tctcaggttc aagcaattct 84660
cctggcccag cctcctgagt agctgagatt acaagcaccg gggaccatgc ccagctaatt 84720
tttgtatttt tagtagagac gggtttcacc atgttggcca ggctggtctg gaactcctga 84780
cctaaagtga cctgcctgcc tcagcctctc aaagtgttgg gattgcaggc atgacccaca 84840
gcatccagcc tattttatat tttattttat tttgagacaa ggtctcactc tgtcacccag 84900
gctggagtgt ggtggtgtga tcacagctca ctgcagcctt gacctcctgt gctcaagcga 84960
tcctcccccc tcagcctccc aagtagctgg gaccacacat atgcaccacc acactcagct 85020
aatatatatt tttttaagac tgggttttgc catatttccc aggcttattt tattttttaa 85080
attgacacat aataattgta catattcatg gggtagacag tgatgctttg atacaaagaa 85140
tggagtaatc agatcagggt aattagcata tccatttcaa ttatttacca tttctttgtg 85200
ttaggaacat tcaatatcct ctatgtattt gaaacaatac gacatgttat tgctaattat 85260
agtcacccta caagactata gaacactgga acatattcct cctatctgct tgtaattttg 85320
tatcctttaa caaacctctc cctatctctc acttcttccc ttcccagcgt ctagtatcct 85380
ctgttcttct ttatcctttc aggagatcag ctttttttta gctttcatgt atgagtaagc 85440
acaagtgctg tttaatgttc ttttcctggc ttatttcacc taacataatg tcctccagtt 85500
tcatccatgt tgctgccagt cagaggattg tattcctttt catggctgaa tagtattcta 85560
ctgtgtgtag ataccacctt ttctttacct atctgctcat ctgttgatgg acatctacgc 85620
tgattcccta tcttggctgt tgtgaatggt gctgtggtaa acatgggcgt gccgatgtct 85680
ctccagtatc atgacttcct ttcctttgaa cagataccta gtaatgtgat ggctgggtca 85740
tatgggagtt ctatttctag cttttctgag gaacctccat actcttctct atggtggctc 85800

tactagttta cgttcccacc aacattgcgt aagagttccc tttttggcca ggcacagtgg 85860
ctcacgctgg taatcctagc actttgagag accaagctgg gtggatcact tgagcccagg 85920
acttcaagac cagcctaggc aacatggtga aacccccgct ctacaaaaaa aaatacaaaa 85980
attagctggc atggtggcag atacctgtga tcccagctac ttggaaggct gaggcaggta 86040
gatcgcttga gcctgggagg cagaggttgc agtgagctga gattgcaccg ctgcactcca 86100
gcctgggaga cagagcgaga tcctgtctca aaaaaaaaaa agagttccct tttctccaca 86160
tcctcaccag catttcttat ttttgtgtgt atgtgtcttt tttttttttt ttttttaatt 86220
ttttttgaga cagagtttca ctcttgtttc ccaggctgaa gtgcaatggc acgatctcgg 86280
ctcactgcaa cctccacctt ccgggttcaa gtgattctcc tgcctcagcc tcccaagtag 86340
ctgggattac ggacgcgtgc caccacgcct ggctaatttt tgtattttta gtagagatgg 86400
ggtttcacca tgtcggccag actggtctca aactcctgac gtcaggttat ccacccgcct 86460
cggcctccca aagtgctggg attacagtgt ctttttgata atggccgtct taattgggtt 86520
gagatgattg acacctcatt gaggttttga tttgcatttc cctgatgatt agtgacgttg 86580
agcatttttt ttcatataca tgttggcctt ttccatgtct tcttttgaga aatgtctgtg 86640
aagaccacct gtccattttt taattggagt gcttatcttt tcgctcttga gacatttgag 86700
ttccatgctt ggtcatggtg gtggattcac cccggtaaac atgagagagc attgctgtcc 86760
agtgtttagt gcacagttaa ggaatgggct ggtggcccct caataagatg cccaaaaagg 86820
agttgccttc tgctcagcag acgcctctca aatggtcttt atattctctc caagggagct 86880
tcccctgcat cggactgtgg cacacgatgc cttgcctctt agaaccatct gactgtcacg 86940
tgctttcaga tcgtgacaac ttcagtgtgc ggggaagagc atgtgaaata aattaccttc 87000
tgcctgtgtg aagcagaaat agctatcgtt acaaagcagg gatttttttc catgcttact 87060
ccccactaaa aatacatgtt tgtttttcta acaggtttcc tccgtttttt gatgacaacc 87120
cgtttggcat ttatcagaaa attcttgcag gcaaaataga tttccccaga catttggatt 87180
tccatgtaaa gtaagtaaac cgtttgcctc atcatgaggt gtgtttattt ttaaagtgtc 87240
taaaatcacc gtgaaagcac gcactcagca ggataccata ttgtgttatt aaatgtgctg 87300
cgcataaact attttggaca gttgtctttt ccacaagaaa agaaaaagat tttcagtatt 87360
ctactttaat gatagcttca cattttaatg agtcttggcc gtattgtgtt cttttggtgg 87420
tatgttatat ttaaaatgag ccaatgaacg agtttgtcat cgtattttat aagagagcag 87480
ttttggaatt tgacaagaag gatcctccta gttctgtggc taacagaact gttgatgggt 87540
ttagcagagt tttatccttt ctctcctgct taaattatgc tcaacctcta agtccttttt 87600
aacgtcaaca ttttcagttc tgagcatgtt gcttcaagga tatttacagt tgctggatat 87660
tggcaacttt ggatgcacat tttataaagg tcgtggcagt agggaaaaaa tatattattt 87720
tcttaaatat aagaacgcgg ctgggcgtgg tggctcatgc ctgtaatccc agcactttgg 87780
gaggttgagg tgggtggaac acgaggtcag gagatcgaga ccatcctggg aaacacggtg 87840

aaacctcggc tctactgaaa atataaaaaa ttagctgggt gtggtggtgg gcgcctgtag 87900
tctcagctac ttgggaggct gagacagcgg aatcgcttga acccaggagg cggaggttgc 87960
agtgagctga gatcgcacca ctacattcca gcctgggcga cagagcaaga ttctgtctca 88020
aaaaaaaaag aatgcttttg gttttctggc taccttaccc aatctggtca actggtagcc 88080
atttttactg taataagcac tgatttctgt ttgtttcact gtatttttca gatgagttga 88140
tgggtgcacc agccccctgg gcacaaacta gagctaaatg catctagttg agaattaaac 88200
ttttcttttc catggtctat tttcttatga gccggtggaa gttcagttca gagaagactt 88260
atttcagggc gatggagcag gttgttttga atatgcccct tatggggcca ctggctacgt 88320
tcagttgagt ttcttggtgg tgaacgcacc ccatcctaac cagaggttct tagtctgaac 88380
agaagataag gtcgtgtttt attggcttga gcatgtgttg tgtttgacac actaagaggc 88440
aggctttaaa agactttagc aagaaaacga ggctccgctt aacaatgagt cgcctagaag 88500
ggacaccctg tccagtgaat ggtaagacag ggaggtgtca ggggaaacag cacctcagaa 88560
gccgactgct ctggaaggaa atagaaagga ttatatagaa cctctctctc aggccccaaa 88620
tcccatctga gttgagtgac attatattaa gtggatggca aagcactttt atgatcgagt 88680
ttggccaggt gaggtgctca tgcctgtaat cccagcactt caggagcaat ccgaagtggg 88740
aggattgctt ggggccaatc caatagtttg aggccagcct ggacaatgta gcaagacccc 88800
atgtctacaa aaaatttaaa aattagctgg gcatggtagc aagtgcctgt agtcccagct 88860
actagggagg ctgagatggg aggactgctt gagcccagga agtcgaggct gcagtgacct 88920
atgattgcac cactgcactc cagactggat gaaagagtga aactctgtgt caagaaaaga 88980
aaaaataata aaattaaaag atataattat atatatatat ggggggggtt tccaatgttc 89040
tgtggaaaca acaacaaaaa aaggaattgc agaaatagaa taaaccagaa gcgtaatcaa 89100
agtctggtct cttgccactt gtccttcttt ctctcctagt ttatcttctt tgagagtagg 89160
gaaccagcag cgaccatctt ggtactgtct gatctaacat gcctctcttt atgcagctcc 89220
cagccccttc cagtgttccc cacagtgccc ctgtttcttt acatacccag tgtgggttcc 89280
ccaagagcag aaacagtgat agatagaagt gaactgacag ttcacttctt cccattattt 89340
gttaaatctt atcaaattgg ctgagaagca agacctcaat gactcatatc tttccttttt 89400
ttggtagaga tgggtatggg gatcttgctg cattgcctag gctgcttttg aactcctggg 89460
ctcaactgat cctcctgcct tgtcctcccc aagtgttggg attacaggcg tgaaccactg 89520
cacctggcct taatgaggca tttttcttat ccaaatcact gtccctggtt gtccatgatg 89580
ctagaatttt gattctctgc tttttaaagt catatgttgg ttcctgcaac ttttatcatt 89640
tgaccaggta aacatgtcgc ctgggcaaat ggcacatcag gattgacatt ccttatagaa 89700
ataaatatca tttaaaacaa aacaccaaaa caaagctttg ggacttgttg ccaattcagt 89760
agaggtatta tctgccggtg ttttttacaa cacctgaaag tacctgcatc tgagaggagc 89820
cagctggtgc caggatcgta aataaaatgt agaattttgg ctacagtgca tcaaaagagt 89880

gccggcagat gtgtcgctta tcccggggtc aagggaccca tctacagctg tggacacttc 89940
tgtcgttcct ttatagggct ttgcatcttc aggggcctct tctcatccat actctttact 90000
ttctagactg ctttataaga gtttgctttg gtattttact ttttctttta aagagacggg 90060
tgtcttgctc tgtcaccccg gctggagtgc agtggcacaa tcatagctca ctgcagcctc 90120
gacgtcctgg gctcaaatga cactcccacc tcagcctccc gagtaactgg tgctacaggc 90180
atgcaccacc atgcctggcc aatgttttga tattttgtag aaacgagggt cttactatgt 90240
tgcccatgct ggtttcgaac tcttggtctc acgtggtctt cccacttcag cccactgcat 90300
agctgggatt acaagccact gtcccctgta ttttaaataa atcttcattt tcttacatgc 90360
caaacaccaa aaaaatttaa aaaaaaattc aaggtcatca aacattttgt tttcttacat 90420
gacacacaca cacacacaca cacacacaca cacacacgtt gaaatttatc aggtgatcac 90480
caaaacattc tgaaataaac atagggcttt ttttcccccc tataatttac accactgcct 90540
gccccattag cgtatacact taaagctgat tggactggga ttataagaaa acaaaggttt 90600
atctaaaata taaaagcata aataaacctg tcttataatt gcactccagt gagctttaag 90660
tgtgtgtggt aagggggcag gcagtggtgt aaatcacgtg ggggacgaag ccccacattt 90720
atttgggaat gttttggggg tatagtgttt gataaccttg attttttttt ggcagggggg 90780
agcatgtcaa aataatgaag gagaaactca ctaagtagtg gaaaggtttc taacttttag 90840
gtgattttta tattccccat cctgctcaaa gcttttcagc cttacaggat ggatacaaaa 90900
tcctagcctt aggtgggcgg acagatggtc tctgagtttt actttgtggc agagaagcgg 90960
tgaagtctga caagcatccc atcctaattc agaaagcatg gtggcaggaa actgtaaaat 91020
ttccgatcag acagcactgg cgagattcac aatctagatg gtggagtctg accctaggac 91080
gtcacctggc ttgtcggaga ctccacagtc tgtcacagaa cctgggagtg gctcagtcca 91140
agttcgtgct acttaccttg gggcatagtt tatgctggga aagcctagac gcttacaccg 91200
gggtcatggg tattggattg ttttggggga cttcaatata ttatattatt atatcatatt 91260
atgtatgtta ttatacataa tgtactctgt gtattttatg tgtacacaca gacacacatg 91320
atcatttcta cttccataaa tatgcatctt ttagaactca cctaatctcc tggaagtctt 91380
caacatcatc tccaactacc catcctaatt gaagcagctt gctgtttttt gttgtcgttg 91440
ttgttgtttg tttttgtttt tttttgagac tgactctacc tctgtcaccc agactggagt 91500
gcagtggctc agtctcagct cactgcaacc ttcgcctcct gggttcaagc ggttcttgtg 91560
cctcagcctc ccaagtagct gggaccacag ttgtgtgcca gcatgcccag cgaatttctt 91620
tttctttttt tttgtagaga tggggtttca ccatgttggc caggctggtc tcaaactcct 91680
cacgtcaagt gatccacccg cctcggcctc cccacagtgc tgggattact ggtgtgagcc 91740
agtatgccca acctccccac ctgctgtact tctgacatgg tctgttattt aaaaggaaga 91800
ttataagtga agaagcagac ttaggagact catcctaatg tgtgtgctta tgggaaagcc 91860
agccttttaa aacaggaatc ccatcaataa gcagaccccc aggacagcgc gtgagtgttg 91920

atgttggtga aagcagatgt cttctatttt gcagggacag cctgacaggc atgtttacgt 91980
taagtgtctg gatcctccct gcaggcacat ataaatttca cccttttcta ctcagaggtg 92040
tattttcttt acaaagatgt aatcaacccc cttcccttag cttcagcctt gcacggggca 92100
acccagcctt gcataggtga tttaagagcc ggtgatggta ggaaataagc actagaaagt 92160
gttttgccaa agtggaaatt gaactgcggt cctcatgtca gatagatctt atctctgttg 92220
ccatgctgcg tttgctgatg ttgatatata atctgtgtac tttttttttt tttgtctcct 92280
tccccaacat agagacctca ttaagaaact gctcgtggtt gacagaacaa ggcgattagg 92340
aaacatgaag gtcagtattt gatcccgtgg gtattcagtg gtacccgatg tgcgtgtctg 92400
ccgtctaaca cccacattgg catgtgcagg tataaaccaa ggcttagcgg tcgtatttaa 92460
tcactgccca aatttcaaat ctggtgtttt atttattttt ttaaatttta cttgaaactg 92520
ccaggaaaga tctatcaaat ctcggtttct gggcccacaa cgtcaaactg ggtctacacc 92580
gtatctcccc tcccctcaag caaggttaat agagcaataa caatgcataa gttattactc 92640
attattttgt ttacttattt attttatttt ttaaacagtt ttacggagag tgaattcaca 92700
cgccatgcca tttacccatc taaaatgtgc aattcggtgg cttttagtat acacacgatt 92760
atgtacagcc acccctgtgg ttaatcttag aacatcttca tcagttcaaa aaaagaaacc 92820
ctgcaccctt cagttatcac tgtgctattc tttcatcctc tcttgcccta agccagcggt 92880
ccccgacctt tttggcacca ggggccagtt ttgtggaaga cagtttttcc acagaccgca 92940
caacctaaat cccttagttg cgcagttcac aatagggtgt gtgctcctat gagaatctaa 93000
tgccactgct gatctgacag gaggcggagc tcaggtgcta atgccagcca tggggagcag 93060
ctgtcagtac aggtgaagct tcgctcacct gcctgctgct cacctcctgg tgtgcggccc 93120
attttggttc ctcataggcc gtggaccact accagtttgt ggcccagggg ctggggaccc 93180
ctaccctaag cagccactaa tctactgact ttctctgtag ggttcggtgt tctgggctct 93240
gctaaaaatg gaattgtaca aaatgtaagc ttttgcctct ggcttctttc tctgagcacg 93300
atgtcttcaa ggttcatcca ggctgtagcc cgtgtcagag cttccttcct tttcctgact 93360
gcatagtatt cgattgtgtg ggcagacctc gttttgttta tccattcatc tatggatgga 93420
catctgggct gtttccacct tttggctttt gtgaatggtt ctgccatgga catgagagta 93480
ggtgttttgt gtgcgcaggt gatgatttta aaagttgtgc atcaggtatg atttccacaa 93540
actttttgtg tggaggcaag tatataattg acaaacagac tattccttaa gtagtatcat 93600
acctgatatg cgtgttttga catccctgaa tttgacagaa ttcatggact ttttgttggc 93660
catttctagc tctggacaac ctcagaggga cttatacatg gaggaacttc catatacgtc 93720
ttgaccctgt aaattacagt ctggagagac acagtacacc ggggtgtgag gcttgaacca 93780
cttggctctc ttggggccct ctgtgtggtt tacgagtctc cagtagctgc cataagaact 93840
gaccacaaac taggggctta aacaacagga atttctgctt tccccatcct ggagacgagg 93900
agtctgagat caaggtgtct caggcctcca gaggctctag gggaggatca ttcctgcctc 93960

tcccagcccc tgggggctcc gggcatccct gggcttatgg ccgcatcact ccagtctctg 94020
tctttcttca cgtggccttc tctgtgtctg tgtctcctct tctgtctctc agaaggatgc 94080
ctgtcattgg gtttagggtc atcctaatgc aggattatct cttctcaaga gccttccctt 94140
aattacattt acaaagaccc tatttgcatg tagggttcca ttcccaggta ctgggagtca 94200
ggacatgggc atatattttg agggccactg cccaatgtgt gataattgtg tctgcttcct 94260
gctagaggct ctagtggggg atccttcctg cctctcccag ctcctggggg ctccaggcat 94320
ccctaggctt gtggccacac cactgcagtc tccacctcca ttttcacatg gccttctcta 94380
tgtttgtgtc tcctcctctg tctcttatga ggacacctgt cactggattt agggcccacc 94440
cttctccagg atgatgtcat ctcaagaacc ttaatttagt tacatctgca aagaccctat 94500
ttccaaataa agtgatattc acaggcactg ggggatagga tgtgcacata tctttttgag 94560
ggacattgtt taccctataa cagtagtcaa gcttctgtcc tttacattat ttgcggacag 94620
caacggatac cttcaggtct ccgaggagcc tatccagcca ggttgcacgg tgtggactca 94680
tctgtgccac ggctcttcat aagattggac gagtcctgtt ttaatgccac agccatttag 94740
aaaagagctg ggtctcttgc tgggactgca gggaatcaca gacgacccat atctagaatc 94800
tgctgggaat ggcgtagtga ccatcatagg taccttccaa gggggaagaa caagtttccc 94860
aatcaaagga attggggaaa agctcacaaa ctcccaagct aggtcttaaa gtctagttaa 94920
agcttcctga tatggagata aactgaaaga gactcaagaa aacactgact ttattttatt 94980
ttttttttaa attaacacat gaaaatggta ttatttagca tgtacaacat gaagttttga 95040
aatatgtacc cattgtggaa tgggtccatt gagttcatta acatatgcct tacctcacat 95100
aattaccatt tatttgtggt gagaacactt aagatctaag aaaaaaattg tttttaagag 95160
ttggggtctt gatgtgttgc ccaggctggt ctcgagctcc tgagctcaag tgatccgccc 95220
accttggcct cacaaagtgc taggattaca ggcaagagcc actgcaccca gcaagatcta 95280
ctcttccaga gattcttgag aatacatcat tagttatagt caccgagctg tgcaagccac 95340
tgactgaact tgttcctcct gtctctctca ctttgtctct tttgaccaat gacttcccat 95400
ctcagcccct tcccagcccc tggtatccac cattctactc tctgcttcta tgagttcaac 95460
ttttttagat ttcccatgta agtgaggtca gactgtattt gtctatctgt gcctggctta 95520
tttctctctc tctctctctc tttttttttt ttttttttga gacacagcaa ggcggaggtt 95580
gcagtgagcc aagatggcgc catgccctcc agcctggatg atggagtgaa actctgtcca 95640
ttgccccgcc cccccaaaaa gaacatagag tatagtaaat agataaacca gggacatagt 95700
catttattat cattaccaag tattaggtgc cgtaggtaac gtatgtgtta gactgttaca 95760
cgcctggcag cgtaacagga gggatgcatt gtcctgtggt tacaatggcc atggcgtcac 95820
taggggatag gagtttttaa gatcattata atcttacagg agcaacactg tatatgtcat 95880
ccattgttga ctgaagcatc attttgaggc tcatgactgt agatacacac ccacacacac 95940
acttttttta tttttatttt ttcaagacag gatcttgctc tgtcatccag gctggagtgc 96000

agtggtgcga tcacagctca ctgcagcctt gacctcctgg cgtcaagtga tcctcccacc 96060
tccgcctccc gagtagctgg gactcagtgc atgcaccacc aaggctggct aattttttat 96120
gtttttttgt agagaccagg tcttactttg ttgcccaggt tagtctcaaa ctcctgggtt 96180
caagcagtcc tcctgcctcg gcctcccaaa gtgctggaat ataaacgtga gccactgctc 96240
ccacccccct cacacacact ttatataaca cacacacact agtttggata ctaatgtgaa 96300
tctcggtata tacatatact ttgtactcta catataaagt gtgtaaagta catatgtata 96360
tgtgtatata tatatataat acatgtgtat atattaacgt gtatgtatat aagtatgtgt 96420
atatttaatg tgtatatatt aacgtgtatg tatataagta tgtgtatata taaatatatg 96480
tgtgtatata ttaacgtgta tatgtgtata agtatgtata tattaacgtg tgtatgtcta 96540
taagtatgtg tatatattaa tgtgtgtatg tgtataagta tgtatatatc aatctatgtg 96600
tgtatatatg tatgtgtgtg tgtatatatt actatgtatg catgtgagta tatacagaca 96660
cacacatcct ttagtatcca aattagaatt ttatctgatg gataaaattt attttattct 96720
cttattcaca gcactcttat ttattcttat tcacaacatt gctgaaggga aaactcaaca 96780
gctattgttt gttgcctgac ttagctatag atcaaaaata cctgttttgc aataagaaaa 96840
gaaaaagttg tcaaaacaac tggatgggaa aacaatagaa aaaattcaca ttactgtttc 96900
cttttgaaga tatggttgtg gacgtattcc tctttaagga taagaaattt gaaactacag 96960
taatgctaac tgatagacgc gttaaattca gatgtactgg cttctgtttc atggaaccta 97020
cgtttatttc ttctgctgtc ccacatttaa ttgataacga catcactaca agtataagaa 97080
aaggaagcag agtgaggttt acggatgagc acttgggagg gatttcagag gtcgtgatgg 97140
ccacagtgtg agccagcgat gcctggtgct acccttgagc atgaagcagg ccgacttgaa 97200
cgcaccttgt taattgccag gccgtgcaat gtcgttccta aggtctctta aactggacat 97260
ccttggtttg agttcccagt cttgttattt acttgctggg tcctacggag caaattaaca 97320
tcattccttg aaccctagtt tctatgtctg taaaatactg agtaagtttg ccaagatgaa 97380
aacaggagac aaactgagaa actactggct gtccaatttt atgtcgagag atggaatcgt 97440
atttaccttt ttcgtctttg gtccggaatg caaatgcatt gcacaggagc agaatgactg 97500
gatttgtttc tgcggcaaaa aagggcaaac aaaacactaa cagctttgct tgcaggtttt 97560
cctggcagct caccctccag gcacagttct ccaaaatgca gcaaacttgg tctgtttgtt 97620
tcaatactaa aataatgtag agattctttg gggtattttt ctttcctgcc ctgtcaaaat 97680
tctgtacact gacgacatgt gttgtttctt ttgaagaacg gggcgaatga tgtgaagcat 97740
catcggtggt tccgctccgt ggactgggaa gctgttccgc agagaaaact gaaggtacaa 97800
cacatatcag tgggtgactc agtatgcccg agctcttcca ttagcaggga ctgcctctga 97860
atcctgggac ttctttattg atggctgaca tgtgattaat ttatataata caactatttg 97920
tctaaaactg ttcataagcc aagttcccca ctctttgagt gtcttagtgc taaccatcta 97980
cctagctccc acaaaggcag gagccaccac acccagtcat ataccacatg ttctttgtcc 98040

attcatccct ggatggacac ttatcccaga ttttgaagca gattttagga gacctggcct 98100
gattcctgac tagctctgtg actgctaaac cctactccct cctctttagt ggaaagcggt 98160
caacaggatt acagagataa gcaagcgtct agcaccatgg tcatcgttca gtgatggtgg 98220
tgtattacat ggagaatatt ctttggaacg tttctggcca aactgcctat ttttaaacat 98280
ctggcttttt ggctgggtgc tgtggctcac gcctataatt ccagtacttt gagaggccaa 98340
ctcaggcaga tcactggaac ccacgagttt gagactagcc tgggcaatat ggtgaaatcc 98400
cgcctctaca gaaaatacca aaattagcca ggcgtggtgg catatgcttg tggtaccagc 98460
tacttgggag gctgaggcac aagaattgct tgataccagg aggcggaggt tgcagtgagc 98520
caagattatg ccactgcact ccagcctggg cgacagagcc agaccctgtc tcaaaaaaaa 98580
aaaaaaaaaa aaaaaaatct ggcatttttt tcaatgtata atacttgtcc atttataaaa 98640
tgaggagact gtgctcagag aagttaagca atgtgtggaa ggttacacag ctgctaagca 98700
gtgtaactat cacaggtaca tatttatggg ggtacatgtt atattttgac acacgcatag 98760
aatgtgtaat gatcaactga gggtaaccag ggtatccatc agctcaaaca tttatcattt 98820
ttatgggttg ggaatattgc aaatctagct attctgaaat atacaatata ttgttaactc 98880
tgcccaccca actatgctgt caaacactgg aacttattcc ttttatctaa cattatgttt 98940
atgcaatatt tcttcatctc cccttcatcc cctagctata cacacttccc accctctggt 99000
aactatcatt ctactctcaa ccttcatgac atccattttt ttagctccca catatgagtg 99060
ggaacacgct gtgtttctca ttctgcgtct ggcttagttt attatttatt atttattttt 99120
tgagacagcg tcttgctctg ttgcccaggc tggagtgcag tggcacgatc ttgctcactg 99180
cagcctccac ctcctggctt caagcagttc tcatgcctca gccccctgag tagctgggat 99240
tacagacgcc cagctaattt ttgtattttt actagagacg gggtttcacc atgttggcca 99300
ggctggtctc aatctcctaa cctcaagtga tccaccagcc tcagtctccc aaagaagtgc 99360
tgggattaca ggcatgagcc gctgcacccg gcctggctta gtttacttaa catgatggtc 99420
tcccattcca ttcgtggtgc tataaatgac aggatttcat tccattttat ggccggatag 99480
tttttcattg tgtgtatgga ccacactttc tttatccttt catccattgg tggacactta 99540
ggttgattcc atatcttggc ttttgtgaac actctgaact gcatatgtta ctgatgggag 99600
tggtcaatgg tagtgttttc acctttgcac ttcagagtta aaagaatgga gaatgcttag 99660
aaatgcttat tcctagtcct cagctcagac cagagtttct ggggctggag cctgggatga 99720
tacgcttctt tttttttttt tttttttttt ttttgagatg gagtctcact ttgtcgctca 99780
ggctcggggt gtagtgcagt ggagtgatct caactcactg caacctccac ctcttggatt 99840
caagtgattc ttctgcctca gcctcccaag tagctgggat tacaggtgtg cgccaccaca 99900
tccagctaat ttttttgtgt gtttttagaa gagatggggt ttcactatgt tggccaggct 99960
ggtttcgaac tcatgacctc aaatgatcca ctctcctcgg cctcccaaag tgctgggatt 100020
acaggtgtca gccactgggc ctgggctgat gcatcatttt gttcaaagtt ttaccttttc 100080

aaatacttta ttgtattaaa ttgcattaaa aaaacaattt gaatacgttt tatctatcca 100140
cattggtcaa aaaccaagtc aatattaaaa cagatatatt gccaagtctc cctcttaccc 100200
cctccaagcc acctgtaggt aatcaccttt tattgttttt ctgtgtatcc ttctagagtt 100260
tctttgtgct aataaaaacc cacatggata catgcattct tatttttgcc actcatttat 100320
acaaatctag agtagcgtag tatgtacatt attatagtct tgctttttgc acttgactat 100380
ataatctgga gacctgtcta catacctggc tagcttcctc attttttttt tttttttttt 100440
tttttttttt tttttttgga gacgaagtct tgctccgttg cccaggctgg agttcagtgg 100500
tgcgatctcg gctcactgca acctccgcct cccaggttca agcgattctc ctgcctcagc 100560
ctcccaagta gctgggatta caggcaccca ccactatgcc cggctaattt ttgtcttttc 100620
ggtagagaca gggtttctcc atgggccagg ctgttctcga actcctgacc tcagatgatc 100680
ctcccacctt ggcctcccaa agtgctggga ttacaggcat gagccactgc gcctagccag 100740
cttcctcatt ttaaaaaata ctgcattgta ttccatcaca tgactgtacc atagcttact 100800
taatactata tatatatatt ttgagacagg atctcactca gtcattcagg ctggaatgca 100860
gtggtgcaat catggctcac ttggagtatc gacctcctgg gctcaagcag ttctcccacc 100920
tcagcctact gagtagctga gactgcaggc acacagcacc acacccggct agttgtattt 100980
tttgtagaga caggggactc cctgtgttcc ccaggctggt ctcaaacttc tgaggctcaa 101040
gcaaccctcc cgcctcacct tcttaaagtg ttgggattac aggtgtgagt ccccacacgc 101100
agcctcattc acaatatttt aattcaaaga ttgccgcttt cataaatgaa gggaagacag 101160
ctttagtgtt ttgcaaatca ttttcatttt taggtacagt gacagaaata aagcggtgtg 101220
tgcagcatcc aagtgtcccg tccctttcta accatgcctt tgtgcttcgg ccatgtctca 101280
cagcctccca tcgtgcccaa gatagctggt gacggcgaca cttccaactt cgaaacttac 101340
cctgagaatg actgggacac agccgcgccc gtgccgcaga aggatttaga aatcttcaag 101400
aatttctgag gacaggagct cacatctgga aggtatatct ttatatttag taattcccaa 101460
aaaatgagac tgactcgacc ccacatccag gtgaggctgc gtttactgag tggggcttaa 101520
cctcatgcac acagaggtca gcagtgaagc agagcaaagg ggattgattg caagggtcag 101580
cgaaataaac acagccatgc ctgtgactcc aggtcagcat gtgaccgtca gaggcatcag 101640
catgacagtc cagaatccat gttctgcatc agaacctgca tgtctaaata agaccgctag 101700
gtggtttgtg tgtgcctgaa tatttgagaa gcccagctcc tctggtccct gttccagaaa 101760
ccctgagtga caggcgagct tccttagaat aattaacaag tattttcaaa agtctcttta 101820
ggtctccttt ggttaaaaaa taagaagaag aaatatgccc tcataggaaa tttgctaagc 101880
ttaattgaag atgactggaa aaggattttg agtctattac ttctttgagc ctttgaaggt 101940
ctattattag tttttaaata ataataatat tttttagaat ccctcatgaa atttgctaag 102000
ctttaattta agatgactga aaaagggttt tgactgtgtt atttccttga gcctttgaaa 102060
atctattatt agtttttaag tatggattaa aaagagcttt cagttactcc tcaattctaa 102120

attgctcggt tatatggctt caattagatc tttcttcctg taacacacga gcaagaaaca 102180
taccagtgaa gcaaaatatt gtcagtgatg tgtctattca ttgacatagc ttgtttttcc 102240
cattttgatt tccattcccg taattttcct gtagtcttcc cagttatgtg aagtggaaag 102300
ccaattggct aaccaacacc attcgaaaca gacaccaaat gtcacaattg ctgactgggg 102360
agaggaacca acctgctgaa agacgtggtg aatgagagcc ctccatcctg ggcaggtgtt 102420
tacatggatg taccaagagg aaagctggat acaaattgaa ataatagaca acaaaatgat 102480
agaggaatag gtggctatga cctcgagaag ataacctgtc tccatggcat ggcttcacct 102540
ctcagtgtgt gatgaacgct ttcacagtgg tgactggttt gtattaacat gagagcgtgt 102600
aactttacca acttcataaa cgctcatgaa atccagttgc gggttttaaa aatcataaga 102660
tgttcctgaa tgtgtttact tctttttgtt tctctgctct actctggggc ttacatttta 102720
gctgactcgc ccataacatc ttacaaattg tgtctctcca ggaaatcttt aaaatgatgt 102780
aaagacttgg tttgctttag tatgaaggac ccctagttga taagccacaa aaatagaacc 102840
tttctctaat agagacccct caattgtaaa atttacttaa agaacaaaaa agcttccatt 102900
tgatggattt ttgtcatata atcagtgaac aatttcatta catcctgtcc ccagctgccc 102960
ctactttccc tgaaaaagat tccacacgta ggaataacct ttctccatct ggtatgcatt 103020
gttgagaaag tatgtgtaag taagtattat ttttgctaag tggaatcatt tttaatatag 103080
tttggctagt ggttggggct ctgtttaatg aatttttttt tctttttgag acagagtctc 103140
gctcggttgc ccaggctgga ttgcagtggt gcgatctcag ctcactgcaa cctccgcctc 103200
ctgggttcaa gtgattctcc tgcctcagcc tcccgagtag ctgggattac aggtgcctgc 103260
caccacacca ggctaattgt tttgtatttt tagtagagat gggttttgcc atgttggcca 103320
ggctggtctt gaactcctga actcaggcaa tctgcccacc tcggcctccc aaagtgctgg 103380
gattacaggc atgagccact gtgcctggcc cagatgcaat attttaatcc gtcttagtaa 103440
ttttatcaag taatttgcac accaaacaca tccctggcat tttcagcaac gagcgattag 103500
ttgcaggaat tttatgcagg aagatatttc ttcatgaagc caaatcttct cctgtaaata 103560
agacaccagt tcagatgaat cactgcactt gagggcagct tagaaactat gctgtagccg 103620
ttcatctttg tttgaatcac cctgtatgcc tttctgaatg tggaggagga atttaagaga 103680
actgactcac tatgtctttt attttacttt gtaataaaat attttctgtc ttgctgtaag 103740
gttctctgag gaagcatgtt gaattgggga tcccattttt cttgtgcagt tcatcatcta 103800
ttggtggata catttatcta tttatggata agcgtatgat agaacattct tcaggctgga 103860
tgtggtggct cctacctgta atcccggcac tttgggaggc cgagttggga ggctcacttg 103920
aggtcaggag tttgagatca agcctgggca acatagtgag accccatctc tacaaaaaat 103980
aaaaaagtta gccaggcgta gtgatacata cctattgtca cagctattct agaggctgag 104040
gtgagaggct cgcttgggcc tggaaagttg aggctgcagt gagccgtgat tgtgcctgta 104100
ctccggcctg gatggcagag ttagacctca tctcaaaaat gaaaacaggg ctgggcgagg 104160

tggctcatgc ctgtaatccc agcaccttgg gaggccgagg cgcatggatc atctgaggtc 104220
aggagttcga gaccagcctg gcaaacatgg tgaaatccca tctctaccaa aaatacaaaa 104280
attacccagg cgtggtggcg ggcacctgta accccagcta ctcaggaggc tgaggtagga 104340
gaatcacttg aacatggagg cggaggttgc agtgagctga gatcacacca ctgtactcca 104400
gcctgggcaa caagagcaag actccgtctc aaacaaacaa accaaaaaaa ccccttacct 104460
ttaaaacatt cttcatttcc tttttattgg cctctccttt tagaattcag gcttatggat 104520
ccttgcaaaa agaattacaa agagaggttt tctttgttat cacttaggga aaggaaacct 104580
ttgtattatc attgagatga tgattacaaa attcatccag ttactgagca actcacagaa 104640
tcttttgtcc tatttcatgc tagaaacagg aagattggaa tctgcctgga acaaagaact 104700
gcacctaagc agaccagaag caaaatgtct tcttcacggc ataaggacat ctccactttt 104760
ctctgtacct gtgtgtatag aaatagatta gagcacagtt gaaattcatg gaactggcat 104820
tatttaagca actggaattc cacactgtag gaaggttttg aaaattgttt ggttgtagat 104880
tttatcttat cctttagtgt tgtgttccta ctgtgatgtc ttggtttttg tcatagactt 104940
aagtttataa gtttgaactg gacttgttcg attataacca caaattgtgt gtgtgtgtgt 105000
gtgtgtgtgt gtgtgtgtgt gtgtgtgtgt atgcctgtgt gtatatatag aagtcattat 105060
ggcagatgca cagaaattgt gcagtgatgt aaatgttcat actttacaga gcctataatt 105120
tttatttttc aatttgtttt ttcaaaaatc tcttctcggg gacaacatct gaagggtatg 105180
ttgcatgcat taaaaaaaat catctcacat gcattttata gttttgggga agaaaatatc 105240
atggggaggt ctaccttcag tatctttagt gcttcttacc tggtaacttg agactttaaa 105300
agaagaaaca aagaggggaa gatatgggag cgaatttatt ccaagaatct acaatgacat 105360
tgaagttgtt ggaggaatgt actgtattta aaaaaacctt ctgtgacaca ttcaaaaatt 105420
tcatctgagc tggatgcagt ggcttgttcc tatagtccca gcactttggg aggctgaggt 105480
gggtggattg cttgagccca ggagttggag accagtctgg gaaacgtggt gagacctcat 105540
ctctacaaaa tacaaaaaaa ttagccgggc atggtggcac gtgagtgtag tctcagctac 105600
tcaggaggct gagatgagag gatcacttga gcctggggag gtccaggccg cagtgatccg 105660
agatcacacc actgcattcc agcctgggtg acagagtgag accctgtcca aaaaaaaaaa 105720
aaaaaaaaaa gaaggcaatg gctttgtgct tttgaagatg gattgaagag aacgtccacc 105780
ttaaggcttt aaaagacagt gaagctgggt gcggtggtgc actcctgtaa ccctgggact 105840
ttgggaagct gaggcaggaa gattgagcct aggagttcga gaccgacctg ggcagcatag 105900
cgagacccca tctctatata aaaaaaaaga tgttaaaaca gtgaaattac agaaacagaa 105960
catagccacc ctttttcatc agtgaccttt tcttcacgtg gaggtcaact cagtaggtga 106020
gaataattag taggttcagc agaataaact ctttacaatt aataactcag tggaaaatga 106080
tgttcagatg gtgaaatgtg gaaatgtttt agacaactct attttcagcc tgtgcttctc 106140
actcaccatc ctgttgggat taacagttga gggccatcgc tgcctaaaca tttagcgtgc 106200

ggtttcccat cagttttacc gtgaatgtga aaaggtgaaa ctggtgctga cttcggcagc 106260
aggtatacta aaattggaat gacacagagg agattagcat ggccccctgc gcgaggaaga 106320
tctgcagatt catgcagcat tccatatttt tttaagaaaa ggttacactg tgcaccgaca 106380
tgtggagcta gaaggaaaag cttttaagtc tcatgtttgc tgactataaa aataaatcat 106440
agtaataata ttgaacattc tactttaaaa attattaata aaaccataaa tgaaacactt 106500
gagcacactt taaaaagtac ataaatccat gagtccatat gcctattttt aggttgtctg 106560
ttttattatc caggatttca tactcagatg agaattagca tcaggaaagc ctgaaatgcc 106620
ttaattgtga actgatatgc aggaaaatga aatatgcaag cacattggtg accttgttct 106680
ttacagacgt cgtgatcagc ctccagccat acagaacaaa tgagaaatta gaagcgattc 106740
caagaacgtg tgatggtgtg aaacccactt tggaccatca cacacatttc agcatttgaa 106800
gtgacacaag ctacctggta actgcccatg gcggggtcat agtttaaatg gcccaatgtc 106860
atgtagaggt taatttaatc aaaatgagct ataaacatgg agcaatcact gtataatttt 106920
aaaatagttt acctggccag gcatggtggc tcacgcctgt aatcccagca ctttgggagg 106980
ccgaggcagg tggatcacct gaggtcagga gttcgagacc agcctggcca acatggcaaa 107040
acccccatct ctactaaaat acaaaaatta ggtgtggtgg cgggcacctg taatcctggc 107100
tactcgggag gctgaggcag gagaatcgct tgaacccggg aggcagaggc tgcagtaagc 107160
cgagatcgtg ccactgcact ccagcctggg ccacagagca agactccgtc tcaaaacata 107220
aaaataaaaa aaatacaaat agctgacccg ggtgggcatt ccaaaatgtc cgcatggacg 107280
atcacgaaac aatcacggtg cgaaatgtcc gcatggatga tcacgaaaca atcacggtgc 107340
agaatgtccg tatggatgat catgacaaac aatcactgcg caaaatgtgg aggaaaattc 107400
attaccataa tcacaacatt tgtatcagaa aaacattaca aatttacttt gcttcatgta 107460
attttacgag ttggcaatat tttcttaaat tggaatactc taagagaata ggacatggat 107520
atgttacaag aagaagtgta ttggggcaag tcttgttttt ctgaagggaa atgccaccgg 107580
cctgctttca taggcattga taaagccagg aaggaaataa agtcacaacc aaatagtata 107640
cactgtctct actgaattct tctaattcct tcgcaacaga agtctaaatc ctgcttagcc 107700
taaacatatc tatagccagc gatgggcaaa ttatatttgc tgtcagcatg atagaataag 107760
aagcagaaac cccccagcag tggggtaaga ttgcgcgctt catcccctca aaatgcagtt 107820
cagccccact ccctctccat gcccatcaaa caactgcaca atcacttgtt actgaatttc 107880
ccattcagcg ttttgtttct ttctgtccat acttaaagac caaatcattt gtttgctttg 107940
aagaaaaagc aaaaacaaga acaaaaacac caaatcattt gaaatcactg tccattaaaa 108000
aaaatcacca gtggaactaa acagttgagt ggtttgggat gtgttcgcca tttctgtgtt 108060
tgacctacat ggcctctaat tctttggacc attgtaactg tgaaactatg gcttaatact 108120
gttaaagacc ccaaggctcc actctgtagc caggtgcacc tcaggctgca caaatacccc 108180
accagaatgt cacaatcatt cattgctcca gctactgagt gaacagggtg caagacagca 108240

ctggcttggg ttgtattttt gcagccagtt aactttgcat caggtctaca cagtccttgc 108300

agcctttgat ttctccagtt gtagagattt gaaatggggt gcccgggttc ctctcgtttt 108360

tccttcaatc tacctagcat gcattttaca cacattggag gagtcgtatg cactgttttt 108420

ctgttgctga tggaggcaag caggccttct aattagtcta acttaaacgg atgggattca 108480

cttaacaaaa gcaagaagaa aagcacacga acaggtgtct taatgcattt ggatagtttc 108540

tttgcggggt gtgtgtgtgt gtgtgtgtgt gtgtgtgtgt gtgtgtgtgt gtgtgtcttg 108600

gtgaaggtga ggggctgtat tttcaccaag ccttttcata atcaagtttg ctttgcaaac 108660

cttatggcct tgcaccccgc agaacagtcc cttcctaata gcagggatgc tctgtcatgg 108720

ttttctgtaa attatcgtgt gttgggaagt tctgttgagg cttagtttga tcttccatgg 108780

tggacacgtc tgttctgtat gtaaaaggca ttacaattgt gttttagcag atgagacttg 108840

aagcctttcc acagtccttg tgctctgaga tggctgttga gctctgctca actgtcgtag 108900

ctgaattctt tctttgcgct gaacactggg cagcctcacc atgttgccaa cgtgcttctg 108960

gggcccctgt cactgccgcc ggatgccgcg cacacaggca gagtgccttg gcaggtttgt 109020

gcaccccttg gcgagccaga actgggaacc ccgcggggtg accccttctc atggggggtc 109080

ggggagggga gtgcctattt ttaatcctgt ctgtttgttg cacaatggaa atcactgtga 109140

tttgtacata tgccctagga aaattttact gctgtctaat ttatgtaata atactgttga 109200

ttccaggttt gtttaataaa actttgtatc ttttcagaa 109239

<210> 5
<211> 176458
<212> DNA
<213> Homo sapiens

<400> 5
agaataacgt cgggtcgggt cagcgggctc tgcagtagtc gccgcagcgg cgatgggagc 60

ggtggggacg aggcggcggc ggcggcagga gggggagcag gtgctggcac aagagcagcg 120

gcttggggga gccggcagca gcagtaacag cagcagcagc cgccgccgcc gccgccagta 180

aacgcggacg gtaccccagg ggactaccca gccggccggc cctggaagcc gcgctcgggt 240

cccgccgcag tcggcggtgg gggatgggca ggcagtggcg gtcccgcctg ccgagggtta 300

acccccgccg gtcccggtcc tgagctggac cagagccctc ctccagaaac ccctgcgtcc 360

gccacggccc aggtaggaca caccccccga ccccccaggg cgccgccctc ccccagtctc 420

gaccccctcc tccggccgca cccctcgccg agtgaccgag cctgctgagg aggggagggg 480

taccggggac tttcagttcg ctgcaagccc ggggctcgtg ccagaccccg tcactctcca 540

tctcggagat cgttctggtt ctgcccgatt tccccgaagg ctgccgcctc gttttgcccc 600

aggctttggg tgtcttcact gaacttggag ggtcacgaag cttcgttctg cgtctctctc 660

tgtgggtgac ccacatatca cttgtaccca tgccacttct ggtaccagca ccgaacacga 720

aggattccta ggaaatctga gcgcttattt cccaccccgc ctccccaact atcatggaaa 780

ggaacatggg actactgaat ctgtttgctg tgttgtaacg acagtgaagg ccgcgacttt 840

cttgtactta gggtgctgaa gtaatgatct gagggatttg ctcgtcattt gctgtggcag 900
agatgatgtt gacaccctgg gatgctgtgg gagggacaaa agactcacgt atatgttctg 960
tcaaatcgga gaagtaaata atccattggg atgccaggat actttattct acagcgaagt 1020
taattttgta tccgtcgagg ctacagtaga ggtaaaaatg acattgagtt gtactttgct 1080
ttgaattaaa gttgatgggt agcagcaatc ctggtattaa ctcttctctc tcatcgcttt 1140
agaaattatt ctttctttga acattaaaga ggacagtttt attatacact tctaataatg 1200
tgaagatgtt ttgtaattca gtagaaattg agtttctgtg tgtgtaggag ggtcatgtta 1260
tgaatgggca catgtagaca cacacgcaga gggaggatcc actcagtgtg ttttgttaac 1320
aatgtttggt tctgagccat tgataatagt tgcattggta tctaaaccct gatttgccac 1380
agtataacac aaagatgtgt tggctgtgta cctttggcta aatggaatag aataaagttc 1440
atttggggat agaaggggga aatgatttta acttatcaag ggaattgaat gctatgtatt 1500
gcttgcttta tttccttttt ttttttaaat aaagaagcag taatgatgac aaatatagct 1560
taatatgatt aatcttgagg catttgtctg aagtcaaaaa atgtttcaaa taatgtgtgt 1620
gttatattgt gatctatgca aattgaaaac attgaaggaa atctttgatt tagaagctta 1680
tgttttcctg tatgataaca ttttatcaag acaccgaagg ggacatatga agcatggttt 1740
taaaggaaag tttctcttaa cattttcttt ttatgataga atgttaggaa atgctttctt 1800
ccctcttcca gttttagaca cagaagaaat taaagtccat aacttcatta atttaatata 1860
taatatcaag ttgatgcaaa tttgtacatt ggttaggggg ctgagaataa tatatcttgc 1920
tcattatctt gtactatggt taatacataa tttagtaaat tctctattgc attatatatg 1980
actttagatt gttgtatctt cttttgattt taagtgttga ttattttaat acttcaggaa 2040
atcttcaaga ggttagacct catttgcttt agcaaattga actcaattat gtggggcttc 2100
aagtatcttt aaatgtaata ctagaaggga agtttgcata atttagacca ttttcgagcg 2160
tattttactt ctaaaaagtg cactaaaaat gtggcaatga taagttattg gtttttcaaa 2220
gtaaatgaat attacagatc cgagttagct gtgatcatgg aatttaaatt tcactttggt 2280
ggggggcagc cactttttta tatgctttat aactttaaaa agtaacatat ccagatgact 2340
gcttgattgc aactcatgag agactctgag ccagaaccac ctacctaaga cactttcaga 2400
ttcgaatcct gagaaactgt gtgagataat aaatgtttgc tgctttaaaa aaaaaaacct 2460
catgcctgta atcccagcac tttgggaggc caaggcagga ggatcgcttg agcccaggag 2520
tttgagacca gcctaagtaa cacagggaga tcctgtctct acaaaaaaca aacaaaaatt 2580
tagccgggca tggtggctca cacctgtggt tccagctact agggagactg agggggtgga 2640
tcggaggttg aggctgctgt gatctgtgat cgctccactg cactccaggc tgggtgacag 2700
agcaagaccc catctcttga aataaataaa taaataaata aatacaaatc tatgacaagg 2760
gaaagtgttt aggatcatta ttgatctagt aaggactgtg ttgatcagta aaataaaaag 2820
tctttgagaa ttatggaaaa tatagctata gcttacttct ttccacactt aaaaaaaaat 2880

cctataggaa atattctttt acaaatatat agcaaacagt gttttcaatt tgagggtatg 2940
ctactcaaaa ttactctagt catgtattca ttttttgttc ttattgaggt ttttgagaga 3000
cactcaaaat gtagctggag ctatttctgt ttgattagta atattgcaat ataccttcaa 3060
tatttatgga tttttgaaat taaatacatt ttatttagtt ttatttccta gtcttttttt 3120
tttttgtcat ccagcatatt gtaaacaatg tgtggtagtg gaagtttttt ccccaggctt 3180
cagtggatag aaccttttat gccgaaagta ctgaaagctt tcaagttaaa tatcgtacat 3240
atttggtgaa agagccttta aggtcatatg aaaaaattca gttttttaga actaaatttt 3300
gagcataagg acaacacata ttactactca aaactgcctc attaaagtct ggtgatcctt 3360
gattataggt ttataataca gattgaaatt atattataac ataatatgaa tgtcaatttt 3420
gctagatagt ttcttctttt cagttggtta tttttaaata gccttcttag tatattttca 3480
ttaatgtcat gtgactttat tcttgatgct taagtgaaca tatagtactt acaaactttt 3540
gtgaacattt tggatttttt ttcctgtgat ttttttttta acctttgtct ctataatcct 3600
tggtgttttg gagtgtttat agctgttttt tattgtatat aggaatttgc tttcacttca 3660
gttagggtaa agcttcccaa tgtgaaaaac ttaagtgtcc tttgaaaaga atctgggatg 3720
taatagtgcc tcctaaagag tggaagttac taagtagctt cttagcttcc tttggtaagc 3780
tatcgagatt taaccatgtt ttcagaatca ttattttttt aaacagagaa ttaacatctt 3840
tattgaaaac aagaatcagc attttaaaat gtttgaatta gaaaacatat ttgccattat 3900
atatgttttt accaaaatgg ttttataatt aatattttta tatttgtctt tttctttctg 3960
cagtttcaaa gtagttgcct ttaaaaaaat ggagttgcag tttccatcat cttgttctag 4020
ccatatgatc attgaaagac tatattttgt tcatattttt ccaacattga attttccacg 4080
ttatatatta tagatgaagt ttgtaaagtc tttttaaatt tcttgatcct ccaagtgctt 4140
cagtttcaaa aatatctaaa agttttatca ccatttttca gccgggtgtg gtggctcaca 4200
cgtgtaatcc tagcactttg ggaggccaag gtaggtaatt gaatcacttg aggtcaggag 4260
ttggagacaa gtctggccaa cgtggcgaaa ccgcatccca actaaaaata gaaaaattag 4320
caggatgtgg tggcatgcac ctgtaatctc agctacttgg gaggctgagg caggagaatt 4380
gcttgaaccc ggaggcggtg gttgcagtga gctaagattg tgccactgca ctccagcctg 4440
ggtgacagag tgaaactctg tatcaaaaac aaaaacaaaa aacatttttc aatgaccaat 4500
aaggcgaaga tgaggggagg aaaataggaa aaaatgaata accaaagaaa aaaatgtgag 4560
ccttgcctct catggaaatt tgatttaaga aaaatcatct ggaggctaaa ttatatggta 4620
gataataaac tagattagga cttcatttct gctggacttc cctagcactt aaaaaattca 4680
acatatccaa gataatagtc agttctcttt ttaacttcct taattctata aataaaggca 4740
ttatcattta gtcatacagg atcaaaacac acattctctc tctctctttt ctttttcttt 4800
ttttcttttt gctccctatt tctcctggat ctcacccaca caaaacctct gctttctttc 4860
tttttttgga gacagggtct tgctctgttg cccaggctgg agtgccatga tcgtagctca 4920

ctgcagcctt gacctcctgg gatcaggcag tcctcccccc ttagcctcct gggtagctgg 4980
gactacgggc atgtgccacc acacttggct aattttacaa ttttttgtat aggtggggtc 5040
ccactatgtt gcccaggctg gtcttgaact cctgaattca agcaatcctc ccacctcagc 5100
ctcccaaagt gcaggaatta taggcatgag gcacaatgcc tagccctctg cattttattt 5160
ccattgctac caccttactt tggtctctgt gaaactttaa actactgtag tcatccccat 5220
ctaaggggca tgcattccaa gacccccagt ggatgcctga aacctcagat agtaccttac 5280
cctatagata attatgcttt tcctatacat acatggctgt gataaagttt ataaattaag 5340
catagtaagg gaataataac aatagctaat aaaatagaac agttatcgta atatgccagc 5400
atcactactc ttgtgtttta gggccattat taagtaaaat aagagttagt tacttgaaca 5460
caagcactgc tatactgaga cagtgatcta acaaccaaga tggctactaa gtgacaaaca 5520
gcacagacgg tgcagataca tgctggacaa agggatggtt cacgtcctag gtgcaacaga 5580
gctgaacagc ttgagatttc atcaccatac tcaaaacaat atacaattta aaacgtatga 5640
attgtttatt tctggaattc tccatttaat atttttggac tgtggttgac cacgggtaac 5700
tgaaactgca gaaagtgaaa ccatggataa gggggattac tgtgtaacag tagcctcatt 5760
ccttgattaa atacttgcta agcatctacc atgttactgt gctaggggtt ggagatttaa 5820
cagccaacaa gacaaataat ctactcactt ttggagctta caaatagtgg aagcagagat 5880
ttgcaggaga aggaattaca agtgttccag gtattatgaa gggaagatgc agacatgagc 5940
atttaacaga cctaacagtc aagaggtggg ttcagggaag aagtttctgt ttcatcagcc 6000
acagttcatt ctacatactt caccagagtc agaggctagt ggatatatta tccctataat 6060
tagttaaata attaaataaa taatgacaaa ataaatgttt ttaaatttaa aagactattt 6120
gaaattaaat gtccacacaa cccatgaaac agctttagac aagttctttc agaacacaag 6180
aacacttgaa agagttttgt cctaaagagt ccccactctc cctccaatat gggtttctac 6240
tttggattct cccaagcctc aattacttga atactgacag agttcctatg ttatggtttt 6300
acactctctg tttgaaccat gagaacttag gactgtgtta ccagaagcca gagtacaagc 6360
catggttaca agaaagaggt gttccaagta gatggctata gtgtctctgt agctagggtc 6420
cagcagctta tcggtgattc attaaatgta caagtgatta ggatataata tattaggata 6480
tattatatta tactgtggct ggccagtttt gctgtttttg aaaaaggaag ttaaggccag 6540
gcgtggtggc tcatgcctat aatcctagca ctttgggagg ctgaggcggg cagatcacga 6600
ggtcatgagt ttgagactag cctggccaat atggtgaaac cctgtctcta ctaaaaaata 6660
taaaaattag ccgggtatgg tggcacgcac ctgtagtccc agctacttgg gaggctgagg 6720
caggagaatc gcttgaatcc gggaggtgga ggttgcagtg agtcgagaca gcaccattgc 6780
actccagcct gggtgacaga gcaaaactcc atctcaaaaa aaaaaaaagg aagttaatat 6840
acttaaaatg ttattgcagt gaagatacag aaaaggttct gtattgattt ttctttgtgt 6900
tagtgggaag atcatagaca taatgctttg ctcggttgat ttggttatag tagattttac 6960

ccgaggaatg attttgttta cccaaggaat gtgtagtgtt ttccttaaca atctggtcat 7020
gtaagttata ctgtacttcc tataagggaa gaatttgttt atgtgttgct agtatggtag 7080
ccagggagag gaaaactagg ttgtttacac ttttgttgtc agtaattgta accatactga 7140
cctgtaaagc ttctctttac tttttttttt ttgagacgga gtctcgctct gtcgcccagg 7200
ctggagtgca gtggcgtgat ctcggctcac tgcaagctct gcctcccggg ttcacgccat 7260
tctcctgcct cagcctcctg agtagctggg actacaggcg cccaccacca cgcctggcta 7320
atttttgtat ttttagtaga gacggggttt caccgtgtta atcaggatgg tctcgatctc 7380
ctgaccccgt gatccgcccg cctcggccta ccaaagtgct gggattacag atgtgagcca 7440
ccacgcccag cctctctcaa tagatatttt acttagattt attgagctca gagagatttc 7500
aggttgccca aagtataatt tacctcccct gaaagttgat aaagaacagt tgaagtgtga 7560
gtggtgaata aacttacctt ctcttctttt gccatcagca aatggggttc tttacaaatc 7620
ttcatttctt tttactaact cttcagagga gctctttttt tttttttttt tttttttgga 7680
gacggagtct cgctctgtcg cccaggtcgg actgaggact gcagtggcgc aatctcggct 7740
cactgcaagc tccgcttccc gggttcacgc cattctcctg cctcagcctc ccgagtagct 7800
gggactacag gcgcccgcca ccgcgcccgg ctaatttttt gtatttttag tagagacggg 7860
gtttcacctt gttagccagg atggtctcga tctcctgacc tcatgatcca cctgcctcgg 7920
cctcccaaag tgctgggatt acaggcgtga gccaccgcgc ccggcctatg ttgcttatta 7980
gtatattaga tgccttgaaa aatttagctg gtgtgtactc tttgtaaact tgtttctaca 8040
catgatcttg ttttatttta aaatgtaaca atttagatac tttttttttt gagatggagt 8100
tttgctcttg tcacccaggc tggagtgcaa tggcgcgatc ttgcaatctc agctcactgc 8160
aacctccgcc tcccgggttc aagcaattct cctgcctcag cctcccgagt agctggaact 8220
acaggcaccc gccaccaggc ccagctaatt tttttgcatt tttagtagag atggggtttc 8280
atcatgttgg ccagactggt cttgaactcc tgacctcagg tgatccaccc accttggggc 8340
ctcccaaagt gctgggatta catgtgtgag ccatcaggcc tggccaaaga gtagaatctt 8400
atataaacta tatgacagtt ataaaactgc tatttggtga attatctagt ttccctccta 8460
aatgtaggag tttattactt tagcctgaga gataatagtg atggtaatag tcttctaaat 8520
ggactctttt tcctcttaca ttatatcatc ctccagctcc tgaaggggtg ccttcaggcc 8580
caccctgtgc caccttgccg actgaaacag aatgatgtct gtcctcttcc tagcctggtt 8640
tttgcagttt gaactcagtt tacctttcag gcctatgtat tctttctccc aataagcacc 8700
cttttcacgt agaccaagct tgtgcaacca gcggcccatg ggccgcacgt ggcccaggac 8760
ggctttgaat gtggcccaac acaaatttgt aaactttctt aagactttat gagcttttgt 8820
agttgtgtgt gtgtgtgtgt gtgtgtgtat ttaactcatc agctatcgtt agtgttagta 8880
tatttcatgt gtggtctaag acagttcttc cactgtggcc tggagaagcc aaaagattgg 8940
acacccctga tgtagattaa tcttatcatt tattttccca tgcacttgta tgttatgctt 9000

atttcctctt ctgtgccttt gcatacctcc ttagttaact tttgccttga aacaaagcac 9060
ctcaaaattt agaggctaaa acaataatta tttattagtt aactgttctg tgggttggca 9120
gtttaggctc agctgaatag tacttgtttt ggtcttgttt gttctcattt atatgaggca 9180
gttggctagc ttgagtttct tctcatgagt ctgggctcca aaagtagcaa aagagggcaa 9240
gtccagtgta taagtacttt tcagggttct gggtcatgtt tgctaatgtc ccattggcca 9300
aaacaaatca catggccaac cccagtttca gagaaggaga aaggaaccaa aggaatgttc 9360
tacctctttt gttagagaca aggtcttgct atgttgccca ggctggattt aaactactgg 9420
gctcaagcaa tcttcctgcc tcagcctctc tagtagctgg gattctaggc atgtgccaca 9480
gtgcccagca cagtcctacc tctcgatggg atatgtatca aggacacatt gcaaagtatc 9540
aaggacacat tgcaaaggaa tgtatgtacg ggaatgggag aaatgtgtgg ctattttgca 9600
atcgaccaga tgtcttctac aaatcatatc caccttttgt aagaaacttt gtatgacaat 9660
cctagctagt attaacctcc ccgttcaatt ttttgtatgc atttggccct tacaaattga 9720
tgtaggttaa ctttttctat tttttcaccg tttttttttt tttttgagac ggagtttcac 9780
tcttgtcacc gaggctggag tgcaatggca caatctcagc tcactgcatc ctccgcctcc 9840
tgggttcaag caattctcct gtttcagcct cccaagtagc tgggattaca ggcacctgcc 9900
accatgcctg gctaattttt atatttttgg tagagacagg gtttcaccat gttggccagg 9960
ctggtctcga actcctcacc tcgtgatctg cctgtctcgg cctcccaaag tgctgggatt 10020
acaggcatga gccactgcgc caggccccta tttttttacc ttttacatcc agatagttac 10080
tgttaatttc tgtttttgta ttatgtgttc tcattcaaag actattttgg aaataattat 10140
tttttgaaat aataactttt cttctcctga ttattaactc attgagggca aagctatgca 10200
ttatgcttct tttttctctt ccctgtattc gtgacaattt attgaatgag catatgttaa 10260
attttcaaat caactgtcca gttatttgcc agaggactat ttagtttcat tcagctgtat 10320
gggaggtgaa agatatttca tttagtgaaa ggttcaatgg taggtattag gctagatgct 10380
agatgagtag aagtgaatga gtagatgctc ttgacctcaa gaaatgtgta ctctattagg 10440
aggacgtaaa ttgtgtgcgt gcttctaagg ttgtttcttt actgcaaacc tatatttctg 10500
cttcatgctc aactcacagg ttagctggct tttttttttt tattgtgtag atggggcact 10560
cttctactct ctctcacact gctagattat tgttgcctaa tctattctat gtctggcttt 10620
ctctgtttct ggtttatata actattatct gttactgttc tttttataag atatattaat 10680
catggtgtat ataaaacata catgtgcagt ttaaggaata attataaagc cagtgttcaa 10740
taaataaaat gttgtcagca tctcagaatc ccattcctga tctcacctgc cttttttgtt 10800
ttgttttgta ttgttttgtt gagatgggat ctccctgtgt tgtttaggct ggtctcaaac 10860
ttctgggctc aagtgatcct cagcctccct agtagctgtg attactacag gtgtaagctg 10920
cctggctttc acctgctttc tttaccccaa aggtagccat tcttatgata tttgtgataa 10980
taatattttt gcttttcttt atagacttac tatctatgtt tgcatctcca tttaacgtag 11040

tttagttttg cttatttttg gactttatgt aaaagggctc atattgtgta tattattttg 11100
tcttgcttaa tttttttcag tgtagtttgt gagattcatt catgtagttc agtatagcaa 11160
tattttcttc attttctttt tagcgtagtt ttgcggtaag tgcaataatg gccccccaaa 11220
atgttcatgt cataatcccc agcacatgaa tatgttaact tatgtggcag aagagacttt 11280
gcatatatga ttaaattaag gctcttgcga gaggaggatt ttccttgatt atctgggtgg 11340
gtctgatgta atcacaaggg ggtgctcata agagggaggc atatactttg tcagagagag 11400
ataaggagat gtgacaatgg aagcaaaagt cagagagaga tttgaagatg ctatactact 11460
gactttgaag gtgggggatg aggccataaa ccaaataatt caggcaagct gggcctggtg 11520
gtgcacttgt attcccagct atttggaagg cgaggcagga ggatcgctta ggcgcaggac 11580
tttgagactg tagtgtgtaa gaattgagcc tgtgaatagc tagctactgc gctccagcct 11640
ggagaacata gtgagaccct gtctcttaaa aaacaaaaca aagcaaagca acaacaacaa 11700
caaaacagaa ttcaggtggg ttctagaagc tagaaaaggt aaggaaataa attctcccta 11760
gagctttagc ccagtagaag cacagacttc tgacctctag agctattaga ttataaattt 11820
gtgttgtttt ttgccactaa atttgtcata tttgttacag caacagtaag aagctgctat 11880
atgaatatat atacaggacg aatatactat aatttatcca ttcttctctt gctggacatt 11940
tgggttgttt gtagttttta ccatttatgg acaatgtgac tatgaacatt cttgtacctg 12000
tttgctggtg tacatgggga aaaagtattt ctagggtgta tacctagtaa aggagttgct 12060
gagtcacagg gtatgcatta tcttcagctt tcttttataa tacagtacaa taccattttc 12120
aaagtacatt cccttggcat gtacactgga cgattagcaa gcaacaacaa gcaacaacaa 12180
agtatattcc tactagaggt gtacaaaagt cctcactttt taatttaaat actttcttct 12240
ttgatattaa ctatgataat gcttccttta tatgggtgag tactcaaaat ttgcttcaag 12300
ttaaaacata tttgagaact aatctatact ttttgttttg agacagggtc ttgttctgtt 12360
acccagactg gaatgcagtg gcacaatcat ggctcactgc agctttgaac tcctaggctc 12420
aagcaatact ccggcttcag ccttctgaat agctaggact atagatgcac accaccacac 12480
ctgactaatt tttaaaattt ttacggacgg catcttatgt tacctaggct ggtcttgaac 12540
tcctggcctc aggcagtctt cccacctcaa cttcccaaag ttctgggatt acaggtatga 12600
gccaccttgc ctctcctaat ctgtactttc gccagtctaa aaatagtagg tggcattggt 12660
ttatttttat tccctttttg atcaactaat tctattaact ttattttaaa agctttgttt 12720
ttttgagaca gggtctcact ttgtcatcta ggctggagtg cagtggcatg attttggctc 12780
accacaactt ctgcctcttg ggctcaagga atcttcccac ctcagcctcc tgagtagctg 12840
ggaccacagg cacatgccac catgccaggc taattattat tattattatt tttttttttt 12900
ttgtagagat ggggttttgc catgttgccc aggatggtct catactcctg ggctcaagtc 12960
catctgcctt ggcctctgaa agtgctgaat ttacagttgt gagccaccat gcccagccaa 13020
aaaaattttt taattgaggt aaaacgtgca aataagattt accatcttta ccattttata 13080

tgtacagttt agtaacaata aatacattta tattcttttt ttcccatttc ttcccccttc 13140
ccctcccctt ttcagcctct ggtaaccacc aatctactct ctatcttcat gagattactc 13200
ttttagctct ggcatatgag tgaaaacata caacatttat ttttctgtac ttggcttatt 13260
tcacttaatg taatggcctt catttccatc catgttactg caaataacag catttcattc 13320
ttttgaatgt gtatacacag tgtataccac attttcttta tccattcatc tggatagaca 13380
cttaggttga ttccattttg gctgttgtta atagtgctgc aataaacatg ggagtgcaga 13440
tatctctttg ctatattgat ttcctttctt ttggatatat acccaatagt ggaattgctg 13500
gataatatgg aagttctatt tttagttttt ttgaggaacc tctatagcgc ctccatagtg 13560
gctgtactaa tttccattcc taccagcagt gtatgagcgt ttccctttct ccatatcctt 13620
gccagcattt gttattgcct ttttcatata agccatttta actggggtga gatgatatca 13680
tattaatctc tattttgtga ggttttgatt tacatttctc tgattagtga tattaagcat 13740
tttttcatat atctgttggc catttgtatt tctttttttt tttcttttaa tgagacaggg 13800
tctcattgtg ttgctcaggc tggcctcaaa ctcctgggct caagtgatcc ttctgctttg 13860
gcctcccaga ttgctgagat tataggcatg agccactgct cttggcccga atgtcttttt 13920
tttttgagaa atgtccattc agatcttttg cccatttaaa aatcagatta tttaattttt 13980
tttgctattg agttgtttgc actccttgta tattctggtt aactccttgt cagatggata 14040
gtttgcaaat attttctccc attctgtggg ttgtctactt cgttgattgt ttcctttgct 14100
gtgcggaagc tttttagttc gatataatcc caattatcta tttttgcctt ggttgcctgt 14160
gcttttgaga ttttacacaa gaaatctttg cctagaccag tgtactggtg tgtttcccca 14220
gtgttttctt ctagtagttt cagagtttca ggtcttagat tcaagaagtc tttaatccat 14280
tttgatttga tttttgtata tggtgaaaga taggggtttt tctattacta ataactttaa 14340
aagcaaaatt ataacaatgc aaatggtata atttaatttg taatcatatt agtatggtaa 14400
ttttcttttg ctgacaaaaa tatttttata tgaatttgat aagtagtatt tgtataatag 14460
gtttcaattc tgggcctccg tgcaaatatg gtaattttct ttaatattca agtggtacag 14520
gaactgtttt ggacagtatt atgtctgtga atgtgactgt ttctcttctg ttgtttagga 14580
aacatgcaga attacataag taatgactgc tttttttttt tgagatggag tttcgctctt 14640
gttgcctagg ctgaagtgca atggtgtgat cctggctcac cacaacctcc acctcccagg 14700
ttcaagtgat tctcctgcct cagcctcccg aatagctggg attacaggca tgcactacca 14760
cgcccagcta attttgtatt tttagtagag acagggtttc tccatgttgg tctggctggt 14820
ctcgaactcc cgacctcagg tgatcggcct gtgttggcct cccaaagtgc tgggattaca 14880
ggtgtgagcc accatgcccg gccagtttta ttgagcacag tcagtatact aggcactgtg 14940
atgaataaac actttacata tatcttagca cttttaatgc ttagtgattg tcattaccct 15000
tttttttaat agatgaggaa attgaggtac agagaagtta attaatgggc ccaatagcca 15060
gaatttgaat ctaggtacag tggtctggtc ttaatctcta ttttgtgagg agttcctgga 15120

gttagaagac aataaacttt agtgttatgt taatatctca gataatactc ttttagggag 15180
ttcttttcat taagtcttgt ttggtctggc ttatgtttct tgaagttcct atacaattta 15240
aaagatattt cctggctggg catggtggct catgcctgta accccagcac tttgggaagc 15300
cgagttggga agatctcttg aattcaggag tttgagacca gcctgggcaa tatggcaaaa 15360
cctgtatctc caaaaattta gctaggtgtg tgcctgtggt cccagctacc tgggaggctg 15420
agacggggag atcatctgag cccaggaggt caaggctgca atgagctggg attatgccac 15480
agtattccag cttgggtgac agagtgaggc cctgtctcga aacaaaacaa aaaacttccc 15540
tttgtgcctc ttatcattct tatttttgtt ctactttatc ttttttactc tatttgtata 15600
tgtgaatcta tatgacttaa attatataca gtttggggcc aagtgtggtg gcacaggcct 15660
gtaatcccag cactttagga ggccaagatg ggcggatagc acttgagtcc aggagttcta 15720
gaccagcctg gccaacatgg cgaaatcctg tgtctacaaa aaatacaaaa attaggcggg 15780
catggtggca tgtttctgta gtcccagcta cttgggaagc tgaggtggga ggatcacttg 15840
agcccagaag gctgaggttg cagtgagccg agattgtacc actgccctac agcttgggcg 15900
acagagtaag accctgcgtc aaaaaaaaaa aaaaattata tacagtttga tgtctcattt 15960
tgagcccttt aattaacttc gccaaatatc tattgattaa ctgcaaagct caatacttgg 16020
ttatggcaga ggataatcat tatgatatgg gtcttcatct tcaaggagtt tataatctag 16080
gtgattgtca gtgtttactt attaatttgt tggctatttt cttagtcttt ttctacccag 16140
caaatttttt caaatgactt ttcaccacat gttattctac atttacaagt tgaatctcca 16200
tttagtatgt tctgtccata ttttgatctt ctttgggtct gtttggcaac acctccagag 16260
ttagcatctt tgtccgaatt cattagcatg cgatttactt cctatgttag gtcattaagg 16320
atattaccaa ggaggaattt atttaatttc tgtcttacag tatctagtgt tcttgcacca 16380
agattttttt tattgtaaat cttactgaaa aaataaatat aaaataattc ttggcaatat 16440
tggtactgta gctaagttat taatttttca ggtataatcc ataatctaga acaaaaaagg 16500
aacctgtggt catgcataac tttgagaaac tatcacccca ttaccagtga agcctggtat 16560
atagttatct attgctgcat aacaaattat tcaaaactta gtgttttaaa acaacattta 16620
ttatctcacg atgtctgtgg gtcaagattc tgggtatgac tcgggtggat actctgtttc 16680
aggatcagtc ataggctgca atcaaggtat gctgcagcaa tcacaagacc cagcttggga 16740
gggttctatt tccaagctca ttctgatgat tgttggcagg attcaattcc tgtagtttgt 16800
ttgagagcct cagttccttg ctagctattg gtctattata agcagaatca tgtaatattc 16860
tagcagaagc tgcctcaatt ctttgctatg tgggcctttc tatagggtga tatggcagct 16920
cgcttaatca gagcaagtaa gtggaaagag ccagagagag agaatgtgag caagatagat 16980
gtcacagtct ttcataacct atctgggaag taacatccta tcacttttgc tgtattctgt 17040
tcattagcgg taagtcacta aatacagccc acacttaagg gaaaggatca atagcaggag 17100
atgagggtca ttgagagcca tttttagaag cagtcagcca aacctgggtg tcttatagaa 17160

ttcctggtct ttcttctagt gttcggttcc taattgggac aagtctattc ttagatttat 17220
ttcagtgtac tgaaggactc cacccatttg tcacatgatt tttccactgg taatttagag 17280
tgagatagtg aaaattattt ggcttttata gttacatctg aggagtgcct agtagtccta 17340
tttaaaattt aaactttctt tttattatgg taaaatatac ataagatttt ctttttaagc 17400
atttttaagt gtacagttca gtggccttaa gtacatttcc attgttctga aaccatcatt 17460
accatctatc tgtaggactt tttcattttt ctaaatggaa actccatacc cgttaaacac 17520
taattttcaa tacctcctca ccatggccct tggtaaccat cattctactt tctgtctctg 17580
aattgattac tctagatacc tattataagt ggaatcatat aatattcatc catttgtgct 17640
gaatcatttc acttagtgtg atgtctttaa ggtttatctg cattttagca tgggtaagaa 17700
ttcccagccg ggcacggtgg ctcatgggcg acagagcctg ggcaacagag cgagactgtc 17760
tcaaataaaa aaaaaattaa aaagaattcc ctttctttct ttctttcttt tttttttttt 17820
tttgagaaac agagtgtcac tctgttgccc aggctggagt gcaatggcgc gatctctctt 17880
ctctgtaaca tccaccttct gggttccagc gattctcctg cctcccaagt agctgggatt 17940
acaggtgcat ggcaccatgc cttgctaata tttgtatatt ttgtagagac agttttgcca 18000
cgttgcccag gctggtctca aacttctgag ctcaagcaat tctcccacct cggccctgca 18060
aagtgctggg attacaggct tgagccactg cgcctggccc tgtttgatta tttgaggaac 18120
tgccgcactg tttttcacag taactgtccc atattatatt cccaccagca atgcagaatg 18180
gctctaattt tttcacatcc tcaccggcac ctgccatatt tattttttat aatggccata 18240
ttaatggaag tgaactggtg tctcattgtg gttttttttt aatatacata agacggagtt 18300
tcactcttct tgcccaggct ggagtgcaat ggcacaatct cggctcaccg caatctccgc 18360
ctcctgggtt caagagattc tcctgcctca gcctcccaag tagctgggat tagaggcacc 18420
tgccatcatg cccggctaat tttgtatttt tagtagagat ggggtttcac catgttggtc 18480
aggctggtct caaactccag acctgaggtg atccacccac ctcggcctcc cgaaatgctg 18540
ggattacagg cgtgagcaac cgcgcctggc ggtggttggt tttttttttt tttggagatg 18600
gagtctcgct ctgttgccca tgctggaggg caatggcacg atcttggctc actgcaacct 18660
ctccctcctg ggttcaggca gttctcctgc ctcagcctcc ctagtagctg ggactacaga 18720
cgtgctctac cacgcctggc taatttttgt atttttagta gagatggggt ttcaccatgt 18780
tggccaggat ggtctcgaac tcctgacctc aggtgatctg ccggcctcag cctcccaaag 18840
tgctgggatt acaagcgtga gccaccgccc ctggccctca ttgtggtttt gatttgcatt 18900
tcccctaatg attagtgatg ttggggctat cttttcatgt ttattgacca tttgtatatc 18960
ttctttggac aagtgtctat tcaagtcctt tgttcatttc ttccaggttg ttattttgtt 19020
gttgattgag ttttaggagt tctttatgta ctctagatgt taatccctta tcagatatat 19080
gatttgtaaa tattttttcc cattttgtgg gttgtctttt cactctgttg atagtattct 19140
ttgatgcaca aaagttttaa atttttttta attaaattct tttttttttt ttttagagac 19200

gaggtctcgc tattttgccc agctggactt gaacttctgg gctcaagaga tcctcccacc 19260
tcagcctccc aagtagctgg gactacaggc acatgctact gtgcctggcc aaaagttttt 19320
aattttgatg aagtccaaat ttattttttg ttttgttttg ttttggtatt atatccaaga 19380
aattattgtc aaatccattg tctgaataca ttacccctat gttttcctct aataattttt 19440
tttttttcaa gacggagtct tgctctgtcg cccaggctgg agtgcaatgg tgtgatctca 19500
gctcactgca acctccgcct cccagggtca agtaattttc ctgcctcagc cttccgagta 19560
gctgggatta caggcatgca ccaccacgcc cggctaattt tttgtatttt tagtagagat 19620
ggggtttcac catgttggcc gggctggtct tgaactcctg acctcaggtg atccacctgc 19680
ctcggcctcc caaagtgctg ggatcacagg tgtgagccac tgtgcccggc cttcctctaa 19740
taattttata attttagctc ttaggtttag ttccttgctt cattttgagt atgtttttgt 19800
atatggtatg aggtaggagt ccatcttttt tcttttacat gtggatatcc agtttcccca 19860
acatcatttg ttgaaaagac tttcctttcc ccattgagtg atcttgggac tcttgttgaa 19920
aatcatttga ccatgtatgg gagagttaaa tattttagct tttaaaaagt tttccttcta 19980
attgtaaaaa tacatgtttt ctgtagtaaa tttacaaaag aggaattccc ccatccacca 20040
gaaaataacc atactacata gaaataacta ctgttttttt ttggtggggg gatggagtct 20100
ggctctgttt cccatggagt gctgtgccac gatctcggct cactgctgca acctccacct 20160
cccgggttca agcgattctc ctgactcagt ctcccgagta gctgggacta taggcacgca 20220
ccaccatgcc cagctaattt ttgtattttt agtagagacg gggttttgct gctgtgttgg 20280
ccaggctatt ctcaaactag tgacctcaag tgatcggcct gtgtcagcct cctaaactcc 20340
tgggattaaa ggcatgagcc accatgccca gccctataac tactgttaat agttggtaaa 20400
tttcagttca ggttttttct gtgcataatt tatttatgtg tacacatttt taatagaaat 20460
gggatcatac atatgtactg ttttatgttt ttaatttaaa acataaagaa cttttctagg 20520
ctgggtgcgg tggctcatgc ctgtaatccc agcactttgg gaggctgagg caggcagatc 20580
atgaggtcag gagttttgag acaagcctga ccaacatggt gaaaccccgt ctcgaccaaa 20640
aacacaaaaa ttggccgggc gtggtggtgg gtgcctgtaa tcccagctac tcaggaggct 20700
gaggcaggag aatcgcttga acccgggcgg tggaggtgca gtgagccgag atcatgccat 20760
tgtactccag cctgggcaac aagagtgaaa ctccatctga aaaaagagaa aataaaagtt 20820
ttatttattt atttatttat ttattttttg agacagagtc ttgctctgtc acccaggcta 20880
gagtgcactt ttcttttctt ttttcttgag atggagtttc gctcttgttg cccaggctgg 20940
agtgcagtgg cgtgatcttg gctcactgca acctccgcct cctggattca agcgattctc 21000
ctgcctcagc ctcctgagta gctgggaact acaggcgccc accagcacac ctggctaatt 21060
tttttctttt tttgtatgga gtcttgctct gtcactaggc tggagtgcag tggcagatct 21120
cagctcactg caacctccgc ctcctgggtt caagtgattc ccctgcctca gcctcccagg 21180
tagctgggat tacaggcacg taccaccacg cccggctaat ttctttgtat tttagtagag 21240

acagggttta accatgttgg ccaagatggt ctcgatctcc tgacctcgtg atccgcctgc 21300
ctcggcctcc caaagtgctg ggattacagg cgtgagccac cgtgcccggc cacacctggc 21360
taatttttgt atttttagtt gagatggggt ttcaccatgt tggccaggct ggtcttgaac 21420
tcctgacctc aagtgatccg cctaccccgg tttccaaaag tcctgggatt acaggtgtga 21480
gaaacttttt atgtttaaat gttttataaa ttggccaggc acagtggctc tcacctgtaa 21540
tcccagcact ttgggagtct gaggcaggca gatcacttga gctcacgagt tcgagaccag 21600
cctgggcaat atagtaaaac ccatctctac aaaaaaaaat atgaaaaaaa ttagccaggc 21660
aaggtgacat gtgcctgtgg ttgcagcttc tcaggaggct gaggtaggag gaatgcttta 21720
gcccaggagg tggagttttc agagagctga gatcacacca cagcactcca gcctgggtga 21780
cagggcgaga ccctctctct ctcccctgcc aaaaaaaatt ctacaaattg ctttttaaaa 21840
ttagttttta ttattagagt tttaacagca cataatttta aaagtttaat ggttatatac 21900
aagttgttat tgaaaaagga gtcttctggc ttctccactg tgtcccattt tcatcctttg 21960
ggaagtattc actttcagta cttttggtat ttacctccac atctcaaact aagatgcttg 22020
tgttactgct gattaatttt tcagttgtag tcattatcta ccagctttcc actatggaag 22080
atgatttaac tttttcactg cccatctcat tcctactaca catgcacagt tagatccttt 22140
tatttttcca ataatgttat atcttaattt tggttaggcc agtagtcaga gtttaggcca 22200
gtactcagag tttaattttg gttaggccag tacttagagt ttaagttatt atgactctgt 22260
caatgctttt tttttctttt tctttttttt tttctttttg agacagagtc tcactctgcc 22320
gcccaggctg gagtgcagtg gcacgattgg ctctctgcaa cctctgcctc ccgagtagct 22380
gggactacag gtgcatgcta ccgtaactgg ctattttttg tattttcttt agagacggag 22440
ttttgccatg ttggtcccga actcctgatc tcaggtgatc ttcctgcctc agcctcccaa 22500
agtgctggat tacaggtatc agccactgca cctggccaga agtacagtat tttctgtttg 22560
gtctggtcat ggatcagtta tggcaaaatg agtggtttaa acgaccatta tcatgacaac 22620
ttgaaccttg attactcatt tgaggagttg tttgtcatcc ccttacccat ttacttgaga 22680
aggccgtcat aagcagttag cctcgacatc cctggctcag cctcctaagt agctgggaca 22740
acaggcacgc accatcacac ctggctaatt ttttatactt tttatagata tggagtttca 22800
ccaagttttc caggctgatt tcaaactctt gggctcaatg gatctgcctg ccttggcctc 22860
ccaaagtgct aggattactg ccatgagccg ctgcgcctgg atggttatct ctttttttga 22920
gtagtgaagc tgattggaag ttccgtgtgt gtgtgtgtgt gtgtgtgtgt gtgtgtgtgt 22980
gtgtgtacca ctactggtat aagttcagtt ttctcagatt cattaaatca gttacctctt 23040
gtacatcttc tgtccaactt gcaaattttt atcattgtct cctgtcttgt atgggttttc 23100
cttgtgaatt tattcaaaga aaaatgccta aaacacatct tatgttctca ataaatactt 23160
gtcgaatgga tgaatgttgc tattgtcctt ccaagaccag ctaatcaata atggcatcct 23220
ttccttttag cccagacttt gcttcaggat ccttctcaac ttaatgtgtc aggctgccct 23280

ggagatcaga tctttttccc accctgtcta aaaggctagg gagcatgact tactcatctc 23340
gtatttcctt cagctattgt cggggttctt gtcaaatgtt tgtttattga aagaatgatt 23400
ggtaaaattt cagatagtag agtattaaaa agtgttcact cattcaaata tataccataa 23460
atcaaggtta ttaatagtaa aaaaaatgat tatagatctc tgttacataa tctagccatt 23520
cccaaactct caggacgtct tactaatctt aaaaattatt ggtactctaa atattctttt 23580
tctttaacat agatttatat ctattgatag ttactgtatt aaaaactaaa agtggccagg 23640
tgcagtggct cacacctgta atcccagcac tttgggaggc cgaggcgggt gaatcacgag 23700
gtcaagagtt caagactagc ctggccaaca tggcgaaact ctatctctac taaaaataca 23760
aaaattagcc aggtgtggtg gcaggcgcct gtaatcccag gtacccggga ggctgaggca 23820
ggagaatcgc ttgaactcgg gaggtggagg ttgcagtaaa atgagatcgt gccactgtac 23880
tccagcctgg gcgacaagag caagaatgtc tcaaaaaaaa aaaaatctaa aactgaaaaa 23940
ttaaaaaaat tttaataata gtaaacctat tacatgttaa atcttttagt tttatttatt 24000
tattttattt ttttggagac agggtctcac tctattgccc atgctggagt gcagtggtgt 24060
gatcatggct cactgcagcc attgacttcc tggcgtctaa gtgatcctct tgccttagcc 24120
tcccagtagc tgggactaca ggcacgtacc accatgcctg gctaattgtt tttattttaa 24180
tcttttgtag aaatggggtc ttgctatgtt gcttaggctg ttcttaaact cctggcctcc 24240
agcgatagtc cttcctcagc ctcccaaagt gctgggatta caggtgtgag ccaccatgcc 24300
tgccctaaaa gttgtatatt tacatttagg tctgtgatac attatgagtt acattttgtg 24360
tagagtgtga gatttagaca ggggttctgt tttttacata tggaaataca attgtttcaa 24420
cataatttgt tgagaagact atcttttctg tattattatt attactatta ttattattgt 24480
tttgagacag agttttgctc tgtcaccagg ctggagtgta atggcacgat ctcagctcac 24540
tgcaacctct gcctcccggg ttcaagcaat tctcctgcct cagcctcccg agtagctggg 24600
attacaggca tgtgccacca tacccagcta attttgtatt tttagtagag acagggtttc 24660
tccatgttgg tcagggtggt ctcgaactcc tcacctcagg ttatccactt gaggcctcgg 24720
cctcccaaag tgttgggatt acagatgtga gccaccacac ccggcctgtc ttttctgtgt 24780
tgaattgcct ttgcatcttt gtcaaatatc atttggccca gaataggcaa atccatacag 24840
acagaaagat ttagtgattt ccaggggctg agagaagaag ggaatgggga gtgactgcta 24900
atgggtatgg ggttttgttt tgggatggtg aaaatgttct gggattagat agtggtaatg 24960
gaatcttttt gatggccagg tgtggtggct cacacctgta atcccagcac tttcggaggc 25020
tgaggtgggc agatcacaag gtcaggagat cgaggccatc ctgtctaaca cggtgaaacc 25080
ccgtctctac taaaaataca aaaaattagc tgggcgtggt ggcaggcgcc tgtaatccca 25140
gctacttggg aggctgaggc aggagaatgg cgtgaacccg ggaggcggaa cttgcagtga 25200
gctgagatcc tgccactgca ctccagtctg ggtgacagag cgagactcca tctcaaaaaa 25260
aaaaaaaaaa tctttttgat gtatacattc tatttttttt ctttctaact tttattttag 25320

gtccaagggg tacatgtgta gatttgttcc atgggtaaat atgtgtctta gtggtttggt 25380
gtacagatta ttttgtcacc caggtaatac acatagtatc caataggtag tttgttgatc 25440
ctgacccact tcctaccctg taccctcaat ggactgtttt tctgtttcat gtgtttatat 25500
ttctgtcctt ttgcagatag ctcactgatc tgattactgt agctttatac agtaagactt 25560
aaaatcatgt agtgtgtttc ctccaacttt attttttaat aaagattttg ggtaattttt 25620
gcttatccac atagatttta gagtcagctt atttatatga aacattctgc ttagatttca 25680
attggaattg tgttaaaact gacagtttgt gtgaactttg tttttttaga tgccttttat 25740
taggtttaga aaattctctt ctattcctag tttgctgaga gttttatacc atgaatgaat 25800
attgaatttt gtcaaatgct tctcctgtat caaatgatag gtcatgtggt tttcattttt 25860
agactattaa tctgctgtac tgcatgaatt gatttttgaa tattgagtca gccttgcatc 25920
cccaggataa accataccgg attgtggtgt attattcgtt ttatgtattg ctggattcag 25980
tttgctacta ctttatgagg aattttcatg tttgtgttta taagggatat gggtctctta 26040
ttttttgttt tctttttgag acaggtctca ctctgctgcc cagcctggag tgcagtgatg 26100
gtatcttggc ttactgcaac cttgactttc ctgggctcag gtgatcttcc cactttagcc 26160
tcctgagtag ctgaggctac aggtgtgcat caccatgtcc agctaatttt tagtattttt 26220
tgtagagata gggtttcacc atgttgccta ggctggtctc aaactcctgg gctcaagaaa 26280
tccacccgcc ttcgcctccc acagtactaa gattacaaac atgagccaca atgcccatcc 26340
tttgactagt tttaatatca agttaatgct ggactcataa aatgagatgg gaagtgtttt 26400
ttcctcttct tttttggaga gattgtataa aatttgtttc atttttcttt taaatgttta 26460
gtagaatttg ccattggaaa aaaaatctag gcctgtatta gtccattctc acactgctaa 26520
taaagactta cctgagactg ggtaatttgt aaagaaaaag aggtttaatg gactcatagt 26580
tccatatgac tggggaagcc tcacaatcat agtgaaaggc aaaggaggaa caaaggcaca 26640
tcttacatgg cagcaggcaa gagagcgtgt gcaggggaac tgccttttat aaaaccatca 26700
gatctcatga gactcactca ccatcatgag aacagcatca tggcagtaac tgccctcatg 26760
attcagttac ctcccactag atccctccca tgacatgtgg gggattatgg gaactacaag 26820
atgagatttg ggtagggaca cagccaaacc atatcatgcc catatttttt tagtaggact 26880
attaaggagg tccctttcac cttgggtgaa ttgtggtagt ttgtagtttt tgatggttgt 26940
ctgtgttcac tttctgtgta taatgttgtt cattatgttt cctaattata tatttatgtc 27000
tgtggagtct atagtgataa cccctgtttc attcctaata ttgattattt gtgagttctt 27060
ttttcccaga tttcctaaaa ggttataaat tgtattgatc tttataaaga gctggctttt 27120
ggtctcattg attttttttt ttctatgacg tttctggttt taatttcatt gttattattt 27180
tgtcttttgt ttcctctgct tgttttgggt ttagtttgtt tgtcttttcc ccctaggtgg 27240
aagcttatag ttttaatttg aaacttccgt gcttattttc aatttaaaaa ttattttaat 27300
attatatata tattttaaga gacagcatct ctgtcaccta ggctggagtg caatggagtg 27360

atcatacctc attggagcct caaccttctg tgcttaaatt atcctcccgc ctcagcctct 27420
tgagtagcta ggactacagg cacccaccac atgcagttaa gttttttaaa aaaatttttg 27480
tagtaacagg gtcttgatag gttgcccagg ctggtcttga actcctggcc tcaagtcctc 27540
ctgccttggc ctcccaaagt gttgggatta taggcctaag tcactgcacc tggcctcatt 27600
tttcatataa gctttcagtg ctatagtttt tcctctaacc attgctttag ctgcatatca 27660
tatgttttat tttccttttt gttcaattca aatattttct aatttgcctg gggactttgt 27720
ctttaaccat ggcttatttg gaacaggcat tggcaaacat tttggttttt tttctttctt 27780
tctttttttt tttttttttg agacagagtc ttgctctgtc acccaggctg gaatgcagtg 27840
gcatgatctt ggctgactgc atactccgcc tcccgggttc cagagatttc tcctgcctca 27900
gcctcccagg tagctgggat tgcaggcaca caccaccaca cctggctaat ttttgtattt 27960
ttagtagaga cagggtttta ccatgttgac caggctggtc ttgaactcct gacctcaggt 28020
gatccgcctg cctcagcttc ccaaagtgct gaaattacag gcgtgagcca ccgtgcccgg 28080
cttttttttt tttttttttt tttttgagac agggtcttgc tctgtcatcc aggctagagt 28140
gcagtcttac aatcacagct cactgcagcc tcaacttcct ggctcaagag atcctcccac 28200
ctcagccttc tgagtagctg ggaccacaga tgagtgccac acacctggct aatttttata 28260
ttttttgtgg agacagtgtc ttgccatgtt gcttaggttg gtcttgaacc cctgagctca 28320
atcaatccac ctgcctcggc ctcccaaaat gctgggacta caggtgtgag cccccgtgcc 28380
cagccaaaca ttttattaaa ggtccaaata ataaatatgt taggcctaag tgtaataccg 28440
tctgtgttgc agttatttaa ctctgctcta gtagtataaa atcagccata cacaacatat 28500
aaatgaatga gcatggctta gttccaataa agtgttattt atataaaaat caggcacctt 28560
gccctgttgt tatagtttaa tttccaagtg tttgtattct tgttatcttg ccgttattga 28620
tttctagctt aataacatta agctagaata tactagcttg ttatcagaga gcatagtatg 28680
tatgatttcc gttcttttaa atttgcaaaa tttgttttgt gacctgggat attatatagt 28740
cttttggtgt acgttccttg tgagtttgaa tgtgtattcc acttttgttg ggtggagtgt 28800
tctgtaaatg tcagttccag ttgtttgatg gttgtttttg attaagttca ctgacatcat 28860
ctgggtgtcc tataattcgc tttaattctg tcactaattg cgtcaagtta atgcagacct 28920
cacaggttac agggctcagt cccacaaagc tgccttcact tcagactgga gccacaagcc 28980
actcaaattt tccctggttg agtataagtt tgggccttcc cacaatcccc ttgggtttaa 29040
taatttgctt agaatgactc acagagttca tgaaagtgct atacttaaaa ttaccttttt 29100
attataaagg atacaaatga acaatcagat gaagaggact atatgataag tttgtaaggc 29160
tcccaagcac gggagcttct ctacttgtgg agccagggta caccaccctc ttggtacatc 29220
agtatgttca ccaaccagga agctccctga gccttgtagt tcagagtttg tttttttttt 29280
tcttttttat ccagctgaat ttaagattta cagagttttt aatatacaga gttcagggct 29340
gggcacagtg gctcatgcct gtaatcccag cactttagga gactgaggtg ggaggatcga 29400

ttgagtctag gagttcaaga ccagcttggg caacataccc agactctgtc tctacaaaaa 29460
agtaaaatga gctgggtgtg gtggcacatg catgtagtct agctacttgg gaagctgaga 29520
tgggagtact gcttgagccc aggagtttga ggctgcaatg agctatgttt gcaccactgc 29580
actccagccg gggcgacagt gagaccccat ctcttaaaaa aaaaaaaaaa aaaaaaagtc 29640
gggcatggtg gctcacgcct gtaatcccag cactttggga ggccgaggtg ggtggatcac 29700
ctgaggtcag gagttcaaga ccagcctggc caaacatggt aaaacccccc gtctctacca 29760
gaaatacaaa aaaattagcc gggcgtggtg gtgcacacct gtaatcccag ctactcacaa 29820
ggctgaggca ggagaatcgc ttgaaccctg gaggcagagg ttgcagtgag ccaagattgc 29880
gccagtgcac tccatcctgg gcaaaaggag tgagactcca tctcaaaaaa caaacaaaaa 29940
gagcttatta cgtaggcatg gttgattaaa tcattggcca tgtgattgaa ctcaatcact 30000
agtaactctt ttcctggaag ttgggaggtg gagatgaagg ttctagtcat gtagtcatat 30060
gctttcaggt gacagccccc atcctgaagc ttggagtcac ttcattagca taacaaaggc 30120
attcatatca cttaggaaat tccaagcact gccaggaatt tggaaatgaa taccaatcct 30180
ctgacacaaa aatgtaagat aggaaaaaat aaactgttct tactactata ctgaatactt 30240
ctggcaaatg acagagaagt gcaatttgct agaatggctc tcagaactca ggaaaacact 30300
tcatttatgc ttactggttt attatctaat aaggatataa atgaggatac aggtgaagag 30360
atacataggg caaagtctgg aaggatcctc agagcaggag tttctttcct catgaagtta 30420
ggatacacta ccctcacaac atatggatat gcttattgtc tcatgtccat atgaagaggc 30480
caccaaacag gtttgtgtga gcaacaaggc tgtttatttc acctgggtgc aggtaggctg 30540
agtccgaaaa gagagtcagc aagggttgtg ggattatcat tagttcttac aggttttggg 30600
ataggtggtg gagttaggag caatgttttg caggcagggg gtggatctca caaagtacat 30660
tctcaagggt ggggagaatt acaaagaacc ttcttaaggg tgggggagaa acaaatcaca 30720
atggtggaat gtcatcatct aaggcaattt tcacttcttt tgtggatctt cagttgcttc 30780
aggccatctg gatatatacg tgcaggtcac aggagatatg gtggcatagc ttgggctcag 30840
aggcctgaca ttcctgtctt cttatattaa taagaaaaat acaacaaaat agtgaagtgt 30900
tggagtggcg aaaaattttg ggggtggtat ggagagataa tgggcaatgt ttctcagggc 30960
tgctttaagt gggattgggg tggcgtggga acctagagtg ggagagatta agttgaagga 31020
agatattggg gtaaggagtg atattgtggg gttgttagaa ggagcatttg tcgtgtagaa 31080
tgattggtga tggcttggat gcggttttgt atcaattgag aaactaaacg gaagatacaa 31140
ggtctgaata aaagaaggag aaaaatagat attaaaggac taagaattgg gaggacccaa 31200
gacatccact tagagactgc ccaagggggt ttagcgtaat tacttgcttg gttggcgagt 31260
ttttgggaaa agaccattag tccgttttac ctttcctgaa gattgaggac ggtaaggggt 31320
atgaagattc cactgaatac caagagcctg agaaactgct tgggtgattt gactaataaa 31380
ggccggtttg ttattggact gtatagaggt gggaaggcca aactgaggaa ttatgtccaa 31440

cagaagggaa gaaatgacca cggtggcctt ctcagatcct gtgggaaagg cctctatcca 31500
tccagttaaa gtatctaccc agaccaagag gtattttagt ttcctgactc aagacatgtg 31560
agtaaggtca atttgccagt cctgggcagg ggcaaatccc tgagcttgat gtgtagggaa 31620
gggagggggc ctgaataatt cctgaggagt agtagaatag cagatggaac actgagaagt 31680
gatttccttg aggatagatt tccatgatgg aaaggaaatg agaggttcta agaggtgggc 31740
tagtggctta taacctacat ggaagagatt atgaaatgaa gacagaatag aatgggcctg 31800
tgaggctgga aagagatatt gtccttggtc caaggaccat ttgtcttgtg tgagaagaga 31860
ttgataggtg gaaacttcag taggagagta aataggagtg accaatgaga aggagaaaaa 31920
ctggccatga gggacagaag ttggaatgct agctgcttct ttagctacct tatcagcata 31980
agtgttgccc tgagcgatgg gatctgatgc cttttgatgg ccgttgcagt gaatgactcc 32040
agcttccttt ggaagtaaag aggccttgag aagagtttct attaaagaga catcaatgat 32100
ggaggaccct tgtgtagtga ggaaactttt cagcccataa cagcatggtg gtgcaggata 32160
tggggtcagt ataaatattg atgcatagtc cctttgcaag agtgagggcc cgagttaagg 32220
caatgagttc ggcttgctga gaggtagtgg agggggcaga gcagtagcct caatgataga 32280
tatggaagct actatagcat agcctgcctt tgcttgtgag tggcgattag gcttggttgg 32340
actgccatcg ataaaccaag tgtgttctgg gtgaggaaca ggaaagaagg aaatatgggg 32400
aaatggagtg aatgtcaggt gtatcagaga gatacagtca cgggggtcag gtgtggtatc 32460
aggaataatg tgggaggatg gattgaagtc cgggccagga ataatggtaa ttgtgggaga 32520
ctcaacgaag tgtgagtata gctgaaggag ctggggagca gaaagtatat gtgtcaggtg 32580
ggaggaagaa aatagatttt ggaagttatg agaactgtag agagtgagct gagcatagtt 32640
tgtgattttg agggcctcta aaagtattaa agcagtggtg gtcactgcac gcagagatga 32700
ggggtaggct aaaacagtaa ggtcaagttg tttggacaga aaggctacag ggtgcagtcc 32760
cggctcttgt gtaagaattc tgaccgcact aaccatgcct aggaaggaaa ggagttgtta 32820
tttcatagaa gggattgggg tttgggagat tagctggaca cgatcagcgg gagagcacgt 32880
gtgttttcat gagaattatg ccgaggtagg taacagatga ggaagaaatt tgggcttgac 32940
tgaagtaatg gaggctgtct gtgaagcttt gcggcagtac agcccaggta atttgctgag 33000
cctgatgggt gtcagggtca gtctaagtga aagtgaagag agactgtgat gaagggtgca 33060
aaggaatagt gaaaaaaaag catgtttgag atctagaaca gaataatggg ttgtggaggg 33120
aggtatttag gataggagag tgtatgggtt tggcaccaca gggtggatag gcaaaacaat 33180
ttggttgata aggcgcagat cctggactaa cctgtaagac tggtccagtt tttggacagg 33240
taaaatgggg gaattataaa gagagtttat aggctttaaa aggccatgct gtaacaggca 33300
agtgataaca ggctttaatc cttttaaagt gtgctgtggg atgggatatt ggcgttgagc 33360
ggggtaaggg tgattaggtt tttaatggat ggcaaggggt gcatcattta ttgccaagga 33420
gggagtagag gtatcctata cttgtggatt aaggtgggga gatacaagga gaggatgtga 33480

aggagtcttt gaactgggga aaaaggcagc aatgaggtgt ggctatagcc cgggaatagt 33540
cagggaagca gataatttag ttaaaatgtc ttgacctaat aagggagctg ggcaggtggg 33600
gatgaccaaa aaggagtgca ttaaagaatg ttttccaagt tggccccagt gttggggagt 33660
ataaagaggt ttagaaggct ggctgtcaat acccacaaca cttatgaagg caagggaaac 33720
aggcccttga aaagaaggta atgtggagtg ggtagcgtcc gtattgatta agaaggggac 33780
ggacttaccc tccattgtaa gagttaccca aagcatctga ggcttccaag gagattgggc 33840
agtgtgtctt cagctgctaa gccaagaaga tctgggaagg agtaagtcag agccttgggc 33900
cagttggaca gtccaatttc cagtggcgtc cctcacagat gggacacgac ttaagaggaa 33960
tcccaggctg tgggcattcc ttggcctagt ggccagattt ccggcacttg aagcaagatc 34020
ctgggggagg aagtcctgaa ggaacgcctg accgctgtgg cttaggcgtt ttgaagtttt 34080
tgtgtgctgg agatatggct ggggttttct ctcacagcag agtcaagtag ttgcaactct 34140
tctctattac tgtacacctt gaaggtgagg ttaattaggt cctgttgtgg ggtttgaggg 34200
ccggaatcta atttttggag tttttctaat gtcgggagcg gattgggtaa taaaatgcat 34260
attaagaata aggcagcctc tggcccgcct gggtctaggg cggtaaagcg tctaagagtt 34320
gctgccaaac gggccatgaa ctggcctggg tttttgtatt tgatgaaaaa gagcctaaat 34380
gctaactgat ttgggagagg tcagataaag aaaaaaggag cattaacctt gagtatgcct 34440
ttagctccag ccacctcttt aagaggaaat tgttgggcag gtgggagagg gctagtcgca 34500
gaacaaaacc ataagccaga ctgggtgtgg ggaggggagg tgatagaagg gttatagggt 34560
gggagagcag aagctgaaga agaattggga cctggcttgg cctggcgagg agcagcctgg 34620
ggagaagggg agaggtcaga tgagtccgta gaaaagaagg attcagagga ctcagagctt 34680
ggggtggaga ctgaaggaac agataggaca gaaagaagaa agatttggaa tgagtctcat 34740
tgggaacaaa tactagggag ggaccaatgt gtaaaagatt gcctggacgt caggcacctc 34800
agaccatttg cccatttttt gacaaacatt atctagatct tgcaggatag acaaatcaaa 34860
agtgccattc tctggccact tggaactact gtcgagtttg tattggggcc aagcggtatt 34920
gcagaaggaa ataaaacgct tagattttag gtcaggcgag agttgaagag gttttaagtt 34980
cttgagaaca caggctaagg gagaagaagg aggaatggag ggtggaaggt tgcccatagt 35040
gaaagaggca agtttaaaga gaagggtaga gacatggaga gaaggggtgg ggagtgcttg 35100
ccccccagga aagtggagaa ggggtagaga caccaaaaga agaggtcggg gggtacttgc 35160
cccccagaaa ggcggtgctt gctgctaagg gtgaaggacc aaggcaggtg accctgcatg 35220
gtcagacacc tctgaaacgt gggtgaataa tcaggcaggc gtccctgcaa tgattaaaca 35280
ccaagggaat gctgccttcc tgagtccgtg actggtgccg gagttttggg tccacggata 35340
aaatgtgtct cctttgtctc taccagaaat gaaaggaatt gaaattaaga gaaggagaga 35400
ttgaagggtg acgccaagat tgaaaggaga aagaggttga ggggtagtga gagaggttgg 35460
agagtaaaaa gggctgctta cccgatttaa aatcggtgag atgttccttg ggctggttgg 35520

tctgaggacc tgaggtgata ggtagatctc ttcacggagt gagggtgagg acaggggact 35580
ggtctcccga aggagtcctg ctgacccagg tctttggcac caaatgtctc acgtgtctgt 35640
gtgaagagac caccaaacag cctttgtgtg agcaacaagg ctgtttattt cacctaggtg 35700
caggtgggct gagcccgaaa agagagtcag caagggtggt gggattatca ttagttctta 35760
taggttttgg gataggtggt ggagttagga gcaatgtttt gtgggcgggg gatagatctc 35820
acaaagtaca ttctcaaggg tgggaagaat tacaaagaat gttcttaagc gtgggggaga 35880
ttacaaagaa ccttattaag ggtgggggga aacaaatcac aatggtggaa tgtcatcagc 35940
taaggctatt ttcacttctt ttgtggatct tcagttgctt caggccatct ggatgtatac 36000
gtgcaggtca caggggatat gatggcttag cttgggctca gaggcctgac acttattagt 36060
ttccaaaact tgtgcttttg tgtttttaat ggagacttca ttaaggcctg attgattaaa 36120
ccattagcca ttggaaatct actcaacctt cagcatctct ctcctcccca gagcttgtga 36180
gtggggccga aagttccagc cctctaatca cagggttggt tcccctggca accagccgtc 36240
tatctttaaa ggtttcctaa agtcacctca ttaacaaaat cccagatgtg gttgaaaggg 36300
gcttgttatg aataacaaaa ttcactccta tcatctttta tctatctgga gctatttcag 36360
gaaccgagta taaaaaccaa atattatacc aaaagatgct cctagcaccc cagtcacttc 36420
agaaattaca agggctttat gagcgctgaa caagaaccag ggacaaagac caaatattta 36480
tatttcttat tattaatata agccacaata tcacaaccaa atatatttta caattatacc 36540
ccaatggtac tgttttagct tctgtattct tgacaatttt ctgtctagtt atttatttgg 36600
gaaaagagtg aagtcttcag ttatagctat ggatttgtct actccagtta taaccacgga 36660
tttgtctgtt tctcctttca gttcattgat ttttatttca tgtgttttga tggtcttttt 36720
aaggtgcata catgtttata atttctttgt aaattggctc ttccatggtt ctgtatctct 36780
ttttatctcc agtgattttc tttgccctga agtcaacact gtcagatatt ataatagata 36840
ctccagcttt tcttttgtta atatttgcat ggtatacttt tttcccgttt cttttaacct 36900
acataataat agtattattt ttgttattat tttcagtggt atttaagtat attcacttta 36960
acctacatat tatttttgaa gtgagtttct tatagaatca tatttatcct gctaatctct 37020
gtcttttcag tggtgtgttt atgccattta tacttaaggt aattacaggt aagtttggat 37080
tcaggtcaac cattctatta tttgttttat gtttgtccct ttatttttgt tcttctgttt 37140
ctttctgtct acttttggat tatcttaaca tgttttggta ttccacttta tttttatttt 37200
ttttgctttt cctgatatct ctttgtgtag tttttaaagc ggttgctata gggcttacga 37260
tatatctact ttttaccatc tacttagagt tattacatta aatattaaat gttatataaa 37320
tattacataa tatgaatatt acattaccta aaataacgca gaaacctaac cgcaatcttg 37380
agtcccttta catgagtccc tttaccattc tacttttttt tttttttttt ttgaggtgga 37440
gtttcgctct tgtcacccag gctgtagtgc aatggcacaa tttcggctca ccgcaacctc 37500
caccttctgg gttcaattct cctgcctcag cctcccgagt agctgggatt acaggcatgt 37560

gtcaccacac ctggctaatt ttgtattttt agaagagacg gggtttctcc atgttggtca 37620
ggctggtctc gaactcccga cctcaggtga tccacctgcc tcagcctccc aaagtgttgg 37680
gattacaggt gtgagccacc gcgcccagcc ctctaccttt tttttggagt agtgttatat 37740
ctgtttacat tggaaatccc ttcagccagt ttttaatttt tgcctttaac ttgcaagcat 37800
atttttgaga actcaagagg agaaaatcag gctcttatac ttccctggat ttctattgct 37860
cttcctccat tgctgttatt ccagattttt ctgtattata attacccttt tctcagaaga 37920
actttccttg acaattcttt aagagcaggt ctgctgacaa cacatagtct taattttcct 37980
ttatctgaga atgtccttat ctctgaagga tattttctct gcatatagaa ttgtgggttg 38040
acagttcttt acttttagta ctttaaatat gtgtttctat ttcttgctgg cctttatgat 38100
ttctgatgag aaatccgcag tcagtcaaat cattgttacc ctataagtaa tatattattt 38160
ttatctagtt atcattatta ttgttattat tattttgaga tgaagtctca ctgtgttgtc 38220
caggctggag tgcaatggcg caatcttggc tcactgcaac ctccaactcc tgggtccaag 38280
tgattcttct gcctcagact cctgagtatc tgggattaca gacatgcacc accacacctg 38340
gctaatatat atatatatat attttgtatt ttaagtagag atgaggtttc accatgctag 38400
ccaggctggt ctcaaactcc tgacctcagg tgatctgcct gccttggcct tacgaagtgc 38460
tgggattata ggtgtgagcc accgtgcctg gctgttttct agttattttt aagtatatat 38520
ttttttctgt ttaattttca gcagtttggt tatgatatgt ttaagagttg tttttgtgtg 38580
tgtgtgtgtg ttgtgcttat tgtattcagt cagcttcctc aatctgttgt ttttttcctt 38640
cacaaaattt gtgaagtttt agctattttt tatttttttc tgtcctaaac tgttacctct 38700
tctttttcaa ctctaatggc acaaatatta gaatttttgg tattgtccca ctagtccctg 38760
gggtctatat tcatttataa aaatatttta tcttcacatt gtgtaattgc tatagattta 38820
tcttccagtt tactgactct ttactctgac actgccattt ttctattgac cccatccagt 38880
gaaattttaa aatttcattt actgtatttt tcacttctaa atttttattt tttttttttg 38940
agacagagtc tcgttctgtt gcctaggcta gagtgcaatg gtgcgatctt ggctcattgc 39000
aacctccacc tcctgggttc aagcgattct cctgcctcag cttcccgagt agctgggatt 39060
acaggcacct actaccacgc ccgctaattt ttgtattttt agtagagatg gggtttcacc 39120
atgttggcca ggctggtctc gaactcctga ccttgtgatc tgcctgcctc agcctcccaa 39180
agtgctatga ttacaggcat gagccaccgc gcctggccta aaattttaat ttctttcttt 39240
atgtctcatt tctttactga gactttatat ctttctattc atttctaggg tgttcacctt 39300
tattttttgg aatatttttc ctgataagtc ctaaatctgt gtcatcttgg catttgcatc 39360
tattgattgt gctttcgtat gtaaattgag atattcttgg ttctttgtgt gcttggtaat 39420
tttggattgt atgctcaaca ttaaaaggtt tttttttttt tttgaaattt gagtcttatt 39480
taaattctgt ggaaaatgtt gatttttttt tttttttttt tttttttgag acggaagctt 39540
gctctgtcat ccaggctgga gtgcagtggc acaatcttgg ctcactgcaa gctccgcctc 39600

ccgggttcac gccattctcc tgcctcagcc tcctgagtag ctgggactat aggcacctgc 39660
caccacgcct tgcctaattt ttgtattttt agtagagaca gggtcttgcc ataatgccca 39720
ggctggtctc gaactcctga cctcaagaga tctgcctgtc ttggcctccc aaagtgctgg 39780
gattacaggt atgagccacc atgcccagct gaagtaattg ttttctgtgt gactatggca 39840
caaactcagt atgcaatgga tgcaatggtt tattttattt tttttttaat ttttttagtt 39900
ccataggttt ttggggaaca gattgcagta gattactggt ttcagtttgt tttgaatttt 39960
tagagattgt cctttatttt tagtgttttt taaaaatttt tgtctcttga acattccatc 40020
attcattttc tgaacaaaat gctaaattta ttgtgtgata tacaagggag agccataatt 40080
gtaggacata atttctgtga atgaaacaaa ctgcccgacc aggtgcagtg gttcatgctt 40140
atgatcccac cattttggga ggctgaggtg ggaggatcac tcaaggccat gaatctgaga 40200
ccagcctggg caacatagtg agaccctgtc tattaaaaat aaacaaacaa acaatgttat 40260
ccaggtatgg tagtgcgtgc ctgtagtccc agctccttgg gaggctgaga tgggaagatt 40320
gcttgagccc aggagttcaa ggatataatg agctatgatt gcaccactgc actccaacct 40380
gggtgacaga gcaagacttt gtttcaaaag aaaaaagaaa agaaaagaaa ctgctggtct 40440
taaataggat ttgaggaatt atggagttca gggattggtg aagtaggagt atttaggaat 40500
tgattgacct tttattatgt aaatgtggtt atgaattgtg gctgttgctg actggtgact 40560
tgctgaagtg atatcattca ttcaaagctt cctttgatgg gcttaggatg ggtttaacac 40620
cagttctgat gattacttgt tgttacggtc ctttctggaa tttggagttt agaacttttt 40680
tttaatcata tgtaaaacat ttacatggtt caaaaatcaa aactatacca cgagatgcat 40740
tcacagaagt ctggctttct ttcttatccc ttccatcctg tttttctttt cttttctttt 40800
tttttttttt tttgagacag gatcttcttc tgtcacccag gctggaatgc agtggcacct 40860
tcacagctca ctgcaacctc aaccttccag gcttatgtga tcctccccgc tcagactcct 40920
gagtagctgg gattacgagt acatgcaacc acatctgcct aatttttaaa tttgttgtag 40980
agacgggatc tccctatgtt ggctaggctg gtctcaaact cccgggctca agcaaccctc 41040
ttgccttagc ctcccaagat gctgggatta caggtgtgag ctaccactcc tggcctgttt 41100
tttttgctat tgagtaaagg ttggtaaaca gtctgcaggc caaatttggc ccactttttt 41160
ttttttttgt aaataagatt ttgttggagc acagccatac tcattcgttt acataattgt 41220
gtatggcttt tgtgctgcag tggcagaatt gagtagctat gacagagaat acagtatgtg 41280
gcctagggac cgaaatattt actctctaga cctgtattga aaaagtttgc tgactcctgc 41340
tataaagaat ccatcatttt cattaagttt tggcttattt cttctttctt tctttttaaa 41400
aaacaaaagc agatacttaa atatactcaa catttatctc ctttttctac aaagtacagc 41460
accctttact gtcttgcctt tttcactaaa caatgtatct tgaaagtcac tctgtatcag 41520
tatctttctc attcctggac tggttacagt tttcatctcc tttaggccat atttttctgc 41580
tatatcttca ttttctacag gtatgctgtt catcttatta gtatcctctt tttcttacaa 41640

tgtccttgaa tgaggtttgg tcaacaaccc ttttatttgt tagtatttat ttcctgtgca 41700
ttttgtaaca ttaaaatatt attagctcta atgatattaa gctctaagtt acaaactata 41760
agcagttgta aattaggtac tatgtctttt cagcagtata ttgttctcag gatagttgtc 41820
tacctagttc aagtgccatt gacatcatgc ttgccctata atctaacaat ctcagttatt 41880
ataaataata tctgtgtttt aacggtgtct ttgtaatgcc tagctatttc actgggcatc 41940
tgtaatagaa agatacgtgg ttttaaaaat acgcatcaaa catttctagg tctttttccc 42000
cttcttccca acaaaatagc ctaattagtg tatagtatta gtagtgcttc ctgatgctgg 42060
aatgttattt gtgttgatta gttaacccta aatactgcta ctattttttg ttgttgttgt 42120
tttaggttaa atggaaacca cccttgggaa ctggatgcct gtgtagctgt tctaccatat 42180
cagtgtattg caatgagtgg gggaggagag cagctggata tcctgagtgt tggaatccta 42240
gtgaaagaaa gatggaaagt ggtgagtgta catctcagag agtgtgtgtg tgtgtgtgtg 42300
tgtgtgtgtg tgtgtgtata catatcgggg ggtaattttt ggtatcctga taaaaatagc 42360
atttcattag gaaatattac atacattact tatatgccta aagcagttat ttacagttat 42420
ttttctatga ttaagctctg tttataccct aattcttact cttgcttcgt atagtactaa 42480
atttttgaac agatgaaaaa aatacagtgt ttgaaagtac aatgagctgg ccgggcgcag 42540
tggctctcgc ctataatccc agcactttgg gaggccgagg cgggcggatc acgaggtcag 42600
gagatcaaga ccatcttggc taacacgatg aaaccccatc tctactaaaa aaaaaaaaag 42660
caaaaaaaat tagctgggcg tggtgcaggc gcctgtagtc ccagctactc aggaggttga 42720
ggcaggagaa tggcgtgaac ccgggaaaaa aaaaggaagt acaatgagct gctctctctt 42780
tttttttttt tattaaggaa aattgtaagt atacataaaa aatagactta tataatgaac 42840
ctccatgttc tcatgactca agttaaatag tcatcaatgt atgattaatt ttgtttcatc 42900
tctctcccag aatattttga agcgatattt aaccttagca tttcttctgt gtatacttca 42960
gtatgtatct ctaaaaaata aggacttact tttaaaaaca taatcataat accattatca 43020
taccttaaaa ataatcattt cttccctgct gactcctccc ccaagattgt gtatcatttt 43080
gtctgttagc atgtagtatt tttcagctac tttctactgt tataaaatac tgtagtagta 43140
ctaaaaaaaa gtaatcattt cttaatatta ccaaatacag ttttagtttt cacattctcc 43200
tgattgtaaa gtattttgaa tagcctgaat caagatccaa ataaaacctg gatattgtaa 43260
ttagttgcca ttacttttca tttctaggtt ccttcctctt tattgctctt tttttttcct 43320
tacaatttat ttactgaaga aattaggtga tttgtctgta gactttccca tggtttggat 43380
tttgctattt gcttccattt ggggttaaca catttctctg tcttgtgtat ttcatgtaaa 43440
ttggtagttg tatctacagg cttgattaga tctgacttta gatttgtttt gagatgggga 43500
catctttttt tcataaatgt tattgtgagc cttcactagg aggcatataa tatcttattg 43560
tccgtctgtc tatctttttt gtggtgttaa cactattgat gatcattacc taggtctgtt 43620
agttagggat tgcagattgg tgatattcca taattcattt attagttgaa atgtatctat 43680

aaggacaagt tttttatcat caattatttg attatcttga ggtacaattt gattaggaaa 43740
acaagataaa tacttgcttc tcttttttaa tttaccagtt ttcaaaacaa tgaattggtt 43800
tcttgccatt ctccaaaagt gatcaattag atttgttttt tagtgtaatt aagaattcat 43860
ggatttaatc aaatttgatg tgtttcggtg aatggtagtt atccttataa atgtttaaat 43920
tgtcttattt gactaagggg acacacttca cattgtcttc tgagttcttt tgacaggatc 43980
ctaatcgtct ttcaatggct ttcttttcct agacctggaa tctgccatct ctctaatact 44040
gcttgtttct taaaggaact atttttgttt gggatatatt taaatatttt tttattgcta 44100
cacagtccac caaagttttt ttccattagt aggtttcaca aagctatttt aatggtttcc 44160
ttaatctatg aacagattgg aaaaagaaga ccctttagaa atagaatttt tattttttat 44220
ttgtatggta caatgtgttt tactgattag gtaagtcagt aggctttgaa aatttacttg 44280
accaaatata aggaaactat gtcattatca tggactagtg cagggagatt cagcagttgc 44340
taatctttta tgtaagtcag acatgagtgt tgctttggtg agaggattat agtaagcact 44400
gtgttaaggc tggtttgttc tccagtggac atatcaaaag gtgacaagtg aaactcttga 44460
ttggcatggt tcccttgtcc tggtaattag aactaatata tctgagcaac ttatctccaa 44520
aatttgggag ggcatagtgg catttttagt taaaaaaaaa atgacaagct cactgttatg 44580
gatggtgttt tagttattta ttgttatata acaaactaca tcaaaattta gaggcttatt 44640
ttgcttacag ttttgtgacg taggaatttg ggaagagttt gcctgggtga tttgtctctt 44700
aaccatgtgg cactaggaag attgttttgg ttagaggatc catttataca tggtttcttc 44760
actcacttgt ccggtatctt gaggtcctta gctcttcacc cccaaccccc aacccccaac 44820
ctggtggctc attttctagg gcatctccat gtggctgtgg cttctcagga tggtcccatt 44880
tctttttttt tttttttttt ttttgagacg gagtcttgct ctgtcgccca ggctggagtg 44940
cagaggcgtg atctcagctc actgcagcct gcgcctcccg ggttcaagcg actctcctgc 45000
ctcagcctcc caagtagctg ggattacagg cacgcgccat cacactggct aatttttgta 45060
tttttagtag agacggggtt ttgccatgtt ggccagtctg gtctggaact cccgacctca 45120
ggtgattcac ctgactcgac ctcccaaagt gctgggatta cagatgtgag ccactgtgcc 45180
tggccagatg gtcccatttc ttacatggta gctagctttc aagagacagg aaatggaagc 45240
tcccaggctg gttaagggca atcctcagag ctggcacagt gtcactttgc cctattctgt 45300
tgatgaaaac agttaagggg aaaatggact ctacctcttg gtgggggagt agcaaggtca 45360
cattgttttc atcagtgtga aaggttttct tcacattgaa gaagagcagg aatggatgat 45420
attgttgtgc ctatcgttgg aaaatacaat cttccacaga ccttacctct attcttcttg 45480
taatctgtgt accccaatca tactaaactg cctctgtctc tttgcctcat aaaccttgat 45540
aatttcttag ggctttgtca ggccaattaa aaaatgctgt attaatgttt aaatggcatt 45600
ataggaatag aaatagacta agaagcaaga acatttactt cagcctagat gttttattct 45660
gtcatttggt aaggtttttt gtgaggtaaa acgtgaagct acctctacca gtccttagtt 45720

agaaaggaat ctgtttggta ctgtttatga gtttttaaac ctacactagg ttttatttaa 45780
tggacaaaat ataaagaatc ataagtatca attttttttt tttttgaggc cagtctcaca 45840
ctgttgccca ggctggagtg caatggtgtg atcttggctc actgcaacct ctgcctccca 45900
ggttcaagcg attctcctgt ctcagcctcc tgagtagctg ggattacagg cacatgccac 45960
cacgcccggc taatttttat aattttagta gagacgggct ttcacgatgt tggtcaggct 46020
gctctcaaac tcctgacttc atgatccgcc cgcctcggcc tcccaaagtg ctgggattac 46080
aggtgtgagc caccgcgccc ggccacttct ggaactttaa aaaaatcttg aactcatcct 46140
gtagtttcaa gtagtattcc tttcccatca ccaaatgaaa ataaagcagg atcggccggg 46200
tgtggtggct cacgcctgga atcccagcac tttgggaggc agaggtgggt ggatcacctg 46260
tgatcaggag tttgagacca gcctggccaa catggtgaga ccccatctct actaaaaata 46320
caaaaattag ccaagcgtgg tggtgcacac ctgtaatccc agttactggg gaggctgagg 46380
caggagaatt gcttgaaccc aggaggcgga ggttgcagtg ggccaagatc atgccactgc 46440
actccagccc tggcaacagt gtgagactac caactcaaaa aataaaaaaa taaaaaaaat 46500
aaaaaaataa agaaggatta tggctcacat gtgcaaagta ataaatatag gattatattt 46560
ataagttagc actttgccaa atgctatggg ggacacaagg aaatagaata gaacaaatgg 46620
ccatctcttt gaaaaggctt agtttctctg tagcaggata ggacattgac aatggaaagt 46680
taagcaacat gacagtttga aaacaagtta tttcatggac aggtctttaa tttaaaaaat 46740
acaaggcaat aatacaagtg ctatctgagg tatggcctgg taaagtgatt aattgaatga 46800
ttaaataaat aattactgta gaaattcaga agataaagaa attaacctag atatagtaat 46860
cagtgaagat caagtggaga aagaggtaga acatggagct ggggtgtctt gagctggtta 46920
tgatttagga ccctgtgcct atgatgactt cctacattat atcacatcct ggatgttgga 46980
taatgtgata aggagacaac ctagtttcag gaaatggaaa aaaattgctg tatctagctt 47040
gaagtatcta cccccacaac ccagctttca gacactacag gtgcacttgc cactgactct 47100
tcagaacttg cttatgatac tcacaccagc atcctgatat tccccagtta agctaacata 47160
tttattttat cagagagaat agtgcttgtt gaacttgctt aaattgattt ttttttactc 47220
ttatttgaat aaatagatgt atagtgaatt ctgggatagt ttggtactag agttctctgc 47280
ctgctctgtg gaccctgtta ggtacctcag agagaatggt aagtggtggt gagagggctg 47340
ggtgattttg tagtgttttt tgtttttttc gtttttgaga tggagtctct ctctgtcacc 47400
caggttggaa tgcagtggca ggatcttggc ttacttctgc ctctgcctcc tgggttcaag 47460
caattctcct gcctcagcct cctaagtagc tgggattaca ggcatgtacc accacgcctg 47520
gctaattttt gtatttttta gtagagatgg gggtttcacc atgttggcca ggctggtctc 47580
gaactcctga cttcaagtga gccgcccgcc ttggcctccc aaagtgctag gtttacaggt 47640
gtgagccact gcactcagcc aggatttcat agtttaaaaa gtttaaaaag tcttccttaa 47700
aattaaaaca acaaatctgg ttgtagactg gcatttgtgg gaagtgattc tttcaaatac 47760

tgtattgttg gtgacatttc tgatgctggc tgttgaccaa attttaaaat tgtttgtgag 47820
tgaaataatt tcattacttt gttgtggata aacctgtggg gaaaggtttt gatgttattt 47880
tcaggcatat gttttcttct tggcatatac atagttaatt taacaatatt taaatatttt 47940
atcaaaaaac agattataaa attatacatt ttctaaatat aagagatcaa ttgctttcaa 48000
gttagaatct tctagtaatt tagagtgtgt gtgttcttat tttctgtgca gttgagaaag 48060
attgggggtg ggggctttgg agaaatttac gatgccttgg acatgctcac cagggaaaat 48120
gttgcactga aggtggaatc agctcaacaa ccaaaacaag ttctgaaaat ggaagttgct 48180
gttttgaaaa agctgcaagg taagcccttt gtagaatata caatttatga tttgtatgtt 48240
aatttataat ttatttgtat cttcctgaac cttaattaca ttctttaagt actaattgaa 48300
tgtactggtg tcagccgggt ataaatacgc tgagcatttt tctcttggaa attttataat 48360
gagttagtag tagtagaagt atgtaattac ttagaataat tgggctttgg gagttctttt 48420
tcatggagta aggataagag agtgtgaatt agcagaattt ttactgcttc ggaaagtatt 48480
gtcataagtc tgctatttgc ctttcttttt taaactacca ataacagata cctctgagtt 48540
gtctctactc aagtctttgg atgttaaata tgttttaatc acagtaagga agctgccttc 48600
aaatcacact gcaggatgga ttaaaaataa tactgttgat tatatgttga ttcatattag 48660
cttcagtttc agagaataac tgtttctggc cctgtcgtca tttttgaagt ccgttttact 48720
acttgggccc tatttgtttt tattacaaga atgaataatt ttttttctga catgaatcca 48780
gtggtataaa accctgaata tattgctagt ttgtgttcac ctttgtattc tcagcttaat 48840
gacatactct ctgatattac tgatgtttaa atgtttgatc tgaaacaaaa ttaaataggg 48900
atgatcattt tcctttgccc caagcgtatc caagtttctc taccagttgg ctcatttgat 48960
tagagaatga tgccagtaaa attatagatc tgtttttata gtaattttta gctcaaactt 49020
agcatctcca caacatggta aatcaggaaa acagtggata gattaacacc tattgtcaat 49080
actggaaaaa taaataattg gagaagaaag aataggtaaa aggatggctc tttctttttc 49140
tgaccttttc tattttctcc acatttctct tgagttgttg ttctcaactc agtggtgtag 49200
aggctacaaa caggtacagc cagagctaca tcttcctggg ttgcacagat cccaggatac 49260
ctgttttact atcagccaag cccatgaagg gtatgtcagc atcagcttgg tgtggatagt 49320
gccacttcac ctttctccta cttggctcca aggagatgct tccaggtgag gcaggataca 49380
gctttgccac cccttcagga ggttctcctg cttattttgt gtatggttta ctcaggattc 49440
tatttgataa aacttgaaga tcagtttagt accttttatg aatgagttag agacccttgg 49500
atgctagctg tctccttttc ttgtatttta tttttcacgt tattaactaa tatatttttc 49560
ctatctgctt ataaccacat gacatcccat tcttaagaac ccttcatttt ccaaaaataa 49620
attgattttt aatcttctgg tccctatttc agtgaggtag tccttgactc tcttctcttg 49680
gcttcctaac ttatgccttc tgcatactta ttcctcacag cttcattttg ttgggggtgc 49740
tttgtaatct ggaccgttct gataacattt ttatggagct gctcttgctg tcatgttggc 49800

ctaatgactt cttactgaat gatcccaatg aaaaggctga atgtaaggaa aaggtattta 49860
accagatgct aaccctttat aggatttgtt gatttttaaa ttgatgttca atttttgata 49920
gctgggataa ggattttttt tttttttttt gagacggagt cttgttctgt caccacactg 49980
gagtacagtg gcatgatctc ggctcactgc agcctccacc tctggggttc aagcgattct 50040
cctgcctcag cctcctgagt agctgggact acaggcatac accaccacac ccagctaatt 50100
tttgtatatt tagtagagat ggggtttcac catgttggcc aggatggtct cgatctcttg 50160
acctcatgat cttcccacct cggtctccca aagtactggg attacagaca tgagccactg 50220
cacctggctg cgattttttt ttttaacaag atgaaaagat attttatgat gaattagccc 50280
acccctaatt ttgttccttt taatagacca attcctcttc tcaaaattca gatattgtct 50340
gttctcacat tccctcagtt ctcaattttc tttctcgtag tcttttctgt acttaacaac 50400
cctagatttt ctcagttcag gcaaaactct cattactagt attttccttt ctctttgacc 50460
ctaaagtgtg aagcccttag catttcaccc catattttct gagtgacctt cccccatgct 50520
gctgtgtcag attactctct tttcaaagtt ttcagaaata ttctggatgt aacctgcaat 50580
tctctaatga atcaagctag actctcgaat tttattatta ttattatttt ttttttgtga 50640
tggagtcttg ctctgttgcc caggctggag tgcagtggca caatatcggc tcactgcaac 50700
ctccgcctcc tgggttcaag tgattctcct gcctcagcct cccaagtagc tgggactacg 50760
ggtgggtgcc accatgccca gctaattttt tgtattttta gtagagatgg ggtttcacca 50820
tgttggccag gcgagtaact ccagacttca gactgtcctc ctgtcctggc tttccaaagt 50880
gttgggatta caggtgtgag ccactacacc tggccaagac tcttgaattt tacttggttt 50940
ttacaaatgt atttgaatgt tttagctact tataaactaa attatctatt tataagccta 51000
ttagatagta cttctatacc ttattattat tattctctaa agtattccta ataagtatag 51060
ccctaaaatt tattaggcct atcctttgat tcacagcatt tttcagagat acatctctga 51120
ataaacattt atgtgtgtct cttcgccctc tccatttgca gctctactat cttcatcctt 51180
tgtttttcag tttatttgct ttgaaactgc aaaggtggtc attgccattc taatctgtta 51240
gattcctttt tcctcaactt taaatgaatg ttttgtaatg atatctacat atcttcactt 51300
ctccattttc tttttttatt acaaaattat tttgttgttt ttttctcagt tttcatgatc 51360
tcttatgctg tatctcaaca caccaggttt tttttttttt ctttttaata agtatagttt 51420
aggccaggcg tggtggctca tgcctgtaat cccagcactt cgggaggcca aggtgggtgg 51480
atcgcctgag gtcgggaatt tgagaccagt ctgggcgaca tggtaaaacc ccatctctac 51540
taaaaaattc aaaatttagc caagcgtggt ggcgtgcgcc tgtcatctca gctaccctgg 51600
aggctgaggt atgagaatcg cttgagccca gaaggcggag gttgcagtga gccaagaagg 51660
cgccattgta ctctagcttg ggcaatggag ccagaccctg tctcaaataa aaaagtatag 51720
atttgcagtg ccattgtaag agaaaaaaca cttctctcct ttgatttcaa atctgatcac 51780
tagtaatttc tacagtaaaa tgatggttcc tcaggaacca tcagtacttc tgaaaaagtt 51840

agatttccat tctagtctta atagaatttg gtacacatgt agaatcctga agtaagacag 51900
gcacatcttt tatttattta tttatttatt tatttattta tttatttttg agacaaggtc 51960
tcactctgtc gtccaggctg tagtgcagtg gcccaaacac agctcactgc agcctcgatc 52020
ctgtggctca accagtcctc tggcctcagc ctccagagta gctgggacca caggtgtgtg 52080
ccaccacact cagctaattt tttttttttt ttttgtagag atgtagagtc tggctttgtt 52140
gcccaggctg gtctcaaatt cctgggctca agcagtcctc ttgcctcggc ctcccagaat 52200
gttgggatta aaggtgtgaa ccaccacacc cagcctgaga caggcaaata ttaaatttac 52260
ttttttccct aatttttaaa agactttttt aaaaagagca gttttaggtt cacagcaaaa 52320
ttgagaggaa agtatagagg tatcctatat attgcctgcc ctcacacatg catgtactcc 52380
ctcagtatca tcatccccac agctgtacat ttgttacaat tgatgaacct acattgatac 52440
atcattatca cccagatccc atagtttaca atagggttca ctcttattga acattctgtg 52500
ggcctggacg aatgtgtaat gacatatatc caccattatg tgtatatagg tgctcctcaa 52560
cttatgatgg ggttatgtcc tgataaaccc atttaagttg aaaaattgta agttgaacca 52620
tcgtatgtca ggaaccatct ctctctattt ttactgccct aaaaatcctc tgtgcactgt 52680
ttccctgcct cctaaactct ggtaatgact aatcttttta ctttctcttt aattttacct 52740
tctccaggtg tcatatattt agaatcatac actattggct tctttcactt agtaatatgc 52800
acttaagttt cctccacatc ttttcatggc ttgataggtc atttcttttt aacatttaat 52860
aatattccat tgtctagagg taccacagtt catttatcca ttcacctact gagggatatc 52920
ttggttgctg tgcagatttt tgtgtggata taagtttctt acttgtttgg gtaaatacca 52980
aagagcatta ttgctggatt tgataataag agtatgttta gttttgtaag taactgccaa 53040
attttcttcc aaagtggctg tatcattttg cgttctcatc agctcatgaa tgagagttcc 53100
tgttgttcca catcctcacc agcatttggt attgtcagtg ttctggattt tggccatcag 53160
ataggtgtgt agtgacatct cattgtcatt aatttgcatt tatctagtga tgtgtgatat 53220
ggagcgcttt ttcatgtgct tatttgccat ctgtatatct tctttggtgg ggtgtcagaa 53280
gtttttggcc cattttaaaa tcaggttatt tgttttctta ctgttgagtt tttagaattc 53340
ttcaaatatt tggatatcag tcttttatca tataggtctt ttgcaaatat tttgaatttt 53400
ctctattgat tttctatgtt taatttgatt gatttctgct ctaattttta ttatttcttc 53460
tcttctgctt taatttgctt tctgttttct agtttcctaa agtaaaaact ttggttattg 53520
attttagatc tttcttttct tttcttttct tttttttttt ttttgagacg gagtctccct 53580
ctgtcaccca ggctggagtg cagtggtgcg atgtctcggc tcactgcaag ctccgcctcc 53640
caggttcacg ccattctctt gcctcagcct ccctagtagc tgggactaca ggcgcctgcc 53700
accatgcccg gctaattttt tgtattttta gtagagacgg ggtttcaccg tgttagccag 53760
gatggtctcg atctcctgac ctcgtgatcc gcccgcctcg gcctctcaaa gtgctgggat 53820
tacaggcgtg agccactgcg cccggcctag atctttcttt tctaatacat gcactcagtg 53880

ctataaattc cccttcaagc acctttttca gtgcatccca gaaattttga tgttatgttt 53940
ttatttagtg tgaaatagtt taaaacttct tatgatagtt ctttgactcc tgtgttattt 54000
agaagtgcat tgtattaatc tccatgtatt tcagaatttt tccagttgtc tttctaatat 54060
gatctctagt ttaattccac tgtgttctga gagcagacat tgtatgattt ctattttttt 54120
aagtttgtta gggtatgttt tatagcactc agaatgtgat ctgtcttggt gaatattctg 54180
tgtgagcatg agaatgtgta ttctgctgtc attggtttaa gtagtctata gatgtcagtt 54240
atatctagtt aattgatgat attgtttagt tcagctgtgt ctttactgat tttccggctg 54300
ctgggtctgt tcatttctga tagaagagtt ttgaagtctc cagctataat agtgcattca 54360
tctttctcct tacagttcta ttgtttttta cctcatgtag tttgaagctc tgttgttaga 54420
tgcatgcatg tttctagata ttatgagaca catagtatat ggcctcttag agaattgacc 54480
cctttatcat tatgtaatac tcttctttta ttattattac tattattatt aattttcttg 54540
gagatgtact cactaggttg ttcaggctgg tcctgaaccc ctgacttcag tgatcctcct 54600
gcctcagcct cccgaatagc tggggttaca ggtgcaagct actgtgcttg gctatgatgc 54660
ccttctttat acttgttcat ttttcttgac atacagcctg ctctgtcaaa ttaatatagc 54720
aatatttact ttcttttgag tattagtatg gtatataatt tacatccatt tactttattt 54780
gtatttacat acttaaagtg agtttgttgt agacaacata caattgggtt ttgttttttg 54840
atttagtctg tccagtctct tttatttggt acatttagac cattgatgtt tgaagtgatt 54900
gtttgtatag gtagattaat atctaccatt tttgttaatt gttttctatc tgttgtcttg 54960
ttctttgttt cttttctttt cttttttgaa atggagtctc actctgtcac ccaggctaga 55020
gtacagcggt gtgatcatag ctaactgcag ctttgaactc ctgggctcaa gtgatcctct 55080
tgctttggct tcctgagtaa ctgggactac aggtctgtgc tgccatgcct agctaagttt 55140
taaaaagttg atggatacag agtctcgcta tgttgcccag accggccccc aactcctggc 55200
ctcaggtgat cctcctgcct cagcctcaca aagtgctggg attacaaggg tgtgcacgac 55260
tgtgcccagc ctgttgttgg tttttttttt ttttttagta ttattatttt tgtcttccat 55320
tctttttctg tcttttgtag tttttgtttt tagatagagt ctcactctgt cgcccaggct 55380
ggagtgccag tgacgcagtc tcggctcact gcaacctctc ctcctgggct caagtgatcc 55440
tcccacctca gcctcccaag tagttgagac tacagatgcg tgccactgtg cccatctaat 55500
ttttgtgttt ttagtagaga cagggtttca ccatgttggc taggctggtt tcatgttggc 55560
caggctggtc taactcctga cctccatcaa ttgacccacc tcgaccgtcc atagtactgg 55620
gattacagcc actgtgcctg gccttctctc ctttcttaac atatcaatta tacttctgtt 55680
tttacttttt ttagtgattg ccctagattt tgcaatacag atttataact aatccaagtc 55740
cactttcaaa taacattata ccacttcaaa ggtaatgtga agatattttc tcgtaataaa 55800
atgatcctaa tttctccctc ctgtccttta tatcattgta gtcatcatat atattcagca 55860
tacataagca tgtatatata tttatatgca caaaatatat acataagcat acataattaa 55920

gtacattgtt gctattgtta ttttgagcaa actgttatct cttagatcaa ttaagcataa 55980
gaaaaataat ttattatgtt gccttcactt tttccttctt cacatctggg tttctaccct 56040
atattacttt ccttctctcc aaagaacttt taacatgtct tgtaagagaa gtctactgcc 56100
aaaaaattac ctcaattttt gtctgagaaa gtctatttct ccttcatttc tgaaggataa 56160
ttttgggggg tacagaatct atgttggtga gtttttctct gaacactttt tttttctttt 56220
tttttgagat ggagtttcgt tcttattgcc taggctgggg tgcagtggcg ctatctcagc 56280
tcactgcaag ctttgcctcc cgggttcaag ggattctcct gcctcagcct cccgagtatt 56340
tgcgattaca ggcatgtgcc accatgcctg gctaattttg tatttttagc agatatgggg 56400
tttttccatg ttggtcaggc tggttttgaa ctcctgactt caggtgatct gcctgctttg 56460
gcctcccaaa gtgctgggat tataggcgtt agccaccacg cctggccttc tctgaacact 56520
ttaatatttc actttgtcct ttgcctgttt taaaataggg tgatttgttt tttattgttc 56580
atttgtttaa gtttcttata aattctgatt attagacctt tggaggatac atagtttgca 56640
aataatttct cccattctgt aggttgtcta tttactctgt tggtagtttc taggttgctt 56700
tgtggaagtt ctttagttca aataggtccc acctgtcaat ttttgttatc gttgcgttac 56760
ttctggggac ttagccaaaa tgcctttgcc aaggccaatg tcaacaaagg tatttcctag 56820
gttttctttt aggatttttt actttgaagg cttacattta agtctttaat tcatcttgag 56880
ttaatttttg tatgtggtga aagatagggg tctagtttca attttctgca tatggccagc 56940
aagttatccc agcaccattt attgaatagg gaggcctttc cacatttctt gctcttgttg 57000
acttttgtca aagatcagtt ggttataggt gtgtggtttt atttctgggt tctctaacct 57060
gttccattgg tctgtgtgtc tgtttttgtg ccagtaccat gctgttttgt ttgtattgtt 57120
gctttatact atagttttaa gttgggtaat gtgatgcttt gttctttttt gcttaagact 57180
gctttggata tttgggttct tttttggttc catatgaatt ttagaatttt agaatttttt 57240
tttctaattc tgtgaaaaat gacattggta gtttgatagg agtagtgttg aatctgcaaa 57300
ttgctttggg tagtatggcc attttaacaa tactgattct tccagtccat gagcatggaa 57360
tgttttaaca tttatttatg tcattcctga tttctttcag caatgtttta tagttctcct 57420
tgtagagatc ttttacctcc tggcttagct gtattcctag gtgtttcatt tatttttcat 57480
tctttttttg gtggctattt aaacaggctt gtgttcttga tttgactctc agcaagaaca 57540
ttattggtct atagaaatgc tactgatttt tgtacgttgg ttttgtatac tgaaacttca 57600
ctgaagttta tcagttctag ggcccttttg gtggaatctt tagggtgttc taggtgtaga 57660
atcatgttgt tagtgaagag agatagtttg acttcttctt ttcctatttg gatgcttttt 57720
atttctttct gttgcctgat tcctcttgct gggatttcca atactatgtt gagtaggagt 57780
ggtaagagta ggcatccttg tcttattcca gttctcatgg gaaatgattc tagcttttac 57840
ccattcagta tgatgttggc tgtgggtttg tcatagatgg ctattatttt tattaattaa 57900
ttaatttatt tgtttatttt tgcaacagag ttttgctcct gttgcccagg ctggagtgta 57960

atggtgcgat ctcggattac tgcaacctct gcctccttgg ttcaagcgat tctccagctt 58020
cagcctcccg agtagctggg attacaggca tgcgccacca cgcctggcta attttgtatt 58080
tttagtagag acagggtttc ttcatgttgg tcaggctgat ctggaactcc cgacttcagg 58140
tcatctgcct gcttccgcct cccaaagtgc tgggattaca ggtgtgagcc accgcgcctg 58200
gccatagatg gctattattt tgagatatgt tcttttgatg cctagtctgt tgagggtatt 58260
tatcatgaag gaatgttgga ttttattgaa aaattttttt tgcatctaat tgagatgact 58320
atatggtttt tgtttttaat tctgtttatg tggtgaatca cattttttta tttgcatgtg 58380
ttgaaccaac tgtgcatccc aagaataaag cctacttgat catggtgaat taacttttgg 58440
atgtgctgct ggatttggtt tgctagtatt ttgttgagga tttttgtgtc tgtgttcatc 58500
atggatattg gcccgaagtt ttctcttttt attgtgtttc tgccaaattt tggtatcagg 58560
ctaatgcagg tttcatagaa tgaattaggg aggagccact catcctcatt ttttttttaa 58620
atagtttcag tcggattgat accagttctt tgtgcagctg gtagaattgg gctgtgaatc 58680
tgtctggttc agagcttttt ttggttggta ggttttttat tactgattca attttgagtc 58740
ttgttgttgg tctgttcagg ttttcacttt cttcctggtt taatattagg agtttgtgtg 58800
tttccaggaa tgtatccatg tcctctagat tttctagttt gtgtgcttag aggtgttcat 58860
aataacttcg gaggatcttt tgtacttccg tgggatcagt tatcatcctt gtcatttctg 58920
attgtgctta tttggatctt ctctttttcc ccctttggta atgtagctag tgctgtcaat 58980
cttgtttatt cttttgacaa attagctctt tatttcattg atcatttgta tggatttttg 59040
tgtctcaatt ttgttcaatt cttctctgat tttatttatt tattttcttt tgctagcttt 59100
gggattagtt tgttctcttt ttttttctat tctatgcaag atattagatt gttaatttta 59160
gatctttctg acttcttgat aaaggcattt agtgctacaa actttcctct taacattgct 59220
ttaactgcat cccaaatatt ttggtaagtt gtgtctctat ttttattaat ttcaatgatt 59280
tttttatttc tgccttaatt tcattgttca cacatgagtt attcaggact aggttaactt 59340
ccatgtattt ttgtagtttt gatagatctt tttggtattg atttctattt ttattgcact 59400
gtggtctgag agtgctaggt tttgattttt tgaatttatt gagacttgct gtatgaccta 59460
gcatgtggcc aatcttagaa tatgttccat gtgcagataa gaagaatgta tattctgtgg 59520
ttattgggtg gagtattctg tagatgtcta ttaggtctaa ttggttgagt gtcgagttta 59580
agtccagaat ttctttgtta gttttctgcc ttgatgattt gtctaatgct gtcaatgggc 59640
tgttgaagtc tcccactatt attgtggggc taagtctttt cataggtcaa gaagaacttg 59700
ttttatgaat ttggatgctt caatattggg tgcatatgta tttaggatag ttaagtctta 59760
ctgcattgaa cctgttatca ttatataata cccttctttg tccttcttga ttgttgttgg 59820
tttaaattct gttttgtctg atataagaat acccagcttt aggccaggct cagtggctca 59880
tccctgtaat cccagcactt tgggaggctg aggtgggtgg atcacctgag gtcaggactt 59940
caagaccagc ctggccaacg tggtgaaacc ccatctctac tagaaataca aaaagttagc 60000

caggcatggt ggtgcacttg ggaggctgag gcaggagaat cgcttgaacc tgggaagcgg 60060
aggttgcagt gagctgagat ccgccattac actccagcct gggcaacagg agtgtaaagg 60120
catttagtgt agcactaaat gccatctcaa aaaaaaaaaa aaaaaaaaag aaagaatacc 60180
ccagctaagg tttaattttt tgtggagatg gggtctcact attgcctgaa ctgaactcct 60240
gggctcaagg agttcagttg gtcttgagct cctgggctca agcgatccaa cccccttgac 60300
ttcccaaagt gttgggatac agggatgagc tactgtgcct ggctaatttt tgttgttgtt 60360
gttgttgttc ctctataggt aaggcttgtt tttcttttcc ctctagcttc attcagaatt 60420
ttttcttttt tttcaaactg cttcttcaga gtcagaattg tttctttatc tttgattttt 60480
gatagctaga aaatagtatg tttagatgta ggtttttggg gttttttgtt tgtttgtttg 60540
ttttgcattt atcccacttg gcgttttctg aatttcctgg atttgtggtt tggtgtttga 60600
tattaacttg gggaaattct tagtcattga ttcatatagt tacctgttcc tttctctctt 60660
tcctttctgg tgttcccttt gtgtatggtt gtgttatgtc ttttatagtt gttccttaat 60720
cctttcttct gtttctttgt tttctttttt tgtctttgtc ctctttgatt ttctcttttg 60780
gaggtttctg tagatacatc ctcaagctca gatacttttt tttttctcct cagctgtgtc 60840
cagtctacta ataagcctat aaaaggcatt catcatttct gttacagtgt tcttgatctt 60900
tagtgttttc ttttcgttct ttcttaggat tttcatctct gcttacatta cccacctctt 60960
cttgcatgct gtctacctta tccattagag ccctcagcat attaatcagt gttgttttaa 61020
attcctgatc tgattgtttc aacatccctg ccatgtttgg ttctgatatt tatttgcttt 61080
ttctattcaa attgtgtttt ttttgccttc tagtattcct tgtatatttt tcttgatggc 61140
tagacataat gtactgcata aaagcaaatg ccaaaaatag actgttagta atgtggtaat 61200
gggtggagag aggggaggca ttccatagtc ctattattag ttctcagttt tttagtgtgc 61260
ctatacttct ggactgtgaa gcacctccta agtgcttcta gtccccccgc accccattag 61320
gtgagacagg atagttagaa tgggttgcat ttgggtattt cctctccgcc agatccatta 61380
agcttcagta aaaccccagg ccaggtatgg tgcactgctc atgcctgtaa tcccagcact 61440
ttgggaggct gaggtgggtg gattgcttga gcccaggaga ttgagatcag cctgggcaat 61500
gtggtgaaat cccacctata caaaacatac aaaaaattat ctggacatgg tggtgtgcac 61560
ctgtagtccc agctactcag gaggctgaga ggtgggaaga tcacttgagc ccagaaggtc 61620
aaggctgcag tgagctgtga ttatgccact gcactccagc ctgggcaaca gagtaacacc 61680
ctgcctgata aaatacaaaa attaaaatta aaattaaata aaaaataaat aaataaaaca 61740
aactccagca ggttaggtgt agttaactta tttctcctga gggcagacct tgttaggaag 61800
aacagaatgc tgtggcatat ttaaaaatgg ttccttttct cttctccctg ctggaagcat 61860
gagggaattt ttctcctata tatactgtaa aaaactagtt gaactcctgg agataaaact 61920
cacaaaagca tgacttctgt ctaatatttc tgtaaatttc agggaacata cctatgacta 61980
ggtacccctg aagttttaaa gtattgggct tgttgacacc tagcctccag caatctgtca 62040

gttgcaattc aggtttttct actctggcac tggttcccgt gtatgattct gctctggtat 62100
gtcataactc tctgtgtctg cccatctctc tttccaattt tggaggcagc agtttgctct 62160
aagaagagtt ggtgattttt tcacttgttc ggctttgtac ttgttgatag aatggcatgg 62220
cagcttccat gttcctcact tgtggaactg gaaatgaagt cccttactct aattttttat 62280
tatgaaaaat ttaaatatgt accaaaatag aattatataa tgaacattca tgtatccaaa 62340
actcagctca gtcttaataa gcatcagcat tttggccaat attgttgcaa atatatccta 62400
ccccttcaac attttttccc cacgtttctg gagtacttta aaataaatta tagacgtcat 62460
ataatttttt gagtatgtat ctctgtctaa caaatggggg ctcctttgtt tttgtaatga 62520
aaatattctt tatagataaa tatactttgt tttatagatt tgtgttccta gtttctagtc 62580
agagaatctg tggagatggg gtagggggtg tttggagatg tatcaaaatt tggcagacat 62640
tactttgcct gtgagagctt agttgatgat tggtgatttt gtgaatccat taactttggt 62700
tttgttgttg ttgttgttgt tgttgttttg ggtttttttt tgagacaagg tcttgctctg 62760
tcgctggact gtgagctcgg ctcactgctg cctccacctc ccaggttcaa gtgatccttg 62820
tgcctcagcc tccctagtag ctgggacgac aggcatgtgc caccatgcct ggctaatttt 62880
tgtattttta gtagagacag agtttcacca tgttggccag gctggtcttg aactcgtgat 62940
ctcaggtgat ccacctgcct cagcctccta aattactggg attacagatg tgagccacag 63000
agcctggccc cattaacttg tacttactag agcatagaaa gtttttgcca cttatccatt 63060
gccaaaaaaa aaaattgtat gaaagttttt atgtaagaac gtattagaag tagctgtgtt 63120
aagcattagg gaaatttgag ggatctgtat tgttgaaaag taaaataata ggctgtagaa 63180
tttcctcagg tgaccagttt taagaatgga ttagagactc attggagagg aaacccacgt 63240
tctcagtttt tctttctttt aatgaatgga cttttttttt ttctttattt tagacaggtt 63300
ctcattctgt tgcttaggct ggagtacagt ggcacaatca tggctaactg cagtcttgaa 63360
ctcctgggct caggcaatcc ttctgtctca gcctcttcag aagctgggac ttcaggtaca 63420
tgccactatg cacaactaat tttttaaaaa aatttttgta gatatggggt tttgctatat 63480
tgcccaggct ggtcttgaac tcctggcctc acacaatcct cctgccttgg cctcccaaag 63540
tgctgagatt acaggcatga gccactgcac ctggccctgg actttacttt ttagaactgt 63600
tttaggttta cacaaaattg agtagaaagt acaaagttcc tgtatacaga cttcccatcc 63660
agttttccct attattaaca tcttgcattc ttgtgataca gttgttacaa ctgatgaacc 63720
aatattgata gatttttttt tttttttgtg agacagagtc taactctgtt gcccaggctg 63780
gagtgcagtg gcgtgatctc agctcactgc aacctctgcc tccaaggttc aagagattct 63840
ccttcctcag tttcctgaat agctgggatt acaggtgtgt gccaccacat ccggctgatt 63900
tttatagttt tagtagaggc agggttttgc catgttggcc aggctggtct tgaatgcctg 63960
acctcaggtg atcccccgcc tcagtctccc aaagtgttgg gatcacaggt gtgagccatc 64020
acgcctggca ttagattttt attaactgaa gtccacagtt tacattaggg gattcttttt 64080

cttttttcat aaaaaaaatt tttttttaag tagagacaag gtctcactat gttgagaact 64140
tgaactgctg gcctcaaatg atctttttgt ttcggcctcc caaagtgatt cttttcttaa 64200
taatgtgaac acagtggtat tgaaaaccta acaaaattaa ccgttatttt acaataccat 64260
ctcatatcta tccagtattt aatttctccc attgtctcaa aaatatcatt ttacaggtgg 64320
ctttgttcaa attaggattt atatacaagg caggcacatt gcattatatt ttgttgccat 64380
gtcacagttt gttctcacct actttttttt ttcccttgcc ttcacatttt ttttgttgaa 64440
ggaatgaagt tcttagtttg ataaaatagt ccgaaatctg gatatggctg attgcatcct 64500
tgtggtgtct tttaatgtgt tcttccatcc ttcatatttc ttgtgaactc ctaattagat 64560
ctagaggatt aatgagattc atgctgttat tattgttggg aagaatttat cacctacatt 64620
cattgttttc attagggatt gcaaaatggt ggttttccta attctctgat tccttctaaa 64680
ttccttaact ggaattattc tacaaagtag aatgttattc catcatttgt ttcccttgaa 64740
atatagttag tataggaagg acaggataaa gggattgagt tttcttctct tttgtttatc 64800
aattttcaga ataataagtt gtggtcttgg ctgggcatgg tggctcacgc ctgtaatcct 64860
agcactttgg gaggctgagg tgggcagatc acaaggtcag gagatcgaga ccatcctggc 64920
taacacggtg aaaccccgtc tctactaata caaaaaaatt agccgggcga ggtggcgggc 64980
gcctgtagtc ccagctgctc gggaggctga ggcaggagaa tggcgtgaac ccgggaggcg 65040
gagcctgcgg tgagccgaaa tcgcaccact gcactccagc ctgggcgaca gcaagactcc 65100
gtctctaaat aaataaatca atatataata aataaataaa taagttgtgg ccttatatga 65160
ccagtgattt gtttttcgtt ttgagtatca ttatgaactc atgccttttt ctgtatttta 65220
tttcaaacca ttgaaatcat tatccttttg aagcttaaat ttcgtatctt tcaccagtag 65280
aagctctttt gcattggctt ctgtgttctt cttgtataac tccagtattc tttgatgtca 65340
tccttgtttt ctggcacagc agagtatttc agatgttcct taaacttttt ctgtgccata 65400
ttcagaatca ggagccttgt ttctcctagt aactctgttt ccttgtattg ggaaacaatt 65460
gagaccacag tatgaccact agggtactaa ttgctattgg gctaacacac ttctctcaat 65520
aggttttaat aaggactgta ttttgccttg tttattatct tgtgcacttt tcaacttgtg 65580
ttatgatact taaaaaaata aattttcagg aggctgaggc aggagaatca cttgaacccg 65640
agaggcctga ggttgctgtg agccgagatt gcgccattgg actccagcct gggtaacaga 65700
gtgagactct atctcaaaaa taaataaaga aataaatttg tattttcttt taaattgtag 65760
gcctcttaaa aagcagagat tatgtatatt taaacttgac ttttatatta tgcttagtgg 65820
caaaataaca gcacaaagta tctttatatt caattatagc atacatttgt aaattgatgt 65880
gggaagatag gataacatac ctattccatt tctggttatt tacttcataa actttattgt 65940
tgattttttt tctgagtaag aaaaataaat actgaggttc tgtaataatt tgctgaagag 66000
tgctataaat actcgttgac agttaccttt ggatgactaa ggctccgagt cccaagaccg 66060
tgtcatatag acagtcaact ctttattata atggaagcca gtcccttgga tcttccagga 66120

aattttcatt tggtttaagg atttgaggtt tttttttttt ttctcttgca tataggttgg 66180
tctaatgttt taggaattca gaatattgta aaattcctat tacagtaagt cctcacttaa 66240
gtctcagtag gttcttggaa actgcaactt taagcaaaac aacgtataac aaaaccaatt 66300
gtttttcctc accaacgtta tgacagaatg actttgaact caataaggtt tttcaaggac 66360
tgaattacgt tacatgttgt tttgcttaaa gcccagaacc tctcaatgac attaaatgag 66420
gacttgctgt atttatattt agtgtatctt tttatcaaag gaaatcttct taggatcttt 66480
aaaagtttat acatcattta ctggcttcta attggtatat aaatatttac ttgagcataa 66540
caaaatgttt ttcttttaaa agtattctgt aggccaggtg cagtaactca tgcctctaat 66600
cctagcactt tgggaggcca aggcaggagg atcacttgag gcccaggagt atgagatcgg 66660
cctgggcagc atagcgagag accctgtctc tacaaaaaat aaaaaaatta gccagctgta 66720
atcatgtgcg ccggtggacc cagctacttg agaggctaag gtaggaggat cacttgagcc 66780
caggagatca aggctgcagt gagccataat tatgccactg tgttccaacc tgggtgacag 66840
agtgagaccc cgtgtctaag taaataaata aaaaaaaaat attctgtaga tctgatttct 66900
catttatttt ttatttttta tttttttttg agacggagcc ttcaactgtt gcccaggctg 66960
gagtgcagtg gtgccatctc ggctccctgc aacctccacc tcccaggttc aagtaattct 67020
tctgcctcag cctcctgagt agctgggatt acaggtgtgc tcgtttattc ttatgtgtgt 67080
tcgctccata gtatgttcaa tctgacgatg ttggttatat ttaaaaatca ctacttaagt 67140
cagtgatgaa tagaaagttg tttggccctt cttttccatg ttagctcttt atgctgcgag 67200
tgatcagcat catctcttgt actttttcat ttttcagtgt accatcctga gtagaactga 67260
tgtttttctg ttttttccct tggatgagaa ggtaattaat tgaaggatta tttaattatt 67320
ttaaattttt ctcctttcca tcatctaaag tgagtggagg tccaactaat accaaaaaat 67380
aacagaataa atgtcattgt ttgataatga aacaatttta ttctcagttg agatatttgg 67440
gaattaattt gtcttgcctc atataaattc aggaaaaact acatttttct ttataggtca 67500
tgaagaggaa atgcttttgc tttgattttc agggattttt ttttttaccc tgaaatactg 67560
gtcctggatt ttatttttgc cttttaggct acagagcaca agctgagagt tggttactgt 67620
tacaggagaa gtaactatta ctgttactgt tataggagag gaactgcacc tgttctgcct 67680
tccctggaga cttccctgaa gtctccagct actgctgcaa agtatgactg ctcctttttc 67740
ccagtcataa gaatagaaga aatatgtaat ttctggatta gacaactggt ttatccagta 67800
tagaagactg tgaaaattgc tccacaggga ccttttgtgg gagggcataa tagttacata 67860
ctgggacctg aaagattagg gatacatcct tacccttcat tctatccttt aaccttatgt 67920
aaacatataa tctactcttt tgtttatttc atcctctgca tttttcttct cctaaagtaa 67980
ctttttgctt gtccttgttg atgttcattg ttccaccaaa gggtgtatgg aggtctctga 68040
gaactatctg ttgaatacgt tcattttcac gtttttctcc ttggaagatg taacattatc 68100
tgtaggcttg aggattcgtt tactcattac tctgctttca tttagaaaaa tgacatgtag 68160

agaataggtc tagattattt taaggagcag aggtctattt ccttgaaatg gaatagaata 68220
tgtgtgtctt tgcctgaaaa cacacgtaca tatttatata tgctccatat gcacaaagaa 68280
catttggaat cacatataac tcaagaagcc cctgaggtga ttagaatcag aggagtcaga 68340
aaaggagaag atattttact ttttcccttt atacccattg tattgtaatg taattaaaag 68400
ctattttgtg ttgtgaactt aatgtataca tagttggtaa tggctgggta cagtggctca 68460
cgcctgtaat cccagcactt tgggaggttg aggtgggtgg atcacttgag gtcaggagtt 68520
caagaccagt ctggccaaca tggtaaaacc ccgtctctac taacaataca aaaattagct 68580
gggcccggtg gtgcatacct gtagtctagc tactcaggtg gctaaggcag gagaatcact 68640
tgaacctggg aggcagaggt tgcagtgagc cgagatcgcg tgactgcact cccgcctggg 68700
tgacagagta agactccatc taaaaaaaaa aaaaaaagtt ggtaatatct tatgtgtcgg 68760
gacatctata ctacttgttt gagtgctaac gatatggtag aacagtgcta ctgaatgtta 68820
ggctcgttga tactttatct tgttttagta tcattcatat ataactaata tccagttcgt 68880
gcataaaccc ttcctccatt tcactaataa acatttttaa gagtgtaaat ggtcacaata 68940
agaaacttga aaatcaacac aacagaataa agtgagaagt aagtttcctt taagcccctc 69000
actttcttcc ccactgttat ctactgttac cggtttcctg tgtatcttcc agaaatgttc 69060
tatgcatatt ataatcagct ctatatatct atgtatagat atatatatac acatatatac 69120
gcacacatat agaaaatcat atatctaaaa taatatataa tcatccatat gtttatatat 69180
gtatgtatat ataaaatccc cctccattac ccaccttcca caacaacata tggaatcaaa 69240
ctatatatgc tgttctgcaa ctcactgtgt cccttaaatt cagtcactaa gatttatttt 69300
ctattcacct gaatgttttg ctgcaagagt gatccttcta acatactaaa cagttatgtc 69360
actccccagc ctttagtaga aactattcat aatcttcttt ttctcatgta aagtccacac 69420
actgtaacat ggcatacaaa acactgaacc gacccctgcc tgcttcttct gctttttctg 69480
tcgaaaatgt tgggcttata cccagagtgc tggtcatcct gaactatttt cagtttcttt 69540
tctttctttt tttttttttt ctgagacaga gttttgctct gtcgccgagg ctggagtgca 69600
gtggcatgat ctctgctcac tgcaagctcc gcctcccggg ttcatgccat tctcctgcct 69660
cagcctccca agtagctggg actacaggcg cccaccacca cgaccggcta atttttttat 69720
tttttttattt ttttattttt ttatttttag tagagacggg gtctcactgt gttagccagg 69780
atggtctcaa tttcctgacc tcgtgatcca ccctcctcag cctcccaaag tgttgggatt 69840
acaggcatga gccaccgcgc ctggccatat tttcagtttc ttaaggctta cagcctctca 69900
cctgcaaact gcttcttttt tctcacactt tgggtaattc ctgctttttt ttcttctttt 69960
aaaaaatgta taatagtcct aatatttata aaagtccaca ggtcatacaa gattcaaaga 70020
caagaagcaa agtacaatta agaacttcac tttttttcac atagatataa tgtccaaaag 70080
tgtatatgct ttaaaaaaat tgtgattcta ctaacttgct ttttccctct tcctcaataa 70140
gattattaca ggatttattt ttaattttta attaagatcc aggttataca agtagtttaa 70200

aataatgagt tctacaaagt ttgttaagaa aaacagtagt ccaggttctg gttccagata 70260
agatggagta agcgcacttc actctgtctc tcccactgaa tgcagctaaa aacctgggca 70320
aaattcttgg agtggctatt tgaggactct gaaaagtaaa ttgtaggaga cagactgggg 70380
aagaagacca aaattcaaag taccattgaa ccagcagtgg gtttaccatt tttttcttcc 70440
agtatacccc agcctgaact taatgtatcc tgcaacttgg aagtgagcat tgacatggac 70500
agaaaatgcg tcgagagaag tcttctagtt ctgactcaag gaggaggaaa ggggtctcct 70560
aatgcccaga gaaagcatgg aaatcctaca tctttctctc tttttttctt ttctttattc 70620
tcttataccc gggcctcaaa cagtccttga cagtggctgc agtggcctca atgggggcag 70680
gcaagagcct aaaatagagg gagaggatcc ttcttctcag atgggaggaa ctgtggtctg 70740
aagagagttg ggaaaatccc cactgctttt tttctccctc tgccgttctg ttgcttggcc 70800
ctgcctgatg tgggcacagt catgggaagt actctgcaga gtggtgttag taaagcccca 70860
gagttctgct ggaagactgg aaaaggggag ccccagtgaa atgaaagatt gtagaagagg 70920
gaagaacttg gaacactgac cacctaaagt tgcttttgga ctcctgagct gacctccaat 70980
ctgtgcatgc atgggtccaa tcctaaacag catatatgga ctttgagaat tgaattatgg 71040
gatagaccac tgcccacatc ccaaatggga aaccacgtgg ggtacttatg gaacacatta 71100
aatataactg caaaggcttt gaaaactgaa ttgacattag aaatacaacc tacagaaggc 71160
cagtcagaac ttgaagcatg aactcaactt ggtcaattac tggctaaagc aaaaatacca 71220
acattcttgg tggaacttga ataagaccta gagtctcata acataatatt caaaatgtcc 71280
agaatacaac ctagaatcac tcagcttaga aagaatcaga caaatctgaa ttcatataga 71340
aaaagataga acatagatgc caatgctagg atcatacaga tgttaaaatt atattaaaaa 71400
gactttaaat cagccattat gactttgcta caagaatgag caggaagata gacaatctta 71460
gcaagggaat agaagatata aagaacaact aaatggaaca tttagaccca agatgtacaa 71520
ttacagacat aaaaaactca ctgaatgggc tcaatagcag aatggacatg atagatgaaa 71580
gattcagtga acttgaaaac agatcagtag aaatgatcta atctgaacaa caaagaaaaa 71640
acatgtaaaa aaaaaaacag agcctcagtg acctatgaga taatatcaaa acatcttaca 71700
tttgtgtcat cagcaatcca gaatgagact gagtttggtg cagcaaagat atataaagaa 71760
ataatgacat acaggctgaa aaggaggaaa taaaactgtt cctgtttgtg gatgacatga 71820
ttgtctatat agaaaatccc aagaatcata aaaacttcct agaataagtg agtttagcaa 71880
gtgatacaag atcaggccac aaaaagcaac catatttctg tgtactagca atgaacaatt 71940
gagaactgaa ttttaaaaag ttcagtaact ccaaaagaag tacatagtgt ataaatgtaa 72000
caaaatatat gccagatctg ggctgggcac agtggctcac gcctgtgatc ccagcacttt 72060
gggaggctta ggtgggcgga tcacctgagg tcaggagttc gagaccagcc tgaccaacat 72120
ggagaaaccc cgtctctact aaaaatacaa aattagccga gcatggtggt gcattcctgt 72180
aatcccagct acctgggagg ctgaggcagg ataatcgctt taacccggga ggtggaggtt 72240

gcagtgagct gagatcgcac cattgtactc cagcctgggc aacaagagtg aaactctgtc 72300
tcaaaaaaaa aaaaaagtat atgtatatat tctgtatgct gaaaactata aaaccctgat 72360
taaagaaata aaagaagatt taaataaatg gagaggcata ccatattcat taattggaag 72420
gttcactata gtaaagatgt tgattctccc caattgatct atagatttaa tgaaataata 72480
ataaaaatcc cagcagtact ttttgtagtt atagagaagc tgattctaaa gttaatatag 72540
aagggcaaag gacctagagt agccaaaaca atgttgaaaa agaagaagaa tgttggaaaa 72600
aaaatcaaat cacatagaga atagatttta agtattctta ttaaaaatgt ctttgtgagg 72660
catgttatta gtttgagcca ttccatcatg tatacatgtc ttcaaaacat catgtttata 72720
caataaaaat gtataatttc tgtcaattaa aaaacaccta gtgagacccg catctctata 72780
aactaagaaa aaaaaataaa ctcaacatgg ttgtacatgt ctgtagtccc ccgctgctcg 72840
ggaggctgaa atgggaggat tgccagagcc caggagtttg agtgcactat gattgtgcca 72900
ccatactcca acctaggtga caaagtgaga tcctgtctaa aaatataaaa tataagcaaa 72960
gaaactatat gattttaaaa cgtagtacag aactaggata atcaagactg tggtattggc 73020
aaagggatag acatatagat caagtggaat aaaatagaga ttttggacat aggctgaccc 73080
acacaaagaa ggccaataat aatctacaaa gttgcaaagg caatttggtg gaaaacgaac 73140
agtcttggtg ttggaactat caggcatcct tttacaaaag taatgaacct ccgcctaaac 73200
tacacacatt atgcaaaaat ttacttaaga tgtgccacac atctaaatgt gagatataaa 73260
gctgtgtaac ttttagaaga aaacatggag aagatctttg taacttgagt ttaggcaaga 73320
agcttgtcaa atgacaacaa aagcatgatt cataaaatta aaaaattgat aatttggatt 73380
tcattaaatt aaaaactttg cttccaaaag acattatgaa gagaatgaaa agacaagtta 73440
cagagtggaa gaaaaatatt tgcaaatcac ctgcaaagga cttattttca gaatatataa 73500
agcactctga aaactcaaca ataagaaaac aaacaattaa aaatatttaa taagctttta 73560
caggtacttc aaagaagata tagagtgtta aatcagttca tgaaaagatg tttaacatga 73620
ctaaacatta ggagaatgca cagtgagata ccactgcaca ccattagaat ggcttaaact 73680
gtacttctga ctggctgcaa cctggggctc ccttgattcc cccctccttg ggtttgatta 73740
acgtgctagg atggctcaca gaactcaggg aaacatgttt accagtttat taaaaaggat 73800
attgcaaaag atacagatga acagctggat gaagagatgg ataagtgtga ggtatggggg 73860
agtggggagc ttctgtgccc tctctgggta ggccaccccc ccagcacttc cacttattca 73920
gcaaaccaga agctcacaca tctcattgtt caagagtctt tatagagctt aatctctagc 73980
ccctcccact cacctttcct ggaggttggt gggtgaggct gaaagttcca atcctctcct 74040
ctaagcactt tgtctttctg gtgactgccc ctgtcctgat tatatagggg cttcgtccaa 74100
gttacctgat taacataaac tcaggtgtta tcaaaagagt aataaataac aaagacattt 74160
ctatcactca gaaaattcca caagttttag aaaccaggtg acaagtacca gggacagaga 74220
ctaaatatat ttcatattat agttggttgt tcctgtcagt gggttccaca tccatgcatt 74280

ccacatctgt ggatgcaacc aaccacagat tgaaactatt aggaaaaagg ctgggcatgg 74340
tggctcacat ctgtaatctc agcactttgg gaggcctagg tgggggtatt atttgagatc 74400
aggagtttga gaccagcctg gccatcagcc tgtctctact aaaaatacaa aaattagccg 74460
ggcatggtgg tgcacgcctg taatcccagc ttcttgggag gctgaggcac gagaattgct 74520
tgaacctggg aggcagaggt tacagtgaga tgagatcagt ccactgcact ccagcctggg 74580
tgacagagca gactgtctca aaaaaaaaaa gaaagaaaaa agaaaaaaaa ttaggaaaaa 74640
aatattgcgt ctgtactaaa tatgtataga cttttttttt ttcctgtaat tatttcctaa 74700
acaatcagta tggcaactat ttgcatagca tttacattgt attaggtatc ataagtaatc 74760
tagagattat atatagtcat gcatcacaca acagcatttt gttcagtgac agacggtggt 74820
cctataagat tataatacta tgtttttagt gtaccttttg cacgtttagg tacataaata 74880
cttaaccgtt gtggtataac tgtctatagt atgcaataca gtaacatgcc atacaggttt 74940
ctagcctagg agcaataggc tgtaccatat aggcctaggc atgtagtagg ctagaccatc 75000
taggtttttg taagcacacc ctatgatgtt tgcacagtga ctaaatcacc caaggatgca 75060
tttctcagca catatccctg ttgttttgca aagcctgact gtatatggga ggatgtgcct 75120
agcttacatg gaaaatagta ccccatcttt tttttttttt tttttttttt ttttttgaga 75180
tgcagccttg ctctgtcgcc caggctggag tgcaattgtg cgatctcggc ccactgcaac 75240
ccccgcctcc caggttcaag tgattctcct gccttagcct cctgagtagc tgggattata 75300
ggtgcctact gcgatggggt ttcaccaggt tggccaggct ggtcttgaac tcctgacctc 75360
aggtgatcca cccacctcgg cctcccaaag tgcagggatt acaggcgtga gctactgtgc 75420
ctggcaggta ccccattttt tatcagagac ttgagcattg gtggattttg gtatccaagg 75480
aaggtcttgg aaccagtcct ccatggatac caagggacaa ctgtatacca cagtggcata 75540
taaggtttta gcttactttt cttgtctgat cctacttccc acctcagccc accttaactc 75600
gtatcaagtc ctctagcctg tcagctctat taattattta tcacatgtac ttagatcctt 75660
tatgactttg tgggtgacct tttagaatac tcttctcaaa aacttgtaaa atctagtgat 75720
ttaatgctta tgtgaagcct cataagccaa atttttgaat cttctttata tccatagcag 75780
tttatgtata atattataga actcatactg tattactttt tttgtttacg tatcttggct 75840
gtgaactctt tgagggcatg gatcttactt aatttatctt gtatttctag tgcctagttt 75900
agtgcttggc atatagtagg tcgtcctgtt tctgttctgt cacagtcttt ctttcccttt 75960
tagttttaat acttatagac aaggtgttac atgattttaa aatctgtttc tgtcatttct 76020
gggcataatg gttaagagca aaggctctgg aattaaactg cttcatgttc tggttgtaat 76080
tttggacaaa gtaacctccc taggcctcta tttctgtatt tgaaaaatgg agaatataat 76140
aatacttaca ttattggcag gattaaatgc atgtaacatg cttgatatag agactggcat 76200
aaaatgcttg catattatgt gtgcatatca tgcttattta tttatgtatt tagagacagg 76260
gtctcactct gtcacccagg ctggagtcag tggcatgatc acagctcact gcagccttga 76320

cctcccagac tcaactgagc cttccactgc agcttcctga gtagctggga ctacaggcat 76380
gtgcccccac acctggctaa tttttgtatt ttttgtagag ttggggcctt gccatgttgt 76440
ccaggctggt aactcctgga ctgaaataat cctcccacct cagccttcca aagtgttggg 76500
attacaggca tgagccacta ctcctggccc atattatgca tgttttatta gccattattc 76560
tattcattta tttacacata ttcattgagc atttaaatca ctctttactg tgccaggtat 76620
tgtgggtatt gtgccaggta ctgttacagc ctagcaggtt cttcttgcct gctgcacaga 76680
aaaagccaat acactgagac tggtgttgca gcagagagtt taataattgc agtgtggctc 76740
attgaggatg atgtgtgata tttctcaaat ctgcctccct gagaatttga caactgggtt 76800
tttaaggata gtttggtggg cagggggcta gggaatgggc actgctgatt ggttgagttg 76860
gggatgaaat cacaggggta ttgaaactat cttcatgtgc tgagtcattc cctgggtggg 76920
ggacgcctgg gtggtgtcag ttggtccact aaaaaatatc tcaaacacca gtcttaggtt 76980
tcacaatagc aatgttatca gtaggggcaa ttgagaagtt acaaatcttt tttttttttt 77040
tttttttttt tgagaccaag tctctctctg tcgcccaggc tggagtgcag cggcgcaatc 77100
ttggctcact gcagcctcct cctcccgggt tcaagcgatt cttctgcctc ggcctcttga 77160
gtagctggga ctacaggcat gcgctaccat gcccggctga tttttgtatc tttagtagag 77220
atggggtttc accatattgg ccagcctggt ctcaaactcc tgaactcatg atctgcccgc 77280
ctcagcctcc caaagtgctg ggattacagg cgtgagccac tgcgcccagc cagaagttac 77340
aaatcttatc accaccagct agtgactcct gagtagtagg caattacaaa aaggcaagtt 77400
aggaaacaat gactagttat tatttatacc taaaccttaa cagaattcag gcccctaccg 77460
taatcccgtt tttggccttt cattagtttt ataaaagcag tttctgtcgc cagagagtgg 77520
gtttgttttg agaaggggcc attgtcatcc ttgctttaaa gttaaactgt aaactaaatt 77580
cctcccatag ccagcttggc ctgtgtgtag ggatgagcaa aggcagttag cttgtgatgt 77640
tagaagcaag atagagtcag ctatgttgga tgtctctcac tgttacagtt tttgcaaaag 77700
tgtttttagt accagaacaa gatagatacg gtccctgtac atgacagaaa agtagtctta 77760
agggatggat aatacaagga aaacactatt taacttattt tttattgttt ctttgaagct 77820
ggaggacaag agtaagttgc taatgaccat cattttatcc tttcattctt actacaatgt 77880
aatagataac actattttgc aaatatatgt aaaatacatg ttaagtgatc aacatgtact 77940
gtgtacattg tgaattattg tgttctccat tgcacccatt cttgctagct tataccccct 78000
ttataaacaa aaacatagag aatgagagaa cagtcttgta actggttgat aacacaggtg 78060
caaaaaaatg agtctttttt ttttttttag gggaaggggt cttgctatgt tgcccaggct 78120
ggtctcaaac tcctggcctc acgcaatcct cccaccttag cttcccaagt agctgggatt 78180
ataggcaaag ccactgcacc tggctttgct gagtcttaat tgtatttatg ttttaagtca 78240
gttaggaggc aaattttttt tagtgtttca tcagtccacc aaatcttcat cttcaagctt 78300
tggcctgtta ctcttcctta ctttcaagca agctccttca aaggctgtct gccctcatat 78360

ttcatattct ctttactctt taacccatta taaactggct tctgcccctt gactcactaa 78420
tgacttccgg tttatgttag tatttctcag cctttctatg tacttgtcaa tgtccacttc 78480
tgccctcata aagtgttctt ctcccttggt ttctgtgata cggctctctc agtttcctcc 78540
tatctttcag gctattcctc tatttccttg agggactctt atttttgctc atgctttgaa 78600
tgttgcattt tctagggcag gcattgtggc tcatgcctgt aatcccagca ctttgggagg 78660
ctgaggtggg aggattgctt aagcccagag tttgagacca gcctgggcaa gatattgaga 78720
ccctgtcttt aaaataataa taataattaa tgtttcattt tccaaattat gtgcttaagt 78780
ttcttttcct cttatattac attcttttac ttgtcaacct tccacgtatt gtccatgaac 78840
taatggttcc tcagtgtaca tctggagctc tgccttttgg tcttagcacc aggcctgaac 78900
ttacaatgtc tactgaattc acttgattgt cctaaaattc aacattttag tatcccttaa 78960
aatctataca gcctactata ttctcatatt ttgatacaac ttttcattta attttgccaa 79020
ttctagtaaa aaactacatc tttaatattg gtcagattta tcttatttta tctatctcta 79080
atccccttat tctttctcgt ggatattgta actatccccc agagtgacct tctccgctag 79140
aattgacacc cttgtggtct acccgcctca ttgccgccac agtgttttag tagttattct 79200
ttatcttaaa atcttttcga tacttctcat taccctgcag tagttttcaa atctctatcc 79260
atgccctacc tgctttagga tcatttaaag aacttttgag gcccggtgca gtggcttatg 79320
cctgtaatct cagcactttg ggaggctgag gcgggcagat cgcttgagcc ccggagtttg 79380
agaccagcct gggcaatatg gtaaaacccc atctctacaa aaaaatacaa aaatcagcca 79440
ggtgtggtgg cacacacctc tagtcccagc tactcgggag gctgaggtgg gaggatcagt 79500
tgagccctcc aggaggcaga ggttgcagtg agccaagatc ttgccactgc acttcagcct 79560
gggcagcaaa gtgagaccct gtctcaaaaa aataaaaaat aacaacgaac ttttgaaaag 79620
ctcatattct ttatctttct ccacagattc tgaatcaata tagaactgag gaatctttaa 79680
aaagttttcc actgccttcg ggtcagtggt gtcaaaaaac aaaaataaag ttttccggct 79740
ggttttcata agtagccagg gttgaaaaac actaaccaat atatgtggct caattctttt 79800
ggggggtctt tgcatgcttt tctttaatga gatttctgct gtacccctgc tcccctctat 79860
gccccagcca gcacattttt gggtttctca gatggaactc aggttgaaaa tcactgggct 79920
aatccacatc agtgaggccc agaaagattg tttgtcttgc tgaagtcata acaatgagta 79980
ccataacata tctgaaattc agggtttata ttcctaacat agttcttttt tattgctctt 80040
ctgtggcttc atatgtattg tatctgggca ggttgatctg aattgaccac ttaatctttt 80100
cctccctaaa taagttacca tacatttgtt tcctcattca tatattgttt atttattcat 80160
tcaaatagtc atttatttga ccacacaaag gaatagtaca gtgcctggga ggagaagagg 80220
taggtagaaa atttgaataa tataagtttt ctatccttaa ggttctcaag atgtatttta 80280
tattcaatca taaaacacta tacagaaaag caaattatta attttttaag agaaaccata 80340
gtatattctt ttccactgtt ttcttttttt atccagggaa agaccatgtt tgtagattta 80400

ttggctgtgg gaggaatgat cgattcaact atgtggtcat gcagttgcag gtaggttacc 80460
ttgaaaacgt atctttaaat tcttcagttt tgatcaaagt atacaattac agtgaatatg 80520
aataaaaagt aacaacaatc ttagtaccat gtgagaatgt tttatactat gcagatggaa 80580
ctgtatttaa acagacttga tacagatttt ccaaaaatga aataattgtc tttgtccgct 80640
atttgagttg acctgtatct ttactgattg tcatctttgc agttttcgat ccctgcatgg 80700
atgaggggat ataatgtgaa tcttgcccct tccacccttc attaccttag gggttccagt 80760
tttccatttg tattctaaag tattgtctta gtctgttttg tgctgctgca gaatacccaa 80820
gactgagtaa tgcataaaga acagagattt atttcttacc attctggagg ttggaaaatc 80880
caagatctag ggacccatat cttgcaaggg acttcttgct gcatcattcc atagtagaag 80940
ttggaagtgc aagtaagtat gcacattagc aaggaaggtg aagaaggggt cccagctcat 81000
cctttttatc aggaacctac ttccaagatt accccacttc cacaccaaaa tatgtctcaa 81060
aataagacat atatatggcc aaaaagcata tgaaagaatg ttcaacatca ctaatcttta 81120
gagaaatgca aatcaaaacc acagtaagac accagctcac accagtcaga atgactatta 81180
ttaaaaagtc agaaaataac agatgctggt gagatggtgg tgaaaaggaa acacttatac 81240
actgctggtg ggaatataaa ttagttcagc tactgtggaa agcagtctag agatttctca 81300
gaggacttaa aacagaacta ccattcaacc tagcaatccc attacagggt atatacccaa 81360
aggaataaaa tcattctacc agaaagacac atgcacttat gtgttcattg cagcactatt 81420
cacaatagca aacaagtgga attaacctta catgcccatc aatggtgaac tggataaaga 81480
aaatgtggta cgtgtatacc atgaaatact acacagctat aaaaatgaat gaaatcatgt 81540
tctttgcagc aacatggatg cagctggagg ccattatcct aagcaaatta atgcaggaat 81600
agaaaaccaa atactgcatg ttcttactga taagtgggag ctaaacattg agtacacatg 81660
gatacaaaga gggggacaat agacaccagg gcctactttg agggtggagc aaagaaggag 81720
gatgagggtc gaaaaactac ctgttggata ctgtgctcac tacctgtgtg acaaaatcgt 81780
ttccacacca aaccccagca acacacaatt tgcccatgtg tcaaacctgc acatataccc 81840
ccgaacctaa aataaatatt gaaaaagaaa aaaaccatag caagtcttgt ctacttgaaa 81900
cttaagcagt agttgagata gaggagaaat gccattcctt tttctttttc ttttcatcag 81960
taacataact ctgttccctg tatatttttt ttttgagatg gagtcttggt ctgtcaccca 82020
ggctggagtg tagtagtgca gtcttggctc actgaaacct ccgtctccca ggttcaagcg 82080
attcttctgc ctcagcctcc caagtagctg ggagtacagc tgtgctccac catgcccggc 82140
taatttctgt atttttagta gagatgggat ttcatcatgt tggccaggct ggcgttgaac 82200
tcctgacctc aggtggtctg cctgccttgg cttcccaaag cgttaggatt acaggtgtga 82260
gccactgccc ccagcctgtt ccctgttctt tagatggtag tggagttagg tcgagggagc 82320
agaggaaaga aattcagaaa aggaatattg gaataatggc tatagtgggg agaagaggga 82380
aacctttcta ttttttctga cactacctct gaccctgaaa acacacacat acacacacac 82440

acacacacac acacaccctg aattatagtc accctcaaag tttaaaaacc atggttcatc 82500
ttgatcttgc tgcctagatc tagaattctc agtatttctt ttcatcttaa gcaaacgttg 82560
ttttgggcat ttggtttctt tattttattt tattttattt tttgagacag agtctcgctc 82620
tatcacccag gctgagtgca atggcgcgat ctcggctcac tgcaacctcc gcctcccggg 82680
ttcaagcgat tctcctgcct cagcttctgg aatagctggg attacaggta gtcgccacca 82740
cgcctcgcta ctttttgtat ttttagtaga gacagggttt caccatgctg gccaggctgg 82800
cattaaactc ctaacctcag gcgatccgcc tgcctcagcc tcccaaagtg ctgggattac 82860
aggtgtgagc caccgcgccc agcaggtttc tttattttct ttcaagttat ctaaataaca 82920
gtgattctag agacatagat cctggcctaa atgcttcctt ttctcatcag gaggcttttc 82980
ttgtttccct cagccatcct tcagatttca tttagattta aagatactaa cgtctgtaga 83040
ctataatctt aaggtctttc tgagtcatgt gattcactaa ctcttgagcc agcctagttt 83100
tatttcctta agcagctatc cctgaacctt tttttttttt tttttttttt ttgagacaga 83160
gtctcactct gtcaccgggc aggagtgcag tggcacaatc tcggctcact gcaacttcca 83220
cctcctgggt tcaagtgatt attgtgcctc agtctctcaa gtagttggga ttacaggcac 83280
acgccaccac acttggctaa tttttatgtt tttaattaga gactgggttt caccattttg 83340
cccaggctgg tctcgaactc ctgacctcaa gtgatccacc tacctcggcc tcccaaagtg 83400
ctgggattac aggcgtaagc caccatgcct ggcctttttt tttttttttt ttgagatgga 83460
gtctcgcttt gtcacccagg ctggagtgca gtggcgtgat ctcagctcac tgcaaccttc 83520
gcctcccagg ttcaagtgat tctcctgcct cagcctccgg tgtagctggg actgcaggtg 83580
cgcgccgcca cacctgtcta atttttgtat ttttagtagg gacggtttca ccatgttggc 83640
caggctggtc ttgaacccct gacctaaggt gatctgccca cctcggtctc ccaaagccct 83700
gggattctag acgtaagcca ccgcgcccag cctctgaacc tttttagatc attaatttgg 83760
atcctgcttt ctctcatttt ggctagtaat aatataactt tttgtgtgac attttatagt 83820
tttcatctgg atttatcatc ttatttgatc ctcattatac ccccatgggg aggagagaac 83880
aagttttatc cctcttttgt acatggagaa gctgagtctc aaggaggttt gtgatttgtc 83940
tggtactagc ctacttgatg ggtattaaag cttggtggaa gcctgaaccc agtattcttt 84000
aggcagtaat aagagaaaac tgttttgtct caggttacta ttgactggag aattatataa 84060
agcagctttt tttctttagt ctgcagagtc tttctgagtc ttagcatata ttcattttca 84120
ttcttgtact tacctaaatc tgactttctg taaatgggaa ggctcctggt actgggaaac 84180
ctaagagact ggtgtatatt aggcaatgat gacctttgtg gcctttggca agttcctatg 84240
gatttgtcta agaacttttc catttgaatt gcatgctata tttttttcct gtcacaagaa 84300
agttaaccca tttaaactcc catctgtaca gtttgtggct gacttgattt gggattaggc 84360
taaagctggt ataaccgcta gagcatattt tctcttattt gacatatgga agataactac 84420
tgggtctcct ccatgtattc agcttcccac agagagtcct aatttattct aaaaaccata 84480

aattcattat tcttaagata aacttgttct tttcctcctg ctgattcaat tacataatat 84540
ctgccttaaa aatttttaag gaacaacctt atctctgttg tagaaattct atttgccatt 84600
aaaatgtttt ttaaaatctt acagagctca agcagttgta cacatttttt ttttctgaat 84660
tccccttaca agaccttagc caggtctcac ctagtatttt gtcaccactg aagctttata 84720
tgagcatctt tagtataacc aaagtgtgat atattgtgat aagtacatca actacagagg 84780
tgcaaaacat tttttcaagt gctgtaacca gtacaactgt aggtctagat tgcctaacac 84840
ctactacctt ttaaaagctt tttattatgg gaaaaattaa acatacacaa agtagagaga 84900
atagtataat ttacacccat ctactcatta cccaacttca acaactatca gtatatggct 84960
aatcttgttt caagtatttt ctctttctcc tgtccctccc gaaattaata gctaatccaa 85020
ggcatatttt atctatatat acatttctag aagacaagaa ctcttttttt tcttaaagta 85080
accacaatac ctaataataa ttccttaata ataacacaaa tatcaaacca atcttcagtt 85140
tcaatgatcg tccatctctt taataaatat tttttttttt acatttggtt tatttgaatt 85200
gagttccaaa tagggttcac acattgctta tctcttaagc ctcttttaat caattggttc 85260
accctctgtc ttttattttc ttgcaatttt cttttcattg tgtttattgt tgttgataaa 85320
aatggaggaa tttgacatgc acacattacc atattctgga ttttgctggt tgtttccctg 85380
tggttgtctt taatatgtat ttctgtcccc tgtattttct ataaattgat gattatataa 85440
gattccagtt ttactttgca aggatacttc agaggtgggt tgtatgcttc ctcttgaatc 85500
acatcaggag gcagtatctg gttgtctgtc tttcagtgag gttaagattg gtcagtgagt 85560
tcagcattgt cagtctgatc catccatttt aaagttacca gtggtttttc tcctaatggt 85620
tttatcaacc catttgtaat catcatccat tattttaatg ggggttgcaa atacagtgtt 85680
ctatcatttt attcttcttt tgtttactag aattcttctt caaaaccact acattcacta 85740
ttaggttatc ctcaagtata ttttatatag gaaaggcagg actactactt gattatttta 85800
tttatcagtt tttagaataa tgaattggtt ccctaatata aacacataat aaatatataa 85860
ctatataata aatccataaa ttcatggatt taacatgttt gatgtctttc aattattgca 85920
gttttatttt tttgagacgg aatctcactc tgttgcccag tctggagtgc agtggcacga 85980
tcttggctcc ctgcaacctc tacctcctgg gttctagcga ttctcctgcc tcaacctccc 86040
aagtagctag gattacaggc tcatgccacc atacccagcg aattttttta tttttagtag 86100
agatggggtt tccccatgtt ggccaggcta atctcaaacc cccaaagtgc taggattaca 86160
ggtacctggc ctacggtttt tttaatattc aaattgtctc atcttttgct acaggggctt 86220
ctttcatgtt ggatctggat tcattttgtt aggactaggc tgcagcagtc tttgatagca 86280
tccttatttt ttggtgccac actgagtacc agactcattc tgtatatttc atactccaga 86340
tctggaatca gccatttctc cgttaagcct tggctccttt taatgggaaa tgatgtttag 86400
agaccaaatt ctagatgatg tgggtgtctg ttgaaagtga gttggtcatt atttttaatt 86460
cttttcaagt ggatggattt aagaaataca ttttaaaaaa tgagaaatta tgccatggtt 86520

catatcgatg tttctaattc aaatttacaa tgatataatt gctaataact tctacttaca 86580
tttttatctc tttcctttta tacacccccc cccttttttt ttttttgaga tggggtcttg 86640
ctctggttgg agcacagtgg agtgatcaca gctcgcttcc gcctctacct cctggggtca 86700
agtgatcctc ttaccacacc ttcctgagta gctgggacta caagtgtctg ctaccatgcc 86760
cagctaattt tgttttgttt tgttttgttt tgtttttttg agacagagtc tctctctgtc 86820
gcccaggctg cagtgcaatg gcgtgatctc ggctcactgc aacctccacc tcccgggttt 86880
aagtgattct cctgcctcag cctcccgagt agcctggact acaggcacgc gctgccaaca 86940
cgcccagcta atttttgtat ttttagtaga gacggggttt taccatgttg gccaggatgg 87000
tctcgatatc ttgatctcgt gatccgcctg ccttggcctc ttaaagtgct gggattgcag 87060
atgtgagcca ccatgcctgg ccctaacttt ttaatatttt gtagtgatga ggtctcagta 87120
tgttgcccag gctggtcttg aactcctggg ctcaagtgat cctcctgcct tggcttccca 87180
aagtgttggg attatagatg tgagccacca cacctggccg acaattttta atgtatatag 87240
atgaaattat atggttagaa tttacatgaa aataattcag tacatgtggg tgggagggga 87300
gaggatatgg atggggaggt gaataataat gaaacaagac tggccatgaa ttgataatta 87360
ttaattctgg gtgaccagta gatacatgaa ggttcattat attatttttt ctccttttgg 87420
gtatgtttca tgtcttcaat ttagcaattc acttgcattt ttattgagat cttgtaaatt 87480
tatagagaaa tgaacttgct tcttttattc attccacatt actatatata gtcctcttcc 87540
tttttttttt tttttttttt ttacaaaatt tggtggcttg tgacatttta ttttatttaa 87600
aagattttta ttttaggttt gggggtacat gtgaaggttt gttacataga taaacatgtg 87660
tcatgggttt ttttttgtat gtaatattac atcacccagg tattaagctc agtacccact 87720
agttatcttt tctgctcctc tccctccttc aatcctcccc gtccagtaga ccccagtgtc 87780
tgatgttttc ttctttgtgt tcataagttc ttgattcttc attttttgaa tgactgcata 87840
tcagtcagta agatgactgt actattattt atgtaactat tcacttatta atgtacttac 87900
aatagtaccc ccttatctga ggagatactt tctaaggccc caagtggatg cctaaaacca 87960
cagatcatac tgaaccctgg acatctgggt aactgattgg gccactaagt gattaatggg 88020
caggtaacaa catgtggata cgctggacta aaggggtgat tcacgtcctg ggcctgacgg 88080
agcaggatgg tatgagattt catcacacta ctcagaatgg cctgtaaagt aaaacttatt 88140
aactgcttat ttctggcata ttccatttaa tatattcgga ccacggttga ctgtgggtaa 88200
ctgaaaccag caaagtaaaa ccatggataa agtaaaacca tgtataacca tggactattg 88260
tatagggtgt tttcggtttt tctttgtaac aaataataat tcattagcag tatttaaagt 88320
ataatttagt gcgtacctgc atgtttttac catgttaata acactttacc ccatactctt 88380
atcagttttg tgtttacctt ctcagatttt gtgagttgat atgttaggct tatattttaa 88440
aatatttaaa tcttttaaag tattactaat gtcataaata aaatttaatt gatttccttt 88500
tgaaagtttt tgtggtgaaa gctgtttaca aataaaattt caattttagg tatattttcg 88560

gcatcacaat tttgattttt gaagaaaaaa ctattggaat gctaataaaa gttattaggt 88620
actctcatgt ggttatacag ttcactgctg tagactgtca cttatttaac gttcgctaca 88680
ggtgcaggga acaacaaatt tccattaaaa ataataacta tggtaaatcc caattaattt 88740
atattatgag aaagctgcat ttaaaaaaga aggtacgaag tatgaaagga tcctgacttt 88800
actgtaactt ttaaactgtt acttgaggag tacttcgtat tttattttga ataatattaa 88860
tctaagagtt tattactttt caaaagtgaa tcaattgtct ttagatgaag tgatgatatt 88920
agtcacatag tacccctaaa gatatattat aaaattgtgg gaatagattg ggatatttaa 88980
atccccttgc gataggagag ccagtctttg ggaggtagtg ctgcagggcc atggtcctgc 89040
tctccttttc agattactgg tttgttacct aagcagcaag gaaccttggt ggagcagcat 89100
catgaaaaag tctgtagaaa agctctgtga aagaggactg taaaagaaga gcactttggg 89160
gtcagagtta gatttggaaa acagctttac attaacagat taacattatt taacctcttt 89220
gaacctcagt ttccttatct gtaaaacaga cacaggaaca gcttctattt agggtactgt 89280
gaagcttcag tgcaatcatg aatggcagac gctggatgta ttcattgctt ttatatactt 89340
ctacaagtaa attgaatgtt tgtcatataa tgtttggtca taataacaat agttagcata 89400
tgttgagtac ctgttgtgta ccacacagta ttctaaaaac tttatatggg gccaggcatg 89460
gtggctcaca cctgtaatcc cagcatgtca ggaggctgag atgggagaca aggagtttga 89520
gaccagcctg atcaacatag cgagactcca tctctattta aaaaacaaaa aacacacaaa 89580
aaaactttaa atataggtct tgtttaatcc tcacagagct ctgtgaggta ggttttatta 89640
gcttgttttt atgaggaaac tgattgaaga aagttaagta gcttgtatga gaatatatgt 89700
taggcagtaa tgaggaatta gtgccctttg tatgtatatg cattctatat acttgatcac 89760
acacacacac acacacacac acacacatat atacattttt tttttgagat ggagtctcgc 89820
tctgttgccc aggctgtagt gcggtggcgt gatcttggct cactgcaacc tccgcctccc 89880
tggttcaagt gattcttctg cctcagcctc ccgagtagct gggactacag gcgcgtacca 89940
ccacacccgg ctaaattttt gtatttttgg tagagacagg gtttcaccgt gttagccagg 90000
atagtctcaa tctcctgacc tcatgatccg cccacctcag cctgccaaag tgctgggatt 90060
acaggcgtga gccactgctc ctggcctgat cacatatttt aaataaataa atataaatgt 90120
aaataatata aattatatat ttgattactt atattttaaa taatcaagac ttttttgtta 90180
tgacttcaga gataaaggga aacatgaatc agaaacatga tatgctaaaa atctggtttt 90240
tctaaaactt gtctgaaaca ttcgtgtgat taagagatct gttgagtgag aggatcacaa 90300
tttgggtaaa attcttgcta ttggctgggt gcagtggttc atgcctgtaa tcccagcact 90360
ttgggaggct gaggcaggtg gatcacctga ggtcaggagt tcgagaccag cctgaccaac 90420
atggtgaaac cctgtctcta ctaaaaatag aaaaatttgc tgggcatgat ggcatgcacc 90480
tgtaacccca gctactcggg aggctgaagc aggagaatcg cttgaacccg ggaggtggag 90540
gttgcagtga gccgacatcg caccattgca ctccagcctg ggcaacaaga gtgaaactcc 90600

ctctcaaaaa aaaaaaaaat tcttgctatt ttgctgaaaa catcttttaa tggtaagtga 90660
gtagtttccc agtcttctct tatactatag ttttagtact gtatttgaaa gtgtttattt 90720
gatttccata gggtcggaat ctggcagatc ttcgccgtag ccagtcccga ggcacattca 90780
ccattagtac cactctccgg ctgggtagac agattttgga gtctattgaa agcattcatt 90840
ctgtgggatt cttgcatcga gacatcaaac cggtagggac cttttctatc cagagcacag 90900
aatgagtttg atacttttaa tgttatgatg ggacacaata aattacaaat gtagctttgt 90960
gtatttgctg tggtaaaata caaggtaata ttttcattcc aatagagacc tggaagcaaa 91020
aatataactt acctgttaat ttttttaagt gataattaca actacaactt attttcagga 91080
gtttttctca tacagtgaaa ataaaaactg agactggggc agtggcacat gcccatagtc 91140
ccagctactc aggaggctga agcaggagga ttccttgagt acaggagagg gagagtacag 91200
tgagctatga tcgctcctgt ggatagccac tgcactccag cctgggcagc atagtgagac 91260
cctgtctctt aaaaagcttt atgtttgact attattatgt gtgctaacat tgccagttac 91320
ttaaccttat gcctaaattg gcattattcc ctggtttctt ttatagatat attggttaag 91380
aaactatata tgttgctttt agttgttcat actgtgttct tgaactattt gttaagtcaa 91440
aaattataag aaatcatcag ttgtgaaaga ccttattttt ctcctgactg ctactgtgga 91500
aatatagtgg ggtttttatt tgctaatctg tttgtcttag aaattgtcat ttccataagt 91560
gtgaaatagg cttttttgtc ctttttggtg tttgattgtt cattaggtga aatgaccgat 91620
ttctgatata ccatatttac tataaatgta tcatatgttg gtacagttga aaagtataac 91680
cgtcagttgt tacatgactt ctaaaattag cttacatgaa aatgctaact aaatttaacg 91740
tagtagagtc agtctatttc tatgcatcat tatttgagaa ctatggtact tggaagagtt 91800
tctattttta tcagaaagcc cctaagtaga acaatattaa aacaaaacaa aaaaaactaa 91860
caattctggt aacaatttgg cttttaccgt cacaagtcct cttacttaca tactcatctt 91920
tgcatttgtg gtcatttaat atcacactga ttttcattag gggaagaata caaagtgtat 91980
tttccttaag tctgttttga aatgtttcta tagattgtat tttaattttg gacttagtga 92040
aattcagctt tatgatgctt ataggaaaat cttttttttt tttttttgtg acagagtctc 92100
actctgtcac ccaggctgga gtgccgtggc gcaatctcaa ctcactgtaa tctccgcctc 92160
ctggattcaa gcaattctcc tgcctcagct tcctgagtag ctggaataac aggcgcgcgc 92220
caccgcaccc ggttaatttt tgtatttttt ttttttttag tagaaacggg gtttcaccat 92280
gttggtcagg atggtctcaa actcctgacc tcgtgatcca cccacctcgg cctcccaaaa 92340
tgctgggagt ataggcgtga gtcaccgcac ctggctgctt ataggaaatt cttttaaatt 92400
tctacggggt tttttttttt tttggtccat ctctgatttc tcctatttta aaaagtaggg 92460
attgggctgt catttaagca ttaacttaga ctagaatttc aagaacttag agttccaagt 92520
ctagttaatg ttgtcttgtg tgtaacctta gtcatttaca tccttttttg ctcaaagttt 92580
acctgatatt aggcactaat agaacccaaa actaaatcat attttaatac cactaaacat 92640

catttactag aatagtatat ttggaacatg tgtgttatat agagcttaaa tagaagaaga 92700
cttaaaatgt aagaatgata aaatgtaagg actaagtatt ataagaatgt atatattttc 92760
cttctttatt tttctagtcg aacttcgcta tgggtcgctt tcctagtaca tgtaggaaat 92820
gttacatgct tgattttggc ttggctcgac aatttaccaa ttcctgtggt gacgtcagac 92880
cagtaagatt tttcatatta ttatcaaata tttgataatt accctggtat ttatagtatc 92940
acacttttta aacaatgcta attatttatc ctaatggatg ttatctaaga agtggcatat 93000
acatttaatg attaaattat agaatgacaa taaattttat tgttgaaatt tgtggcttca 93060
aaaaatgtat attagaggga gatttagagt ggtcttatag taagaacaca cattgaccct 93120
ttgagattag gggtcatttt tgcagtatga gaccctttct acatgctata attctatttc 93180
ggttattgta ctttttaatc cactcccaaa tatccacttc actgatattc tctatttggt 93240
gccacactgt catcacattt tcttttactt gtttaatcaa gctgtccttt atttctgaaa 93300
acatatctat aatggctact ttgaaatctt tttctgttaa atccaatatc tggtcactct 93360
cacaggcagt ttctgttgcc tgcctttttc cccctatgtg taagtcatac tttcctgttt 93420
ctttgcatgc ctcatgattt tttgttggaa actgggcatt ttaggtaata tattacagca 93480
actctgggta ctggtctccc ccttctaggg cttgttgtta ttttttgttt agtgtccgac 93540
aggatcattt tagtgaaatc tattccgttc ttcctccctc cctgccaacc atacacacag 93600
tattaagact ctgctgttgc tcttcagggt ggtgcgtctt tgggtatgac cacaattaca 93660
ctgtgatggt agtggtacca gtaaggctca cttctgctct ttccttggcc acatccagct 93720
gttgaacttc actaattgca agccagttac tttattgttt atgacaatat cttgggggca 93780
taaatacctt gactaattta atcaaattgt ggcttctaaa atggaatcat ttctgaagtc 93840
agtgtttgat cattgttctg acgtcaggtc cttcagctat cttttccccc tagttctctc 93900
ctgcccagct attctctaag ctgtatgttc agtgaatttc ctcccagttc cctttaccac 93960
aacctctatt tttgataggg cttttaggtt tgttaaaaag aaaacattag gcaaactaaa 94020
ttgaacagag tttagttgag caaagaatga ttcatgaatc aggtagcccc gagaataaga 94080
ataggttcag aatgactccg gggctgtcac atggtcagat aacatgtctg aacataaaaa 94140
gtaaaatgag tacagaaaat ttgaggtaca gaaacagctg gattggttat agctgggcat 94200
ttgctttatt tgaatctgtt ttgaacagtt ggctgatgtg gttggctgag acttggctat 94260
ttgttacaag agtagtttac agtctattta cacatcaagt taggttacca tcagtacctg 94320
taggccagcc ataaaacatg tacagaggca gttttaggcc aaacttaatt tagcaataaa 94380
accgtaaggc attgatgtca ctctgtcaat atcgtaaatg gacttatttg gtctccttat 94440
gggattcaaa tgagaccaag tcttatttgg tctcagctcc tgtctgtttt gggatctgtc 94500
tccttcttta aagttttggt ttgatgaaat taataataaa tctaaaataa aaaataaata 94560
taaaaataaa agttaatgtc atactttttt ggattgtggt tgaaataatt ttctcatatt 94620
ctactaatgt aaaatcaaaa gttcccattg tagatttgtt tcatatgtac aattatttat 94680

ttattgctag gaaacttgaa ttggttgttt ggactgtcta agtataccgt catgttatct 94740
gcagaaatct gtattttgat tttgtttatt ttgtcttgct atatatatat atattccaaa 94800
agagtgtaaa aaacattaaa aaattaagga agaataacaa aacatcactg gcctccatct 94860
aggttaagaa atggcatgtt accgtcttag aagcatgctg tgggtccttt cctgatcata 94920
tccctttttc ttcctcaagc agcactgaca aatacagctt actatgatga tagcaatgtt 94980
ctgtatctgt actgtcttat atggtagcca ctagctacat gtggctatag agcacttgga 95040
ttgtagccag agcaaatgag gaactgaatt ttattttatt ttattttatt ttttcgagat 95100
ggagtctcgc tctgtcgccc aggccagagt gcgatggcgc catctcgact cactgctacc 95160
tccgcctcct aggttcaagt gattctcttg cctcagcctc cccagtagct gggactacag 95220
gcgcccgcca ccacacccag ctaatttctt tttgcgtttt tagtagacat ggggtttcac 95280
catgttggcc aggctggtct tgaactcctc acctcaaggg atccaccctc tttggcctcc 95340
tgaagtgctg ggattacagg catgagccac cacacctgaa ctcaggccca ggaactgaat 95400
tttaaattca attaatttta agtggctatc atattaccca gtgcagccct tgacatactc 95460
tccattctat attttgtgtt aactaatttc tttgttttct ttcttctctc ttctgcagat 95520
cttgagggta cttgttttgg agttagggag cagatgcaga tagctgccat gacaggcctt 95580
catgtttagc ttataaattt aacccaatct acttctaatt tttttatcca gtgcctctct 95640
ccctcttccc tctcatattc ctttccctct gctttttaca tttattatta ttatttaaag 95700
agacagggtc ttgttctgtt gcacaggcgg gagtgcagtg gtaaaatcaa acccattgta 95760
gcctcaaact actgggctca ggtgatcttc ctgcctcagc ttctcaaagt tctaaaatta 95820
caggtgtgag ccaccgtacc tggcctcctc tgctttttta ttattttatt attattatta 95880
tttgcttttt cccctgattt gatgtatatt gttgtcattt tttattatta tttttcattt 95940
ctattttggg tttggggcac ctgtgcaagt ttgttatata ggtaaactca tgccatgagg 96000
gtttgttata tagagtattt catcacctgg ttactaagcc tagtacccaa tagttatttt 96060
ttctgctcct ttctttcctc ccaccctcca tcctgaagta gtccccagtg tacatcgttc 96120
cctttgtgtc tacgagttct catcataaca gatactgttt tattatcaca gtaaataccc 96180
tgttggctga tcaatatgta atagtagttg gtctgttaat tgaaaagatt cattaaggat 96240
gaagaatcat gacatattac tttaagcatt ttttatattt ttctttgttt tctaagtctt 96300
tttgttttca agtagttaag actcataaga agttacaaaa atagtacagt taccgtgtac 96360
cttcatccag cttcctccag tgataatacc ttacataatc atagtacatt gtcaaaactg 96420
ggaaattgat gttggtataa tattattaac tcaagtacag acttagattt taccaggttc 96480
aacatgcacg ttttggtggg ggaggggtgg tatatgaaat tttatcacat gtatagattc 96540
atataaccaa ctgctatagt ttgaatattt gtcccatcca aatctgatgt tgaaatttga 96600
ttccagtgct ggaagtgagg cctaatgggc agggtttggg tcgtgggatg gatcacgcat 96660
gaatagatca ctcccctccc ttgggagcgg aggggtcgtg agttctcact ctgttagttc 96720

ttatgagagc tgattgttaa aaagagcctg gcaccttccc ccaccttctt gcttcctttc 96780
acaccatgtg acctctgcac atgcctcctc tgctttgcct tctaccatga atggaagcag 96840
cctgaagccc tcaccagaag cagatgctgg tgccatgttt cttgtacaac ctgcatgcag 96900
aactgcgaac caaacagacc tcttctttat aaattttcag cctcaggtat tcctttatag 96960
caaggatgcc caatcttttg gcttccctgg gccacattgg aagaagaaga attgtcttgg 97020
gccacacata aaatatacta acaatagctg atgagctaaa aaaaaaaaaa atcacacaaa 97080
aaaaataatg tttaggaaag tttgtgaatt tgtgttgtgt tgtgttcaaa gccatcctgg 97140
gccacatgtt gggcaagctt gttttgtagc agtacaaatg gactaagaca tcaacaccat 97200
aatccagata caaaactatt ctatcaccac gaaagaactt cctcaaaaca taaatgtata 97260
tttatttacc aatcttgtgg tataggacca tcagtcagaa ggcattacag agggaatgat 97320
ggagtgtatc tgttatacca gtgtggtgtt tacgaagttg gttttgacag cctctactct 97380
ttatcacctt taagatgtat tagttacatt aagtagggtt gtgaggggca aagaagtcct 97440
aaactatata tatatatgca acactttgat tcataatttc atagttgaac ttctgtttac 97500
ctaatgattt gaatctgcct gttcattttt tgtatgtatc ctgcttttaa ggcattcccc 97560
agtttcagcc cttcagctgg catctcacac tggccctttg tgtctaagct cttggagtga 97620
catgttttct cgttgctccc ttcctaacca ttcacctgaa acttccagtg cctaggattg 97680
tagtaactta cataagaaaa atgtttaaag tctttttcct ctgttagtat ttcagaattt 97740
agctgaattt gagatatttt cttaattcca ttgggagtag ttgtattaca tagttctcct 97800
tgaatttttt cccctgatcc tttccctttt gttagcaata ggccagggct cattcctaga 97860
gtcattctct tctttcttga tttattctcc taggagttca tcccatctct aacatacatt 97920
ccctccttta cattcacact gcttctgtcc ttgtactaat tttgttgttt cttgttttaa 97980
ttgttaaaat agtttcctca ctcatctctc tgtcttctcc tttcaggcta tctgttatac 98040
ttccaccaga gtcaacatcc atccagagca ttttctcaat tgttactttc cagcttaaaa 98100
atattttatg aattccaatt acctactgtt ataagccaaa ttaaagaaga ccttaatttg 98160
acatttaagt ccttccttaa tctgacttca acctattttt ctgttttttt ttctctcatg 98220
gtctgctgca gtcatatcag atttgtttct ctaacaccaa cagtactttc ctccctcttt 98280
gcattgttca ctatgttctt tttacttaaa atgcatctca ttttttctgt tgtaattctg 98340
tcagcccttc aaggcccggt ttattttttc tttttgtttt tcatttgaat atctaggtat 98400
caaataaaat atgctcatag attgttaaat actttcactt ttttactttc tttttctttc 98460
aaaatgtttt taaatataaa tttcaaatag ataatatatt catgtgatgc agaaattaaa 98520
gaattaaaaa tatatcaaaa ctaaacactt atactctttg aaagacattg ttaagcaaat 98580
gaaaagacaa gtcacagatg gggaaaaata tttgcagaac gcacatctga taaaggtttt 98640
atatccaaga tctgtaaaca actcatacaa ctcaataaaa agaaaacaac acaataaaaa 98700
tggactaaag atttgaatag acattttatc aaagaagaga tacaaatggc aaataagcat 98760

gttaagagat ggtcaatatc attagttatt agggaaatgc aaattaaaac cctggcaaga 98820
ggctgggcat ggtggctcac acctgtaatc ccagcacttt gggaggccaa ggcaggcaga 98880
tcacgagatc agaagattga gaccatcctg gctaacacgg tggaactccg tctctactaa 98940
aaatacaaaa aattagctgg gtgtggtggc gggcaccctg tagtcccagc tacttgggag 99000
gctgaggcag gagaatgggg tgaacccggg aggtggagct tgcagtgagc caagatcacg 99060
ccaccgcact ccagcctgga gacagagtga gactctgtct caaaaaaaaa aacaaaaaaa 99120
aaacaaaaaa caaaaaccct ggcaagatac cactgcctac cagttagaat ggttaaaaca 99180
aaaaccagac aatgctaagt gctgacaagg gacagaaatc attggttgtc agagactagg 99240
ggtgggaggg attgactcaa aggggcatta aggaaactta ctggagtgat ggaaatattt 99300
gatatcttag ttgtgatggt agttatatga ctttatgtca gtcaaaactc atagaactgt 99360
atacataaag tatgaatttt actgtaagta aatgatacct caataattaa aagtaaagca 99420
aattaaacct gacttgaaaa taaattaaaa agttaacagt gaaataaatc tccatttcac 99480
ctctgcctat tccaaatcta ctcatttcct atactgtctc tgattcttcc ccctgctccc 99540
tacattcacc tttaggtagc tgtactgtta ttagtttctt taatatattc ttccagaatt 99600
tctttattca catacaataa atacaaatga acattcttaa ttttcccttt ttgattcaaa 99660
aggtagcatg atatacattc tatacctttc tttttttcac ttgtcagtgt atgtagacga 99720
tctttccatg ttagtatata gaacttcctc attctttttt tttttctttt agacaaagtc 99780
tcactctgtt acccaagctg gagtggagtg gcacgatctc agctcactac agccatggct 99840
cagcagtcct cctgccacat cctcctaaat agctggggga ttctcccatc tcagcctccc 99900
aagtagctgg tagctggggc tataggtgtg tgccaccatg cccagctaat tctttaattc 99960
tttttttttt agagacgggg gtctcactat gatgttgccc aggttggtct tgaactcctg 100020
gcctcaagtg agcctcctgc cttacactcc ccaaattctg gaattatagc catgaactac 100080
tgtgcctggc ccgacccctt tttttttttg cagcttcaga gtgtattcca ttatttgggc 100140
atactataat ttatttaatc agtgtctcct attggcagac atttaggttg cacaaaatct 100200
tttgctgtca caaacactga tggtagtgac tgactttgta catccactat ttttgcacaa 100260
atgcaagtat atctgtgaga tagatttcca gaagtgaaat tgtgagacaa agtgaatata 100320
tgcatttaca attttgataa atattgctga agtgccatca attgaggaag tttcaagaga 100380
ttatttgttt caagacccag taaaatgctg cctattccta agacttgtcc ttcctctctc 100440
ctactacatg gaactaatcc ttacctgtcc cgagtgttta tatttcattg tgcttcacta 100500
atgatattca ttataatctg ctttaaattg gaatcatttg catatgtgac tgtttttctc 100560
cagttgggtt tgagttgtct ttactacatt aaataaggtc attgaatttc cttgatctga 100620
cattatttct ttttcttatt agaattagat gagatatgca aactggtatt atttctttga 100680
ctacttatgt caagatttct ctaaacagct gatttctgtt ttctctattt cagtctttct 100740
atggcagaag taaggtcaga gccaggcagg ggaaactctt aagtagaaag agtctcctgg 100800

gcttgacttg gctagaatcc atcagggaga agtctagatg cccaatcaac tgtggactag 100860
gctgcagaat gtgataataa gtctggagat tttcattctg acagttactg gaatgaaaat 100920
aattgggcaa aattacacaa cacaaaaaaa ttctatgtta gctgaagggt catgtcaggt 100980
aatgaataaa agtacaacct ctgaaacgtt atttaccaaa aaagattcat agtagacttt 101040
catatattct cttagacaat tttcttctaa aaagagaact ctccaacctt agttccatca 101100
atcaaatgga gaatattagg tattcctgtt aatcatgatg tcttaggaaa atattgagat 101160
ccactgacta tgattgtcaa tgaatgacag ctaagaaaat aatggggatt tttttaaaga 101220
ttgcaggaaa ttttaattac tgagacttgc cattaaatac agaatatttt aaaatagttg 101280
tcttaaaaac caaacaaaag taaagggaaa gagagtattt aatgaaggaa gaaatgaaat 101340
agctaacttg attaaacata gattataaaa atgagcaagt ggaggaagaa cacatagatt 101400
gtttatcctg tgcaataaca tacaaccaaa ttattctggt aaggactttt tttttttttt 101460
tgagacagag tctcactctg ttgcctggct aatttttgta tttttagtag agatggggtt 101520
tcaccatgtt ggtcaggatg gtgtcgatct cttgaccttg tgatctgccc gcctcagcct 101580
cccaaagtgc tgggttacag gcatgagcca ctgcgcccgg ccaggatttt taaaataacg 101640
tttactatct tttctgacta caaaagtaat acatgttaat tgagaaaaat ttgaggttac 101700
ttaagaaaaa aaaaacccaa aaattatact aatgaaaagt gtgtctatga tcattttcaa 101760
gtttggcagt tgtcatattg agctacagtg ccacccacga atcgtcaggg tactgacttg 101820
attcttgtgg agcagtagaa tggagacaat gtttgtttgt ttgttttgtt ttttgttttt 101880
tttttttgag acggagtctc gctctgtcgc ccaggctgga gtgcagtggc tcaatcttgg 101940
ctcaccgcaa gccgagatcg caccactgca ctccagcctg gttgacagag cgagactccg 102000
tttcaaaaac aaacaaacaa aagtaataca ggttgaatat cccttatttg aagtgttttg 102060
gatttcagat tttttgtttg tttgtttttt gagacggagt ctcgttctgt cgccccggct 102120
ggagtgcagt ggcgcgatct ccgctcactt taagctccgc ctcccgggtt cacgccattc 102180
tcctgcctct gcctcccgag tagctgggac tacaggcacc tgccaccaca cccggctaat 102240
ttttttgtat ttttagtaga gatggggttt caccgtgtta gccaggatgg tctctatctt 102300
ctgaccttgt gatctgcccg cctcagcctc ccaaagtgct gggattacag gcgtgagcca 102360
ccacgcctgg ccgaatggag acaatgttga aagaccttag ttaatgcctg gttttactat 102420
ttactaactt ttgtgtcctt agacaagtta tatacttctc agagcctata tctatatttg 102480
taaaatgagc aagacaaaga cctcgcccgc acagagttta tattctatag actaaaagaa 102540
gacaggcagt aaacaaatta aaatagatta gccctatgta gtggcacatt ccagtagttc 102600
cagctacttg ggaggctgag gcaggaggat cacttgagcc caggaatttg aggctgctgt 102660
gaactatgat tacaccactg cactatagcc tgggctacag agcgagacct catctcttaa 102720
aaaaattaaa atctaaacaa atgggcaata aacagaataa gaatataaat aaaattgatt 102780
tgggggtagt aaaggtgtta taagaaagaa agaaagcaga gtaaaagaat agagtggcac 102840

cacagatggg ggagcagtat tttagattag gatgattaag gaaagtttct ctcaggaggg 102900
acatgtgagc agaagcctta aaatggagta gaagagctag ccattggagg gattcaggga 102960
gctggcattc cagctggaga gaatcgtgtg cactgttttg agctaaaacc aaaattggtc 103020
atgaaagaaa ccatggtaaa tccattgtgg aaggagacaa atgccagatc atgaaaagtc 103080
ttgtaggcta agtaaaaagc ttggacttta ttctaagttg agttctctga ggactaggat 103140
catgtatttt tcatgttggt gttatttcta gtatgtggca catactaagc atccgataac 103200
ttgaattttt aatacatgaa ttgcatatgg aggtagggta caagttgcca ccacctttga 103260
attgagtttg aatgaaatag ccaagatatg aagagcttgg tatctcaggt ttacttagtg 103320
attcctcacc atctaaatat atttatttgt atcagaacct gaatattaat gtatatacta 103380
tgatacttac cacgattatg ggtatatgaa agcatttaat gttttctaat atagagaatt 103440
gtatagatgt cataaatgcc tctgtatttt aaaatgtagg taggaaaaaa ctggactgaa 103500
aattaaaatt ttatcagttt ttttttttaa agatgtgatt tagagataat ctctctagtt 103560
tcttgtaatt tcatagttaa aagttagaaa ttaaatagtg ttttattgtg acaataaagg 103620
acgtgtttct tccatttact tcatggttca agtgtgctga atgtattgct gtttgaagct 103680
catttattat tctttgcatg gcttcgttgt agcctcgagc tgtggcaggt tttcgaggga 103740
cagttcgtta tgcatcaatc aacgcacatc ggaacagggt aggtatctga actagagtca 103800
tatctgtata agtatgctaa tagcttaaat cagattgact tcttttttat tttattatta 103860
ttttatatat atatatatat gtatatttgt tgagacagag tcttgcactg tcgcccgggc 103920
tggagtgcag tggcccgatc ttggctcacc gcaacctctg cctcccgggt tcaagtgatt 103980
ctcctgcctc agcctcccaa gtagctggaa ttacaggtgc ccgccaccac gcccagctaa 104040
ttttttgtat ttttagtaga gaaagtgttt caccatgttg gccaggctgg tctcaaactc 104100
ctagcctcat gatcacctgc ctcagcctcc caaggtgaga ttgacttctt tacaaagtac 104160
aaagatgtgg aaagtgcttg agagttgagg atccctttaa tagctacaaa gttgcttgga 104220
atgttcgtcc taattcattc ttgtgtggca ctaatgataa tggaattggt aatactaatg 104280
cacggtatag tgtatcgtgg ataatagata tgtgcagctg cttggaaaat aaaattggat 104340
ttgccagaaa agacgatagc taagtatatt tatgtctttg ggaaatctgt gtccttaatt 104400
agatataagt attaacaaat ttaatataga agtttgggag gctgaggtga gcagatcaca 104460
aggtcaggag tttgagacca gcctgaccag catagtgaaa ccccatctct actaaaaata 104520
caaaaattag ctgagcatgg tggcatgtac ctgtagtccc agctactcag gaggctgagg 104580
caaaagaatc actcgaacct gggaggcgaa ggttgtggta agctgagatc gcaccactgc 104640
actccagcct gggcaacaga gcaagactct gtctcaaaaa aaatatacac atatatatat 104700
atatatatat atatatatat atatttattt atttattttt atatatatat atttttatat 104760
agagagaggg agaatttgga agagattgaa catctcttcc aaatttgaga cagagcaaga 104820
ctccgtctca aatatatata tatatatata tttttttttt aaatatatat atatatattt 104880

ttaaatatat atatattttt aaatatatat atatattttt aaatatatat atatttaaat 104940
atatatatat agagagagag aatttggaag agattgaaca tctgtgtact atttcaagaa 105000
attatttggg aatataatac tatcaatacc taatgaaatg tgggagtcat cattttatct 105060
gtaatatgta tctaatctga tatccctctc cccaccaaac cttttgtttt catgaagatt 105120
tcaagtgcag acaattttaa aatgtttgtt ttagtaaatt atccctttcc tcttgggtta 105180
aattttaaaa atatctcccc tcaccccctt actttggtcc ctttgttctg cgctgacttt 105240
ttccccttca tcccgaactt tatgagtagg aaaactgggc atttttatgg attatcagtc 105300
ctttaacttt tttgcatgga gttgggaaac tgagcaagtg attccttgat aaaataggga 105360
aaaaagtatt tcttgttgat tcttttcttt accttgttcc tgttctgata agccaaaatg 105420
agaacttgac caatatatga taggaagagt atactgtggt cttaaatgct atgctgcagt 105480
taatttggct tcttatggcg tgattataaa ttgaaaatag taaagctatt tttctcggtg 105540
tgtagcactc tgctaagcat ttaaactcat ttggtttgat tcagtgttgc tttatttcat 105600
gatatctagt catttactta tttactttgt cttgaaaaga agctctcttc ttttcctgat 105660
agattcttac aaaagtttag aaatactagg ttttccagtt aattttatta aagaattatg 105720
tcaacattgg aatctgtgaa aatacattat ttttaagata aacataagta ttttcaggga 105780
ttctaatagc tacataagca ttttcaaaat agctaataga gattatgtac ttttactgtt 105840
ttaatttaga cagtggcaat ttcttctaaa agcctttcca gcacagtgtc ctctaacagt 105900
tactttgata acttgcttct ttgttactca tctaaccttg tagaggaatt tgatagtata 105960
taacagtggt tcttaaccct cttttctgct cccagcacac ctgaggggta gaatgctttt 106020
actcagtaac atagttcaca acgattgtta gtttgtgagg tgatgcagga gtgcacctct 106080
tggaacttcc acagttgctt gtctaaactt aaaaggtttc ccagctaatt ctaacatgct 106140
tcttttttcc atactctgaa accctgattt gataatatct aaaaataata cactctattt 106200
ctctttttct ggttccttaa actttacttt ttagagacag ggtcttgttc tgttgcccag 106260
gctggagtgt agtggcagga tcatagctta ctataacctt gaactcctgg gctcaagcaa 106320
tcttctcact tcagccttct cagtagctag gaccacaggt gcacaccact gtacctggct 106380
aattttttaa tttttcatat taacagtctt gctatgttgc ccaagctggt ctcaaactcc 106440
tggcctcaag cagtcttcta gccttggccc ccgaaagtgc tgggattaca tgcatgagcc 106500
accatgcctt gccaaatctt aaagttttaa cataaataat tcttaaataa tacaaggact 106560
tttaaaaaca cttgagcctg gccaacatgg taaaaccccg tctctactaa aaatataaaa 106620
attagctggg tgtggtggcg ggcgcctgta atctcagcta ctcgggaggc tgaggcagga 106680
gaattgctgg aacctgggag gcggaggttg cagtgagccg atagtgcccc atcgcactcc 106740
agcccgggct gacgacaacg agactctgtc tcaaaaaaca aacagaacaa aaaaacactt 106800
gaactctgtc atactgtctt attgttgttt tacattttaa tcttgttaat atttatctca 106860
atactttgaa aaaattactt tcttctgctt cttgggtcac tgttgagaac atctttgtta 106920

ctttatttaa ttgttgttac tttttttttt ttaagagatg gagtcctgct ctctcgccct 106980
ccttcgccca ggctggactg caatggtgtg atcttggcta actgcaacct ccgcctcctg 107040
ggttctagca attctcctgc ctcagcctcc cgagtagctg ggattacagg catatgctgc 107100
cacgcccagc taattttttt attttttaat agagacgggg tttcactgtg ttgcctaggc 107160
tggtctcgaa ctcctgagct caggcaatcc accagcttca gccttccaaa gtgctaggat 107220
tacaggtgtg agccactgcg ctcggcctta atttgtcctt tttaaagttt tattttattt 107280
tttatttttt gggagagtct tgctctgttg cccaggctgg agtatactgg tatggtctcg 107340
gctcactgca gtctcaacct cctgggctca agcagccctc ccacctcagc ctcctgcata 107400
gctgggacca caggcattca ccaccacact tggctaattt ttgtattttt tgtagagacg 107460
gtgctttgcc atgttgccca ggctagtctt gaactcctga gctcaagcaa ttcacctgtc 107520
ttggcctccc aaagtgctgg gattacaggc atgagcaacc gtgcctagcc aatttgtcct 107580
ttttatttga atacttttgg tatcttctct gtctttggag tcctgcattc aaatgtgcct 107640
aggtgtgggt ttttaaatat ttatctgttt gggatttgat gtgtttccta accaaaatgt 107700
tcatgtcttt ccttgattct ggaagattct tatattccct ttttcccttt ctctttatac 107760
tctttttccc agatcaccaa attgatacat tagactttct tagattttct tattctgtcc 107820
tttgtgctgc ctcttaagtt cactactgga ttctctaatt ctttgtgtct ctgtgctgca 107880
ttctagataa tttcttcata tctgttttcc aattgatttt tttctgctgt gtttggtcag 107940
cttttaagcc tgcccattga gtttttaatt tctataattg cgctttttat ttctagatgt 108000
tctttttaat cattttccaa atctggttgg gttttggttt gtttttatta aatatggttc 108060
ttttctgatg actgattcca tttttcattt taacatttta aacactcttc gtatcctata 108120
attctatttc tgaaatcctg ggatactttt ttctactctt tgtagcttct tttgacatta 108180
gcttattaat atactttata aatttgaata atgaacatat gtttggtgag gctttttcta 108240
tagcacttct atgaggtctg aatttaggga tgtatttatc ctgagaggat aaatttgctt 108300
ctgtcattcc tgcagggcat gatcacccag aagttaattt caagttaatt tctaggcttg 108360
gtttttcaga ccacatgggt atatatttaa cccccaaatc taaatgaggg acagacctgt 108420
ggtaacaaat tcccaggaca atttttttaa aactcatttt tttcttttga tagcaattaa 108480
gttttacaga aaacaattac agtataatgt acacaccaaa tactgcacta cggatgtaac 108540
ccttttaggg ccctagctat atggtggggg tggggtgtat ctcagttaca actcatgtcc 108600
tatgtaggcc caaggccttt atgcctgtcc ccttcctggt tgataaactc atgacagatg 108660
cccccacagc agccaaaact tcagcatcag cttgcttccc ttatttctac cttttaaaaa 108720
atagctttta agagttctct ttcttctaaa tgaacacatt aactaatggg ctgttatatt 108780
acttccacca tttctatgtt ttggagtgtc ctttgtttca aaaagttttt caatttgtat 108840
attctgccat atagattgct gtatagctat gtaaatttat atatatatat atgtttatca 108900
tataattttt tgggaaataa atttacattt ttagcctaat aaggtgaaac aaaatggtta 108960

gaccttgaac aatgtaatgt actcacaagt aataatgtta tgtgactgga ttctgttttg 109020
tttatgaagg aaatgggaag acatgatgac ctttggtcct tattctacat gttggtggag 109080
tttgtggttg gtcagctgcc ctggagaaaa ataaaggaca aggtaatttt gggaatggag 109140
atgtcaattg tggtagaaga aataaaaatt tctgctgaat aaacattttt gttgaaaata 109200
tacttatgta taagacttaa aatctacaag cattttcact atgtcccaat atatgtattt 109260
ttctgtgtga gctgacatta gagatcaaat taatgttatg agatttttct gtgtacatgg 109320
agaatagata ctaattgctt ctgattaatt tatagccctc cctcttagct tttaatttta 109380
ttagttctcc cctttgtttt tgtaagtcaa gtaagctttt agtttttgat tgtcatatta 109440
tattgtttga tttagaaaac tgtaaacaca tgtgttgata aagaaaagta gatcagattt 109500
aagataaatt attttgagat gggtgcaatg gctcacatct atgatcctag caccttggga 109560
ggctgaggca agaggatcac ttgagcccaa aaattcgaga ccaggctgga taacatagtg 109620
agaccccaat ctttacaaaa attttttaaa aattagctgg gcatggtggc aagtgcctat 109680
agtcccagct atttgggaag ctgaggcagg aggatcactt gagtctcaaa ggtcgaggct 109740
gcagtgagtt gtgatcatgc cattgcactt cagcctgggt gacagagcaa gaccctgtct 109800
cagaaaacta aataaataaa ttattttttc tggggactat cgatacactg atgcagaact 109860
ctgtgctact tgtttcgttc tgcattatag tttttttccg ctttaaaaca ctgtacatta 109920
agctgttagt aagagctcag ttcttgcttt atgcttaatt acccactatc tcattcccta 109980
gtacataccc ttgagacggt tactgagggg ctcttaatgc ctaaccacca agagaaattg 110040
actgtagctc tttttctctt tacttcccag gagcaagtag gctctattaa ggagagatat 110100
gaccacaggc tcatgttgaa acatctccct ccagaattca gcatctttct agaccatatc 110160
tcttctttgg attattttac aaaaccagac taccaggtaa ccatctttgt gagttctgtg 110220
ggttagttat tttctctctt agtctatgct ttcactcatt gtagctttga aggtgttata 110280
aatgagtagg ggaggtcctt agaatacgac tgttttaata catcctgaga aacatggtga 110340
aattaactta tgatgagagt gtcagcgtgt gtgttatgtg tttcatccct ctggatggac 110400
ttaacttcat tcgagtttta ggtttattca tcttgcaaca gattttccta ttcctgcatg 110460
attcattcgg cgtatagaac tgctgacagt tgaaattatg taaattctat agctaatttg 110520
ttgaccagaa ggaaatgtcc aactaggtaa tgcatctgtc aagggcttta gggcagcata 110580
tcatatgtta atatgctatg ggacagcggt cagtgaaaat tggctccatt ttgcctgtgg 110640
tcagagaatc atttccttag agcatcagtt aatgagcctt agaagggttg actaggttta 110700
ctacaaatta catccctaac cccaaccagc ggtatttcag aagattctgt tgctctcagg 110760
gagaatttgg tcagggctta atacctaata attgctacca ctgaaaacat aaatgcggcc 110820
acacatagat taataagaat atgtattagt atatcatcct tatttacaaa ggacaataca 110880
tagactttac tgcatgttaa tggtagttaa attctacagt gttgtcagtg ttgtgcagtc 110940
aaaagactaa gaagtatcta tagcacagta aatgtatttg catatagtaa ttattgctct 111000

gtgaaattgc tgcttttgta taagatacaa acaggatata aagcaaagag gcaacataca 111060
gccttgagtc cttagagacc ttaaattggt gagtttgtct tctcatgcat atgccaattt 111120
gtgttgtaag caatagggac tgtctaacaa attgagaaaa tacactgtgt gtgagttgtt 111180
ttggaatgtg ttcttatatt tgtcttttgt atgtattttc tgaaatgtat acagttcaca 111240
ttacttaaag caatgaaatt atacctgtaa gactagattc aagaggagct gaaaattgtt 111300
ccaactttcc catcttgagt gctaatagtt tcttattgac aaaatttgct taggggaatt 111360
taggacagta aaaaactttg atcacattct cttcttaaac ctatatctaa agaagactat 111420
catatttacc agctgaagtt tgaaacttaa tgattatttt aaactaacaa attattgttc 111480
tcaagttttt cagttggaca ttctcctaag ggcttttcta tcacttaata acaaaacaaa 111540
gtttggtaat tttccatttt tgaaagaaag agaacaggca taacattact ttgtttcaca 111600
gattctgtaa ataatccagc tctttcctgc tgtgatcttt cctaagcagt gagtgagaac 111660
agagggcagg tggacaggag aaggataatt gaaaaaagat gttatgtaag tgatgggtgt 111720
gttctaatga ctagtatcaa tgtgacatca ggtttcaaaa gcctgagctt tgcagcttct 111780
gcctcttgtt tctctactca gtcttcatct tgagaaaaat aactctactt ttaatctgaa 111840
tggtctcgtg aattcatgct gtccctgtga ttaattttac agtttgagat tcattcctga 111900
atttgatttt acttactgtt ctttcaagat tttattttct tcatttaaaa aaattttttg 111960
agacggggtc tcggtctgtc agcaaggcta gagtgcagtt ggtgcaaaca tggctcactg 112020
cagcttcaac ctccaagact caagtgatcc tcccacttta gcctcctgag tagcttgatc 112080
tacaagtgtg caccatgcct ggcttttttt ttttattttt tttaaagcag agatgaggtt 112140
tccctatgtt gcccaagcag gtctcaaact ggcctcaagc aatccttctg ccttggcctc 112200
acaaagtgct gaaatagcca gcatgaagcc accatacctg gtcttttctt tcaaaatttt 112260
aggtttctca tgggacaacc aataaagtta aaaaaaacaa taaacctcct ccagcaacaa 112320
gcaaaaagca tatccatatt aaatatgact gcttccctag ctacatcaaa ggtcaaatag 112380
caatgatatg tttccagctt tgtctattct gagctaagac caaagtcagt ttttttgtta 112440
taatcaaagt gttaaatgat gcagttttac agagccactt tcactttgtg tatattttat 112500
aaatctttaa aaattggaat atcttgccat agaatattat tacttcaaca cgttgcacat 112560
ggccaacacc tcatccaata gctgctgctg ttcactttga catcagttgt ggacctagag 112620
agagtagcta ttttgttttc taccaggaat ctttcatgtg tctctcactt tacagtaaag 112680
gtcgaaattt gcttttgaga agtattatat tattttggga aattacatca agtttatctg 112740
tacttagtat ttatgtatga gtctgttttc ctaactagct tttaaatcct ggagaaacag 112800
ccatgtctta tttttttaac cccatattac acctggcacc taaattttca acaaatgtta 112860
ggcaaattta attaagtctt tactttggat caacagtctc ctacacagag aatacaagaa 112920
aaattaaccc tataagtgca taaataatgt tttctgtctg ttaaaataaa tgtgaagata 112980
tcagaattat aaacaagatt aaagcagata tgttcctata aatattgttt ttgtcagatt 113040

ctgaatatta taacaactat aagagacaga gtgggccaag cacagtggtt catgcttgta 113100
atctcagcac cttgggcgat tgaggcagga gtatcacttg aggccaggag tttgagacca 113160
gcctgggcaa catagtaaca ccctgtttct acaaaaaaca aaacaacaac aacaacaaaa 113220
aacaacaaaa aaaattagcc aggtgtggta gcatgcacct gtagtcccgg ctacttggga 113280
aacggagatg ggagggttac ttgagtgaat caaggctgca gtgagccata attgtgccac 113340
tgcactccag cctgagtgac agggtgagac tccatctcaa aaataataat aataataata 113400
ataataggat cacgtgatcc tggtaacaca aatatcttta ttagagtgtt caaatttgtt 113460
aagcatcact tagcaagttg agaagactaa tggaatccat aaatatacac accgttggtg 113520
agcctgaaac taaaatgtgt atctcaacca gttcacccac tgctacaacg ttgctaatat 113580
cagaggatag gatagagagg aaatgcaaag ctaagcaagc ttcattaatc ttcttaatat 113640
actttccagg tttaggaatg gggctattta aatgtagatt acataaaaat gcagtagatt 113700
tgcctcttgc tttgggggac atagagcagt tgtaggatcc tctgactctg ataacatctg 113760
aattaaattt ttctgagggc atagatgcta caaggttgag ctgaaaacaa gggggaacct 113820
ccgatcaaat agagtggtca aggaaggtct ctttctaaag ctaccatgta aggcccaggg 113880
gtgtccaatc ttttggcttc cctgggccac actggaagaa gaagaattgt cttgggctac 113940
acataaaata cactaacagt aatgatagct gatgagccaa aaaaaaaaaa aatcacaaaa 114000
aaatctcata atgttttaag aaagtttaca aatttgtgtt gggtcacatt caaagccatc 114060
ctgggttaca ggttagagaa gcttgattgt aagccaagcc ccaaaagatg ggattgtgtt 114120
ggccatgtga agagcaaaaa taagaggtag aatgttacaa gccaagggaa gaacatgtac 114180
atgcatgggc cctaatggga gaaaggactg aaagaagaca agtatggtca gacaataaat 114240
aaggagagag accaggttga ggtgaggttg gaaagagtca gaaaccagat aatgcagggc 114300
cttgtaggcc tttttaagga gtttggattt ttattctaag tacaacacaa agcgttggaa 114360
gtgttataaa caggagcatg gtgatccaat ttgtgcttta agaggattac ttttgcggtt 114420
atgtgcatcc aaatgaaaca aaggagaatc agagactagt tgggaaggta ctgagataag 114480
ctgagctgtg ccttggaaag aaataaattt ttttaaatca gtgtattttg ttttgttttg 114540
ttttttgaga catagtcttg ctctgttgcc caggctggag tgcagtggtg tgatctcggc 114600
tcactgcaac ctctgcctcc tgggttcaag tgattctctg cctcagcctt ccaagtagct 114660
gggaccacag gcacatgcca ccatgcccag ccaatttttg tatttttttg tagagacagg 114720
gttttgccat gttggccagg ctggtctcaa actcctagcc tcaagtgatc cacccacctt 114780
ggcctcccaa agtgctggga ttacaggcat gagccaccat gcctggccta aaaatcagtg 114840
tcttaacaca ataaaggttc ttgatcatat aatacattag tcaagttgta cagctctcct 114900
aggcaactct taagagtaac ttgaaaatcc aggctctgcc catatattga cactgctgtc 114960
tttaatacat ggccttttac tattatcagg aaagggtata gtgtggatga tggcttaggg 115020
gttttacagc aggcctggaa gtgatctaca acactggtgt atgcccagta gtcagaactc 115080

aatcacgctg gcccaattct aactgcaggg gaggctagga gatagagtat tcccttgtgg 115140
ccaggaggca agatgatgat ggccttgact tgagagaagt aatagagatg gagggaaatg 115200
ggagaattgt tgcctctgcc ccattacgcc ttatctcctt tcattcccca ttgtacttat 115260
gtgatctagt catattagac ccctcgatgc agtcagatga gactaccagc tcttctccca 115320
gtgtgcccta cctggaacgt gcctctgttt cataacactg cttgtcttgt gaaattgaaa 115380
gcccaactca gatgtcagtt tctccgcagc ctcctctgat tccttcaatt ccttccatat 115440
tccatactgg agtagtttct tcggttcaac tcttagagca gcagtacttt gttttgtctc 115500
tttattatat aagctcctta agagccaggg ccttgcacat ttatctcaat tttctcaaga 115560
acatagcaca atgataggca tttaataaaa gtgaaaaatt taattaaatc tgcttgatta 115620
ttgcttctat tgaatgtaaa ccacccaaag acaggactgt gttttgttaa catttgtatt 115680
cccagcacct aggacagtag gcagtcaatg tatgtctgaa acaaggagtg gatatttgat 115740
gtagctatag aatacaatag agtcacacta tgtttacatg actctcttaa attcagctgt 115800
gtgttcagga ggtagaaaaa tcttaacgtc tgtgattctg cctgtccttt ggcttagtca 115860
tgctttatgg tgagggtaat gatttagtgc cctcatgcat ctgctattct ctcagtcttc 115920
tcgttgacag cagttcagtg gccccaaatc aggctgcacc agtatgagtc cagtagttga 115980
aagcagagta ctttttttgg tacaaatgtg actgctgtct gcagccagct tactttatcc 116040
acattcatct gaggaaaaga cgtctattct agagctggag gaaaaactgg ctctgttgca 116100
cctattgaga aatggtagta ttctgtactt tatggacatt ttaaggcact ttcaagaata 116160
atttcaacta tatgctgttt ttaccttgcc tgctgactgg ctgtaactct tctttagtag 116220
aaatagcacc atacttaaaa gacctgttta aattgatgct ttgccacaca ctgtttggat 116280
aacctttcta gatctcagtt ttctagtctg tgaaatggtg gaaatactga cttcacagca 116340
ttgttgttaa gatttttttt ttttttttga gatggagttt tgctcttgtc acccaggctg 116400
gagtgcaatg gcgtgatctc agctcactgc aacctctacc tcctgggttc aagcaattct 116460
gcctcagcct cccaagtagc tgggattaca ggcatgtgct accacgccca gctaattttg 116520
tatttttagt agacatgggg tttcaccatg ttggtcaggc tggtctcgaa ctcctgacct 116580
caggtgatcc gcccacctca gtctcccaaa gtgccgggat cacaggtgtg agccatggca 116640
actggccatt gttaatattt ttaatgaggt aattatgata ataatttata aattttaaag 116700
tatcatgtaa atgtaagaaa tgtaagtagt tttacatgta ccacagtgat gctgcatttg 116760
cttgtaatca tctctctctt tttttttttt tttttttttt tgcactcctc gctggagtac 116820
aatggtacaa ttttggctca ctgaaacctc cacctcccgg gttaaagcaa tcctctcatc 116880
tcagcctccc aagtagctag gaccacaggt gtgcaccacc acatctggct aatttttatg 116940
tttttagtag agatggggtt tcaccacgtt ggccaggtct cgaactcctg acctcaggtg 117000
atcggcccgc cttggcctac cagagtgctg ggattacaga tgtgagccac cgcaccctgc 117060
ctaaaatcat ctcatttttc ttcactcagc attaccttct atgttaatag aacttctttt 117120

atttcctctt tgctgctttg tcctgtcaca gcaataagaa aatgttgttt acctttttta 117180
ttggccttgt gaggaggcac tgaaaagcct tagtgtacta tatttccagg cagtttacct 117240
tttggactga ctgagcccct gaaagggaag actgatatca tccagcactt ttggctgagt 117300
aagtgaccat gctgccttac cctgcctcag gtcctggggc agccaaccac atggggcact 117360
tctgaatctc cagtatctta atatacagtc atgtgccaca taatgatatt tcagtcaatg 117420
atggattgca tatacgacag tggtcccata agcttataat actgtgtttt tactatacct 117480
tttctatatt tagatatgtt tacatacaca aatacttacc attgcataac aattgcctac 117540
agtattcagt acaataacac gctacacaga taggtagcct agtagcaata ggctatacta 117600
tatagcctag gtgtgtaata ggctgtacca tctagctttg tgtaagtaca tgtattagtg 117660
agggttctct agagagacaa aactaatgga atagagatat atatataaag gggaatttat 117720
taagtattaa ctcacatgat cacagggtcc cacaataggc cgtctgcggg ctgaggagca 117780
gggagagcca atctgagtcc caaaactgaa gaacttggag tctgatgttg gaggacagga 117840
agcatccagc atcagagaaa gatgtaggct gggaggctac gccagcctca ccttttcaca 117900
tttttctgcc tgctttatat tcactggcat ctgattagat agtgcccaga ttaaggatgg 117960
gtctgccttc cccagcccac tgattcaaat gttaatctcc tttggcaaca ccctcacaga 118020
cacacccagg atcaatactt tgtatccttc aatccagtca atttgacact cagtattaac 118080
catcacagta cactgtatga tgcttgcaca atgaaatgcc tgtcgacaca tttctcagaa 118140
tgtatccccg ttgctatgtg acacccgact taatggtgaa aacaggtaaa ggttgcagct 118200
tcttcaactc attctcttta ttttatttta ttttattttt gctggtttag tttgttagag 118260
gatgggtgca gttaaatttt tttctttacc aatgttgcta aaatccccaa gaagagcaga 118320
gaagtaaaga ctgtgtgcag accaagaagc agcttctcca ctccttctgg gattccgtct 118380
ggttcggccc gcctgcctcc accatgtcca tcagggtgac ccggaagtcc tacaaggtgt 118440
ccacctctgg cccccaggcc tttaacagcc tctcctacac gagtgggcct ggtgcctgca 118500
tcagctcctc gagcttctcc cgaatgggca gcagcagctt ccggggtggc ctgggtgcag 118560
gatatggtgg ggccagtgga ggcatcacca ccatcactgt caaccaaagc ctgctgagcc 118620
ctcttaacct ggaggtggac cccaacatcc aggccgtgcg cacccaggag gagaagcaga 118680
tcaagaccct caacaacaag tttttctcct tcatagacaa ggtacggttc ctggagcagc 118740
agaacaagat gcttgagacc aagtggagcc tcgtgcagca gcagaagatg gctcggagca 118800
acatggacaa catgttcgag agctacatca acaaccttaa gtggcagctg gagactctgg 118860
gccaggagaa gctgaagctg gaggcggagc ttggcaacat gcatgggctg gtggaggact 118920
tcaggaacaa gtatgaggtt gagatcagta aatgtacaga gatggagaat gaatttgtgc 118980
tcatcaagga gtatgtagat gaagcttaca tgaacaagat ggagctggag tcttccctga 119040
aagagctgac tgccaagatc agcttcctca ggcagctgta tgaagaggag atcgggagct 119100
gcagtcccag atctcggata catctgtggt gctgttcatg gacaacagcc gcttcctgga 119160

catggacagc atcatccctg aggtcaaggt gcagtacgag gagattgcca accgcagcca 119220
ggctgaggct gagagcatgt actagatcaa gtataaggag ctgcagacgc tgggtcagaa 119280
gcatggggat gacctgtggt gtgcaaagac tgagatctcc tagatgaacc ggaacatcag 119340
ctggctccaa gctgagactg agggtctcaa aggccagagg gcttccctgg aggctgccac 119400
gcagataccg agcagcgtgg ggagctagcc attaaggatg ccaatgccaa gctgtctgag 119460
ctggaggccg ccctgcagct agccagtcaa gacatggcgc ggcagctgcg tgagtaccag 119520
gagctgatga acgtcaagct ggccctggat atcaagatcg ccacctacag gaagctgttg 119580
gagggcgagg agagctggct ggagtctggg atgcagaaca tgagtatcca tatgaagacc 119640
accagcagct atgcaggtgg tcagagcttg gcctatgggg gcctcacaag ccctggcctc 119700
agctacggcc tgggctccag ctttggctct ggcatgggct ccagctcctt cagccacacc 119760
agctcctcca gggccgtggt catgaagaag atcgaaaccc gtgatgggaa gctagtgtct 119820
gagtcctcca acgtcctgcc caagtgaagc ccctcccagc ctgtccctcc tgcggctgcc 119880
gcagagccta ggagggaggc cactgtgcag gggaacacag ggaacgggag acccacccga 119940
ggctcagccc tagccctcag cccatccgcg agggagttta ctacctgggg accccccctt 120000
gcccatgcct ccagctatga aacaattcaa ttgctttttt ttttttttcc caaaataaac 120060
ctcggctagc tctgccaaaa aaaaagactg tatgcatttc tttccctcta ttgcatctac 120120
ttatcactga agtggggcaa tttcttttgc tctaaataat ctgcctttca atcagccaag 120180
ccaacattgc catagtgctc tcctgcttaa gtactttatt gatccactaa ttttccattt 120240
attaactgag aattcaggaa tcaggattct tagtgagttc ttaatatcac cttacaaata 120300
attcttggtt ttgcaaaagc tggacttggt ctacactctc tttgaaaggg cagactggga 120360
tttcatatgg aggtaaatat gatacagtta ttgattgcat tttgtgctaa ggatgagagg 120420
agtgcttccc taaggcattg tgtgtatgtc agatgactga ttgccataaa gaataagggt 120480
attttaggta gtaggagctt gcaccaagca tctatgtagg ctgcatatag gtatatagtc 120540
aatttatttt agaataatat atttttgttg gttaattttt tctagttatt aaaagtaata 120600
tgtggtcatt ataaaagttt tagaattaac agaaaaatag aaaatctaga ttattcagaa 120660
tctcattatc ctgacatagt aattgacttt tattatttat ttatttattt tgagacaggg 120720
tctcactctg tcacccaggc tggagtgcag tggtgtgatc ttggctcact gcaaaccctg 120780
cctcctcggg ctcaagtaat cctcccacct cagcctccca agtagttgag accagaagtg 120840
tacaccacca cacctggcta attttatgta tttttggtag agatagggtt tcaccgtgtt 120900
gcctaggctg taattgactt ttattacttt gtatcttgaa taatttgctt ttgaaaaatc 120960
attgtattga atatcccatc agatttaaaa tggactgctt cttattcata agtgcaagta 121020
gaagaggggt tgacacaaat aatccacaca gtggccaaat aaaaagtaat gcagggccgg 121080
gtgcggtggc tcacgcctgt aatcccagca ctttgagagg ccgagacagg aggatcactt 121140
gaggtcagga gttcaagacc agcctggccg acatggcaaa accctgtctc tactaaaaat 121200

acaaaaaaat ttagctgggc gtggtggcac atgtctatag tgtcagctac ttgggaagtg 121260
aggcaggaga atctcttgaa cccaggaggc agaggttgca gtgagctgag atcacaccac 121320
tgcactccag cctgggcaac agagtgagac tccgtctcaa aaacaaacaa acaaacaaaa 121380
gtaatacagg ttgaatatcc cttatttgaa gtgttttgga tttcagattg tttgtttttt 121440
gagacggagt ctcactctgt cgcccaggct ggagtgcagc agcatgatct ccactcactg 121500
caagctccgc gtcccaggtt cacaccattt tcctgcctca gcctcccgag tagctgggac 121560
tacaggtgcc cgccaccaca cctggctaat tttttcatat ttttagtaga gacggggttt 121620
caccacgtta gtgaggatgg tctcgatctc ctgacctcgt gatccgctcg ccttggcctc 121680
ccaaagtgct agaataacag gcctgagcca ccgcgcccag tggatttcag attttttagg 121740
attttgtaat attttgcatt atattggttg agcaacccaa atcgaaaatc cgaaatgccc 121800
cagtcagcat ttcatttgag catcacttca gcacccaaaa ttgtgttttg gaatattttg 121860
gattttcgat tttcagattt gggatgctca acctgtgtag catttttgcc tgttcattac 121920
cttcttcaag ttctccctcc tcttttcctc ctcttcaaac aacttcccct tcacttaact 121980
gcttctcttc cctacatcca tttacagaag attcactttc ccgaagataa ttgcttctgg 122040
tgtttctcac actgtttcac cagttactcc tctggcagcc aagtataagc atatttctca 122100
agaagtctgg gaatagcctt acagtatcaa ccataagtcc aaaatttctt tgtattctcc 122160
tgatttatct taaaggttca tttaaatttt gccttccaac ttctttgtgt tcattgtatt 122220
atgaaatatt gtaataaatg tcatacctct gtgtaaaata cttgaggaag ctgtgccaca 122280
ttgtcaaatg actttaaatt ctctctggca tatggctgca tcccctgggg gtacatgtta 122340
ccctagtttg agaaacttag actctaaatg taggtgaatt tgtattttct aagaatgtat 122400
tattaagtaa aataaggagt cagatgtata ctagattttc agagattacc aatgaagaga 122460
tatgggacat gccttttaga gggaagcgtt ggaatcattt tgaagatttt tttcagattt 122520
ttcccttctc cctttccagt ttgagaatta ctgccacagt gagtccctgt tctgatggga 122580
aagtgctttg ttccccagtg acattgggat ataaaaaaga atgagaggct gggcatggtg 122640
tctcatgcct ataatcccag cacttaggga ggccgaggtg ggtggatcac ctgaggtcag 122700
gagtttcaga ccatcctggc caacatggtg aaaccccgtc tctactaaag atacaaaaat 122760
tagctgggca tggtggcgtg tgcctgtagt cccagctacc caggaggctg aggcaggaga 122820
gtcgcttgaa cccgggaggc gaaggttgca gtgagccgag atcgcaccat tgcactccag 122880
cctgggcaag aaagtgagac tccgtctcaa aaataaataa ataaaaataa aaataataaa 122940
aaataaaaaa ggcatgagga ctattcaaat ttaacaggca cacacatata tatatataca 123000
cagacatgta ttcacacact acatatactg tatatactat atagatgact tacaaggtaa 123060
tttttattct caaatctatt tgactcactg aattaagagt tttctgtttt tattatttat 123120
cataaccaaa tgtgatgtaa cacattcagt taccaaaaaa agtaagtccc tgacttgtcc 123180
tgtttctact attccaatct gaggaaggtt aggatttagc gccaccctgc tcccgaatta 123240

gaaaattatt ttgctagatt agaaatgttt ttctctaaaa tatctgcaga cctctgccca 123300
gtctgaccat ttgttcccac tagggtgcct ttaagtgcct cagtgctttt agctttccta 123360
tttcttttgt tctgtgtcct ccatctttct gcctcctgct gctggtgtct aatatcacca 123420
tcgtagtaac aaactgtttc taatttgagg tccagtgcca tagaatacat tttctaagac 123480
atacaatact gtgacagatg taagaaaata attccatgat aaaaggacat ctgacataaa 123540
agccatttag gatcattcct gttgcagcac agtacagaca gagaatttag cagtggcagt 123600
ctaagtgctc acatagcaag gctcccctgt atggggattc caccttccag gaagcatgca 123660
tctcactctg tgagaaccta tgagaacctt gatttcagag ttaggagaga gggtcaaagg 123720
tctggcactt caattcccta ggttccagtc catctttatt tcctcctttg tgttttctac 123780
ggccctctac ctctgacatt tggagaagtc taagctataa cgtaggtgac ttacatgaac 123840
aaaaatagga cccaaataaa atttctcaga ttttataact atgtcttttc cagttccctt 123900
tatcttttta gtttcttttt ctgtatttac aattcttcct tttggaactt aaatgaaaga 123960
ttaaatcatg tgagtcagta aatattatta caaattaaca tgtattattg gaatgttttt 124020
aggaatgtta tgaataaggt aatgtatgag atcctggaga cacaatgatg agtaagctca 124080
gattaatgtc agcatcttgg agcatatagt ctgggttgaa agagaaatta attaaataca 124140
gaaataacta tacatttaca actgataaca tttgatcatc ctccttagtg aaatggtttc 124200
ttcactttag tctagaatac cagactcgtg gttttcctgc ctcactggcc actccttttt 124260
tgtttccttt gctgagtctt tctcttcttt cacctaagta ttggagtact ctaggctata 124320
gcccttgatc ttttgctctc tattcttcct caataatacc agctggttct atggctttaa 124380
ataatatctc tctctctctc tttttttttg agacccgagt ctcgctctgt cacccaggct 124440
ggagtgcagt ggcacaatct tggctcactg cagtctctgc caggctcaag caattcttgt 124500
gcctcagcct cccgagtggc cgggactaca ggcacctgcc accacacctt gcttattttt 124560
gtatttttag tagagataga gtttcaccat gttggccagg ctggtcttga acccctgatc 124620
tcaagtgatc cgcccacctc agcctcccaa agtgctggga ttacaggcgt gagccaccgt 124680
gcgtggccag tatctcttga tatctatatg ctgatgacac ccaaatttat atctgcattt 124740
cctattcctc cctaaactca ctgctttcca tgtcactgca ctgacccagt ggctcaggac 124800
agaaatctat gtgcagttct tatcaccttt actattacca tgctggttca gctgacagta 124860
atctcttaac tggtcatctg ttccaagtac cctcattccc atccctgttt atggcatcta 124920
ttgaccactt aatagctgaa gtaatctttt tttttttttt ttttttggag aaagattctc 124980
attccgtcac ccaggttgga gtacagtgac acgatcttgt ctcactgcag cctccgcctc 125040
ccaggttcaa gcaattctca tgcctcagcc tcctgagtag ctgggattac aggcgtgtac 125100
caccacaccc ggctaatgtt tgtattttta atagagacag ggtttcacca tgttcgccag 125160
gctggtgaag taatattttt aaagcctaaa tcagacctgc gcaaaatcct ccaaagaagt 125220
gctgatttca ggtctggggc aggaaatgta caagatgaat ctcagaggat aggatagtaa 125280

cagagttcca gaaatgatga ggagagaata aagctataat cataaaattg gaaatgttgc 125340
ctatgacaga aacaatagag gccttttatg agacccttct taagatcaca tcaaacttta 125400
ggaattgaaa gacaaaaaat gaaattatac aggatataaa tcaaactaac agaatataaa 125460
tcaagcaatt ttttgataat ttatatatgg agacacacaa acatgcacac acatatttta 125520
agaatttttt aaacctgcaa aaaaaaaaaa aatgaaggaa agcacaatga acttctatat 125580
tccccttacc tagacacgtt acatgttaac actggctact tttttaggtt gaaacatata 125640
aaaagtcctt gttttgtttt gttttgacaa ataaatgata aaatagatgg ttcaattgaa 125700
tgcacaaaca tatacactta cacacaacac acatccttta gccaaccatc tgagagtagg 125760
ttatagatat gatgcttcat tatgtatccc tcgaaaacag aaacattcta cacaacggag 125820
aaaaatcaac attaattcac tatcatcatc tacatatagt tcctattcaa atttcgcaat 125880
ttgagccatc agtttttaaa gaaaatcaca ttacaaaatc attttatgag tctggtgtgc 125940
tttctcttat agaataaagt gactatgttt actgttcact tactctagtt tttttctttg 126000
tggtttagct tcttacatcc gtgtttgaca atagcatcaa gacttttgga gtaattgaga 126060
gtgacccttt tgactgggag aagactggaa atgatggctc cctaacaacc accactactt 126120
ctaccacccc tcagttgcac actcgcttga cccctgctgc aattgggtat gtcctaaaca 126180
gatcatttgg tggtgtcttt cctggatgta ttatatttgc aaaaaattac ttcatccaga 126240
ttccctcaga tgaccaaatg ctaagacatc cgggaaagcc tgtggatctc ctcgaattaa 126300
cattgctctt atgtacgcct ttggaattac ttgctttaga tagtactaat agattatact 126360
gtgaaatggg agcttagagc ttagagctta tgtgacttgt tgatatcaaa tttataatgg 126420
gtattcttca aaatgaggac cagcattagt tgtgtttcca aataacatat ggaaccctga 126480
aaagaatgtg ccacagttat atctaactat actgtgccaa agtttagtgg gaacaaagca 126540
tgtaacatgt tcctcttaaa gtgatttaac cttcataatg tattaagaaa aggttatgat 126600
ctatttccgt gatcttagtt ttttattgtt aatgtttttt cctctacgtg tattagagtt 126660
ccttttgtct tttattttgt ttttttattt gtcttttttt tttttctaat tttttacctg 126720
ctttggttgt ttcaaacttt ttggtcaaaa aaaaaaaagg taaataccca aaatgctgag 126780
gcagaaggat cacttgagcc caggagttag aggctgcagt gggctatgat tgtgccacta 126840
cactctagcc tatgcaacag agtgggaccc gtctctgggg aaaagaaact gaatagggac 126900
aaattatcat acaatttaag ttaattggga cattatacca ctcatccatt gcttttattt 126960
tttttttttt taaacatgga gtttcattct tgttgcccag gctggagtgc agtggcacaa 127020
tctcagctct gcctcctggg ttcaagcgat tttcctgcct cagcctccca agcagctggg 127080
attacaggcc tttgccacca cgcccggcta attttgtatt tttagtagag acggggtttc 127140
actatgctgg tcagtctggt ctcaaactcc tgacctcaag tgatctgcct gtcttgttct 127200
ccaaaagtgc tgggattaca ggcgtgagcc actgcgccgg cctgattttt ttttttaaga 127260
gatggagtct ggctgtgtta cccaagctgg agtgcagtgg ctatttatag gcacagtcat 127320

agtgcactat ggcctcaaat tcctgggctg aagcaatcct cctacctcag cctctctgga 127380
ctacagacat gcacccttgt gcccagctgt gatttttata ttgaaagatg ggatcactag 127440
aacttgttga atcatcggaa gagttttcat agactcacat aactggatag tatcactgac 127500
tatcatgtct aagcattcct aaatgtgaga ccctgactag acccagaatc accacggatg 127560
tttttaaaaa acgcctattc ctgggaccta ccctagacct actgaatcag aatgcctaga 127620
agtggggcac agaagtccat atgcatcaag tatttctcat gcttatcaga gtttgggatc 127680
cactgcccta ggtccagcca ccctatctta caagtgagga aataatccta acactgaagt 127740
aaatccaact ttaggtaaat attagtggca gagctggagc cagtccagaa attttgttct 127800
agtgtttttg tccttcccct ttgattgttg ttagaaataa gtctcaaact tgcaaaaatc 127860
caggtgatag cacttcaggg acctaaagac aatgaaggca gtttatgttc ccatcttcca 127920
atattttctc caaaataaca cagaataaca aagaaggaaa actaaagcta cacagaaaaa 127980
ccatgtcctc agcgtaactt gtagaagatg cctaagcttc aaaataactg taaataaaaa 128040
gagagaaaaa aatgagacca caaatcctag tgcagtatct ttcatgctct tgcctcacct 128100
acccctgcca gaagacttta taaagacaga tcaagaaaaa ctgtggggaa gatgaaaatt 128160
agcaatggga tgtaaggttg atctaaaacc agtgccatac tccagctgtc tttcgatccc 128220
aaaaaataaa taaataaata aaaccagtgc tagaaagact aaacccatgc taataaggaa 128280
aatactaaaa agtgtcctga tagatcagat gatgaatttc agggaagtaa aaatatgcat 128340
aattcaaaag aaaaaaaagg gagaagtgtc cttcattagt ggagtgacgg aggaaaaaag 128400
atgtatcaat ttgggtccag tcaggagaca gaaactacac agtaatttga atagggaaaa 128460
aaaatcaaag aattatttat ggctacacaa taattataca tatatatggg atacatgcaa 128520
tattttaata catgcatgca atgtgttgtg atcaaatcag ggtcattagg atatccatta 128580
cctcaaacat ttatcatttc tttgtgttgg gaacatttca aaccttctac ctattttgaa 128640
atatacacta gtatttttgt ttgtttgttt gctacagagt ctcactctat cactgtccag 128700
gctggagtgc agtggcgtga tctcggctca ctgcaacctc cgcctctcag gttcaagtga 128760
ttctcctgcc tcagccttcc gagtagctgg aattacaggc atgtgccacc atgctaattt 128820
gtgtattttt agtagagagg gggtttcacc atgctgggca ggctggcctc gaaatcctga 128880
cctcaggtga tccacctgcc tcggcctccc aaagtactga gatcacaggc acgagccacc 128940
acgcccagcc tacacaagct attgttaact atagtcagta atagggaatt ggaataacat 129000
aggattgatt aataagaaat aaagataaat ataaagaata caggtacagg aatagcagat 129060
acaaggtcca gccatgatgc ctaggactga gtgagattaa acatccaagc aagaactgac 129120
ccacccctgc ccaagggctg aggttcagac cttattggga gaggaagaaa tgaaatcata 129180
attgattgaa ctcaggaaag aaatagaagg caaagacaaa attatctcat aattgaagaa 129240
tatattaaaa agtgcacaaa ggagaataga cataagtgaa aatttaatag aagtcattga 129300
ataaggacaa agaaaaaacc ctgagagaat gaaaatgaga taattcaaaa gagttagaga 129360

gaaggtaatg caaattgaag gcagagaaga aataacagtc aggttgttgg agtccctgaa 129420
gaagaaaaac aaaataatat gttaaaactg agaaaccaaa tagtagaagg ttagatttaa 129480
aaacagagaa ctggctgggt gtgatggctc atacttgtaa tcccagcagt ttgggaggct 129540
gaggcaggcg agtcacaaag tcaggagttc gagaccagcc tgtccaatat ggtgaaaccc 129600
cgtttctact gaaaaataca aaaattggcc aggcaccgtg gctcacgcct gtaatcccag 129660
cactttggga ggccaaggcg ggcggatcat gaggtcagat cgagaccatc ctggctaaca 129720
cagtgaaacc ccgtctctac taaaaataca aaaacaaaat tagccaggca tggtggcagg 129780
tgcctatagc cctagctact cgggaggctg aggcgggaga atggcgtgaa cacgggaggt 129840
ggagcttgca gtgagctgag attgagccac tgcactccag cctggatgac agagcgagac 129900
tccatctcaa aacaaacaaa caaacaaaaa aaacacacaa aaattagctg ggcatgatgg 129960
cgcacacctg tagtcccagc tactccggag gctgaggcag gagaattgct tgaacctgtg 130020
aggcagaggt tgcagtgagc tgagatcatg ccactgcatt ccagcctggg caacaacgtg 130080
agactccgtc acaaaacaaa caaacaaaaa ctcacagaga actaagagca tttaaaaagg 130140
tttatgtcca aagataagca ttagaacaaa aaggcaaaac ttctgaaata cccacccccc 130200
aaattattta taaattaaag tacgggccag gcacagtggc tcatgcctgt aattccagca 130260
ctttgggagg ccaaggtgga cagatcactt tgagctcagg agtttgagac catcctgggc 130320
aacatggtga aaccctgtct ctacaaaaaa tacaaaaatt agctgggtgt ggtgttgtgc 130380
acctatagtc ccagctactc cgaaggctga ggctggagaa tggcttgagt ccaggaaatg 130440
gaggttgcag tgacctgaga tcgcgccact atactccacc ctgggcagca gagtgagatc 130500
ctgactcaaa aaaaaaaaaa atagacaaag aatacatctt gtagaggaag aaatatagca 130560
agtatagcaa gatactgtgc acataataat aaaatactat gacaaagctt tcctcctatg 130620
ttctcttcta ggagttttat agtctcaggt cttaacatat aggtctttta tacattttta 130680
gttgattttt gtgtattgtg taaaataggg gtccaattcc tttgcacgtg gaaatccagt 130740
ttttccggca ccatttattg aagagcacca tttattggat cccactgtgt ccttttggtg 130800
cccttgttga aaattagttg accaaataag gtatctgcac tctcgtgttc actgcagcag 130860
tatttaccat agccgagata caggaacaac ctaaataccc actgatggac aaattgataa 130920
agaaaatgta tatgtgtgtg tgtgtgtgtg tgtgtattgt atgtatttgt atatatatat 130980
gcacacacaa tggaatatag atgatgtata aatacttaca cacacacaca taatggacta 131040
ttattcagct ttttattttt ctatatttat ttttttgaga ctgattcttg ccctgttgct 131100
caggctggag tgcaatgaca tgatctctgc tcactgcaac ctccgcctcc tggattcaag 131160
ggattctcct gcctcagact cctgagtagc tgggattaca ggcctgcgtc accacagctg 131220
gctaattttt gtatttttat tagatatggg gtttcaccat gtttaccagg ctggtctcga 131280
actcctgacc tcaggtgatc cacctgcctc aacctcccaa agtgctggga ttacaggcat 131340
gagccgctac acccgacctt attcagcttt taaaaagaag gagatcctat catttgccac 131400

agcataggtg gaactaaaag acatgcttag tgaaataagc cagacagaaa taaaaatgtt 131460
gcatgatctc atttatatgt gaacaaaaaa catatatata tatatacaca cacacacgta 131520
tatatatttt aaagcactca aatacacaga gattgagaat gatacagtgg ttaccacagg 131580
gggttcaagg agggaggaag gaaaggaaat ggggagatgt aggtcaaagg atataaaata 131640
gcagatatgt gggatgaaca agtgtggaga tctaatggac agcataagga ataaccttta 131700
taaaattgta ttttcagaag tcttggacat aaataaaaat aaaattgtat tagagatttt 131760
tgttaaataa gtagatttta gctgctcttt gtcacaaaaa aagtaactgt gagttgatag 131820
gttaatctgc ttccctgtaa tgaccatttt actgtctatg tatatcctat tacatcatgt 131880
tacaaagctc aaatacaatt taaggaagta aaatactaca gcagacttaa gaccaaacac 131940
atcagtcaca gcaataaata tacatggggt taactcacct attaaaagga aaacattttc 132000
agtttgggtt atatagctat attcatatgc tgtgtataag agacacacac ctaaaaaaag 132060
taagaaagag atggacaaag atatatcaga aagatagaaa agagaagtga tcctaataac 132120
agatgatgta gaattcaagc caaaacaaaa aacttagaaa agatggaagg gcacttttta 132180
atgccaaatg ccacaattca caagtgggat acaaaattta ttaatattta gtcaccagaa 132240
tcatgacact cacctttggg aaacaacaac tgcaggatat tcaaggagac ataaaaacac 132300
actcatagtt ggagatgtta atgtaccacc ctcagtttaa gacagatcaa gtgaacagag 132360
aataggaaga tagaagactt aaacagcgtg atcgtagtaa ggtagatcat atgtatgttt 132420
tctgaactct ataccttgat gatagagact atacctcctg cccaggggcc cctggaacat 132480
tcacaaaaat tgatcaggta ttaggtcaca aacaaacatg taagtagttc cataaagtag 132540
aagtattaca ttatctgatc acactgcaag aaaactagaa attaccaaca aaattaaaat 132600
atcaaaggcc cttttactgg aaatttaaga acctattaaa ctacagtcat gtgccgtgta 132660
atgacatcgc aatcagtgat acaccgcata tatgacagtg gtcctataag attacaatac 132720
tgtagtttta ctctaccttt tctatgttta gataatgtta taatacccaa atacttaacc 132780
attgtgttac acttgcctcc agtattcaga acagtgacat gctgtacagg tttgtaacct 132840
aggagcaata ggttatacca tatagcctag gcacgtaata ggctatacaa tatagttttt 132900
ttaagtacgc tgtgatcatt tggataatga tgaaattgct taacgatggc atttgttaga 132960
atgtatcccc gttgttaagc gatacatgac tgtactcttg ggtgaaaagg aaaataaaaa 133020
ctaaaagtta tagaaaaaaa tgataatgta aacactactt atcagaatct atgagataac 133080
tttagaacaa agatcagagg aaaacctatg gcattaaaca aggaggtaca taatgtcagt 133140
atgtctcatt actgatgata cttatgttga caatttggtt acagtggcca gttctttcta 133200
ctataaagtt actgtttttc tctttgtaag aaataatctt gtgggaagat atttgcaact 133260
atgtcagcac tgtgtttctt gtcatacttt tcctattaat tgtagcatcc atcagtgaat 133320
cttgcccaca ggcctaacca ttactgtctg ccctctaaga aaaagtttgc caacttctga 133380
tttttttctg tttgcaaatg atatggtaga atacctggag aatcagtgtt aaagagaaaa 133440

atttatagtg tatgtcccag aaccctgaat ttcttcgtag agggagaatg aaagtttaga 133500
aggtaagagg acgctttaaa tcatgagcca gcaagcttta tctataaagg gtcagataaa 133560
tatctgtgag ctatgctgtc tctgtcacta ctgtcattgc tgttatagtg caaaagctgc 133620
catggccaac gtgtaaacag acgagactga ctgtgttcca ataagtctat taaaaaaaaa 133680
taggtaagca ggccagattt ggcccacaag cactagtttg ccttagatta tggggaatga 133740
aatatgaaaa ggatattcgt tcttactatt ttttctcata tctcttcagt tcttccacac 133800
attttcttac ttattccctt aatctgcttc agttatataa taccttgcct ggaaacacag 133860
accatgtgct aataacactc ttatgtatac tttctcactc attcatattc ctgaacatga 133920
tataattttc ctgggaagga attatttctt tccatattgc atactcaaga tttggatcag 133980
agtttaggaa aagaaacaat aaccagggaa ggagaaagga atggagaata aatgcagagg 134040
gagggtctgg gtgggagagg aacctgagtc ctataaaaga gagcctgtgt ggagaagatg 134100
gacgaggtcc ttggagtgtg tggtatcata ttgaagcata agagataaga gaaaaagtag 134160
gattgaatga aagtagaagt gtattcagag atggaggtgt ggtttgtttc ctgaggtgtg 134220
aaagaaggaa atgcaaagga attgaggtag ttggaaaaaa agcagaatag tggctagaca 134280
taagggaaaa agggacttga aagaatactg agtgattaaa atggaaaaaa ggtgacgata 134340
atagactaag cttcttgagt tcaaatattc atctatttcc ctttctcttt cctcaggaat 134400
agagggaatt ctttcaataa acatttggat taccatgtag tacatgtatg ttagtgtcca 134460
ttatgactct tttatcaata aataaattat attgaactaa tatctgaaaa atctgttagt 134520
agccagttta tttctgatct ttaattatct ctataataaa tctgaactat tgatttgaac 134580
tatttgaact attttaggcc aaactacctt ttaaatatgc ttccactgtc agaactacag 134640
atactacttt actaggttgt aagttttttt tttttctttt cttttttttc ggggagccat 134700
tatcagtgct tttctagatt atatccttgt aaattgttga taaagaggtt cattctagca 134760
ttgatccatt taggcaaagg tagtggtgaa ggtcatcggt taccaagtca ggtggaacaa 134820
gtccctgaaa aatcactggt cttctcagag catatgaaat actcgtgaca ccagtgccag 134880
gatctaactg tgacccttaa agtggctcac tgagaggatg ttgccactca cagtaattac 134940
aggagtcagt ataatgtact gaagactaat ttttctactt tgagacattc caggtcactt 135000
ctaagaggag gtttcattgt gtgctaagta ttgtgttcag tcagcaatat ttaaaaaaaa 135060
aaaaaaaaac cctagaatat agagttacag aactggcagg agccttttca cttggtgaag 135120
gaaaactgtg aactagaaaa gcctctcatg ggagggtcta agggagcgat gaccaaccat 135180
ataaataaga cctggaatta ctttgaaaga aatatataga caaataccaa ttaatatttg 135240
ctattttttc ccccaggtca gttcatgctg aactatatca actgtataat tacagaaagt 135300
ctagtttccc ctctagggca agaatttcct tcctctttgt gttgctaggg ccttgcatgt 135360
agtaggtgct tagtaaaaag tctgcaagtg gccgggcacg atggctcaca cctgtaatcc 135420
cagcactttg ggaggccaag gtgggtggat cacttggggc caggagttcg agaccagcct 135480

ggccaacatg gcaaaacccc atctctacta aaaatataaa aaattagctg ggcatggtgg 135540
tgcatgcctg taatcctagc tatttgggag gccgaggcag gagaatcgct tgaaccccgg 135600
gggcggaggt tgcagtgagc agagattatg ccactgcacc ccagcctgag agacagagct 135660
tgactctgtc tcaaaaaaaa aaaaaaaaaa aaaatctgca agtgaaaaag taatacacat 135720
ttcacactgt aaaaaaataa catagctgat tgatctgaca cctgtttatt tcattatggc 135780
actgcgaaag ttgattatac aaattaggtc attcttgtca tagtcaacta aatcaatcaa 135840
gaggccaggg agaaagtact tggggcacat agcacctgct ccaagaattt aatttcttac 135900
aagcccagct gctgaaatgg cctgctgtga ctccaagact aaatttacat acctctgtca 135960
ctcaccagtc agagcctgcc agctcccaaa agcttctctg atgccaatga actttctttc 136020
aaaacagtat atgacatctc tctaataaaa cccccaacct ctcctttggt ctttagatat 136080
accagagacc cactcagtct atgtgtgtgc cccaaattgt cattcttgct tcccaaatgg 136140
aatgttaatt tggagattca tctctgtatt ttatttggag ttcaacagca cttaacattt 136200
tcctgtttag aatttttaaa aattctctaa cacgccgggt gccgtggctc acacctgtaa 136260
tcccagcact ttggtaggcc aaggcaggca gatcacctga ggtcaggagt ttgagaccac 136320
cctgcccaac atggcgaaac cctgtctcta ctaaaaatac aaaaaatagc ccagtgtggt 136380
ggtgggcgcc tgtaatccca gctactcagg aggctgaggc aggagaatcg cttgaatccg 136440
agaggtggag gtttcagtga gccgagatcg cgtcactgca ctccagcctg ggcgacaaga 136500
gtgaaactct gtctcaaaaa aagaaaaaaa atgttgttat aattgacaga gacttctatg 136560
aaaatagatg aaatgctttt tacttttaaa gtgattgtca aattagggtg ttattgtttt 136620
agattttgta ttttaaaatc agcttgaaaa ttctaattga tcaaaatttt aaagttttat 136680
ttcgtaggat tggcatgacc taagtttttt ttttcttttt cttttgtttt tgttttgttt 136740
tgttttttga gctggagtct cgctctgtca cccaggctgg agtgcagtgg cgcaatcttg 136800
gctcactgta acctccgcct tctagattca agcaattctc ctgcctcagc ctcataagta 136860
gctgggatta caggtgccca ccaccacgcc cagctaattt ttgtattttt agtagagaca 136920
gggtttcacc atgttggcca ggctggtctt gaactcctga cctcaagtga tctgactgcc 136980
tcagcctccc aaaatgctgg gattacaggc ataagccacc atgcccggcc aacctaagtt 137040
ttcaaggtgc tttttttttt tttttaactc tcttaattat gtgtggagtt catttaaatg 137100
aagaaataaa atgatgtaag atagtctcat agtaattttt gattcttgcc ttttttttct 137160
cccttcatat gtagaattgc caatgctact cccatccctg gagacttgct tcgagaaaat 137220
acagatgagg tatttccaga tgaacagctt agcgatggag aaaatggcat ccctgttggt 137280
gtgtcaccag ataaattgcc tggatctctg ggacaccccc gtccccagga gaaggatgtt 137340
tgggaagaga tggatgccaa caaaaacaag ataaagcttg gaatttgtaa ggtatgagta 137400
ccttataaac aacagaaatt tatttcttac agttcagaag gctgggaagt ccaagaagga 137460
aggctaggca gatttggtat ctggtgaggg ctgttttgtg gctcctcctt gctgagtcct 137520

catgtggtag atggggaaag ggatctcttt tgggcctctt ttgtaaggac accactctca 137580
ttcatgaggg ctctgccttc ttgacctaat tacctcccaa ggggcccacc tcctaatacc 137640
ctcacattgg gagttagaat ttcaacatgt gaattttggg gggacacaaa cattcagact 137700
atagcaggtg tatatgttca gtcatgggag tcatttgcaa aatgatctct ggatcgatgt 137760
gatatgccac aatgtgattt ggctgttgac aaagcacaat ctggatgaat agtaatcagg 137820
aatgagtagt ggaaagtaga cagggagtca gtgtcagatt tttctagtat cagctgtggc 137880
tgtctttatg gcaccaggaa caaactgttc ttatagcaaa aaaaaaatca ctagtgacat 137940
tctcagttgt cagcccatat ggatatccgt gtatgcgaga gaatgaagtg gcgcttgaca 138000
tggaagatct tagttatttt ggtttctgtc ttttactcac tatcactatt taatctcagt 138060
gaaatttgaa tggtagatag ttttgaccct aagtccatta tttgtcatca tggtgatgtg 138120
cagtttattg aatttataat gtatagtgct gtacattaca agctctcatt ttgaacatct 138180
ggataaagca ttaagctaaa tattgaagac aatttactaa gctatgtgct gtgcagttta 138240
agatacatct tttaccttta aggagcttac ggtctctttg ggtagatatg tagctgcaca 138300
agcaaatata atacaagaca gaacattgtg caaaacattt aaaaaaatta aaatgcatgc 138360
agatggggtg ggagttggga acatgtctta tggaagaagt ggttgaagta gaagtataag 138420
taaaaatatg tacatgaggg ccaggcatgg tggctcacac ctgtaatcct agcactttgg 138480
gaggccaagg tgggcggatc acctgaagtc aggagttcaa gaccagcctg gccaacatgg 138540
tgaaaccctg tctctactaa aaatacaaaa attagcaggg tttggtggtg ggtgcctgta 138600
atcccagcta cttgggaagc tgaggcagga gaatcgcttg aacctgagag gtggaggttg 138660
cagtgagcca agatcatgcc actgcactcc agcctggatg acagagtgaa actccacctc 138720
aaaaaatata tatacatata tacatgaaaa agtgcatgga tagagcttat aactttagga 138780
atttattggt catctaggag aaaaattcaa aacagctata tttatctttt aaaatctact 138840
tatactatat ataaaagatt tcatatatat catgtcaaag catagtaata taagcatgtg 138900
aatccaccat ctgccctaag aactagaata ttttaacata ttccttctat atttatgctt 138960
ctaccctatc cccactgagg taaccgctgt cttaggttag attccataaa agcagagcta 139020
caaaacaaac cttaataagt ttaaataatt gaaatcacac atactatgtt ctctaaaaat 139080
attggagtta atctagtaat cagtgacata aagacaacag gaaaatctct aaacacttgg 139140
aaattataca gcatatttct gtataatcca tgggtcagag aggaagcctt aaaggaaaat 139200
tttttaaatt tacatagaat tgaatgaaat cgaaaccaca acatatcaaa atgttgggga 139260
ggtagctaaa gcagtactga gagagaaatg tatagcacta aatacttaca ttaggaaaga 139320
taaggtttta catcaatagt ctaaatccta cctcaagaaa gtagaaaaag aagcagcaga 139380
ataagctaag acaagcagaa agaaggtaat gaatatctct gttactgtat tgcaaaagtt 139440
ttggtgtgtt gtatttttat tctcattctg ttctgaatat ttttccgtaa tgattttttt 139500
catccatgag ttaagtgcat acaagtttag agtttttaca tctgccaggt gattaaaagt 139560

tttgtttttc tttatataat gactatttct attctgttcc tactattttt tttttctagc 139620
attaaagcct attttgtcag atgttaatac agttatgcca actttctttt gggtagtaca 139680
tgcctggtgt atcttttttc tgcctttatc tttctatttt ttctcattct gttgtgactg 139740
ttgttaagtg gagtataagt ggacaatctt acgtctttat gttttaatta gtcagcttaa 139800
ttgatttaca tttagaatta ttattgtatt tgtgtattta ttttctgaga cagagtctcg 139860
ctctgtcacc cagtctggag tgcagtgaca tgatctcggc tcactgcaac ctctgcctcc 139920
cgggttcaag tgattctcct acctcagcct cccaagtagc tgaaattaca ggcacccgcc 139980
accacgcttg gctaattttt gtacttttag tagagagggt tttgccatat tggccaggat 140040
ggtctcgaac tcctgacctg aggtgatttg tccgcctcag cctcccaaag tggtaggatt 140100
acaggcacaa gccacgacgc ccagccaaat tatttttata tgaacttgtt tctcttttaa 140160
atttctattc agttttttaa tgcctttttc ctgcctttct tttcattaca gattttgttg 140220
gtatttttca tcttctgttt tctctttgta cttactgggg atttaaacat tctaattctg 140280
ttctcttggt agttgtcctg gaaatttccc agttattttt aacttaaaag tctaaaatta 140340
agcaatatcc taactacctt tctgatactg caaggactgt agaacatttt aacttcattc 140400
acccttctcc caacatttta gtactcttct aattttaatt ctttctttta aaaaaaaaaa 140460
aagaaaaaga atgacttgcc ctggcacagt ggctcacacc cataatccca gcactttggg 140520
aggccaaggt gggaggaaca ctagagccca ggagttcgag accagtctgg gcaacatggc 140580
aaaaccctat ctctaccaaa aaaaaaaaaa aaaaaaaaaa gttagccggg catgttggca 140640
cgtacctgta gtcccaggta cttgggaggc tgagggtgga ggatcgcttg aacctgggag 140700
gagaagctgc agtgagccaa gtttgcgcca ctgcactcca gcctaggtga cagagtgaga 140760
ctctgtctca aacagacagc aacaaagaag aatgacttga attagattat tatttatttt 140820
atttattttt tgagacagag tctcgctctg tcacctaggc tggagtgcag cggtgcgatc 140880
tcagctcact acaacctccg cctcctgggt tgtgattctc gtgcctcgtc ctcccaagta 140940
gctgggagta caggcacgtg ccaccacacc cgactaattt tttgtatttt tggtagagat 141000
ggggcttcac cgtattggcc aggctaagtc ttgaactcct ggcctcaagt aattcacctg 141060
cctcgacctc acaaagtgct gggattacag gtgtgagcca ccgcgcccag ccttgaattg 141120
gattattaat gttgttcttt tatacaagta atgtttatat ggactttcaa agacgtttac 141180
catttccttt catttctgaa agtccttggc tcatctttct tccggaaata tttccttcag 141240
aagttccttt acctcatgtc ttttggtaga ggactctttc tataaatgta ttttcttatc 141300
tataaatgta cttagtttag cttcattctt tcaagatagt ttatgccaaa tataaagtcg 141360
ggggtttttt tggtttgttt tctttctttt tttttttttt tgtgacaggg ttttgctttg 141420
ttgcccaggc tggagtgcag tggcgcaatc tcagctcact tcagcctcca cctcttgggt 141480
taaagcgatt ctcctgcctc agcctcccga gtagccggga ccacaggcat gcaccaccac 141540
acctggctaa ttttttgtgt gtatttttat tagagacagg gttttaccgt gttggctagg 141600

ctggtcttga actcctggcc tcaagtgatt cacccacctc cctcccaaag tgctgggatt 141660
ataggcgtga gccgccacgc ccagcccaaa tataaagtta ttgatagtta tttttcctct 141720
ggcttcagtc actggtgttc agaaacttac tgtcattcta attgtttttt tctttttaag 141780
tgattgtttc ccccaccttc atctttcctt cccagctact ttttagttct tgttcttgtc 141840
tgtggtgttc tgcgttttca ctatgatgtt tctaagcata tatttatctt gcttaggata 141900
tcttgtgtcc cttgaatatt tttattcata tttttgtata aaaatatgtt ctgggaaata 141960
acagccattc tgtatctgaa tgttactacc tcttcattct ttctattatc tctttctagg 142020
actccagtta gacatatgtt atggcttttt attgtatcca tcatatctct taatatcttt 142080
tttcataatt ttcttcttat cttgctgggt ttgttttgtt ttgtttttgt ttttgttttt 142140
ttgagatgga atcttggtct gttgcccagg ctggagtgca atggcacgat ctcggctcac 142200
tgcaacctcc gtctcctggg ttcaagtgat tctcctgcct cagcctcctg agtagctggg 142260
attacaggcg tgtgccacca tgcccagcta attttgtttt gtatttttag tagagatggg 142320
gttttactat gttggccagg ctagtctcca actcctgacc ttgtgaccca ccctccttgg 142380
cttccgaaag tgctggggtt acagatgtga gccaccacgc ccggcctatc ttgctgtttt 142440
ttattctagg taatttcttt ggctgtgtct tccacttcac taattaataa tttcaacctt 142500
ttttgttttt tttttttttg cttacttgct gcttaacttg ttaattgaat attaaatatt 142560
aagttaatca atcatttaat tccaaataac cctttgttga tctttgttat aataaccttt 142620
atttatttaa acatttatgc atagctggtc tatattttat attggaacca gttcaatatc 142680
tgaaatattt gggagtttta aatgtttttc ttaattttga ctattatttc tgttgtgtca 142740
atcatggtag caggccttct catgtattga ttctctctct tttttttttt ggctaattaa 142800
ttaataattg atgggagttc tggagaccta aattggggtt tggggaaact ttccttcaag 142860
gaagactggc ttgtgtttct gctgctaccc tgagtatatc accaatttga gccagttagg 142920
catcctcttg agggtctcta atcagagtag atgtcccaga cttccctctg tcaccacact 142980
ccttatataa ggctttgctt ccctgataat gtgctgctgt aggcacctgt gcttaaatag 143040
tcccaccccc tgggctgcct cacagtcatc ctccttctcc tctgcctcag ctccttagta 143100
gtctggcccc caggcttttt gttgtttttt gttttttaga gggagaatga gaaaagaggt 143160
atagcgactc aggcttggca gcctctccta aattgtgaga ccacttatgt ctcataggga 143220
acatcctttc caggtttcag tgtcaggtta gtggaatggg atactacaag cctcatgtca 143280
gacataattc aaaaatccaa gtctgttaaa catttttaga ttatttatgt ttgtgccaaa 143340
agtattttaa aaacctaaaa taatactgaa actgagtata aattaagaaa acacatttaa 143400
taaagagtta atagataaga atgtaatgaa gtaaactagc caaaatatcc taatcatctg 143460
aaaacacatc taatcattta tttatattat tttgaaaggc ttcaatgatt cctctcatgt 143520
gtgttgccta gaatgttttt catgatttta ttttatattt tggataggct gctactgaag 143580
aggagaacag ccatggccag gcaaatggtc ttctcaatgc tccaagcctt gggtcaccaa 143640

ttcgtgtccg ctcagagatt actcagccag acagagatat tccactggtg cgaaagttac 143700
gttccattca cagctttgag ctggaaaaac gtctgaccct ggagccaaag ccagacactg 143760
acaagttcct tgagacctgg tataaaatag tgtatttttc tttttaaagc ttctaaggta 143820
ccattattat tgttgtcatt gttgttatta ttattgtata tttctgttac ataaagtctt 143880
tcaaataaga aatccttgca tttttgtaac actgagtcta ttcagctcca attttcatcc 143940
atgtttttaa ttattattat cctgattctt aattattata aattctatag catgtccttt 144000
ggctttggaa gctgagcagt aagagctgat gacttcctaa cactaggtac aagttaaatg 144060
aacattttta cagtaacttt gtttagaaag taatctcttc cacacaacag tgtagtgctg 144120
gagagggcat gataaagatg gcattaggca gagatgaggg gaatacataa aggaggggaa 144180
aaagtaattc atacacaagg gacggtgagt tcaattcact ttagtgaaga ccctctagga 144240
gtaagatact gtgggaaaac agataccaat aagtatatca tgcttgccct cgagagtttg 144300
caatctacct agagagaaag gaaggtgaaa cttgagagat ctatatacat aggtaaagat 144360
tgtagtgcat ggttttgagg cacattatcc ctacaacaaa ttttgataac agaagacaga 144420
aatgaaaaaa gtgtgggata tatttatgaa ttatagaaac ttcagtagta ttcgcagcat 144480
taagacctaa tttcctttaa tagtagtcat gcccttcaat ggtgcatctc tagtggctac 144540
tattggtaat gaaatattag gttagaggga ctgtgaattt gactaagatg gtaattactg 144600
tttcccaaga caattcagcc acttctggcc tactccaatc tggcgtccat tttaaacaat 144660
ccttcacata tgtgtgttct tcctcacatg gtaatcattc atagctatgt tatgtaagca 144720
catgtctaca cgctcacacg atcgaaagga tggatggggc attggtttat acattttgag 144780
ttgtggctca tattgagaat aaatatcaac atacaatagg gataaaagct cagttcaata 144840
aaaagttggt ttgacaaaat aagggaacta aaaagggcct tgctgaggaa agaactgagc 144900
tccatcttcc aaagcaggat gtggcaggta gagaagagca ttatcaatgc tgtgaaactt 144960
tccttgtgct ttatccatct ttttttctct catctttgtg tccatattag ttctttatgg 145020
agcttatata ttatataata aatgtttcct taggacagtt gagtaacttc tttccatttc 145080
cttaagcctg gagaaaatgc agaaagatac cagtgcagga aaagaatcta ttctccctgc 145140
tctgctgcat aagccttgcg ttcctgctgt gtcccgtact gaccacatct ggcactatga 145200
tgaagaatat cttccagatg cctccaagcc tgcttctgcc aacacccctg agcaggcaga 145260
tggtggtggc agcaatggat ttatagctgt taacctgagc tcttgcaagc aagaaattga 145320
ttccaaagaa tgggtgattg tggacaagga gcaggacctt caggatttta ggacaaatga 145380
ggctgtagga cataaaacaa ctggaagtcc ttctgatgag gagcctgaag tacttcaagt 145440
cctggaggca tcacctcaag atgaaaagct ccagttaggt ccttgggcag aaaatgatca 145500
tttaaagaag gaaacctcag gtgtggtctt agcactttct gcagagggtc ctcctactgc 145560
tgcttcagaa caatatacag ataggctgga actccagcct ggagctgcta gtcagtttat 145620
tgcagcgacg cccacaagtc taatggaggc gcaggcagaa ggacccctta cagcggtaag 145680

aaatttgttt ttattattat tctctgttat ccactcaagg tgctattaag aaaggactaa 145740
cagttgatca actaagatac ttcagtacag gcctaaattt tgcagatggc ccagggagtg 145800
attttgtact aagttctata gtcaaccctc caatatgaaa tttttcaaaa agtgaggtct 145860
tcttttatta tatcaaactc aatacatgaa atgtcattat aacctaattt tagaataaca 145920
tttttaatta gactacaagc gctagctatc tatgtgtgtt tatatatgtt acaatttttt 145980
gaagttccta gcaattttta ctatacattt actgaggaag gcatctattt aacccttcat 146040
ggtttgttct ttacactgta cactgttggg aggcctgagc ctttaattcc ctttctcttt 146100
cataaagtcc acccaggaat ttctcatact tcatattcct gttaaacggt aattttctaa 146160
cattgatggc cactgttcaa aatgcagagt cccaggcctc acttccagaa cttttgtttt 146220
agcaggtctg gggaagagcg tgctcaggaa tcctcatttt aacaagcagg tagaggcggc 146280
atttatgata caggtcttct gcaaaggaaa cttttgagga acgactattg tttattttca 146340
gttttgggca ttcagctgga gaagcctgtt atcctgttac cagtaaacag tggtgcccct 146400
ggtgactgtg tgaggaggag ccccagaaaa gccagagatg gccaagcaaa gccatgaaaa 146460
gcccccaggc taaataagta actgtggaca caaatcttag gcacccacag gtacatctgt 146520
ccttatgtga tgagagctac caggtttcct taaattccat aatcatctag cagtccccct 146580
cccccaactc accagaaaca gcattgttcc ccaaactgaa aggttttcta ttagaacaca 146640
attccacatc aagtgcaaaa cagtgggaaa agataccagt gaaaatacat tttgtgtaca 146700
gttctgagaa atttgcctgt gctccaaaat agggaaataa aggcctgcct gcttcttcac 146760
cagagttagt tagcaggtcc ctctttcctg ggactgcttt gtatagttag gcaggttgtt 146820
tgcagcataa aggaatctag ccaggaaaca aattagggct gaaatctacc ccagactttg 146880
ctttccaaac tgtgcatctt ggcacagggc tgtttccttt tggaagggca ttcttttcca 146940
aattgtacaa agctatcatg tgggctagag ggaggtaact actaactccc tccctccctg 147000
cttcatcctt tctcccaacc cacctttcct ctttccattc atccactctt tccaccttcc 147060
caggttcact ttgttcttta actctattat gctgtgtact aacttttgct ctagcatatt 147120
tccagcagca ttctggtgtg cctcccttca tggaggcttt ctaaaacctt tgctccattt 147180
ctcctggtat tttttgccct ctctcgacgc tgtcctattt acaaagattg tgctcatggt 147240
tctcttacac tactgcggtg ttttctctga caagttgttg ataggcttgt gttcatgtat 147300
gtgatgtttg accttatttt tagatatctt tgaattattt ttctttcttt ctttctttct 147360
ttctttcttt actgagacac agtctcactc tgttgcccag gttggagtgc agtggtgtga 147420
tctcggctca ctgcaacctc cgccttctgg gttcaagtga ttctcctgcc tcagcctccc 147480
aagtagctgg aattacaggt gcccaccacc aagcccggct aatttttttg tattaattga 147540
aagtctctaa tgaaatgtca gctgcatcaa ttgtatactt atttctcagt tttttatttc 147600
tattttagag gctaggtctt cctcagtcac ccaggctgta gcacagtgga atgatcatac 147660
cttactgtaa ccttgagctc ctgggctcca gtgatcctcc catgccagcg tcccaagtca 147720

ctaggactac aggcatgagt caccacaccc agctaatttt gctatatttc agggatagca 147780
agttggcacc aacctgtcag gttggcaatg aaaacttaca tctaaacagt taccttaaac 147840
aacctgaagt gatttgtaat ggggctgttg ttgttgttgt tgttgttgtt ttgtagagac 147900
agggtttttc catgttgccc agactggtct caaattcttg tgttcaagtg gtcctcccac 147960
attggcctcc caaagtgctg ggattacagg tgtgagctcc taaacctggc taatttttca 148020
atcttttgca agacggggtc ccactgtatt gcccagactg ggctcaaact gctggggtcc 148080
agagattctc ctacctcagc ctgtctataa tcttgggatt atagacatga gccactgtac 148140
ccagtcagtg cttcatttta atattacctt tagaaacagt gtaaatgtgg ctgggcacta 148200
gtaacatcac ccatatgtta tccattgcca ttgctatgta acaaattagc ccaaatttag 148260
catcgttaaa caacagtcat tatttcacac aatttcttgg agctagaaat gcaagagtgg 148320
cttagttcag tggttctagc tcatgtgtgg tctctcatga ggtggttgtc aagctctcag 148380
tcagggttaa aatcaattta aaagtttggc tggaaaggga agatccactt ccaagctcac 148440
tcacatgact gttggcaggc ctcagttcct tactggctgt tagccagagg ctctagttcc 148500
tcgccatttg ggcctctcca tagctgacat gacatctggc tttcccagtg agtgaggtga 148560
gagagacaga aatgggggaa gagaggtgaa gagaatgcat tcccaccgag atggaagcca 148620
cagtgtcttt tctaatctgt cagaagggac ataccatcac ttctgccata ttctattagt 148680
cacaaagacc aacgctgtac aatatatgag ggtctatacg agggtgtgaa taccaggaga 148740
cacctctggg tcattgggaa ccattttaga ggctgtctac cattattacc ttacgtttat 148800
actttacaga atacttccct atttgatctt aactgcaact ccatggaaca agcagaggga 148860
atattatttt ctcttttcgt accgagggca ctaagcctca ggacagggag aatccatgaa 148920
gagtcagacg ctggtaggct agacttggtg ttccgtccct cgtgccagga gattacacct 148980
tacaccagtc ttctgtccct acaccagggc attgccagca tactgaaaga ctttcctaat 149040
tggcttgttt cttagagatc gagaaaagga aagggggttt aaaataatgc acaaagaagg 149100
aacagtacca aaagaaaaag agtagcagta tttttaataa cctgttagcc aacatctttt 149160
gagagaattc ctctgactgt atttacagga ctaccaatac cagcactaat aaaatttaaa 149220
ttagcagtta gttgtattgc cttaattatg tttatttctt catacctgtg aagtatctgc 149280
taagttccag gctttacgct atacttcagg gataacaagt tggcaccaac ctgtcaggtt 149340
ggcaatgaaa agttacatct aaacagttaa tttaaacaac ctgaagtgat ttgtagtggg 149400
gctgtttaca ggaatgggga cacagacata tctgcctagg acttaggagt ggaatttggg 149460
atatctttat tttctcaact aatttttttt tagcatggta attaaaaatc tttctgggaa 149520
ggttaaggaa tgcttcaggc cgggcgtggt ggctcacgcc tgtaatccca gcactttagg 149580
aggccaaggc tggcggatca cgaggtcagg agttcaagac cagcctggcc aagatagtga 149640
aaccccatct ctactaaaaa tacaaaaaac tagccaggcg tggtggcagc acctataatc 149700
ccggctactc cggaggctga ggcagagaat tgcttaaaaa cctgggagcc ggaggttgca 149760

gtgagccgag atcgcgccac tgcactccag cctgggcgac agagcaagac tctgtgtcaa 149820
agaaaaaaaa aaaggaaaga aaaaaaagaa atagttccat gtggctttcc tggcaaggca 149880
tgctttgctt atttggatcc cttgtataaa ctacagtgac tgtgtgaaag ctcatggagt 149940
tattcagagc ctccactgac acaggaatga tccacacgtg tattaataga ggtgtgcttt 150000
aataatgtga aaaggtgaag actctttggg ttggttacca tctgtttaac ttcttaaaat 150060
ggattttgca gaagggttga atgtatgtat attgactggg gattggccaa atgtcctctg 150120
tctgaccatg aattcctatt tttgatctgg aaaatttggt cattataatc ataggaaagt 150180
ttttttgtaa ttgtatttga tattacactt caacctatgt acagtcagaa aggatcagtg 150240
attggatgga ctgcactgga aagtaaagaa agcccatttg tgaagtgctt gagtacagac 150300
tggaatatca cttgggagat ttttgtggaa cctcagcatg caatggttga tatactcatg 150360
tgtgcaacat ttgttgagtg tctactgtga gttgggtact atgctaagta ccaagaataa 150420
aaagcccagt tagtcacctc actgctttca agggtcttca atctagtaaa gaaacagttt 150480
aagtgttata ggtgctgtga tagaaatttt gtgttgtaga agttggacta gatttatccc 150540
taagttgtct ttattattat tattattata ctttaagttc taggatacgt gtgcatgacg 150600
tgcaggtttg ttacataggt atacatgtgc catgttggtg tgctgcaccc atcaactcat 150660
catttacatt aggtatttct gctatccctc cccctgcccc ccaccccacg acaggccctg 150720
gtgtgtgatg ttccccaccc tgtgtccaag tattctcatt gttcaattcc cacctatgag 150780
tgagagcatg tggtgtttgg ttttctgtcc ttgtgatagt ttgctgagaa tgatggtttc 150840
cagctgcatc cacgtccctg caaaggacat gaactcatcc tttttatggc tgcatagtat 150900
tccgtggtgt atatgtgcca cattttctta atccagtcta tcattgatgg acatttgggt 150960
tccaagtctg ctgttgtgaa tagtgccaca ataaacatac atgtacatgt gtctttatag 151020
tagcatgatt tataatcctt tgggtatata cccagtaatg gcattgctgg gtcaaatggt 151080
atttctagtt ctagatcctt aagaaatcgc cacactgtct tctacaatgg ttgaactagt 151140
ttacactccc accaacagtg taaaagtgtt cctatttctc cacatcctct ccagcacctg 151200
ttgtttcctg actttttaat gatcgccatt ctaactggtg tgagatggta tctcactgtg 151260
gttttgattt gcatttctct gatggccagt gatgatgagc attttttcgt gtgtctgttg 151320
gctgcataaa tgtcttcttt tgagaagtgt ctgttcatat cctttgccca ctttttgttg 151380
gggttgtttt tgtcttgtaa gtttgtttaa cttctttgta gattttggat attagccctt 151440
tgtcagatga gtagattgca aaaatgttct cccattctat aagttgcctg ttcactctga 151500
tggtagtttc ttttgctgtg caggagctct ttagttttaa ttaaatccca tttgtcaatt 151560
ttggcttttg ttgccattgc ttttggtgtt ttagtcgtga agtccttgcc catgactatg 151620
tcctgaatgg tattgcctag gttttcttct agggttttta tggttttagg tctaacatgt 151680
aagtctttaa tccatcttga attaattttt gtataaggtg tatggaaggg atccaatttc 151740
aactttctac atatggctag ccagttttcc cagcaccatt tattaaatag ggaatccttt 151800

ccccatttct tgtttttgtc gggtttgtca aagatcagat ggttgtagat gtgtggtgtt 151860
atttctgagg cctctgctct gttccattgg tctatgtatc tgttttggta ccagtaccat 151920
gctgttttgg ttactgtagc cttgtagtac agtttgaagt caggtagtat gatgcctcca 151980
gctttgttct tttggcttag gattgtcttg gcgatgcggg ctcttttttg gttccatatg 152040
aactttaaag tagttttttt ccaattctgt gaagaaagtc attggtagct tgattgggat 152100
ggcattgaat ctataaatta ccttgggcag tatggccatt ttcacgatac tgattcttcc 152160
tatccatgaa catggaatgt tcttccattt gtttgtatcc tcttttattt cattgagcag 152220
tggtttgtag ttctccttga agaggtcctt catatccctt gtaagttgga ttcctaggta 152280
ttttattctc ttagtagcag ttgtgaatgg gagttcactc atgatttggc tctctgtctg 152340
ttattggtgt ataggaatgc ttgtgatttt tgcacattga ttttgtatcc tgagactttg 152400
ctgaagttgc ttatcagctt aagaagattt tgggctgaga tgatggggtt ttctaaatat 152460
acaatcatgt catctgtaaa cagggacaat ttgacttcct cttttcctga ttgaataccc 152520
tttatttctt tctcttgcct gattgccctg gccagaactt ccaacactat gttgaatagg 152580
agtggtgaga gaggacatcc ctgtcttgtg ccagttttca aagggaatgc ttccagtttt 152640
tgcccattca gtatgatatt ggctgtggtt ttgtcataaa taactcttat tattttgaga 152700
tacgttccat cgatacctag tttattgaga gtttttagca cgaagggctg ttgaattttg 152760
ttgaaggcct tttctgcatc tgttgagata aacatacggt ttttgtcatt ggttctgttt 152820
atgtgatgaa ttatgtttat taatttgcat attttgaacc agcattacat cccagggatg 152880
aagccaactt aatcgtggtg gataagcttt ttgacgtgct gctggattcg gtttgccggt 152940
attttattga ggatttgtgc atcaatgttc atcagggata ttggtctaaa attctctttt 153000
tttgttgtgt ctctgtcagg ctttggtatc aggatgacgc tggcctcata aaatgagtta 153060
gggagggtgc cctctttttc tattgattgg aatagtttca gaaagaatgg taccagctcc 153120
tctttgtacc tctggtagaa tatggctatg aatccgtctg gtcctggact tcttttggtt 153180
ggtaggctat taattattgc ctcaatttca gagcctgtta ttggtctact cagagattca 153240
acttcttcct ggtttagtct tgggagggtg tatgtgtcct ggaatttatc catttcttct 153300
agattttcta gtttatttgc atagaggtgt ttataatatt ctctgatggt agtttgtatt 153360
tctgttggat ggtggtgata tcccctttat catttttatt gcgtctattt gattcttctc 153420
tcttttcttt tttattagtc ttgctagcgg tctttcaatt ttgttgatct tatcaaaaaa 153480
ccagctcctg gattcattga ttttttgaag ggtttttttg tgtctctatc tccttcagtt 153540
cttgccttct gctagctttt gaatgtgttt gctcttgctt ctctagttct tttaattgtg 153600
atgttagggt gtcaatttta gatctttcct gctttctctt gtgggcattt agtactataa 153660
atttccctct acacactgct ttaaatgtgt cccagagatt gtgtcacatt gtgtctttgt 153720
tctcactggt ttcaaagaac atctttattt cttccttcat tttgttattt acccagtagt 153780
cattcaggag caggttgttc agtttccatg tagttgtgca gttttgagtg agtttcttaa 153840

tcctgagttc taatttgatt gcactgtggt ctgagagaca gtttgttata atttctgttc 153900
ttttacattt gctgaggagt gctctacttc caactgtgtg gtgaactttg gaataagtat 153960
gatgtggtgc tgagaagaat gtatattctg ttgatttgag gtggagagtt ctgtagatgt 154020
ctattaggtc tgcttggtgc agagctgagt tcaagtcctg gatatccttg ttaaccttct 154080
gtctggttca tctgactaat gttgacagtg gggtgttaaa gtctcccatt attattgtgt 154140
gggagtctaa gtctctttgt aggtctctaa ggacttgctt tatgaatctg ggtgctcctg 154200
tattgggtgc atatatattt aggatagtta gctcttctta ttgaattgat ccctttacca 154260
ttgtgtaatg accttctttg tctgttttga tctttgttgg tttaaagtct gttttatcag 154320
agactaggat tgcaaccctt gctttttttt tttttttttt tttgctttcc attcgcttgg 154380
tagatcttcc ttcgtccctt tattttgagc ctatgtgtgt ctgtgcacgt gagataggtc 154440
tcctgaatac agcacactta tgggtcttga ctctttatcc agtttgccag tctgtgtctt 154500
ttaattgggg catttagctc atttacattt aagattaata ttgttatgtg tgcatttgat 154560
cctgtcatta tgatgttagc tggttatttt gccagttagt tgatgcagtt tcttcctagc 154620
atcgatggtc tttacaattt ggcatgtttt tgcagtggct ggtaccaatt gttcctttcc 154680
atgtttagtg cttctttcag gaactcttgt aaggcaggcc tggaggtgac aaaatctctc 154740
aacatttgct tgtctgtaaa ggattttatt tctccttcac ttatgaagct tagtttggct 154800
ggatatgaaa ttctgggttg aaaattcttt tctttaagaa tgttgaatat tggcccccac 154860
tctcttctgg cttgtagggt ttctgccaag agatctgctg ttagtctgat gggcttccct 154920
ctgtgggtaa cctggccctt tctctctggc tgcccttaac atttttcctt tcatttcaac 154980
cttggtgaat ctgacaattt tgtgtcttgg agttgctctt ctcatggagt atctttgtgg 155040
tgttctctgt atttcctgaa tttgaatgtt ggcctgcctt gctagattgg ggaagttctc 155100
ctggataata tcctgcagag tgttttgcag cttggttcca ttctccccat cactttcagg 155160
tacaccaatc aaatgtagat ttggtctttt cacatagtcc gatatttctt ggaggctttg 155220
ttcatttctt tttactcttt tttctctaaa cttctcttct cgcttcattt cattaatttg 155280
atcttcaatc actgataccc tttcttccac atgatagaat cggctactga agcttgtgca 155340
tgcatcatgt agttctcgtg ccatggtttt cagctccatc aggtcattta aggtcttctc 155400
tacgctgttt attctagtta gccattcgtc taatcttttt tcaaggtttt tagcttcctt 155460
gcaatgggtt caaacatcct cctttagctc ggagaagttt gttattagcg accttctgaa 155520
gcctacttct gtcaactcat caaattcatt ctccgtccag ctttgttccg ttgatggcga 155580
ggagctgcaa tcctttggag gggaagaggt gctctggttt ttagaatttt cagcttttct 155640
gctctggttt cttcccatct tcccatcttt gtggttttat ctacctttgg tctttgatga 155700
tggtggccta cagatggggt tttggtgtgg atgtcctttc tgtttgttag ttttccttct 155760
aacagtcaga tccttcagct gcaggtctgt tggagtttgc tggaggtcca ctccagaccc 155820
tgtttgcctg ggtatcacca gcagaggctg cagaacagca aatattgcag aacagcaaat 155880

attgctgcct gatccttcct ctggaagctt cgtctcagag gggcacctgg ctatatgagg 155940
tgacactcag cccctactgg gaggtgtctc ccaattaggc tacacgggtg tcagggaccc 156000
acttgaggag gcagtctgtc cattctccga gctcaaactc catgctggga gaagcactgc 156060
tctcttcaga gctgtcagac agggacgttt aagtttgcaa aagtttctgc tgccttttgt 156120
tcagctatgc tctgccccca gaggtggagt ctacagaggc agcaggcctt gctgagctgc 156180
ggtgggctct gcccagttcg agcttccctg gctgctttat ttacctactc aagcctcagc 156240
aatggcagac gcccctcccc ctgccaggct gctgcctcac aagttgatct cagactgctg 156300
cgctagcagt gagcaaggct ccgtgggcgt gggacccgct gagcctggcg caggatgtaa 156360
tctcctggtg tgccgtttgc taagaccttt ggaaaagcac agtatttggg cgagagtgtc 156420
ccatttttcc aggtaccgtc tgtcacggct tcccttggct agggaaggga aatcccccaa 156480
ccccctatgc ttcccgggtg aggcgatgcc ctgccctgct tcagcttgcc ctccatgggc 156540
tggacccact gtccaaccag tcccagtgag atgaaccagg tacctcagtt ggaaatgcag 156600
aaatcacctg tcttctgcgt cagtcatgct gggagctgca gactggagct gttcctattc 156660
ggccatcttg ggacagaacc cctaaggtgt ctttcaatac tgcatgaagg ggctagaaaa 156720
gtgactgttt aatcctgtag ccttgtatat aatatgcaat tacaggcctg ccaaaaaata 156780
tttttgtttt agtccatctt ttctgggctt ctattctttg gatatgagtt gtggcttatg 156840
atttttagta gtcccacaaa atttttataa ttggaaaccc ctttaaagac acattaaact 156900
ttcaataatg agtgacttgt ctggggagtg ggatgacaag gtaaggacaa aagcagcttt 156960
tacttttcat tcacgtaggt tttgaggctt taggttttta gttttgccca cctttgtggc 157020
aaagactgct gccttcccca ctcttcccca tccaccctca cctcaatcca cattggccaa 157080
agtggaagat accatgtatg gcctcttaaa aaagttataa ttggcacata ataatcatac 157140
atatgtatga ggtacatagt gatgtcttga cacatacaat gtatagtgac tagatcagtg 157200
taactagcat ctacatcatc tcaaacgttt atcatttctt tgtgttggga gcattcgata 157260
tcctttcttc tatctatttg aaaatatata ttattgggcc agacgcagtg gctcatgcct 157320
gtaatcccag cactttgaga ggctgaggca ggtggattac ctgaggtcag gagttggaga 157380
ccaacctggc taacatggtg aaacgctgtg tctactaaaa acacaaaaat tagacaggcg 157440
tgatggggca tgcttgtaat cccagctact tgggaggctg aggcaggaga actgcttgaa 157500
cccaggaggt ggaggttgca gtgagcaaag atcgcactct gtgctccagc ctgggtgaca 157560
gagtaagact ctgtctcaaa aaaaaaaaaa aaaaaaaaaa aaaaaaaaaa aaaaaaatat 157620
atatatatat atatatatat atatatatat atgtatatat atatatgtat gtatatatgt 157680
atatatatgt gtatatatat atatacgtat atatatatat atataaaatt gttaactgta 157740
atcatcctat agtgctatgg aacactagaa gttattcctt ctctctagct gtaattttgt 157800
atcctttaac aaatctttcc ctatctcccc gttcccgcta ctctttccag cctctaataa 157860
cctctcttct actttttact tacgtaaaat caactttttt ttagcttcct catatattag 157920

tgagaacatg cggtatttgt ctttctttgc ctggcttatt tcatttaaca taacagcctc 157980
cagtgccatc catgttgcca tgaatgacag gattttattt tttatggctg aatagtattc 158040
cattgtttgt ttataccaca ttttctttta tttatttatt tatttatttt ttgagacagc 158100
atctcactct gtcacccagg ctggagtaca gtggtggtga tcttggctca gtgcagccac 158160
cacctcccgg gttcaagcaa tgctcctgcc tcagcctcct gagtagctgg gactacaggc 158220
acatgccacc atgcctggct aatttttgta ttttttgtgg agactgggtt tcaccatgtt 158280
ggccaggctg gtctcgaact ctgacctcag gtgatccacc tgccttggcc tttcaaagtt 158340
ctgggattac aggcatgagc caccgtgccc ggccgctata ccacattttc tttatccact 158400
catctgttgt tgtacacttg ggttgattcc atatcttggc tattgtgaac agtgatgcag 158460
taaacgtgag tgtgcagata tctcttcaat atactgatct tcccagtagt gggattgctg 158520
aattgtatgg tagttctatt tgtagttttt tgaggaacct acatactttt ctccatagta 158580
gtaactgtac tagtttacat tcccactgta ttactctgtt ctcatactgc tgataaagac 158640
atacccaaga ctgagcaatt tacaaaagaa agaggtttaa ttggacttac agttccacat 158700
ggctggggag gcctcacaat catggtggaa ggcaaggagg agcaagtcac atcttacatg 158760
gatggcagca ggcaaagaga gcttgtgcag gaaaactccc ccttataata accatcagat 158820
ctcatgagac ttactcacta tcacgagaac agcatgagaa agacctgccc ccatgattca 158880
gttacctccc actgggtccc tcccacaaca tgtgggaatt caagatgaga tttgggtggg 158940
gacacagcca aaccgttaca cccacaaaca ctgtatttga gagttctctt ttctctgcat 159000
ccttgccagc atttgttatt tttttgtctt tttgatgata gccattttta ctggggtgat 159060
acaatatctc attgtggttt ttatttgcat ttccctgatg actagtgata ctgaacatgt 159120
tttcatgtat ttgttggcca tttctatatc ttcttttgag aaatgtctgt gtagaccatt 159180
tgcccatttt ctaatgagat gtttttcttt cctttttttt tttttggct gttgagatgt 159240
ttgagtttct taaatattct ggatattaat cctgttagat gaatagtttg tagatatttt 159300
cttccatttt gtaggttatc ttttcactct attgactgtt tcctttgctg tgaagaaggt 159360
tttcctttgc tatcatccca ttagatgaat agtttgtaga tattttctcc cattttgtag 159420
gttatctttt cactctattg actgtttcct ttgctgtgaa gaaggttttt agtttgatat 159480
aatccagttt atttttttgt tgcctgtgct tttgaggtct tattcataaa gtatttccct 159540
agaccagtgt tctgaaacat ttaccctatg ttttcttcta gtagttttgt agtttcaggc 159600
cttgcattta ggtctttaat ccattttgag ctgattttta tataaggtga gagatggggg 159660
tctagtttca ttgttctgca tgtggatatc cagttttccc agcatcattt attgaagaga 159720
ctgttctttc cccagtgaat gctgtggtgc ctttgtcaaa ctcagttggc tgtagataaa 159780
tggatctatt tctggattct ctgatctgtt ctgttggtct atgtgtctgt ttttatgcca 159840
gtaccatgct gttttggtta ctatagcttt gtagtataat ttgaaattag gtagtgttat 159900
acctccaact tttgttttgt tttcttttgt atttttgctt aggattgctt tagatatttg 159960

gggtcggatt gtttttctat ttctgcaaaa aatgtcattg gtattttgat agggattgca 160020
ttgaatctgt agcacttcag atactatagg catttaaaca ctattaattt agcaatatta 160080
attcttccaa tacaagagca tgagatacct ttccatttta ttgtatgttc tcttcagttt 160140
ctttcattag tattttctgg ttttacttgt agagatcttt cacttctttg gttaaattta 160200
ttcctagtta ttttatttgt taagcaaaca aataaaatac agaaaatcta gtagatcctg 160260
taggttttct tcattaaaaa attttatttt tgtctgcctg agttattcaa aagacctgtc 160320
ttccaattca gaaattctgc ctggtctagt ctgttgttga agctctcaat tgtatttttt 160380
atttcattca ttgaattctt tttttgtttt tttttttttt tttttgagat ggagtctcac 160440
tctgccaccc aggctggagt gcagtggcat gatctcagct cactgcaacc tctgcctcct 160500
ggttcaagca attcttctgc ctcagcctcc tgagtagctg ggattacagg tgcccaccac 160560
cacacctggc taatttttgt atttttagta gagacagggt ttcaccatgt tggccaggct 160620
ggtctcaaac tcctgacctc aagttatctg cccaccttgg cctcctaaag tgctggtatt 160680
ataggcgtga gccaccacac ccggcccatt taattcttta gtgctaagat tcctttggtt 160740
cttttttatg atatctattt ctgttgaatt tctcattgaa atcatgaatt gtttttctga 160800
tttaactgaa ttgtctatct ttattctctt atatgtcact gagtttcttt aaaattatta 160860
tttagatttt tttctgggat tttgtataat tccttatgat tggggtcttt tactggagaa 160920
ttatcattcc ttgggtgttg tcatatttcc ttgctttttc atgtttgatg tgtccctatg 160980
ttgatttcta tgcatctggt agaacagttg actctccaaa ttttatggag tagatttcac 161040
agggaaatac ttgggtcttg gggtatcagt tgggtgtggt gcctttgctt tgatgctagg 161100
tggatgcaat aatgtagtct tcatgcaatt tcttcagctg taacccatgc tggtgatgtt 161160
tgcaagtgtc tctgtggcct aaggtgagag tttgtggcag tgttggcatg gtttttctgg 161220
gtatggtctc accaggctgc ttctcaggtc agggtctgca tgtgcagaca agtgggtcag 161280
ccagtttgga gtctggctca ctggggttgg ggccacagtg ctgttactct ggccagggac 161340
atgggctcat ggctgctggg ctgggctggg aggcacacct gccaggggca ggctcagcag 161400
gctgtttctc aggtccaagg cgcaggtatg cagccgctca gctggcctgg gggtgtatct 161460
gccaggagct ggcctgagcc agctgtccct gagtgctggc aaatacactg acctgaaggt 161520
gggtcagctc ctcaggggtt tggaagcctt tcctgctcaa gggtgtgtct gccctgtgtg 161580
ggactgccag actgtttctc tacctggaag tgtgcatggc aggggttagt tttcctgctg 161640
tgcaggactc aaatcacagc tgatcctgag cccaggctcc aagcagtcag ggtcatgaca 161700
ttgtagccac cagccacctg tgtgagcttg agggaatgaa gatggagtcc aagggctgga 161760
caggtgcagt ggcttctggc ccccagaaca gggtacactc cagaagtggc tctggcctta 161820
gggtggcatt aagctgtggc agcttggctc atgggtgggt gggaatgctt accttgttct 161880
cctaatctgg agcaaagcag ctgtgtgaat ttttagcagc tctccaaact aggctccagg 161940
ctccaggctc gctaggactg ttggtttctc ttgtagtaag gattgtaggt gtttgcagtg 162000

gcagtgaggg ctgatgaggc tgttctgctt acctttttcc cacaacagga agtccctcct 162060
gactccagcc caatctgggc ttgggtaacg gggtggcaga ggctgggtgc ctccatgctg 162120
ccttcttggg cttctttttt tttttttttt tttttgagat ggaatctcac tttgtcaccc 162180
aggctggagt gcaatggtgc gatctcggct cgccacaacc tctgcctcat gggttcaagt 162240
gattctcctg cggcagcctc ccaagtagct gggattacag gcatgtgcca ccatgcctgg 162300
ctaattttgt atttttagta gaaacggggt ttctgcatgt tggtcaggct ggtcttgaac 162360
tcctgacctc aggtgatctg cctgcctcgg cctcccaaag tgctgggatt acaggcgtga 162420
gccaccgcgc ccagcctata attttcaaca tttctataaa gtattatata aagctgcttt 162480
agacaaaaaa ggtctatggt agaatttgaa tggctaaaat ttaacttctt aataggccag 162540
gctgtattag gtaccaatac attattctat gtatatctct gctcaattat ttgtctttgt 162600
caacaaacaa aaatgagaca tagaaatttc atataccaca ttcttttcat ttggaagaaa 162660
ggctatttgg aatggatgtt gaatttcgca gtttttatta tcttagtaac attacaatcc 162720
ttgaatgaga gaaaagtaaa aggataatct gtaagttaaa atgcaaatgg tagttggttt 162780
gcagctcatt gtttctcact agtggctagt ttgaaacatg gggtgactga aaaatagtga 162840
ggctgcttta cctgaactat taaataagac actaattttg gtcaaacagt actaggcact 162900
aatagaacta agaaactatg aaagaaatgg aaaatattga gatccttttc taattcttta 162960
gaaggatgta catggtacaa ttacagaaca gaagaaagga aatgattgat taatgccata 163020
atgaaatgtt tgtgaattat ggaaattaaa gatataaaag atgcatatgg cttactggca 163080
gattatttat ttggtccatt tgtagagcgc cccaagtatg agaactgctt ttatgtcttt 163140
atagtttaag gaagctcttt gcagctacac ttagaattag ctttctcttc tctatactca 163200
actactgatc ctaggttttc tccttttatt ttgtcagata atgttattgc agactcagtt 163260
tccctgactt tgagagtgtg tagtacatgt cagtaggctg tactgtgctg tgcacaactt 163320
tcaaaatcct gaagtcctat ctatgacttc ttggctgtgt ttttcctcat ttgtgaattt 163380
gtattagtaa tagtactcta accgggcaca gtggctcata cctgtaatcc cagcactttg 163440
ggaggcctag tcaggaagat ctcttgagcc caggagttca agaccagcct gggcaacata 163500
gtgagaccct gtctgtgcaa attttttttt tttttaatta gctggtgtgg tgctacatgc 163560
ctgtagtctc agctacttgg gagtctgagg tggagggatt gcttgagccc aggagttaga 163620
ggctgcagtg agctgtgatt gcaccactgc acctagcctg ggcgacaaag caagactctg 163680
tctctaaaaa taataataat aattaaaaaa tgataatagt acctgattaa aatataaaga 163740
agctatgaaa tggggtttgt aaaaatgatc tgggaaatat gaaatttaaa gtactatgga 163800
aatattatat gagagtagtt tatgcacagt ttatcataat tgctcagaat cttttcaaga 163860
aaaattctcc catctggtga ttcatatata tttttattgt tgttgctttg gtgttttgtt 163920
tttgtttttg tttttgtttt ttttgaggtg gggtattgct atgttgccca ggctggtctc 163980
aaactcctgg gctcaagcaa tcttctgcct cagcctcttg agtagctggg attacaggta 164040

caggccacca tgcttgggtc atatatattt tctatgtata aaattgccac tctttttttt 164100
ataccatatg tatgtgcaca tttatatcct gaatctaaaa tgagttaatc tcagaactgt 164160
agaaattaac cctagcttca atctcagaag accaatggag tttttgtttt gtttgttttg 164220
ttttgagaca gagtgtcgct ctgttgccca ggctcgagtg cagtggcacg atcttggctc 164280
actgcaacct ctgcctccca ggttcaagcc attctcctgc ctcagcctcc caagtagctg 164340
gtattacagg cacccgccat cacccccagc taatttttgt atttttagta gagagagggt 164400
ttcaccatgt tggccaggct ggtcttgaac tcctgacttc agatgatccg cccacctcag 164460
cctcccaaag tgctgggatt acaggcatga gccaccgtgc ttggcctgga gtattatttt 164520
tatagcactg ctgtgttcat tttgacgaat gtacttagaa ttacatgtaa taattaggtg 164580
actggttttt tttgtttttg tttttcagac agggtccagc aatctggctc tgtcacacag 164640
gctggagtac agtggcatga tcttggctca ctgcatcctc cgcctcccag gttcaagcaa 164700
ttttcccacc tcagcttccc gagaagctgg gattacaggc gcccaccacc atacctggct 164760
aattttttgt atttttagta gagacagggt ttcaccatgt tggccagtct ggtctcaaac 164820
tcctgacctc aggagatcag cctgcctcgg ccccccagag cgctgggatt ataggcaaga 164880
gccaccacac ctggcccgat tttgaatata ggttatcatc tcccttggta tttgacattt 164940
aatataattt cttacacttg cttttcttat catggtcact tttgggtgca tttactaaga 165000
agtaaatttt gaaaagtgaa ttactataaa ttttagattt caacatcact ttcaaaatat 165060
actagaaaat tgccatctgt ctaaatatat tttagaataa ttaaaacaga aatatgagat 165120
aacttcctga aggaaaacag taccaataga acctaatgct ttttcccaag gtcattttag 165180
atttgtgctt attctaagag tttagctcta aacccaaatt gcctgtcttg gattagcctg 165240
aaagatcctc aggaccgtgg ttgcatttgg gagctattcc aggacattag ttaggtgttt 165300
tttttcctgg gctggactta ggacaagctt tacatgtcaa tgagctttac agatccatgt 165360
ccaactaata gtattcctga cacaaagtga aatagatctg aacatcttag ctgaccctgg 165420
aaccacatgg tttcttggtg tctttggact agagatcgac tctaaactaa atctcctatg 165480
actaatccaa attcattttg cattgtattc agaccaattt ctgtagtttt cctagaactt 165540
aatgggtctc attaagtgat ttgaacttac ttgatttctc aaaaagactg tttttcttta 165600
ttagtttata tagttatttt cagcctactt acatgtcact ttaggtgacg tgaaaataat 165660
aaaaacttta ggtatcttaa ggaaggacat tcttattaga tattgaatct tattcatata 165720
gaattaaaat tacctcagtg gaaggggggt agattttcac ttctcatagg cttgattttt 165780
tttttttttt ttttttttcc tgagatggag cttcactctt attgcccagg ctggagtaca 165840
atggcacaat ctcggctcac cacaacctct gcctctcggg ttcaagcgat tctcctgcct 165900
cagcctcctg agtatctggg attacaggca tgtgccacca cgcccagttt attttgtatt 165960
tttagtagag acggggtttc accatgttgg ccaggctggt ctcgaactcc cgacctcagg 166020
tggtctgccc acctcggcct cccaaagtgc tgggattaca ggcgtgagcc accgcaccca 166080

gcccgcttga tctttaaaaa ttggtcaggt gtgggctggg tgcggtggct cacacctgta 166140
atcccagcac tttgggaggc cgaggtgggt ggatcaccta aggtcgggag ttcaagacca 166200
acctgaccaa catggtgaaa ccccgtctct actaaaaata cagaaattag ctgggcatag 166260
tggtgggcac ctgtaatccc agatacttgg gaggctgagg cagggagaat cacgtaaacc 166320
caggaggcgg aggttgcggt gagccaagat tgcaccattg cactccagcc tgggcgacag 166380
agcaagacaa catctcataa aaaaataaaa taaaataaaa taaaaaattg gccaggtatg 166440
gtggcttaca cctttaatcc aagctctttg ggaggccaag gcagatggat cacttgagct 166500
caggagttcg agaccagcct gggcaacagg tgacacccca tctctacaaa aatgcaaaaa 166560
ttagccagat gtggtggtgc atgctgtagt cccagctact cgggaggctg aggtgggagg 166620
atcacttgag cttggaaagc agaggttgca gtaagccaag atcacaccac ttacacttca 166680
gtctgggtga cacagtaacc gtgtctcaaa aaaaaaaaaa aaaagaaaac attagagaaa 166740
tcatgaaaca ttagtaaata cctttgtatg tctttacttg taggaagata taatcctagt 166800
atttaataga atttatggaa aacaacaaaa tgcatgcaaa tttggagttt ttcaatgaac 166860
aattatttgc caataatttt ttcctgtaat cctgtatctg gattctggca gaaaatagat 166920
ggcatactca aagggacttg gagagaattt aatgtaagga attttttaca gatgtatggg 166980
cagagttaag agagccagtg ggaccagtaa cagagggagc cattcctacc atgagtcctg 167040
aaaggggagt agaagtgggt gttcgcagaa cttggcgagg gtagtagctg agagagaggc 167100
cataagaagg gagctgtggc caggatagtg gaatgtagct gtcactagtg ccacagtcct 167160
cagtggggaa gcaaggagaa tcattaccca gccccctctt tcctctaacc ctctgattgc 167220
ctgttggtgc catccattga gcagatctaa ccacaaatca gaaagccagg aaacccaggt 167280
ggtgcagtct atagaaatta gtcttccagg gcatacagca caccaaagaa ggggcaggga 167340
taaacctaca gggagagact gttctgcaca gtttctgagt agggtagctt gagtgtcaat 167400
cattgttatt cagaattgtt catttttctt ataagttggg cagacctggt caacatctgt 167460
gttcttccta taaattagaa ttggagatgt taaatctcaa agcatgcatc tttgttgata 167520
ttttacataa ttgcatttta attttttctt ccccttctcc agattacaat tcctagacct 167580
tctgtggcat ctacacagtc aacttcagga agctttcact gtggtcagca gccagagaag 167640
aaagatcttc agcccatgga gcccactgtg gaactttact ctccaaggga aaacttctct 167700
ggcttggttg tgacagaggg tgaacctcct agtggaggaa gcagaacaga tttggggctt 167760
cagatagatc acattggtca tgacatgtta cccaacatta gagaaagtaa caaatctcaa 167820
gacctgggac caaaagaact tcctgatcat aatagactgg ttgtgagaga atttgaaaat 167880
ctccctgggg aaactgaaga gaaaagcatc cttttagagt cagataatga agatgagaag 167940
ttaagtagag ggcagcattg tattgagatc tcctctctcc caggagattt ggtaattgtg 168000
gaaaaggatc actcagctac tactgaacct cttgatgtga caaaaacaca gacttttagt 168060
gtggtgccaa atcaagacaa aaataatgag ataatgaagc ttctgacagt tggaacttca 168120

gaaatttctt ccagagacat tgacccacat gttgaaggtc agataggcca agtggcagaa 168180
atgcaaaaaa ataagatatc taaggatgat gacatcatga gtgaagactt gccaggtcat 168240
caaggagacc tctctacttt tttgcaccaa gagggcaaga gagagaaaat cacccctaga 168300
aatggagaac tatttcattg tgtttcagag aatgaacatg gtgccccaac ccggaaggat 168360
atggttaggt catcctttgt aactagacac agccgaatcc ctgttttagc acaagagata 168420
gactcaactt tggaatcatc ctctccagtt tctgcaaaag aaaagctcct ccaaaagaaa 168480
gcctatcagc cagacctagt caagcttctg gtggaaaaaa gacaattcaa gtccttcctt 168540
ggcgacctct caagtgcctc tgataaattg ctagaggaga aactagctac tgttcctgct 168600
cccttttgtg aggaggaagt gctcactccc ttttcaagac tgacagtaga ttctcacctg 168660
agtaggtcag ctgaagatag ctttctgtca cccatcatct cccagtctag aaagagcaaa 168720
attccaaggc cagtttcatg ggtcaacaca gatcaggtca atagctcaac ttcgtctcag 168780
ttctttcctc ggccaccacc aggaaagcca cccacgaggc ctggagtaga agccaggtaa 168840
tgctgtgtgc tctccctgtg ttaagtgtgt aacaccattt ctatatctgc tttattgtag 168900
tccaccaatg gcaacatttc tgtgttgccc ccttctcagt atctttcttt cctgcctttc 168960
ccaggcctta cagcctagtg cagtagaagt aatcagattt tttccaattt attgcggtaa 169020
aatacacata acataaaatt tagcatcttt tgtttcgttt gtttttgaga cagagtctcg 169080
ctctgtcacg caggctggag tggagtgact caatctcagc tcactgcaac ctctgcatcc 169140
cgggttcaag agattctctt ctgcctcagc tacccaagta gctaggatta caggcacctg 169200
ccaccacacc aggttatttt attttatttt tttaaactag agacagggtt tcgctatgtt 169260
gggcaggctg gtctcaaact cctggcccca agtgatccac ctgccttggc ctcccaaagt 169320
gctgggatta caggcttgag ccactgtgcc cagctgtatc ttaatcattt ttaagtgtac 169380
agttcagtgg tagtaagtac attcacattg ttgtgttaca cttaatattc taaagttata 169440
acactttcat ttggatttat atcagcttaa cttcaatatc atgcagaaac tgctcctttc 169500
agtctccatc cccactcctt tcagctgttg atgttacaaa attacatctt tatacattgt 169560
gtgttcaaac acataaacta ataattttta aaatgcatta atctcttaaa ttatgtaaaa 169620
aacaaaatgt ggaattttaa ctgtccatgt gtttgccttt attaagatct ttatttattc 169680
atatggcttc aagttcctgt gtggcgccct ttcatttcaa cctacaggac tccctttagc 169740
atttcttgca agacaggtct ggtggtaaca aactccctta gcttttatct gagaatgtct 169800
taattactgc ctcatttttg aaggacagtt ttgccagata taggattctt agttgacatt 169860
tcttttagta ttttaaaaga agtactttaa agtactaaag tacttccagc ctcaatgttt 169920
ctgatgagaa gtgttctgat aatttttttg agaatctcaa gattctctgt ctttgtcttt 169980
tgacagttta attataatgt gtctcattgt gggtctctga gttcatccta cttcgagttt 170040
gttgagcttc tgggatgttg tatattcatg tctcttatga cttttgggga gtttgcaggc 170100
tttttttctt ttttttaagt attctctctg cctctttctc ttttctcctt ctggaactcc 170160

cacaatgtgc atgcaagtct gcttgatggc cccacaggcc ctttattgtc tgtttacttt 170220
tctttactcc tttttttttc tgtacctcag actcaatact ttccattgtc ctgtcttcaa 170280
gtttgctgat tttttcttct gcctgctcaa atctgctctc gaatccatct agtgaatttt 170340
ttattttagt gattgtactt tttagttcca aatttttttt ggttcttgtt aggttttctg 170400
tctcttattt atatttccat tttgttcata cattgttttc ttgactttct ccgtgtcttt 170460
ctttagtttt tcaagcatcc ttaagagagt tgtttcaaag tctttgtcta gtagctttgc 170520
tatcaggtct ttttcaggta caatttctgt tgatacattt ttttctcctt ggaatggacc 170580
atcctttcct gtttctgtgt aagttttttt attttttttg ttgaaaattg gatatttgaa 170640
tctaataatg tggtaactct gaaaataaga ttttcccctt tcttcgggat ttgctgtggg 170700
ttgtttttgt ttctttttat ttttgagaca ggatctcact ctgtcaccca ggctggagtg 170760
cagtggaata atcatgactt attgcagcct ctaactcctg ggctcaagtg atcctcctgc 170820
ctcagcctcc tgagtatctg gaactacagg tgcatgccag cacacctagc taatttgttt 170880
gtttgttttt ttgatggttg tagactgtct ctgtgctgag gaccagcctg agatgtaaac 170940
ctaaggtctc agaccttttc taagcctgta ctttttcctg actaagcata gtagctttct 171000
gatttcccca gtatatgtgg ttggttttga atatacaagt ctttaatatc tggctccgaa 171060
gaagggaaaa aagaaaaatg agggaagaag aaaaaagagg actggccctt ttaatcccct 171120
agcagtcact tcagccagag gagaaccagt ttgtaacaat ggggaaaaat gtaagagtta 171180
ctacctacct ctttctctgc tcttctgtga tcagaagcag caatgagtaa tcagagcaca 171240
gatccccagt aattggagga cagggttctt tttgcccacc tggttcttgt aagctgtgtg 171300
cacactgctc cagaaataag tgcacagctg cctgccatgg ggccgggaat gggggatggg 171360
tagctgttaa ctgtgctaag atctgaaata gactaaaatt aactgtaatt gacagtccaa 171420
gccttctcct ggaagttgca agcctttggt agactccaga tttccaagat aattacatca 171480
gacagattct gccagtgcag ttgcctaggt ggggagacag attcctagtg cttcctactc 171540
tactgtcttc ccagaatctt ctctgatcca ggttaatttt tggctctccc actttctagc 171600
agtaagaacc tccatttcct catctacgga gtgggcaaaa tatttgctta atagttttgg 171660
gagaatgaaa caaagccaca atcatctagg agttggtaca ttgcttagca tgtaataaag 171720
attatgtctg tctccagttg cattttgatg atcagcaatt ttctagcttt gttcagtcag 171780
aatcccatta tgtcccataa tgccattatg atgtcaaatg ctagtgaaga atacgttgtc 171840
catgatttga taaatggacc ctggtaacag tgctgtttat cttgattata atgaattaaa 171900
ccaaaaaaat cctatagatt tccctaggtc tgatggtata tacaaccata catagtaaag 171960
atactgcttt agtatatact gcatgctata ctttttattt aaatattatc ttgatatatt 172020
tgaccacaga atgtttatat tttgaggaag aggggaaaat attgggaggt ggtaaaccag 172080
tgaagagtgc tttccagggc caaatgtggt ggctcacacc tgtaatccca gcactttggg 172140
aggctgaggc aggcagattg cttgagctca ggagtttgag accagcctgg gcaacatggt 172200

gaaacctcat ctctacaaga attagccagg cacgggtgct acacagctgt attcccagct 172260
acttgggagg ctgaggtggg aggatccttc tatgagttgt tgtctgtctt catgtcaaaa 172320
cattctattg ggttagtttt ttttttttct tttgagacgg aatcttgctc tgtcacccag 172380
gcaggagtgc agtggtgcga tcccggctca ctgcaacctc tgcctctcaa gttcaagcga 172440
ttctcctgtc tcagtgtccc gaatagctgg gattacaggc acctgccacc acacccagct 172500
aattttttgt attttagtag agatggggtt tcgccatgtt ggccaggctg atctcgaact 172560
cctgacctca agctatccac ccacctcaac cccccaaagt gctgggatta caggtgtgag 172620
ccaccgtgcc ctgggttatt ttctgtaact aacccccttc tctgcaaatt atattgaaga 172680
tattttatcc tagtctgaat tttaacttgg gttgtagtgc ctttggtttg atagaaaagt 172740
ttagtgttca tggaactgaa cattttttat taattgtgtt ttatatcatg cataggaagg 172800
tccttcaacc ctaatatttt ttaaaaggta gtaatttcca gtacccttat agttttgtgc 172860
tttgtgtata aatttactat cgatctaaag tgtatcatta tatatgatgt gaagtttttt 172920
tccaagtagt tagtgatatt gcaatactca tttcccatag aaacatgggg atctttttgg 172980
attcaatgct ctgttccatt catttgtctg ttcctttgct gtacactttc cattaccata 173040
gttttattat tagaggggcc ctagggtcct tctcactacg taatttggca ttgccactaa 173100
aatatattgt agactgcttt atagcaggcc ctccccaccc cagggctgca cggcaggagg 173160
cattaccgcc tgagctccgc ctcctatcag atcagcagtg gcatcagatt cccataggag 173220
cgtgaaccct gttgtgaact gcacatggga gagatctagg ttgtacgctc cttatgagaa 173280
tctaattaat gcctgatgat ctggggtgga acactttcat cctgaaacca tccttgcccc 173340
tcctcccccc atcgtggaaa aattgtcttc cacgaaactg ctccttggtg ctaaaaaggt 173400
tggggaccac tgctttgtag tttataaaaa cacttttata tgttccattt tctttgattc 173460
ttaggccaaa ccagaaagac atttaggaca aagaagacat ttgctattca tatgaatact 173520
ctaagaattc tgaggcccaa gggccggggc actacggttc atgcttgtaa tcccagcact 173580
ttgggaggct gagacaggag gattgctaga gctcaggagt ttgacaccag cctgggcaat 173640
gtggcaaaac cgtctctaca caaaatataa aaattagcca ggcatgcact acaggtgcac 173700
acctgtcatc ccagctactc aggaggctga ggtgggagga ttgcatgagc ccaggaggtc 173760
gagactgcag tgagctgtga tcatcccact gcacttcagc ctgagtgaca gagcaagacc 173820
ctgtttaaaa aaaaaaaaaa aaaaaatctg aggcccaagc tcagttgcca gtatggagtg 173880
ggcagtcagt tccagcctcc accctcctta atcccagact attgacagga atagaaactg 173940
aggtccacag aaaccaatga ctactctttt ctcttgtaaa ttattaaatg agttgaatgt 174000
acatttatct gtagttcctc ctgttgctga gtgtagttca tgttgaagac ttaggggtga 174060
cagatccttg gcagaaaaaa gaaaattaaa aaaaaaaaca cttacattgt gggatttcgt 174120
gtttgtagga ttcctgtcct aacatcctta cacagtatgg tttactctct gtcttatgtg 174180
ctaacatgct agagacaatt taacatgcta gttaaattca tctgaaaatt agcagaataa 174240

acatggcata ggaggaaggg aacgaatacc atttaagggc ttcttcatgg cagatgttca 174300
tctacgtact tgggtcactg cccttggtct gttcagtgac cctgtgagat agacagtatt 174360
cccattgaaa gcagggagag attgaagaat ttgtccaggg tcatacaact agtatatgac 174420
agagtaaccc tgtatcctgt tagtctattt tctgatttct acacattcac attatgattt 174480
tgggacattg tgacttttta ggcacacttg aaatgaatta aaattaaaat atattttctc 174540
tgttctttta aggctacgca gatataaagt cctagggagt agtaactccg actcagacct 174600
tttctcccgc ctggcccaaa ttcttcaaaa tggatctcag aaaccccgga gcactactca 174660
gtgcaagagt ccaggatctc ctcacaatcc aaaaacacca cccaagagtc cagttgtccc 174720
tcgcaggagt cccagtgcct ctcctcgaag ctcatccttg cctcgcacgt ctagttcctc 174780
accatctagg gctggacggc cccaccatga ccagaggagt tcgtccccac atctggggag 174840
aagcaagtca cctcccagcc actcaggatc ttcctcctcc aggaggtcct gccaacagga 174900
gcattgcaaa cccagcaaga atggcctgaa aggatccggc agcctccacc accactcagc 174960
cagcactaaa accccccaag ggaagagtaa gccagccagt aaactcagca gataggagcc 175020
aggctgcatc tctttgaaag gtgtgagatc ttcctcctaa acctgatgca tgtgtgtccc 175080
tgtactttct atgtaaaaaa atcagtgttg atcttctctt gcaaaagaaa gtaacatgat 175140
caattattta taagaagaca taatacatga taaggaatta cctaaggcag gcagcaagta 175200
gattaggaat caatgtcttt gtacaagaag gaaaaataga gcaaaaatcc aagggggaga 175260
aactcattaa aatgagctct cattttttaa gctgcctttg aaacaaaaga gttgaggata 175320
ggagatagaa tggaatttta ggggggttgc ctaatttttt taagcctcaa ttcaaagatt 175380
atatagcaaa agtgaaactt cttgtttgat attttcattc aaaactttcc caccctgaag 175440
agtcattgat cagatattag attatataag aagtctgttg ccagggagcc agtattcatg 175500
tatatttggc ttgtgtgttt atttcgtgta ttgagaatga acacctttac tttgcctcat 175560
tcctagtacc ctccctggag ttcagatttt tttttaaaat tttgtatgtc tcgtctgatt 175620
caatctctct gcttttattt tatggtccta gttgtactat caaatccaat tacttttttt 175680
taggtccccc tgattttttt tttttagagc aagagttctt aacatattac atttttatta 175740
tgaaaaataa gaaagttagg taaaggaaag aaaagtctaa ctagagctat tttgcaggct 175800
ttagtgttta gggagagaaa gaaagtgtgg gttaatagcc ttcaagatag aagatgccct 175860
ttcatctctg ttaagtgtcc tcctttagaa acttgagtag aaggaaaact gaccagagta 175920
gactgcttcc ttaagtcttc tgggttccaa ctgtttgtaa tatcagcatc caagatgata 175980
cgagggaagc acaatgcttt ggactgtgat ttgagattta gaaataaatt agatatatta 176040
ttgaggctta gaatcctcaa actttgtatt ttatacattt agccaataag gaattaatat 176100
ctggggaaat aaatttaggc aaatatttct tttttaatgt tttattacct gcttctcctg 176160
tgttttagtt caacatttgg gcttcttggc ctgattttca tacaatctca atttacgaag 176220
ctgtaaagag gaagatattt gttctaatct cactcttcta ataggaatca ggcaaatgaa 176280

agtctaccag acttttaaaa tgggctgttt ttatactctc taggtgtttt gtgttgtaaa 176340

gaccttatta aggtcaggta aattggtctg cttgctgttg aaatttgcct tctagcaaac 176400

atatgtgctt tctgtttgac cttgtgtttg ctgccaaacc taatacagtt gaattggg 176458

<210> 6
<211> 123932
<212> DNA
<213> Homo sapiens

<400> 6
aaggggagat gctaccattc gctcctcagg acgagccctg ggaccgagaa atggaagtgt 60

tcagcggcgg cggcgcgagc agcggcgagg ttagtgtggt ggccaagacg ggctggagcc 120

aacccccgcc tccttcccct caagaccttg gctggggacg gagagaggag aggcgtaggg 180

ccgggaagcg ggctcccggg caagccccct tgatgctgcc tccactgccc tttcgctggg 240

gagcgggcgg ggatgagacg gcgggctcgg gcttgcggag tgagtgcagc ctgggcccgg 300

gctcggcctt gggcggagcg cctgcctggc gggccacgcc agagttgttg tgactaagtt 360

tcttcacttt ccgacccgaa ggggaactgg gggactggcg gggtgctgtg cctgactccg 420

cgcgtccctt tttctccgcc gcgcctagtt tgctggctgg gaacattttg ggggacagct 480

gcggactcca gctcgctgtt gaaccccgga cgctttacga acccagtgca acaaacacac 540

caaggtgact aagtaactct ggggacattt gcacataggc acgagtgttc cctggtaaca 600

gctttctcgt ttctctgtgg cttgttatgg ttccttgtaa tgacccatgt cgagatacac 660

tggcgaccta tctccagtcg ccaggtttca ggtgatgttc tactgcttcc tctcaggtcc 720

ccttgattct actgcactct tgctccaccc cttctccttt ccacaaccgc tgtccctgcc 780

ccgttggtct gtaaaagaag acgcaaagta aaccccagcc cttataaata agtcgaatct 840

ttggtaagac ttagcgaggt tcaaccaaaa tactttggtt cacctctctt cccactcttc 900

ttactctgaa actgttctgg ttgggctttg gtcagctgtg cagtgagaac ttagatgtac 960

acaggtgctt tgtttttatt gttgttataa aagcgggcag catttaaaca caaaaacgtg 1020

tcttttgtga tagaaagttg gatccttttc tgataactta atcgactgcg tactactcca 1080

gtagaggcca ccatacacct cctaaaaata gttggtgaaa ttgattcaca gtaggatatt 1140

ttgaggtaag actctcaatc ctcgacatct aaagggtgca actttgtctt caagtacttg 1200

ctccatttaa aaaaaataca catttggctg ttgtatgatt accttgtttt tttttcttta 1260

aatttttttg ttatttatca actaaactgg ggttaagatt acatgctaga ataacatgtg 1320

gtaaggaata tagtggaaat aaattcagaa gtaccctaaa tctgaaccct ctatacttac 1380

aggaaatatt ttactagtaa accaagcaat tccaatatac gttttaatat tttacatctc 1440

gtaagaacaa gataaatgaa taggcgttcc tttcttgttg aaatgtggga atgctaactg 1500

tttgcacaat tgtctagcaa gtctattgaa ggggagagga actttgttat attcatgtat 1560

gtaacttact gaacttatag gcatttagtt tataaaaaca gttaaatgct ttgatataag 1620

gatgggatgg gaggtgggaa tccttttgca tgcctactat tttattattt ctgttaactt 1680
tgaatgtagt atgttttaaa ttgtaattac cacatatggt ttttttattg tttgcagtat 1740
gttttaggta ttgtagagat ttaacaatgg gtctctctac ctcagcaatt tggaaaaata 1800
ctcgagtgga aattgttaat ccctatgaag taaaacgaaa ggtgaaggta atgtggtgaa 1860
gatagtattt ctgccttctt tctctaaaac agttatgatt ggttagattt aaccctgcaa 1920
gttacttaat aaaaaatatt ataggtgaaa actaaatgct acatttgaac agacccactt 1980
agtacttaga agagtttgtt tgtttttaat tatattcttt tgataagaag taaacaagtt 2040
tacttttgat aatttatatt ttaattacaa atttgttgat gttggcttta aaaatgctaa 2100
ttttgagtac aagctctaca accctcaatt gtgataattt taggactaag agtgtttttg 2160
ttgtagaata tatattttta aaatgtgcta gaatccttaa agaaaatgta ggtaagaatc 2220
tgttaacttt aatgtcaaaa gaaataattt ccgtccattt ctgtgtgtgt tgaatctggt 2280
aagattaaag attatagaca ctaccatata ttttggagtc tttttgtatc ttttacctgt 2340
cttgcttgca tggacagttg ttaacctttg aattatcatt cagaagtgtg taaattggtt 2400
gtttgagact cttaacatgt tttcccacag gaaaaactta ttaagggtac ttatattttt 2460
agatcagtac ataatagcga ggtataatga tgccgagcct aaccactgtt tgtaaaattg 2520
ttctgtggtg aaatgtgtca ggtttcaacc aataactagc ttggttccag tgagaggtag 2580
agaatgggcc tgaaaagggg aaacctggaa tcttttggtt tctgggaaga gagttcttag 2640
aagaggggaa atagggtaag caagaaactg attgtgagag ctcttgcagg gactgtctca 2700
atgaggtcta ctgggaatag gaacttcaaa gggcatcacc cctcacgcag tcacaattta 2760
agcttttgac tggattctgt caggatcaag aaagaggttt tacttgagat gggatgggta 2820
ttggtcattc ctgggcaagg atcataaaga acaacctgtt tgactaatgt gaacttttca 2880
tgatttaatg ccatgcatgt tacagtttgt tcgcttagtt gtttgttttt tggtgaggaa 2940
ccaagtgctt ctttatcaag ttgatgatgt aattttcaca tttcatatca tagtaaacgt 3000
gtagtacttt tatattattt ctttagcaat gctgttttta tttaagcaag ttagtttttt 3060
tttttttttt tttttttgga gtcagggtct ttctatgtca ttgtacattg tccaggctgg 3120
cctcgaactc ctggactcaa acaatcctca tgcctcaggt tcccgagtag ctgggactac 3180
aggtgctcac aaccatgcct gtctaaacaa attagttttg agagtatgta gtttactgat 3240
aaaaccaatt tgggaaaaac ttctagtttt aattaatttt catgagaccc tatagtaata 3300
gaggaaagaa cagctttgaa ggcaggtaaa tctgggttct tgtccgagct ccatgccata 3360
ctcatgatgt gacacaatcc acgttaccta acctctctga acctcacttt ctttgtctat 3420
aagttggaga aaaaacatct tcaagagttg ttatgtaaag aaaattcagt gctgggttta 3480
ataaacttag caagatgttt tgcacttagt ctctagtaca caatagttcc ttctcttttc 3540
ccacagtcat catgcctctt cccatgcccc taaagtctga actattgtca tctatgttaa 3600
aatcctggga ttcagataac catggcctta gccgcagcct tccatttcgt aagcagtttt 3660

tcttaagacc ccttcctaaa tttgatcatt tcatctaagt atagtggtgc tctcactgtg 3720
aatgtttgta atcacagata gggtgatcca gagataaaat acaaagttat atttagaaac 3780
agtggaagga taagaagaga cagataattt catttattat atctggctga tgaggtgttt 3840
cattgaaatt tttaattttt taaaaaaatt ctgaactctt gtcatggtag gaattggttc 3900
aattcatgaa gcatttattg agtacaaggc agtgctgtac atagtgctgt aatgtgaaat 3960
gcctaaaaat ataccacaga gtcccaccct acccccacag gtttacattc aggtagtaag 4020
acaggcatgt atggaaattg ttattgtaaa aggcacaaga ttatcaacaa tggggtggtg 4080
gtggtggtgg tgggggtggt ggtggtttag gaatggatct ggtaaggctt tgtgaaggag 4140
gtggaatttg agcagggcat tagaaagtga taatattttc acagacagat gaaaggagca 4200
tatttcacgt ggatgggcac aatataagaa aaggcatgga aatgttctga aagaaggaag 4260
aatctgatac tgttgtaagt ctgtgatgtg ggagagggga tatagtgaga gataaggaat 4320
gtattaaagg taagctaggc ccaggttgaa tgggactttt taagccagtt taagggttca 4380
attttatctc ataaatagga gccactgaag gtttttgagg acatgagtga cattattaga 4440
tttgtgcttt gaaaaataat tgacggtagt gaggaatggg ggagatatgg aaggtgggga 4500
attccagcta ggaggctatt gtgttagtgg aagtagtagt gagatttttg aactagtaca 4560
gaaggggtag aaaagagatg actgatggga gagacctggc agaggtagaa tagttacaat 4620
ttggtaactg attagatgtt ggagagtaaa aagaaataac aaacaattga gattagaagc 4680
gtcctggtag gaattaattc aatgtaacag gtatttaatg catacctgcc aatataagtc 4740
tctgtattag ccaccagact aatataaaaa taaatagatc tttgcagtat agggagatag 4800
aaagctaaaa tgaaatctcc cctttctaat attgtattga ataatagcat tccactctga 4860
aggtttctga tcgtgcttag aatgcttaaa ttctttacta ccaaaggtga atacacttat 4920
tactagctaa aacattttta attcttttta aatgggcctt aggcctgtaa ttcttgtttc 4980
accaatgttc atcttcagta gtcagcagcc gtgagtacag agtcctattc tgaaaataat 5040
gaccagtact tatttctgga atgtggaagg agagcaggga catctgtatt ttttctatgc 5100
tttcaccagg tttatttgga atagacaagc ggggaggagt cagagggcag tggcccctcc 5160
ctgccacagg tgccttctca gttcctccaa gtcaatcttt ctttaagcca gtctgatctg 5220
gaaatattca ggtcagttcc ttcagggcag gtcctctata tttccctacc ttgattagtt 5280
catcaagaga gctaatcatt acctctgtgt tccatgataa ggcctactga tagtgtagtt 5340
gcttttctgg gccatattgt tttagtacct accctctcat agtctctctc atctagaatt 5400
ttgatgagga aatgcaagat ttttttccct tcctgtaatt tctttcttgg tgctatcatt 5460
gtatgtagca gcggttctta agatttgata agcacttgga atcttgaggt ataagccttt 5520
cagtactttg tcagaatttg ctatgtaatg tttattcatt tatgttattt ccaaggatta 5580
tttactgttg ctgccaatat ttttttttgt tgtgtatttt ctttattttt gtttgttttt 5640
aggtaggaaa ggagttagat gggagagaga tttagagagc tgagctaaat ctggcttttg 5700

cagataagag acgttttcta aattgccctg gagacattct gtaactgatt tcactgctag 5760
attttaatct gtatctctac attagaaaca ggtcctgctg tagttttgtg tgcacgtgtg 5820
tgtgtgtgtg tgtgtgtgtg tgtgagagag agagagagag agagagagag acagagaaag 5880
agggaaggtg gagtagagtg catgtgtgtt acttgtgatt tgcttcattt taccaagcat 5940
ttgagatggt atatgggaaa tataatctga taactatttt gatattgagg ataagggaaa 6000
acaaagtagg agatatatga aaagggtgga gggtagggga tgaagggtaa aatagagcac 6060
aaataagcag gctgtaaggg tctatgacat gtttaaaata tagttgaatt ataaatttgg 6120
ccttgagttt tcttagctgg tttttctcaa ctctggctgt gtgttatatt tacctgggag 6180
tgtaaaaaaa ttatccatgc cagtgttcca ctcagagatt cacatttaac taatctagag 6240
ggggcctggg catctattcc ccagatgctt atgatgcaca gccagggtta aaaaccacag 6300
ctctaggcca aagtaaaaaa agaaatagtc acgtagttgt cattattcaa taggaaaaaa 6360
agaatattaa tttgtaagct agcaattagc tctgcagtaa attatctgtg aattgtgata 6420
tttttctgtg tgttttattt tgttgttgtt ttaagatatg actggagcca aattgggttg 6480
tttgaactgc cctgtatgtt cttgctcttt agttttgagg aatttgcttt tccagtagca 6540
ttaaaaacaa aatttagtac cgtagctagt tttgatatag ttcagccata taactaagag 6600
gttaaataac ttgttcaccc attttactag ctgagtaagg agatagtgac tctgttttga 6660
atgacaaata tcccattaca tctctaacaa aaaatttcaa aacccatggg taattattac 6720
taagaaatcc ctctgcctct cctgtaaagt gattgaagac aaactttaag aggaaatata 6780
gcaatttaaa cacccaatac aaatttttta acctgaaaaa acgttggaat ccattccatt 6840
taaaggtatc gaatcttaag ggagaaagag ataatagcgt ttattgagca tgaactatgt 6900
accagctagt actagatgca tcatagatta tctcagtttt attcattctt caaaacatct 6960
tggtaagaca gttatgtctt tttaacagaa tagaaagata catgcaagtt agtaggaaat 7020
acgtctggaa tttgaaccta gattatttta aattcatgct ttttcctcta tacattgcta 7080
tctcccccct aaggacaata ttgaaaataa ggagagaata tagatacgtt gttagcaacc 7140
atattagatc agcagtcaga tgtgctgatt caatagccat gcctgtctat ttaagttaaa 7200
taaatgtcaa caaaatccca gcaagtctgt ttttttgttt tgttttgttt tgtaaacaga 7260
caagcatgtt ctaaaatgta tatgtaaaag tacaagacct agaagctcta aaacaatttt 7320
gaaaaagaaa aataaaataa ggaattactc tattggatat tacgttttac tatgtaacta 7380
taataaagac aatgtggtat tggtggaaga atagacacgt agatcagtag accagaatag 7440
agtacacaga aatagagcca cacaaataca ctcaactgaa ttttgagaaa aaatgcaaaa 7500
aaataaaaaa ataaagaaca agatcagctt ttaaaatctg aggaaatttt agggggaaag 7560
ttgctacctt tttcaaaaag gcagaacttg aaatttgcac tttctttctg aagattctgg 7620
ggaaattatt acataaaaca tttttgctat tttcagttac tacagttgaa attagtcatt 7680
aagacataag tgaaaatgga acatgaatta tcctaaaaat cataagcttg ataagagttg 7740

aggtataggg atttattact ctacttaaag gatcagagtg gacagaactt cagaaaacaa 7800
aggagaaaaa cgtcatatga ggggaaatgt ggactgaatg tcaggtttca aatgactttt 7860
agtttttctt aaaatgagag aggtttggtg tatgtgttaa agaaattgta ctaattgaga 7920
atagtcaaaa ttcaaaatta agggaaatag ataagccatt aggtctgatt tgataaaaat 7980
attttgcatt catgattgtt cacctggagt agatgcagtg atgaatgaat gctgttggaa 8040
acaattacat ttcaaactta atgggcatat gcagaaaacc atatgttgat tatagagaca 8100
tatacaagaa tgatagaaca tatataagat aatttgtgta tgattttgta ctttatttta 8160
cagatggaga agagtaactc tagaccttct ggcacaatca taaaacattt attgtgttta 8220
tcatttgtag agcaattaca ttggaataag tgaatactta tagaatctct taagggaggt 8280
cagtcaatac ggattttcaa aaaagacatg gtcattcagc aacttcatat ctatgaacaa 8340
tattaatttg attttatttg gtctttattc atgatattat gtgtatcagt agttctttct 8400
tttttattgc tgtgcactag tctgttatgt gaatgtacca cagttgattt gttcattaat 8460
aagttgaagg acatttggat ttgggttgtt accagtgttt ggtgattatg aataaagcca 8520
ttatacacat ttgtatgaaa atttcggttt ggacataact tttcacttat attgggtaaa 8580
cacttaggag tgaggttgct gcgtgagaat tccagctgct ctgagtactt gccagcactt 8640
agtcttgtca gattaagtga aaaaaaatag ctattctaat tggagtgtag gggtatctaa 8700
ttgtgatttt aacttgcatt tccctaatga cacatgttgc taagcatatt ttcatatgct 8760
tatttgccat ccgtatatat tctctgatga gttgtctttt caaatttttt gaaattgttc 8820
aagttgggtt gttttcttat tattgaattt tgagagttct ttatattcca aatgcaagtc 8880
ctttattaga taccgatttc caaatatttt tcccacatgg tctgtctttt cattctctta 8940
ataaagagtc gaggtttttc atcttagcga agtccagttt atcagttttt tttttctttt 9000
atgaattgtg tttttggtgt tgtatctaag aaatcctagc ctaatccaaa gttacagaat 9060
ttttccctta agttttcttc tagaagtttt ttagttatat ttcttaattt aggtctttga 9120
tccatttcta gttaattttt gaatatggtg ggtagtgtga tagaagtcta cttttttaca 9180
caggtatgtc cagttgctct gccaccattt gttgaaaaga ctacctgttc tctattgaat 9240
tgcctttgta ctatatcaaa aatcattttg tcacatatgt gtagttttca taataatttt 9300
aatgaattag tcctgtggaa cttcagaatt gcacttactg agcatagata tatggtgtta 9360
ataaaattaa ttaatacaat taataaaatt cttgaggttg tttctgtatt gggtttagtg 9420
aaatattctt tgttaaaaaa caaaataatt tctgtgatat attttctttg cagtcattaa 9480
ttttcaaaac attaagtgac tgttcttaga cccttttata gatgaggaaa cctaatggtg 9540
ctagaagttt tatgtgaact ttctcattct gcagtgatac ttctctgctt atctcctccc 9600
ccatatttgt acattacccc taacattttt tatatagcag tttgtgacct gttagttgct 9660
tagtattaga catgaaagct acaagactga ataagcttca gttcatattc tagaaactat 9720
ctctttgcca agaaaatggg agcctggatt gttggagctt tggtaaccat cattaacata 9780

gatttaattt tgctcttttt aaataaaatt attttggctt ggcacggtgg cttatgcctg 9840
taatcccagc actttgggag gcagcgtgcg aggaatactt gagcccagga gtttgagacc 9900
agcctgggca acatagtgag accctgtctc taaaaagaat aaaaaattag ctgtgtgtgg 9960
tggtgcatgc ctgtggtccc agctacttgt ggagctgagg tgggaggatc acttgagcct 10020
gggaagtcaa ggctgctgtg agccatgatt gtgccactgc actccagcct gggtgacaga 10080
gcgagacctt gtctcaaaaa atttatattt tagtttttaa aattgacaaa taattgttgt 10140
atattccttt tggttttaag aagagaatga tattcgttct ttgagaaaca ttttaaaact 10200
accttcttta accttggtta atgatactca gtgcctcatg tcttcctatg tgaaagagga 10260
aatatgagaa taatgtaggt tgggtttgta ttgagaatat tgtgctaatt aagttgattt 10320
taaacttaag gaaatataaa cagttgggtt agccatctca gattctttac aaattcagaa 10380
taccatagag gataatagct ggttccctag agctagaatg cccgagttta aattctggct 10440
ctttatttat ttactgtgtg accttggaca agttacttct ctgtagttta cttggtcgct 10500
ctatctgtaa aatggggata acagtagcat ataatatatg taaagcactt agaagagtgt 10560
cttttccatt gtaagcactc agtaaatgtt attattttta aatatatcac atctgcaatc 10620
tgagttattg aagattaaca ggcttgcaag ttaaaaaaat caactattgt ataatgatat 10680
ttaagtatca tatataatta tgcagttatt agtatttctt ctttaaaggt atttatatac 10740
aaatacacat ttctcctttt tgaccatctt cttctacctg tgacttgaat tttgaaacat 10800
tttattcttc cttgattagc tgtagtaagg ctgtcttttt ttcgtattca tttctcaaga 10860
ggatggtact gcttttgctg aacttatatt ttctttattc actgagcttc tgttttctgt 10920
agtctattga cttgtgattt tctctgagta gttttttgac agcaaacatt tattaatgga 10980
aaaaaaccca cacatatcag gtagaatttg acctcaagct aaggttgtgt aatagctgtc 11040
tatttttgac ttttttccac atataaaccc attttagttt ttaaattttt tcctttattc 11100
tccattgttt atgaagtgat gaaagaatag agtaatatgg tgtaggacat ttaggaactg 11160
tgactcactg ggataaagtt gttttatcct gtcactagct tgctgtgtgt ttttaggtat 11220
gtgttattta attaatcagt ttcttcacat ataaaatggg aattaacatg tttatgatta 11280
ttttgagaag taacacaatt gtactttgta tataatgttt gtcaagaata ataaagttct 11340
ttttcctttc aaaaaatgac taaagattta ggtgttctag acaaagtact tctttaaccc 11400
tgatatataa ctcagtggac aatttaaact taattcatac cccaaaggta gtattttaat 11460
gtgattatat agacttcata tcttgaagag gttacaacag tgagaattac tacctcagaa 11520
tgtcatgtgt gtattcaggt tgcatatgta catgacttca tttataccac atgaatactt 11580
atatttttgg aaagtaaact tcccaaatgc atgtgttgta gcccatatga gactggccag 11640
acagccagtg cctttgcatt gggaacactt tggagctatt caaaaccaat aaagtcatta 11700
gctctgattt cagctaagca gagtaaacaa attcctgcct gaatagaaac caggaagctg 11760
catttctgtc attgaaacaa ggttgtaaaa actcaaatgt tctggaaata ccttctaatg 11820

tttatgcaat attgacatac ttagtttaac tcagtagtaa cagtagttac tcagtgactc 11880
agtagtaact cagtagtaat agtagtacaa ctactagtcc tttttaaaag catgatgata 11940
attggatttt ttttaattgg ttctaaatat tattttgcat gcactcacca aattatgatt 12000
aaatatgtat agtgttgtgt tactagatag ggttcatttg gttattgctc tcaatttctg 12060
tcttgataac tgaaaactaa ttaactgtgt atttacgcat ttttccataa tgtacaaaga 12120
aatatctgaa ctgaaggaca atatattatg tcacaaattc caacaaattt agccttatag 12180
aaagattttt aaaaatattt tggttgttaa tgttttaatt ccatactcct gtggcattat 12240
tcacaggttc actaatagat ctttcccacc cttttgcttt tctaatatat taatacccga 12300
tttcctccta ctcaaataag ccatcaagaa agtagagcct cggtatgtat ggcagttcag 12360
tttgtgccgt attcttgctt tgcatagctc cccttactct tggctatgta atacctagca 12420
tataaatgct atgtaaatag ctgttaatag agataacatt attgctatgt aatacctagc 12480
agtataaatg ccatgtaaat agttgttagt ggagataatt taaaatatag aggaagagat 12540
ttaacagata aataatagag atcatttaaa gtacagagga ggatgtgcat agttatatgc 12600
aaatgccaca tcattttata tacaaaactt gagcatctgt ggattttggc atccacgaga 12660
ggtcctggaa ccaatctccc acgtatactg aggttcataa tgtgatttcc atgcaagata 12720
tgaaatacaa agaatttcta acattttttt tcttgaaaac aattttatga ctaacccaaa 12780
taatatattt ttaatgtgat tttgaaaaag ctgaattcct ctttgctgtt agcatttaag 12840
agtcagaaat gggaagtaaa atgacttact gtaacttcgt catgaaatta tgtttgaaag 12900
ttggttttaa acatttttag taagaaaact aataaaaatc gaaggaaaaa taatggccat 12960
tgcttattat tgaagttatt ttataaattt tattcattta gatcccagat tttagtcatt 13020
ccattttgta aatatttttc cagtgtctat tatgttccag atattatgct aggtactggg 13080
gtaaccaaga gtaatatgat gagatccctt tcctccagga gattacagta tgatagagag 13140
atggaagaga cacttaacaa gtaacacagt gtttactgat agaagtcttc atagggtaag 13200
gtggaaggaa aaagggaaag tgttaacgtt gagtcttgat aaagatgagt tgacattcaa 13260
aagcacaggt aattcaaaat ggcaaatact ccaaagatca ttcatattta actttggact 13320
tggttttatg ccttaatttg aacatggata tgttttacca gctaactgct tttacaacag 13380
agcctcatga ttgcatttaa atacctgact aaagtaatcg cttggaggct gtatactctt 13440
gtcccattct gagactcttt taaaaaaaag acatctcttt taaattgatt tgaaacataa 13500
ttgccctttc ttaagtcctc cctgaagcag tgacaataat taggtgggtg tatttcaaac 13560
ttcttgaggt acaaactaca agaatattgt ataattagaa agccagaaag gatcaaagta 13620
aatcatttaa aatgagtatt gtagttgtta tcagatttct cataagcagt atgttaacta 13680
tataatttaa tctaaaagtt caaaaagatt aaagtgtttt ggggaaatga ccatagatat 13740
taataaacct ttgaaaatat ttggtagtat aaaagacaca gtacaaatta aaacttgcct 13800
ggtgaatcca aaaaaacaaa ccaactaaat cttatttttg gaaatgtctt gcttaaaaga 13860

ctgttagata aattgataaa gtgtgttata gtaaaaatat acaaagatac ttttttattt 13920
ctctccaatt tgtgtttctt aaagcaaaga gtatgttcat gagtatgtat atacctttat 13980
gggttggttt gggagattca tttcacgttg accaaaagtg atcctatcat aattttaaaa 14040
gtgatctttc cacaacagta tgacatatca aaactacact agggctgggc actgtggctc 14100
atgactgtaa tccctgtgct ttgggaagct ggggcaggag gattatttga gcctaggagt 14160
ttgaggttac aatgagctgt gatcgcactg ccactgcact gcagcctggg gcgacacagc 14220
gagatcctat ctcaaaaaat aaataaatga tacaaaaaaa ctaaattaag actattgagt 14280
caatactatt atgatagtga tatggcagta agttttgatt cacatatttt ttattctcta 14340
tacaggcagc attattcacc tctgatgaat tattagctaa aatgttttta tttttttctg 14400
tattgttttt atcagaaaaa cattcacaca tcatgcattt gttgagcaca tgtcagacac 14460
tgtgccagct gctttaggaa cattgtatga atcctgacaa tcctaggttg ctcattttac 14520
agatgaagaa attgcaacta agaaagctta ataaactttt caaaggcagt aaaagtagag 14580
ctttagagtt tgaatcttgg tctgcttgac actgaagaat tgctgttttc atgacatact 14640
atgcctgcct aatgaatgaa acacagcatc attcctcaag gagctttaag tctagatgca 14700
catgacaaga aatttacctg atgtgatgta ttactaatct tatttttagc attaaaattt 14760
taaattgtct tatatatata aatatatata tgaaacataa aatttgccat cttaaccatt 14820
ttaaagaata cagttcaaaa gtgttgagta cattcacatt attgtgcagc caatctacag 14880
gactgttttt accttgcaaa actagaactc cttacacgtt acacaaaagc tttccattct 14940
cccctttttg ccagccccag caaccacctt tctactttcc atctctatga atttgactat 15000
tctagatacc ttatataaat ggaaccatat agtatttgtc attttatgac tggcttattt 15060
caattcatat aatatcatca tggttcatgt tgcagcgtgt gtcagaattt cctttctttt 15120
taaggttgaa tgatattcca ttgtatgtat ataccacatt ttgtttaact taccatctgt 15180
cgataaatac ttgggttgct tctacctttt ggctatttgt aaataatgct gctatgaaca 15240
taggtgtaca aatatctttt tgagaccctg ctttcgattt ttttggttct ataccctgaa 15300
gtgggattgc tagatcatat ggtaattcta ttttaaattt ttaaggaatt gctatactgc 15360
tttttatagc agctatacca ttttacgttg tcaccaacag tgcacagagg ttccaatttc 15420
tccacatcct tatgtatggt ggaattattt tgaaaaagta aattccagga tatattctgg 15480
atttactttc catttcaaca aagccatgct gttgataatt catgttgaga gggttagttc 15540
ttaagatact gagatatccc tgattgttaa aggaatatga aattatgttc ttgcggtgat 15600
tcgtcaagga tctaggacca gaaataccat ttgatctagc aatcccatta atgggtatat 15660
acccaaagga ttataaatca ttctaatata aagacacatg cacacgtatg tttattgcag 15720
ccctatttat tgcagcacta tatataaaca ctatatataa acacgcatgt ttattgcact 15780
actatttatt gcagcaaaca cttggaacta acctaaatgc ccatcagtga tagactggat 15840
aaagaaaatg tggtacatac acatcatgga atactatgca gccataaaaa gaatgagttc 15900

atgtcctttg cagggacatg gatgaaacta gaaaccatca tccttagcaa actaacacag 15960
ggacagaaaa ccgaacacca catgttctca ctcataagtg ggatttgaac aatgagaaca 16020
catggacaca gggaggggaa tatcacacac tggggtatgt cgggggttgg cgggaaaagg 16080
gcggggagag tattcggaca aatacctaat gcatgtggag cttaaaacct agatggcgag 16140
ttgataggtg gagcaagcca ccatggcaca tgtatacctg tgtaacaaac ctgcacgttc 16200
atcacatgta tcccagaact taaaataaaa ttttaaaaaa aggaattacg ttctctatac 16260
tgtgcttaag ttagtgattg atttttcgga aagcaggatt aaaggacaaa aatagaactg 16320
ctcaatttta agtttattga aactaaaatt aaaaatttgg ccccacttaa acatttgtcc 16380
tgggtcactt actagtatat agtttacagt tttatttcta ccaaatgaga acaaatatgt 16440
gagtttaata agtaaagatc tattttaaaa tgattaaact gtaaaatttg tctctggaaa 16500
gtctgtttca ggggagaatt tttgatcata gacacttttg attgtattac ctagtgctgc 16560
aaaaaattac cataaattta gcagctttaa ccaagacaca tttattatct cacagtttct 16620
atggatcagg aatctgggct tggctcaact agatcctctg cttagggccc taccaagctg 16680
taatcaacac atgggtcagc ttggtcctct gcttcgattc ccacaaaaat gtaatcaagg 16740
tttcagctgt tctgcattgt tatatagagg cttgactggg tgaataatct acttttacac 16800
tccctcagat tcttggtaga gttcctttcc ttgtggttgc aggactgagg gccctggctt 16860
cttgctggat gttgagtgga ggctgctttc aactcctaga ggctgcctgt agttcctaga 16920
ggatggccac agtccctcgt tatggggact ttctcaacat ggtcacttaa ttacttcaat 16980
caagtcagca aggaaagcct ctagagtctt atatgacaca gtgtaatcat atatgtaaaa 17040
tctcatcgcc tttgccatat tctattggtt ggaattcagt gataggccat gttcacattc 17100
aaggggatgg ggattataga agggcataaa cagcaagagg tgaggatcat gggggcctat 17160
cttagagtgt ccaacacatt gcttaattgg taggtttctt tctctgtttt attttgtcct 17220
gggcttaata tataggcata gatttttttt gttttgtttt ttgttttttt ttgcctttga 17280
gccagagtct cactctgtcg cccaggctgg aatgcagtgg cgtgatcttg gctcactgca 17340
acctccacct cctgggttca agtagttctc ctgcctcagc ctccccagta gctgggatta 17400
caagtgcatg ctgccacacc cagctaattt ttgtattttt agtagagatg gggtttcact 17460
atgttggcca ggctggtgtc gaactcctga cctcaggtga tccacccgcc tcagcctccc 17520
aaagtgttgg gattacagac gtgagccact atgcctggcc ggatctttat tttaggtaga 17580
tgtggaccca cctttatctt tagctctaat ttacttttgt tatttaaata aagtgaaaaa 17640
attgaataga agaagtcttc tttacaaaag ctatcagtga ctaatatgta aatgtaaagc 17700
ttatttgaga gacatatttt cttttttttt tttttttttt ttagaaggag tcttactctg 17760
ccatccaagc tggagtgttg tggcataatc tcggctcact gaaacctcca cctcctgggt 17820
tcaagcagtt gtgcctcagc ctcccatgta gctgggatta caggcgtgtg tcactatgta 17880
cagctaattt ttatattttt agtagagacg ggtttcacca tgttggccag actgatctcg 17940

agctcctgat ctcaggtgat gcacctgcct tagtttccca aagtgctggg attacaggca 18000
tgagccacag cacctggcca atattttttt ttactaggtt gattttgggg atcctggaga 18060
atacttgaaa atatataata ttgtcaaatt tattgtggaa actgctgtcc tgtgtatgtt 18120
ttgttaatca tttgcatcac ccatatttat tctcactttt tatctagttc ttatttgtgt 18180
atctctctac tgtcttcttc ggaaatgtgt atctctctac tttcttcttc agaggtgcac 18240
acaaataagg aaaaagtaga ttttattcta gttagatgtg ggcctacttc taagtttcaa 18300
tatatctttc agcaccaagc tacaagctct gttaacttcc tccttttgtg taacactaag 18360
gaacaggttt atgtatcata gataacagta aactctttta ctaaactatt tgtattcata 18420
tgttcactat cccataccac ctccaattcc aaggatctga aagggatttt ttttttttta 18480
atttctcagt tcagttttcc ttgatgagtt gctttggtca ataatagtaa gaaagcgata 18540
aatttgaagt gacctatgtg attttctggg atcagatgag tacagcaact cagtttagga 18600
tttctgtttg tcccgcatat ttgtaagtgc tttaagtctt ttctgataaa atgtgaaaag 18660
aatttttaaa gtagtcttct ctaagccctg gtacgagagt ctgttcactt ggattttgaa 18720
atatctgagt aaattattac tacctttatt gttgatgata aacattccag aatcatatgt 18780
agaatttttt tcctctcatt tggcacttaa ttggggaaag cagcatgtct gcttatcttt 18840
tcaaattaca aattttattt ttgaaataaa gatttattat cccagagaat tcagaaatta 18900
tttttgtttt tatgtggatg aaagtgattt aaattatagt aagtatcaat gagtatacac 18960
tgtggttctc cgtaggatga atattcttat attgaaaacc tgataccttg agggtagatg 19020
agatgggagc aatggagtgt tctgtttcta aaggccattt ctgactgaga taatttccag 19080
gatagagtga tcttcttgac tcattcacat tcattttatt tctagttcct tcagacccac 19140
agcttcatga taaattagtc ataagatgat atgaatagga agtaaccagt aactaaggta 19200
acagaggaaa gcaatatttt tcttttttta tggataatcc tcttcctatg cataatcctt 19260
ttttctatgg ataattctga gagtcatttt atggcaaaaa tttaattatc acgattctgt 19320
cttaatattg atttctggac tgttttccga atgctcttct ttctatatgc ttacaatgaa 19380
tctgtcacag ttcttttttt aatcccacat agtctgtttt gttgagctaa ggtggtagat 19440
ggtgttatca tttaatgaaa tgggaaaccc tgaagaaaga gcagatatga agcaggggtc 19500
aagaattcct ttttgtattg gttatttttg agagacatct gagataaaca tgtggaaatg 19560
tcaagtatgc agttggatat atggatcagg aactcagagt tagtcactga gtaaacgtga 19620
atcatgttag taaattagct gcgtggggaa agaatgtgga gtgggatgag aaggtgatcc 19680
tagtccattt cctgaggaat tacagtgttt caagggtatt gaaaaaaagc agcaaaggaa 19740
attggagtaa aataaaagga gtgtatggta ttacagaagc taaagactca aataggagag 19800
tggtcacctg tgacatgctg cagagagatc aaataagata atgactgatg gatccgttag 19860
caaagtagac atcattactg actttattga ccattgctgg ttttcaatgg gatttttttt 19920
tagaatagag atgctagttg agtaaataat gctaatttac tttttaataa attggtttcc 19980

aatttttcat atagtaaaat tcactctttt tcatgtaggg ttctgagatt aaaaaatgta 20040
tagagttgtg taaccatcat cacaatccag ataaagagtt gttcgatgac ccaccctccc 20100
cagcacacac acacacatac acacactctt cccatgtgat gcctctttgt agtcagcctc 20160
ttccctatcc ctaactcctt gcaaccacta atttcttctc cattcctttt agttttacct 20220
tttacagaat aacatataaa tggaatccta cagaatgtag ccttttgaat cttgcacctt 20280
ttatttacaa tgtgtttggg attcatccat gttgttgaat gtatcagtat attattcttt 20340
tttattgctg agtgatactc gtcttcctct acccccaacc cgttgaacca attatcttta 20400
ttctttccct acagttttgc tttattcaaa gtatcttata catgaagtca tacagtattt 20460
agccttttgg atttggcttc tttcagttag cagaaacgct tttaagattc atcgatgttg 20520
ttacaagtat caacagttct cttcttttta ttgatgagta aaaagtagta ttctttttat 20580
ccactcatca gttgaagacc atcttgattg tttccaattt atgacaatga taaataaagc 20640
tgctgtaaaa atgtatgtac agatttgtgt cttaacatgt ttccactgat ttcgggttaa 20700
tgttctctgt tttctggtct ccatgatttc ttacgagaaa tctgcagtca ttcagtttga 20760
tgttcctctt tatgtaatgt gtggtttttc tctcactcct ttcaatattt tccccatttt 20820
tcctttggtt taaagtagtt tacttaagat gtgtcttggg gtggatttct ttaattttat 20880
tctgtttggg atttactgaa cttcttgaaa ctgtaatttt ttttttcaaa tttgggaaat 20940
gttcagccat tatttatctt gaaaatacag ctttagtgag atagaattca cataacatat 21000
aattcaccca tttaaaatat acaattcaat actttttatt atattcacaa atatgtacaa 21060
ctatcacaac agtaaatttt aaaagttctt catcacatca aaaaggaaca ttgtaccctt 21120
tagttatgac ctccatctcc acatacctca ccccaaccaa ccattagtct gctttctgtc 21180
tctatacatt tccctgtttt cattatttca tatgaatgaa attgccttta tttcgttaag 21240
tgattttttc agtattactc tgttatttta tgggatgtta atgacatgga ttttagaatg 21300
tctcatagtg tttcatatgt ctctgaggct ctgtttattt ttcatcattt tcactctttt 21360
cttcatattg gatgacatct attgatctgt cttaaagttt atttcctcag ccacctgcat 21420
tcttttattg agtctatcca gtatttttaa aatttttcat atgattttta gttctagaat 21480
tacttacatt tgtttttttt catagtttct atttctctgc tgagaacctt ttatcatttc 21540
attcaggtgt gtttattttt atccagaaaa gcatagttat agtagctgct ttaagattct 21600
tgtcagatag ttcaactttg gagccatctt ggagttgata cttgttgata atttgtttgt 21660
ttcagcaggc aatctaccta gttaggttga ctccaaagtt atgtcttgtc ctctctggat 21720
aatggttcca atgttaattc agttttcaga gagtttgggt cagcactctg catgtgccac 21780
tcattggatg tgctaattca gtggttagtc tgagacgagc aattattggt attgtagttc 21840
agtactcaaa gactttgtta tgtttcttta gtcctttctg cacacatgca gttttttgtt 21900
gtgaccagga cttgtgttgg ttcatacaca gaattagggg atctgctctc ttcattctat 21960
cctctctgat ttcttccaca ttcttcaatt cccatgggcc ctttattctg ttctgctggc 22020

cacaaatatg agtttctctc agagtcttag ctttttatgc tggaatataa ctttacacta 22080
ttggagctgc cttttggatg aagtaaagaa gagagtgcaa aaaaaataac agggattacc 22140
ttcacatttt tcttataaaa gggacctctt ttcctgcttc ctctggccag agagagagat 22200
tttctcttga tcagggcttg ccttggagtt ggactcagca tcaaaaaaga gaaagtgtgt 22260
acatggggaa tcttggattc tccttatgtt tacaggctca aaagtgcgtc ttttcctggt 22320
cctttgtcta gaaaatggga tttttcttgg aatttttgtt gatgtctact gtgtaatttt 22380
gtgactcatt ctctcctctg ctcaaattca ggagataaag gtagaaaata aacctaggga 22440
attcatcaga gttaattttc aagttttgat cttccctaat taatttgttt ttgtttcatt 22500
ttcagagttc ttatgtagtt tctttttgta ttctacataa ggattttagt ttataatagg 22560
gggagatagg cttatgagct tatttcattt tgactgtcac aagaaactct aatttacttt 22620
ttaatttttt tttctgttga cttacaaatg ggcagtggta aaaatccttt ttatctagtt 22680
cgtcttcctg ttgcctaatt ttcaaacatc ttagttcagt gtttggcaga ttgtaagggt 22740
gcagatagga aatattttaa gctttgcaga catagggtct ctgtcacaac tggtcaactc 22800
tgctgttgta gtgcaaaagc agccatagat aagatggaaa caaatgggtc tggctgtgtt 22860
ctagtaaaat ttttacagaa acggacaaga ggtcagattt agctcatggg ttatagtttg 22920
tagaaccttg atctaagtct tctactttac tgtttcttga attagttgtt aaagaatgag 22980
tagtattacc tactgagctc tgcatgttga cttgttttgt ttccatgatt acttacatct 23040
ttctcctata gtttattttt tcttctgtat agtatgtcat gatgtttaat taactgaatg 23100
aaagaggaat tcacaacagt gcaattatat aaaactgttc ttattataat ttgctttgaa 23160
tataccccca tggtagattt ctgtggcatt ttttttcctg tcataaatac ttggttgggg 23220
acaatttaat gaaagtacat aggttatata cttagtgtat agttatctgt tattgcaaaa 23280
ctctaaacaa tatgaaatta agcataaagc tctgaaaatc aaagtttctc aaggataatc 23340
tgggaaccat gttaatagtg ttccttttct aagtacttct atgtgtcctt tgtccttttg 23400
tttttcagta agtgtggatc ttgttagatt gtggaaggaa atgcctaggg ttatattttt 23460
cttagttatc actcttgttt taatactttt gaacctcaat ttttgaacaa tgtttttcag 23520
gtaaatggtc ttaaaatggt tgatgagcca atggaagagg gagaagcaga ttcttgtcat 23580
gtaagtattt atgttaattt attaagccac atttttagca tataaggaaa aagaatacag 23640
ggaaaggaga aaagctccta tcaaacaaat atattcaggt aagttggtaa taagcaaatg 23700
gaattattaa taactcaact gcagtttaaa atataggaca ggttttcctg acagaaatac 23760
ttatgcatgc atttttaaaa ataaacagct ttattgagac ataatttaca tgctattgaa 23820
ttcacccatt ttaagtgtaa tggataaaat tttcagttgc cagtgtatga gtaactgaat 23880
tattatactt aaatatagaa tcctttgaga agattatctt caacctcagg gtagaaagga 23940
tatctgactt ggaaatattg aactgtataa gcataacttg aaatcattta aaatcaattt 24000
cctggctttt agaaaggatt atgtgtaaat tatttcagaa gtttctaact agttttaaag 24060

actgcttagc cattagacca ttctatgtaa tgcctagata ctagagtctt attttacaag 24120
atctagtttt acagagagca ttcttaagat ttgtagggag aatctgacta aatcagcatc 24180
tacatttatg ttgttatttt atggttgtta tttctataga tattttcata gagttataga 24240
tttttttttt tttttttttt ttgagacgga gtctcgctct gtcgcccagg ccggactgcg 24300
gactgcagtg gcgcaatctc ggctcactgc aagctccgcc tcccgggttc acgccattct 24360
cctgcctcag cctcccgagt agctgggact acaggcgccc gccaccgcgc ccggctaatt 24420
ttttgtattt ttagtagaga cggggtttca ccttgttagc caggatggtc tcgatctcct 24480
gacctcatga tccacccgcc tcggcctccc aaagtgctgg gattacaggc gtgagccacc 24540
gcgcccggcc gagttataga ttttttaagc tggaaagaac ctcagagata ttatgtaata 24600
cttcattgta tggattagtc tactgaaact tggagagtct gtgatttata ctaggtgata 24660
aaagtagcta gtagcagaat caagacttag ggcagtatgc ttccttttag gtcagatata 24720
ttatgtagac ctgaagaagt agaagatgag gagaacatta tccttttcct atttggttgt 24780
ggatgaaatc aactttgatg acccctccac caaaacttgt tcataattgg aattagctca 24840
gatagaaacc agcagaggga tttaaattgg gtttgtccaa aactctcctt tgaaatgttc 24900
tatgtaacta tttctgcagt tagtaaccat gtaacaacac ttttaatttt gattgacaat 24960
atgagagata caatgttgtc tttgtagctt ttgcagttta ctgaatatta ataatatatt 25020
gtaattttga gtataattaa atgtaaacta tcaaaaccat tatctagaga tttgtctaca 25080
gttcatatgg actaataggc atagtaatat taatgttgcc agtttataaa agataaagga 25140
gtataaggaa ctattaaagg cagtggagaa ggagaaggct gcttattata ttttagcatt 25200
aataaattca caaggatatc atggcttaca ccacagggga acagtgtgtg tggtgcataa 25260
actcaaggac acatacacag ggtgattggc aaatatttac ctttataatt ttagtagaag 25320
ataagtcagg aagttcagtt tttagtggaa aggcagtaaa aatatacagt gctagttact 25380
tacatgatct accttatgca atcaacttca gtggctaaaa ggtagagtgt gaaattttgg 25440
agagtaaagg acctagaatc acttccaagg atttgattta gtttgtggcc tgagttatct 25500
ggacttgaaa ccatgtactt tgggtgtgtg atggttaaag tactcaattt gtgtcaataa 25560
aactacatga gtgattaaaa acaccatatg tatttatccc ttaaattttt taaatataca 25620
gctacgattt ttttctttgt gattgtcatg tgtttttttg tggttggctt ttttttttac 25680
tgttttaaaa tagtgtagga ttgtagagac tgctctgtga tggtaagtat taggtaacag 25740
gttgctgata aactatatct aatatcacat ttcaccacct agtatcctac ttctttcagc 25800
aatgtcaagg ttagaaaaag tttgttatga tttgtctcct ttagtagttg gctgacatga 25860
gttaactttt tatttgttgt caaaataagc ataactgcct tatatttggt agtttatgat 25920
ccattggtat ttatttttaa aactctaccc agtatctgtt ttttctgaaa taaaatacat 25980
atttttaatg ctatcaatat cttaaaaatt tcaaagaata ttcagttagc aattagattc 26040
tttgttgatt acattagtgc aaggtcagca acatgtttag tctctaaagt gatttatatt 26100

tttattatgc agagatgtaa atgtcatagt gaactgaaaa tgtatatttt gaaaaacaat 26160
gctggatatc ctgatttttt taagccctga atttcttatt ttaaaaacca ttcttgacat 26220
gtgtctgtta agaattcttt caaaaatact tttttacaag tgtgttttac ttagactcag 26280
tggaaccaca ggtaggagct atttcaagct gagaatgcct gctttctgta tggtggattt 26340
ttttttcctc tatttgtctt tctattctga tgcacattta gccttagtat gcttcagtat 26400
tttttctctt gcaatgctga tgtttacttc taatttctat tcagttactt aaaaaactgg 26460
aaaccttgtc ctacagatat gcaaaactct cagatgaata attattgttc aactaaaaaa 26520
tgttggtcag ccactataaa gtcgagttat gctgcaccat caggtatgca aagataagta 26580
ctacagagag tatctgactc aggatggttc aacttatgag tttcaacttt atagtggtgc 26640
aaaagtgata ttcagtatgt tcctcaactt acaataggat taagtttgga taaactcact 26700
gtaagttgaa agtgaatttt caacttacaa taggtttatg gggacaacac tgtcataagt 26760
ccaggaacat ctgtacaaat aagttcagta acttcaattt ttatggatgt tttgtggttt 26820
tcaaagttct cagttgatcc ttataactga gatctgaaat agagtaagta ttactgccaa 26880
tttgtagatg agggaactaa ggagctgacc ctcgtaagta gaataaacca aaccaagtgg 26940
gaactagttt gccggcattc tttggttttc agtcataatg cagactttta caaaactaag 27000
tgttctgcag ataccttcca ttgtgtacat aattttgatg tgcttagttc accctcatgg 27060
cattttggca tttatattgg tattacgctt agctttcttt caaagtacac taatatttga 27120
catggttttc aatgacccca tattattcac tgaatgagaa gggtatatcg agctgctctt 27180
ctgggtcttt gcatatatgt ttcagcaaat ccacaactaa atgattcttg gatttttagg 27240
gtgatctttt caccaagctg gcctgtgtac ctacaaataa agctgatgtt tgaatagatg 27300
aatgttttat tattaaataa aaatgaacaa gttcaggctt taaaaatact tcttttgttt 27360
tgatttgttt tctggaggtt agttccacag tcatatgaaa actgctgtaa ttgagttata 27420
atagtatatt gctattaggt tgtcatcagc tttttctctt ttcaattaac aggtgattcc 27480
atcttcctaa acaaatcagt tatcaaatga atggagcaat agtagatttg acagttcatt 27540
ttgtacttta acagtgtgtt ttcccagtgt tacagcactg ttggccctct ggattaaaaa 27600
gatgatcaca gattatagag gtcagaacat actattttgg ttgtggttga gccttcatac 27660
tttttctgta tatcactgat tactttatgg tcgcaaaaaa ataataaaac ctaattaata 27720
gtcatcctgg cctcagattc agatttttta tttgatgtag aaagactagg ggtccttatt 27780
tagaggttat taaatcttca tttcacagta gcaacattat tttcaaagat cagctaagat 27840
tatgtaccat gtgtagtttg ctagggctac cctaaccaaa taaccacaga tgggtggctt 27900
aaacagcaga aatttatttt cccaagatgc tgcattctat aagattgaga tcaaggtgtt 27960
ggcagggttg gtttttctga atcctctttc cttagcttat agatgagtct tctccttgtg 28020
cctttacgtg gtcattcctc tgtaggtgta ggtattctaa tttcctcctc aaataaggat 28080
gtcagtcata ttgaattaga acccacccat atggcctcat tttaccttaa ttacttcttt 28140

aagggcccta tccctaaata cagtcatgtt ctgaggtact gggagttagg actttaacat 28200
attaactgtt aggggacata atttagccca tagcatactt gtagcttttt gtttctttga 28260
cggatgtgat gtttctgaca aagttctcta tcctcaaatt cacagtttat ttctgtatcc 28320
ccagtgtgca gcacagtgtc tgataatagg atatgtgctt aatgaatgtt gatttgctgg 28380
gtgaataata agagtgcata tatgggaaga taaggtgata tagcacaact ttatcatacc 28440
aggataggga atttttttaa ggatgattta ctctcaggaa aaaagaaata tatcacttat 28500
aagtacatta ccacctattt caggctagat tgctgctact atcaagcttt tgaaatggtg 28560
cagaacctga agacagggag gtggagggaa gaaagagggt ttcagttgac aaaaagggac 28620
attataatag taaaaatcaa taaacatagc cttgggatgg ctgagaaatt ttaggcaata 28680
gacaagaatg gggcagggag cagatagaat aagacagaat aagtattgga aatggtgaca 28740
caggagggta tcacatactt tgagagtaaa aaagagctag tggcgtagaa atgataaaag 28800
tgttaagtag ctatatataa acacatacac acatgtgtat atgtgtgtgc acgcatgtat 28860
gcacatatgt gtatgtattt gtgtgtatat ctattatgta tctgtataac tataaatctt 28920
agaagcaccc aaatattgtt tttcaatcct ggccatgtgt ccgaatcaac tggggaggct 28980
taaaaaatac acatgcttac gttttcctgt agttctactg aaccacaatc tctgagggtg 29040
ggacccaaac aagtgcagta aataaatatt tcttctacag gtgattctta tgggcaacca 29100
gggcattaca aattgtctgt gtaaaggcac agtagtcata atttggtgat ataatatgtg 29160
gtagttgttg gcctaggaac atatgtcttt atttaaaaag gcaaatggct aagcattagg 29220
ttaatattca ggggttattg tgtcctttat ttgtgttgag tacaacacat actcagcaca 29280
gtgtaataaa aatactgaca atggataata tggaagaagt ttatattctt cacagtgtat 29340
gggaagatat gcttatttta tttagaaata ttgttatcac aaagttggtt atattcaaag 29400
ctgacacaat ttcattagcc agaatgaatc tcctgtttcc ttatttcctt tatatattta 29460
aaaaaagaaa tcgtgggtat atatattttt tgttttgtag actatattta agaataattt 29520
tgagatttct tattgtttaa ttttaccttt aaaatcttta ttttctattg tatatattta 29580
aggtgtacaa tgggatgttt tcatatacat tgtaaaatta ttatgattgt taagcaaatt 29640
aacatccatc atctcacagt tgccctattt tttggtggta agcactgtct tcattttaaa 29700
aagtatatat tttttcctgt ttcttttata atattgagca gatttctttg tctttctgct 29760
ctcatgagat caaaaaagta ttgtgctaag cattaggaat aaagagatca atgtggtgta 29820
atacctgcat ttaagaagcc cacagtccag tgggaaagaa agacatggta attagttgtt 29880
cggacacagt gtaacatttt acattggctg tctcctggga aaagtgattt ttctcatcta 29940
gttgcatttt catattggga gctttttaga gtactgctgc tgaaaattga aagacattgc 30000
attttagagg atgggcatgt gagcattatt aataaaagtt aaaagggcat tggacaaaca 30060
ttttcatttg gaaaacctgc ttaggtttgg tgaaactaac tatagtacaa tttgtttcct 30120
aactctgaac acttctaaaa aaactgatgc caatacttat agtgataatc taaacataag 30180

cagggacctg aagtaccttc ttgaacataa aaatattata ttgtgttcag tatagaatca 30240
cagacattga ggggacctta gagacccatc taacaaaacc cctttgtcat gtgagaaaac 30300
tgaaggagag agtgatttgt gatttagcca aggtttctta gcttataggt gcatacagat 30360
atattatact ttaatatagt ccccaaatgt cagctacaaa gcatctatat ttgcattaaa 30420
aaaggttgtt tactttttta aaaaaggacc tctttcaaat agggaaattc acttccatag 30480
ccgattgatt ttaatttaat ttttattttc agtaaacatt tataaattac catcactatt 30540
taaaagggtc tggtgaaata ctattttgat gtataagaaa catctgatat actttcaaaa 30600
tcagttattc ttcatttctt gttgatttgc tggtagttcc taacacctac aattaaaatt 30660
tctttgccta ggtacccccc ttgagatagt gaatgcctga ctacctatat tccatggaag 30720
ttattttagc tgctatgaag tctagctcat ttgggaccat actcttgttc actttggctt 30780
caatcaccat tgactaatca gttaattgaa gagtgaatag attgagaaca ttcttggcat 30840
ccaggctaaa attagtatgc tataatggaa tgctaccaag atttatagac attgtgatta 30900
agtagtcatg taaagttaaa ttctgtgggt cttagttttc ttctttatac agtcagagag 30960
ttggataaga tatcccttcc aactctgtta ttttgtgata tacgtctgtc atcctagcta 31020
ttgtgcttct gtagggacta gtattaatta cagctcactt gaatgtagtc atcacagctg 31080
agtggagtcc tgcatcaagg ggaagaacca ctcactgggt ggctttcttt ctgcttcacc 31140
caggtggtac gaaatctggc atataattta tgtataaaat ttcagtaaac gggccatagc 31200
ctggaaacat ttgttggggc tctgcagatt ttgttggtct ttgccagttt ctttttcatt 31260
gatgtggtta gttggccatt taaacattta aaatagtata gtgaaatact atttaaaaaa 31320
gctcttgagg tcacgttttt tggtcatgcc ccacaattta caaggtttta ttttgacttt 31380
ttctgcctct acctcattag tcacagtcca ttgaagggtc ttgagactgt tgttaacaat 31440
tttattatat tgctaccaag ctggaaggga aggctgaagc atagatttct ttttacattt 31500
tatttacatg aaattatgta gtagggaagg tggtggtggt gcatctgtgt gtgctgtatg 31560
tgttagaaaa cagggaaata aggatactgg tagcccataa aactctctgc tacattgagg 31620
gagttgggta tttctattat gacttctgat tttaaatgga actaatcaga agatgttgaa 31680
aatggattca agtattgttt ggatttcttt ttacaggatg aaggagttgt taaagaaatc 31740
cctattactc atcatgttaa ggaaggctat gagaaagcag atcctgcaca gtttgagttg 31800
ctcaaggttc ttggtcaggg gtcatttgga aaggtaatga ataatttttt tccccatttg 31860
ttcctcttct aaacatttag caaaaaaagt gaacacatac gtagaaatct gattaatgag 31920
tcgtttctaa acttctgaac ggatgctcac aaaggtaatt ctttaatttt attcctttca 31980
tttctgttgt acttttatta ccatttcttt gtacttctct gtaacaatca aggttttcca 32040
attccctagg cttactgatt tcttgaggga aaaatatagc atactcttta ttagctgcct 32100
aaatcaaagt agcttgattt ttgtaaaaaa tggtattcct atctccttgt tcccatgctg 32160
gcatgaatac agcttgtttt ctcctggtga tttaatctac atttgttagc tatgacacag 32220

aaatcagaat ttatgtctta attcatgtat gaattcgtct gtcatctgtt aaagtagaac 32280
aagggtgtgt aaagtagaac aagggtagtt actgggtgat atgtggttta tttaagtctt 32340
cagagagctt acatttcaag gcagaaagca aaattgagta agaataacta ttatagtcgt 32400
tactaaagtt gtttattaag cacttaataa catggtttta gaaaagaagt gtataggtct 32460
ggttcctgac ctttatatac ctaacatcta atacatgaaa attattgcat acacatagta 32520
acagccttta gaaaaacagc aatttagaaa aaccgcaatt taaggggaga aagataaaga 32580
gaaactacta atttcaagtg gctgagattt agttttaaag gggaaaaata aggcaattga 32640
gcataggtta tttatagtgc tctggaacat tttaaaataa agacattttt attttattgg 32700
gttttagaaa tggggtcttg ctctgtcacc caggctgtag tgcagtggca tgattgtagt 32760
tcactgtagc cttgatctcc tgggctcaag tgatccttca tccccagctt ctcaaggagc 32820
tgatactaca ggtgtgcacc accacaccca gctaattaaa aaaaaaaatt tctggacaca 32880
aggttttgct gtgttgctca ggctggtctc gaactcctag ccttaagtga tcttcccacg 32940
ttagcctccc aaggaaagac atttattgaa cgctcagtgt gttcacaaca atgtataagc 33000
agtattagtg actataaaat agatgtcatc ttaaagtaac ctgatattta tgcattatag 33060
aatataacgt gaaatataaa gaaacatttt caggacacaa aaaagacaaa ctgagatgaa 33120
ttatggcttg gcagtgaatt ggtacaggag atatataaga cagggagata ggacttgaac 33180
agtgttctga ggagagaaaa actagaagga actgttaata gattggtggt ggtggtggtg 33240
gtggtggtga tgaagttggt ggggaatgtt tatagaactg atctgtagag taggcatgta 33300
tattatatac aactttattt taaagtttct aaaagtgtaa tattctttta gctattttgc 33360
actttatagc tatttttatg gtgttaattt tagaagaaca gagatgaagt tattttatat 33420
tccagatgta taaatgctta aatatttgac ttaaataaaa tctcaccagg caaacatttc 33480
aagctattaa aattacctgc tttgtctttt gatgtatgta tttactcata tatgcttttg 33540
tttatcttca gctttgttcc agaaaggatt taaggctttc atagatgaat attaaataaa 33600
attaaaaata agtagtgaag atgatggaaa cagaaagctc agagcaggaa agattagatg 33660
aagccaggag tacactctgt tcacaaaagc atgacataag ggtctgtaag tccttttcta 33720
gtaggtcaaa aattagactt aagttttcta gcagccatta taaagaagct aatatatttg 33780
agtgtattat aattgacagt attttaaagg tggaaacgga ttaaacagac cagagaaatt 33840
tctctcatgg gttttcttaa agaaaatact gtataacata atgaaatata ttcaataaca 33900
tcatgttaaa tatagcaaca tgatgaaaag ttgtcaatgt agtgcttaaa ttcagggaaa 33960
tcttagcttt gtctgtgctg ggatttagtt ttaagaagaa tatcattgaa accttttaat 34020
tcctaatatt aaagtacaaa atagtgatct ttatgtagtt catcaaccaa agaaatatta 34080
aggaggtggc ttttttttcc ccctaggata tgaacatact tgcttgtcag tgttacatca 34140
tttgcggagg cattgtgtag gtgtgacttg ttatatgctg aacctattca ggaatttggc 34200
caaggctgtg aatcagataa gtgtaggagc aattccaaga gactttgagc tgcagagtct 34260

taagtttttc ttcaaaagtt ccattgttaa aatgcagtaa atgggatttt agcccatata 34320
aagccttata acattaaatg tttttattgt ctatttaaca gtgggggaac cctgctaaag 34380
tcaacttaaa attgtagttt aaggaagcat gcattattat catgaaaggt gcatatacaa 34440
ttctgtccta agcacaccaa gtataatgct attaatgatc attaatgtat gggttgaaag 34500
gtatactaaa caacttacat taattatttt aaaacacaaa cacatgaaaa tacctttgaa 34560
attgtctgat tagtcgtcct tatatatcat gtgccaaata atatttatat aaaacataca 34620
ttttaatatt tgtggattat aaaaaaatac ttttgctttt gtgatagtaa agactttaag 34680
tacaggactt aaaaatgaat ttttgaaaat atcgaatact tgtactctta aataagtgat 34740
caactctcaa gtgatagttt ttaaaaacat atgcttacat cttaaaattt cctgaagttg 34800
gttctttctc ttcaaacagc atttaaatat gtgtgatctt ctagttcact gcagttactg 34860
aatattctta taggaaaata taaacgtcca tgtgattgag agaaaattat attggagtaa 34920
ctcacccatt cttttggaca atttcccatg attggattag ggtgactgaa aagaagcctc 34980
ttatcaatat agatacgttt ttaggtccac tagtttgaag ccatgcaaca acttttttac 35040
tttaattatt tctttaagca ccctatcctc aaatacagtc atgttccaag gtactgggag 35100
ttaggacttt aacatattaa ctttgagggg acataattta gtccatggcg tacttgtatc 35160
tatttcttta aagggcatga tgtttctgac aaatttctct gtcctcaagt tcacagttta 35220
tttctgtatc ccccagtgtc cagcacagtg tctgatacag aatatgtgct taatgactgt 35280
taatttgttg attgaataac taatgagtac atatatggga agataaggtg atatagcaca 35340
actttattat accaggatag ggaaattttt caaggatgat ttaggaaaaa agaaatagat 35400
cacagataag tacattgtca ccttatttca agttaggttg ttactactgt caagcttttg 35460
gaatggtgca gaacctgaag aaagtgaggt ggaaggagga gggagagttt cagatgacaa 35520
agaaggacat tataatagta aaaatcaata aacatagcct tgggaaggtt gagaaatgtt 35580
aggcaataga caagaatggg atggaaaaag aactactttt gtagttgatt ttgggaaggg 35640
ggcagtgaga tacgatattt tctcatgcta ggaaagtcac tcataagacc tgaaaaaaaa 35700
gaaaaaacag atcaggtgtt aaacaggtta cttctctaga ctgcgaatga tggatcctat 35760
taaatgccat attgggatac tactgaatat tagaagaact aggggaaaaa agtggctcct 35820
ttatgcaaca gtgtcttcag aaagcctaga ctctttcttt cttccttgct tgcctcttct 35880
cactcttcat attccgttct ttacaatccc actaagggct atgttttgta agagtggttc 35940
tgaaattgtg gtctccgtac ccctggacaa tcccaagact tttcagggag tcctcaagtc 36000
aaaactactt cacagtcatc ctaagatatt gtttgtacaa ctgctggcat gttggccaat 36060
caaggcagtg acttcaaatg gtaccagtag tcattgtagt cttcatttta ttacatatgc 36120
agtttaagaa tgtccttgat gagacattaa caattattac atttattaaa tctcaatcct 36180
tgaataccct tcttttgaat aatatgcgat gaaataggaa gtacatgtga agccccttag 36240
ccacgtaaca aaatagaatg gttgtctcaa ggcagaggac ttgtgcaaat tttttgagtt 36300

gtgagctgaa ccaaccactg tttttatgga tcaccacttt tccttgaaag gacaataaac 36360
tatggttacc taaatatgct tatttcgtag gtgttttctc aaaaatgaat ggagtgagcc 36420
tgttcataga aaaacaactg atactctttt tgtcaatggt agaatctgag ctttccagca 36480
aataattgaa tttaggcaaa tgtatatttg ccacattgac gtcattttct tgatgcagct 36540
atacttattt ttattatgct tcccttcatt gcacttcaca gatatccttc acttcattgc 36600
gcttcacaga tatgtatgtg tgtatatata tgtatatgtg tgtgtgtata tatatatatc 36660
tatatatatc ctatatatct atatctatat atatcctata tatctatatc tctatatatc 36720
ctatatagct atatatatat ctatatatcc tatatatatc tatatatcct atatatctat 36780
atatatccta tatatatcta tatatatcta tcctctatat atctatcctc tatatatcta 36840
tcctctatat atctatcctc tatatatata tcctctctat atatcctctc tctatatatc 36900
ctctctatat atatcctcta tatatatatc ctatatatat cctctatata tatatcctat 36960
atatatcctc tatatatata tcctatatat atcctctcta tatatcctat atatatcctc 37020
tatatatata tatcctatat atatcctcta tatatatata tcctatatat atcctctcta 37080
tatatatatc ctatatatat cctctctata tattctctat atagtccgtg tctcatattt 37140
tggtaattct tgcagtgttt caaatgtttt ccttactgtt atatctgtta tggttctttg 37200
tggtctgtga tctttgatgt tagtgttgtc attgtttcgg ggtgccatga accacgcaca 37260
cataagacag tgagcttcat tgataactgg tgtgtgtgtt ctggctattt tactgaccag 37320
ctgtttcccc atctctctct gtctccttgg gcccctctat tccctgacct gcaagaatat 37380
tgaaattggt ctaagtaata actctatagt ggccttggag tgttcaaggg aaaggaagtg 37440
ttctgcattt gtcactttaa atcaaaagct acaaattatt aagcttggtg aggaaggcat 37500
gtcaaaatct gaggtaggct aaaagctaag gttcttgtgc caaacagcca agttgtgaat 37560
gcaaaggaat gttcttgaaa gaaattaaaa ttgctactcc agtgaacata taaataagaa 37620
gaaagtgaaa cagccttatt actgatatgg agaaagtttt agtggtctaa atagaagatc 37680
aaagcagcca caacactccc tctagccaaa gcctaatcta gagcaattcc ctagctctct 37740
ttaattctgt gaaggctgag agaggtgagg aagctgcaca agaaaagttt gaagccagca 37800
ggggttggct catgaagaga aaaaggaaag aagccatttt cgtaacataa aagtgtaagg 37860
taatgctaca gatgctgatg taaaagctgt agcaggttag tcagaagatc tagctaaagt 37920
aactcttgaa ggtggttaca ctaagcaaca ggttttaatt gtagaagaaa cagccttctg 37980
ttggaagaag atgccatcta gcctttcata gctagagcga agtcagcgcc tagcttcata 38040
gcttggaagg acaggctgac tctcttgata gaagctagtg atcctggaac ttttaagttg 38100
aagccaatgc tcatttgcca ttccaaaaat tctagggcct ttaaaaatta tgttaaatct 38160
actatgccta tgctctataa atagaacaaa gcctgaatga cagcacatct gtttatagca 38220
tggtgtgcta agtattttaa gcccactgtt gagacctact gttcagggaa aaaaaaagac 38280
atttctcaaa atattactgc tcattggcaa tactcctagt tacccaagag ccctgataga 38340

gatgtacaag gagattaatg ttgttttcat gcttgctatt acagtatcca ttctgcagcc 38400
catgaaacaa agaataattt tgactttcaa gccctattat ttaagaaata cattttgtaa 38460
ggctatagtt gctgtagatg gtgattgctc tgatgaatct gggcaaagca aattgaaaac 38520
ctctggaaag gacttgctat tctagatgtc attaagaaca tttgtgattc atgggaggaa 38580
gtcaaaatat caacattaac aggagtttgg aagaagttga ttccaaccct tatggatgac 38640
ttggaggggc tcaagacatc attggaggaa atcactgaaa atgtggtgga aatagcaaga 38700
caattagaag tggaactaaa gatgtgacgg aagtgctgca gtctcatgat aaaacttgaa 38760
tggatgagga gttgctcctt gtggatgagc aaagaaaata gtttttttga ggtgaaatct 38820
acttctggtg aagatgctat gaacattgtt gaaatgacag taaaggcttt agaatattac 38880
gtagacttct ttaataaagc ggtaggattt gagaggactg agttcacttt tgaaagagat 38940
tctactgtgt ataaaatgct atcaaacacc atcaaattct atagaaaatt atttcatgaa 39000
aggaagtcaa tctgtgcaac aaacttcatc attgtcttat tttaggaaat tgccacagcc 39060
accctaacct tcagcagcca ccaccctgat cactcagcag ccatcaacat tgaggcaaga 39120
ccctccacca gcaataagat tatatctcac tgaagactca gatgatcatt agcatttttt 39180
agcaataaag catatttaat taaggtatat agggtcataa tatttgtttg ctctttatta 39240
aggtaacaaa aagtgggcct gggtcttaaa aggaggtatg tagctcaagt ttgtctttca 39300
ttatgttatt tatctcttac atcacaaaat tctcaaagtt cagtaccagg tcatctattt 39360
taaaaatttt cctttctata gtagtattta tatgcattat gatttattaa atcataacag 39420
tttttttgat taaaaactaa attaaatcta agaaaagtga gaaaataata atgtaaatta 39480
tagaataatt tataggagaa ggtataactt ttttcattta tttttttaaa ttgacaaata 39540
gttgtacata ttcgtggggt acatagtgaa gtttcaatac gtataatgta tggggatcag 39600
atcagggtaa ttttgcatcc ttcaacaaat ctctcactat ctctcccttc cctctatggg 39660
aaggcatatg tttataaaat tagagaaaat gcaaggatat aagtgtatgc tggtgtgttt 39720
gtgtagagca agaatgacta gaaatgtgaa ttttcctttt tttttttgtt caattttcta 39780
ttaggttttt cttgttagaa agaagaccgg tcctgatgct gggcagctct atgcaatgaa 39840
ggtgttaaaa aaagcctctt taaaaggtag aaaattagaa agtttattaa aatagaattt 39900
gacaaagtgt agtttaagaa atatgctgtg tattacttgc ataaggtgaa tagatgtagc 39960
tatttttaac attggtattt gtggcagttt ttctaacctg taggtaatat tactagattt 40020
gatattggca tatgccttaa cttagtgaga gtgcaccccc gtaaaaattt gatgctagaa 40080
aaagtgacat atttcattta atttaaactt attaacttaa ttaggatata ttctcttctt 40140
tcaagattct tggtttttcc ttttctatct gtgtcagtat tacatattga gaaagcacaa 40200
ccatttgcac acactatctt ttagggtgtg ataaaaatac cgcttatgat cagttttaat 40260
cagtaaactc atttgaacaa ttaaaagaga ttaataggcc gggcatggtg gctcacacct 40320
ataatcccag cagtttgaga ggctgaggtg ggctgaaccc ttgagcccag cccaggagtt 40380

caagaccagc ctgagtaacc tggtgaaacc ctgtctctac aaaaattaca aaaatgagcc 40440
aggtgtagtg gcatgcacct atagttccag ctgctcggga agctgagatg ggaggattgt 40500
ttgagcctgg gaggtggagg ttgcagtgag ctgagattgt gccgctgcag tccagcctgg 40560
acaagagagc aataccctgt ctcaacaaca acaacaacaa aagatgaaca cataataaat 40620
gcatattagc tacatacctg tttatgccac cttaacctgt ctctagatgt aatgaatatt 40680
tttgctttct gagaatatgg tcattatcta ggaatatgaa aactgcaaat ttcggcattt 40740
aggaagttac gttttgtatt tttatatttg ttgtttggta aattattgat ggactcttaa 40800
ctgaaatgat gtaagagagc aatatattgt acctttatgt aatttttagt tcgagacaga 40860
gttcggacaa agatggagag ggatatactg gtggaagtaa atcatccatt tattgtcaaa 40920
ttgcactatg gtatgtaatc tttttttatt cttttattta ttttaatcct tatttattaa 40980
tatggaatta gagaattatg atttcctttc acacaatact ataacaggaa ataaatactt 41040
tagacttagt atattcagct acgtaaaatg tatttgtctg aaataaatat cagtatttag 41100
ttattagtta tatgtccttc atttttctaa gttgattaac ttgtttaaag taacaagata 41160
gtgtttttca cttacctgtc tggtagtatt tatgtacttt gctaggctgt aacttcataa 41220
tcttagacta taaactattg tgataattta cgtaaaacat tcagttgctt gttcataatg 41280
accacactta tctgtttcct ggttatattc cagaggacca tgtagttatg ttagtctact 41340
tgagttattt ttttatactt aatgggagag gactaccagt aagaagcagt agatgtaatg 41400
gaaagaacat gagctttgga gttagacatt tctgaatttg aatcatggct ccattgctaa 41460
tttaacatat ggttgagatc aactctatct atttatctat cgtctatcta cctctgatat 41520
agtgtacttt gtcacttcca tttttctata tttaaaaaag tagcagctgt tgattttatt 41580
cttagtgtaa ttttgacaaa taatacatta aaatatatac ccagttatca ctattaaaat 41640
cccataagaa atttccaccc attattcttt tactccttgt tatactaaca gaataaaaaa 41700
agtaacatct aaagaccttc ccgtgtctgt agtaggtcca tcagtaaaca cttattgtgt 41760
gcaaaaatac tgtattaaat tcagtgggct atagaagtga aacacttttt gacctcaaga 41820
aatttataac ctatagagaa cacagagatg taaagaaatt aattgcataa atcaaaatgg 41880
aataagtact attaaggagg tataaatata ataagatctg aaaagtagca gcaagtaatt 41940
tcaataaaga agaggttgag gaagctgttg aaaaatgtga aaagggggaa atcctggaaa 42000
gtttaataga ggaagtggca tttttcttca gctttataga atttgtagat tttcagtagg 42060
tttgataggc taagtttagg caattgcagt ataacatgct tcataaatta ggaaattttt 42120
ggtggtagaa gttctgtgta aagccacatt caagggtgat tatgttctgg gaaggggttt 42180
atagctattt ctacactgga ttaaacactg ggtttaattt tgagatgtta tccattaatg 42240
aaccatgtga gtaaatactg taaaattcta aacatatatt aatttaaaag catactaagc 42300
tttttaagcc tttgagatta tcctgttact atattctaat ttttaattag cctttcagac 42360
tgaagggaaa ctgtacttaa tactggattt tctcagggga ggagatgttt tcacaagatt 42420

atccaaagag gtaagtatct gtacattttt actgtatttt cttaaaacaa aactaagaaa 42480
aatcactttt gttcttctgc taattacatt tttcctattg aaattttaaa tggtaactac 42540
tctgtttcat tgcatagtat ttggtaatgt gcctgttaat ggcattttct atcatctcaa 42600
tgtgctactc attattttct taggcaaaag aacaatatga ataactggat aaagtagata 42660
cgggcaagcc taggactttg gctactcaag ctacctaaac acctctggga gtagtgttta 42720
ttcattttaa aatagcttta tggaggtata attgacatac aataaactgt acatactaaa 42780
gtatacaatt tcataagttt ttacatttgt atataactgt gaaaccatca ttacaataag 42840
aataattagc atatctgtca ttcccgaaag ttttcttgtg tccctttgtg atgccgtcct 42900
cctgccctaa ccctactgaa tctgctttct gtcactattt ggtttgtact ttctaggagt 42960
ttatataaat ggaatcatat agtatattca ttttttttgt ctggcttaac ataagtactt 43020
tgagattcat ctatgttgta tgtactaata gtttattcat ttttattgat gggtagtatt 43080
actgattgta tggatacatc atgatttgtt tatcattcac ttgttgatgg atttttgggt 43140
cattgcagtt tttgatatta ctaataaaac tgctataaac attcatgtac aagtgattgt 43200
atcatatgtt ttcgtttctc atgggtaaat acctaagagt ataatgactg ggtcatgtgc 43260
taggtatatg tttaactttt aaagacactg ccaaactgtt ttccaaagtg attatataaa 43320
ttttacattc ccaccagcag cgtaagagag tttcagttgc tccacatcct tgccaacatt 43380
tggtatggtc agtcttttaa aatttttgaa cattagatta gtttttagtg gtatcccatt 43440
gtggttttga attacatcac actaatgact aatgacattg agcatctttt catgagctca 43500
tttgccatct atcctcttgg taaaatgtct gtttaaattt ttagcctgtt tttttttatt 43560
ggattgtttg ttttcttatt gagttttgag agttcttcat atattttgga tgtaagtcct 43620
tgatcatata tgttatttgg aaatattttc ttctggtcta tagtttgtct tttcattctc 43680
ttaacagtgt attgaaaagc agaagttctt aattttggtg aagtctgatt tataggattt 43740
tttcccttta ttaattgtgt ttttagtgtc ctttgtaagg catatctgcc taatcacagc 43800
taaagatttt ctcttatgtt ttctagaggt tttgtagttt gcattttaca tctagattta 43860
ggatccgttt tgagttcatt ttagtatatg gttacaggta tgtgcagaag tccttttcat 43920
ttgttcacat agatagccag ttgttatagc atcattattg gaaaagtctg tcttttcttc 43980
actgaattac cttacatttt tcttgaaaac cagttgatca tatatatgaa ggcctatttc 44040
tggatactct gttctgttca ttgatccatt tgtctatctt tatgacaata ccacactgtc 44100
tgaattactg tacctttata atatgtcttg aagtgaggca gtgcgagtcc ttgagctttg 44160
cttttctttt tgaaagtttg gctcttctag gtcctttgca tttccatatc aattttaaaa 44220
tcggcttttc actttgtaca aaaatgtcct gctgggatat tggttggaat tttattgaat 44280
gtataggtca atttgcagat aagttacatt ttagtaatat tgagtctttt aattgatgaa 44340
cgtggtatct ttctcaattt atttaggtca tctttaattt ctcgcagtat tgttttcagt 44400
gtaattcttg caattctttt gtcagattta cctctaagta aatcctgttt ttaatgttat 44460

tgtaaataga attgacttcc taatttcaat tttaaattgg atggaaacac aaccgatttt 44520
ttgtgtattg agcttgtttc ccacagtcta gccaaactta atacttctag cagcttttag 44580
aaatagattt caaaggttga gaaagttttc tgtaactatt tttgagagtt ttataaaaaa 44640
tcaggaatca atttcagatt tattcaaatg ctttttcttc atttattgag attataattt 44700
tttttagtct tactgtggtg aattacatcg attgatagtc agatgttgaa ccaaccttgc 44760
atttatggat gaaccccact tagtcttgat gtattaccct ttttataaat tgttggacga 44820
tatttgctga atatttaaga atttttgcat ctatttttat gagggatatt tatgattttc 44880
ttgtattatt atgtctattt ttggtattag agtaattctg actccataga atgagttggg 44940
tgtagttttt gcctctttaa ctttctggga aagtttatgt agattcaatg ataaattgcc 45000
cttaaatgat tgatagaatt caccagtgaa ggcatctgaa cctgaagttt ttcttatagg 45060
aaagtattta actccaaaat caatttatgt aatagacttg ggttatttat ttttgagtga 45120
gctttgctaa cttgtgtctt ttaaggaatt tgtctatttc atcaaagtta tgaaatttat 45180
tggcataaaa atgttcataa tattccctta ttgtcctttt tgtagttgtg gaattgttag 45240
tgatgtcact gctctcactt ttgatgttgg taattcatgt ttgctctgtt tttgttctgc 45300
ttagcttcac tagagattta tcaattttat tactctcaaa gaaacagctt ttggtttcat 45360
tgatttttct gtattctatt tctgttctat tacattgttt tctgctttaa tcttttttac 45420
tgcttatttt atgtattagc ttttcttttt ctggttttta agatgggagc tgaagttatt 45480
gatttgaggc ctttcttttc taataatgta ggtgtttagt gttataaatt cccctctgaa 45540
tactgcttta gggtcatctc acaagttttg atatgcatca tttttatttt ctctcagttc 45600
agtgtacttt atgatttcct ttctatttct ttgacccatt gatatttaga agtctgctta 45660
atttacaaat atttgggcat tctttcacat atctttctat tgttgatttc tactgtaatg 45720
ttcttgtggt cataaagcat actttgtatg actttaattc ttttaaactt atatttgctt 45780
tatgcttcag aatgtcgtgt atcttggtaa atgttccatg tccacttgag aagaatgtgt 45840
attctgctat ttttgggtgg tggaaggttc tataaatgtg gactggatca agttgtttga 45900
tagtgatatt aaattgccta tattctttct aattttctgc ctattctaca aatttttgag 45960
acaaggattg acaccttttt actttaatta tgaatttgta tatttcttct tgaatttctg 46020
ttagtttttg tttcatatat ttcaaagttc tgttattagg tacataaacc tttaggttta 46080
ttattccctc ttgatgaata gaccccttca tcgttatgat atgacatgac tgactttatc 46140
cctggtaatg ttcttttctt caaaatctta tttgatacta acatggtcac accaactatt 46200
ttctaaaaaa gtagtgttag tttggtatat ctttttccat attttaactt cagcctatgt 46260
gtgtctttat attttaatgt gtggtactta aggaagcata gtttccttgt tttttaatcc 46320
aatctgagag tccacgtctt ttaattggga tattagacct gttacattta atatgattat 46380
ttttgtagtt ctatttaaat ctactatctt gctatttggg attttttttg gtcccatttg 46440
ttctttgttc ttgtttccct cttctcctgg cttcttttta attacttttt ttttttttac 46500

aatttcattt ttatctctgc tgttgtcttg tagatattat ccataacact ttgttgtgta 46560
atttaatagt taccttagga tttgtagtat aagttttaac tcagaggtca ggaaaatatg 46620
gtcttcaggc taaatttggc cagttatttt tctttgtaaa taaagtttta ttggaacaca 46680
gacatactca tttgtttaag tattgtctgt gccttatttt gtacttcaat ggaatcatac 46740
ttgcatagtc atttttactg gatagcaggc atggtggatt taattttctt acatgctaga 46800
tacctttatt ttcatataaa tagtatttag cttttttcta ggatgcagtt cattttcttg 46860
taaacacttt gatcattttg ggccttgtgt ttaagctttg tcatgtggga ctagggaagt 46920
atttagttta agattaattt ttcctcagta tgaagcatgt cctttcttat actctaacaa 46980
agactctttg aattatgagg cttttcattc taacttttgg gaagaggagc tatctcagtc 47040
ctttgtgagc tgagtgggtt ctttctttta atcttttaag gtttttcttt tcctgggctt 47100
gggcttgggt agtttcctca gatccatgtt tgactatata tatatatatg tatgtttttt 47160
tttttttttt ttttgagatg gagtctcagt ctattgcccc ggctggagtg cagtagcgtg 47220
atctcagctc actgcaacct ctgcctcccg ggttcaagtg attctcctgc ctcagcctcc 47280
tgcatagctg ggactacagg tgcacagtta gcccggctaa ttcttgtatt tttagtagag 47340
atggggtttc accatgttgg ctaggctggt ctcaagctcc tgacctcaag tgatctgcct 47400
gccttggcct cccaaaatgc tgggattaca ggcatgagcc ccagtgcctg gcccatgatt 47460
gacaattttt tattagatgg cagacattgt gacatttagc ttgttaaggt gatgaacatt 47520
tttgtatttc tgtaactatt attgatctct gttctgagat ataaaactag tttgattatt 47580
ataggtcttt taaagttcgt tagattggac taacacagct ttcagtcggg ttaattttgc 47640
tccactactg aagcaaaatc cttctgatta ctttatctga tgtcctatga attatgagat 47700
tttctgctct ggctgaaggc aacaggaact attcctggcc catcgtgagt tcatcgactg 47760
gttcctctaa cccttcgttg ttttcctggt ttagggtaat gtcctcactc atatgtgctg 47820
agtatttctc cactgaatac ttgaaggaga ctgtctgcat atctacagag ttctctttgt 47880
gcaattttct cttctctaat actccatcct gtgaattttg tcctccttct ctaagactct 47940
ctgttctggc ttatctactc aggaagtctt tcaggctcca cctgacttag ccctccctgt 48000
actaccactt ggaaactctt tccaggtagt aagctaaggc agttgtgggg catacgtcat 48060
ttgtcccctg tctctcagta ttcagtgtcc tttgttgcct gatgtccaat gtcttaaaag 48120
tatcatttca tacgttttgt cctgtgtttt agatttttca ggcatgagat taaatcctgt 48180
ctttgtctgc ttcatttttc tttaagtagt agtctgggag tagcatttta attgggtgat 48240
agtagttcaa tgtcttactt ccagaattta cagtgtagaa tgtgggagag ctactgttga 48300
ttcccagagc tctggaactt gctgtgataa aggaacagtg tgtttctggt atgctagcca 48360
gtgcttaagt ggctaaggtc tctggcttat atctgtctac ctccagtgta gaatgtgcca 48420
tgcgtgagca tagggtgtgt tattggtata ttattaacta tagaacataa ttcaatatct 48480
gaaaaggatt gtctaggacc cccttctaag gagggtcttt aaaggacctt gttaaaaact 48540

ggtaccaagg catagaagga caacagtata ataaggctcc attattcatg atttcttgcg 48600
ttagatttta gaagtgtcac aaatatgttt gagaaattct tgtagtcatt tctaattttc 48660
ttttaaactc ttcagacctc cattctcgat gtgggactat ctaaacccat actagcacag 48720
agatgcagtc agtaagtttt gattcctaag tctggaatat taggtaagag caagcatttt 48780
aaatgcgtta cccaaaagat gataaattag tacattgatt gagcagcctt ttaaaaaatg 48840
attcaatggt tacttctgta gacagcttat taataattag atgtttgttg gttatacttc 48900
acttgtgtgt acaaaaaatt gttaatccaa aatgaggaca tgtttttata ccttagaaaa 48960
agacttttta aatacatttt tgtatgtttt aaagaatttt ataatgtatg tcatttgtat 49020
tttaaaggga aagataccta cccaaatttc ccttcctcag aatgtacaca cacacacaca 49080
cacacacaca cacacacaca cacacaaata cacaggtaaa caaacagaac tcttaagctg 49140
tgctctattt tccttattca agaaaatgtg gtagaatgaa tggctgatta ttgtaaagct 49200
gtcagttctt ctaaaattaa tttattggct taacaaaatt ccaaataaag ccacaatagg 49260
gatttttgtt tgtttgatgt tgacagaatt attttatagt ttccttggaa aagtaaacaa 49320
atgcaaagaa ataatatttt tgtttgtttg ttttttgaga cagggtcttg ctctgtcccc 49380
aggctggagt gcagtggcac tatcctggct cattgcagcc tcaacctccc atgctcaggc 49440
aatcctcatg cctccgcctc ccgaatagct tggactacag aagcgtgcgc cactatacca 49500
ggctattttt ttttttcagt agagatggga tcttgctatg ttgcctaggc tggtctcaaa 49560
ctcctgggct ccagtgatcc tcacgcgttg gcctccccaa attctcttgt ttaaaacttg 49620
gtaatgtgca taagcaaaac attatagtat caacacaaga ttggaaacat actgtatagc 49680
ccaaaaatat cctgttatag attacttacc ctatatcatt atgataaagg aagaatcaca 49740
aattgtggca aagaggttgt tcaataaaca gtatttgaga aattgcttaa atatttccaa 49800
aaatcatttt aaaatgccat ctctaccata tacaaaaata aatcttactt acataaaaaa 49860
gctaaatctt gaaaaacaat taccctataa taaaatgcag ctaaatattt tgttgaccat 49920
tggatggaga atgtctcctt aaacattaaa ataatgaagg aaaaatcata cagaaatcat 49980
catacatttg actagcaaaa attaaggatt actgaacatc aaaaactagc acaaaattgg 50040
ataaatattt gcagctaata tgatagaaaa ctgacatttt taacatttaa gtgacttgta 50100
ccaattgaca aaacagtaag tcagtaatag aaaataggca caaacattac tgataattca 50160
caaaaaagta gaaagactac taaacatgaa taacaatgtt cagcctaaaa atcaaaactt 50220
aaatttaaaa aataatgcca ttttcacaca tcaaatttga aaaggctttt tacagggtat 50280
attccatgct atcaaggatg tggtaagaca gatgctttcg taaactgctg atgggagtgg 50340
aaattactat aacctttttg gaaaataatt ttatcgtgta tgtcaggaat cttggactat 50400
ttatgctcct tgacccagca ttccattttt aaaaaaaaat tcttaattca ttttgagaaa 50460
atgatcataa atatcagtaa gatgtacata tgcatattgc agagttattt caaatagaga 50520
aaaaatggaa ataacctaaa tgtcaagaag ggaatgtttt aggtaaatta cagtaaattg 50580

gtatggttat attgtgcagc tattgaaaac tatttataaa taagttttaa agacatagaa 50640
tgcttatgat atagtattaa ctggggataa atgaggtcac taaatagtac ttatacagta 50700
gtgtgatttc aactgtatta aaatacacat ggacacattc acacacagat atgcacaaaa 50760
aatttgaaaa gcagatgaca aaatattaaa agtgctttac tcaagagtac tggagtttag 50820
gataattttc attttgtagg gggggcaatt tatgagtagg acgattagtg ggttggaaag 50880
cattattggt agtaaaccta aaatttgttt ttagaaaagc aaacattgca tcacagcatg 50940
tggcagaagt ggcttctggg aaagagttca ttcttatgga caagtatccc tgagttcatg 51000
tcttgggcct ccttcttaac tagctgtgtt attttggaca agttacttaa tcactctgag 51060
tttctttctc catttgcaag gtgttcctaa tacctacctc atagagtttg gaagctataa 51120
taacataatg attgtacagt gcctggcaca taattgctac ttagtaatgt tttatttgca 51180
tctatcatga ggctttctac caagacaacc tcatgacaag gtgataggct tgcataactg 51240
taactaggca aagttttttt ctaatttagt tttaaataat ttttatttag ggttttctta 51300
cgtattagct ttcagagaac attcttatta tccttggtta gaagttgaat aggcaacagt 51360
tatttttatt cttaataaaa agtcaagtga aagtgatgtt tttatatttt taccattata 51420
atattcaaat taatgctccc aaaacttaga taataaacaa tctttgtttt tctattcatc 51480
attttaaaaa gtagttagca tatcagaagt tattaactac taattctctg caattttact 51540
agtaacctgg atctgcattg tgaaatgcag tggccgtaat tctaaatggg caagcaggtt 51600
gtaagctatt atgacatgta ctttatgcaa ttttgagcct tcttaataat ccagaggttt 51660
ctttatgaaa ataacccatt tttagaaaaa tatttgattt tgccaatccg gagttcttgc 51720
taaaccttaa aaattacatc taatggctga ttagtgtcca gttctatgtg taaatgaata 51780
ctcactagtg aatgaattta gatgtaaacc tataataata gtaccatatg gttatatata 51840
tagtttttta aagtttacaa agcttttttt ccacataaac tgatttgatt cttacgacaa 51900
ccttgttagg tatatacagc agatattatt gtcctgtttc acagatttag aaactgaggc 51960
ccaaagggct aagtgacatg tctgatatta cactgccaat tctgatttca agttcattac 52020
tcttcacttc atcatagcag ccgtatttcc agacacaaaa tgattaatac agtgccatta 52080
cactcgtata cattactaac agaagaggct tggatccacc ccttaggtca gagatgagtt 52140
cacaattgtg tgtaagataa gggtactaat ttaacaactt gtggcagtgt agtaaaagct 52200
ttgggtttgt agagccccta aaagtgaaat ttctgagaaa tgatactaga cagtaaaaga 52260
gagaatggta taaatagtgg aattctcaga gctaaaacaa aataacttct atatttcaaa 52320
taccctaagg gttactatat tatagcagat tactataatt aatattggta ttatgaggca 52380
aagtcacttt ataaaaggtg aatcttgttc tttgcatttc gtgtgagaag atatacaaac 52440
aacataatat ttcagaaaga tttacattaa aatggttaat atcgaagaga cacattctta 52500
aagagtttag tttcatttat atagaaatct tatttctctg gtattgccaa taattatgcc 52560
agtcaaataa gaattgactg tgcttattat cttattattt tcctagattt gtctgtctac 52620

tcattttgaa agaagtattt ttaaatatac tgaatattga aagaaagatc aaatgataaa 52680
tcattatttt gttctaggtt ctgtttacag aggaagatgt gaaattctac ctcgcagaac 52740
tggcccttgc tttggatcat ctgcaccaat taggaattgt ttatagagac ctgaagccag 52800
aaaagtaaag ctgtattctt ttgttttcat gttgtctgta tttctggaac tgctttttgt 52860
caaactctgt tagatctttt aaatattata gggaattcta tatgaattat atgaaactta 52920
tttgtaaaaa gtaatctttg tgagacgtac attgacttta agttaatcat ttaaattttt 52980
tacatatatt aaatagatac tgacctatct tagttttatg atttttatgt aaatgtttta 53040
tgtaaaatat tttattccat cttgttgaat gtgaattttg atttacagca ttttgcttga 53100
tgaaatagga catatcaaat taacaggtat gcagattttc ttattatacc cttagccact 53160
tgattcatat tttttattat tttaatataa atttgtctaa gcctgttttt ttctctttct 53220
gaatttgatt tgcaatttat ttgtctcttt agtgaattaa atgttacttg atggttagac 53280
tgctctcaat atgttctaat tgtaatatta aaaaattgta cattagatac acttatcctg 53340
aataagaaat gatcgttttc taggtaacaa tacatcatct tagtttgagt ctagacagaa 53400
agatgtatca caagtagtca tcacatttga aatatagtga caaccttccc tccaccttcc 53460
ctaactcctg gcaaccactt atatgtccta catctctata attttgttat tttgagagtg 53520
atatagaaat agaatcatac agtagtcaca ctactgagca tttatcctag aaaaataaaa 53580
acctatgttc acataaaagc ctgtatatga atgttcataa caactctatt tgtaatagcc 53640
caagactgga aattacctaa atgtccttca gaaggggaat ggataaatgt aaattgtcat 53700
acttccatat gatgacatac tactcagcag taaaaaggaa agacctgttg agacatccag 53760
caacttggat gaatctcaag ggcattgtgc tgagcgagcc agtctaaaaa cgttacatgt 53820
gttttcaata gatagtaaat tctgtatttt attatttgaa tgcttgaaca gagataagat 53880
gaagatgtag tctttacaga taattttaaa taggtacagt ttaaaggtaa gttgctttaa 53940
attcctgtgt atactgcatc aatgcaaaaa taaaatatca aacagttacc tcaaaagctg 54000
acataaatat gaaaaagttc aaataaagtt tagtgactct tattgattca cagcctgatt 54060
atctttttca tgtacaaaag aaatgatgtg ggttcgaaga tatttaaaga gaaaaaggaa 54120
aggaaaataa cagtagggaa cagtagagtt ttgagatttt tatataatac ataaatttta 54180
ttttatagca attaaaaaga taattaaaaa ttaattcctc ttttcatttt tgcttaataa 54240
agtatacctc ttttgtcaga ggtgagaaag agaggtggga aaaagagtag aaaaaaacct 54300
atttttctct ctctacatta aggttaatta cgtaaataat gatgccaagt ttatttgtat 54360
ttagtgttct cattttaatt ctgcttttga tagattttag agacttaaac tgtctgagga 54420
gggaagaata gataggatca atattaattt ataagcaagg gacacaaagc atgtaacagg 54480
aagaaatgtg atttataatt ttttttaaca attcaattta ttttccttgc aatggcacaa 54540
acatttcttt taaagaatgt gtacaataat aaatattaga taatttacag ttttatgatt 54600
ataaccttaa ttcaaggttt tataatgact acatttatgg atttaccccg acagcattcc 54660

aatgaggttg gttaaagtat gaccactcac agcaagtatt ttaacccatt tatgcctgag 54720
gctgcaatgt tttgaatttt tgcagtcaga ctttgatgat gaccttgagc agtagaatat 54780
aaataactcc cacatgctta gcgttccaat aatggaacac taggcataaa tgggtttaag 54840
agttctttta taatgtttct tttgatttat tttatattct ttatctttct ctttaatttt 54900
tcttgtttgc tttctttctc ctagggaggg tgtttgctag cattctgttt tccaaaaata 54960
attgcaggag aatggcaata cttttttttt tttcactagt ccactttaaa aattctccag 55020
acataaggaa tggacctagt atctggaatg acaggctact ctgaaattac aatggactat 55080
agaaagtttc ctgttgtaac tgtgctttga gtctgattgg cctcgttctg agattatgat 55140
tgttataaca gatcagtaga atgagagtgg atgagagtgg atattcccaa agatctacat 55200
ccttgctaat atattattta tattatatat tattatatta taaaacttaa gtttttttcc 55260
tgaaatcctg catccttttc atagtctgta taaaatccct tgtatagttt atataaaaat 55320
aactttataa tatgacttgc ctctttagaa aaattatttc atcataatat cccattaaaa 55380
agtacagctc gaatacagaa tttatttgaa ctgtggttgc tataatctgt tgcatttttt 55440
aaattttatt ttttttaaga gacagagtct tactctgtca cccaggctgg agggcagtgg 55500
caggatcaca tcttatcaca gccttttttt aaattattat tattatcaca gcctttaact 55560
cctgggctca agccatcctc ccaccccagc cttctgggta gctgggactt caggaacatg 55620
ccaccacacc cagctatttt ttgtgtgtgt ggcgacggag tcttgctata ttgcccaggc 55680
tggtcttgaa ctcctggcct caagcaatca tcctgccacg gcttcccaaa gtgttggggt 55740
aacaggtgtg agccactgtg cccagcctat aatgtgtttt ctgagagaca taattcagaa 55800
gcaacttcta caaagagagg gaagaaaaat atacagtggt cagagatgta aatcacacag 55860
tacttgagct gagtatttaa gagaaaaatg tatacttgag aaaataattc tatgtagttt 55920
ttatagttac ttgcaaacta atcactgaga ttgcttttat ttatagtaac tattcatcaa 55980
attattatat tctgagtgtg cttttaaagt ttaaagtctt agaaaagagt catttagatt 56040
acaaagatgt gaaacactga ccctcaaacc taccccccaa aataaccctt ccattatgta 56100
gttgaattgt acattttctt ttattttcaa attagtgatt tggaaaaacc actgtataac 56160
atatggcttg tctaatcttg tagctagttt tatagttgta caattttcgt cagttgattg 56220
ctgagcaaat tatagttgtt aaataacagt ttagtcagaa atagtacggc aaaggaactc 56280
tgtggattgg cataaatctc tgaaaggtaa cagaagaatg agaaaattct tctctccccc 56340
cttctctctg gaatggccaa agaaactagt gttcttctcc acaagctgtg ttatattcag 56400
ctgaactgac ctcgaagact agatgttaat tatatttact tttgtaaatg gaatgtgcag 56460
agtttacaag tttcagtata cagagagaac atagtggcac agtgaaaaga tttttaaaaa 56520
ttataccaaa agttaaaata cattaagcta ttatctaaga ataagtgatt tttttaaatg 56580
aaaatgttga gcatagttag aaaatcagtt ttgaggggac ctcaaattaa gtgttaatgt 56640
tgatatttta ttgtaaacaa gcaatatagg gcttataaac ttttttattt ttaaaatgaa 56700

agtatacttt acatatatta aaactcatcc ttctgatgtc ttttggtgtt gggactatcc 56760
cgtgtcttga ctgcatcaat gtcagtgtcc tggctgtggt tatagtacta tagttttgca 56820
agattttacc atgggaaaca ctggataaac aggatccctg tagtttttca tgtaacagca 56880
tgcgaatctg caatcatctc aaaatataaa gtttaatttt taaaaatcac ttttgttgta 56940
tgtatagttc tgtaagtttt gacaaatgcg tacagccttg taactatcac caaaataaaa 57000
atatgaaaca gttccataac cctcctcccc caaattctcc ccttataagt cagtcctccc 57060
tttaccccaa gccaactacc agtctgcttt ctgttcctgt atatatgctt tttccataat 57120
gtaattatga acagaggcat acagtataca gccttttgag actggcttca tttaattagc 57180
atagggcatt tgagattcat ccatattgtt ggctgtatca gtgagtgttt ttttaaattg 57240
ctaagtagtg tttcatttta tggtggtacc atgatttgtt tatccattcc tcaatgagga 57300
ggcatttgag ttatttcctg ctttgggcaa tgatgaataa agccactgta aaacttcatg 57360
tataggtttt ctttttacca taaattttta tttcagttaa gtaaatacat aaggagtgag 57420
attagttgat catatgttga gtatatttaa ctaagtgaga aaacttcaaa ctgatttcca 57480
aagtggttgt aatattttgt atacccaccc acaatgtcca gtggatctgt atcctcacta 57540
gcattttgta ttgttagtat tttttatttt agctattcta ataggcatta ggtagaatta 57600
tcatggtttt attttgtatt tttctaataa ctaatgacac taagcacatt ttcatatatt 57660
tatttctttg agaagtgtct attcagatct tttgcccatt aaaaaattgg attgtttgtt 57720
tggttttcag cctagaatgt tttatatttt ttaaaattta aatttaaatt ttgtgggtac 57780
atacgtgtat atatttatgt gttgtatgag atattttgat acaggcatgc aatgcctaat 57840
aatcacatca gggtaaatgg ggtatataca tcacctcaag catttatcct ttatattaca 57900
aacaatccaa tcatatgcat ttagttattt aaaaatgtat aattaaatta tttttgacta 57960
tactcaccct gttgtgctag ctaatattaa gtcttattta ttctaactat tttttgcacc 58020
cattatccat ccctcatccc ctaagtccct tcccccgtac ccttcccagc cactagtaac 58080
catccttcta ctctatttcc atgagttcaa ttattttaat tttcatctcc cataaataag 58140
tgagaacata agtttatttt tctgtgcctg gcttatttca gttaacacag tgacctccag 58200
ttccactcac aatgcagatg acaggttctt actctttttt atggatgaat agtactctgt 58260
tgtgtataag tgccacattt tctttatcca tttgtctgtc aatgggcatt tagattgctt 58320
tcttatcttg gttattaaaa acagtgctgc agcaaacatg ggagtacaga tatctcttca 58380
ttgtactgat ttcctttctt ttaggtttgt acccagcagt gggattaatg gatcatatgg 58440
tagctctgtt tttagttttt tttgaggaac ctccaaactg ttctccatag tggttgtgct 58500
gatttacatt ctcaccaaca gcgtatgagg tttccttttt ctctccatcc ttggcagcat 58560
ttttttattg cctgactttt gaataaaggc ctcattttag ggagaaatta tatctcattg 58620
tagttttgat ttgcatttct ctggtgatca atgatgttga gcaccttttc atatgcctgt 58680
ttgccatttg tatgctttct tttgaggatt ttttattcag atcttgtgct cattttttaa 58740

ttggattatt agactttttc ctgtagagtt gtttgagctc cttatgtatt cttgttacta 58800
atcccttgtc agatgggcag tttgcaaata ttttccctta ttctgtgaat tgtctcttca 58860
tgttattgat tgtttccttt gccgtgcaga agctttttaa cttgatgtga tccagtttgt 58920
ccatatttgc tttgatcacc tgtgcttgtg ggctattact taagaaatct ttgcccagtc 58980
caatatgctg gcaagtttat ccaatgtttt cttgtagtag ttttattgtt caaggtctca 59040
gatgtaaatt tttaatccat tttgttttta tttttgtata tagcaagagc tagagatgta 59100
gtttcattca tctgtatatg catatccagt tttcctagca ccatttattg aacacaccgt 59160
cctttaccca atgtatgttc ttggcacctt gtcaaaaagg aatttactgt agatacatgg 59220
atttatctct gggttctcta ttctgttccc tgttctatgt ttctgttttt atgacagtac 59280
catgttacta tagctttgta gtataatttg aagttagata atgtgattcc tccagttttg 59340
ttctttttgt gtaggtagct ttatctatac tgggtatttt gtggatccat ataaatttta 59400
ggactttttt ttctatttct gtgtagaatc tcattctact ttgaaaggta ttgtgtttaa 59460
tctgtagatt gctttgggta gtatggacaa ttttaacaat attgactctt tcaattcatg 59520
aacttggaat atctttccat tttttgtgtg tctttctcaa tttcttgcct cattttagta 59580
cattcctttt tacatgtctt taatttcttt ttatcagtgt tttgtagttt cattgtagag 59640
atattttact tcttggttaa ttactatgta tttgatttta tttttagcta ttgtaagtgg 59700
catcactttc tggatttcat tttcagattg ttctctgtcg gcctgtagaa atgctactga 59760
tttttgtgtg ttcattttgt atcctgcaac tttgctgagt ttgtcagttc taatagtttc 59820
ttggaggagt ctttaggttt ttccaaatgt aagataatac catcagtaaa aaaggataat 59880
ttgacttctt cctttctcac ttggatgccc tttttctttc tcttgtctga ttgctctggt 59940
taggacttgc agtactatgt tgattaacag tagtgaaagc aggcatcctc attgtgttcc 60000
aggtctcagg ggaaaggatg acagtttttc ccaattcagt atgatactaa ctgtgggtgt 60060
gtcatatatg gctttgatta tgttgaagca tgttccttct atacccaatt ttctgagggt 60120
tttttttttt atcatgaaga gatgttgagt ttcattgggt gctttttcaa catcagttga 60180
aatcatcatg tggtttttgt ccttcttttt gttggtatga tgtgtcatat taagtgattt 60240
gcatatggtg aaccatcctt gcatacctgg gacaaatctc acttggtcat gatgaacagt 60300
ctttttaatg tattattgaa ttcaatttgc tagtatcttg ttgaggattt ttgcattatt 60360
attcatcaga gatattggcc tgtagttttt tgttgtctta tctttgtctg gtttgggtat 60420
cagggtaata cttgcctcat agaatgactt tgggagtatt ccctcctgct ctattttttg 60480
gaatattatg aacaggattg gtattaattc ttctttaaat gtttggagga gtgtagcact 60540
gaagccatca ggccctgggc agttcattac tgggagactt tttattatga tgttgatctt 60600
actaattgtt attggcctct tcaggttttg gatttcttgg taggttttac gtgacaagga 60660
atttgtccat tttctctaga ttttcccatt tattggcatg tagttgctca tagtagccac 60720
taatgaccct ttgaatttct gtggtatcag tcgtaatatt tcctttttca tctctaattt 60780

taattattgg ggtcttctcc ctgtttttct ttgatagtct ggctaatggt ttatcaattt 60840
tgtttgtttt ttcaaaaagc aagtttttat ttcattggtc ttttatatta ttttcctcat 60900
ttaaaattca tttatttctg ctctgatatt tattacttct tttctccgac taatttgggt 60960
tcaattcact tgcttctcta gttctttaag atacatcata aagttatttg aatttttttt 61020
tgatgtaagc actttcagct ataaatttcc atcttagtac tgctttcact gtattccata 61080
ggatttttta tgttgtattt ctattatcat taatttcaag aaatttttaa atttctttct 61140
taattttttt attgactctc tggtcattca ggaacatatt gttttatttc tatgtgttta 61200
tatagtttac aaaattcctc ttgttgttga cgtctagttg tattcagttg tgatcacaga 61260
agatgcttga tattatttca gatttttgaa tatattaaga cttgttttgt gatctaacat 61320
atggtctatt cttgagaatg atccatgtgc tgaggagaag aatgtgtatt ctgcagcctt 61380
tggatgaaat gttctgtaag tatctattag gtccactttt tctatggtgc agattaagtt 61440
cgatatttca ttgttgagtt ttctgtctgg gagatctgtc caatgctgaa agtggggtgt 61500
tgaagtcacc ggctgttact gtattgagtt ctatatccct ctttatctct aataatattt 61560
gctctgtata tatggttgct ccagttttgg gtccacagat atttataatt tttctatctt 61620
cttgcaggat tgaccccttt atcattatat aatgacctcc atttcccttc ttataatttt 61680
tgccttcaaa tctattttgt atgatataac tactcctgct cctttgtggt ttccattggc 61740
atggaatatc ttttcccatt tctttatttt cagtctgtat gtatctttat gggtgaagtg 61800
tgtttcctgt aggcaacaga tcattgtttc ttgttttttc atggttcatc cagtctgtgt 61860
attttgattg tttagtccat ttacataaaa tgttattatt aataaatcgg gacttactca 61920
tgccattttt ttagttgttt tctggtcttc tcttccttgt tttcttcctc cctgccctcc 61980
ttttactgaa ggtgattttc tgtggtgctg tgatttagtt tctttctctt tatttattta 62040
tttatgtatt ttttgagatg gagtcttgct ctgtcgccca ggctggagtg aagtggtgca 62100
atcttggctc actgcaagct ccacctcccg ggttcacgcc attctcctgc ctcagcctcc 62160
caagtagctg ggactacagg cacctgccac cacacctggc taattttttg gtatttttag 62220
tagagacagg tttcaccgtg ttagccagga tggtctcaat ctcttgaact tgtgatctgc 62280
ccgcctcggc ctcccaaagt gctgtgatta caggcatgat ccaccacacc cagccttttt 62340
ctctttattt tttgcatatc tgttgtatgt ttttcggttt gagaatacca tgaggcttgc 62400
caatatgatc taatacctct tattttaaac tgatgaaaac ttaacactga ttgtccaaac 62460
aaacatgcaa aaaaacctga taaaaacact acactttgaa ttcatccccc cacttttcaa 62520
gttttcattg cttctcttta tgtcttattg tactatgttt tgaaaagttt ttgtagttat 62580
tatttgattg gttcatcatt tagtctttct acttaagagg aatttatacc ccacaatgac 62640
agcataataa tattccctgc atttttcttt atgcttacta ttatagtgag ttttgtactt 62700
tcagatgatt tcttcttgct tattacatac ttttctttca gattgaagaa ctccctttag 62760
catttcttat aagacaggtc tggtgttgat gaaatctctc agttattgtt tgtctgggaa 62820

gatctttatt tctccatcat gcatgaaaaa tatgttttgc cagttataat agtataaagt 62880
aatttttttt cagcacttta aatatgtcat gccactctct cctaacctgt aaagtttcca 62940
ctgaaaagta tgctgccagg tgtattgggg ctccattatg tcatttgttt attttctctt 63000
gctgctttta ggatacttta tcaatgaact tttggagttt gattattaaa tgccttgagg 63060
tagtcttctt tggtttaaat ctgttggatg ttctataact ttcttgcact tgtatgttta 63120
tttctttctc taggtttggg gagttctctg atattatccc tttgaataca cttcttatcc 63180
gtctttctct gtctactttc tattcaagtc cagtagctct tagacttgcc cctctttggc 63240
tgttttctag atcttgtagc catgctttat tctttttttt ttttaatttt gtctcctctg 63300
tgtattttca aatagcttgt cttcaagctc acaaattatt ttcactcctt gatcagttct 63360
tctattaaga gactgtcatg cattctttga tatgtcaagt tgcatttttc aactctagaa 63420
tttctacttg attcttttta attattttaa tctctttatt aaagttatct gatagaatta 63480
taaattcctt ctcttcgtta tctttagttt cgttgagttt cctcaacaga gctattttga 63540
attatctatc tgaaagcctg catatctatc tgtttctcca gaattggttc ctggttcctt 63600
atttagttca tttggtgagg tcatgttttc ctggatggtc ttgctgcttg tagatgcttg 63660
ggagtgtatg ggcattgatg agttagattt ttaatttggt cttggactcc gggcttgttt 63720
gtacctgtcc ttcttgagaa gactgtacag gtattcgctg ggagttgaac cccaagccca 63780
ataatgctgt ggttttatag gctcctagag gtaccccatt ggtggccttg gataaattct 63840
ggaagaattc tctgtattac cagtcagaga acctcattct tttccattat tttctcccaa 63900
acaaatggag tctatctctg tgctgagcca cctggaatgg ggatgtggtt atgcaatcac 63960
ccctatggcc gccaccacac agactgcatt gaatcaaacc ggaagccagc acaacactgg 64020
gtctcgacct tggctcacta taactactac ctgggtaaaa cctattttca ctcaaggcca 64080
tagtgctcta cagtcagcag atgttaaggc caaacaggtt tgtgtccttc ctttcagggc 64140
aatgagttcc cccaggcccc gggcaggacc aggcatgctg tctgggagcc aaggattaga 64200
gtcaaatacc ttagaaattt acctgaagtt ctattctact gcagctaagc tggcactcag 64260
accataatcc agaatctttg ttgcacttcc ctcccctctc cacagggaga agggcctctc 64320
cctatagcca tcagcaccag cagcccatag caggggttct gccaggccac tggcaatgtt 64380
cacttaaagc ccagtggctc ttcagtcaac aagtggtgaa tgctgccagg ctgggaactc 64440
gttcttcaga gcactgggct cccatatgcc ccagggcaga tccagaaatt atatccaaga 64500
gcctaggctc agactcaaga gacctcaaga gcctgcttat tggcctaccc cactgtggct 64560
gagctggtac ctaaggtgca ttacctttct gtctgctttt ctcaaacaga aggaatcttt 64620
ttctgtagct accacagctc tcaatttgtt ggatcacacc tgaagccagc atttctcaga 64680
gcccaaggcc catggtgtac tacctgggta tattgctgct ggtggttgtt catggcccaa 64740
gagctcgtta atcagcaggc aatgaatcct gccaggactg ggttcttctc ttcaagacag 64800
tgggtttcct tttggcccag gatgtgtcta gaaataccat ccatgagcta gggcctggaa 64860

tgggaacctc acaactctgc ccagtgtcct gtcctactat ggctgagctg atatctaaga 64920
tgcaagacaa agtcctcttt actttttgtt ccttcctcct taagcagaag gaaggagtca 64980
cttttgttgc tgtgctgtct ggggttgggg gaatggtggc acaagtactc ccttagccac 65040
cctggctggt atctccctag gtcacatgcc accctagtcc actggctcta attccagttc 65100
aacactagta gttgcctagg aattgcaggg tttgcatctt agactgcctt ttaagtttac 65160
ctaggacccc agagcacttt ggcccaagat ggcgaggctt gctgaggagt tcaaatttag 65220
accactggga caggcaattc ccttctgtct agttcttgtc caaaagcttc ctctggccgg 65280
gtgcagtggc tcatgcctat aaacccagca ctttgggaga ccgaggtgga tggatcatct 65340
gaggtcagga gttcgagacc agcctgacca acatggtgaa accctgcctc tactaaaagc 65400
acgaaaaaaa ttagctgccc gtggtggcgg gcgcctgtaa tcctagctac tcaggaggct 65460
gaggcaggag aatcacttga acccgagagg cggaggttgc agtgagcaag actctgtctc 65520
aaaaaaaaaa aaaaaaaaaa aaaaaactta aaaaaaataa caaaagcttc ctccatatgt 65580
gggtgctggc tgaggccagc atgactttgc tgtctgctgt aacagggcag cactgaattc 65640
aatgtaaagc cccccagttg ctgagctttc cctcccctat ctgcaaagac tctcctcact 65700
gcatggctgc tgctggggca tggggaaggg gtgtcatcag tgattcatga ctgtctcttc 65760
taccctcttc agtgcctctt tcagcaatat gaagttaaaa ctaagtactg tgattactca 65820
cccgattttt gattcttgtg atggtgcttt tctgtgtgca gagagttgtt aaactttggt 65880
gttcctgcag aagctacaaa tggtataggc ttctattttg ccatcttgct ccatcgtttc 65940
ctcctgtagc ttctttttca ttttcttaat accattttct gaagaacagt tttgtttatt 66000
ttgatgatgt ccagtttgtc attatttttc ttttatggat tatacttttg atgtatctaa 66060
gaaatccttg tctaacccaa gatcacaaag aatttctatt ttcttttttg taaattttat 66120
agttttaggt tttacatggt ttacgatcca ttttgagtta attatttatg gtatgaggat 66180
gaatttttct atatctctat cctacttgtt ctggtaccat ttgttgaaaa gactgctttt 66240
tctccatgga attgcttttg cacctttgca agaaattaag tgacaatata gagccagttc 66300
tgttacagtg tgacatatgt gatctaaatc actgcagtat gcataaatcc atagtatata 66360
aaccacagaa cttatgggga aaaggaggca aggggcataa ctttaaaatg tttgatcagt 66420
gatacacaaa agaaaagaaa gtaaaaaggg tagcattgtt ctgtatgttc taaatgatta 66480
agaaatacat aaagactgca ataaatacag ttccttacct tgaaaaagac ccgaagtttg 66540
cttgtggaag tgggtgtcag aaaagttaca gtttgtaagt tataatgatg tagtggaagt 66600
tagattatct ggaattggaa ggaaaatttt aacgctagag gtggacagga gtggctcata 66660
acaaatacag tgaattagct ggtagatgtt tgaggtgtgt gcatatgtgc attttatgta 66720
tttctacttt cctaagctca gctgattcag ttttctgtgc tcatgtaacg cttcctgcag 66780
acaaaatcac tgataagcca gtgtgaaatt cacgttatac ttagattgtt tgctaagata 66840
tcaattgcat tagaacaaat ttgcattttc aaaagaagca ttatagcaga attaactgta 66900

tatgtcagtc tatcattgta cactcttctg ttccattgat atatatgtcc aacctttttc 66960
cggtacccca ctatcttgat taccgtagct ttatagtaag tgttgaagtc aggtgtagtg 67020
taagtccaac tgttttcttc cttttcaaaa ttgtttggac tattttattt tcttgacttt 67080
tttatacaaa ttgtagaatc agcttattga tttctacaaa agaaattttg ctgggatttt 67140
attgaagttt cattgaattg gtatatcagt ttggggagaa ttgatacctt aacaacatta 67200
aatcgtccaa ttcatgaaca tggtataact tcccatttat ttaggtctac tttgatttct 67260
ttcatcagtg tttccagttt tcagcatata attcttgcat atatatttta tagctgtaga 67320
tgtatattta tacctgtaga tttcatgttt gatatttttg gtggtattat aaatggtact 67380
ttttaaattt cgtcttccag ttgttcatta gtaataccaa gaaataagat tgatttttat 67440
tattgacatc tttagctcct tgaaggcttt gaacaaaaat ggaagctcac caaagccaaa 67500
gcaaaatttg cttttttaat attttaaaaa gtataagtta ctatttctgt tttgtcttag 67560
tctattttaa aacttagatc aatacggcag ctttgattta attgttgttc cctcacattt 67620
tttgcactat attctagtgt agacctgaaa gcatgtattt tgaggatgag atttcatgga 67680
aggatcttta aatgacctta aagagtattg ttatgaaata caggctgctt tctcttattg 67740
atctcacagg aactttctta agagtaactg taggccatcc ataaataatt gtaatgttta 67800
atacattatt ttgtttcttt tgacgttttc tattttctga agtttttcag acacattttt 67860
tggtaatact gttattatag attttggact cagcaaggag tcagtagatc aagaaaagaa 67920
ggcttactca ttttgtggta cagtagagta tatggctcct gaagtagtaa ataggagagg 67980
ccattcccag agtgctgatt ggtggtcata tggtgttctt atggtaagta gcattatttg 68040
ttttaattat gccaacctgt gattgatatt acttataatt ttattttaat tcaaattttg 68100
ggatttagca ggtcagaaag gggttctgat aatttataat taacaccaaa aaccttgtga 68160
ttggctagct gttacacaat aaaaattaag attttggtgt tctgatttat tttaaagttg 68220
ctagtaaaaa ggtaaatgac agcattgtta ttataaacta ttcatatttg atacttatga 68280
aaaggtctgt cagattctat atttcagaat gatgagttta tcagttacat ggtattttaa 68340
ttgaatgtta cagaacttag caccttacca tatagtgtct tattgtcaga ttgttccatg 68400
tattccctag aaaggttata aacctgttga gcagaggaat catgtcttat actacttttc 68460
atctcccata gtatcaagca tagtgagcat ttcagtagat gcttaataca gactgactaa 68520
tttggtatga gattaagcat agaatttgca gtagtcagta ggatgataga taaatagctg 68580
ttgaattaga tataatttgc tttggaggtc tgatccattt tgtccattta tcactgagta 68640
gcacatcaaa tgacacatca gcatgttaag tgtctcttat aagcctgaca tattaggagg 68700
cctgtggggt aggaactgat tctaagtaaa atatttggtc tttttcaatt ggagtagcta 68760
aataaactct caagaagtac cttaggagtt cagggaagat ttaatgtaag ctgatgtagg 68820
caaactgaaa gagttggaac ttaaattaag ccctgaagga tggataaaat ttaaatagtt 68880
gtgtgtcaag aaggagagtt attgtgatca tattagcaaa gacttggaga accagtttga 68940

ctagagaagt ggagaaagag ggcaaaggtg gaggaagtga agctataaac ctataagcat 69000
ttatacatga ttctctgggt aggaggaaac ttttagattc ttaaacaggc tggaaattag 69060
agaaaattgt attttaggag gattagcctt gtaagtggat tgtaatggaa gaaactagaa 69120
ataggaagac aaaagagatc tttaaagaca attgtaagat tatagagatt tcaaagtaag 69180
cagttgttaa ccttggtagc aaattagatg acacagatca aaaagaaatg aattgtcaaa 69240
gataaaaatg cctataaaat ttgtgagctt tagggggttc aaagaatggt aagttaatag 69300
aagagatagc tgaagaggac agctggtttt ggaagtggga aaaaataatt tttaacatgt 69360
agcataggtt actgtaggtt aatgactcac cactgggtgg tgataaacta ggaggggtag 69420
tggagagaga atcagaatca tttggagacc ttaatacgta tatattaact acccaacgtg 69480
tagtttcttt gagggaagat gtgagtccta tatacgatcc ttccttcatt ctctccctga 69540
agttctcacc tctactcctc cagtctttgg tggcgtacca ttacttaaga tttgggaagg 69600
aaagctggag tttgagagag attaagcctt gaacagcaga ttttgggaga cattaagata 69660
cagaagagac ttgaatgtat aagctcactt agattgtgag ttcagataaa tgagtagaag 69720
actaaggcca aagccattat tgaatctaag tttgagattt aaatactggt gatacaaaaa 69780
cttctcttta atgtaccgta atgattttta gaggtagtct ttttttatac cattatttaa 69840
tgttctagtt agtcacattg cagtcagttc tctttgagaa aatgttttcc ttcttctgtg 69900
tacaactaag gtgtatgttt cttttttaaa gctattggca tgtgtgtgtg tggcaggggg 69960
tggtggtggt ggtgtgttct gctgcttcta cttctttttt ggctcctgtt ttgggggcag 70020
tggagggaat gagaatggtt aaatctttct tgggcatgta tgaatttgaa atgcttttct 70080
gcttattttt cctttccctt tattattaaa cattctcttc aaaagagtat atttgtttta 70140
acagaaaatg cattgtgtgt attttcaaat tgctttggct ttgagaacat agccgaatta 70200
gaactgagga agaattggtt ggtttcatct gctacaggtc accatcagtt gagctatata 70260
tatagagaga gagagtggga taccagaagt atatttcttt aagtgaccta gttttctggt 70320
cccattttga tctactatca tgtagatctt acaatttgat gattatatag aagtctcagt 70380
ttttttctag agaatcttat tatatattct aaggtgttct aattgtgtgg tgtgttttgt 70440
ttgtttgttt tagtttgaaa tgcttactgg tactctgcca tttcaaggta aagacagaaa 70500
tgagaccatg aatatgatat taaagtaagt gtaaacagta tgtttttctt ttgggaaaaa 70560
aatctcttgc tttcagtttt gaaaaaatta ttgcggtttt aaaataatac aagagtacct 70620
ttttctcact gtcttgttcc cttctaccat atacaggact gtacccttgt aaaagcactt 70680
ttaaaagttc tttttgagat ttttcaaatc aaaaatgtgg ctaaatttaa gtgaaacttt 70740
ctaaaatcct ctgtttcaga gcaaaacttg gaatgcctca atttcttagt gctgaagcac 70800
aaagtcttct aaggatgtta ttcaaaagga atccagcaaa tagattgggt attgcttatt 70860
ttcttagctt ttatagaagt aactgcatgt tttgggagga taattaaatc tttatgtttt 70920
cttgtttgat ttgagcacat tacttctata cgtagaattt ttcattttgt gtgtgcattt 70980

cagacaaagt aaactctgtt atgtccatga tttaaaactg aagtagtcta gttaatgaaa 71040
gcttctgttc aacctattat tatgtatata ttatgggtta cttcttttga aataataaac 71100
agattctccc ttgtacaggt aatcttttat atatttgaag atagatattt tgcccccccc 71160
caatattctt caaagtaaat attatgtttc tgtaactact tttttgaccc ctttctggta 71220
gcctttccct gtatccagat gcgatcctat tatttatgcc cttgtgtgaa aatttcagtg 71280
tcactttatt cctttgtacc tttcaatttg gcatataaat atgaggtcat aagataaatt 71340
aggaatgaaa aaatatgtat ttaagtttaa gtattatgtt taatactatt tgatttgatt 71400
tgggagacac tttattgtag ttcaatatga atgttagtta ttttttatca cagtaaaatg 71460
aaaggaccag ctaataaaat gccagataag aaatcatgta tttttctttt caaggaatgt 71520
atgaaatatg tgtattcctg tttacagata gcagtctgga agccagggag caagactaaa 71580
aaaaatcata aaaataaaat atcatttctt ttttttatag gatcagaagg agttgaagaa 71640
atcaaaagac atctgttttt tgcaaatatt gactgggatg taagttaagt agaaagcttg 71700
ttattgaata acttcatatg accaacacag gatattaaag atttattgct tttaatctaa 71760
ataacaaatc agtggaatat ttacatacat atttattttt ctttttctta aaaaaataat 71820
ttagatctgg gggtgcattt gtgggttttt tccgtggatg tattgcataa tggtgggttt 71880
ggatttctaa tgtacccatc aaccaaacag tgaccagtag gtaatttttc aaccctcaat 71940
tcccttttaa cttctcccct tttggagtcc tcggtgtcta ttatttccat ctgtatgtcc 72000
atgtgtatcc attgtgtagc tcccacttat aagtgagaac atgggatatt tgattttctg 72060
agttatttca cataggataa taatctccac ctccatccat gttgctgcaa agataatgat 72120
tttattcttt cttatggctg catagtattc catggtgtct atatacctat tttctttatc 72180
caattaacca ttgatggaca cttagattga ttccatgact ttgctattgt gaacagtgct 72240
gtgatagaca tacaagtgca ggtgtccttt ttatataatg atttattttc ctttaggtac 72300
atacctatta gtgggattgc tgggtcaaat ttttttgatt attagttctt tgagaaatct 72360
tcatatggtt tttcataaag gttgtactaa tttacattcc cattaacagt gtataagtgt 72420
acctgttttc tcccacatcc atgccaacat ctgttatttt ctaacttttt aataaaagcc 72480
ctcctgactg atataaaatc atatttcatt gtggttttaa tttgcatttt tctgatgatt 72540
agtgatattg agcatttttc catgtttgtt ggctgcttgt atgtcttctt ttgtaaaatg 72600
cgtgttcata tcctttgccc accttttaat aggtttgttt gtttgtttgt ttacctgttg 72660
agttgtttga gtaccttgca gatctcgata ttagtctttt gtcagaggca tagtttgcaa 72720
atattttatc ccattctgta ggtcatcttt ttactctatt gattatttat ttggctgtgc 72780
aggaaattaa ttaagtccaa tttgtctact tttgtttttg ttgcatttgc ttttgggatc 72840
ttagtcataa attctttgta tagatcaatg tccagaagag ttttgcctag gttttcttct 72900
aggacctttg tagtttcaca tcttatgttt aagtcttcaa acaatcttgt gttaattatt 72960
gtaaacagtg agagataggg gtccagtttt attcttcttc atgtggctag gcaattttcc 73020

caataccatg tgttgaatag agtgttctct cctcattgtt tattgttgta gattttgtag 73080
aaaatcagat ggctataggt atgcagcttt atttctgggt tctctgtttt gttccattga 73140
tctatgtgtc tatttttgta caagtactct gctgttttag ttactatagc cttgtagtat 73200
aatttgaaat taggaaatgt gatgcgtctg gctttgttaa ttttacttag ggttgctttg 73260
gctatttagg ctgttttttg gttctgtgta aacattagga ttgttatttc taattctctg 73320
aaggatggca ttcatagttt gataggaatt gtattgaatg tggatattgc tttggtcagt 73380
atgttttttt ttgttgttgt tttgtttgtt tgtttgtttt ttgacagcgt ctctctctgt 73440
cacccaggct ggaatgcagt ggcacaatca cagctcactg cagctttgac ctcctagact 73500
taagcaatcc tcccacttca gcctcctgag cagctgggga tacaggtgta tgccaccgtg 73560
tctggctaat ttaaattttt ttggtaaaga tgaggtctcg gtatgttgac taggatggtc 73620
tggaacttct gggttcaagc gattctcctg cctcagcctc ccaaagtgct gagattacag 73680
gcgtgaacca ctgtgcctgg actggtatgg tcattttaat gatattgatt ctttcaattc 73740
atgagcatga gatgtttctc catttgtcca tttgatctag tttctttcat ctgtgtttta 73800
tagttctcct tgtagaactc tttcacattc ttggttaaat gtatttgtag gtattttact 73860
ttttgcggct gttgttaatg agattgttaa ttagaacttt catttggttt tcagttagaa 73920
catcactgtt gtatagaaaa gctactgatt tttgtatgtt gattttattt ttttaaacat 73980
tactgaattt gtttatcaag tctaggagtc tgttggagga gtcttttctg ttttctcagt 74040
atcatattat atcatcaatc aatagaggta ctttgacttc ctcttttcca atttggatgt 74100
attttatttc tttttcttgc ttgattgctc tggctaggac tttccagtac tgtgttgact 74160
aggaatggta aaagtggaca tccttgacct gtttcagttc ttgaaaggaa tgctttcaac 74220
tttcctgcat ttggtatgat gttggagtat gttccttcta tgcctattat atggagggtt 74280
tttatcatca agtgatgttg aattgtctcg aatgcttttt ccgcatctat tgagatgatc 74340
acatgatttg tgtttataat tttgtttatg tggtgattta catttattga ttagtaaatg 74400
ttgatccatc ctttcatccc tagaataaaa tccacttgat catgattaac tattttttgc 74460
atgtgctgtt ggatttggtt tgctagtatt tcgttgagga tttttgcatc tatattcatc 74520
agggatattg gctggcagtt ttcttttttg gttgtgtcct tacctgattt tgctctcagg 74580
atgataccgg ttttgtagaa ttaattagga aggattccca actcctcaat ttttgggaac 74640
agttttagta agattggcac cagctcttct ttgcatgtct ggaaaaactt gtttctagac 74700
ctgtgtggtc ctggcttttt ttgatggaac atttttttat tactggctca gtttcactgt 74760
tcctttggtc tgtttgtgtt ttctgtttca tcctggttca atcttgggag actgtatgtt 74820
tccaggaatt tatccatcat ctagcttttc tagtttgtgt gtatatacat gttcatagta 74880
atcactgatc atattttata tatatttccg tggtattcat tgcaatgtgt gcccttttca 74940
atttctgatt gtacttgtta gaatcttctc tcttttcttc ttggttaaac catctagagg 75000
tctatcaact ttgtttatta tttcaaagaa ccagcttctc attttgttga tccttttttt 75060

ggtcgcaatc tcattttatt cagcgaaatg agacttagtt attttttgtc ttctgctagc 75120
tttgggtttg gtttttcttg ttattctact ttcgtgtagt ttgacaatag gtcattgatt 75180
tgagatcttt ctgtgttttt gatttaggca tttaacacta aaaactttcc tcttagcact 75240
gctttagctg tgtctcagac gttttgttat gttctgtctc tattttcatt catttcaatt 75300
tttttattcc tgccttgatt ttgttgttca cccaaaagtc attcatgagc aacttgtttc 75360
atttccatgt acttgtgtag ctttgagagt tccccttggt attgatttct aattttattc 75420
tactgtggtc caataggata cttgatgtga ttttgatttt cttgaaagta ttgaaatttg 75480
ctttatggtc aagcatatgt tcagttttgg agagtgttac atgggcggtg gagaaaaatg 75540
tatatgctgt agttgttgat tagaatattc tgtaaacatc catttggtct agagtccagt 75600
ttaagtccag agtttctttg ttgacatttg ccttgatgac ctgtctagtg ttgtcacttg 75660
gtattgaagt cccccactat tattgtattg ccatcaatct cttttcttag gtctattagt 75720
agttgttttt gaatctgagc gctctggtgt tggatgaata tatatttagg ataattaaat 75780
cttttggttt aattgagccc ttatcattat ataatgcctt tctttgcctt ttcttactgt 75840
tgctggttta aagtctgttt tatctgatat gagaatggct acttcggctt tgttgtgttt 75900
tttatttgca tgctatatct ttttccaccc ctttacttta agtctgtaga tgtgcttagc 75960
tattctgtgg gtctcttttt ggcagcagat ggttggtttt tgtctgttac ccaacttgct 76020
ggtctacgta ttttggatgg aacatttagg catttatgtt gaaggttata ttgatatatg 76080
aggttttaat cctgtcataa tttgctagct agttgccttg gattctcaat tgtatgattg 76140
ttttatagga tctttgagct ttctacttat gtgtgccttt atgatggtga gtgacatact 76200
tttgtttcca tctttagaac gtctttgagc attttttatg gggcctgttt aatggtgata 76260
agttcctctc gtgttttctt ttcggttaat gactttatgt cttcttcatt ttatgaagct 76320
tattttggct ggatataaaa ttcttggatg gcacttttta aaggaagcta aaattaggtc 76380
cccattctct tctggcttgt taaaatttct gctgagaagt ccactgtcag tctgatgaga 76440
tttcctttat tggtgatttg atgcttttct ctagcttctt ttatgattta ttctttcaca 76500
ttgaacttgg atagtctggc gactatatcc ctcggtcatg tttgtattct gtagtgtctt 76560
ctaggtgttc tctgaattgc ttatatctgg atatctacgt ctctaacaag attggggata 76620
ttttcctgaa ttagttcctc aattatgctt tccaaacttc ttttcttttt tctttctgcc 76680
tataagttgc aagtttggtc actttacatt atcccatatt tctcaaagac tttgttcatt 76740
ttttaaaaat tatttattct ttaattttat ctgatttaat tcaaaagacc agccctcatg 76800
ctcaaaatta tttcttatgc atggtctagt atgttattaa agttttcaat tctgttttga 76860
aattcctttc atcttttttt cacttccaga atttaaatat atatatatat atatatttat 76920
ttatctttta tctcctgaat tgttttctga tttctttgtg tttgtttgta tgtgtttgac 76980
tttattttgg atttcattga gcttccttac aatccatatt ctgaatgttt tatctgtcat 77040
ttcaaagttt tcatttgatt aagattcatt gctaggtagg tactgtgacg ttttggggat 77100

gtcattacat tctttttctt cgtagtcctg aagttcttat gttggttcct tcttatctgt 77160
agaagctgtc acatattact tttgaattta ttttcatgtg gacgtggttc accccctgcc 77220
gccgcccctc ttggggggtg tgactgtaga gtaggttggg aagtagccag aagtcttttg 77280
gctttgtttc tataggcctg tgcacttctt agtcaggttt ttatgttggg ttttacagtt 77340
caatctatag ggcagtaggt ggcatttacc agtacaagcc agctgtggca aaagcagatc 77400
ggtatgcact tgatatttgt ttattgtgtg gtgctctatg ttgtttcaga tgatgggctg 77460
gacagtggaa tgccaggttc cctgaccttt ttgtcccatg aggggtgagg ggaaagggag 77520
ctaggcagag cttgattgtc tggcttgcac acaaataccc caatgatgaa ctggggagcc 77580
agccctgaca ggcatggttg ggggagtacc tggtgagcag tgccaaggtc tcagcagaga 77640
gtaggaagac tagcttcaca tcctagacag gtagcaatat gttctattta tctatcacac 77700
ccctgactca gagctcatgg ctctcagttc agatccacaa ctgccgtgta tctccagacc 77760
gcagtatagc tgatagccac agaagacact tgtctatggg agttgtttct gggtggaacc 77820
tcctgaatca gtccaataca cacagctttg tgactcacct gtttgccaat gggtaatgct 77880
tctgctttct gtagatatca cagaggggct ccacttttta gcacacacag ttgggtatta 77940
gctgtggcag tatcgtctgg ttgggtccct ctgagctcag accccaggtt gaggcatggt 78000
catgtactag cagtggtaga caggactagg caaacttcca attccttatc ccccagaatg 78060
cccaggagac agcatgtatg agtcctgtaa gaagtggacc aggaccaggc cagtggactt 78120
gttaagtctc tagactcctg ctgctggcta tgatagggag tgatgggctg gttcttggag 78180
tcacaggcag aactctcaag cagaggcagg gagagtactc tggaagtggg agtcctaggg 78240
tacatcatgt gcctgtggag gctgggatct cagaagggcc tacaaaacac ttaggcagtt 78300
gcagaatgct caggcagttg gtgcatcaat gcatattgca gacctgtggg gctgggctct 78360
cgggagcgcc cacagaatgc tcagaatata gcaaaacact caggtgattg ggtccctatg 78420
gctgatcaca ggcctgtggg agctggtctc ttaaaagggc ccacaggatt ctcagattat 78480
cacagaacgc tctggtggtg ggattcccat ggctgatcac atgctttggg gtctgggctc 78540
tcagaatggc ctgcagaatg cttatgtggt cgaagtccca aggcccatca caggcctatg 78600
gggggctagg ctctcagaag gcctccaggt aacaggtgaa atagttggga gcagggccgc 78660
tgggctgctg gcctttcact ggggaaggca gaccccttca gctgaagcaa tgaaggttgg 78720
cagctatggg gtgcttggcc cacctgcact tccctcctga aggagcagca gtggatttca 78780
ctttggtggg cacacaaagg tgcccagttt ccccactgtc tccttggccc aggagtggca 78840
agtgtgatgg tggtgttggg cgcagctatt gagggagctg aagagcgggt cacgagcctc 78900
tgggggctgg gctctaagaa gaacaccagg ctgtggccag tatatacatg taagggcagg 78960
gcagctgtgc tgtagaccca acactgaaga agggaggcct catttagaag ggaacagctg 79020
agaccggcag tctttgcagc gtgcattctg ctcactcctc tgtactgcag ctgcagtatc 79080
tgtctttgag gtgtgcacaa gtaccagcct ccctgctccc tgcctagtga ggtggcagca 79140

gccggcgcca ggctgctcag ggatcaaaag tccatgggat tccacatggg cttgagcagt 79200
acctctgcac agattccagg cagctccgtg tattaaactg gaggcctagg ggattcaagg 79260
gggtttacct atggttagca ttgaaagtct gtggcagaat tgtggaatcc tgggggtctc 79320
tcactcaatc ttcccttccc tgcatcgaag atctcctggc tccacaccag tctcagttca 79380
cctgacggca tggctttgtt ctcttctttc tgtgattccc aattccatca cttctctgat 79440
gaattctagt gtggcctctt agaaaatcta cttgaagtgt caatatttat tgctattttg 79500
attcctcacc atgagagagg catccactag cccactagct gtttctagta agccataatg 79560
aactaaaagt catgtttaca tatttaaata cataaaaatt actcaaaagt aattccctat 79620
ttatttttcc ccactgtggg actgtaagct ttttaaaaaa tagatttcaa aagaagtggc 79680
ttttatatat tattagaaat tttctatgcc tttcaaaagc atagatttta gcaagataga 79740
ttgttagacg actaaatttt caattatctg atacaagcct aaacagtaca tttaacgtat 79800
attttttatg tgaaaacaat attttagatg aaatatgcac tttttaaaaa taattattga 79860
ttttgaaagc cttgagcatt ttagttatta ctgtgctgta tttgcatggt tatattatca 79920
atgagcattc aaaaacttct gatatccaaa attgagcatt ccttgtgaat tcagcttgtg 79980
gcatcattgc tatgtttctt ggcctcatta catcttgaac attatctctc aaaattttag 80040
aatttggtta acagttatct gaaactatga aattgttagt cttctattat ggactgtgaa 80100
tattatgttc tgctttgttt tacaaagtat aataattttt aaagtcttaa tacgttttgt 80160
gttatctggt tgtcccttct agaaattata taaaagagaa gttcaacctc ctttcaaacc 80220
tgcttctgga aaaccagatg atactttttg ttttgatcct gaatttactg caaaaacacc 80280
taaaggtaat gcatgtttat ctgtaaaatc agagatttta tttaagtaga tagtttaaaa 80340
ataaactttc ttgtgaagta aaaactaaaa gaatggtaaa tttgcagaga gttgatttgt 80400
ccatgggaaa tatggatcct atacaaaaca ttgcttgggc agctgtcaag tagatgtttt 80460
aaatgggaaa tatttatcag gaaggaagga acttggtcaa agggtggcaa gtttatttct 80520
ttgttttata agtcctttgg catgctaatg atttacagga tggggtagga ggtaagacgt 80580
gagatatatt actctgtctt ttacagtttc atacatacac tcaagaaata ttgaatacct 80640
actatgtact aagcaatgag gatgcagtgg tgaaaagaac agacaaaaaa tactgactaa 80700
aatggagcat gtatacagat gggaggagac agataatata taataaatca aatatataat 80760
atgttagtag catttaatgg tttcctttat agttaaagaa attctgacac tttatatcat 80820
agaattctgt gtgtagttat aattaaatgt aattatttgg acattagatt ctcccggttt 80880
gccagccagt gcaaatgctc atcagctctt caaaggattc agctttgttg caacttctat 80940
tgcagaagaa tataaaatca ctcctatcac aagtgcaaat gtattaccaa ttgttcaggt 81000
aaattccact taatcacatt attcagtttt gggataatta tttctctgtt attgctgatg 81060
tatttttaat cgtttttttt aaagtgattt ttaacttctt tgtttatcta ttggtataaa 81120
tcatgaatta aatatgtccc tgtgtgtttt ggaaaagagc agacagtggt aacttttaat 81180

tatatttatg tggcctcttt tgaataaaat agaagggtga tgtataagct agttgcttta 81240
ttatgttgcc tgtgggtatc aagaaaggat tggttcacgc catttataaa tgtcgataaa 81300
acttctgtag aatgaattat tctagtttaa ggcttaagat gaatgtatat ttaggacaaa 81360
agacagaaaa tgtgaaaata tttcattctc cttagtattt tactttttaa aaataaacag 81420
ataaatggaa atgctgcaca atttggtgaa gtatatgaat tgaaggagga tattggtgtt 81480
ggctcctact ctgtttgcaa gcgatgcata catgcaacta ccaacatgga atttgcagtg 81540
aaggtattgt ctctgagcct agctttcttg tttgttttat gcatgtaatt taaattatga 81600
gtcaactgac aaacattttc agtggagtaa atgaagatag gaaagataag atgatgagaa 81660
agaactctga gacttaatat tttcattttt gaatttcttt tcaagccttt gttatgtagg 81720
aacagtgtat ttcatgaatt tttgcatttg aataattttg ttatacatgt attatgttaa 81780
gattgagcag ttgtgataat tacaatcacg tcatcattgg tacaatttca gtgaaatagg 81840
atataatgtt aaaattttat ttttttctgt taagtttttt acttatgttt ttaaatgtta 81900
aaataatatg aggtaagtat tgttcttgag tttttaagca tttcttcaaa aatataacca 81960
aatggaaaat actcatttta gacaaaataa tttgtttgcc tcagatattt ttcttttttc 82020
gtatttctta gatcattgac aaaagtaagc gagacccttc agaagagatt gaaatattga 82080
tgcgctatgg acaacatccc aacattatta ctttgaagga tgtaggtatt tctaaatcta 82140
ggtatcagct aagctaatga agttatgaat tggaacttta tttttcttat tgaaactttt 82200
aatgtcttta gcatatttat tttcatgcct caaaaatctc aggactctta aattgccact 82260
ttaattcttt tttttcccat gattctatac ttctagtctt atagaatatc aaaaataact 82320
atgatgattg gtaaatgaga atttcctcat tacatgaata tattctggct ggttaactag 82380
ttaacattgt agctaacacc aattattatt ctgtagtgct atcaatacag aagatacagg 82440
agactgggga tctgccaaat tcttgaatat tttactttct tgattgtata gtaattggct 82500
atagcattat ggattgtttc tggagtgaaa tagtcttagt ctatagtttt gtaaaatgaa 82560
tgtataactc attaatatgt tactgaatgc tcacttttca cataaggaga taatgttctt 82620
ttctctttat acaagacaga aaagtcaact atcaccttga tcaatttgtg tcagaaaatg 82680
atatgcactt tgatttgtaa tctttaaact actgtggtgt gtgtgtgtgt gtgtttatca 82740
tacatgccct gagtgaaaag tgtataaata cttttcttat ttgttttcct tggtatacta 82800
ttggaattca accagtattt aagataccaa atcagataat gaactgaggg aagggtataa 82860
aaaataaata atgtatttac tttcagaaag aagcttacgt ttggggaaga gaagtagcta 82920
taatacagta aaaaaattga gatagaggtc atttgagagg cacagagttc tattttttaa 82980
tttctttctt tgatgaactg caacctataa cttcaaaagg caataaaatt atatgtttaa 83040
catggtttca tgggactgtg taggtgactg tttagccaat tcaaccttta gtacagcctg 83100
tactgcatta agagtaaagg aataatgtct tttttgtttc taatactaga gggaagtcta 83160
gtagaaaggt aaatataatt aaaatctaat ttaagaatta tagaattgta attataaata 83220

ataacatttt aaaactgcgt tcttttgtag gtctttgatg atggtagata tgtttacctt 83280
gttacggatt taatgaaagg aggagagtta cttgaccgta ttctcaaaca aaaatgtttc 83340
tcggaacggg aggctagtga tatactatat gtaataagta agacagttga ctatcttcat 83400
tgtcaaggag taagttgtag ttaacttttt tctttctatt ctgggaccta tatcttcatg 83460
aaaatatagg agatgatgcc acctaaaata agttgctttt attcacatat atttctatag 83520
agaatatcct aatagaactc acaatgtagt gaagtttatg gatttaggaa ataattattt 83580
tctctctgcc aattggccct gatttaatag catattcatt taaaaagcgg tttgaattat 83640
ataaatgtac ctaactttga ccttgaaatt atgaaactta gacttaaaag tcaaacataa 83700
aagaattaac caaagacata cagtgactat tattcaagtt acctaacata taaagttcag 83760
attacctaac atgtatcttg aaccttgcca gctccttccc taaatacagt aagaaatgtg 83820
tattctaaac atttcaagta ttactatatt aaattttgtg gtgaaggggc aggcttgtta 83880
ttttgcttcc tgtcctttca taaagaaggc aaatgaactg agttacaaaa aaagggaact 83940
tttaatatat ataactgtat aaaaaaacaa agtagggagt aatttaaaaa tttaagtttg 84000
aatcagttgt aatagtagat atgtaccaac ttaataaaga aatttaaaat ggcaatttag 84060
aattgacata atgaaaactc tgcatgagcc cctcttataa ttagtaaaaa tagtactcaa 84120
ttttgtgtgt ggtttaataa agtgtgtttt cagtattttt taatatataa tgctaaacag 84180
tagcttttta taatttaatt atttgattgt ctttaccaac cctctctaaa ataaagcatg 84240
aagagaattt ttgagaaaag catcataggc gtctcttctt tattattgta tgaaccttca 84300
ggttcttact tcaattaact aaattaataa gacacatatt gagcatttct acaggccagg 84360
cattgtaaaa agtgttgtgg attcaaacat gagtgagacc tggtccttgt ccttaaagag 84420
ctttagcata gtaaactctg attaaagata aggataactt acatgaaata gagagtacta 84480
gtagggaact tatcacatgg gggaatttca gcattttttt ttaccagtcc tacattttat 84540
ttgtttcttc tttattgttt ctatttttct gctgagactt cccttttttt gctgagagtt 84600
taatgcattg aaagcatgtt ttgttttact ttgttgagga tcattatgat agtcacttta 84660
aaatccttat ctgttttagc atggttcatc ttggaatcag aatttattga ttttcttaag 84720
actgtgttct attttcttgg ttctttgtat aacagataat ttgggattgt atcctaagca 84780
ttataataat cagttataga gaatttcata tttcattatt tttctccaat agttattatt 84840
gtttcatttg tttaagtggg taattttctt ggctgggctt gaactgaaaa ctagttttgg 84900
ggccagcagc tctggttttt gttcatatta tttgtcttta tacaaattat tttgatcctg 84960
ttccacacat ttttgataca aggatcagtt ctggctcttt cctttataat attcctccac 85020
tcagcattca gagttttcca gcatctcctt tctggtcctc taagccagaa ggtctgaggg 85080
tttttccaca tgtctttacc accactgtgt accataggga ttgcacttaa acccaggcta 85140
aaagccacag atatggaaac ctccttgtat ggtttcctgt gttccctcct tcaacaaagt 85200
gtgtactcct catcagagta tatctgcttc tgttcattct ccagtgcctt cagatagttg 85260

atttttgtat tatatccagg ttttatggtt gtttacttca aggagggggg tgttgagtag 85320
ggtcttagtc cattatggac taagtctaaa cttagtttag tttgtaagtc aggaactgtt 85380
cttatatgtt atgtataatg ctaagtgata taaattatgg catgtccaaa agtggatctg 85440
agaattgagc ttagatatat tgagattaga aatggttaac aaaactagtg gcttttgaga 85500
caatagaaac tggaacatct atctgacaga cataatttcc tgtgtatatt ttaggctatt 85560
aagcttgagc aaaatgtcat tttcaaaatt ttgtaatttg gaaagtttct ttttctcaga 85620
tatttagtta attatataga tataatgttt tatttctcag atatttagtt aattatatag 85680
atataatgct tttttccttt atattcaagg ttgttcatcg tgatcttaaa cctagtaata 85740
ttttatacat ggatgaatca gccagtgcag attcaatcag gatatgtgat tttgggtttg 85800
caaaacaact tcgaggagaa aatggacttc tcttaactcc atgctacact gcaaactttg 85860
ttgcacctga ggtattcctt aaaaacttca ccatttgcct ttcattcttt tcctttagtt 85920
gaatctatat gatgccatat gaatgttatg gtaggcagat aaaagctgtt accttgccct 85980
tttcagtttt aaagttcttt aaatcaggga attctcatag cccctaatat aaatatttac 86040
actggtaact agcatctaaa accaaccaga atgtaataat caggctattc tattctgttg 86100
ggttatatac taatttcaga atcatatcat tttagagttg ggaggtaggg aaaagatagc 86160
cacagcttca atatattatg tattctaaat gatgaaactg gctcgaggta cttcaatgac 86220
ttattcaaga cccagcattc acttaataac aaagctagga tagattcatc aggtatgtta 86280
aattgtactg aacattaggt aatttatata gattagcatt ataaataaca tataagggca 86340
gtgctatcct cgattgtctt taggatcttc taactcccgg agaaaatagc cacacagggg 86400
tttttgtaag ccgtggggat ttttcttgga aaataatatt tatatctaaa tattagggtg 86460
aagaatcagg aaaggtgatg ctcttaggga gaagagtcag gaaaggggag gctgtggtac 86520
agatttcaca tttgggaagc tgtaacattc attctaacca tggggatgag agctttgaca 86580
gtagcaattt ccatagcagt gaaagtgaag ttggggaaaa atgaatcaag agatggcatt 86640
ggccatataa gttctttata agctttcttt gtcaagtcag tagctgttct tatatgttat 86700
ggataatact gacatgtaat tacatattca atgaaaatca aaatacctaa aactcttcag 86760
atttgaaaaa attgatgtgt caatcaaagg agaagcagag taaaataatt tgagaagaat 86820
tcctatcttc tatatatttt atgattttat ttttatatca aatactagct ttttcaggga 86880
aatattgcac atcagatata ttaacaataa gccacttgta ttttaatgta aatcagtctt 86940
tattttaaag tagctactat aaaaataact ttaataatga aaatagaaat atagaaaata 87000
ctatactgat attgaagaac aggcagaaca tctgttttat aagcttttta tgataagaga 87060
tttgactaag aaattatgta tgtatccgga gaatgtgatt taatctatat tttaataaaa 87120
ttcataaaat aatgtacaag tttttatctg ttacaacata ttgtactgcc ctaaggtgta 87180
tgagataata gcctgcacat aaaaaaatta gcctttttag gacatttcag tggttttata 87240
ggaggacaga atatttgaga tagtgctctt ctgttatgta tagcatatat ggtcatcata 87300

aaggtcctca taaatattct aacagattta tacagtagac ataagagcag agtatagaat 87360
aaccatttta atagccaaat atttaatatt gacttaatct tgtggaccta aactacttag 87420
tattatggca acttctcact ggaaaaatgt taaggtgaat atgtaccata tgaacatatc 87480
aacaacagac attaaaatga aaaatatata atatgtccgt gtttagatta atggttaggt 87540
aaacagtttt agattaatgg ttaggtaatc aaacccagag agtagaatac agcccattga 87600
tgtttttttc ccaacagacg tacactgtag taagacactt ttaccattgt aatagtgaag 87660
gttttcaggt gggcatctgg agaccacttc tctctaacta gtgcttgaac tacctttgac 87720
tagtatttgt attttcaaaa tactttgaaa gttttatttt tgagtgatga agcccatgct 87780
aggaatcaat aatatatttg taaattgtta ttaatgaacc acagagagta tttttagtga 87840
ggtttgtttc tgaaatttca gacgctttca aaaggtatta gtaaaatata aaaatgcatt 87900
cttttttaaa ctagaattgg aaagattcaa atatcgatac agacatttca ataaaaaatg 87960
gcatttatgg gggtatacat tcattatttc acaatgcata aagtttttgc taagagctac 88020
actgattatt gcaattttag cttaacaata aggagaaaat attcttttag tttttagatt 88080
atgcatcagt ttgatcaacc tcttggggtt tattaggtag atgtgctttt ctactatacc 88140
tatgttgcaa aaagtgaata tatctctaaa atattttatt cttcgataaa ggcaaatcca 88200
ctacgttata ttattcaact gttattataa gcctactgag gttaaagaag ttcctgaatt 88260
aatggggctt acaaaaaagt tgagggatac tgtgtctgta gctttttata aactaatttt 88320
gaagttaact ctcctttgct ctttctctgg gctgtcatat agtttgcccc aaacgttaat 88380
tgatttgagc aacaggaatg aggagctctg tgtatgttac tttctgctga cttgggaagc 88440
caaatgagag agagacttat gccactgact actttaattg tgacttctat ctttgtattt 88500
aacttgatac aagtaaaaag gtgtgaaaca taacctctgt actctttcat tcatcctacc 88560
gctctgcctt gtttctttta tgattatatg ctcactaggg cattgagact agctctagaa 88620
aaaaagtgga ctgtttcttc tttacctaaa acaagttttt catgtttggc cctaattaag 88680
caatatgaca cctcctgcgc aagtctgtcc tcataactag gaaggttata ggagtatatg 88740
gtacctacag ctgacttgat taattctcac ctgtccaagt agtttctgtg cagtggccct 88800
caaccttagc tatgcattgg atcacttggg aaactttaaa aagcttccta agggaatcag 88860
ggaaaacatg gtgatgattg gaactgtaag atgtgatgag aaaaatgaag agataaggga 88920
ataaaatcac tagaagtcta tgttttacta gagtatgcca agttaacatt taccatgata 88980
tgagtggttt agctatacct tgttatttgt taagtacttt atagttccct acttgtatta 89040
tttaaataag ccaattatgc ttttatatcc tctttaccct cttaattttg cacatatgtg 89100
tgtttttatg aatacactaa gactaggaaa aaagcatatt tcaaatttta agtttgttat 89160
atactcttta ctcatgtttg gcagttgtca aagccaaaca ctaacatttt tagcctttta 89220
tagatgagaa attcatgctg gtggttatat aatccccagt gttaatgcat taataatgtt 89280
tctattttgt tcctatgcct agaggcttta gcaatgggct tgtttttcta aatggaaaga 89340

aaatggcctc atttcatctc tttatttttc taacgaactc tattaacttc gtagctaaat 89400
ctcacatgaa tacatagcaa atgtattatt tgatttttat taacactggc cccaaaaaac 89460
agcttcctaa attattgtat gaaaaataat gaagccattc agtatttttc aaatgtgtac 89520
ttgtggtcca gaaatatact gctgttttca agacattgac cttgtatggt aaaatataaa 89580
ttcattctgc ttttgtgaga ttatatatta tcttgccata tcttgtgtag taaagaggca 89640
gttatcatag atattaaaaa cacaggctta aataagactt cgtgttctac tacttactag 89700
ttttgtaatg ttgggtaagt tacttaacat tcacttcagt ttcctcatct tcaaaattgg 89760
aatattgtct acctcacaga atcagtgaca gaattaaaag ccttagtact gtatatagta 89820
tacattaaaa tctctgtaca tgttaatctt aagtttagat ttctctatat ctggtttcct 89880
aagaggagga gagaaaactc attgaggtgt caggggttat gtctttacta caagtgcata 89940
aaatgattga agtttttata ttatgaactt ttgtgagatt attagaagta aaacaacttc 90000
tttgggagaa aattgagttt atattgagta acaccatata atgagtattt ctttcttagg 90060
ttcttatgca acagggatat gatgctgctt gtgatatctg gagtttagga gtcctttttt 90120
acacaatgtt ggctgggtaa gaaacatata ttcaaaagca ttaacattgt tatttgaatt 90180
tcatctgtat atcttcatct agaaagagaa tagattaaaa taactttaag gaaaatgaca 90240
tttacatttc aaaatattaa tattgattgt tacacataaa attaaaaaac ataaagcaag 90300
gtgtagaatg aggcactact aatttatgac ataataattt taaaaggcag ttgtcaccca 90360
gaactaaaac ttttaaaaat aaagtgcatt tctattttaa tcttgaaaat atttatttaa 90420
tcataaaata attgcatgtt tcaccaacaa cttgcccact gaacttgttg atattgagtt 90480
gatttttttt aagcacttga attctaacta ttctggcact agagatttag taagattgtg 90540
caaatctaag tagtaacaat actaaactgc agaaacacac acaactacct tcccttgaaa 90600
ggataaagaa aagctcaaaa caaatgtaat aacaattgga ctatgttaat gttgatactt 90660
tacaaataat acaagtacta ttttactctc aataatgagt agcactttta tttagaagtg 90720
aaaacttatg gatgtagaaa ttattccaag agggttgtga tagagaaaat ggccaagttt 90780
agcaagtaat tataatgtac ttagacatat attttaaaga gtgcatttag tatagtttaa 90840
atatgcttga cataccaaga gctctataaa tatgttataa aaatggtgac tctaaattgt 90900
ttaatggctt ggtgttaatt acacatctca gtacatttca catactttta ggtattacaa 90960
atttatgcaa aatatttagt tatagacaac agtaaatact gaagttaagg gtagctaaag 91020
tttgcatgtc aagcacaatt aatctacatg attgatctac ataataaatg taatggctat 91080
ggagttttgt gtcgatatac atatttaacc aaccaagttt tcatagatat taataaaaag 91140
taatgataac cgtttccact gatgataatg atgaaatgtt aatgaataaa ttgaaacaag 91200
acaataaata cattatgaat atatgaaatt aacatttctt ttttactagg tatatttgtt 91260
tgctaaccta tgccttgaat atgtgtgttt tagtgttgtc tagttttaag actgaagctt 91320
tacatgaaaa gccaccttta ttttatttta cattgattag ctaatcagag acaaaagaaa 91380

taattgatgt aaacaagatt ctccatttta ttaaacattt tattaaggtt aaatttatgg 91440
tttgaaatat aaaatttgac tttaacagtg aaaggaaata tgagatgata ttcttattca 91500
ctgaagtaaa tatttgcatg tagtaaatat atgtatctct ttcattgttt gggtatgtta 91560
ccctctagtg tctattaaaa tctattttat taatagctac actccatttg ctaatggccc 91620
caatgatact cctgaagaga tactgctgcg tataggcaat ggaaaattct ctttgagtgg 91680
tggaaactgg gacaatattt cagacggagc aaaggtataa attataatgt tttgttttgt 91740
tgcattcatg ttaaattgct caaatcccta agttatagct tagtgtgtgt ttgcagagta 91800
tctaatggtg tcatatgtaa gtgttttgaa ttaacacatc tatgagtgaa ttgaacagtg 91860
tacaaaggat aacttttggt tttaccatgt ttctgggcca ccttcctttc tgatcaattt 91920
acatatgtgc agggaatatt aactagttcc tgtattaacc aaaaccaaaa atgtaaattt 91980
tggaaacagg gtatatttaa agccaactaa agtttatttc tagatttacc atgtatttcc 92040
atatcatttt cttttagctc agaaattgac ttgattatgt agacaatttt tataatgcta 92100
acttattctt agagaatgaa tggactatgt atctgtggca cctggatatt cattcctgat 92160
ggcttagagt ggtccagtag aaatataatg caaggcaaat acactttaaa aatttctagt 92220
agccacatta aattttttaa gtgaaattaa atttaataat atgtgttgtt taatctaata 92280
tatccaaaat actattcaat ataaaaattt agatattctt tactttttct tactgagtct 92340
tcaaaatgta ctatatattt tatacttaaa gccgtgtgaa tatggactac ccacatttta 92400
atacttaata gccacatgtg gccagtggct gccatattag tgaagtttca gcataactgg 92460
ccttatttat ttgggggaaa aaatagtcaa cccaggcttg aaaactaatt tcttacattt 92520
ataattttta aattttaagt tttaaatgaa aactatcact tgtcaccatg tggcttttag 92580
gtcccagaag cttttaagct acaatttgaa gtagaacaca ttttatgaca aaagtaattg 92640
cttatagtta acatgtgcta attatctcaa gaacactagc cagtttgtag aatacattgc 92700
aaagtaactc cagcatcctg attattgtca ggataaataa actggggatt tgtccctaac 92760
ttttaaacat tagaaaaaca gacaagggat tgttcttgga tcttaacctt agtatgagta 92820
tgctgtgtgc tatagtagaa ggaacacttg aatggaagcc aggaaaactg agcgctaatc 92880
tttctttggt tccttataac tccaaaattc atttctatgc aatttggttt atttgtaatt 92940
tgcaaaattt ccccatgtaa tgtgattaga actactagta ttattcatta aatatgtatt 93000
ttctagtcat ccagtttatg cttcagaaaa tacattttac caaggatcaa ttttaatgtt 93060
taaagccatg catatgaatc tcctgtgttc cttttaaaaa tatctgctga gatcaggctg 93120
ttgacagttc ctggttggct cacagttacc catgtcagtt atctccgtta acatttccaa 93180
gaatctttgt agaacaagtt ctccacttgc aaggtctttt atttatttat gtatttattt 93240
atttatttat ttttattttt tttgagacgg agtcttgctc tgttgcccag gctggagtgc 93300
agtggcgcga tctcggctca ctgcaagctc tgcctcctgg gttcacgcca ttctcctgcc 93360
tcagcctccc aagtagctgg gactacaggt gcccgccacc acgcctggct aatgtttttt 93420

atttttttta gtacagatgg ggtttcatcg tgtttgcaag gatggtctcg atctcctgac 93480
ctcgtgatcc gcctgcctcg gcctcccaaa gtgctaggat tacaggcgtg agccaccaca 93540
ccaggcctcc acttgcaaaa tctttaaagt agaactcttt tttttttttt tttttttttc 93600
cctttaaggc agttagccca ttgccaaaag gtttactgac ttaaggctgg aaatgtcttt 93660
ctgagctata attccaagga cttctccaca gctaagtgag atgcctcata ccagtattag 93720
gtgattcttt gtgtggacag aacagagcat tttcatcttg tgtttaaagc aatttgttgg 93780
cttcagctcc tcaccacttt ctataccagt ctcctattac taacccagta atccctatgc 93840
aaaacaaaca aacaaacaaa caaacagaaa aagaaaacgc tgatggtgat tcctgtaggt 93900
ttaagggctt aaatctcaaa ctttgtgtta ggagtaacgg gagtgtgctg aaagggcaag 93960
caatacaaca agtcatacca aaaagccaca ttgttctctc ctaagcccca accccactcc 94020
actcctgtgg ccagtggtcc aaacagaaaa taactggaga aattgaggag gtcaaaggat 94080
tagggaacta agcgttatgt gaattcacta gcaagatgta cagaatgctt gtgtttacat 94140
tgttttttat ggaattagca gaataaaact gatctatttt aaaaatgcaa aaaaaaatct 94200
gctgaaatag atggggactt aacattgcca attgtgctca aattttcagt tttctgtgtt 94260
ccatgtattt tatgtttagg cctatctggt gtttaagtaa taatacatat taaagcttgg 94320
ttttattctt acgtctcttt tagcctggta atctggaaaa cttctcatag cataagatat 94380
tagggatagt gttgacatat ctcataaggt ctcttagata atcccttttt atggatcggg 94440
tgactttagt tctgtctttg aaacttttgt aggagctatg agaacctttt aagaaactat 94500
ctcatgtacc ttttaacagt tctactgctt acatttgttt taaactaggc atgaaagtaa 94560
acttgttcag tttcccataa cctcttaaac tagtacactt tagttgtgag aactgtgaca 94620
tttgctgccc atcagacata gattatttat tgcctcttca tttgtctctt ttaaaaagat 94680
taacttgtac ccagtgaaca atctctcacc atctcctcac ccaccactcc ccatcctctg 94740
gtaaccacta ttctactgtc tatttctatg agaacaatgt tttagattcc acatataagt 94800
gacatcatgt gatatttgtc tttctgtgcc tggctgtttc acttaacaca atgtcctcca 94860
ggttaatcca cgttgccaca aatgatagga tttcattctt ctttatggct gaatggtgtt 94920
tcattgtgta tatacacact atattttctt tatcaattca tatgttgata gacacttagg 94980
ttgattccat atcttggcta ttgtgaataa tgcttcagtg aacctgagca tacagatagc 95040
tctcctacat actgattcca tttcctttgg atatatatcc agcagtggga ttgctggata 95100
atatggtaga attttttgag gaaccactat actgttttcc ataatggctg tgctaatttg 95160
cattctcacc agcagtgtgt aagggttccc atttctccac atctttgtca acacttattt 95220
ttttattttt ttgatagtag ccactctgat tggagtgagg tgatgtctca ttgcattttt 95280
tttttttgat acggagtctc actctgtcgc caggctgcag tgcagtggcg tgacctcggc 95340
tcactgcaac ctctgcctcc caggttcaag cgattctccc acctcagcct cccgagtagc 95400
tggcattaca ggcaggtgcc accacaccca gctaattttt gtatattcag tagagatgat 95460

ctttcatcat gttggccagg atggtctcga tctcttgacc tcgtgatctg cccacctcag 95520
cctcccaaag tgctaggatt acacgcttga gccatggtgc ccagccctca ctgcagtttt 95580
gatttccatt ttccagatgg ttagttattt tgagcacctg ttcatgtagc acctggtgct 95640
ttattttgtc tatttggtga ggtcatggtt ccctgaatat tttttttttg aaaagtataa 95700
tttttttata attttaagtt ctgggaaaca tgtgcaggat gtgcagattt gttacatagg 95760
taaacgtgtg ccatggtggt ttgctgcacc tatcaaccca tcacctaggt aataagccct 95820
gcatgcatta gctatttatc ctgatgctca ctctcctcct gcccctcccc caacaggacc 95880
aggtcctgat tgtgtgtggt ttccttccct gtgtccatgt gttctcattg ttcagctccc 95940
acttatgagt gagaacatgg agtgtttggt tttctgtccc tgcgttagtt tgctgaggat 96000
aatagcttcc agctccatcc atgtccctgc aacagacatg atctttttta tggctgcata 96060
gtattccatg gtgtatattt acctcatttt ctttagtcta tcattgatgg gcatatgggt 96120
tgattccatg tctttgctat tgtgaatact gctgcagtga acatacacgt gcatgtatct 96180
tcataataga atgatttata ttcctttggg tatataccct gtaatgggat tgctgggtca 96240
aatggtattt atggttctag atcctttagg aatcaccaca ctgtcttcta caatggtcga 96300
actaatttat gttcccatca acagtgtaaa agcattcctg tttctccaca gcctcaccag 96360
catctgttgt ttcttctact tttcctctac tgttttccca ttactatttg gcatgtactt 96420
tattagagca tgtactacat tctgcctggt catatattca cttgggtata tttttgttct 96480
tcacacttag gaagccttaa ataacatact tattttgcat tttctatcac atctagcata 96540
atgagtactc aattggatac tttttcataa gctaatcatt tcttctggta tatcttgcat 96600
tgttaattta cacagcaagt gtttttgata cattttatac acatgttgtc ctattgtgag 96660
tgactataat ttgcagtaag cagtgagatt ttacataaat atttattttt cattttggct 96720
tgaagtcgct tttaatagaa gatgcttttt tcatgtgttt acaggttata gatttataga 96780
gctagaaggc aagtagtccg tcacctcatc attttgcaga taaataaacc aaggcatata 96840
gaatttaact tacacattaa tgagtggcag agttggaact gattttcagt ttagtactct 96900
tttcaatcca ctcacattag ggaaaacttc ctagaggaaa aaaggattta ttaccttgtt 96960
tcatgtagat taaatggagg aatgagaggt ctgtaaccct ttaacaattg aaggctcgaa 97020
ggaaagctat tattgttgac ccactttgtt tctgtagtaa tggacatgag gtatacagaa 97080
ccttattacc tgatatgttt tggctgtgtc caaactcaaa atctcatctt gaattgtaat 97140
ccccataatc cccatgtgtc aagggagaca acagatggag gtaactgaat catgggggca 97200
atttccccca tgttgttcgc gtgatagtga atgagttatc atgagatctg atagttttat 97260
aagtgtttgg tagttccccc gcgtccattc cccttcctga cttccttgct tgcgctcctc 97320
cttctaccgt gattgtaagt ttcctgagac tgtcccagcc atgcaaaact gtgagtcaat 97380
taaacctctt tatacatttc ccagtcttgg gtctgtcttc atagcagtgt gagaatggac 97440
taatacatta tcctttacat gatctgtact acagagcagt tttacagtta aaataatata 97500

ttgaagttca tctagaactc atagtagtac cacagttttc aaatgcctca tgccaaatta 97560
tactagtgaa agactacact atctaagcca agctaggaag gtacattagc aaatgcaata 97620
atcagaaatc aacccttgtt ttttctgaaa gataattatt tattattatt tgtgatcaga 97680
gcaggtgttg ctgaagtata tatggaagtt attcatttat actgaggata tatgaattgt 97740
tacacatttg accaattaaa catgcttaca gaatgagtaa taaaaaagtt ttacctcaga 97800
tacatgatta cattgaaaat agaagtgatg aattattaat cagtattttc caaactttaa 97860
tttggaatta aatttgagta ctgtgagaat aggaaaattg ctatgctcct atattcatgt 97920
atgttcaaag tatttagagt tttgctggct tttatccatt taaaatggac attaaaaata 97980
agaaacctga gtacattgag aggggccaag atggccaact ataagtaggt gcagtccaca 98040
gctttcatgg agagcagtga aaatggtgtg ttaattcagc accttcaact gaaatataca 98100
gtttcgtgta ttgggactga ctaggcagac aactcgaccc atggaggaga aagaatagca 98160
gggtggggtg atgacacacc tcagagtggc acggagacaa aggaaccccc agccaaggca 98220
agtggtgagc gattgtgtga ccccacctag agaaccgtgc ttctcccatg gatctttgca 98280
acccatggat cagtagattc cctcatgagc ccatgccacc agggcctttg gttcaatata 98340
cagagctgtg aggagtctca gtggggcagc tgctgaggca catacagaga cccaagaccc 98400
aagagttttg catactctgg ctccaggaat tccagcaagg cagaagaccc atctatactt 98460
ctatcctagg aagggacctg aatccaggga gccaggcagc atcattctgt gggccccact 98520
tccatggcac ctcacaagtt aagacccact ggcttggaat tccagccaac cagcagcaac 98580
aggctggaaa tggtctgaga tggaccaagt tccaggggga ggggtgacca ccaactctct 98640
ggtttggttg ggtcagtcat tctaacctgc cagctccgag gagtccaggc agtccagacg 98700
aggaggagtc cctgatgaag cagcacagca gctgtgccag attgtggcca gactgcttct 98760
ttgagtggga ccccaatcca tctctcctca ctggacagat cctttctgga ggacttcagc 98820
acttcagcca gggttatata gagagaaatt tgatgtttcc ctaggatgga gcccctggtg 98880
ggaggagtgg caccagttac tgcagttcag tcaacagcca ttctagcctg cctgctctgg 98940
gaagtccagg cagtccagag gaggaagagt cccccacaac ccagcacagc tgctgtgcca 99000
gatcatagcc atactgcttc tataactggg accctgatct atccctgttc actgggcggg 99060
gcctccctat gggaatttca gccactccag ccaaggttat tgggacagaa ctctgatttc 99120
tccctgaggt agggcccccg gggaaggggt agccaacatc tctgcaggtt gttcgactca 99180
gtctttccag cctgctgtct ctggggaggc caagcagtca ggacaaggaa gggttccccc 99240
aatgcagcac acctgctcta ccaaaaagca gccagactgt ttcttaaagt gggatcctga 99300
tcccatccct cctgactggg tgagacctcc caacagggat ctccagacac ctcctacagg 99360
agcatacagg tcagcaacag gtcagtatcc ccctgggaca gaactcccag agtaaggagc 99420
agattgtcat ctttgctatt tcacagcctt caccagtgat acttccaggt aagggaaaat 99480
caatgaaagc tagggtgtgg agcagtcccc cagcaaacca cagcagctct atggaaaagt 99540

ggcttgactt ttaaaagaac aacaaacaga aaacaacaca aaacaacagc accaacgaaa 99600
aagacaccac aaaaacctca ctcaaaggtc agcaacctca aagatcaaag gtaggtaagc 99660
tcacaaagat gagaaataat gcaaaaatgc tgaaaactca aaaaggcaga ggacctcttc 99720
cccttcaaat gactgagatg gctgaattga cagaagtggg cttcagaagg tgggtaataa 99780
caggctttgt tgagctacag gagcatgttg taacccaatg caaagaagct atgaatcatg 99840
ataacacaat agctggtaac aggagctgac aactagaata gccagtttag agaggagcat 99900
aagtgacctg atggagctga aaaacacaag aacttcacaa cgtaatcaca agtatcagta 99960
gcagaataga ttaagcagag gaaaaaatct cagagcttga agactatctt tctgaaataa 100020
gacaggcaaa caagaataga gaaaaaagaa tgaaaacaaa caaaacttca gagaaatatg 100080
ggactatgta aaaagaccaa gcctccaact gattggggta ccggaaagag atggggagaa 100140
tggaaccaag ttggaaaaca cacttcagga tattatccag gagaacttcc ccaacctagc 100200
aagaaagacc aacattcaaa ttcaagaaat ccagagaacc ccagtaagat actccaccag 100260
aagatcaacc acaaggtaca taatcatcag attctccaag gttgaaatga aagaaaaaat 100320
gttaagggta gccagagaga aaaggccagg tcacctacaa aaggaaactc attagactaa 100380
cagctgacct ctctgtggaa accctacaag ccaaaagagc ttgggggcca atattttaac 100440
attcttaaag aaaagaattt ccaacccaga atttcatacc tgggcaaaat aaacttcaca 100500
agcgaaggag aaataaggtc tctttcagat gagtaaatgc ttagggaatt cttcacgacc 100560
aggcctgcct tgcaagagct cccgaaggaa gcactaaata tgaaaaggaa aaactggtac 100620
cagccactac aaaaacacac tgaagtacac agaccagtta cattatgaaa caaccacata 100680
aacaagtatg caaaataacc aactagcatc ataatgacaa gaccaaattc acacataaca 100740
atattaacct taaatgtaaa taggctaaat tcctcaatta aaagacacag aatgccaagc 100800
tggataaaca gtcaagaccc aatggtatgc agtctttaag agacccatct cacctgcgaa 100860
gacacacaga gacgcagaat aaaaggatgg aggaaaattt accaagcaaa tagaaaacag 100920
aaaaaagcag gggttgcaat cctagtttct gacaaaatac actttaaaaa atcaaaagac 100980
aaagaagggc attacatgat ggtaaagggc tcaagtcaat aaggagaact aactatcata 101040
aatatatatg cacccaataa aggagcatcc agattcataa agcaagttat tgagagactt 101100
ataaagagac ttagactccc acacaataat agtgggcgac tttagaactc ctgtgacaat 101160
attatacaga ttatcaagac agacaattaa caaaaacctt caggacttga agatttttgt 101220
atcaagagga cctgataggt atctttggaa ctctccacca aaaatcaaca gaatatgcat 101280
tcttctcatt gctaggcggc atttacttta aaattgatca cgcaattgga aataaaacac 101340
tcctcagcaa atgcaaaaaa aaagaaaaac tgaaatcata acaaacagtc ctctgaccac 101400
agcacaatca aattagaact caagattaag atatttactc aaaaccacac aactacacgg 101460
aaattgaaga aactactcct gaatgactcc tggataaata atgaaattaa ggcagaaatc 101520
aagaagttct ttgaaactaa tgaaaacaaa gagacaatgt accagaatct ctgagactca 101580

gctaaaacag tgttgaggga aatttatagc acaaaatgtc cacatcaaaa agttagaaag 101640
atctctaatt aacaacctaa catcacaaca gaaagaacta gagaaccaag ggcaaacaaa 101700
cacaaagcta tgagaataca ggaaataacc aaaatcagag ctgaactgaa ggatatagag 101760
acacaaaaaa ccctcaaaaa gtcaacaaaa ccaggggctg atttttaaaa aacaaaatta 101820
ataaaataga caactagcta gacagaagag agaagaatca aatagacaat agacacaatc 101880
agaaattata agggggatat caccactgat cccacaaaaa taaaaacaac caccagagaa 101940
taaacacctc tatgcacata aactagaata tctagaataa tggataaatt cctggaacat 102000
gcaccctcct gtgacaacca ggaagaaatt gaatccctga atagaccaat tgtgagttat 102060
gaaattgagg cagtaatagc ctaccaacca aaaaagccca ggaccagatg gattcacagc 102120
tgaattctac cagaggtaca aagaagagct ggtaccattc cttctgaaaa tattccaaaa 102180
aatcgaaaag gagggactcc tccctaactc attttatgat gctagattca tcctgatatc 102240
aaaacctgac agagatgtaa caacaacaaa taactccagt tcagtatccc tgttgaacat 102300
caacacaaaa atcctcagta aaaactggca aactgaattc agcagcacat caaaaagctt 102360
atccaccaca atcaagttgg tttcatcccc aggatgtaag gttggttcaa catatgaaaa 102420
tcaataaatg tgattcatca catagacaga actaaagaca aaaaccacaa ttatctcaat 102480
aatgcagaaa attcaacatc acttcatgtt aaaaatgctc aataaactgg gtactaaagg 102540
agcatgcctc aaaatgttaa gaaaataata tgagaaacct acagccaata tcatactgaa 102600
tgggcaaaag ctggaagaat tccccttgaa aacaggcaca agacaaagat gccctctctc 102660
accactccta ttcagcatat aattggaatt tctggccagg gcaatcaggc aagagaacaa 102720
aataaagggt attcaaatag agaagtcaaa ttttctttct ttgcagatga cttgatctta 102780
tatctagaaa accccattgt ctcagcccaa aagcttctta agctgatgca acttcagcaa 102840
atctcaggat acaaaatcaa tttgcaaaga ttgctagcat ttctatgccc caacaacagg 102900
caagcggaga ggcaaatcat aaatgaactc ccattcacaa ttgctacaaa aagaataaaa 102960
cacctacgaa tacagctaac aagggaagtg aaggacctct tcaaggagaa ctacaaacca 103020
ctgctcatgg aaattaggac acaaacaaat ggaaaaacgt cccatgctca ttgttaggaa 103080
gaatcgatat catgaaagtg gccatactga ccaaagcaat ttatagatta aagggtattc 103140
ccactaaact accattgaca ttcttcacag aattagaaga aactatttta aaatttatat 103200
agaaccaaaa aatagcccaa atatctaaga caatcctaag caaaaagtac aaagctggag 103260
gcatcacact acttgacttc aaactatact acaaggctac agtaaccaaa acagcttgat 103320
acttgtacga aaacagacac atagaccaat ggaacagaat agaaactcag aaataagact 103380
acacatctac aaccatctga tctttgacaa acctgagaaa aacaagcaat ggggaaagga 103440
ttccttattt aataaagggt gctggggaac tggctagcca tatgcagaaa attgaaactg 103500
gaccccttcc atacacctta tacaaaaatt aactctagat ggattaaaga cttaaatgtt 103560
aaacccaaaa ctataaacac cctagaagaa aatctaggca ataccattca ggacatagaa 103620

acaggcaaag atttcaatga caaaaatgac aaaagcagtt gcaacaaaag caaacaattg 103680
acaaatggga tctaattaaa ctaaagagct tcttcacagc aaaagaaact atcatcagag 103740
tgaacagaga gactacagag tgggagaaaa atttttcaat ctatccatct gacaaaggtc 103800
tagtatttac aatctgcaag gaacttaaac aaatttacaa gaaaacattc aaaagttgtc 103860
aaaggagaag aacagacact tctcaaaaga agacatttat gtagccagaa atataggtaa 103920
aaaagctcaa catcactgat cactagagaa atgcaaatca aaaccacaat gagataccat 103980
ttcacaccag ttagaatggc aattattaaa aagtcaagaa acaacaaatg ctggcaaggc 104040
tgtgcataaa taggaacact tttacattgt tggttggaat gtaaatttgt tcaaccatta 104100
tggaagacag cgtggtgttt cctcagtgaa ctagaatgag aaataccatt tgacccagca 104160
atcccattac tgggtatata cccaagggaa tgtaaataat tctcttataa agatacatgc 104220
gtacatatgt tcactgcagc actatttata atagcaaagt catggaatca acccacatgc 104280
ccatcaatga tagactggat aaagaaaatg tggtacatat ataccatgga atactatgca 104340
gccatgaaaa ggatgttctt tgcagggaca tggatatagc tggaagccat tatttttagc 104400
aaactaactc aggaacagaa agccagacac tgagtattct cacgtataag ggagctgaac 104460
aatgagaacc catggacaca tgtcggagaa caacacacac tgggccctgt tatggggatc 104520
aggggaggca gagcctcagg ataaatagct aatgcatgct gggcttaata cctaagtgac 104580
gtgttaatct gtgtagcaaa ccaccatggc agacgtttac ctatgtaaca aacctgcgca 104640
tcatgcatgt ttttttcttg taaaattgtt taagttcctt gtagattctg gatattaacc 104700
ctttgtcaga aggatagatt ccaaaaattt tctcccattc tgtaggttgc ctgttcactc 104760
tgatgatagt ttattttgct atgcaaaagc tctttagttt aattagatcc catttgtcag 104820
ttttggcttt tgttgccatt gctttttgtg tttttgtcat gaagtagttg cccatgccta 104880
tgtcctgaat gatattgcct aggttttctt ctagggtttt tatggtttta ggttttacgt 104940
ttaagtcttt aatccatctt gagttaattt ttgtgtaagg tgtgaggaag ggatccagtt 105000
tcagttttct gcatatggct agccagattt tttaatgcca tttattaaat agggaatcct 105060
ttcctctttg cttgttcttg tcaggattgt caaaaaccag atggtcgtag atgtgtggca 105120
ttatgtctga ggcccctgtt ctgtttcgtt ggcctatata tctgttttgg taccagtacc 105180
atgctgtttt ggttactgta gccttgtagt atagtttgaa gtcaggtagc gtgatgcctc 105240
cagctttgtt ctttttgctt aggattgtct tggctataca ggctcttttt tgcttccata 105300
tgcaatttaa agtaattttt tctaattctg tgaagaaagt caatggtagc ttgatgggaa 105360
tagcattgaa tctataaatt actttgggca ctatggccat tttcacggta ttgattcttc 105420
ctatccatga gcatggaatg tttttccatt tgattgtgtc ctctcttatt tccttgagca 105480
gtggtttata gttctccttg aagaggtcct tcacatcgct tgtaagttct attcctatgt 105540
attttattcc ctttgtagca aatgtgaata ggagttcccc cattatttgt ctctcttttt 105600
gtctgttatt ggtgtatagg aatgcttgtg atttttgcac actgattttg tatcctgagg 105660

cgttgctgaa gttgcttatc agcttaagga gatttttggc tgagatgatg gggttttcta 105720
aatatacaat aatgtcatct gcaaacagaa acaatttgac ttcctctcct cctatttgaa 105780
taccctttat ttctttctct tgcctgattg ccctggccat aaattccaat actgtgttga 105840
ataggagtgg tgagagaggg cattattgtc ttgtgccggt tttcaaaggg aatgcttcca 105900
gcttttgccc attcagtatg atattggcta tgggttcctc ataaatagct cttactattt 105960
tgaaatatgt tccatcaata cctagtttat tgagagtttt tagcatgaac gggtgttgaa 106020
ttttgttgaa ggccttttct ggatctgtta agataatcat gtggtttttg tcattggttc 106080
tgtttatgtg atggattaca tttattgatt ggcatatgtt gaaccaacct tacatcccag 106140
ggatgaagct gtctttttcg tggtggataa gctttttgat gtgctgctgg atttggtttg 106200
ccagtattta ttgaggattt ttgcatctgt gttcatctgg gatatgttgt tgtttctctg 106260
gaaggtttgc tctcaggatg atactggcct cataaaaaga ttagggagga gtccctattt 106320
ttctattatt tggaacagtt tcagaaggaa tggcagcagc tcctctttgc atctctggta 106380
gaagttggct gtgaatttgt cttgtcctgg actttttttg gttggtaggc tataattact 106440
gcctcgagtt tagaatttgt tattggtata ttcaggcatt cgacttcttc atggtttcat 106500
cttgggaggg tgtatgtttc caggaatttc tctatttctt ctagattttc tagtttattt 106560
gtgtagagtt gtttatagta ttctctgatg gttgtttgta tttctgtgag atcaatgatt 106620
atatcccctt tgtcattttt tattgtgtct atttgattct tctctctttt cctctttaat 106680
agtgtggcta gcagtctatt ttgttattct tttcaaaaaa ccagctcctg gattcattga 106740
ttttttgaag ggttttcgtg tctctgtctc cttcagttct gctcttagtt atttcttgtc 106800
ttctgctagc ttttgaatct gtttgctctt gtttctctag ttcttttaat tgtgatgtta 106860
gattgtcgat tttagatatt tcctgctttc tcctgtgggc atttagtgct ataaatttcc 106920
ctgtaaacac tgctttagct gtatcccaga gattctggta cattgtgtat ttgttctcat 106980
tcatttcaaa gaacttcttt atttctgcct taatttcatt atttacccag tagccattcg 107040
ggagcacgtt tttccctttc catgtagttg tgtggttttg agtgagttcc ttaatcctga 107100
gttccagttt gattgcactg ttttcctaga gactgtttgt tatgatttcc attcttttgc 107160
atttgctaag gagtgtttta ctttcaatta tgtggtcaat tttagaataa gtgtgatgtg 107220
gtggtgagaa gaatgtatat tctgttaatt tggggtggag agttctgtag atatctatta 107280
ggtccgcttg gtccagagct gagttcaagt cctgaatatc cttgttaatt ttctgtcgtg 107340
ttgatctaat attgacagtg gggtgttaaa gtctcccact attattgtgt gggagtctaa 107400
gtctctttgt aggtttctaa gaacttgctt tatgaatctg ggtgctcctg tattgagtgc 107460
atatatatct aggatggtta gctctttttg ttgcattgat ccctttacca ttccctttac 107520
atctttgatg tcggtgacct tcggatgggg tttttgtctg gacatccttt ttgttgatgt 107580
tgatgctatt cctttctgtt tgttagtttt ccttctaaca ctcaggcccc tctgctgcag 107640
gtctgcctga gtttgctgga ggtccactcc agaccttgtt tgcctgggta tcaccagcag 107700

aggctgcaga acagcaaaga ttgctgcctg ttcctttctc tggaagcttc atcccagagg 107760
ggcacctgcc agatgccagc cagagctctc ctgtatgagg tgttaggggc acctgccaga 107820
tgccagccag agctctcctg tatgaggtgt ttgttgaccc ctgctgagag gtgtctccca 107880
gtctggaggc acgggggcca gggacctact tgaggaggca gtctgtccct tagtagagct 107940
cgagcgctgc gctgggagat ccactgctct cttcagagta ggcaggtagg aacgtttaag 108000
tctgctgaag ctgcatccac agctgcccct tcctccaggt gctctgtccc agggagatgg 108060
agttttatct ataagcccct gactgtggct gctgcctttc tttcagagat gctctgctta 108120
gagaggagga atctagagag gcagtctggc tgcagcagct ttgccaagct gcagtgggct 108180
ctgcccagtt tcactttccg ggtggctttg tttacgctgt caggggaaaa ctgcctactc 108240
aagcctcaat gatggtggac gcccctcccc ccaccaagct caagtgtcca agtttgactt 108300
cagactgctg tgctggcagc gagaatttca agccagtgga tcttagcttg ctgggctctg 108360
tgggggtggg atctgctgag ctagacctct tggcttcctg gcttcagccc cctttccagg 108420
ggagtgaata gttctgtctt gctggcgttc caggcatcac tgaggtatga aaaagaaaaa 108480
aaaaaaaact gcagctagct cagtgtctgc ccaaaaacgg ctgcccagtt ttgtgcttga 108540
aacctagggc cctggtggtt taggcactca agggaatctc ctggtctgtg ggttgcaaag 108600
acggtgggaa aattatagta tctggtctgg aatgcatcgt ttcttatggc tcagtccctc 108660
acggcttctc ttggataggg gagggagttc cctgacccct tgcgcttcct gagtgaggcg 108720
atgccccacc ctccttcagc tcgccctctg tgggctgtac ccactgtcta accagtccca 108780
atgagatcaa ctacgggtac ctcagttgga aatgcagaaa tcacccacct tttgtgttga 108840
tctagctggg agctgcagac tggagctgtt cccattcggc catcttatca gccctcaaga 108900
acttatatgc tagactgttg agttttttta ttttttttta agcaactata gtggggtaga 108960
attgataaca aatgaaatgc atgtatagta tatagtgtta tgacatatat acacacccat 109020
gaaaccatca tgaaaatcaa gataatgaac atatccatta ccactaaaca tttcctcatg 109080
ctgttttgta attccctccc tcctgcccta tatcttcaat ttataatctt ccacccacct 109140
cattctctgg cacacacagt tgtgctgtct gccactacgg gtttgtatat ttttttaaat 109200
gtttataaat taaatcataa gtctgcagtc ttttgatctc ttttatttaa taataatttt 109260
gagatttatc tatgttgttg aagtacagca atagttaatt ctattttatt gctggataat 109320
atttcttttt atggatacat cattgttcat ctgttcacct gttgatggac atttgtgttg 109380
ttttgtgttg ttaaccaata aagctgctat gaatatctat gtgccagtgg ttttatgtgt 109440
gtatatcttt tttttttgta taaataccta tgagcagaat gattgagtca tgtgtttaat 109500
gttttaagaa accactcaat tgttttacaa attatctgag ctattttaca ttttcaccat 109560
tagcatatga aaattttagt tcttccacat ctttggcaac actggatatg atccttttta 109620
aaaaaattag ctattttaaa aggtgtgtga tactatttga aagtagtttt aattcgcatt 109680
tccctagtga ctaatgatgt tcagcatttt tcatgtgttt atttgccatc catttatctt 109740

ttacctttcc ctttttcact gggtttttga gtgttaaatg caagttctct gtcagatcta 109800
tgctttgcaa ttattttctt tttatggatt gtctcttaat tctcttaagt gtctttcaaa 109860
gaagtcattt ctaatttgat aaagattatt ctttttcttt cgtatatcat gtttttgata 109920
ttagagctaa taaacttttg ccttatgcaa gatttcaaat acagaggttt cccaacttat 109980
gatggtttga ctgactgatg attttttgac ttaggataga gtgaaatcaa tatacattca 110040
atagaaactc tactttgaac ctttgaattg ttatcttttc ctgggctagc aatgtgcagt 110100
aaaatactct ttggcaatgc taggtagttg cagccagcca cagctcccag tcagctatgt 110160
gatcatgagg gttaacaatc agtagtctgt atttttcaag gtgtaacccc ttaataaatc 110220
agagcatctg tattttcccc tgtgttttct tttagaagtt gtatcatttt aggttttatc 110280
tttaggtcaa tgttcttttt tgattcaaca tatatatata tggtacaaca tatggtcaaa 110340
gttcattttt acatgtgaat atgcagttgt tccagtacca ctttttgaaa ataatatcct 110400
ttcttcacca aattgcattt gtatttatat caattgtcca aatatccatg gttctgtttc 110460
tacacttcct attctgtttc actgatctac ttgtctgtct ttacactatt accatactat 110520
cttcgttact gttgctttat aattaaattt tgaaatcagg taccatgagt gtttcgactt 110580
ttttcttttt cagagttttt tgagtttttt tgttgttgtt ttgctatttt gggtgctttg 110640
cattgccata tgaattttat aatcagcttc tcaatttcta tcagaaaaag aagcctgcta 110700
ggattttgaa tgagatggtg atggagctat agatcaattt gggaagaatt gacattttaa 110760
cagcattgac ttttcttatc tataaacaca gtatctcttt ccagttaggt ctttactttc 110820
tctcaacaat gttttatacg tttaaatata caggtcttaa acatctttta tcatatcttt 110880
tccccaaata ttttgttttt aatattaatg gcattaaaat tttattttct tttgttgcta 110940
gtatgcagaa atgtgattat attgatcttg tatgttagaa gcttgctaac ctcacttatg 111000
aattctagta gcttttttct agattctatc agtttttcta catagatggt aatagttttt 111060
ataaggacgt tttagtgctt gatttctaat atggatgtct tttttcttaa ttttcaatgg 111120
ctagaacttc cagtatagtg ttgtagaagt ggaaagaatg cacattcttg ttttgttcct 111180
gatcttgagt gtaagcattt atttttcacc attaaggatg atgttagctg tgggttttat 111240
atggatgctt ctaattaggt aaaagttcca ttttgtttct ggtttgatga aaatttgtat 111300
tggagatgaa tgttggattt tgtctaagcc ttttcctatg tctgctgtga taatcacatg 111360
attttcttta ttgttgttac attgatatac atttgaatta cgtttatttt caaatgctaa 111420
gccaaccttc tattcccagg ttaagtacca ctcgatcata atagactata tatagctaaa 111480
tttgatttgc taaaattttc cttacaattt ttgcatctgt gttcatgagg gatgtggatg 111540
tactatttta ttgtaagtat ctttgtctga ttttgatatt ggtgtaatag tggtcttaaa 111600
tgagttgaga aacattccct cctcttcaaa tttctgaaaa aagttattta gaattagaat 111660
ttcttattct taaatgtttg ttgggattca tcagtgaagc catctggacc tacagttttc 111720
tttgttggaa atatttaact ataaattcaa tttcttaaaa aggtatatgg ctatttcggt 111780

tagttgtttc ttcttgagtg agcactggca tttttttatt tcaaagaatt tgtccattgt 111840
caaacacatt ggcacaaggt ttttcttata atattttctc actaactggt taatatctgc 111900
tattaaccac tgtaatctat actgatgtct attttctcat ttcagctatc atggatttgt 111960
gtatttctct ttgcagttat aatggttttt gcttcatgta ttttgacgct ttgtttttac 112020
atgaataaac atttataatt ttcttctctt gattacttat cccctctgtt atgaaatgac 112080
cttctttatg acgtgtaata tttgccatta tttttgaaag atatgttcac acaatataaa 112140
attattggtt gacttttttt cagtatgtta aatatgttgc tccatagtct tcccatttga 112200
attttttttt tctgacagga aatctgttgt tatccttgtt tcacatgtaa catgtccttt 112260
tttctctagc tacttttaag gttttctctg tgtcttgggt tttgagtgat ttgggtttga 112320
tgtgccttga tgaaattttc ttgatttttc ttgtacttgg gttttattga ccttattgta 112380
tctgtgaatt tatggttttc atcaaatttg gacatttggg ggccattatt tatttaaata 112440
ttcttttctg ctcccaattt tcttctggga cttcaactgc atatatatat tagaccatgt 112500
gaagttgtct cacagctcac tcaaactgct cttttgttgt tctatactct cgtgtttctt 112560
tttggaaagt atctattgtg tatatttgat ttagtttcat tatttattat ctcacagttt 112620
ttatctctag aagtttgatt tgaggttgtt tgtttgtttg tttgtttgtt tgttttgcga 112680
cggagtctcg ctctgtctcc caggctggag tgcagtggcg caatcttggc tcactgcaat 112740
ctccgcctcc caggttcaag taattctcct gcctcaacct cctgagtagc taggattcag 112800
gggtgcacca ccacgcccag ctaatttttg tatatttagt agagacgggg tttcacaatg 112860
ttggccaggc tggtctcgag ctcctgacct catgatccac ccgccttggc ctcccaaagt 112920
gctgcgatta caggcgtgag ccaccatgcc cggccttgag tcttttttta tatcttctct 112980
atctctactt aatttctgaa catcaggaat acaattataa taactattta acatccttgt 113040
ctgctaattc tgacatctgt gtcagttctg ggtcagtttt gattgattct tttctttctc 113100
ctcatttttg tgctttttgc ctgcccacta atttttgaga agataccaaa tacggtgaat 113160
tttaccttgg tgggtgctag atacttttaa attcctataa gtattctatt ttcttaatgt 113220
caaatttgtt tttcttaaga tttattttta attaacacat aaaaattgta catatttatg 113280
aggtatagtg tgatgtttca atacatgtgt atattgtatg atgttcaaat caggataatt 113340
aacttatcca tcaccttaaa catttagcat ttctttgtga tgaaaacatt caaaatcctc 113400
ttttttagct gttttgaaat gtataataca gtatcgttat ttatatttac tctactctgc 113460
aacagagcac cagaacttac ttctcctatc tgtctataat tttgtaccca tttgctaatc 113520
tctccccatc cctccctcaa ccctaacctc ctcattccct agtaaccact gttctactct 113580
gtacctctat gagatcagct tttgtaaatt ccacaaatga gtggatcagg tggtatttgt 113640
atttctgtgt ctggcttatt ttctttaaca taatgtcttc caggtttatc tatggtgcca 113700
caaatgacag catttctttt ggccaatagt attccattgt ttatatttac cgcattttat 113760
aatccactat ctgttggtga acacataggt tgcttttata tcttgtctat tgtgaatcgt 113820

gctgcagtaa atataggagt gcagatatct tttcaatata ctgttctcat tttgtttggg 113880
taaatagcca gtagtgggac tgctggatca tatgttagta ctgtttttaa ttttttgagg 113940
aaatgccatg cagttttcca tagtggctgt actagtttac attcccacca atagtatagc 114000
gcatttccca tttctttaca tccatggcag catttgtcat ttttgtacta gccattctaa 114060
ttggggtgag ataatatccc attatggttt tgatttgtat ttctgtgata attagtgata 114120
ttgagcattt ttccatatac ctattggata tttgtattta ttcttttgaa aaaaaaaagt 114180
ctatttagat tttttgacca ctttttaatc aaattatgta tttttttgct attatgttgt 114240
ttgaatgttt tatatattct ggatattaat cccttttcag atatgtactt tgcaaatatt 114300
ttctactatt ctgtagattg tttctttcct ctgttgatta tttcctttct tgggcagtaa 114360
ctttttagtt tgaggtaatc ccatttgtct atttttgctt ttcttgcctg tatttttgaa 114420
ttcttgccca aaaattctta tccaaaccaa cgtcatgaag catttccccc atgttttctt 114480
ctagtagttt tatagtttca ggtcttacat ttaagtattc aatccatttt gtgttgattt 114540
ttatatatgg taagagatgt tggtctagtt ttattcttct gcatatagat gtccagtttt 114600
attggcacta tctattgaag agaccatcct ttacccaatg tgtgttcctg ctgcctttat 114660
tgaaaatcag ttggttgtaa gagcatcaat acatttctgt gttgtctgtt ctgttccatt 114720
ggtctatatg tttattttta tgctgactcc atcctgtttt ggttactata gctttataga 114780
atactttgaa gtcagtgtta tgcctccagc tttgttcttt ttgctcaaaa tgactttggc 114840
tatttggagt ctttcgtgct tccatacgaa ttttataatt ttttctatgt ctgtgaagaa 114900
tgccattgat accttgatag agattgcatt gaatctgtag attgggtagt atagacatat 114960
caacaatatt aattattgct attcatgaaa atggaatatt tttccattta tttgtatcct 115020
cttcagtttc ttccatcagt gttttatagt tttcattgta gagatcattc acatccttgg 115080
ttaaatttat tcctctctat ttttttgcag ctcctaaatg gaattgcttt ctcaatttct 115140
ttttcagact gttcactctt gtcatataga aatgctagtg atttgtgtat gttgcttttg 115200
tatcctgcaa ctttactgca ctttgtttat tctaacaatt tttggtggca tctttagtgt 115260
tttctataaa taagataatg tcatctgcaa atagtaacag tattatttcc ttctttccaa 115320
tttgcatgcc ccttatttct tttgcccaat tgttctggct aggacttcca gaactatgct 115380
gaataaaagt ggtaaaagtg gacatatttg tcttattcca tatcatacag gaaaagcttt 115440
caaccttccc ctattcagta tgatattagt tgtgggtttg tcatacgtga cctttattaa 115500
gttgaggtat gtttcttcta tacctaattt gtttaatgtt ttttatcatt aatggatgtt 115560
gaattttatc aaatgctttt tctgcatttt aaatttatca tatggcgtct gtccttgatt 115620
ctgttgatgt gatgtattat gtgttaattt gcatgtgttg aaccatcctt ttattcctga 115680
gataaattat acttgattat gttaaatgat gtttttaatg tgctgttgaa ctcagtttgc 115740
tggggatttt tgcatctatg ttctttaagg ctattggcct atagtttttg ttgttgtgtt 115800
cttgtctggt tagaacagtt gggttattct ggcttcatac aatgagtttg gcaggattct 115860

atcctcttta atcctttgga attgtttgaa aaggattggt atttgctctt aagaattcag 115920
cagtgaaggc atcagatgat gggattttca ttgatgggag actttttgta actgatttaa 115980
tcctgttact ttctattggt ctgttcagat tttctatttt gtccattcaa ttttggtaga 116040
ttttatatat ccaggaacgt atcaatttct tctaggtgtt ccaatttgtt ggcatatagt 116100
tgtttgtaat agtctcatag ttctttctat atctgtggta tcagttgtaa tgtcttcttt 116160
tccattggat tttatttatt tcaatcatct ctcttttatc ttagtcccat taaaggtttg 116220
tcaattgtgt ttatcttttc ataaaaccaa ctctttttca tttatcattt tttttattct 116280
ctattttgtt tatttcttct ctgatgttta ttatttattt tcttctaatt ttaggtttag 116340
tttgctcttc attttcctat aagtatgctt gaactttatt ctgggcagca gttaagttac 116400
tggaaacagt ttcatacttt tgggtcctcc ttttaaattt ttttaggcag gaccagacta 116460
gtgtttagtc tagggctact cactatttag gcaaggtgct tctaaatact ctgtaatata 116520
cttcatcaat tatgatgttt tccattttgg cgggtggaaa caggcaatac accccacctg 116580
tgtgagtcct gattttcatc cagtgctttc agatagttct ttccaaggtc ttgggttgtt 116640
tccccacgtc tttatcagta gtaatctgct agataattta gtgagacctt ttatagagcc 116700
ttgtaattct ctctctctgc tcctcacacc cctctcatac cctgtcctgc taactatgtc 116760
tgccttggtc tcccctgagt ctcagttcta tctcctcagt tcaaacccac aaggcttcac 116820
ctgagtttcc cttacctgca cagcagctag aaaactctct caggttggta agctgaagca 116880
atcacaatgc taacctgatt gtttcccact attaggaatc actttccttt attgcctaac 116940
atctgatatc ctgaaaactg ttgacatatt atatatttta tgtttttttt taattggttg 117000
tttcgggcag ataggtacat ccagtccttg gtatttcatc ttgaccagaa gaagaaatcc 117060
ttggactatt gaaatttttt catttttcca ataaccattt aggtttgttt cattgaaatc 117120
attgttatct tctttttagt attaattgtc catttatttt tgcacatgtt tccccagtat 117180
ggcttttgat tggttgccct agtcccttac atggtttcaa ccaattacgt tattcttcag 117240
aatctgatag tactacttag agtctttagt ttcactgctt ctgtgattga tgaaacgttt 117300
gcatacgatg attttgtttc tatattagaa cagttttttt ttggttaatt ttactttaag 117360
ttctggaata cttgtgcaga acgtgcaggt ttgttatata ggtatacatg tgccatggtg 117420
gtttgctgca cctagcaacc cgtcattcag tttttaagcc ccgcaagcat taggtatctg 117480
tcctaatgct ctccctcccc ttctccctaa cccccgacag gccgcagttt ttaatgttcc 117540
cctccctgta tccatgtgtt ctcattgttt aactcccact tatgagtgag aatacctggt 117600
gttttgtttt ctgttcctgc gttagtttgc tgacaatgat ggtttccatc ttcatccatg 117660
tgcctgcaaa ggacatgatg gctgcataat attccatgtt gtatatgtgc catttattct 117720
tcatccagtc tgtcatcgat gggcatttgg gttggttcca agtctgtgct attgtaaata 117780
gcgctgcaat aaacataaat gtgcatgtgt ctttatacaa gaatgattta taatatttgg 117840
gtatataccc agtaatggga ttgctgggtc aaatggtatt tctggttcta gatccttgag 117900

gaattgccac actgtcttcc acaatggttg aactaattta cactcccacc aacagtgtaa 117960
aagcattcct atttctccac atcctcgcca gcatctgttg tttcctgact ttttaatgat 118020
caccactcta actggcatga gatgttatct cattgtgttt tcaatttgca tttctctaat 118080
gagcagtgat gatgagcttt ttttcatatg tttttggccg tataaatgtt ttcttttgag 118140
aagtgtctgt tcatatcctt tgcccacttt ttgatggagt tgtttctttc ttctaaattt 118200
gtttaagttt cttgtagatt gtggatatta gacctttgtc agatggatag attgcaaaaa 118260
ttttctcccc ttctgtaggt tgcctgttca ctccaatgat agtttctttt gctgtgcaga 118320
agctctttag tttaattaga tcccatttgt ccaatttggc ttctgttgca agtgcttttg 118380
gtgttttagt cataaagtct ttgcccattc ctgtgtcctt aatggtataa tccaggtttt 118440
cttgtagggc ttttatggtt ttaggtttta tatttaagtc tttaatccac cttgagttaa 118500
tttttatata aggtgtgagg aaagggtcca gtttcagctt tctacatata gctagccagt 118560
tttcccagca ccatttatta aataggggat cctttcccca ttgcttgttt gtgtcaggtt 118620
tgtcgaagat caaatggttg tagatgtttg gtgttattgc tgaggcctct gttctgttcc 118680
atcggtctat atattggttt tggtaccagc accatgctgt ttcggttact gtagccttgt 118740
agtatagttt gaagtcaggt agcatgatgc ctccagattt attctttatg cttatgattg 118800
ttttggctat atgggctctt ttttggttcc atatgaaact taaattagca ttttctaatt 118860
ctgcaaagaa agtcagtggt agcttgatgg ggataacatt gaatctataa attactttgg 118920
gcactatggc cattttcacg atattgactc ttcctatcca tgagcatgga atgtttttcc 118980
atttgtttgt gtcctctcat ttctttgagc agtggtttgt ggctctcctt gaagaggtcc 119040
ttcatgtccc ttgttaagtt gtattccttt attctcttta tagcaattgt gaatgggaat 119100
ttaactatga tttggctctc tgcttgtgta ttattggtgt gtaggaatat ttgtgatttt 119160
tgcacattgg ttttgtatcc tgagactttg ctgaagttgc ttatcagcat aaatagtttt 119220
taggctgaga cgatagggtt ttctgaatat acaattatgt catctgtgaa cagagacaat 119280
ttgacttcct ctcttcctat ttgaatacct ttatttcttt ttcttgcctg attgccctgg 119340
ctggaacttc caatactatg atgaatagga atggtgagag agggcatcct tgtcttgtgc 119400
tggtattcaa tgggaatgct tccagctttt gcccattcag tatgatattg gctatgggtt 119460
tgtcataaat agctctattt tgagatatgt ttcatcaata cctagtttat ggcggttttt 119520
agcatgaagt agtgttgaat tttattgaag gccttttctg cgtctattga gataatcgtg 119580
gtttttgtca ttggttctgt ttatggaaag catttagccc atttacattg aaaattaata 119640
ttgttacgtg tgaatttgat cgtgtcatca tgatgctagc tggttatttt atacattagt 119700
tgttgtagtt tcctcatatt gtcattggtc tttatatttt gatgtttttg cagtggctgg 119760
taccagtttt tcctttccat atttaatgct tcctacagga gctcttataa gcagacctgg 119820
tggtgacaaa aatctcacag catttgcttg tctggaaagg attttatttc tcctttgctt 119880
atgaagttta gtttggctgt atatgaaatt ctgggttgaa aattcttttc tttaagaatg 119940

ttgaatattg accctcactg tcttcaggct tgtagggttt ctgcagagag atctactgtt 120000
agtctgatga gcttcccttt gtaggttaac ccgacctttc tctctggctg cccttaacat 120060
tttttccttt gtttcaaact tggagtatct gatgattatg tgtcctggga ttgctcttct 120120
caaggagtat cttagtggtg ttctctgtat ttcctgaatt tgaatgttgg catgtcttgc 120180
taggttgggg aagttctcct ggatgatatc ttgaattgtg ttttccaact tggttccatt 120240
ctccccatca ctttcaggta catcagtcaa tcgtagattt gattcacata gacccacatt 120300
ttattggagg ctttgttcgt tccttttaat tattttttct ctaatcttca tgttttgttt 120360
cattaatttg atcttcaatc tctgatatca tttcttctac ttgatcaatt caactatcca 120420
tacttgggta tgcttcacga agttctcttg ctgtttttca gctccatcag gtcatttatg 120480
ttcctctcta aactggttat tctagttagc agttccttta accttttatc aagtttcttg 120540
ccttccttgg attgggttag aacatgctcc tttagctcag aggagtttgt tattacccac 120600
cttctgaagc ctacttctgt caattcgtca atatcattct ctgtccagtt ttatgccctt 120660
gctggagagg agttgcgatc atttggggga aaataggcat tctatttttt ggaattttca 120720
gcgtttttgt gctggttttt cctcattttt gtggatttat ctgccttcga tgttagaggc 120780
taatgacttt tatatggagt ttttgtgtgg gagttatttt tgttgatgtt gaagttgttg 120840
ctttctgtgt gttagttttt cttctaacag tctggcccct attctgcaag tctgctgcag 120900
ctttctggag gtccactcca gcccctgttc accttggtat caccagtgga ggctgcagaa 120960
cagcaaagat tgcttcctac tccttcctgt ggaagctccg tcccagagcg gcaccagcct 121020
gatgccagct ggtgctctcc tgtatggtgt gtctgttgac ccctgttggg aggtctctcc 121080
cagtcaagag gcacaggggt cagggaccca ctttaggagg cagtctgtcc cttagcagag 121140
ctagagcact gtgctgggag aatcgttgtc aggatcagct gctctcttca gagctggcgg 121200
gcaggaaagt ttaagtctgc tgaagctgcg cccacagttg ccccttcccc gaggtgctct 121260
gtcccaggga aatggggttt tatctataag cccctgactg gggctgctgc ctttcagaca 121320
cgccctgccc agagagaagg aatctagaga ggcagtctgg tcacagccgc tttgctgcgc 121380
catggtaaat tctgcccagt ccaaacctcc cggccccctt agcattgtca ggggaagacc 121440
gcctactcaa gcctcagtaa tggtggacac ccccccgccc ccgccgccac caagttcagt 121500
tgtcgcagtt tgacttcaga ctgctgtgtt ggcagtgaga atttcaagcc agtggttctt 121560
agcttgctgg actccgtggg agtgggaccc actgagcaag accacttggc tccctggctt 121620
cggcctcact tccagaggag tgaatggttc tgtctcgctg ggattccagg caccactggg 121680
ttacgaaaaa aaaaaaaaaa actgcagcta gctcactgtc tgcccaaatt gtctcccagt 121740
tttatccttg aaacccagga ccctggtggt gtaggcacac cagggaatct cctgatttgt 121800
ggattgcaaa aaccatggga aaagtgtagt atctgggctg ggtagcacag tccctcacag 121860
ctttgcttgg ctgagggaga gaggtcccct ggctccttgc acttctcatg ttgggcgatg 121920
ccccacccta cttctgctca ccctccgtga gttggaccca ctggctaacc agtcccagtg 121980

agacgaactg ggtacctcag ttgtaaatgg agaaatcacc caccttctgc tttggtctca 122040
ctgggagctg ctgaccggag ctgttattcg gccatcttgc cagattctcc gaaaatgctt 122100
attaaggagt ttacttctca tttctctaaa agatacttgg gagagacagt ttatttaaaa 122160
gtattacatc tcttaatata ttgaaaactt ttcctgtgag gttatattaa atttacaact 122220
tttttaattg caacagagat aacataagca aacgtacact tactttgatg aaaaacttaa 122280
aatgcactgg ggactactat gatagaagcc acttgctaac attggattta tggtttgctt 122340
tcttaatatg ttatatagta agccaccatt tattgaatgg attaactaca tgggaccctc 122400
ccttctatgt tttatctgtg tacacatacc cttacacctt ggggcccagc ttaacagcaa 122460
agttcaatag ctcctgtctc ctgctgaaca tttagcatat tccaggataa cttctgccat 122520
tttacatttt cctcctccgt cactaatcac acatccaatt ccacagattt ggcatcttcc 122580
gatttaaaag tggtgtataa ggatttagta tatattgaaa aatatctctc aagattctct 122640
tgagagttaa ctgtgtagag tatagatttt actttgcata aatgtaggtg aaataaacag 122700
attgcaaggc tattgatgct gtcacatttt cagcaaatgt aaaattatgt atgtatacat 122760
gtatatatat atacacacac atttttttta atttaggatt tgctttccca tatgcttcat 122820
atggacccac atcagcggta tactgctgaa caaatattaa agcactcatg gataactcac 122880
agagaccagt tgccaaatga tcagccaaag agaaatgatg tgtcacatgt tgttaaggta 122940
aaacaaacat acttttatgt gccttcgaga tatcagatac ctaataaacc cattgtgtgc 123000
cacttgctga tgacacttaa ataattcctt aaaatatctt tattttttaa agatagattt 123060
ttttaaatta acaaccatgt acttttctta gcttacttta ctactgcctt tcagatgaaa 123120
caataaaaat ttcttgattg tacctgccgt gcttttgaga gatgctggaa ttaggattgg 123180
gctaaatgca agtttcatat tgaaataaaa gattgctgtt agttcttaat ttataactgt 123240
tcgtgaaacc attcctatat ccatatacat tgaatcaagg ttttaaatgt tagtgtttta 123300
acaatttttt gtaaaaagca tagttattga aatgaaaaac atacaagtaa tattgattca 123360
tcagtgcaca gatctaatat attcaattat tttcctagga aaacattttg ttctagatat 123420
atgtctcatg atatcatgtg aaagtttttt ttttttttaa ctccagaatt tgtacttagt 123480
ttgtggcatt ttctcattac tttagggagc aatggttgca acatactctg ccctgactca 123540
caagaccttt caaccagtcc tagagcctgt agctgcttca agcttagccc agcgacggag 123600
catgaaaaag cgaacatcaa ctggcctgta agatttgtgg tgttcctagg ccaaactgga 123660
tgaagatgaa attaaatgtg tggctttttt cctattctta tcaaaggcat cgttgtctgc 123720
taaattactt gaatattaag taatattaaa tccccatttt taggggaagt gagatttaaa 123780
aaaccattca caggtccaca atattcatac tatgtgtttg cagtagtgtt caagtgttta 123840
tttaagcata taattggtgt ccaccaggtc ctcacaactt ctctgcacac aagcttctaa 123900
aattcctttc aaataaagtt actttaatat tt 123932

<210> 7

<211>  21
<212>  DNA
<213>  Artificial

<220>
<223>  forward primer

<400>  7
   tgcctgcagc ccccttcaac a                                                    21


<210>  8
<211>  24
<212>  DNA
<213>  Artificial

<220>
<223>  reverse primer

<400>  8
aggggcctcg ggatcactt cttc                                                 24


<210>  9
<211>  24
<212>  DNA
<213>  Artificial

<220>
<223>  forward primer

<400>  9
gcaccacggg gctcttctac tctg                                                24


<210>  10
<211>  24
<212>  DNA
<213>  Artificial

<220>
<223>  reverse primer

<400>  10
cacatcattc gccccgttct tcat                                                24


<210>  11
<211>  19
<212>  DNA
<213>  Artificial

<220>
<223>  forward primer

<400>  11
gcgagcagcg gcgaggtaa                                                      19


<210>  12
<211>  23
<212>  DNA
<213>  Artificial

<220>
<223>  revers primer

<400>  12

caagggccag ttctgcgagg tag                                              23

<210>  13
<211>  22
<212>  DNA
<213>  Artificial

<220>
<223>  forward primer

<400>  13
gcggagccgg cagcagcagt aa                                               22

<210>  14
<211>  24
<212>  DNA
<213>  Artificial

<220>
<223>  reverse primer

<400>  14
agcccccacc cccaatcttt ctca                                             24

<210>  15
<211>  20
<212>  DNA
<213>  Artificial

<220>
<223>  forward primer

<400>  15
accacagtcc atgccatcac                                                  20

<210>  16
<211>  20
<212>  DNA
<213>  Artificial

<220>
<223>  reverse primer

<400>  16
tccaccaccc tgttgctgta                                                  20

## Description

The present invention relates to marker for sunitinib resistance formation and modulators thereof for the prevention and reduction of sunitinib resistance of hyperproliferative cells, in particular cancer cells. Further, the present invention relates to pharmaceutical compositions comprising agents suitable for anticancer therapy in combination with the modulators for treating proliferative diseases.

Cells of a multicellular organism can be induced by growth factors which bind at specific receptors on the cell surface, to proliferate and to generate certain tissues such as blood vessels. Protein kinases, e.g. tyrosine kinases, are part of intracellular signal cascades which regulate cell division, differentiation and/or apoptosis and thus are involved directly or indirectly in the control of cell proliferation. For example tyrosine kinases are involved in the transmission of growth signals from receptors on the cell surface to the nucleus, where the cell division is started. These signal cascades are often disturbed in hyperproliferating cells such as cancer cells, for example due to mutations in the sequence of tyrosine kinase encoding genes involved in the cell cycle regulation. Compared to the corresponding wild-type cells such hyperproliferating cells may exhibit a decreased or increased level of one or more tyrosine kinases. Therefore, one approach for the treatment of proliferative diseases lays in the application of low molecular weight organic substances acting as kinase inhibitors and therefore functions a cell proliferation inhibitor and/or apoptosis activator. An example for such a substance is sunitinib.

Sunitinib, also known as SU11248, which is the active ingredient of the therapeutic agent Sutent^{®} is an orally administered, small molecule. So far Sunitinib received approval as multi-targeted receptor tyrosine kinase inhibitor for the treatment of renal cell carcinoma (RCC) and imatinib-resistant gastrointestinal stromal tumor (GIST). Sunitinib has become a standard of care for both of these cancers. The drug is being investigated as a possible therapy for other proliferating diseases, like breast cancer and melanoma. Administration of SU11248 inhibits intracellular signal transmission of multiple receptor tyrosine kinases leading to growth inhibition, prevention of invasivity and induction of apoptosis of cancer cells.

Most patients who show a positive response to sunitinib develop a resistance to the drug after a period of treatment as experienced with other kinase inhibitors such as Gleevec that have been in clinical use longer. Drug-resistance to chemotherapeutic agents constitutes a major problem in the treatment of cancer. Hence, drug-resistance formation remains a key reason for bad prognosis in cancer and its prevention or reduction is pivotal for a significant and long lasting clinical success of these drugs.

Therefore there is an urgent need to provide an effective prevention of drug-resistance in the treatment of proliferative diseases. The term "proliferative diseases" as used herein refers also to tumors, cancer, malignancies and their metastases.

This objective of the present invention is solved by providing targets which are the reason for the drug resistance especially the Sunitinib resistance together with compounds and pharmaceutical compositions which can be used to address these targets in order to overcome the drug resistance and especially the Sunitinib resistance. Further advantageous embodiments, aspects and details of the invention are evident from the pending claims, the description, the examples and the figures.

Drug resistance is a diminished or failed response of an organism, disease or tissue to the intended effectiveness of a chemical or drug. Drug resistance is a consequence of evolution and is a response to selection pressures affecting all living organisms. This can also be seen in cancerous tumors where some cells may develop resistance to the drugs used in chemotherapy. By avoiding a particular drug by testing a specific tumor's phenotypic chemotherapy resistance, unnecessary toxicity may be avoided.

The present inventors could show that up-regulation of the kinases PRKX, TTBK2 and RSK4 strongly and specific correlate with sunitinib insensitivity. A specific down-regulation of PRKX, TTBK2 and/or RSK4 gene expression resulted in an enhanced therapeutic effect of sunitinib especially in sunitinib desensitized cells. A reduction of PRKX, TTBK2 or RSK4 increases the sunitinib induced apoptosis of cancer cells and the sunitinib inhibition of cancer cell migration. Further, it was shown for the first time that these genes are expressed in different cancer cell lines in a rate which is characteristic for sunitinib dependent resistance formation. There was no correlation in the protein expression of these genes observed in sorafenib, which is another kinase inhibitor, desensitized cells.

Therefore monitoring the expression of PRKX, TTBK2 and RSK4 is an excellent marker for the development of sunitinib resistance of hyperproliferating cells. A marker gene as defined by the present invention is a gene of which expression correlates to the drug-resistance, preferred to sunitinib resistance of a hyperproliferating cell.

The present invention relates to a method of determining if a subject suffering from a proliferative disease has developed a sunitinib resistance, comprising or consisting of:
measuring the expression level of PRKX, TTBK2 and/or RSK4 in a sample comprising diseased cells from said subject, wherein an increased PRKX, TTBK2 and/or RSK4 expression level is indicative for a sunitinib resistance. It is preferred if the subject is a cancer patient, particularly a human cancer patient.

Preferably, the method of the present invention determines the gene expression of the resistance marker PRKX, TTBK2, RSK4, allelic variants and homologs thereof having at least 70-80%, preferably at least 80-90%, more preferably at least 90-95%, and most preferably at least 95-99.9% or more identity with the sequences of SEQ ID NO:1, 2 or 3 over the entire length, provided that said allelic variants and homologs exhibit the characteristics of a marker gene as defined above. Mostly preferred, the target polypeptide is PRKX as defined in SEQ ID NO:1.
Preferably, the marker gene encodes a polypeptide (marker polypeptide) selected from PRKX (SEQ ID NO:1), TTBK2 (SEQ ID NO:2) or RSK4(SEQ ID NO:3), allelic variants and homologs thereof as defined above. More preferably, the marker gene is a PRKX nucleic acid having the sequence of SEQ ID NO:4, a TTBK2 nucleic acid having the sequence of SEQ ID NO:5, or a RSK4 nucleic acid having the sequence of SEQ ID NO:6.

The proliferative disease said subject suffers from is selected from the group comprising or consisting of acute lymphoblastic leukemia, acute myeloid leukemia, adrenocortical carcinoma, aids-related cancers, anal cancer, appendix cancer, astrocytomas, atypical teratoid/rhabdoid tumor, basal cell carcinoma, bile duct cancer, bladder cancer, bone cancer, osteosarcoma and malignant fibrous histiocytoma, brain stem glioma, brain tumor, central nervous system atypical teratoid/rhabdoid tumor, astrocytomas, craniopharyngioma, ependymoblastoma, ependymoma, medulloblastoma, medulloepithelioma, pineal parenchymal tumors, supratentorial primitive neuroectodermal tumors and pineoblastoma, brain and spinal cord tumors, breast cancer, bronchial tumors, burkitt lymphoma, carcinoid tumor, carcinoid tumor, gastrointestinal tumor, central nervous system lymphoma, cervical cancer, chordoma, chronic lymphocytic leukemia, chronic myeloproliferative disorders, colon cancer, colorectal cancer, craniopharyngioma, cutaneous t-cell lymphoma, mycosis fungoides and sézary syndrome, endometrial cancer, ependymoblastoma, ependymoma, esophageal cancer, esthesioneuroblastoma, ewing sarcoma family of tumors, extracranial germ cell tumor, extragonadal germ cell tumor, extrahepatic bile duct cancer, intraocular melanoma, retinoblastoma, gallbladder cancer, gastric cancer, gastrointestinal carcinoid tumor, gastrointestinal stromal tumor, gastrointestinal stromal cell tumor, gestational trophoblastic tumor, glioma, hairy cell leukemia, head and neck cancer, heart cancer, hepatocellular cancer, histiocytosis, hodgkin lymphoma, hypopharyngeal cancer, intraocular melanoma, islet cell tumors, kaposi sarcoma, renal cell cancer, kidney cancer, langerhans cell histiocytosis, laryngeal cancer, chronic lymphocytic leukemia, chronic myelogenous leukemia lip and oral cavity cancer, liver cancer, non-small cell lung cancer, aids related lymphoma, mycosis fungoides and sézary syndrome, non-hodgkin lymphoma, lymphoma primary central nervous system, macroglobulinemia, malignant fibrous histiocytoma of bone and osteosarcoma, medulloblastoma, medulloepithelioma, melanoma, merkel cell carcinoma, mesothelioma, metastatic squamous neck cancer with occult primary, mouth cancer, multiple endocrine neoplasia syndromes, multiple myeloma/plasma cell neoplasm, mycosis fungoides, myelodysplastic syndromes, myelodysplastic / myeloproliferative neoplasms, myelogenous leukemia, myeloid leukemia, multiple myeloma, myeloproliferative disorders, nasal cavity and paranasal sinus cancer, nasopharyngeal cancer, neuroblastoma, oral cancer, oral cavity cancer, oropharyngeal cancer, osteosarcoma and malignant fibrous histiocytoma of bone, ovarian cancer, ovarian epithelial cancer, ovarian germ cell tumor, ovarian low malignant potential tumor, pancreatic cancer, papillomatosis, parathyroid cancer, penile cancer, pharyngeal cancer, pineal parenchymal tumors of intermediate differentiation, pineoblastoma and supratentorial primitive neuroectodermal tumors, pituitary tumor, plasma cell neoplasm/multiple myeloma, pleuropulmonary blastoma, pregnancy and breast cancer, prostate cancer, rectal cancer, transitional cell cancer, respiratory tract cancer, retinoblastoma, rhabdomyosarcoma, salivary gland cancer, skin cancer, melanoma, skin carcinoma, small cell lung cancer, small intestine cancer, soft tissue sarcoma, squamous cell carcinoma, squamous neck cancer, supratentorial primitive neuroectodermal tumors, t-cell lymphoma, mycosis fungoides and sézary syndrome, testicular cancer, throat cancer, thymoma and thymic carcinoma, thyroid cancer, transitional cell cancer of the renal pelvis and ureter, trophoblastic tumor, gestational, ureter and renal pelvis, urethral cancer, uterine cancer, endometrial, uterine sarcoma, vaginal cancer, vulvar cancer, waldenström macroglobulinemia and wilms tumor. The method is preferably used if the cancer is kidney cancer, breast cancer or melanoma.

The method according to the present invention is preferred if the sample is a sample comprising tumor cells, e.g. a tissue sample or a body fluid sample. There are different possibilities to obtain such a sample. Tissue or cell samples can be taken from almost any part of the body. How this is done depends on where the tumor is and what type of cancer is suspected. The most common biopsy types used in cancer diagnosis are: bone marrow biopsy, endoscopic biopsy, needle biopsy, excisional or incisional biopsy, skin biopsies.

The method according to the present invention further refers to a determination, of the expression of PRKX, TTBK2 and/or RSK4 on the protein level, or of the expression of PRKX, TTBK2 and/or RSK4 on the nucleic acid level. Thereby the expression level between different samples, e.g. between a cancer and a non-malignant cell sample are compared.

Gene expression is the process by which information from a gene is used in the synthesis of a functional gene product. These products are often proteins, but in non-protein coding genes such as rRNA genes or tRNA genes, the product is a functional RNA. Several steps in the gene expression process may be modulated, including the transcription, RNA splicing, translation, and post-translational modification of a protein.

The expression level of genes can be determined on the nucleic acid level, preferably on the level of mRNA. Levels of mRNA can be quantitatively measured by Northern blotting. A sample of RNA is separated on an agarose gel and hybridized to a labeled RNA probe that is complementary to the target sequence. The labeled RNA is then detected. Alternatively the level of mRNA can be measured by reverse transcription quantitative polymerase chain reaction (RT-PCR followed with qPCR) to compare mRNA abundance between samples, e.g. between a cancer and a non-malignant cell sample.
RT-PCR first generates a DNA template from the mRNA by reverse transcription, which is called cDNA. This cDNA template is then used for qPCR where the change in fluorescence of a probe changes as the DNA amplification process progresses. With a carefully constructed standard curve qPCR can produce an absolute measurement such as number of copies of mRNA. Relative concentrations of DNA present during the exponential phase of the reaction may be determined by plotting fluorescence against cycle number on a logarithmic scale (so an exponentially increasing quantity will give a straight line). A threshold for detection of fluorescence above background is determined. The cycle at which the fluorescence from a sample crosses the threshold is called the cycle threshold, Ct. The quantity of DNA theoretically doubles every cycle during the exponential phase and relative amounts of DNA can be calculated, e.g. a sample whose Ct is 3 cycles earlier than another's has 23 = 8 times more template. Since all sets of primers don't work equally well, one has to calculate the reaction efficiency first. Thus, by using this as the base and the cycle difference C(t) as the exponent, the precise difference in starting template can be calculated (in previous example, if efficiency was 1.96, then the sample would have 7.53 times more template).

Amounts of DNA are then determined by comparing the results to a standard curve produced by real-time PCR of serial dilutions (e.g. undiluted, 1:4, 1:16, 1:64) of a known amount of DNA. To accurately quantify gene expression, the measured amount of DNA from the gene of interest is divided by the amount of DNA from a housekeeping gene measured in the same sample to normalize for possible variation in the amount and quality of DNA between different samples e.g. between a cancer and a non-malignant cell sample. This normalization permits accurate comparison of expression of the gene of interest between different samples, provided that the expression of the reference (housekeeping) gene used in the normalization is very similar across all the samples. Additionally "tag based" technologies like Serial analysis of gene expression (SAGE), which can provide a relative measure of the cellular concentration of different messenger RNAs, can be used.

The expression of genes on the protein level may be detected by a Western blot against the protein of interest. A sample is separated on a polyacrylamide gel, transferred to a membrane and then probed with an antibody to the protein of interest. The antibody can either be conjugated to a fluorophore or to horseradish peroxidase for imaging and/or quantification.

Alternatively an enzyme-linked immunosorbent assay (ELISA) may be used which works by using antibodies immobilised on a microtiter plate to capture proteins of interest from samples added to the well. Using a detection antibody conjugated to an enzyme or fluorophore the quantity of bound protein can be accurately measured by fluorometric or colorimetric detection.

In mass spectrometry-based methods relative quantification is possible to compare protein abundance between samples, e.g. between a cancer and a non-malignant cell sample. This can be achieved by labeling one sample with stable isotopes alone or incorporated into protein crosslinkers, which leads to a mass shift in the mass spectrum. Differentially labeled samples are combined and analyzed together. The differences in the peak intensities of the isotope pairs accurately reflect difference in the abundance of the corresponding proteins.

The method according to the present invention is preferred if the expression level of PRKX, TTBK2 and/or RSK4 is increased in said diseased cells at least 1.5-fold, preferred at least 2-fold and more preferably at least 4-fold compared to the expression level in normal cells from said subject. The term normal cell is defined as a cell preferably of the same tissue or organ of the subject which does not grow in an unlimited manner and show a normal function and shape. These non malignant cells can also be called wild-type cells.

Altering the amount and/or activity of PRKX, TTBK2 and/or RSK4 in a cell leads to a change in the sensitivity of the affected cell to sunitinib as the present invention could show.

Thus, in a further aspect, the present invention relates to molecules or agents which modulate or affect a marker gene or protein as defined above, e.g. its expression and/or activity, and to compositions comprising at least one of said molecules or agents for therapy, prognosis and/or diagnosis of a proliferative disease, e.g. cancer disease.

The term "modulate" or "modulating" within the context of the present invention relates to the ability of a molecule or a composition comprising at least one of such a molecule (so-called modulator) to influence the synthesis, activity or biological function of PRKX, TTBK2 and/or RSK4 and thus to change the sensitivity of the cell to sunitinib or another chemotherapy. A target structure for a modulator according to the present invention may preferably be PRKX, TTBK2 and/or RSK4 as a nucleic acid or a polypeptide.

Modulation of a target structure according to the invention within a hyperproliferating cell leads to an increased sensibility to sunitinib or another chemotherapy of said cell. The preferred hyperproliferating cells are cancer cells as defined above and especially preferred cancer cells derived from kidney cancer, breast cancer or melanoma.

A modulator can be a molecule which interacts directly or indirectly with a target polypeptide, wherein, as a consequence of the interaction, the activity, e.g. the enzymatic activity or biological function is inhibited, blocked or decreased. A modulator may inhibit the interaction of a target polypeptide with its components of the signal transduction cascade, e.g. with its substrates, co-factors or chaperones. The inhibition may be reversible or irreversible or may be competitive. A modulator further activates or enhances specific the degradation of a target polypeptide. The modulator may interact with the target polypeptide, e.g. by binding the target in a manner leading to conformational changes, masking, immobilization, binding and/or degradation of the target. A preferred modulator inhibits the enzymatic kinase activity of a PRKX, TTBK2 and/or RSK4 polypeptide. Thus all known inhibitors of PRKX and all known inhibitors of TTBK2 and all known inhibitors of RSK4 can be used according to the present invention to reduce, prevent, eliminate or overcome the drug resistance against Sunitinib or to reduce, prevent, eliminate or overcome the development of drug resistance against Sunitinib. Moreover the effect against drug resistance against Sunitinib or the effect against development of drug resistance against Sunitinib can be increased by inhibiting more than one target such as by inhibiting PRKX and TTBK2 or PRKX and RSK4 or TTBK2 and RSK4 or PRKX and TTBK2 and RSK4. The same increased effect can be obtained by decreasing the activity of more than one kinase such as PRKX and TTBK2 or PRKX and RSK4 or TTBK2 and RSK4 or PRKX and TTBK2 and RSK4 or by decreasing the expression of more than one kinase, i.e. decreasing the expression of PRKX and TTBK2 or PRKX and RSK4 or TTBK2 and RSK4 or PRKX and TTBK2 and RSK4.

Compared to an activity level observed in untreated cells of a given cancer cell type or tissue, an decreased enzymatic activity means a change in the activity by at least factor 1.5, preferably by at least factor 2, more preferably by at least factor 5. The enzymatic activity may be decreased by binding of a modulator and competing with a substrate for entering the enzymes active site or binding site, or circumvent the enzymatic reaction by a binding to cofactors. A modulator can also cause a relocation of the target polypeptide in the cell which then inhibits the enzymatic reaction.

If the target structure is a nucleic acid such as DNA or RNA, e.g. a PRKX encoding DNA or mRNA, a modulator can be a molecule interacting directly or indirectly, e.g. intercalating with the target nucleic acid, wherein as a consequence of the interaction, the expression, i.e. transcription and/or translation, of said target nucleic acid is inhibited or downregulated, preferably inhibited. A modulator directed against a target nucleic acid may also be a molecule which enhances the cleavage or degradation of this nucleic acid.

Therefore the present application relates also to a modulator for
(i) the inhibition of a polypeptide selected from PRKX, TTBK2 and/or RSK4
   or for
(ii) the inhibition, deactivation, degradation, intercalation, cleavage or down-regulation of a nucleic acid selected from PRKX, TTBK2 and/or RSK4
for reduction or prevention of sunitinib resistance of a proliferative disease.

The term "PRKX polypeptide" or "TTBK2 polypeptide" or "RSK4 polypeptide" refers to the kinase PRKX or the kinase TTBK2 or the kinase RSK4, respectively, while the term "PRKX nucleic acid" or "TTBK2 nucleic acid" or "RSK4 nucleic acid" refers to a nucleic acid such as DNA or RNA, mRNA encoding the kinase PRKX or the kinase TTBK2 or the kinase RSK4, respectively.

Further the present application relates to a modulator for
(i) the inhibition of a polypeptide selected from PRKX, TTBK2 and/or RSK4
   or for
(ii) the inhibition, deactivation, degradation, intercalation, cleavage or down-regulation of a nucleic acid selected from PRKX, TTBK2 and/or RSK4
for the manufacture of a medicament for the treatment of a proliferative disease.

The present invention further comprises a method of treating a proliferative disease comprising:
administering a subject in need thereof a therapeutically effective amount of a modulator for
   (i) the inhibition or activation of a polypeptide selected from PRKX, TTBK2 and/or RSK4 or for
   (ii) the inhibition, deactivation, degradation, intercalation, cleavage, up-regulation or down-regulation of a nucleic acid selected from PRKX, TTBK2 and/or RSK4.

The term "a subject in need thereof" refers to a cancer patient having the risk to develop a drug resistance against Sunitinib or to a cancer patient which has already developed a drug resistance against Sunitinib.
The term "a therapeutically effective amount" refers to an amount of a modulator sufficient to at least reduce in-vivo the drug resistance against Sunitinib.

Preferably, a modulator according to the invention is used in medicine and more preferred for the treatment of a proliferative disease selected from cancer, tumors and metastases.

The modulator according to the invention is preferably applied for the prophylaxis and the treatment of a proliferative disorder selected from the group consisting of acute lymphoblastic leukemia, acute myeloid leukemia, adrenocortical carcinoma, aids-related cancers, aids-related lymphoma, anal cancer, appendix cancer, astrocytomas, atypical teratoid/rhabdoid tumor, basal cell carcinoma, bile duct cancer, bladder cancer, bone cancer, osteosarcoma and malignant fibrous histiocytoma, brain stem glioma, brain tumor, central nervous system atypical teratoid/rhabdoid tumor, astrocytomas, craniopharyngioma, ependymoblastoma, ependymoma, medulloblastoma, medulloepithelioma, pineal parenchymal tumors, supratentorial primitive neuroectodermal tumors and pineoblastoma, brain and spinal cord tumors, breast cancer, bronchial tumors, burkitt lymphoma, carcinoid tumor, carcinoid tumor, gastrointestinal tumor, central nervous system atypical teratoid/rhabdoid tumor, central nervous system (cns) lymphoma, cervical cancer, chordoma, chronic lymphocytic leukemia, chronic myelogenous leukemia, chronic myeloproliferative disorders, colon cancer, colorectal cancer, craniopharyngioma, cutaneous t-cell lymphoma, mycosis fungoides and sézary syndrome, endometrial cancer, ependymoblastoma, ependymoma, esophageal cancer, esthesioneuroblastoma, ewing sarcoma family of tumors, extracranial germ cell tumor, extragonadal germ cell tumor, extrahepatic bile duct cancer, intraocular melanoma, retinoblastoma, gallbladder cancer, gastric (stomach) cancer, gastrointestinal carcinoid tumor, gastrointestinal stromal tumor (gist), gastrointestinal stromal cell tumor, germ cell tumor (extracranial), germ cell tumor (extragonadal), germ cell tumor (ovarian), gestational trophoblastic tumor, glioma, hairy cell leukemia, head and neck cancer, heart cancer, hepatocellular (liver) cancer, histiocytosis, hodgkin lymphoma, hypopharyngeal cancer, intraocular melanoma, islet cell tumors (endocrine pancreas), kaposi sarcoma, kidney (renal cell) cancer, kidney cancer, langerhans cell histiocytosis, laryngeal cancer, leukemia (acute lymphoblastic), leukemia (acute myeloid), leukemia (chronic lymphocytic), leukemia (chronic myelogenous), leukemia (hairy cell), lip and oral cavity cancer, liver cancer, lung cancer (non-small cell), lung cancer (small cell), lymphoma (aids-related), lymphoma (burkitt), lymphoma (cutaneous t-cell), mycosis fungoides and sézary syndrome, lymphoma (hodgkin), lymphoma (non-hodgkin), lymphoma primary central nervous system (cns), macroglobulinemia, malignant fibrous histiocytoma of bone and osteosarcoma, medulloblastoma, medulloepithelioma, melanoma, melanoma intraocular (eye), merkel cell carcinoma, mesothelioma, metastatic squamous neck cancer with occult primary, mouth cancer, multiple endocrine neoplasia syndromes, multiple myeloma/plasma cell neoplasm, mycosis fungoides, myelodysplastic syndromes, myelodysplastic/myeloproliferative neoplasms, myelogenous leukemia, myeloid leukemia, myeloma (multiple), myeloproliferative disorders, nasal cavity and paranasal sinus cancer, nasopharyngeal cancer, neuroblastoma, non-hodgkin lymphoma, non-small cell lung cancer, oral cancer, oral cavity cancer, oropharyngeal cancer, osteosarcoma and malignant fibrous histiocytoma of bone, ovarian cancer, ovarian epithelial cancer, ovarian germ cell tumor, ovarian low malignant potential tumor, pancreatic cancer, papillomatosis, paranasal sinus and nasal cavity cancer, parathyroid cancer, penile cancer, pharyngeal cancer, pineal parenchymal tumors of intermediate differentiation, pineoblastoma and supratentorial primitive neuroectodermal tumors, pituitary tumor, plasma cell neoplasm/multiple myeloma, pleuropulmonary blastoma, pregnancy and breast cancer, primary central nervous system (cns) lymphoma, prostate cancer, rectal cancer, renal cell (kidney) cancer, transitional cell cancer, respiratory tract cancer, retinoblastoma, rhabdomyosarcoma, salivary gland cancer, sarcoma (ewing sarcoma), sarcoma (kaposi), sarcoma (soft tissue), sarcoma (uterine), sézary syndrome, skin cancer (non-melanoma), skin cancer (melanoma), skin carcinoma (merkel cell), small cell lung cancer, small intestine cancer, soft tissue sarcoma, squamous cell carcinoma, squamous neck cancer, stomach (gastric) cancer, supratentorial primitive neuroectodermal tumors, t-cell lymphoma, mycosis fungoides and sézary syndrome, testicular cancer, throat cancer, thymoma and thymic carcinoma, thyroid cancer, transitional cell cancer of the renal pelvis and ureter, trophoblastic tumor, gestational, ureter and renal pelvis, transitional cell cancer, urethral cancer, uterine cancer, endometrial, uterine sarcoma, vaginal cancer, vulvar cancer, waldenström macroglobulinemia and wilms tumor. It is especially preferred if the proliferative disease is selected from kidney cancer, breast cancer or melanoma.

A modulator according to the invention may be selected from:
(i) nucleic acids, in particular small interfering RNA (siRNA), micro RNA (miRNA) or a precursor thereof, oligonucleotide aptamers, anti-sense oligonucleotides, or ribozymes;
(ii) peptidic compounds, in particular antibodies or antibody fragments or peptidic aptamers;
(iii) small organic non-peptidic molecules, i.e. molecules having a low molecular weight; and
(iv) combinations thereof.

Such compounds or agents may have the ability to specifically modulate at least one target as described herein, e.g. due to binding to the at least one target, and can therefore be used for detecting, preventing, treating and/or monitoring conditions associated with, accompanied by and/or caused by the occurrence of drug-resistance or drug-desensitivity of hyperproliferating cells, preferred sunitinib resistance or desensitivity.

According to the invention the modulator may be an inhibitor acting on the protein level or the modulator may be an antibody or an antigen-binding fragment or a derivative thereof or the modulator may be an aptamer or the modulator may be an inhibitor acting on the nucleic acid level or the modulator may be an antisense molecule, a ribozyme, a siRNA molecule or a miRNA molecule or a precursor thereof or the modulator is Sunitinib.

In one embodiment, the modulator is directed against a target polypeptide, i.e. a modulator acting on the protein level. Further, the modulator may be directed against a portion of a target polypeptide, e.g. an extracellular and/or soluble fragment of said target polypeptide. Preferably, the modulator is a peptidic compound, e.g. an antibody or an antibody fragment. Such a modulator preferably acts as an inhibitor of the target polypeptide. In the context of the present invention, the term "antibody" covers monoclonal antibodies, polyclonal antibodies, multispecific antibodies (e.q. bispecific antibodies) formed from at least two antibodies, antibody fragments and derivates thereof as long as they exhibit the desired activity. The antibody may be an IgM, IgG, e.g. IgG1, IgG2, IgG3 or IgG4. Antibody fragments comprise a portion of an antibody, generally the antigen binding or variable region of the intact antibody. Examples of antibody fragments include Fab, Fab', F (ab') 2 and Fv fragments, diabodies, single chain antibody molecules and multispecific antibody fragments.

Particularly, the antibody may be a recombinant antibody or antibody fragment, more particularly selected from chimeric antibodies or fragments thereof and diabodies. For therapeutic purposes, particularly for the treatment of humans, the administration of chimeric antibodies, humanized antibodies or human antibodies is especially preferred.

The antibodies according to the invention may be coupled to a labeling group, particularly for diagnostic applications, e.g. the detection of sunitinib resistance. Examples for suitable labeling groups such as radioactive groups, fluorescent groups or other labeling groups are known in the art.

A modulator directed against a target polypeptide, i.e. modulator acting on protein level, may be an aptamer, i.e. an oligonucleic acid, a peptide molecule or a combination thereof that specifically binds to the target polypeptide. An oligonucleic acid aptamer may have a sequence length of between about 20-100 nucleotides, preferably about 25-75, more preferably about 30-50 nucleotides. A peptide aptamer generally consists of a variable peptide loop attached at both ends to a protein scaffold. The variable loop length is between 5 and 50, preferably about 10-30, and more preferably about 10-20 amino acids. An aptamer according to the invention may be coupled to a labeling group, particularly for diagnostic applications. Examples for suitable labeling groups such as radioactive groups, fluorescent groups or other labeling groups are known in the art. Further, particularly for therapeutic applications, the aptamer may be coupled to an effector group, which may be effective in the treatment of proliferative diseases, e.g. a cytotoxic group such as a radioactive group, toxin or another effector group as known in the art.

Aptamers are mostly short single stranded DNA- or RNA-molecules, which can bind a specific molecule because of their 3D-structure. Aptamers may be prepared by chemical synthesis and selected by a systematic evolution of ligands due to exponential enrichment as known by the person skilled in the art.

Further, the modulator may be a small organic non-peptidic molecule. Such a molecule has a low molecular weight between 100 and 1000 g·mol⁻¹ and preferred between 300 and 500 g·mol⁻¹. Said molecule may act as an inhibitor of an enzymatic activity of a kinase, e.g. a serine-threonine kinase. The term small molecule refers to low molecular weight organic compound which is by definition not a polymer. It the field of pharmacology, it is usually restricted to a molecule that also binds with high affinity to a biopolymer such as proteins, nucleic acids, or polysaccharides. Small molecules are broadly used as enzyme inhibitors, thus they are preferred modulators for the inhibition of the preferred target polypeptides in the present invention.

In a preferred embodiment, the modulator is an antibody, an antigen-binding fragment thereof or an aptamer directed against a PRKX, TTBK2 and/or RSK4 polypeptide as defined above or against a portion thereof, e.g. an extracellular and/or soluble fragment of said polypeptide. Such an antibody is preferably monoclonal.

A monoclonal antibody may be obtained by the hybridoma method as described by Köhler et al. (Nature 256 (1975), 495) or by recombinant DNA methods (cf. e.g. US patent 4,816,567). Monoclonal antibodies may also be isolated from phage antibody libraries using techniques which are known to the person skilled in the art.

In a further embodiment, the modulator according to the invention is directed against a target nucleic acid, i.e. modulator acting on the nucleic acid level. Preferably, the modulator is a nucleic acid compound, e.g. RNAi inducing molecules like siRNA, miRNA, anti-sense oligonucleotide, ribozyme, a precursor or a combination thereof.

A RNAi-inducing molecule may refer to a nucleic acid molecule, wherein at least one polynucleotide strand of said nucleic acid molecule has a sequence which is sufficiently complementary to a target RNA, preferably to a target mRNA, in order to effect its processing, i.e. its decomposition. In order to have an RNAi-inducing effect, it is necessary that the complementarity between the RNAi-inducing molecule and a region of the target RNA is sufficient, in order to effect a hybridization and a subsequent processing. For example, the complementarity is at least 80 %, preferably at least 90 % and most preferably at least 99 %, whereby the 5'- and/or 3'-ends as well as the overhangs of an RNAi-effector molecule may also contain nucleotides which are not complementary to the target RNA.

SiRNA (small interfering RNA or short interfering RNA or silencing RNA) used according to the invention is a double-strand of RNA and/or nucleotide analogues with 3' overhangs on at least one end, preferably either ends. Each RNA strand of the double-strand has a 5' phosphate group and a 3' hydroxyl group. Preferably, each RNA strand of the double strand is 19 to 30 nucleotides long, more preferably 20 to 28 nucleotides and most preferably 21 to 23 nucleotides. The 3' overhang on the end of a RNA strand is preferably 2 nucleotides long. In a particular preferred embodiment the siRNA double-strand consists of two 21 nucleotides long RNA strands each having a 2 nucleotides long 3' overhang. SiRNA molecules further refer to single-stranded RNA-molecules having a length of 19-30 nucleotides, preferably 20-28 nucleotides and particularly having a length of 21-23 nucleotides, whereby the single-stranded RNA molecule is for at least 80%, preferably for at least 90% and more preferably for more than 99% complementary to a sequence of a target RNA, in particular of a target mRNA, and a binding of siRNA to the target RNA effects a sequence specific decrease. Preferably, siRNA molecules have overhangs of 1-3 nucleotides on the 3' end. Methods for obtaining siRNA molecules are known to the person skilled in the art.

MiRNA is a single- or double-stranded RNA molecule of 19-30, preferably 20-28, and more preferably 21-23 nucleotides in length, which can regulate gene expression. MiRNA is generally synthesized at first as a precursor, which is then processed to the major form having a sequence which is at least partially complementary to messenger RNA of a target molecule according to the invention.

An antisense oligonucleotide may be a single, double, or triple-stranded DNA, RNA, PNA (peptide nucleic acid) or a combination thereof (e.g. hybrids of DNA and RNA strands) having a length of between about 10-100, preferably 20-50, and more preferably 20-30 nucleotides in length, which can interfere with mRNA targets by hybrid formation and therefore inhibit translation of said mRNA.

Ribozymes are catalytic RNAs which possess a well defined structure that enables them to catalyze a chemical reaction. Apart from naturally occurring ribozymes they can be made artificially and be tailored to interact with nucleic acids and proteins. Ribozymes are also preferred modulators for inhibition of the preferred kinases in the present invention.

Precursor molecules, e.g. precursor molecules of siRNA and/or miRNA may be a substrate for the siRNA/miRNA-biogenesis-apparatus of the target cell. This comprises, for example, RNA precursor molecules such as double-stranded RNA (dsRNA) or short hairpin RNA-molecules (shRNA), which are processed by enodribonucleases such as Drosha and/or Pasha to siRNA-molecules or miRNA-molecules, respectively. Dicer is another endoribonuclease that cleaves double-stranded RNA and pre-microRNA (miRNA) into siRNA about 20-25 nucleotides long, usually with a two-base overhang on the 3' end. Dicer catalyzes the first step in the RNA interference pathway and initiates formation of the RNA-induced silencing complex (RISC). The RISC complex with a bound siRNA recognizes complementary messenger RNA (mRNA) molecules and degrades them, resulting in substantially decreased levels of protein translation and effectively turning off the gene.

DsRNA-molecules or short hairpin RNA-molecules (shRNA) having a length of more than 27 nucleotides, preferably more than 30 up to 100 nucleotides or longer, and mostly preferred dsRNA-molecules having a length of 30-50 nucleotides, can be used.

Further precursor molecules according to the invention may be DNA constructs encoding dsRNA, shRNA, siRNA and/or miRNA, whereby the coding elements are controlled by regulatory elements allowing an expression of dsRNA, shRNA, siRNA and/or miRNA in the target cell. Examples for such control elements are polymerase II promoters or polymerase III promoters such as, for example, U6 or H1.

SiRNA, miRNA, ribozymes and antisense oligonucleotides may be coupled with a labeling group, e.g. for diagnostic purposes or may be coupled with an effector molecule known in the art and they may be applied to a target cell by any technique which is known to a person skilled in the art, such as transfection of exogenous siRNA, miRNA, ribozyme and antisense oligonucleotide or of an appropriate vector, e.g. viral or non-viral, producing a single transcript which can be processed into a functional siRNA, miRNA, ribozyme or antisense oligonucleotide.

A modulator molecule acting on the nucleic acid level as described above may exhibit analogs of one or more nucleotides within its nucleotide sequence. Said nucleotide analogs may, for example, increase the structural stability of the RNA molecule or the stability towards ribonucleases. Ribonucleotide analogs are well known to the person skilled in the art and are modified compared to the original RNA molecules by base modification, sugar modification, e.g. modification of the 2'-OH group of ribose and/or phosphate backbone modifications.

In a preferred embodiment of the invention, the modulator is an inhibitor of the expression of a target nucleic acid, which preferably acts by down-regulation or knock-down of the target. Down-regulation or knock-down of the target results preferably in a reduction of the steady state level of the target mRNA or polypeptide by at least factor 2, 5, 10 or more, or in a disappearance of the target from the cell. In a particularly preferred embodiment, the expression of a target nucleic acid selected from the group consisting of PRKX, TTBK2 and/or RSK4 is inhibited.

Mostly preferred, the modulator acting by down-regulation or knock-down of the target expression is a siRNA, miRNA, anti-sense molecule, or precursor thereof.

A modulator as defined above, e.g. a modulator directed to a target polypeptide and/or a nucleic acid selected from PRKX, TTBK2 and/or RSK4 increases the inhibition by sunitinib of cell proliferation, migration and/or invasion of cancer cells, wherein said cancer cells are preferably characterized in that they exhibit an over-expression of at least one of said target molecules. Preferably, the cancer is selected from kidney cancer, breast cancer, melanoma, glioblastoma, non-small cell lung cancer (NSCLC) or metastatic renal carcinoma (mRCC).

In another embodiment, the modulator may be an inhibitor acting on the protein level or wherein the modulator is an antibody or an antigen-binding fragment or a derivative thereof or wherein the modulator is an aptamer or wherein the modulator is an inhibitor acting on the nucleic acid level or wherein the modulator is an antisense molecule, a ribozyme, a siRNA molecule or a miRNA molecule or a precursor thereof.

Such a modulator increases sunitinib effect on cancer cells characterized in that they exhibit a reduced or impaired expression of at least one of said target molecules.

As outlined above, a modulator according to the invention or a composition comprising at least one of such a modulator are suitable for enhancing the effect of sunitinib, e.g. inhibiting hyperproliferation and can therefore be used in human medicine, e.g. for the treatment or prevention of proliferative disease, i.e. conditions associated with, accompanied by and/or caused by the occurrence of hyperproliferation, e.g. cancer, tumor or metastases.

For use as a medicament, the modulator or composition may be formulated as a pharmaceutical composition. Therefore still another aspect of the present invention deals with pharmaceutical compositions comprising at least one modulator as defined above as an active ingredient, together with at least one pharmaceutically acceptable carrier, excipient and/or diluents. The pharmaceutical compositions of the present invention can be prepared in a conventional solid or liquid carrier or diluents and a conventional pharmaceutically-made adjuvant at suitable dosage level in a known way. The preferred preparations are adapted for oral application. These administration forms include, for example, pills, tablets, film tablets, coated tablets, capsules, liposomal formulations, micro- and nano-formulations, powders and deposits as well as formulations for inhalation.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one modulator according to the present invention and/or a pharmaceutically acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, aerosol preparations consistent with conventional pharmaceutical practices. Other suitable formulations are gels, elixirs, dispersable granules, syrups, suspensions, creams, lotions, solutions, emulsions, suspensions, dispersions, and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Common pharmaceutically acceptable carrier, excipient and/or diluents can be used. Carriers like lactose, starch, sucrose, cellulose, magnesium stearate, dicalcium phosphate, calcium sulfate, talc, mannitol, ethyl alcohol (liquid filled capsules) can be used. Suitable binders include starch, gelatin, natural sugars, corn sweeteners, natural and synthetic gums such as acacia, sodium alginate, carboxymethylcellulose, polyethylene glycol and waxes. Suitable excipients are sugars like for instance sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. Further substances can be used like lubricants such as boric acid, sodium benzoate, sodium acetate, sodium chloride, magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine; disintegrating agents (disintegrates) such as starch, methylcellulose, guar gum, modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures; coloring agents, sweetening agents, flavoring agents, preservatives; glidents are for example silicon dioxide and talc; suitable adsorbent are clay or aluminum oxide. Suitable diluents are water or water/propylene glycol solutions for parenteral injections. Sugars such as lactose, sucrose, mannitol, and sorbitol are used in juice or other oral liquid formulations and starches derived from wheat, corn rice, potato and celluloses such as microcrystalline cellulose are used in solid oral formulations. The medicament comprising a modulator as described above can be formulated to be suitable for any known dosage form.
The composition may be administered by one dose per day or may be divided up to several doses. Furthermore, one may administer the pharmaceutical composition of the present invention in a targeted drug delivery system, for example, in a liposome coated with a tumor-specific antibody. The liposomes will be targeted to and taken up selectively by the hyperproliferating cells of a target tissue.

The effective amount of the active agent, i.e. the modulator, in the composition may be determined by the skilled person without undue burden depending on the kind of active agent and the kind of conditions to be treated. For example, about 1 µg/kg to 15 mg/kg of a modulator may be administered to a human patient, e.g. by one or more separate administrations or by continuous infusion. A typical daily dosage may range from about 1 µg/kg to about 100 mg/kg or more, depending on the factors such as age, gender, weight, tumor-thickness and condition of the person to be treated etc.. For repeated administrations or several days or longer, depending on the condition to be treated, the treatment is sustained until a desired suppression of disease symptoms occurs.

A pharmaceutical composition as defined in the present invention may be further administered as a part of a combination therapy or combinatorial therapy. In the context of the present invention, "combination therapy" or "combinatorial therapy" also refers to the simultaneous administration of two or more active agents, wherein at least one of these agents is a modulator of PRKX, TTBK2 and/or RSK4 according to the present invention. The two or more active agents may be administered simultaneously in one single pharmaceutical composition or more than one pharmaceutical composition, wherein each composition comprises at least one active agent. However, the term "combination therapy" or "combinatorial therapy" refers also to other types of therapy such as radiation which are used at the same time. A preferred embodiment of the invention relates to a pharmaceutical composition for the treatment of patients over-expressing PRKX, TTBK2 and/or RSK4.

The antiproliferative agents as defined by the present invention which are administered together with the modulator are preferably selected from the group comprising or consisting of: sorafinib (Nexavar^{®}), chlorethamine, cyclophosphamide (cytoxan), trofosfamide, ifosfamide (Ifex), melphalan (Alkeran), mechlorethamine hydrochloride (Mustargen), chlorambucil (Leukeran), busulfan (Myleran), thiotepa, 2-cyano-3-(3,4-dihydroxyphenyl)-N-(phenylmethyl)-2-propenamide, carmustine, carboplalomustine, dacarbazine, procarbazine, erlotinib, temozolomide, treosulfan, estramustine, N-(3-(5-chloro-2-(2-methoxy-4-(4-methylpiperazin-1-yl)phenylamino)pyrimid in-4-yloxy)phenyl)acrylamide, nimustine, methotrexate (MTX, Mexate), 5-fluorouracil (Fluoracil, 5-FU), 6-thioguanine (Thioguanine, 6-TG), 6-mercaptopurine, (N-[-4-[(3-Chloro-4-fluorophenyl)amino]-7-[3-(4-morpholinyl)propoxy]-6-quinazolinyl]-2-propenamide), sunitinib, N-(3-chlorophenyl)-6,7-dimethoxy-4-quinazolinamine, fludarabine (Fludara), floxuridine (FUDR), cladribine, pentostatin, gemcitabine (Gemzar), cytarabine, azathioprine, raltitrexed, capecitabine (Xeloda), 4-phenethylamino-6-(yderoxyl)phenyl-7H-pyrrolo(2,3-d)pyrimidine, cytosine arabinoside, deoxycoformycin (Pentostatin, Nipent), lapatinib, thioguanine, 7-(4-(3-ethynylphenylamino)-7-methoxyquinazolin-6-yloxy)-N-hydroxyheptanamide, mercaptopurine (6-MP, Purinethol), paclitaxel, docetaxel, hydroxycarbamide (hydroxyurea), imatinibe, Miltefosine^{®}, N-(3-(5-chloro-2-(4-(4-methylpiperazin-1-yl)phenylamino)pyrimidin-4-yloxy)phenyl)acrylamide, cetuximab, amsacrine, 4-[(3-Bromophenyl)amino]-6,7-dimethoxyquinazoline hydrochloride), pentostatin, bexarotene, tretinoin, asparaginase, trastuzumab (also known as Herceptin^{®}), alemtuzumab (also known as MabCampath^{®}), rituximab (also known as MabThera^{®}), N-[4-[(3-Chloro-4-fluorophenyl)amino]-7-[[(3"S")-tetrahydro-3-furanyl]oxy]-6-quinazolinyl]-4(dimethylamino)-2-butenamide, glucocorticoids (prednisone), hydrocortisones, gefitinib, dexamethasones (Decadron), (E)-N-(4-((3-chloro-4-fluorophenyl)amino)-3-cyano-7-ethoxyquinolin-6-yl)-4-(dimethylamino)but-2-enamide, estrogens [fosfestrol, estramustine (Emcyt), chlorotrianisene (TACE), diethylstilbestrol (DES)], (R)-6-(4-((4-methylpiperazin-1-yl)methyl)phenyl)-N-(1-phenylethyl)-7H-pyrrolo[2,3-d]pyrimidin-4-amine, aromatase inhibitors [anastrozole (Arimidex)], LHRH (buserelin, goserelin, leuprorelin, triptorelin), N-(3-Chloro-4-((3-fluorolphenyl)methoxy)phenyl)-6-(5-(((2-(methylsulfonyl)ethyl)amino)methyl)-2-furanyl)-4-quinazolinamine bis(4-methylbenzenesulfonate), flutamide (Eulexing), bicalutamide (Casodex), leuprolide (Lupron), goserelin acetate (Zoladex), [4-[[1-(3-fluorophenyl)methyl]-1H-indazol-5-ylamino]-5-methylpyrrolo[2,1-f][1,2,4]triazin-6-yl]carbamic acid (3S)-3-Morpholinylmethyl ester, vandetanib, cyproterone acetate, N-(3-(5-chloro-2-(4-(4-methylpiperazin-1-yl)phenylamino)pyrimidin-4-ylthio)phenyl)-acrylamide, panitumumab, progestine (Medoxyprogesterone acetate [(Depoprovera)], megestrol acetate (Megacel), tamoxifen and toremifen, daunorubicin (Daunomycin, Cerubidine), doxorubicin (adriamycin), liposomal adriamycin, dactinomycin, mitomycin C, (R,Z)-5-((2,6-dichlorobenzyl)sulfonyl)-3-((3,5-dimethyl-4-(2-(pyrrolidin-1-ylmethyl)pyrrolidine-1-carbonyl)-1 H-pyrrol-2-yl)methylene) indolin-2-one, bleomycin (Blenoxane), epirubicin (4-epi-adriamycin), idarubicin (idamycin), AMG102 (Amgen, Inc), dactinomycin (Actinomycin D, Cosmegen), mitoxantrone (Novantrone), AMG-208 (Amgen, Inc), mitomycin C (Mitomycin), plicamycin (Mithracin), amsacrine, actinomycin D, vincristine, vinblastine, SCH900105 (AV-299) (Aveo Pharmaceuticals Inc.), vindesine, etoposide, XL184 (Exelixis, Inc), N-((2R)-1,4-Dioxan-2-ylmethyl)-N-methyl-N'-[3-(1-methyl-1H-pyrazol-4-yl)-5-oxo-5H-benzo[4,5]cyclohepta[1,2-b]pyridin-7-yl]sulfamide, PF-04254644 (Pfizer, Inc), N-(1,3-Benzodioxol-5-ylmethyl)-4-benzofuro[3,2-D]pyrimidin-4-yl-1-piperazinecarbothioamide, teniposide, cisplatin, carboplatin, 9S-(9α,10β,12α))-2,3,9,10,11,12-hexahydro-10-hydroxy-10-(methoxycarbonyl)-9-methyl-9,12-epoxy-1 H-diindolo-[1,2,3-fg:3',2',1'-kl]pyrrolo[3,4-i][1,6]benzodiazocin-1-one, oxaliplatin, vinorelbine, etoposide (VP-16, Etopophos, VePesid), ARQ197 (ArQule, Inc), teniposide (VM-26, Vumon) topotecan (Hycamtin), irinotecan (Camptosar), carmustine (BCNU), lomustine (CCNU), JNJ-38877605 (Johnson & Johnson, Inc), streptozocin (Zanosar), 1,3-Dihydro-3-[(3,5-dimethyl-1H-pyrrol-2-yl)methylene]-2H-indol-2-one, camptothecin, topotecan, SGX-523 (SGX Pharmaceuticals, Inc), irinotecan, aminoglutethimide, formestane, (3Z)-N-(3-Chlorophenyl)-3-({3,5-dimethyl-4-[(4-methylpiperazin-1-yl)carbonyl]-1H-pyrrol-2-yl}methylene)-N-methyl-2-oxo-2,3-dihydro-1 H-indole-5-sulfonamide, XL880 (Exelixis, Inc), exemestane, letrozole, anastrozole, MGCD-265 (MethylGene, Inc), interleukin-2, interferon-α, genestein, erythropoietin, G-CSF, ibritumomab (Zevalin^{®}), levamisole, asparaginase (Elspar), pegaspargase (Oncaspar), hydroxyurea (Hydrea, Mylocel), 2-(4-(1-(quinolin-6-ylmethyl)-1H-[1,2,3]triazolo[4,5-b]pyrazin-6-yl)-1H-pyrazol-1-yl)ethanol, procarbazine (Matulane) and imatinib mesylate (Gleevec), Neovastat, bevamizumab, avastin, angiozyme, 3-[(1R)-1-(2,6-dichloro-3-fluorophenyl)ethoxy]-5-(l -piperidin-4-ylpyrazol-4-yl)pyridin-2-amine, endostatin, angiostatin, combrestatin, vitaxin, geldanamycin carboxyamido thiazole, celecoxib and tirapazamine.

The invention further refers to a composition comprising a modulator of PRKX, TTBK2 and/or RSK4 and one further antiproliferative agent. It is preferred if the composition comprises sunitinib as the further antiproliferative agent.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

The invention will be described in further detail by the following figures and examples.

### Figure legends

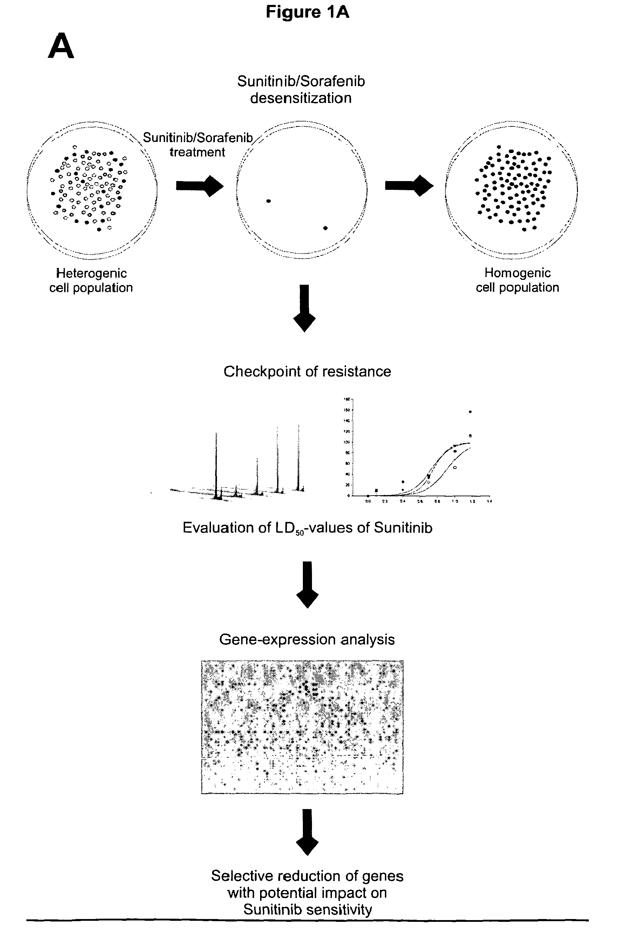

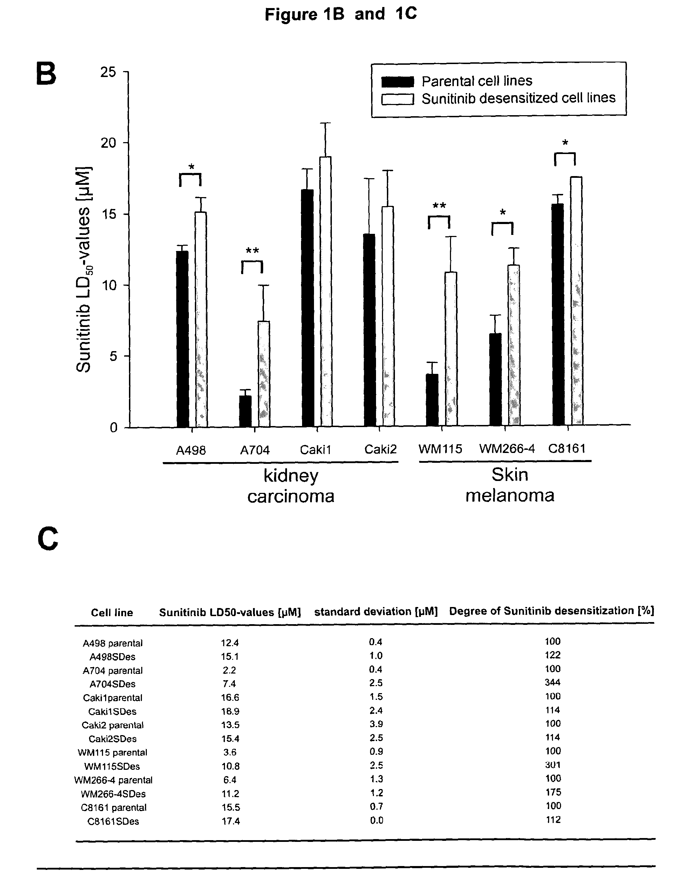

**Figure 1****:** Apoptosis inducing effect of Sunitinib in parental and Sunitinib desensitized kidney carcinoma- and melanoma cell lines. (A) Workflow: Parental, heterogenic cell populations were treated with Sunitinib or Sorafenib (20 µM) to generate homogenic Sunitinib or Sorafenib desensitized cell populations. The desensitization was monitored by measuring the half maximal lethal dose (LD₅₀-values) of Sunitinib and Sorafenib. Comparative gene-expression analyses were used to identify genes with a potential function in triggering Sunitinib insensitivity. Specific reduction of candidate-genes was used to determine functional relevance in Sunitinib resistance formation. (B) The LD₅₀-values for Sunitinib treatment vary between the parental cell lines independent of the cancer type. Compared to the parental cell lines, the Sunitinib desensitized cell lines showed alternating LD₅₀-values for Sunitinib treatment. The degree of desensitization against Sunitinib treatment varied within the Sunitinib desensitized cell lines. (C) List of the LD₅₀-values for Sunitinib treatment of parental and desensitized kidney carcinoma and melanoma cell lines after 72 h. Black bars: parental; grey bars: Sunitinib desensitized.

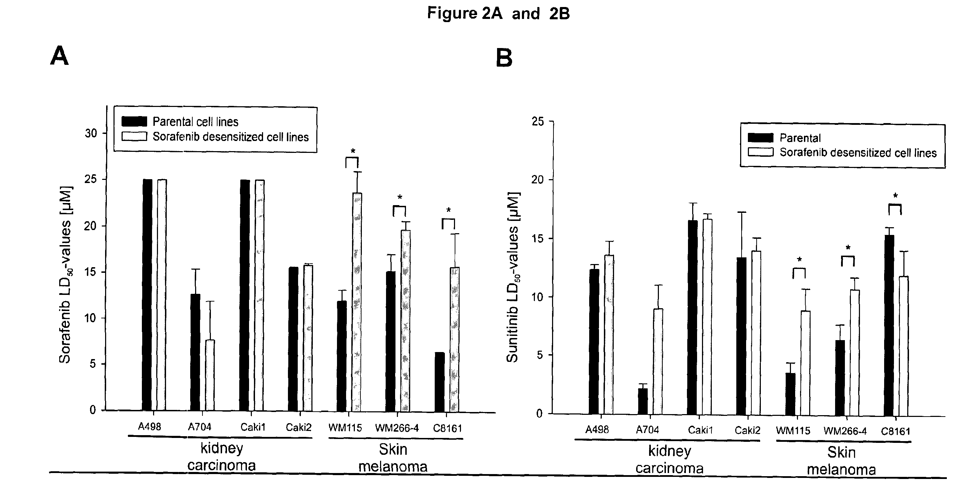

**Figure 2****:** Apoptosis inducing effect of Sunitinib and Sorafenib in parental- and Sorafenib desensitized kidney carcinoma- and melanoma cell lines. (A) No Sorafenib desensitized cell populations could be selected with Sorafenib treatment out of parental kidney cell lines. The parental kidney cell lines A498 and CAKI1 demonstrated Sorafenib insensitivity. In comparison to the parental melanoma cell lines, the respective Sorafenib desensitized cell lines showed alternating LD₅₀-values for Sorafenib and (B) Sunitinib. Black bars: parental-; grey bars: Sorafenib desensitized cell lines.

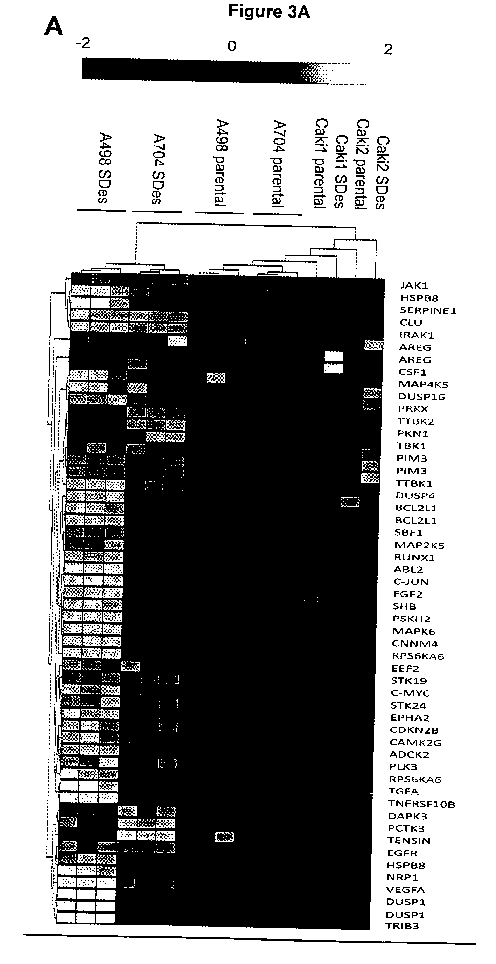

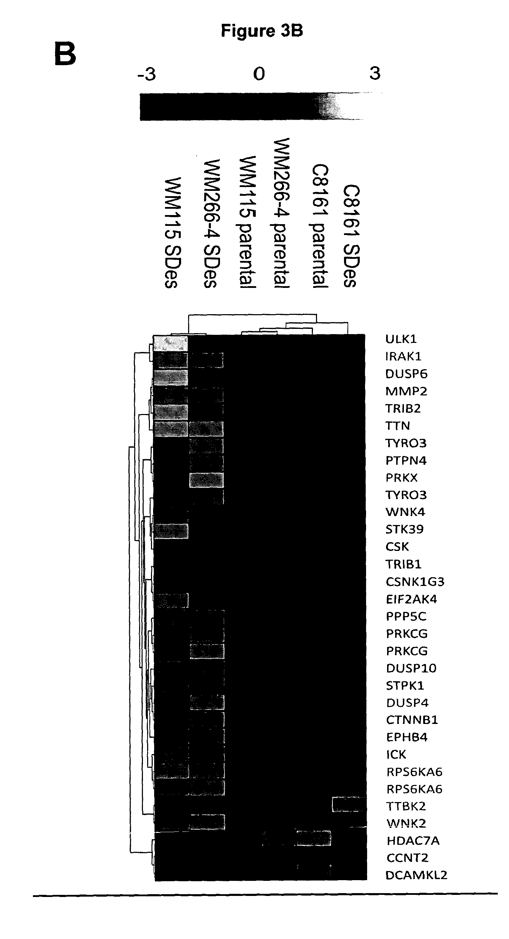

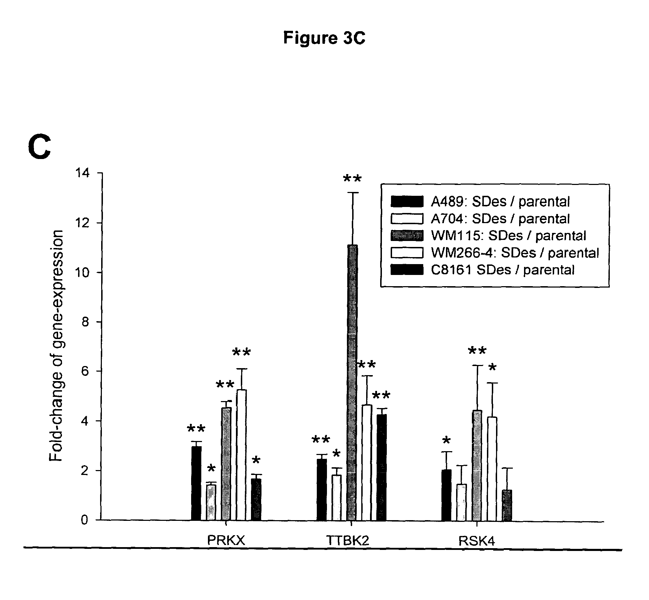

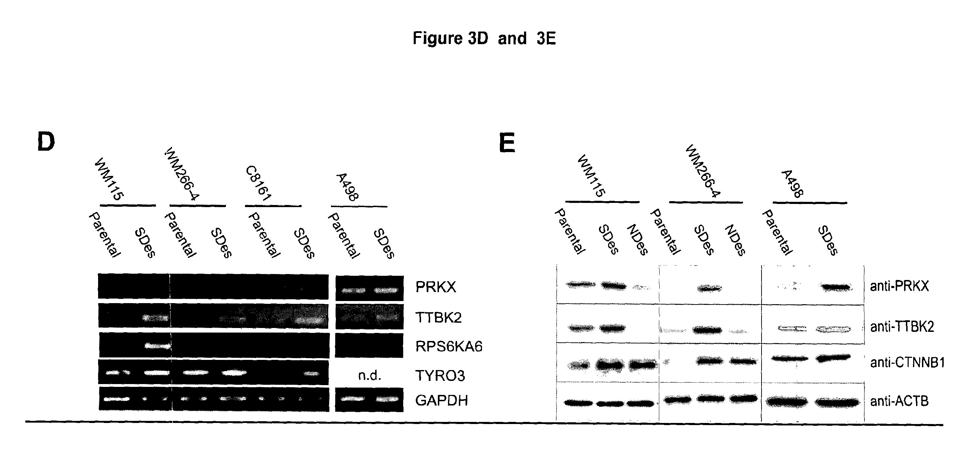

**Figure 3****:** Comparative expression analysis of parental and Sunitinib desensitized (SDes) cell lines.
   Significance Analysis of Microarrays (SAM) of kidney- (A) and melanoma (B) cell populations. SAM revealed subsets of genes that correlated with the degree of Sunitinib desensitization. Five genes displayed this correlation in both kidney carcinoma- and melanoma cell lines: IRAK1, PRKX, TTBK2, RSK4 and DUSP4. (C) Fold-changes of the gene-expression of PRKX, TTBK2 and RPS6KA6/RSK4 in Sunitinib desensitized melanoma cell lines and A498^{SDes}. (D) Semi-quantitative RT-PCR analysis validated gene-array results for four kinases of particular interest. Gene-expression was translated into (E) protein-expression for PRKX, TTBK2 and CTNNB1. Color code: Gene-expression scale from log2 (-2) to log2 (2) (kidney) and log2 (-3) to log2 (3) (melanoma), yellow to blue. Gene-names highlighted in red displayed a similar correlation of gene-expression and Sunitinib desensitization in kidney carcinoma- and melanoma cell lines. Abbreviations: n.d.; not determined.

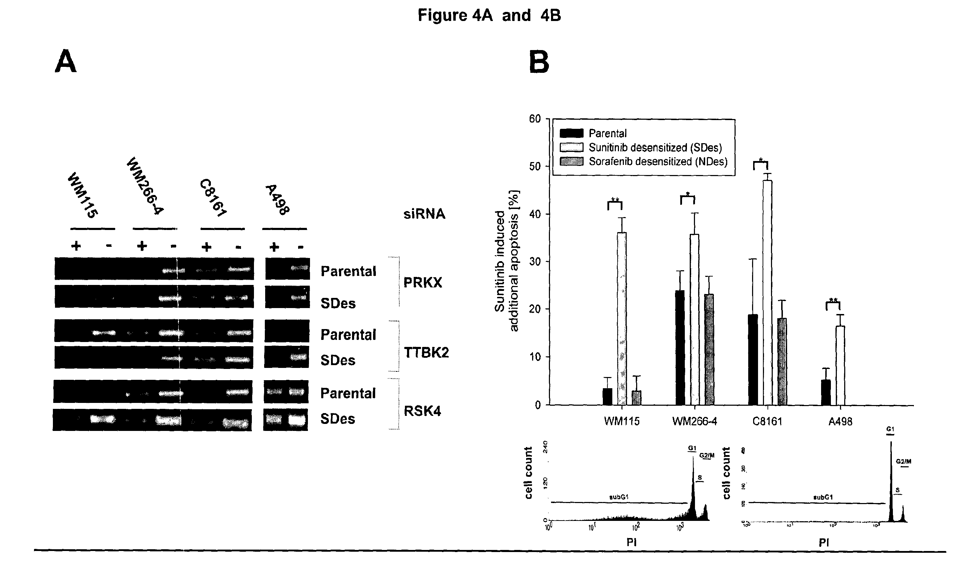

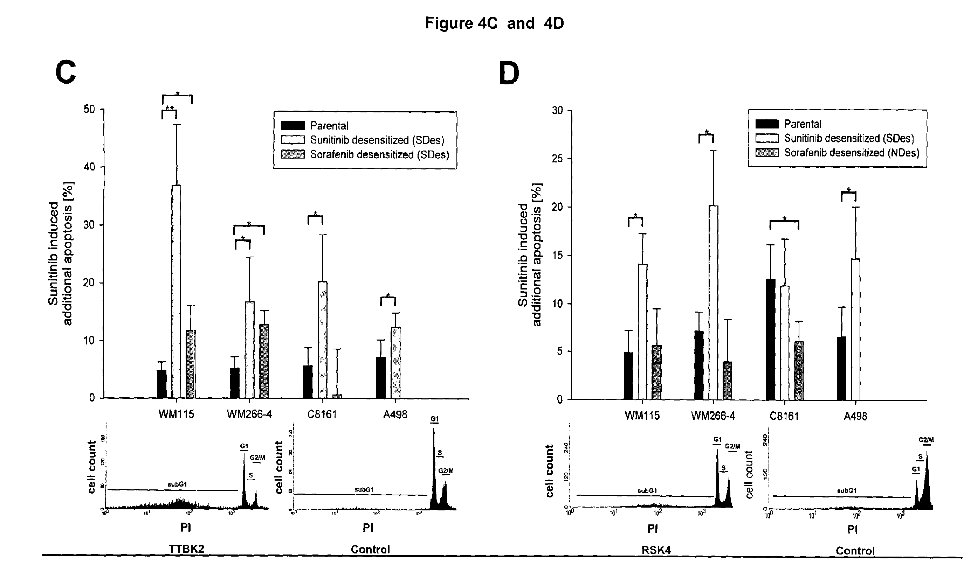

**Figure 4****:** PRKX, TTBK2 or RSK4 reduction increases Sunitinib induced apoptosis in correlation to the endogenous gene-expression.
   (A) Knock-down efficiency was verified by RT-PCR 96 h after transfection, ranging between 50 % and 90 %. Upon (B) PRKX or (C) TTBK2 reduction, a Sunitinib sensitizing- and a strikingly higher re-sensitizing effect was measured in the parental- and the corresponding Sunitinib desensitized cell lines. The effect of PRKX reduction in the Sorafenib desensitized melanoma cell lines matched the one within the parental cell lines. TTBK2 reduction resulted in an additional Sunitinib induced apoptosis, ranging in between the parental- and Sunitinib desensitized cell lines WM115^{SDes} and WM266-4^{SDes}. In C8161^{NDes} cells, the effect upon TTBK2 reduction matched the one of the parental cell line. (D) RSK4 reduction caused Sunitinib sensitization in the parental and a higher re-sensitization in the Sunitinib desensitized cell lines. While within WM115^{NDes} and WM266-4^{NDes} the re-sensitizing effect matched the one of the parental cell lines, within C8161^{NDes}, the effect was weaker. Results of one representative experiment are shown in the lower panels of (A), (B) and (C). Note that y-axes differ in the displayed bar charts. Black bars: parental; light grey bars: Sunitinib desensitized; dark grey bars: Sorafenib desensitized.
**Figure 5****:** Cell morphology is not changed due to the Sunitinib desensitization process.
   No changes in shape or size due to the Sunitinib desensitization process were observed in the cell lines WM115, WM266-4, C8161 and A498. Magnification: 40X
**Figure 6****:** Reduction of PRKX, TTBK2 or RSK4 increases Sunitinib increases Sunitinib inhibition of cancer cell migration.
   (A) Microscopic visualization shows the increased anti-migratory effect of PRKX, TTBK2 or RSK4 reduction on Sunitinib treatment in the parental and Sunitinib desensitized cell line A498. The effect was highest for PRKX, followed by TTBK2 and RSK4 reduction. (B) Bars depict the quantification of increased Sunitinib induced anti-migratory effects due to gene-expression reduction. Data shown are the means ± standard deviation of two independent experiments. black bars: parental; grey bars: Sunitinib desensitized.

### Examples

### Example 1: Sunitinib induces apoptosis in kidney carcinoma and melanoma cell lines

To establish appropriate cell systems for the investigation of Sunitinib resistance formation, we identified four kidney carcinoma (A498, A704, CAKI1 and CAKI2) and three melanoma cell lines (WM115, WM266-4, and C8161) based on their apoptosis response to drug treatment. This effect was monitored by Propidium iodide- (PI) assays and displayed as half maximal lethal dose-(LD₅₀) values after Sunitinib treatment for 72h.

### Cell culture

Human kidney carcinoma and melanoma cell lines A498, A704, CAKI1, CAKI2, WM115, WM266-4 and C8161 were provided by M. Herlyn (Wistar Institute, Philadephia) and R. Gillies (Arizona Cancer Center, Phoenix) or purchased from the American Tissue Culture Collection. CAKI1, CAKI2 and C8161 cells were cultured in Dulbecco's modified Eagle's medium (DMEM; Gibco Life Technologies Inc.), supplemented with 10 % fetal calf serum (FCS), 2 mM glutamine, and 1 % nonessential amino acids. A498, A704 and WM115 cells were cultured in Minimum Essential Medium (MEM; Gibco Life Technologies Inc.) supplemented with 10 % FCS, 2 mM glutamine, and 1 % (v/v) nonessential amino acids. WM266-4 cells were cultured in RPMI medium (Gibco Life Technologies Inc.), supplemented with 10 % FCS and 2 mM glutamine.

### Characterization of cell line sensitivity to Sunitinib

To analyze Sunitinib sensitivity of the cell lines, equal cell numbers were seeded into 12-well plates and allowed to attach over night. The cells were treated with Sunitinb concentrations between 2.5 µM and 20 µM for 72 h and apoptosis was determined according to Nicoletti's procedure (Riccardi and Nicoletti 2006). BD CellQuest Pro software (Becton Dickinson) for subG1 peak detection was used to determine the extent of the apoptotic cell formation. Statistical evaluation of the half maximal lethal dose-(LD₅₀) values was assessed by using SigmaPlot (Systat Software, Inc., version 10).

In all cell lines, a dose-dependent apoptosis inducing effect of Sunitinib was observed. The seven cell lines displayed varying Sunitinib sensitivities with no apparent difference between cancer types (Fig. 1 B/C). The cell lines A704 and WM115 exhibited the highest sensitivity to Sunitinib treatment with LD₅₀-values of 2.2 ± 0.4 µM and 3.6 ± 0.9 µM. Lower sensitivities were observed for the cell lines A498 and WM266-4 with LD₅₀-values of 12.4 ± 0.4 µM and 6.4 ± 1.3 µM. The highest LD₅₀-values were monitored for the cell lines CAKI2 (13.5 ± 3.9 µM), C8161 (15.5 ± 0.7 µM) and CAKI1 (16.6 ± 1.5 µM).

### Example 2: Generation of Sunitinib- and Sorafenib desensitized polyclonal cell lines

To identify genes that counteract the therapeutic effect of Sunitinib, we treated parental tumor cell lines with 20 µM of Sunitinib in regular intervals of three to five days two to seven times, mimicking the patient treatment regimen in the clinic (Pfizer 2006). The progress of Sunitinib desensitization was monitored by determining the rate of apoptotic cell appearance after 72 h of treatment.

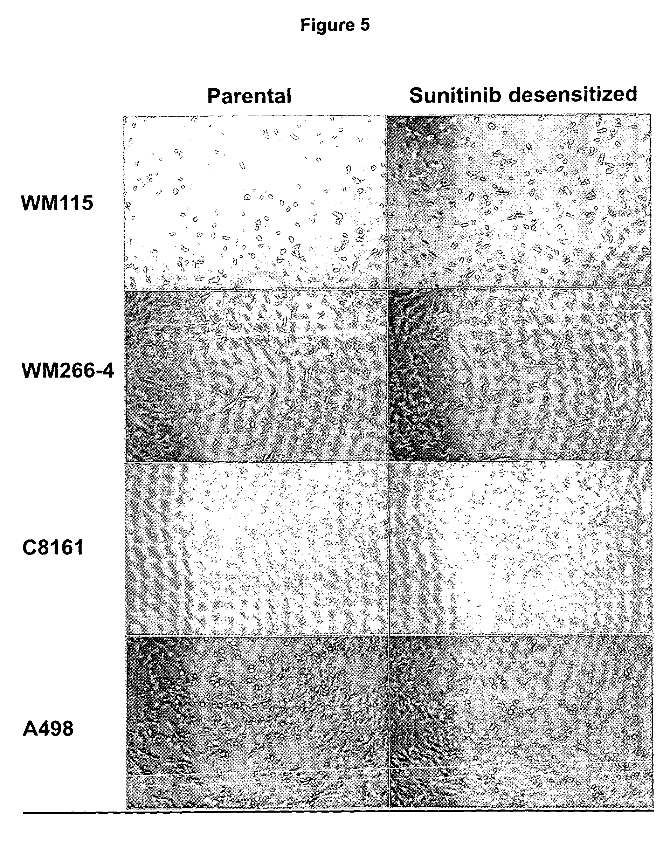

Compared to the parental cell lines, the Sunitinib resistant cell populations displayed alterations in the sensitivity against Sunitinib, whereas the degree of the desensitization varied. Notably, no change in cell-morphology as a consequence of the selection process with Sunitinib was observed. The exception to this was A704, which underwent changes in size and shape upon desensitization against Sunitinib (Fig. 5). The Sunitinib desensitized cell lines WM115^{SDes}, WM266-4^{SDes}, C8161^{Sdes}, A498^{SDes} and A704^{SDes} exhibited an increase of the LD₅₀-values of 301%, 174 %, 112 %, 122 % and 344 %, respectively (Fig. 1 B/C).

To determine the drug specificity of the selection process, we also established Sorafenib selected polyclonal cell populations using the same conditions. The Sorafenib selected kidney cell lines A498^{NDes}, A704^{NDes} CAKI1^{NDes} and CAKI2^{NDes} displayed no differences in the Sorafenib LD₅₀-values, suggesting that they are not prone to the Sorafenib selection procedure. Accordingly, the respective Sunitinib LD₅₀-values for these cell lines were similar in comparison to the parental cell lines. The exception to this was A704^{NDes}, which surprisingly demonstrated an increased Sunitinib LD₅₀-value of 9.0 ± 2.5 µM (419 %). The Sorafenib desensitized melanoma cell lines WM115^{NDes} (197 %), WM266-4^{NDes} (130 %) and C8161^{NDes} (249 %) displayed decreased sensitivities against Sorafenib compared to the parental cell lines (Fig. 2A). Interestingly, these cell lines also demonstrated altered sensitivities against Sunitinib treatment (Fig. 2B). WM115^{Ndes} and WM266-4^{Ndes} cells showed an increase of the Sunitinib LD₅₀-values of 249 % and 168 %, respectively, indicating related desensitization pathways of the two drugs, which also share similarities in their therapeutic target profile (Bayer; Pfizer 2006). Moreover, C8161^{Ndes} cells exhibited a decrease in Sunitinib sensitivity of 77 %. This decrease and the by trend diverse Sunitinib sensitivities between the Sunitinib and Sorafenib selected cell lines suggested that Sunitinib selected for different cell sub-populations with diverse genetic backgrounds than Sorafenib.

### Example 3: Expression of PRKX, TTBK2 and RSK4 kinases correlates with Sunitinib insensitivity in parental and Sunitinib desensitized kidney carcinoma- and melanoma cell lines

The differential potencies of Sunitinib to induce apoptosis in the parental kidney carcinoma- and melanoma cell lines and their desensitized derivative cell populations, suggested different mechanisms within the cells to evade the therapeutic effects of the drug. Assuming that the differences are caused by differentially altered gene expression profiles, we performed macro-array analyses to examine a potential correlation between transcriptional profile- characteristics and Sunitinib resistance. The focused cDNA-array used included kinases, phosphatases and genes playing a role in the cellular signal transduction network, such as those implicated in angiogenesis, metastasis, proliferation and survival. The cells analyzed included the kidney carcinoma lines A498, A704, CAKI1, CAKI2, the melanoma cell lines WM116, WM266-4 and C8161 and the corresponding Sunitinib desensitized cell population derivates.

### Gene-expression analysis

Plasmids were expressed in E. coli DH5α bacteria cells and plasmid cDNA was extracted by using QIAGEN Plasmid Maxi Kits (QIAGEN GMBH, Germany) following the manufacturers' protocols. The cDNA-plasmids were denaturated at 95 °C, incubated with 0.35M NaOH and 0.3M NH₄O acetate and stained with bromo-phenol (0.1 M) and printed on Hybond-N+ nylon transfer membranes (Amersham Pharamacia Biotech, GE Healthcare) using the MicroGrid II with connected TAS suite (Genomic Solutions®, version: 4.00.2). The cDNA was synthesized by using 5 µg extracted total RNA, oligo-dT Primers (K1, SMART; Clontech Inc., USA) and Avian Myeloblastosis Virus reverse transcriptase (AMV; Roche Applied Science, Germany).
The cDNA labeling was assessed by using the Megaprime Labeling Kit (GE Healthcare) with deoxyadenosine 5'-triphosphate, [alpha-33P] (GE Healthcare/PerkinElmer) following the manufacturer's protocol. A phosphor-imager system (Fuji BAS 1000) was used to quantify the hybridization signals.

### Statistical analysis

To identify Sunitinib sensitivity related expression patterns, we performed a "Significance Analysis of Microarrays" ("SAM") (Tusher, Tibshirani et al. 2001) using the Multi Experiment Viewer (SAEDD et al. 2003) with subsequent hierarchical clustering (Eisen, Spellman et al. 1998) employing three different groups: Group 1: parental cell lines; Sunitinib desensitized sub-populations with a high (Group 2) or low (Group 3) degree of desensitization. P-values were obtained by comparing the gene-expression levels of each Sunitinib desensitized- with the respective parental cell line. Data shown are the means ± standard deviation of at least three independent experiments. P-Values are symbolized by an Asterix: p-value ≤ 0.05 or double asterix: p-value ≤ 0.001.

For the kidney carcinoma lines, SAM revealed 49 genes, which were upregulated in the Sunitinib desensitized cells A498^{SDes} and A704^{SDes} but not altered in CAKI1^{SDes} and CAKI2^{SDes} (Fig. 3A). For the melanoma cell lines analyzed, SAM evaluation revealed 26 up- and 3 downregulated genes in WM11^{5SDes} and WM266-4^{SDes} cells. In C8161^{SDes}, demonstrating a low degree of Sunitinib desensitization, only ten genes displayed in- or decreased expression (Fig. 3B). As both gene-expression patterns strongly correlated with the degree of the Sunitinib desensitization, a role of the identified genes in Sunitinib insensitivity was indicated. While the subsets of the identified genes differed between the kidney- and melanoma population, five genes, including four serine/threonine kinases and one phosphatase, displayed a similar change of gene-expression: interleukin-1 receptor-associated kinase 1 (IRAK1), protein kinase, x-linked (PRKX), ribosomal protein S6 kinase (polypeptide 6; RPS6KA6/RSK4), tau tubulin kinase 2 (TTBK2) and dual specificity phosphatase 4 (DUSP4). Thus, these genes were of particular interest as they might be involved in Sunitinib resistance formation independent of the cancer type. Additionally, tau tubulin kinase 1 (TTBK1) expression was also elevated within A498^{SDes} and A704^{SDes} cells, indicating a major role of the TTBK family in mediating Sunitinib resistance in kidney cancer. Notably, the gene-expression fold-changes of PRKX, TTBK2 and RSK4 were higher in the melanoma cell lines WM115^{SDes} and WM266-4^{SDes} than in A498^{SDes} and A704^{SDes} cells (Fig. 3C). Other identified genes of interest, since they have been associated with melanoma cell survival (Saadeddin, Babaei-Jadidi et al. 2009; Zhu, Wurdak et al. 2009), were TYRO3 protein tyrosine kinase (TYRO3) and β-catenin (CTNNB1), whose increased gene-expression was specific to the melanoma population.
Accordingly, PRKX, TTBK2, RPS6KA6/RSK4, TYRO3 and CTNNB1 expression was validated by semi-quantitative RT-PCR and/or SDS-PAGE (Fig. 3D/E).

### SDS-PAGE

Antibodies against PRKX (ab67446), TTBK2 (ab67839) and β-catenin (BD610153) and Horseradish peroxidase-conjugated rabbit secondary antibodies were purchased from Abcam, BD Transduction Labs and Bio-Rad. Anti-β-actin, and horseradish peroxidase-conjugated mouse secondary antibodies were purchased from Sigma. Cells were lysed with RIPA lysis-buffer containing proteinase- and phosphatase-inhibitors. PVDF-membranes were blocked with 3 % milk-powder. Used anti-body dilutions ranged from 1:500 to 1:1000. Western Lightning Plus® (PerkinElmer) was used to detect the chemoluminescence reaction.

Note that parental A704 and A704^{SDes} cells, due to the shape and size changes, were not taken into account for further analysis. The increased protein-expression of PRKX and TTBK2 was specific to the Sunitinib desensitized cell lines and no upregulation was observed in the Sorafenib desensitized melanoma cell lines WM115^{NDes} and WM266-4^{NDes}, indicating their specific role in conferring Sunitinib resistance. In contrast, β-catenin expression was increased in both Sunitinib- and Sorafenib desensitized WM115 and WM266-4 cells (Fig. 3E), suggesting an expression based resistance mechanism that is common to the two kinase inhibitors.

### Example 4: Reduction of PRKX, TTBK2 or RSK4 triggers Sunitinib sensitization

Our gene expression analysis strongly indicated a connection between Sunitinib insensitivity and a function of PRKX, TTBK2 and RSK4. To confirm the role of these genes in this phenotype, we determined whether selective reduction of their expression by siRNA would be sufficient to restore Sunitinib sensitivity.

### Knock-down of candidate genes

The cells were seeded with equal numbers in 6-Well plates and transfected with 40 pmol siRNA (PRKX (AMBION, cat. no.: s224410, s224411, s11195), TTBK2 (AMBION, cat. no.: s44812, s44813, s44814), RSK4/RPS6KA6 (AMBION, cat. no.: s223751, s223752, s223753) and 5 µl lipofectamine RNAiMAX (Invitrogen) following the manufacturers' protocols. To determine the knock-down efficiency, semi-quantitative RT-PCR was performed (Primer list, see table 1).

**Table 1: Primer used for RT-PCR**

| **Primer Name** | | **Sequence (5' to 3')** | **Annealing (C°)** |
|---|---|---|---|
| TYRO3 | Forward/SEQ ID7 | TGCCTGCAGCCCCCTTCAACA | 61 |
| | Reverse/SEQ ID8 | AGGGGCCTCGGGGATCACTTCTTC | |
| PRKX | Forward/SEQ ID9 | GCACCACGGGGCTCTTCTACTCTG | 66 |
| | Reverse/SEQ ID10 | CACATCATTCGCCCCGTTCTTCAT | |
| RPS6KA6/ | Forward/SEQ ID11 | GCGAGCAGCGGCGAGGTAA | 64 |
| RSK4 | Reverse/SEQ ID12 | CAAGGGCCAGTTCTGCGAGGTAG | |
| TTBK2 | Forward/SEQ ID13 | GCGGAGCCGGCAGCAGCAGTAA | 58 |
| | Reverse/SEQ ID14 | AGCCCCCACCCCCAATCTTTCTCA | |
| GAPDH | Forward/SEQ ID15 | ACCACAGTCCATGCCATCAC | 55 |
| | Reverse/SEQ ID16 | TCCACCACCCTGTTGCTGTA | |

To determine the impact of reduced gene-expression on Sunitinib induced apoptosis, cells were cultured in the presence of Sunitinib for 72 h. Apoptotic cell formation was monitored according to Nicoletti's procedure. To measure the influence of reduced gene-expression on Sunitinib anti-migratory effects, transfected cells were serum starved in medium containing 0.1% FCS for 24 h.

Then, cells were seeded onto a membrane with 8 µm pores of a modified Boyden chamber (Schubert and Weiss) containing 500 µL serum-free medium. 10 % FCS served as chemo-attractant. After treatment with Sunitinib for 12 h, migration was evaluated visually and via crystal-violet staining with subsequent photometric analysis.

To analyze the increased Sunitinib-induced apoptosis (AdA) upon gene-expression reduction compared to the control siRNA, apoptosis was determined using PI-Assays after 72 h. Gene knock-downs ranged between 50% and 90%, as estimated by scanning densitometry (Fig. 4A).
When PRKX was reduced, WM115 showed a slight increase in sensitivity to Sunitinib treatment compared to the control siRNA transfected cell line (AdA^{WM115}: 3.2 ± 0.4 %, Fig. 4B). A more distinct effect on Sunitinib sensitivity was observed upon PRKX reduction on the cell lines WM266-4, C8161 and A498 (AdA^{WM266-4}: 22.4 ± 6.7 %; AdA^{C8161}: 19.4 ± 2.4 %; AdA^{A498} : 6.5 ± 3.2 %), which correlated with their higher endogenous expression-levels of this kinase. Interestingly, the Sorafenib desensitized cell lines also displayed increased Sunitinib sensitivity subsequent to PRKX reduction, matching the effect within the respective parental cell lines (AdA^{WM115NDes:} 3.9 ± 4.8 %; AdA^{WM266-4NDes}: 23.0 ± 5.4 %; AdAC^{8161NDes}: 18.1 ± 8.8 %). In the Sunitinib desensitized cell lines, however, this effect was strikingly higher. PRKX reduction in WM115^{SDes} cells resulted in an additional apoptosis of 36.1 ± 3.2 % compared to the control. Similar observations were made in WM266-4^{SDes}, C8161^{SDes} and A498^{SDes} cells, where PRKX reduction triggered Sunitinib sensitivity (AdA^{WM266-4SDes}: 34.1 ± 4.9 %; AdA^{C8161SDes} **:** 35.8 ± 9.9 %; AdA^{A498SDes}: 14.7 ± 4.4 %)_{.}
The reduction of TTBK2 expression had a minor effect on Sunitinib sensitivity in parental WM115 and WM266-4 cells, whereas, correlating with a slightly higher endogenous TTBK2 gene-expression, in C8161 as well as in kidney A498 cells the effect was more pronounced (AdA^{WM115}: 4.8 ± 1.5 %; AdA^{WM266-4}: 5.1 ± 2.0 %; AdA^{C8161}: 7.3 ± 4.8 %; AdA^{A498}: 7.1 ± 3.1 %, Fig. 4C). In the Sunitinib desensitized sub-populations, however, the re-sensitization effect was clearly higher compared to the respective parental cell lines: WM115^{SDes} 36.8 ± 10.5 %, WM^{266-4SDes}: 16.8 ± 7.7 %, C8161^{SDes}: 20.3 ± 8.1 % and A498^{SDes}: 13.6 ± 0.4 %. Interestingly, increased Sunitinib sensitivity upon TTBK2 reduction was also observed in the Sorafenib desensitized cell lines WM115^{NDes} and WM266-4^{NDes}. Here, the additional apoptosis ranged in between the parental and Sunitinib desensitized cell lines and did not correlate with the observed endogenous protein-expression (AdA^{WM115NDes}: 11.7 ± 4.4 %; AdA^{WM266-4NDes}: 12.7 ± 2.5 %). Within the Sorafenib desensitized sub-population C8161^{NDes}, no Sunitinib sensitizing effect upon TTBK2 reduction was observed (AdA^{C8161NDes}:-2.2 ± 6.8 %). As PRKX and TTBK2 reduction sensitized the cells for Sunitinib treatment in strong correlation with their endogenous gene-expression, their crucial role in compensating the therapeutic effects of Sunitinib is conceivable. The relative gene-expression of RSK4 was increased in the Sunitinib desensitized- compared to the parental cell lines. Like for PRKX and TTBK2, the increase in gene-expression strongly correlated with the degree of desensitization. Hence, we reduced the RSK4-expression, to examine its potential role as a cellular mechanism to evade Sunitinib induced apoptosis. Reduction of RSK4 resulted in an increased Sunitinib induced apoptosis rate in the kidney carcinoma- and melanoma cell lines (Fig. 4D). Within the parental cell lines, a small increase in sensitivity against Sunitinib was observed (AdA^{WM115}: 4.8 ± 2.3 %; AdA^{WM266-4}: 7.1 ± 2.0 %; AdA^{C8161}: 12.5 ± 3.6 %; AdA^{A498}: 6.5 ± 3.2 %). In line with the higher endogenous RSK4 gene-expression, the Sunitinib desensitized cell lines WM115^{SDes} (AdA: 14.1 ± 3.2 %), WM266-4^{SDes} (AdA: 20.2 ± 5.7 %) and A498^{SDes} (AdA: 14.7 ± 4.4 %) demonstrated notable higher, Sunitinib induced apoptotic values. However, within the Sunitinib desensitized cell line C8161^{SDes} - with no change in RSK4- expression - no change in Sunitinib sensitivity compared to the parental cell line was observed (AdA^{C8161SDes}: 11.9 ± 4.8 %). RSK4 reduction in the Sorafenib desensitized sub-population WM115^{NDes} resulted in an increased Sunitinib sensitivity comparable with the sensitization of the respective parental cell lines (AdAWM115^{NDes}:5.6 ± 3.8 %). In WM266-4^{NDes}, the additional apoptosis was by trend-, in C8161^{NDes} notably lower than for their respective parental cell line (AdA^{WM266-4NDes}: 5.3 ± 5.2 %, AdA^{C8161NDes}: 6.0 ± 2.1 %). It is noteworthy that PRKX, TTBK2 or RSK4 reduction without a subsequent Sunitinib treatment did not result in a change in the level of apoptosis (data not shown).
Summarized, specific RSK4 reduction led to an increased Sunitinib sensitivity in terms of apoptosis induction. Like for PRKX and TTBK2, the strong correlation of endogenous RSK4 expression and Sunitinib sensitivity as well as the effects of RSK4 reduction on Sunitinib induced apoptosis suggest its role in mediating Sunitinib insensitivity.

### Example 5: Reduction of PRKX, TTBK2 and RSK4 increases Sunitinib inhibition of cancer cell migration

Since Sunitinib inhibits cell-migration (Osusky, Hallahan et al. 2004), we consequently addressed the question whether PRKX, TTBK2 and RSK4 also had an impact on Sunitinib anti-migratory activity. For this purpose, we specifically reduced these genes with subsequent Sunitinib treatment and performed Boyden Chamber migration assays with parental and Sunitinib desensitized A498 cells.

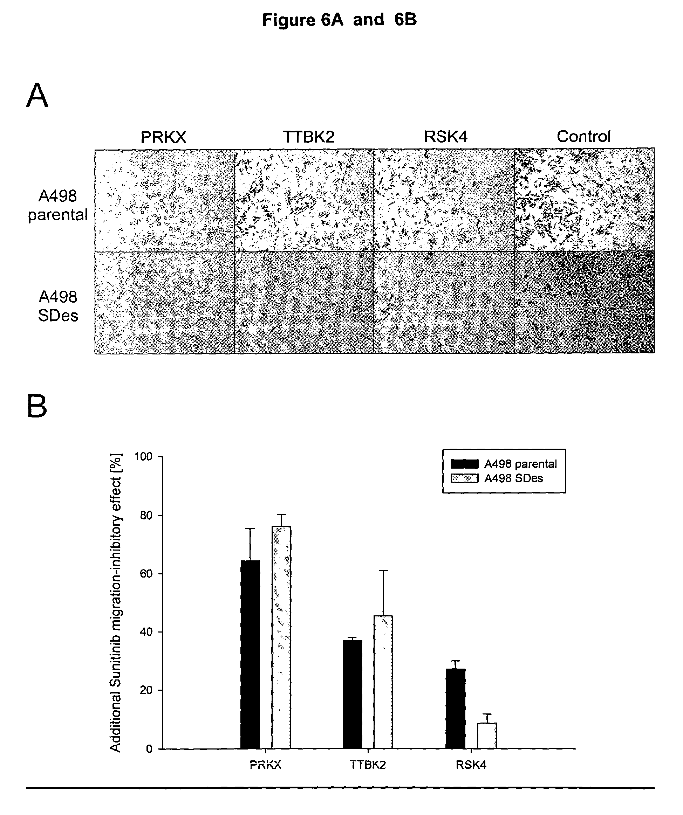

In parental A498 cells, PRKX reduction resulted in an 64.4 ± 11.1 % inhibition of migration compared to the scrambled siRNA control (Fig. 6). TTBK2 or RSK4 reduction, with 37.1 ± 1.0 % or 27.3 ± 2.7 %, led to a less distinct increase in Sunitinib inhibition of migration. The reduction of PRKX and TTBK2 in A498^{SDes} cells matched the results of the parental ones: Here, PRKX and TTBK2 reduction initiated an increased Sunitinib inhibitory effect of 76.4 ± 4.1 % and 45.5 ± 15.6 %. RSK4 reduction also resulted in an increased Sunitinib anti-migratory effect (8.6 ± 3.1 %), which was weaker compared to the respective effect in the parental cell line.

Summarized, PRKX, TTBK2 and RSK4 reduction not only mediated induced apoptosis, but also affected the Sunitinib induced anti-migratory effects. Hence, an overall Sunitinib sensitivity triggering effect by these kinases is indicated, which underlines their striking role in the formation of Sunitinib resistance.

## Claims

1. A method of determining if a subject suffering from a proliferative disease has developed a sunitinib resistance, comprising:
measuring the expression level of PRKX, TTBK2 and/or RSK4
in a sample comprising diseased cells from said subject, wherein an increased PRKX, TTBK2 and/or RSK4 expression level is indicative for a sunitinib resistance.

2. The method of claim 1, wherein the subject is a cancer patient, particularly a human cancer patient.

3. The method of claim 1 or 2, wherein the proliferative disease is selected from the group consisting of acute lymphoblastic leukemia, acute myeloid leukemia, adrenocortical carcinoma, aids-related cancers, anal cancer, appendix cancer, astrocytomas, atypical teratoid/rhabdoid tumor, basal cell carcinoma, bile duct cancer, bladder cancer, bone cancer, osteosarcoma and malignant fibrous histiocytoma, brain stem glioma, brain tumor, central nervous system atypical teratoid/rhabdoid tumor, astrocytomas, craniopharyngioma, ependymoblastoma, ependymoma, medulloblastoma, medulloepithelioma, pineal parenchymal tumors, supratentorial primitive neuroectodermal tumors and pineoblastoma, brain and spinal cord tumors, breast cancer, bronchial tumors, burkitt lymphoma, carcinoid tumor, carcinoid tumor, gastrointestinal tumor, central nervous system lymphoma, cervical cancer, chordoma, chronic lymphocytic leukemia, , chronic myeloproliferative disorders, colon cancer, colorectal cancer, craniopharyngioma, cutaneous t-cell lymphoma, mycosis fungoides and sézary syndrome, endometrial cancer, ependymoblastoma, ependymoma, esophageal cancer, esthesioneuroblastoma, ewing sarcoma family of tumors, extracranial germ cell tumor, extragonadal germ cell tumor, extrahepatic bile duct cancer, intraocular melanoma, retinoblastoma, gallbladder cancer, gastric cancer, gastrointestinal carcinoid tumor, gastrointestinal stromal tumor, gastrointestinal stromal cell tumor, gestational trophoblastic tumor, glioma, hairy cell leukemia, head and neck cancer, heart cancer, hepatocellular cancer, histiocytosis, hodgkin lymphoma, hypopharyngeal cancer, intraocular melanoma, islet cell tumors, kaposi sarcoma, renal cell cancer, kidney cancer, langerhans cell histiocytosis, laryngeal cancer, chronic lymphocytic leukemia, chronic myelogenous leukemia lip and oral cavity cancer, liver cancer, non-small cell lung cancer, aids related lymphoma, mycosis fungoides and sézary syndrome, non-hodgkin lymphoma, lymphoma primary central nervous system, macroglobulinemia, malignant fibrous histiocytoma of bone and osteosarcoma, medulloblastoma, medulloepithelioma, melanoma, merkel cell carcinoma, mesothelioma, metastatic squamous neck cancer with occult primary, mouth cancer, multiple endocrine neoplasia syndromes, multiple myeloma/plasma cell neoplasm, mycosis fungoides, myelodysplastic syndromes, myelodysplastic/myeloproliferative neoplasms, myelogenous leukemia, myeloid leukemia, multiple myeloma, myeloproliferative disorders, nasal cavity and paranasal sinus cancer, nasopharyngeal cancer, neuroblastoma, oral cancer, oral cavity cancer, oropharyngeal cancer, osteosarcoma and malignant fibrous histiocytoma of bone, ovarian cancer, ovarian epithelial cancer, ovarian germ cell tumor, ovarian low malignant potential tumor, pancreatic cancer, papillomatosis, parathyroid cancer, penile cancer, pharyngeal cancer, pineal parenchymal tumors of intermediate differentiation, pineoblastoma and supratentorial primitive neuroectodermal tumors, pituitary tumor, plasma cell neoplasm/multiple myeloma, pleuropulmonary blastoma, pregnancy and breast cancer, prostate cancer, rectal cancer, transitional cell cancer, respiratory tract cancer, retinoblastoma, rhabdomyosarcoma, salivary gland cancer, skin cancer, melanoma, skin carcinoma, small cell lung cancer, small intestine cancer, soft tissue sarcoma, squamous cell carcinoma, squamous neck cancer, supratentorial primitive neuroectodermal tumors, t-cell lymphoma, mycosis fungoides and sézary syndrome, testicular cancer, throat cancer, thymoma and thymic carcinoma, thyroid cancer, transitional cell cancer of the renal pelvis and ureter, trophoblastic tumor, gestational, ureter and renal pelvis, urethral cancer, uterine cancer, endometrial, uterine sarcoma, vaginal cancer, vulvar cancer, waldenström macroglobulinemia and wilms tumor.

4. The method of claim 3, wherein the cancer is kidney cancer, breast cancer or melanoma.

5. The method of any one of claims 1 to 4, wherein the expression of PRKX, TTBK2 and/or RSK4 is determined on the protein level, or wherein the expression of PRKX, TTBK2 and/or RSK4 is determined on the nucleic acid level.

6. The method of any one of claims 1 to 5, wherein the sample is a tissue sample or a body fluid sample comprising tumor cells.

7. The method of any one of claims 1 to 6, wherein the expression level is increased in said diseased cells at least 1.5-fold compared to the expression level in normal cells from said subject.

8. A modulator for
(i) the inhibition of a polypeptide selected from PRKX, TTBK2 and/or RSK4
or for
(ii) the inhibition, deactivation, degradation, intercalation, cleavage or down-regulation of a nucleic acid selected from PRKX, TTBK2 and/or RSK4
for reduction or prevention of sunitinib resistance of a proliferative disease.

9. The modulator of claim 8, wherein the proliferative disease is selected from cancer, tumors and metastases.

10. The modulator of claim 8 or 9, wherein the proliferative disease is selected from the group consisting of acute lymphoblastic leukemia, acute myeloid leukemia, adrenocortical carcinoma, aids-related cancers, anal cancer, appendix cancer, astrocytomas, atypical teratoid/rhabdoid tumor, basal cell carcinoma, bile duct cancer, bladder cancer, bone cancer, osteosarcoma and malignant fibrous histiocytoma, brain stem glioma, brain tumor, central nervous system atypical teratoid/rhabdoid tumor, astrocytomas, craniopharyngioma, ependymoblastoma, ependymoma, medulloblastoma, medulloepithelioma, pineal parenchymal tumors, supratentorial primitive neuroectodermal tumors and pineoblastoma, brain and spinal cord tumors, breast cancer, bronchial tumors, burkitt lymphoma, carcinoid tumor, carcinoid tumor, gastrointestinal tumor, central nervous system lymphoma, cervical cancer, chordoma, chronic lymphocytic leukemia, , chronic myeloproliferative disorders, colon cancer, colorectal cancer, craniopharyngioma, cutaneous t-cell lymphoma, mycosis fungoides and sézary syndrome, endometrial cancer, ependymoblastoma, ependymoma, esophageal cancer, esthesioneuroblastoma, ewing sarcoma family of tumors, extracranial germ cell tumor, extragonadal germ cell tumor, extrahepatic bile duct cancer, intraocular melanoma, retinoblastoma, gallbladder cancer, gastric cancer, gastrointestinal carcinoid tumor, gastrointestinal stromal tumor, gastrointestinal stromal cell tumor, gestational trophoblastic tumor, glioma, hairy cell leukemia, head and neck cancer, heart cancer, hepatocellular cancer, histiocytosis, hodgkin lymphoma, hypopharyngeal cancer, intraocular melanoma, islet cell tumors, kaposi sarcoma, renal cell cancer, kidney cancer, langerhans cell histiocytosis, laryngeal cancer, chronic lymphocytic leukemia, chronic myelogenous leukemia lip and oral cavity cancer, liver cancer, non-small cell lung cancer, aids related lymphoma, mycosis fungoides and sézary syndrome, non-hodgkin lymphoma, lymphoma primary central nervous system, macroglobulinemia, malignant fibrous histiocytoma of bone and osteosarcoma, medulloblastoma, medulloepithelioma, melanoma, merkel cell carcinoma, mesothelioma, metastatic squamous neck cancer with occult primary, mouth cancer, multiple endocrine neoplasia syndromes, multiple myeloma/plasma cell neoplasm, mycosis fungoides, myelodysplastic syndromes, myelodysplastic/myeloproliferative neoplasms, myelogenous leukemia, myeloid leukemia, multiple myeloma, myeloproliferative disorders, nasal cavity and paranasal sinus cancer, nasopharyngeal cancer, neuroblastoma, oral cancer, oral cavity cancer, oropharyngeal cancer, osteosarcoma and malignant fibrous histiocytoma of bone, ovarian cancer, ovarian epithelial cancer, ovarian germ cell tumor, ovarian low malignant potential tumor, pancreatic cancer, papillomatosis, parathyroid cancer, penile cancer, pharyngeal cancer, pineal parenchymal tumors of intermediate differentiation, pineoblastoma and supratentorial primitive neuroectodermal tumors, pituitary tumor, plasma cell neoplasm/multiple myeloma, pleuropulmonary blastoma, pregnancy and breast cancer, prostate cancer, rectal cancer, transitional cell cancer, respiratory tract cancer, retinoblastoma, rhabdomyosarcoma, salivary gland cancer, skin cancer, melanoma, skin carcinoma, small cell lung cancer, small intestine cancer, soft tissue sarcoma, squamous cell carcinoma, squamous neck cancer, supratentorial primitive neuroectodermal tumors, t-cell lymphoma, mycosis fungoides and sézary syndrome, testicular cancer, throat cancer, thymoma and thymic carcinoma, thyroid cancer, transitional cell cancer of the renal pelvis and ureter, trophoblastic tumor, gestational, ureter and renal pelvis, urethral cancer, uterine cancer, endometrial, uterine sarcoma, vaginal cancer, vulvar cancer, waldenström macroglobulinemia and wilms tumor.

11. The modulator of claim 10, wherein the proliferative disease is selected from kidney cancer, breast cancer or melanoma.

12. The modulator of any one of claims 9 - 11 for use in medicine.

13. The modulator of any one of claims 9 - 12, wherein the modulator is an inhibitor acting on the protein level or wherein the modulator is an antibody or an antigen-binding fragment or a derivative thereof or wherein the modulator is an aptamer or wherein the modulator is an inhibitor acting on the nucleic acid level or wherein the modulator is an antisense molecule, a ribozyme, a siRNA molecule or a miRNA molecule or a precursor thereof.

14. A pharmaceutical composition comprising a modulator of any one of claims 9 - 13 and one further antiproliferative agent.

15. The composition of claims 14 comprising sunitinib as the further antiproliferative agent.
